# EUROPEAN PATENT SPECIFICATION

(11) **EP 2 311 468 B1**
(45) Date of publication and mention of the grant of the patent: **15.01.2014**
(21) Application number: 10178204.3
(22) Date of filing: 06.08.2004
(51) Int. Cl.: A61K 31/711, C07K 14/82, C07K 16/30, C12Q 1/68, G01N 33/574

(54) **Gene overexpressed in cancer**
In Krebs überexprimiertes Gen
Gène surexprimé dans le cancer

(30) Priority: 08.08.2003 JP 2003290704
(43) Date of publication of application: 20.04.2011
(62) Divisional of application: 04771625.3
(73) Proprietor: Perseus Proteomics Inc., Tokyo 153-0041 (JP); Chugai Seiyaku Kabushiki Kaisha, Kita-ku Tokyo 115-8543 (JP); Aburatani, Hiroyuki, Musashino-shi, Tokyo 180-0003 (JP)
(72) Inventor: Aburatani, Hiroyuki, Tokyo 180-0003 (JP); Hippo, Yoshitaka, Tokyo 201-0004 (JP); Taniguchi, Hirokazu, Tokyo 154-0003 (JP); Chen, Yong xin, St. Louis MO 63105 (US); Ishikawa, Shumpei, Tokyo 110-0008 (JP); Fukumoto, Shin-ichi, Sapporo-shi Hokkaido 060-0035 (JP); Shimamura, Takahiro, Fujieda-shi Shizuoka 426-0075 (JP); Kamimura, Naoko, Tokyo 171-0021 (JP); Guo, Ying qiu, St. Louis MO 63105 (US); Yamamoto, Shogo, Tokyo 103-0006 (JP); Ito, Yukio, Tokyo 153-0041 (JP); Ito, Hirotaka, Shizuoka 412-8513 (JP); Ohtomo, Toshihiko, Shizuoka 412-8513 (JP)
(74) Representative: Vossius & Partner

(56) References cited:
- WO-A2-02/068579
- WO-A2-03/061681
- US-A1- 2003 054 368

## Description

### Technical Field

The present invention relates to a cancer-associated gene, a protein encoded by the gene, and an antibody recognizing this protein. The gene, protein and antibody of the present invention can be used in diagnosis and treatment of liver cancer and development of a therapeutic drug of cancer.

### Background Art

Heretofore, a number of genes have been found whose expression level changes as being associated with malignant transformation of cells and antigens that can be used as a cancer marker, and numerous studies on such genes have been carried out. However, it is still difficult to specifically detect or treat a specific cancer. Therefore, in this technical field, identification of another cancer-associated gene or protein that can be used in diagnosis and treatment of cancer has been demanded.

There are prior art documents related to the present invention: EP 1033401; US 2002022248; US 200.2042096; US 200208150; US 6337195; US 6362321; WO 9738098; WO 9920764; WO 9929729; WO 0006698; WO 0012702; WO 0034477; WO 0036107; WO 0037643; WO 0055174; WO 0055320; WO 0055351; WO 0055633; WO 0058473; WO 0073509; WO 0100828; WO 0109317: WO 0121653; WO 0122920; WO 0151513: WO 0151628; WO 0154733; WO 0155355; WO 0157058; WO 0159111; WO 0160860; WO 0164835; WO 0164886; WO 0166719; WO 0170976; WO 0173027; WO 0175177; WO 0177168; WO 0192578; WO 0194629; WO 0200677; WO 0200889; WO 0200939; WO 0204514; WO 0210217; WO 0212280; WO 0220598; WO 0229086; WO 0229103; WO 0258534; WO 0260317; and WO 0264797.

WO 03/061681 A2 relates to certain diseases which involve energy homeostasis and organelle metabolism.

WO 02/068579 A2 relates to DNA microarrays for the detection of gene expression.

US 2003/0054368 A1 relates to lung related polynucleotide and the polypeptides encoded by these polynucleotides, particularly "lung antigens", and the use of such lung antigens for detecting lung cancer.

An object of the present invention is to provide a gene and a protein that can be used as an agent for diagnosing and treating cancer.

### Disclosure of the invention

The present inventors have found that the expression of specific genes is elevated in a cancer tissue, and have completed the present invention. The present invention provides a protein encoded by a gene having a nucleotide sequence represented by SEQ ID NO: 15.

The present invention provides a gene having a nucleotide sequence represented by SEQ ID NO: 15, a protein encoded by the gene.

Such a protein is useful as a composition for diagnosing or treating liver cancer.

In addition, in a composition of the present invention, the gene has a nucleotide sequence represented by SEQ ID NO: 15.

In another aspect, the present invention provides a cell or a vector that expresses the above-mentioned gene.

Such a cell or a vector is useful for producing a protein of the present invention, producing an antibody against the protein, as well as diagnosing or treating liver cancer and the like.

In still another aspect, the present invention provides a protein having an amino acid sequence represented by SEQ ID NO: 72. Such a protein or a fragment thereof is useful as an antigen for producing an antibody or is useful for diagnosing or treating liver cancer.

In still another aspect, the present invention provides an antibody recognizing the above-mentioned protein or an antigen-binding fragment thereof. The present invention also provides a cell producing such an antibody.

In still another aspect, the present invention provides a polynucleotide having a nucleotide sequence represented by SEQ ID NO: 15 or a nucleotide sequence complementary thereto.

Such a polynucleotide is useful for diagnosis of liver cancer, production of a protein, and as a primer, an antisense and siRNA for inhibiting gene expression.

In still another aspect, the present invention provides a method of identifying a compound having an anticancer activity comprising the steps of: bringing a cultured human cell into contact with a test compound; and identifying a compound that causes a change in the expression level of a gene containing a nucleotide sequence represented by SEQ ID NO: 15 in the cell as a compound having an anticancer activity.

### Brief Description of the Drawings

Fig. 1 shows the results of an expression analysis of a cancer-associated gene TEG1.
Fig. 2 shows the results of an expression analysis of a cancer-associated gene TEG2.
Fig. 3 shows the results of an expression analysis of a cancer-associated gene TEG2.
Fig. 4 shows the results of an expression analysis of a cancer-associated gene TEG3.
Fig. 5 shows the results of an expression analysis of a cancer-associated gene TEG4.
Fig. 6 shows the results of an expression analysis of a cancer-associated gene TEG5.
Fig. 7 shows the results of an expression analysis of a cancer-associated gene TEG6.
Fig. 8 shows the results of an expression analysis of a cancer-associated gene TEG6.
Fig. 9 shows the results of an expression analysis of a cancer-associated gene TEG7.
Fig. 10 shows the results of an expression analysis of a cancer-associated gene TEG8.
Fig. 11 shows the results of an expression analysis of a cancer-associated gene TEG9.
Fig. 12 shows the results of an expression analysis of a cancer-associated gene TEG10.
Fig. 13 shows the results of an expression analysis of a cancer-associated gene TEG11.
Fig. 14 shows the results of an expression analysis of a cancer-associated gene TEG12.
Fig. 15 shows the results of an expression analysis of a cancer-associated gene TEG13.
Fig. 16 shows the results of an expression analysis of a cancer-associated gene TEG14.
Fig. 17 shows the results of an expression analysis of a cancer-associated gene TEG15.
Fig. 18 shows the results of an expression analysis of a cancer-associated gene TEG16.
Fig. 19 shows the results of an expression analysis of a cancer-associated gene TEG17.
Fig. 20 shows the results of an expression analysis of a cancer-associated gene TEG18.
Fig. 21 shows the results of an expression analysis of a cancer-associated gene TEG19.
Fig. 22 shows the results of an expression analysis of a cancer-associated gene TEG20.
Fig. 23 shows the results of an expression analysis of a cancer-associated gene TEG21.
Fig. 24 shows the results of an expression analysis of a cancer-associated gene TEG22.
Fig. 25 shows the results of an expression analysis of a cancer-associated gene TEG23.
Fig. 26 shows the results of an expression analysis of a cancer-associated gene TEG24.
Fig. 27 shows the results of an expression analysis of a cancer-associated gene TEG25.
Fig. 28 shows the results of an expression analysis of a cancer-associated gene TEG26.
Fig. 29 shows the results of an expression analysis of a cancer-associated gene TEG27.
Fig. 30 shows the results of an expression analysis of a cancer-associated gene TEG28.
Fig. 31 shows the results of an expression analysis of a cancer-associated gene TEG29.
Fig. 32 shows the results of an expression analysis of a cancer-associated gene TEG30.
Fig. 33 shows the results of an expression analysis of a cancer-associated gene TEG31.
Fig. 34 shows the results of an expression analysis of a cancer-associated gene TEG32.
Fig. 35 shows the results of an expression analysis of a cancer-associated gene TEG33.
Fig. 36 shows the results of an expression analysis of a cancer-associated gene TEG34.
Fig. 37 shows the results of an expression analysis of a cancer-associated gene TEG35.
Fig. 38 shows the results of an expression analysis of a cancer-associated gene TEG36.
Fig. 39 shows the results of an expression analysis of a cancer-associated gene TEG37.
Fig. 40 shows the results of an expression analysis of a cancer-associated gene TEG38.
Fig. 41 shows the results of an expression analysis of a cancer-associated gene TEG39.
Fig. 42 shows the results of an expression analysis of a cancer-associated gene TEG40.
Fig. 43 shows the results of an expression analysis of a cancer-associated gene TEG41.
Fig. 44 shows the results of an expression analysis of a cancer-associated gene TEG42.
Fig. 45 shows the results of an expression analysis of a cancer-associated gene TEG43.
Fig. 46 shows the results of an expression analysis of a cancer-associated gene TEG44.
Fig. 47 shows the results of an expression analysis of a cancer-associated gene TEG45.
Fig. 48 shows the results of an expression analysis of a cancer-associated gene TEG46.
Fig. 49 shows the results of an expression analysis of a cancer-associated gene TEG47.
Fig. 50 shows the results of an expression analysis of a cancer-associated gene TEG48.
Fig. 51 shows the results of an expression analysis of a cancer-associated gene TEG49.
Fig. 52 shows the results of an expression analysis of a cancer-associated gene TEG50.
Fig. 53 shows the results of an expression analysis of a cancer-associated gene TEG51.
Fig. 54 shows the results of an expression analysis of a cancer-associated gene TEG52.
Fig. 55 shows the results of an expression analysis of a cancer-associated gene TEG53.
Fig. 56 shows the results of an expression analysis of a cancer-associated gene TEG54.
Fig. 57 shows the results of an expression analysis of a cancer-associated gene TEG55.
Fig. 58 shows the results of an expression analysis of a cancer-associated gene TEG56.
Fig. 59 shows the results of an expression analysis of a cancer-associated gene TEG57.
Fig. 60 shows the results of an expression analysis of a cancer-associated gene TEG58.
Fig. 61 shows the results of an expression analysis of a cancer-associated gene TEG59.
Fig. 62 shows the results of an expression analysis of a cancer-associated gene TEG60.
Fig. 63 shows the results of an expression analysis of a cancer-associated gene TEG61.
Fig. 64 shows the results of an expression analysis of a cancer-associated gene TEG62.
Fig. 65 shows the results of an expression analysis of a cancer-associated gene TEG63.
Fig. 66 shows the results of an expression analysis of a cancer-associated gene TEG64.
Fig. 67 shows the nucleotide sequence and the amino acid sequence of a novel gene K#1.
Fig. 68 shows the alignment of a novel gene K#1 with the gene of GenBank No. XM_067369.
Fig. 69 shows the results of analyzing the amino acid sequence motif of a novel gene K#1.
Fig. 70 shows the nucleotide sequence and the amino acid sequence of a novel gene K#2 (clone 11).
Fig. 71 shows the nucleotide sequence and the amino acid sequence of a novel gene K#2 (clone 18).
Fig. 72 shows the comparison of the amino acid sequences of a novel gene K#2 (clone 11) with human LIN-28, Caenorhabditis elegans LIN-28, Xenopus laevis LIN-28, Drosophila LIN-28 and mouse LIN-28.
Fig. 73 shows the expression of C20orf102 gene in lung squamous cell carcinoma.
Fig. 74 shows the detection of a C20orf102 protein molecule in a variety of cancer cell lines and a culture supernatant thereof using an anti-C20orf102 antibody.
Fig. 75 shows the results of an expression analysis of C20orf102 protein in lung adenocarcinoma tissue using an anti-C20orf102 antibody.
Fig. 76 shows the detection of an hNotum protein molecule in a variety of cancer cell lines and a culture supernatant thereof using an anti-hNotum antibody.
Fig. 77 shows the results of an expression analysis of hNotum protein in lung cancer tissue using an anti-hNotum antibody.
Fig. 78 shows the detection of a K#2 protein molecule in a cell line forcibly expressing K#2 and a variety of cancer cell lines using an anti-K#2 antibody.
Fig. 79 shows the results of an expression analysis of K#2 protein in liver cancer tissue using an anti-K#2 antibody.
Fig. 80 shows the detection of a KIAA1359 protein molecule in a cell line forcibly expressing KIAA1359 and a variety of cancer cell lines using an anti-KIAA1359 antibody.
Fig. 81 shows the results of an expression analysis of KIAA1359 protein in stomach cancer tissue using an anti-KIAA1359 antibody.
Fig. 82 shows the detection of a PEG10 protein molecule in a cell line forcibly expressing PEG10 and a variety of cancer cell lines using an anti-PEG10/ORF2 antibody.
Fig. 83 shows the results of an expression analysis of PEG10 protein in hepatocellular carcinoma tissue using an anti-PEG10/ORF2 antibody.
Fig. 84 shows the detection of a DUSP9 protein molecule in a cell line forcibly expressing DUSP9 and a variety of cancer cell lines using an anti-DUSP9 antibody.
Fig. 85 shows the results of an expression analysis of DUSP9 protein in hepatocellular carcinoma tissue using an anti-DUSP9 antibody.
Fig. 86 shows the results of an expression analysis of Cystatin SN protein in large bowel cancer tissue using an anti-Cystatin SN antibody.
Fig. 87 shows the results of an expression analysis of SFRP4 protein in stomach cancer tissue using an anti-SFRP4 antibody.
Fig. 88 shows the detection of a SFRP4 protein molecule in a culture supernatant of COS7 cells which were forcibly made to express SFRP4 using an anti-SFRP4 antibody.

### Detailed Description of the Invention

The present invention provides a composition for diagnosing and treating liver cancer utilizing a specific gene whose expression is elevated in a cancer tissue and a protein encoded by the gene.

### Protein

In a first aspect, the present specification discloses a protein encoded by a cancer-associated gene represented by any of SEQ ID NOs: 1 to 65 or a fragment thereof. Preferably, a composition of the present invention comprises a protein having an amino acid sequence represented by any of SEQ ID NOs: 66 to 123 or a fragment thereof.

The protein or the fragment thereof of the present disclosure is useful for diagnosing or treating cancer or as an antigen for producing an antibody.

In the composition of the present disclosure, the protein or the fragment thereof may be a variant in which one or more amino acid residues are substituted, added or deleted from the above-mentioned sequence, as long as it has a desired immunogenicity. It is preferred that such a variant has an amino acid sequence with an identity of at least 80%, preferably 90% or more, more preferably 95% or more with the above-mentioned amino acid sequence.

The identity of amino acid sequence is calculated by dividing the number of identical residues by the total number of residues in two sequences to be compared and multiplying by 100. Several computer programs for determining the identity of sequences using standard parameters are available, for example, Gapped BLAST or PSI-BLAST (Altschul, et al. (1997) Nucleic Acids Res. 25: 3389-3402), BLAST (Altschul, et al. (1990) J. Mol. Biol. 215: 403-410) and Smith-Waterman (Smith, et al. (1981) J. Mol. Biol. 147: 195-197).

It has been already known that a protein having an amino acid sequence which has been modified by deletion, addition and/or substitution with another amino acid of one or more amino acid residues in a certain amino acid sequence retains the original biological activity (Mark, D. F. et al., Proc. Natl. Acad. Sci. USA (1984) 81, 5662-5666, Zoller, M. J. & Smith, M. Nucleic Acids Research (1982) 10, 6487-6500, Wang, A. et al., Science 224, 1431-1433, Dalbadie-McFarland, G. et al., Proc. Natl. Acad. Sci. USA (1982) 79, 6409-6413).

It is preferred that an amino acid residue to be mutated is replaced with another amino acid residue in which the classes of the property of the amino acid side chain is conserved. Examples of the property of an amino acid side chain include a hydrophobic amino acid (A, I, L, M, F, P, W, Y, V), a hydrophilic amino acid (R, D, N, C, E, Q, G, H, K, S, T), an amino acid having an aliphatic side chain (G, A, V, L, I, P), an amino acid having a hydroxyl group-containing side chain (S, T, Y), an amino acid having a sulfur atom-containing side chain (C, M), an amino acid having a carboxylic acid and amide-containing side chain (D, N, E, Q), an amino acid having a base-containing side chain (R, K, H), and an amino acid having an aromatic containing side chain (H, F, Y, W). The parenthetic letters indicate the one-letter codes of amino acids.

A protein that is functionally equivalent to a given protein can be prepared by a method known to those skilled in the art, for example, by introducing an appropriate mutation into the amino acid using a site-directed mutagenesis method (Gotoh, T. et al. (1995), Gene 152, 271-275; Zoller, M J, and Smith, M. (1983), Methods Enzymol. 100, 468-500; Kramer, W. et al. (1984), Nucleic Acids Res. 12, 9441-9456, Kramer W, and Fritz H J (1987) Methods. Enzymol. 154, 350-367, Kunkel, T A (1985), Proc. Natl. Acad. Sci. USA. 82, 488-492, Kunkel (1988), Methods Enzymol. 85, 2763-2766).

The protein of the present invention can vary in its amino acid sequence, molecular weight, isoelectric point, and presence or absence of a sugar chain or the form of a sugar chain depending on a cell or a host used for producing the protein or a purification method as described later. For example, when the protein of the present invention is expressed in a prokaryotic cell such as E. coli, a methionine residue is added at the N-terminus of the amino acid sequence of the original protein. The protein of the present invention also includes such a protein.

The protein of the present invention can be prepared as a recombinant protein or as a naturally occurring protein by a method known to those skilled in the art. In the case of a recombinant protein, it can be prepared as follows. DNA encoding the protein of the present invention is introduced into an appropriate expression vector, and the vector is introduced into an appropriate host cell. Then, the resulting transformant is collected, and an extract is obtained. Then the protein is purified by chromatography such as ion exchange, reverse phase, or gel filtration chromatography, or by affinity chromatography with a column on which antibodies against the protein of the present invention are immobilized, or combination of one or more of these columns.

Further, when the protein of the present invention is expressed in a host cell (such as an animal cell or E. coli), as a fusion protein with glutathione S-transferase protein or as a recombinant protein supplemented with multiple histidine residues, the expressed recombinant protein can be purified using a glutathione column or a nickel column, respectively. After purifying the fusion protein, it is also possible to remove regions other than the intended protein from the fusion protein by cutting with thrombin, factor-Xa or the like as needed.

In the case of a natural protein, it can be isolated by a method well known to those skilled in the art, for example, by purifying an extract of tissue or cells expressing the protein of the present invention with an affinity column having antibodies that binds to the protein of the present invention, which will be described later. The antibody may be a polyclonal antibody or a monoclonal antibody.

The present disclosure also encompasses a fragment (partial peptide) of the protein of the present invention. The fragment of the present disclosure can be used in producing an antibody against the protein of the present invention, screening a compound binding to the protein of the present invention, or screening a stimulator or an inhibitor of the protein of the present invention. In addition, it can be used as an antagonist or a competitive inhibitor of the protein of the present invention.

When the fragment of the present disclosure is used as an immunogen, it is composed of at least 7 amino acids or more, preferably 8 amino acids or more, and more preferably 9 amino acids or more. When it is used as a competitive inhibitor of the protein of the present invention, it comprises an amino acid sequence of at least 100 amino acids or more, preferably 200 amino acids or more, more preferably 300 amino acids or more.

The fragment of the present disclosure can be produced by a genetic engineering technique, a well-known peptide synthesizing method, or by cleaving the protein of the present invention with a suitable peptidase. The peptide synthesis may be carried out by, for example, a solid phase synthesis method or a liquid phase synthesis method.

Moreover, the present invention provides a vector into which the DNA of the present invention is inserted. The vector of the present invention may be useful for maintaining the DNA of the present invention in a host cell or expressing the protein of the present invention.

When E. coli is used as a host, it is preferred that the vector has an "ori" to be amplified in E.coli and a gene for selecting the transformed E. coli (e.g., a drug resistance gene that can be selected by a drug such as ampicillin, tetracycline, kanamycin, or chloramphenicol). in order to amplify and prepare the vector in a large amount in E. coli (such as JM109, DH5α, HB101 or XL1Blue).

Examples of the vector include M13-series vectors, pUC-series vectors, pBR322, pBluescript, pCR-Script and the like. In addition, when it is intended to be used for subcloning and excision of the cDNA, pGEM-T, pDIRECT, pT7 and the like may also be used in addition to the above-mentioned vectors.

When the vector is used to produce the protein of the present invention, an expression vector is especially useful. When the expression vector is intended to be expressed in E. coli, the vector should have the above-mentioned characteristic for amplification of the vector in E. coli. Additionally, when E. coli such as JM109, DH5α, HB101 or XL1-Blue is used as a host cell, it is essential that the vector should have a promoter that can efficiently drive the expression in E. coli, for example, lacZ promoter (Ward et al. , Nature (1989) 341: 544-546; FASEB J. (1992) 6 : 2422-2427), araB promoter (Better et al., Science (1988) 240: 1041-1043), or T7 promoter, Examples of such a vector include other than the above-mentioned vectors, pGEX-5X-1 (manufactured by Pharmacia), "QIAexpress system" (manufactured by QIAGEN), pEGFP, pET (for this vector, BL21 expressing T7 RNA polymerase is preferably used as a host) and the like.

Further, the vector may contain a signal sequence for protein secretion. When the protein is secreted into the periplasm of E. coli, pelB signal sequence (Lei S.P. et al., J. Bacteriol. (1987) 169, 4379) may be used as a signal sequence for protein secretion. The introduction of the vector into a host cell can be carried out by, for example, a calcium chloride method or an electroporation method.

Examples of the vector used to produce the protein of the present invention include expression vectors other than those from E. coli, for example, expression vectors derived from mammals (e.g., pcDNA3 (manufactured by Invitrogen), pEGF-BOS (Nucleic Acids. Res. 1990, 18 (17), p5322), pEF, and pCDM8); expression vectors derived from insect cells (e.g., "Bac-to-BAC baculovirus expression system" (manufactured by GIBCO BRL), pBacPAK8); expression vectors derived from plants (e.g., pMH1 and pMH2); expression vectors derived from animal viruses (e.g., pHSV, pMV, and pAdexLcw); expression vectors derived from retroviruses (e.g., pZIPneo); expression vectors derived from yeast (e.g., "Pichia Expression Kit" (manufactured by Invitrogen), pNV11 and SP-Q01); and expression vectors derived from Bacillus subtilis (e.g., pPL608 and pKTH50).

When it is intended to express the protein in an animal cell, such as a CHO cell, COS cell, or NIH3T3 cell, it is essential that the vector should include a promoter necessary for expression in such a cell, for example, SV40 promoter (Mulligan et al., Nature (1979) 277, 108), MMLV-LTR promoter, the EF1α promoter (Mizushima et al. , Nucleic Acids Res. (1990) 18, 5322), and CMV promoter. It is more preferred that the vector additionally has a gene for selecting the transformed cell (e.g., a drug resistance gene that allows for selection with a drug such as neomycin or G418). Examples of the vector having such a characteristic include pMAM, pDR2, pBK-RSV, pBK-CMV, pOPRSV, pOP13 and the like.

Further, when it is intended to stably express the gene and to amplify the copy number of genes in a cell, a vector (such as pCHOI) having a DHFR gene that compensates for the deficiency may be introduced into a CHO cell deficient in nucleic acid synthetic pathways, and amplified with methotrexate (MTX). Further, when it is intended to transiently express the gene, a COS cell having a gene expressing SV40 T antigen on the chromosome may be transformed with a vector (such as pCD) having SV40 replication origin. The replication origin may include those derived from polyoma virus, adenovirus, bovine papillomavirus (BPV) and the like. Further, in order to amplify the gene copy number in a host cell system, the expression vector can include as a selection marker, the aminoglycoside transferase (APH) gene, the thymidine kinase (TK) gene, the E. coli xanthine guanine phosphoribosyl transferase (Ecogpt) gene, the dihydrofolate reductase (dhfr) gene or the like.

Further, the present disclosure provides a host cell into which the vector of the present invention is introduced. The host cell is not particularly limited but may include, for example, E. coli, various animal cells and the like. The host cell of the present invention may be used, for example, as a production system for producing and expressing the protein of the present invention. There are in vitro and in vivo production systems available for producing the protein. Examples of the in vitro production system include a production system using a eukaryotic cell and a production system using a prokaryotic cell.

When the eukaryotic cell is used, for example, an animal cell, a plant cell or a fungal cell can be used as a host. The animal cells known in the art include mammalian cells, such as CHO (J. Exp. Med. (1995) 108, 945), COS, 3T3, myeloma, baby hamster kidney (BHK), HeLa and Vero; amphibian cells such as Xenopus laevis oocytes (Valle, et al., Nature (1981) 291, 338-340); and insect cells such as sf9, sf21 and Tn5. CHO cells preferably used in the invention include those deficient in the DHFR gene such as dhfr-CHO (Proc. Natl. Acad. Sci. USA (1980) 77, 4216-4220) and CHO K-1 (Proc. Natl. Acad. Sci. USA (1968) 60, 1275). Among the animal cells, the CHO cell is particularly preferred for mass expression. Introduction of the vector into the host cell can be carried out by, for example, a calcium phosphate method, a DEAE-dextran method, a method using cationic liposome DOTAP (manufactured by Boehringer Mannheim), an electroporation method, lipofection or the like.

A plant cell derived from Nicotiana tabacum is known as a protein production system and may be subjected to callus culture. Fungal cells known in the art include yeast such as the Saccharomyces genus, for example, Saccharomyces cerevisiae and filamentous bacteria such as Aspergillus genus, for example, Aspergillus niger.

For prokaryotic cells, various production systems utilizing a bacterial cell are available. Examples of the bacterial cell include E. coli such as JM109, DH5α and HB101, and the like. In addition, Bacillus subtilis is also available.

Such a cell is transformed by a desired DNA, and the resulting transformed cell is cultured in vitro to obtain the protein. Cultivation can be carried out according to a known method. The culture medium used for animal cells include, for example, DMEM, MEM, RPMI1640, or IMDM. A serum supplement such as fetal calf serum (FCS) may be used in combination with the medium, or serum free culture may be carried out. The pH of the culture medium during the cultivation ranges preferably from about 6 to 8. Cultivation is generally carried out at about 30 to 40°C, for 15 to 200 hours, and the culture medium may be replaced, aerated, or stirred as needed.

On the other hand, examples of the in vivo protein production system include a production system using an animal and a production system using a plant. A desired DNA is introduced into such an animal or a plant, and the protein is allowed to be produced in the animal or the plant, and then collected. The term "host" in the present invention encompasses such an animal and a plant.

For animals, various production systems utilizing a mammal or an insect are available. The mammal includes goats, pigs, sheep, mice and cattle (Vicki Glaser, SPECTRUM Biotechnology Applications, 1993). In addition, the mammal may also include a transgenic animal.

For example, a desired DNA is prepared as a fusion gene with a gene encoding a protein such as goat β-casein that is specifically produced into milk. Next, the DNA fragment containing this fusion gene is injected into a goat's embryo, which is then implanted in a female goat. A desired protein can be obtained from milk produced by a transgenic goat which is born from the goat that had received the embryo or offspring thereof. To increase the amount of milk containing the protein produced by the transgenic goat, an appropriate hormone may be administered to the transgenic goat (Ebert, K.M. et al., Bio/Technology (1994) 12, 699-702).

Further, a silkworm may be used as a host insect. The silkworm is infected with a baculovirus into which DNA encoding a desired protein has been inserted. The desired protein can be obtained from the body fluid of the silkworm (Susumu, M. et al., Nature (1985) 315, 592-594).

Further, tobacco may be used as a plant host. DNA encoding a desired protein is inserted into a plant expression vector such as pMON 530, and this vector is introduced into bacteria such as Agrobacterium tumefaciens. Then, tobacco such as Nicotiana tabacum is infected with the bacteria and the desired protein can be obtained from the leaves of the tobacco (Julian, K. -C. Ma et al., Eur. J. Immunol. (1994) 24, 131-138).

The protein of the present invention obtained as above can be isolated from the inside or the outside of the host cell (such as medium) and purified to a substantially pure homogenous protein. Any method commonly used for isolation and purification of protein may be used for purifying the protein. For example, chromatography column, filtration, ultrafiltration, salting out, solvent precipitation, solvent extraction, distillation, immunoprecipitation, SDS-polyacrylamide gel electrophoresis, isoelectric focusing, dialysis, recrystallization and the like may be suitably selected and combined, whereby the protein can be isolated and purified.

Examples of the chromatography include affinity chromatography, ion exchange chromatography, hydrophobic chromatography, gel filtration, reversed-phase chromatography, adsorption chromatography and the like (Strategies for Protein Purification and Characterization: A Laboratory Course Manual. Ed Daniel R. Marshak et al., Cold Spring Harbor Laboratory Press, 1996). Such chromatography can be carried out by liquid chromatography such as HPLC or FPLC. A protein highly purified by using such a purification method is also encompassed by the present invention.

The protein can be optionally modified or a peptide portion can be removed by treating the protein with an appropriate protein-modifying enzyme before or after the purification of the protein. Such a protein-modifying enzyme includes, for example, trypsin, chymotrypsin, lysylendopeptidase, protein kinase, glucosidase and the like.

As shown in Examples described below, PCR primers were designed based on the gene sequences of cancer-associated genes represented by SEQ ID NOs: 1 to 65 (see Table 1) and the expression level of the cancer-associated genes in human tissue was quantified by quantitative PCR using cDNA obtained from human normal tissue and cancer tissue. It was found that the expression of the cancer-associated genes of the present invention is elevated in certain human cancer tissue.

The expression of a gene having a nucleotide sequence represented by any of SEQ ID NOs: 1, 2, 28, 29, 30, 31, 32, 51, 52, 60 and 61 was found to be elevated in lung cancer. Thus, a protein encoded by the gene having a nucleotide sequence represented by any of SEQ ID NOs: 1, 2, 28, 29, 30, 31, 32, 51, 52, 60 and 61 or a fragment thereof is useful for diagnosing or treating lung cancer. The gene preferably has a nucleotide sequence represented by any of SEQ ID NOs: 1, 2, 28, 29, 30, 31 and 32, more preferably has a nucleotide sequence represented by SEQ ID NO: 1 or 2.

The expression of a gene having a nucleotide sequence represented by any of SEQ ID NOs: 3, 4, 5, 6, 7, 8, 9, 10, 11, 12, 13, 22, 23, 24, 25, 26, 27, 33, 34, 35, 36, 37, 38, 39, 40, 41, 42, 53, 54 and 55 was found to be elevated in stomach cancer. Thus, a protein encoded by the gene having a nucleotide sequence represented by any of SEQ ID NOs: 3, 4, 5, 6, 7, 8, 9, 10, 11, 12, 13, 22, 23, 24, 25, 26, 27, 33, 34, 35, 36, 37, 38, 39, 40, 41, 42, 53, 54 and 55 or a fragment thereof is useful for diagnosing or treating stomach cancer. The gene preferably has a nucleotide sequence represented by any of SEQ ID NOs: 3, 4, 5, 6, 7, 8, 9, 10, 11, 12, 13, 22, 23, 24, 25 and 26.

The expression of a gene having a nucleotide sequence represented by any of SEQ ID NOs: 3, 7, 20, 21, 46, 47, 48, 49 and 50 was found to be elevated in large bowel cancer. Thus, a protein encoded by the gene having a nucleotide sequence represented by any of SEQ ID NOs: 3, 7, 20, 21, 46, 47, 48, 49 and 50 or a fragment thereof is useful for diagnosing or treating large bowel cancer. The gene preferably has a nucleotide sequence represented by any of SEQ ID NOs: 3, 7, 20, 21, 46, 49 and 50, more preferably has a nucleotide sequence represented by any of SEQ ID NOs: 3, 7, 20 and 21.

The expression of a gene having a nucleotide sequence represented by any of SEQ ID NOs: 14, 15, 16, 17, 18, 19, 43, 44, 45, 56, 57, 58, 59, 62, 63, 64 and 65 was found to be elevated in liver cancer. Thus, a protein encoded by the gene having a nucleotide sequence represented by any of SEQ ID NOs: 14, 15, 16, 17, 18, 19, 43, 44, 45, 56, 57, 58, 59, 62, 63, 64 and 65 or a fragment thereof is useful for diagnosing or treating liver cancer. The gene preferably has a nucleotide sequence represented by any of SEQ ID NOs: 14, 15, 16, 17, 18, 19, 45, 56, 57, 58, 64 and 65, more preferably has a nucleotide sequence represented by any of SEQ ID NOs: 14, 15, 16, 17, 18, 19, 64 and 65.

The composition of the present disclosure comprising a protein encoded by a cancer-associated gene represented by any of SEQ ID NOs: 1 to 65 or a fragment thereof can be used as a vaccine against cancer. By administering to a targeted human or another animal the above-mentioned protein or an immunogenic fragment thereof together with an appropriate adjuvant, or as a fusion protein with another suitable polypeptide, immune response will be induced in the body of the human or the animal. Alternatively, the composition of the present disclosure may be administered in the form of a cell expressing the above-mentioned cancer-associated gene or fragment thereof.

Further, the composition of the present disclosure can be used to diagnose whether a subject is affected with a specific cancer by determining whether the subject has an antibody against a protein encoded by a cancer-associated gene represented by any of SEQ ID NOs: 1 to 65.

### Antibody

In another aspect, the present disclosure provides an antibody recognizing a protein encoded by a cancer-associated gene having a nucleotide sequence represented by SEQ ID NOs: 1 to 65 or a fragment of the protein, or an antigen-binding fragment of the antibody. Further, the present' disclosure provides a composition for diagnosing or treating cancer comprising the antibody or the binding fragment thereof. The antibody of the present disclosure can preferably recognize a protein having an amino acid sequence represented by SEQ ID NOs: 66 to 123 or a fragment thereof. The present disclosure also provides a cell producing such an antibody.

The term "recognize" means that an antibody binds to the above-mentioned protein encoded by a cancer-associated gene or a fragment thereof under specific conditions with higher affinity than it binds to another polypeptide.

The antibody of the present invention may include monoclonal antibodies, polyclonal antibodies, antibodies having an ability of specifically binding to an antigen determinant, variants and derivatives of antibodies such as T-cell receptor fragments.

The type of the antibody of the present invention is not particularly limited, but may include a mouse antibody, a human antibody, a rat antibody, a rabbit antibody, a sheep antibody, a camel antibody or the like. Also included are a genetically recombinant antibody which has been artificially modified for the purpose of lowering heterologous antigenicity against human such as a chimeric antibody, a humanized antibody or the like. The genetically recombinant antibody can be produced by a known method. The chimeric antibody is an antibody comprising antibody heavy chain and light chain variable regions of a non-human mammal such as a mouse, and the antibody heavy chain and light chain constant regions of a human. It can be obtained by ligating DNA encoding the variable region of a mouse antibody to DNA encoding the constant region of a human antibody, which is then introduced into an expression vector and introduced into a host for production of the antibody. A humanized antibody, also referred to as a reshaped human antibody, is obtained by transplanting a complementarity determining region (CDR) of an antibody of a non-human mammal such as a mouse, into the complementarity determining region of a human antibody. A general technique of genetic recombination is also known. Specifically, a DNA sequence designed to ligate a CDR of a mouse antibody to the framework region (FR) of a human antibody is synthesized by a PCR method using several oligonucleotides constructed to have overlapping portions at their ends. Then, the resulting DNA is ligated to DNA encoding a human antibody constant region, which is introduced into an expression vector, and the vector is introduced into a host to produce an antibody, whereby a humanized antibody can be obtained (see European Patent Application No. EP239400, and International Patent Application No. WO 96/02576). The human antibody FR that is ligated via the CDR is selected to allow the complementarity determining region to form a favorable antigen-binding site. As necessary, an amino acid in the framework region of an antibody variable region may be substituted such that the complementarity determining region of a reshaped human antibody forms an appropriate antigen-binding site (Sato, K. et al., Cancer Res. 1993, 53, 851-856).

Methods for obtaining a human antibody are also known. For example, a desired human antibody having an antigen-binding activity can be obtained by sensitizing a human lymphocyte with a desired antigen or a cell expressing a desired antigen in vitro, and fusing the sensitized lymphocyte with a human myeloma cell such as U266 (see JP-B-1-59878). Alternatively, a desired human antibody can also be obtained by using a desired antigen to immunize a transgenic animal having the entire repertoire of human antibody genes (see International Patent Application Nos. WO 93/12227, WO 92/03918, WO 94/02602, WO 94/25585, WO 96/34096 and WO 96/33735). Further, techniques to obtain a human antibody by panning with a human antibody library are known in the art. For example, the variable region of a human antibody is expressed as a single chain antibody (scFv) on the surface of a phage using a phage display method, and a phage binding to the antigen can be selected. By analyzing the gene of the selected phage, the DNA sequence encoding the variable region of a human antibody binding to the antigen can be determined. If the DNA sequence of scFv that binds to the antigen is identified, an appropriate expression vector containing the sequence is constructed, and a human antibody can be obtained. These methods are already well known and described in WO 92/01047, WO 92/20791, WO 93/06213, WO 93/11236, WO 93/19172, WO 95/01438 and WO 95/15388.

Moreover, the antibody of the invention may also be a low molecular weight antibody such as an antibody fragment or a modified antibody as long as it can bind to an antigen. Specific examples of the antibody fragment include Fab, Fab', F(ab')2, Fv, Diabody and the like. To obtain such an antibody fragment, a gene encoding such an antibody fragment is constructed, the gene is introduced into an expression vector, and then the gene may be expressed in a suitable host cell (see, for example, Co, M.S. et al. , J. Immunol. (1994) 152, 2968-2976; Better, M. and Horwitz, A.H., Methods Enzymol. (1989) 178, 476-496; Pluckthun, A. and Skerra, A., Methods Enzymol. (1989) 178, 497-515; Lamoyi, E. , Methods Enzymol. (1986) 121, 652-663; Rousseaux, J. et al., Methods Enzymol. (1986) 121, 663-669; and Bird, R. E. and Walker, B. W., Trends Biotechnol. (1991) 9, 132-137).

The modified antibody may also include an antibody bound to any of a variety of molecules such as polyethylene glycol (PEG). In addition, a radioisotope, a chemotherapeutic agent, a cytotoxic substance such as a bacteria-derived toxin may be attached to the antibody. A radiolabeled antibody is particularly useful. Such a modified antibody can be obtained by subjecting the obtained antibody to chemical modification. The method of modifying an antibody has been already established in this field.

Further, it is possible to use an antibody whose sugar chain is modified for the purpose of enhancing the cytotoxic activity or the like. Techniques to modify a sugar chain of an antibody have been already known (e.g., WO 00/61739, WO 02/31140, etc.)

Further, the present invention also encompasses a multispecific antibody having specificity for at least two different antigens. While such a molecule is generally binds to two antigens (i.e., bispecific antibody), the term "multispecific antibody" in the present invention encompasses an antibody having specificity for two or more (such as three) antigens. The multispecific antibody can be a full length antibody or a fragment of such an antibody (e.g. F(ab')₂ bispecific antibody).

Methods for producing the multispecific antibody are known in the art. Production of a full length bispecific antibody comprises co-expression of two immunoglobulin heavy chain-light chain pairs in which the two chains have different specificities (Millstein et al., Nature, 305: 537-539 (1983)). Because of the random assortment of immunoglobulin heavy chain and light chain, plural hybridomas (quadromas) co-expressing the heavy and light chains are obtained as a mixture of hybridomas respectively expressing different antibody molecules. Thus, it is necessary to select those producing a correct bispecific antibody. The selection can be carried out by affinity chromatography. In another method, antibody variable domains with the desired binding specificities are fused to immunoglobulin constant domain sequences. The constant domain sequence preferably comprises at least part of the hinge, CH2, and CH3 regions of the immunoglobulin heavy chain constant domain. It is preferred to contain the heavy chain CH1 region necessary for binding with the light chain. DNA encoding the immunoglobulin heavy chain fusions and, if desired, DNA encoding the immunoglobulin light chains, are inserted into separate expression vectors, and are transfected into a suitable host organism. Insertion of the respective genes into separate expression vectors will allow for adjustment of the expression ratios of the respective chains. This method is convenient when unequal ratios of the respective chains will provide an increase in the yield of the obtained antibody. It is, however, possible to insert the genes encoding plural chains into one vector.

In a preferred embodiment, a bispecific antibody is composed of a hybrid immunoglobulin heavy chain with a first binding specificity in one arm, and a hybrid immunoglobulin heavy chain-light chain pair with a second binding specificity in the other arm. By allowing an immunoglobulin light chain to be present in only one arm in this way, the isolation of the bispecific antibody from other immunoglobulins can be readily carried out. As for the isolation method, see WO 94/04690. As for the production method of the bispecific antibody, see the method of Suresh et al. (Methods in Enzymology, 121: 210 (1986)). To decrease the number of homodimers in the final product obtained from the recombinant cell culture and increase the ratio of the heterodimers, one antibody molecule including CH3 of an antibody constant domain may be modified, where one or more amino acids with a small side chain present on the surface and binding to the other molecule are replaced with an amino acid with a larger side chain (e.g. tyrosine or tryptophan), and an amino acid with a large side chain in the region corresponding to the other antibody molecule is replaced with a smaller one (e.g. alanine or threonine), whereby a cavity corresponding to the larger side chain in the first antibody molecule is created (WO 96/27011).

The bispecific antibody also include a heteroconjugate antibody in which one antibody is coupled to avidin and the other is coupled to biotin or the like (U.S. Pat. No. 4,676,980, WO 91/00360, WO 92/200373, and EP 03089). A cross-linking agent to be used in the production of such a heteroconjugate antibody is well known, and is disclosed in, for instance, U.S. Pat. No. 4,676,980.

A method for producing the bispecific antibody from an antibody fragment has also been reported. For example, it can be produced by utilizing a chemical bond. First, F(ab')₂ fragments are produced and are reduced in the presence of sodium arsenite, a dithiol complexing agent, in order to prevent disulfide formation within the same molecule. Then, the F(ab')₂ fragments are converted to thionitrobenzoate (TNB) derivatives. Then, one of the F(ab')₂-TNB derivatives is reduced again to the Fab'-thiol with mercaptoethylamine, and the F(ab')₂-TNB derivatives are mixed with an equimolar amount of the Fab'-thiol, whereby a bispecific antibody is produced.

Various methods for producing and isolating bispecific antibodies directly from the recombinant cell culture have also been reported. For example, a method for producing bispecific antibodies using leucine zippers has been reported (Kostelny et al., J. Immunol., 148 (5): 1547-1553 (1992)). First, the leucine zipper peptides from the Fos and Jun proteins were linked to the Fab' portions of different antibodies by gene fusion. The antibody homodimers were reduced at the hinge region to form monomers and then re-oxidized to form antibody heterodimers. Another method is available in which a heavy-chain variable domain (VH) is connected to a light-chain variable domain (VL) via a linker which is too short to allow pairing between these two domains to form a pair of complementary other VL and VH domains, whereby two antigen-binding sites are created (Hollinger et al., Proc. Natl. Acad. Sci. USA 90: 6444-6448 (1993)). In addition, dimers obtained from a single-chain Fv (sFv) have also been reported (Gruger et al., J. Immunol., 152: 5368 (1994)). Further, trispecific antibodies instead of bispecific antibodies have also been reported (Tutt et al. J. Immunol. 147: 60 (1991)).

The "antibody" in the present invention also encompasses antibodies described above.

The antibody and the antibody fragment of the present invention can be produced by any suitable manner such as in vivo, cultured cells, in vitro translation reaction or a recombinant DNA expression system.

Techniques for producing monoclonal antibodies and hybridomas are well known in the art (Campbell, "Monoclonal Antibody Technology: Laboratory Techniques in Biochemistry and Molecular Biology", Elsevier Science Publishers, Amsterdam, The Netherlands, 1984; St. Groth, et al., J. Immunol. Methods 35: 1-21, 1980). The above-mentioned protein encoded by a cancer-associated gene or a fragment thereof may be used as an antigen to immunize any animal (mouse, rabbit, or the like) which is known to produce antibodies by subcutaneous or intraperitoneal injection. An adjuvant may be used in the immunization, and the use of such an adjuvant is well known in the art.

Polyclonal antibodies can be obtained by isolating antisera containing antibodies from an immunized animal, and detecting the presence of an antibody with the desired specificity by using a well known method in the art such as an ELISA assay, Western blot analysis, or radioimmunoassay.

Monoclonal antibodies can be obtained by excising spleen cells from an immunized animal, fusing the spleen cells with myeloma cells, and producing hybridoma cells that produces monoclonal antibodies. A hybridoma cell that produces an antibody recognizing a desired protein or a fragment thereof is selected using a method well known in the art such as an ELISA assay, Western blot analysis, or radioimmunoassay. A hybridoma secreting the desired antibody is cloned and cultured under appropriate conditions, the secreted antibody is recovered, and purified by a method well known in the art, such as ion exchange column or affinity chromatography. Alternatively, a human monoclonal antibody may be produced by using a XenoMouse strain (see Green J. Immunol. Methods 231: 11-23, 1999; Wells, Eek, Chem Biol 2000 Aug; 7 (8): R185-6).

The DNA encoding the monoclonal antibody can be readily isolated and sequenced using a conventional method (e.g., by using an oligonucleotide probe capable of binding specifically to genes encoding the heavy chain and the light chain of the monoclonal antibody). The hybridoma cells serve as a preferred source of such DNA. Once isolated, the DNA is inserted into an expression vector, which is then transfected into a host cell such as an E. coli cell, simian COS cell, Chinese Hamster Ovary (CHO) cell, or myeloma cell that does not otherwise produce immunoglobulin protein, whereby a monoclonal antibody is produced in the recombinant host cell. In another embodiment, an antibody or antibody fragment can be isolated from an antibody phage library generated using the techniques described in McCafferty et al. (Nature 348: 552-554 (1990).

The above-mentioned antibody can be detectably labeled. Examples of the label include radioisotopes, affinity labels (such as biotin and avidin), enzymatic labels (such as horseradish peroxidase and alkaline phosphatase), fluorescent labels (such as FITC or rhodamine), paramagnetic atoms and the like. Methods for accomplishing such labeling are well known in the art. The above-mentioned antibodies may be immobilized on a solid support. Examples of the solid support include plastic, agarose, sepharose, polyacrylamide, latex beads and the like. Techniques for immobilizing antibodies on such a solid support are well known in the art.

As described in Examples below, the cancer-associated gene of the present invention shows elevated expression in a specific cancer tissue, therefore the antibody of the present invention is useful as a diagnostic marker for cancer. The expression of the protein encoded by a cancer-associated gene can be detected in a tissue or cell using the antibody of the present invention in a method such as Western blotting, the ELISA method or histological staining. A sample (such as biopsy sample or blood sample) derived from tissue of a subject is brought into contact with the composition of the present invention under conditions so as to form an immune complex. The presence or the amount of the protein encoded by a cancer-associated gene in the sample can be determined by determining whether the sample binds to the antibody. In this way, diagnosis of cancer, monitoring of progress or cure of cancer, and prediction of prognosis may be carried out. The diagnostic composition of the present invention may be provided as a kit for detecting the presence of the above-mentioned protein encoded by a cancer-associated gene in a sample. Such a kit may contain, in addition to the above-mentioned antibody, a washing reagent, a reagent that can detect the presence of the bound antibody such as a labeled secondary antibody, a chromophore that can react with the labeled antibody, an enzyme, and an antibody-binding reagent, and a usage guideline.

Further, the antibody against the protein encoded by the cancer-associated gene of the present disclosure has some specificity for a specific cancer cell, therefore it may be used as a therapeutic agent for cancer or may be used in a missile therapy, where a drug is allowed to specifically target cancer tissue. Preferably, the composition of the present disclosure is used in diagnosis and treatment of lung cancer, stomach cancer, large bowel cancer and liver cancer.

The therapeutic agent of the present disclosure may be formulated with a pharmaceutically acceptable carrier well known in the art by mixing, dissolving, granulating, tableting, emulsifying, encapsulating, lyophilizing or other processes.

For oral administration, the therapeutic agent of the present disclosure can be formulated with a pharmaceutically acceptable solvent, excipient, binder, stabilizer, dispersant or the like, into the dosage form such as a tablet, a pill, a sugarcoated pill, a soft capsule, a hard capsule, a solution, a suspension, an emulsion, a gel, a syrup or a slurry.

For parenteral administration, the therapeutic agent of the present disclosure can be formulated with a pharmaceutically acceptable solvent, excipient, binder, stabilizer, dispersant or the like, into the dosage form such as an injectable solution, a suspension, an emulsion, a cream, an ointment, an inhalant or a suppository. For injectable formulation, the therapeutic agent of the present disclosure can be dissolved in an aqueous solution, preferably in a physiologically compatible buffer such as Hanks' solution, Ringer's solution or a physiological saline buffer. Further, the composition can take the form of a suspension, a solution, an emulsion or the like in an oleaginous or aqueous vehicle. Alternatively, the therapeutic agent may be produced in the form of powder, and an aqueous solution or a suspension may be prepared with sterilized water or the like before use. For administration by inhalation, the therapeutic agent of the present disclosure is powdered and formulated into a powder mixture with a suitable base such as lactose or starch. The suppository formulation can be produced by mixing the therapeutic agent of the present disclosure with a conventional suppository base such as cocoa butter. Further, the therapeutic agent of the present disclosure can be formulated as a sustained-release preparation by encapsulating it into a polymer matrix or the like.

The dose and the dose frequency may vary depending on the dosage form, the administration route and the patient's symptoms, age and body weight, however, the therapeutic agent of the present invention can generally be administered at a dose of about 0.001 mg to 1000 mg, preferably about 0.01 mg to 10 mg per kg of body weight per day, which can be taken once to several times a day.

In general, the therapeutic agent is parenterally administered, for example, by injection (subcutaneous, intravenous, intramuscular, intraperitoneal injection or the like), or by transdermal, transmucosal, transnasal, transpulmonary administration or the like, however, the administration route is not particularly limited, and it may be orally administered.

### Polynucleotide

In still another aspect, the present disclosure provides a polynucleotide having a nucleotide sequence represented by any of SEQ ID NOs: 1 to 65 or a nucleotide sequence complementary thereto or a polynucleotide that can hybridize under high stringent conditions to any of these polynucleotides.

Further, the present disclosure provides a composition containing a polynucleotide having a sequence comprising at least 12 consecutive nucleotides of a nucleotide sequence represented by any of SEQ ID NOs: 1 to 65 or a nucleotide sequence complementary thereto or an oligonucleotide with a length of at least 12 nucleotides that can hybridize under high stringent conditions to a polynucleotide having a nucleotide sequence represented by any of SEQ ID NOs: 1 to 65.

Such a polynucleotide is useful for diagnosis of cancer, production of a protein, a primer, an antisense or siRNA for inhibiting gene expression and the like. The cancer is preferably selected from lung cancer, stomach cancer, large bowel cancer and liver cancer.

The expression of the cancer-associated genes of the present disclosure represented by SEQ ID NOs: 1 to 65 is elevated in a specific human cancer tissue as shown in the following Examples. Therefore, the composition of the present disclosure maybe used as an agent of antisense oligonucleotide, ribozyme, siRNA or the like for silencing the expression of the cancer-associated gene, and as a probe or a primer for detecting the cancer-associated gene. It can also be used for producing the protein of the present invention.

The polynucleotide or the oligonucleotide contained in the composition of the present disclosure may be single stranded or double stranded, and may be DNA, RNA, or a mixture thereof or a derivative of PNA or the like. Such a polynucleotide or oligonucleotide may be chemically modified at the internucleoside linkage, the base moiety and/or the sugar moiety, or may have a modifier at the 5' end and/or 3' end. Examples of the modified internucleoside linkage include phosphorothioate, phosphorodithioate, phosphoramidethioate, phosphoramidate, phosphorodiamidate, methylphosphonate, alkylphosphotriester, formacetal and the like. Examples of the modified base moiety include 5-fluorouracil, 5-bromouracil, 5-chlorouracil, 5-iodouracil, hypoxanthine, xanthine, 4-acetylcytosine, 5-(carboxyhydroxylethyl) uracil and the like. Examples of the modified sugar moiety include 2'-O-alkyl, 2'-O-alkyl-O-alkyl or 2'-fluoro modification and the like. In addition, a sugar other than ribose may also be used, for example, arabinose, 2-fluoroarabinose, xylulose and hexose.

The polynucleotide of the present disclosure includes a polynucleotide having a nucleotide sequence represented by any of SEQ ID NOs: 1 to 65 or a nucleotide sequence complementary thereto or a polynucleotide that can hybridize under high stringent conditions to any of these polynucleotides. A polynucleotide that can hybridize under stringent conditions generally has a high identity. The term "high identity" as used herein means that it has an identity of 70% or more, preferably 80% or more, more preferably 90% or more with a nucleotide sequence represented by any of SEQ ID NOs: 1 to 65.

The identity of a nucleotide sequence can be determined by using the algorithm BLAST by Karlin and Altschul (Proc. Natl. Acad. Sci. USA, 90: 5873-5877, 1993). The programs called BLASTN and BLASTX have been developed based on the above algorithm (Altschul et al. , J. Mol. Biol., 215: 403-410 1990). In the case of analyzing a nucleotide sequence by BLASTN based on BLAST, the parameters can be set, for example, score = 100 and wordlength = 12. BLAST and Gapped BLAST programs may be used with its default parameter. The specific techniques of these analysis methods are known in the art (http://www.ncbi.nlm.ni-h.gov.).

Further, the present disclosure encompasses a polynucleotide encoding an amino acid sequence represented by any of SEQ ID NOs: 66 to 123. Such a polynucleotide may be used in the production of the protein of the present disclosure. In addition, since a polynucleotide having a nucleotide sequence represented by any of SEQ ID NOs: 1 to 65 or a sequence complementary thereto is overexpressed in a cancer cell, it can be used as a probe for diagnosing cancer by detecting such a polynucleotide.

Further, the composition of the present disclosure may be provided as a nucleic acid construct to be introduced into a cell to produce a desired antisense, ribozyme or siRNA in the cell.

When the polynucleotide or the oligonucleotide of the present disclosure is used as an antisense, ribozyme, siRNA or the like, the polynucleotide or the oligonucleotide may have a chain length of preferably at least 12 nucleotides or more, more preferably 12 to 50 nucleotides, particularly preferably 12 to 25 nucleotides. Such a polynucleotide or oligonucleotide may be a variant in which one or more nucleotides are substituted, added or deleted from the above-mentioned nucleotide sequence, as long as it has a desired antisense, ribozyme or siRNA activity. Such a variant may have a nucleotide sequence with an identity of at least 70%, preferably 90% or more, more preferably 95% or more with the above-mentioned nucleotide sequence. Alternatively, such a polynucleotide or oligonucleotide may hybridize under high stringent conditions to a polynucleotide having a nucleotide sequence represented by any of SEQ ID NOs: 1 to 65.

The term "hybridization" means that DNA or RNA corresponding to the DNA binds to another DNA or RNA molecule by a hydrogen bond interaction in a solution or on a solid support. The strength of such an interaction can be evaluated by changing the stringency of the hybridization conditions. Hybridization conditions having various stringencies may be used depending on the desired specificity and selectivity. The stringency can be adjusted by changing the concentration of a salt or the concentration of a denaturing agent. Such a method of adjusting the stringency is well known in the art and described in, for example, "Molecular Cloning: A Laboratory Manual", Second Edition, Cold Spring Harbor Laboratory Sambrook, Fritsch, & Maniatis, eds., 1989.

The stringent hybridization conditions mean conditions in the presence of 50% formamide at 42°C in 700 mM NaCl or equivalent conditions. One example of the stringent hybridization conditions is hybridization overnight at 42°C in a solution containing 50% formamide, 5 x SSC, 50 mM NaH₂PO₄ (pH 6.8), 0.5% SDS, sonicated salmon sperm DNA (0.1 mg/mL) and 5 x Denhardt's solution; washing at 45°C with 2 x SSC and 0.1% SDS; and washing at 45°C with 2 x SSC and 0.1% SDS.

The polynucleotide or the oligonucleotide of the present invention can be produced by a method known to those skilled in the art. For example, it can be synthesized with a commercially available DNA synthesizer (e.g., 394 synthesizer, manufactured by Applied Biosystems) using a protocol known in the art. Alternatively, it can be produced based on the sequence information disclosed in the instant application by the PCR amplification technique well known in the art using a suitable template and primers in combination.

Further, the polynucleotide or the oligonucleotide of the present disclosure can be prepared by constructing a cDNA library from cells expressing the polypeptide of the present invention and performing hybridization using a probe having a part of the sequence of the polynucleotide of the present disclosure. The cDNA library may be prepared by, for example, a method described in the document (Sambrook, J. et al., Molecular Cloning, Cold Spring Harbor Laboratory Press (1989)), or a commercially available DNA library may be used. Alternatively, it can be prepared by preparing RNA from cells expressing the polypeptide of the present disclosure, synthesizing cDNA with a reverse transcriptase, synthesizing oligo-DNA based on the DNA sequence of the present invention (e.g., SEQ ID NO: 1), and amplifying the cDNA encoding the polypeptide of the present disclosure by PCR using the synthesized oligo-DNA as a primer.

Further, by determining the nucleotide sequence of the obtained cDNA, one can determine the translated region of the cDNA and the amino acid sequence of the protein of the present disclosure. Further, the obtained cDNA can also be used as a probe for screening a genomic DNA library to isolate genomic DNA.

More specifically, mRNA is first isolated from a cell, tissue (e. g. , a lung cancer cell, large bowel cancer cell, liver cancer cell or stomach cancer cell) or the like in which the protein of the present disclosure is expressed. The isolation of mRNA is carried out by a known method. For example, total RNA is prepared by using guanidine ultracentrifugation (Chirgwin J. M. et al. Biochemistry (1979) 18, 5294-5299), or AGPC method (Chomczynski P. and Sacchi N. Anal. Biochem. (1987) 162, 156-159), and mRNA is purified from the total RNA using an mRNA Purification Kit (Pharmacia). Alternatively, mRNA can be directly prepared by using a QuickPrep mRNA Purification Kit (Pharmacia).

From the obtained mRNA, cDNA is synthesized using a reverse transcriptase. The synthesis of cDNA can also be carried out by using AMV Reverse Transcriptase First-strand cDNA Synthesis Kit (Seikagaku Kogyo). Alternatively, cDNA can be synthesized and amplified according to the 5'-RACE method (Frohman M. A. et al. Proc. Natl. Acad. Sci. U.S.A. (1988) 85, 8998-9002; Belyavsky A. et al. Nucleic Acids Res. (1989) 17, 2919-2932), using a 5'-Ampli FINDER RACE Kit (manufactured by Clontech) and polymerase chain reaction (PCR).

A desired DNA fragment is prepared from the obtained PCR products and ligated to a vector DNA, whereby recombinant vectors are prepared. These recombinant vectors are introduced into E. coli or the like, and a desired recombinant vector is prepared by selecting a colony. The nucleotide sequence of the desired DNA can be determined by a known method such as the dideoxynucleotide chain termination method.

By taking into account the frequency of codon usage in the host to be used for expression, the nucleotide sequence of the DNA of the present disclosure can be designed to be expressed more efficiently (Grantham R. et al. Nucleic Acids Research (1981) 9, r43-74). The DNA of the present disclosure may be modified by a commercially available kit or by a known method. Examples of the modification include, for example, digestion with a restriction enzyme, insertion of a synthetic oligonucleotide or an appropriate DNA fragment, addition of a linker, insertion of the initiation codon (ATG) and/or a stop codon (TAA, TGA, or TAG).

The oligonucleotide of the present disclosure may be used as a nucleic acid probe for detecting a cancer-associated gene in a sample. The probe of the present disclosure is selected to have a nucleotide sequence comprising at least 12, 20, 30, 50, 100 or more consecutive nucleotides of a nucleotide sequence represented by any of SEQ ID NOs: 1 to 65 or a nucleotide sequence complementary thereto, and to hybridize specifically to a specific region of a cancer-associated gene. DNA is extracted from a sample such as tissue or blood, or mRNA is extracted and cDNA is synthesized. DNA or cDNA is brought into contact with the probe under the conditions that allows hybridization to occur, and the presence or the amount of the probe bound to the sample is detected, whereby the presence or amount or variation of the cancer-associated gene or a transcript thereof in the sample can be detected.

The probe may be immobilized on a solid support. Examples of such a solid support include, but are not limited to, plastic, agarose, sepharose, polyacrylamide, latex beads, and nitrocellulose. Techniques to immobilize the probe on such a solid support are well known in the art. The probe can be visualized by labeling with a standard labeling technique such as radioactive labeling, enzymatic labeling (horseradish peroxidase or alkaline phosphatase), fluorescence labeling, biotin-avidin labeling or chemiluminescence. The composition of the present disclosure can be provided as a kit for detecting the presence of a cancer-associated gene or a transcript thereof in a sample. Such a kit may contain, in addition to the above-mentioned probe, a washing reagent, a reagent that can detect the presence of a bound probe, and a usage guideline.

Alternatively, the diagnostic composition of the present disclosure may contain a pair of primers that can amplify a nucleotide sequence represented by any of SEQ ID NOs: 1 to 65. With the use of these primers, a desired sequence is amplified by polymerase chain reaction (PCR) by using an appropriate cDNA library as a template. The PCR products are analyzed by a technique such as hybridization or nucleotide sequencing, whereby the presence or amount or variation of the cancer-associated gene or a transcript thereof in a sample can be detected. Such a PCR technique is well known in the art, and described in, for example, "PCR Protocols, A Guide to Methods and Applications", Academic Press, Michael, et al., eds. 1990.

For use as a primer, it is preferred that the oligonucleotide of the present disclosure has a sequence comprising at least 12, preferably 12 to 50, more preferably 12 to 20 consecutive nucleotides of the nucleotide sequence represented by any of SEQ ID NOs : 1 to 65 or a nucleotide sequence complementary thereto.

The polynucleotide or oligonucleotide of the present disclosure can silence a cancer-associated gene using an antisense molecule that binds to mRNA encoded by the cancer-associated gene and inhibits its expression, or a ribozyme or siRNA that cleaves mRNA. Methods of controlling gene expression using an antisense, ribozyme or siRNA technique are well known in the art. For example, the composition of the present disclosure may be administered together with an appropriate carrier, or a vector encoding an antisense, ribozyme or siRNA may be administered to induce its expression in vivo.

The term "ribozyme" means a nucleic acid molecule having an enzymatic activity of cleaving mRNA. The ribozyme generally shows an endonuclease, ligase or polymerase activity. Various types of trans-acting ribozymes such as hammerhead type and the hairpin type ribozymes are known in the art.

The term "antisense" means a nucleic acid molecule or a derivative thereof that hybridizes specifically to genomic DNA and/or mRNA and inhibits its transcription and/or translation to inhibit the expression of the protein. The binding may occur through general base pair complementation, or in the case of binding to DNA duplexes, through specific interactions in the major groove of the double helix. The target site of the antisense nucleic acid is preferably the 5' end of mRNA, for example, the 5'-untranslated sequence up to and including the AUG initiation codon. However, it is known that the 3'-untranslated sequence of mRNA or the sequence of the coding region is also effective in inhibiting the translation of mRNA.

The term "siRNA" means a double-stranded nucleic acid that can effect RNA interference (RNAi) (see, for example, Bass, 2001, Nature, 411, 428-429; Elbashir et al. , 2001, Nature, 411, 494-498). The siRNA can degrade mRNA in a sequence-specific manner, thereby inhibiting the expression of a gene. The siRNA is typically a double-stranded RNA with a length of 20 to 25 base pairs containing a sequence complementary to a target sequence. The siRNA molecule may contain a chemically modified nucleotide or non-nucleotide moiety.

Further, the polynucleotide of the present invention may be used in the production of the protein of the present invention.

### Screening

In still another aspect, the present disclosure provides a method of identifying a compound having an anticancer activity. This method comprises the steps of: bringing a cultured human cell into contact with a test compound; and identifying a compound that causes a change in the expression level of a gene containing a nucleotide sequence represented by any of SEQ ID NOs : 1 to 65 in the cell to be a compound having an anticancer activity.

Any of natural or synthetic compounds may be used as the test compound, and a combinatorial library may also be used. The expression level of a cancer-associated gene in a cell can be conveniently measured by the above-mentioned quantitative PCR method, although any other methods known in the art may be used.

### Detection method

The present invention provides a method of detecting cancer comprising the step of measuring the expression level of the gene or the protein of the present invention. Specific embodiments of the detection method will be described below, however, the detection method of the present invention is not limited to such a method.

In one embodiment of the detection method of the present invention, an RNA sample is first prepared from a subject. Subsequently, the level of RNA encoding the protein of the present invention contained in the RNA sample is measured. Then, the measured RNA level is compared with that of a control. In another embodiment, a cDNA sample is first prepared from a subject. Subsequently, the level of cDNA encoding the protein of the present invention contained in the cDNA sample is measured. Then, the measured cDNA level is compared with that of a control.

Such a method includes any of the methods well known to those skilled in the art, for example, Northern blotting, RT-PCR, DNA array analysis.

In the DNA array analysis, a cDNA sample is prepared by using RNA from a subject as a template, and is brought into contact with a substrate on which the oligonucleotide of the present invention has been immobilized. The intensity of hybridization of the cDNA sample with the nucleotide probe immobilized on the substrate is detected to determine the expression level of the gene of the present invention contained in the cDNA sample. Subsequently, the measured expression level of the gene of the present invention is compared with that of a control.

The cDNA sample may be prepared from a subject by a method well known to those skilled in the art. In a preferred embodiment for preparing the cDNA sample, total RNA is first extracted from cells or tissue (e.g., lung, large bowel, stomach, liver, etc.) of a subject. Total RNA may be extracted by a method well known to those skilled in the art. Total RNA may be extracted by a conventional method or kit that provides highly pure total RNA. For example, a sample is pretreated with "RNA later" (Ambion) and total RNA is extracted using "Isogen" (Nippon Gene). Specific procedures may follow the attached protocols.

Subsequently, cDNA is synthesized with a reverse transcriptase using the extracted total RNA as a template to prepare a cDNA sample. The synthesis of cDNA from total RNA can be carried out by a method well known to those skilled in the art. The prepared cDNA sample is detectably labeled as needed. The labeling substance is not particularly limited as long as it can be detected, and may include fluorescent substances and radioisotopes. cDNA may be labeled by a method generally used by those skilled in the art (L. Luo et al., Gene expression profiles of laser-captured adjacent neuronal subtypes, Nat. Med., 1999,117-122).

Those skilled in the art can appropriately determine the intensity of hybridization between cDNA and a nucleotide probe, depending on the type of substance used to label the cDNA sample. For example, when the cDNA is labeled with a fluorescent substance, it can be detected by reading the fluorescent signal with a scanner.

In another embodiment of the detection method of the present invention, a protein sample is first prepared from cells or tissue of a subject. The level of the protein of the present invention contained in the protein sample is measured. Then, the measured protein level is compared with that of a control.

Examples of a method for measurement include SDS polyacrylamide electrophoresis, and methods utilizing the antibody of the invention, such as Western blotting, dot-blotting, immunoprecipitation, enzyme-linked immunosorbent assay (ELISA), and immunofluorescence. Alternatively, it is possible to diagnose cancer by measuring the expression level of the protein of the present invention rather than measuring the expression level of the gene of the present invention.

In the above-mentioned method, the subject is diagnosed to have cancer or to have a high possibility of developing cancer when the expression level of the gene or protein of the present invention increased significantly compared with that of a control,

Further, the present invention provides a diagnostic drug to be used for detecting cancer. Examples of such a test drug include a test drug comprising the oligonucleotide of the present invention (including a substrate on which the oligonucleotide is immobilized), and a diagnostic drug comprising the antibody of the present invention. Any type of antibodies may be used as long as it is suitable for use in a diagnostic test. The antibody is labeled as needed.

The above-mentioned test drug may contain, in addition to the oligonucleotide or the antibody as an active ingredient, sterilized water, physiological saline, a vegetable oil, a surfactant, a lipid, a solubilizing agent, a buffer, a protein stabilizer (such as BSA or gelatin), a preservative or the like.

### Detection of C20orf102

In another aspect, the present specification discloses a method of diagnosing cancer by detecting C20orf102 protein. The method of the present disclosure is characterized by detecting C20orf102 protein. C20orf102 is a secretory protein with a secretory signal at the N-terminus, and its amino acid sequence and the gene sequence encoding this sequence are described in GenBank Accession No. NM_080607 (SEQ ID NOs: 2 and 66). In the present disclosure, C20orf102 protein encompasses both full-length protein and a fragment thereof. The fragment is a polypeptide containing a given domain of C20orf102 protein and it may not have a function of natural C20orf102 protein. The secretory signal of C20orf102 protein corresponds to 1 to 24 amino acids in the amino acid sequence represented by SEQ ID NO: 66 (Psort Prediction: http://psort.nibb.ac.jp/).

It was found that the expression of C20orf102 is elevated at the protein level with a very high frequency in a cancer cell, particularly in lung cancer, liver cancer (e.g., moderately differentiated liver cancer) or pancreatic cancer. In addition, it was shown that immunohistological diagnosis may be carried out by using a monoclonal antibody specific for C20orf102.

The C20orf102 protein to be detected in the present disclosure is preferably human C20orf102 protein, however, it may be any C20orf102 such as dog C20orf102, cat C20orf102, mouse C20orf102 or hamster C20orf102.

C20orf102 to be detected in the present disclosure may be C20orf102 of pre-secreted type, however, it is preferably C20orf102 of post-secreted type. C20orf102 is a secretory protein with a secretory signal at the N-terminus, and is secreted to the outside of a cell after being produced in the cell. C20orf102 of post-secreted type means those C20orf102 proteins present outside the cell.

Detection includes quantitative or non-quantitative detection. For example, non-quantitative detection includes simple determination of whether or not C20orf102 protein is present, determination of whether or not a predetermined amount or more of C20orf102 protein is present, comparison of the amount of C20orf102 protein with that in another sample (e.g., a control sample, etc.). Quantitative detection includes determination of the concentration of C20orf102 protein, determination of the amount of C20orf102 protein and the like.

The test sample is not particularly limited as long as it may contain C20orf102 protein, but is preferably those collected from the body of an organism such as a mammal, more preferably those collected from human. Specific examples of the test sample include cells, cell homogenate, blood, interstitial fluid, plasma, extravascular fluid, cerebrospinal fluid, synovial fluid, pleural fluid, serum, lymph fluid, saliva, urine and the like, preferably blood, serum or plasma. Further, a sample derived from a test sample, such as a culture solution of cells collected from the body of an organism, is also included in the test sample of the present invention.

Specific examples of the cancer to be diagnosed according to the disclosure include, but not limited to, liver cancer, pancreatic cancer, lung cancer, large bowel cancer, breast cancer, renal cancer, brain tumor, uterine cancer, lung cancer, stomach cancer, prostate cancer, leukemia, lymphoma and the like. Preferred are lung cancer, liver cancer and pancreatic cancer.

Liver cancer is classified into poorly differentiated liver cancer, moderately differentiated liver cancer, well differentiated liver cancer and the like. Any liver cancer may be detected according to the present invention. Preferably, moderately differentiated liver cancer is detected.

Lung cancer is further classified into lung adenocarcinoma, lung squamous cell carcinoma, lung small cell cancer, lung large cell cancer and the like. Any lung cancer may be detected according to the present disclosure. Preferably, lung adenocarcinoma is detected.

In the present disclosure, the subject is diagnosed to have cancer or to have a high possibility of developing cancer when C20orf102 protein is detected in a test sample, or when it is determined that the amount of C20orf102 protein detected in a test sample is higher compared with that of a negative control or a normal healthy subject.

In a preferred embodiment of the diagnostic method of the present disclosure, the diagnostic method is characterized by detecting C20orf102 protein released from a cell and present in the blood. Particularly preferably, C20orf102 protein or a fragment thereof present in the blood is detected.

C20orf102 protein contained in a test sample may be detected by any methods, but is preferably detected by an immunological method using an anti-C20orf102 antibody. Examples of the immunological method include radioimmunoassay, enzyme immunoassay, fluorescence immunoassay, luminescent immunoassay, immunoprecipitation, immunonephelometry, Western blotting, immunostaining, immunodiffusion method and the like. Preferred is an enzyme immunoassay, and particularly preferred is an enzyme-linked immunosorbent assay (ELISA) (e.g., sandwich ELISA). The above-mentioned immunological methods such as ELISA may be carried out by a method known to those skilled in the art.

For example, a general detection method for detecting C20orf102 protein in a test sample using an anti-C20orf102 antibody may comprise the step of immobilizing the anti-C20orf102 antibody on a support, adding a test sample thereto, incubating the sample to allow for binding the anti-C20orf102 antibody to C20orf102 protein, washing, and detecting C20orf102 protein bound to the support via the anti-C20orf102 antibody.

The support to be used for immobilizing the anti-C20orf102 antibody in the present disclosure may include an insoluble support made of an insoluble polysaccharide such as agarose or cellulose, a synthetic resin such as a silicon resin, polystyrene resin, a polyacrylamide resin a nylon resin or a polycarbonate resin, glass or the like. Such a support may be used in the form of beads, a plate or the like. In the case of beads, a column may be filled with these beads. A plate may include a multiwell plate (96-multiwell plate or the like), a biosensor chip or the like. The binding of the anti-C20orf102 antibody to the support may be effected by a commonly used method such as via a chemical bond or physical adsorption. All these supports are commercially available.

Usually, the anti-C20orf102 antibody may be bound to C20orf102 protein in a buffer. The buffer may include, for example, a phosphate buffer, a Tris buffer, a citrate buffer, a borate buffer, a carbonate buffer or the like. Incubation may be carried out under commonly used conditions, for example at 4°C to room temperature for 1 hour to 24 hours. The washing step after the incubation can be carried out in any way as long as it does not inhibit the binding of C20orf102 protein to the anti-C20orf102 antibody, for example, with a buffer containing a surfactant such as Tween 20.

In the method of detecting C20orf102 protein of the present disclosure, a control sample may be prepared besides the test sample to be analyzed for C20orf102 protein. The control sample includes a negative control sample that does not contain C20orf102 protein and a positive control sample that contains C20orf102 protein. In this case, C20orf102 protein in a test sample can be detected by comparing the result with the result obtained from the negative control sample that does not contain C20orf102 protein, or the result from the positive control sample that contains C20orf102 protein. In addition, a series of control samples having incremental concentrations are prepared, and the results from the respective control samples are obtained as numerical values to create a standard curve. C20orf102 protein contained in a test sample can be quantitatively detected from the numerical value of the result from the test sample based on the standard curve.

In a preferred embodiment, C20orf102 protein bound to the support via the anti-C20orf102 antibody may be detected using an anti-C20orf102 antibody labeled with a labeling substance. For example, a test sample is brought into contact with an anti-C20orf102 antibody immobilized on a support, washed and detected using a labeled antibody that specifically recognizes C20orf102 protein.

The labeling of the anti-C20orf102 antibody may be carried out by a commonly known method. Any of the labeling substances known to those skilled in the art may be used in the invention, such as a fluorescent dye, an enzyme, a coenzyme, a chemiluminescent substance or a radioactive substance. Specific examples include radioisotopes (such as ³²P, ¹⁴C, ¹²⁵I, ³H and ¹³¹I), fluorescein, rhodamine, dansyl chloride, umbelliferone, luciferase, peroxidase, alkaline phosphatase, β-galactosidase, β-glucosidase, horseradish peroxidase, glucoamylase, lysozyme, saccharide oxidases, microperoxidase, biotin and the like. When biotin is used as a labeling substance, it is preferred that a biotin-labeled antibody is added and then avidin conjugated with an enzyme such as alkaline phosphatase is added. The labeling substance may be bound to the anti-C20orf102 antibody by a known method such as a glutaraldehyde method, a maleimide method, a pyridyl disulfide method or a periodate method.

Specifically, a solution containing an anti-C20orf102 antibody is added to a support such as a plate, and the anti-C20orf102 antibody is immobilized on the support. After the plate is washed, the plate is blocked with, for example, BSA, gelatin, albumin or the like in order to prevent unspecific binding of proteins. Then, the plate is washed again and a test sample is added to the plate. After incubation, the plate is washed a labeled anti-C20orf102 antibody is added. After appropriate incubation, the plate is washed, and the labeled anti-C20orf102 antibody remaining on the plate is detected. The detection can be carried out by a method known to those skilled in the art. For example, in the case where the labeling is carried out with a radioisotope, the protein can be detected by liquid scintillation or an RIA method. In the case where the labeling is carried out with an enzyme, a substrate is added, and the enzymatic change of the substrate such as chromogenic change can be detected with an absorption spectrometer. Specific examples of the substrate include 2,2'-azinobis(3-ethylbenzothiazoline-6-sulfonic acid), diammonium salt (ABTS), 1,2-phenylenediamine (o-phenylenediamine), 3,3',5,5'-tetramethylbenzidine (TMB) and the like. In the case of a fluorescent substance, the change can be detected with a spectrofluorometer.

In a. particularly preferred embodiment of the method of the present disclosure, C20orf102 protein can be detected with a biotin-labeled anti-C20orf102 antibody and avidin.

Specifically, a solution containing an anti-C20orf102 antibody is added to a support such as a plate, and the anti-C20orf102 antibody is immobilized thereon. After the plate is washed, the plate is blocked with, for example BSA in order to prevent unspecific binding of proteins. Then, the plate is washed again and a test sample is added to the plate. After incubation, the plate is washed and a biotin-labeled anti-C20orf102 antibody is added. After appropriate incubation, the plate is washed, and avidin bound to an enzyme such as alkaline phosphatase or peroxidase is added to the plate. After incubation, the plate is washed and a substrate corresponding to the enzyme bound to avidin is added, and then C20orf102 protein is detected through the enzymatic change of the substrate as an indicator.

In another embodiment of the method of the present disclosure, C20orf102 protein can be detected using one or more types of primary antibodies specifically recognizing C20orf102 protein and one or more types of secondary antibodies specifically recognizing the primary antibodies.

For example, a test sample is brought into contact with one or more types of anti-C20orf102 antibodies immobilized on a support, the support is incubated and washed, and then C20orf102 protein bound after washing is detected by the primary anti-C20orf102 antibodies and one or more types of secondary antibodies specifically recognizing the primary antibodies. In this case, the secondary antibodies are preferably labeled with a labeling substance.

In another embodiment of the method of the present disclosure, C20orf102 protein can be detected by an agglutination reaction. In this method, C20orf102 can be detected using a support sensitized with an anti-C20orf102 antibody. The support sensitized with an anti-C20orf102 antibody may be of any types as long as it is insoluble, does not cause a non-specific reaction, and is stable. Such a support include, for example, latex particles, bentonite, collodion, kaolin, fixed sheep erythrocytes or the like. It is preferred to use latex particles. The latex particles may include polystyrene latex particles, styrene-butadiene copolymer latex particles, polyvinyltoluene latex particles or the like. It is preferred to use polystyrene latex particles. Sensitized particles are mixed with a sample, and stirred for a certain period of time. The degree of agglutination of the particles becomes higher as the concentration of anti-C20orf102 antibody contained in the sample is higher, therefore, C20orf102 can be detected by observing the agglutination with the naked eye. In addition, the protein can be detected by measuring the turbidity due to agglutination with a spectrophotometer.

In another embodiment of the method of the present disclosure, C20orf102 protein can be detected with a biosensor based on the surface plasmon resonance phenomenon. With the biosensor based on the surface plasmon resonance phenomenon, the protein-protein interaction can be observed with a small amount of unlabeled protein in real time as indicated by a surface plasmon resonance signal. For example, binding of the anti-C20orf102 antibody to C20orf102 protein can be detected with a biosensor such as BIAcore (manufactured by Amersham Biosciences). Specifically, a test sample is brought into contact with a sensor chip on which an anti-C20orf102 antibody has been immobilized, and then C20orf102 protein bound to the anti-C20orf102 antibody can be detected as a change in the resonance signal.

The detection method of the present invention can be automated using various automatic testing device, where a large number of samples can be tested at once.

It is also an object of the present disclosure to provide a diagnostic drug or kit for detecting C20orf102 protein in a test sample for diagnosis of cancer, where the diagnostic drug or kit contains at least the anti-C20orf102 antibody. In the case where the diagnostic drug or kit is based on an EIA method such as an ELISA method, the drug or kit may contain a support for immobilizing the antibody, or the antibody may be immobilized on the support in advance. In the case where the diagnostic drug or kit is based on the agglutination method using a support such as latex, the drug or kit may contain a support to which the antibody has been adsorbed. In addition, the kit may contain a blocking solution, a reaction solution, a reaction termination solution, a reagent for treating a sample or the like as needed.

### Production of anti-C20orf102 antibody

The anti-C20orf102 antibody to be used in the present disclosure may be of any origin, of any type (monoclonal or polyclonal), and of any form, as long as it specifically binds to C20orf102 protein. Specifically, any of known antibodies can be used in the disclosure, such as a mouse antibody, a rat antibody, a human antibody, a chimeric antibody or a humanized antibody. The antibody may be a polyclonal antibody, but is preferably a monoclonal antibody.

Further, an anti-C20orf102 antibody to be immobilized on a support and an anti-C20orf102 antibody to be labeled with a labeling substance may recognize the same epitope of the C20orf102 molecule, but preferably recognize different epitopes. The sites to be recognized are not particularly limited.

The anti-C20orf102 antibody to be used in the present disclosure can be obtained as a polyclonal antibody or a monoclonal antibody by a known technique. A monoclonal antibody derived from a mammal is particularly preferred as the anti-C20orf102 antibody to be used in the present disclosure. Examples of the monoclonal antibody derived from a mammal include those produced by a hybridoma and those produced by a host transformed with an expression vector containing a genetically engineered antibody gene.

A monoclonal antibody-producing hybridoma can be principally produced using a known technique as follows. An animal is immunized with C20orf102 as a sensitizing antigen according to a common immunization method. The immunocyte is obtained and fused with a known parent cell by a common cell fusion method. A cell producing a monoclonal antibody is screened by a common screening method.

Specifically, a monoclonal antibody can be produced as follows.

First, C20orf102 to be used as a sensitizing antigen for obtaining an antibody is prepared by expressing C20orf102 gene/amino acid sequence described in GenBank Accession No. NM_080607. More specifically, the gene sequence encoding C20orf102 is inserted into a known vector system, an appropriate host cell is transformed with the vector, and then , a desired human C20orf102 protein of interest is purified by a known method from the host cell or the culture supernatant thereof. Alternatively, C20orf102 protein may be purified from a natural source.

Subsequently, the purified C20orf102 protein is used as a sensitizing antigen. Alternatively, a partial peptide of C20orf102 can be used as a sensitizing antigen. In this case, the partial peptide can be obtained by chemical synthesis based on the an amino acid sequence of human C20orf102, or by introducing a portion of C20orf102 gene into an expression vector, or by digesting native C20orf102 with a protease. Any site and size of C20orf102 may be used as a partial peptide.

Any types of mammal may be immunized with the sensitizing antigen, but is preferably selected in consideration of its compatibility with the parent cell to be used in cell fusion. Generally a mammal include a rodent such as a mouse, a rat or a hamster, or a rabbit, a monkey or the like.

An animal is immunized with a sensitizing antigen using a known method. In a commonly used method, the sensitizing antigen is injected into the mammal intraperitoneally or subcutaneously. Specifically, a sensitizing antigen is diluted and suspended in an appropriate amount of phosphate buffered saline (PBS), physiological saline or the like, and mixed with an appropriate amount of a common adjuvant such as Freund's complete adjuvant as needed. After being emulsified, it is administered to a mammal for several times every 4 to 21 days. Additionally a suitable carrier may be used upon immunization of the sensitizing antigen. In particular, when a partial peptide with a small molecular weight is used as a sensitizing antigen, it is preferred to bind it to a carrier protein such as albumin or keyhole limpet hemocyanin for immunization.

After a mammal is immunized as described above and the increase in the desired antibody level in the serum is observed, the immunocytes are taken out from the mammal and are subjected to cell fusion. Preferred immunocytes include, in particular, the spleen cells.

A mammalian myeloma cell may also be used as a parent cell for cell fusion with the above-mentioned immunocyte. Preferably, known variety cell lines are used as the myeloma cell such as P3 (P3x63Ag8.653) (J. Immunol. (1979) 123, 1548-1550), P3x63Ag8U.1 (Current Topics in Microbiology and Immunology (1978) 81, 1-7), NS-1 (Kohler, G. and Milstein, C. , Eur. J. Immunol. (1976) 6, 511-519), MPC-11 (Margulies, D. H. et al., Cell (1976) 8, 405-415), SP2/0 (Shulman, M. et al., Nature (1978) 276, 269-270), FO (de St. Groth, S. F. et al., J. Immunol. Methods (1980) 35, 1-21), S194 (Trowbridge, I. S., J. Exp. Med. (1978) 148, 313-323), and R210 (Galfre, G. et al., Nature (1979) 277, 131-133).

The cell fusion between the immunocyte and the myeloma cell may be carried out principally according to a known method such as a method of Kohler and Milstein et al. (Kohler, G. and Milstein, C., Methods Enzymol. (1981) 73, 3-46).

More specifically, the above-mentioned cell fusion is carried out in a usual nutritional medium in the presence of, for example, a cell fusion-promoting agent. The cell fusion-promoting agent include, for example, polyethyleneglycol (PEG), Sendai virus (HVJ) or the like. An auxiliary agent such as dimethylsulfoxide can also be used to increase the fusion efficiency as needed.

The ratio of the number of the immunocyte to the myeloma cell to be used may be appropriately determined. For example, the number of the immunocyte is preferred to be set at 1 to 10 times that of the myeloma cell. The culture medium to be used in the above-mentioned cell fusion includes culture media suitable for the growth of the above-mentioned myeloma cell line, for example, RPMI 1640 culture medium and MEM culture medium, and a standard culture medium which is used for this type of cell culture. Further, a serum supplement such as fetal calf serum (FCS) may be used in combination.

In cell fusion, predetermined number of above-mentioned immunocytes and myeloma cells are thoroughly mixed in the above-mentioned culture medium, a PEG solution previously heated to about 37°C (for example, an average molecular weight of about 1000 to 6000) is added at a concentration of 30 to 60% (w/v) and mixed to form a desired fusion cell (hybridoma). Then, the process of sequential addition of an appropriate culture medium, centrifugation and removal of a supernatant is repeated to remove the cell fusion agent and those which are undesirable for the growth of the hybridoma.

The resulting hybridoma is then selected by culturing it in a standard selection culture medium such as HAT culture medium (a culture medium containing hypoxanthine, aminopterin, and thymidine). The cultivation in the above-mentioned HAT culture medium is continued for sufficient time (usually from several days to several weeks) so that cells other than the desired hybridoma (non-fused cells) will die. Then, a hybridoma that produces a desired antibody is screened and monocloned by a standard limiting dilution method.

A desired antibody may be screened and monocloned by a known screening method based on an antigen-antibody reaction. For example, an antigen is bound to a support such as beads made of polystyrene or the like or a commercially available 96-well microtiter plate, then a culture supernatant of hybridoma is added. After the support is washed, an enzyme-labeled secondary antibody or the like is added to determine whether or not a desired antibody reacting with the sensitizing antigen is contained in the culture supernatant. The hybridoma that produces a desired antibody can be cloned by a limiting dilution method or the like. The antigen used for immunization may be used in the screening procedure.

In addition to the above-mentioned method where an animal other than human is immunized with an antigen to obtain a hybridoma, it is also possible to sensitize a human lymphocyte in vitro with C20orf102, and the resulting sensitized lymphocyte is fused with a human myeloma cell having the ability to divide permanently, whereby a desired human antibody having the activity of binding to C20orf102 can be obtained (see JP-B-1-59878). Alternatively, C20orf102 is administered to a transgenic animal having the repertoire of all the genes for human antibody to obtain a cell producing the anti-C20orf102 antibody. The cell is immortalized and a human antibody against C20orf102 may be obtained from the immortalized cell (see International Patent Application Nos. WO 94/25585, WO 93/12227, WO 92/03918 and WO 94/02602).

The thus prepared hybridoma that produces a monoclonal antibody can be subcultured in a standard culture medium, or can be stored for a long period of time in liquid nitrogen.

In order to obtain a monoclonal antibody from the hybridoma, the hybridoma is cultured according to a standard method and an antibody is obtained as the culture supernatant, alternatively, the hybridoma is administered to and grown in a mammal compatible with the hybridoma and an antibody is obtained as the ascites or the like. The former method is suitable for obtaining high-purity antibodies, whereas the latter is suitable for mass production of antibodies.

According to the present invention, a recombinant monoclonal antibody produced by genetic engineering techniques can also be used as a monoclonal antibody. The antibody gene is cloned from the hybridoma, introduced into an appropriate vector and introduced into the host cell to produce a recombinant-type monoclonal antibody (see, for example, Vandamme, A. M. et al., Eur. J. Biochem. (1990) 192, 767-775, 1990). Specifically, mRNA encoding the variable (V) region of the anti-C20orf102 antibody is isolated from the hybridoma producing the anti-C20orf102 antibody. The isolation of mRNA is carried out by a known method such as guanidine ultracentrifugation (Chirgwin, J. M. et al. Biochemistry (1979) 18, 5294-5299) or the AGPC method (Chomczynski, P. et al., Anal. Biochem. (1987) 162, 156-159) to prepare total RNA, and then a desired mRNA is prepared by using an mRNA Purification Kit (manufactured by Pharmacia). Alternatively, mRNA can be directly prepared by using a QuickPrep mRNA Purification Kit (manufactured by Pharmacia).

cDNA coding for the variable (V) region of the antibody is synthesized from the resulting mRNA by using a reverse transcriptase. The synthesis of the cDNA is carried out by using AMV Reverse Transcriptase First-strand cDNA Synthesis Kit (manufactured by Seikagaku Kogyo) or the like.
Alternatively, cDNA may be synthesized and amplified by the 5'-RACE method (Frohman, M. A. et al., Proc. Natl. Acad. Sci. USA (1988) 85, 8998-9002, Belyavsky, A. et al. , Nucleic Acids Res. (1989) 17, 2919-2932) using a 5'-Ampli FINDER RACE Kit (manufactured by Clontech), PCR and the like.

The desired DNA fragment is purified from the resulting PCR product and ligated with a vector DNA. Then a recombinant vector is constructed therefrom and introduced into E. coli or the like, and a colony is selected, whereby a desired recombinant vector is prepared. The nucleotide sequence of the desired DNA is checked by a known method such as the dideoxy nucleotide chain termination method.

Once the desired DNA encoding the V region of the anti-C20orf102 antibody is obtained, and the DNA is incorporated integrated into an expression vector containing DNA encoding the constant region (C region) of a desired antibody.

In order to produce the anti-C20orf102 antibody to be used in the present, disclosure, the antibody gene is incorporated into an expression vector so as to be expressed under the control of the expression regulatory region, for example, an enhancer or a promoter. Subsequently, a host cell is transformed with the expression vector, and the antibody is expressed in the cell.

The antibody gene may be expressed in the cell by separately introducing DNAs encoding the heavy chain (H chain) and the light chain (L chain) of the antibody into expression vectors and co-transforming a host cell with the vectors ; or by introducing DNAs encoding the H chain and the L chain into a single expression vector and transforming a host cell with the vector (see WO 94/11523).

In addition to the above-mentioned host cell, a transgenic animal can be used for the production of a recombinant antibody. For example, an antibody gene is inserted into the middle of a gene encoding a protein produced specifically into milk (such as goat β-casein) to prepare a fusion gene. A DNA fragment containing the fusion gene comprising the antibody gene is injected into a goat's embryo, which is then introduced into a female goat. A desired antibody can be obtained from milk produced by a transgenic goat which is born from the goat that had received the embryo or offspring thereof. To increase the amount of milk containing the desired antibody produced by the transgenic goat, an appropriate hormone may be administered to the transgenic goat (Ebert, K.M. et al., Bio/Technology (1994) 12, 699-702).

In addition to the above-mentioned antibodies, an artificially modified genetic recombinant-type antibody, such as a chimeric antibody or a humanized antibody can be used in the present invention. Such a modified antibody can be produced by using a known method.

The chimeric antibody may be obtained by ligating the DNA encoding the V region of the antibody obtained as described above with DNA encoding the C region of a human antibody, introducing the resulting DNA into an expression vector, and introducing the vector into a host for production of the antibody. By using this known method, a chimeric antibody useful for the present invention can be obtained.

A humanized antibody, also referred to as a "reshaped humane antibody", is obtained by grafting the complementarity determining region (CDR) of an antibody from a non-human mammal, such as a mouse, into the complementarity determining region of a human antibody. A general technique of genetic recombination is also known in the art (see European Patent Application EP125023 and WO 96/02576).

Specifically, a DNA sequence designed to ligate a mouse antibody CDR to the framework region (FR) of a human antibody is synthesized by PCR using as primers several oligonucleotides constructed to have overlapping portions at the ends of both CDR and FR (see the method described in WO 98/13388).

The framework region of the human antibody to be ligated via the CDR is selected such that the complementarity determining region will form a favorable antigen-binding site. As necessary, amino acids in the framework region of an antibody variable region may be substituted, so that the complementarity determining region of a reshaped human antibody forms an appropriate antigen-binding site (Sato, K. et al., Cancer Res. (1993) 53, 851-856).

C regions from the human antibody is used as the C region in the chimeric antibody or the humanized antibody. For example, Cγ1, Cγ2, Cγ3 or Cγ4 can be used for the H chain, and Cκ or Cλ can be used for the L chain. The C region of the human antibody may be modified in order to improve the stability of the antibody itself or the production process.

A chimeric antibody is composed of the variable region of an antibody derived from a non-human mammal and the constant region derived from a human antibody. On the other hand, a humanized antibody is composed of the complementarity determining region of an antibody derived a non-human mammal, and the framework region and the constant region derived from a human antibody. Since the antigenicity of the humanized antibody is expected to be reduced in human body, the humanized antibody is useful as an active ingredient of a therapeutic agent of the present disclosure.

The antibody to be used in the present disclosure is not limited to the whole antibody molecule and may be a fragment of the antibody or a modified fragment thereof as long as it binds to C20orf102. It includes a divalent antibody and a monovalent antibody. Examples of the fragment of the antibody include Fab, F(ab')2, Fv, Fab/c having one Fab and a full Fc, and a single chain Fv (scFv) where the Fv of the H chain and the L chain are linked via an appropriate linker. Specifically, an antibody is treated with an enzyme such as papain or pepsin to provide a fragment of the antibody. Alternatively, a gene encoding such an antibody fragment is constructed and introduced into an expression vector, and the antibody fragment is expressed in a suitable host cell (see, for example, Co, M. S. et al., J. Immunol. (1994) 152, 2968-2976, Better, M. & Horwitz, A. H. Methods in Enzymology (1989) 178, 476-496, Academic Press, Inc., Plueckthun, A. & Skerra, A. Methods in Enzymology (1989) 178, 476-496, Academic Press, Inc., Lamoyi, E., Methods in Enzymology (1989) 121, 652-663, Rousseaux, J. et al., Methods in Enzymology (1989) 121, 663-669, Bird, R. E. et al., TIBTECH (1991) 9, 132-137).

The scFv can be obtained by linking the H chain V region and the L chain V region of an antibody. In the scFv, the H chain V region and the L chain V region are preferably linked via a linker, preferably a peptide linker (Huston, J. S. et al. , Proc. Natl. Acad. Sci. U.S.A. (1988) 85, 5879-5883). The H chain V region and the L chain V region in scFv may be derived from any antibody described as an antibody in this specification. For example, any single chain peptide having 12 to 19 amino acid residues may be used as the peptide linker for ligating the V regions.

DNA encoding scFv can be obtained by amplifying a fragment by PCR using as a template a DNA portion encoding all or a desired amino acid sequence of the sequences of DNA encoding the H chain or the H chain V region of the above-mentioned antibody and DNA encoding the L chain or the L chain V region of the above-mentioned antibody with a primer pair that defines the both ends thereof. Then the fragment is amplified with a combination of DNA encoding a peptide linker portion and a primer pair which defines both ends to be ligated to the H chain and the L chain.

Once DNA encoding scFv is prepared, an expression vector containing the DNA and a host cell transformed with the expression vector can be obtained according to a standard method. The scFv can be obtained from such a host according to a standard method.

These antibody fragments can be produced in a host by obtaining the gene thereof in the same manner as described above and by allowing it to be expressed. The term "antibody" in the present invention also encompasses these antibody fragments.

A modified antibody, for example, an anti-C20orf102 antibody conjugated with any of a variety of molecules such as a labeling substance can also be used in the invention. The term "antibody" in the present invention also encompasses such a modified antibody. Such a modified antibody can be obtained by chemically modifying the antibody obtained as above. Methods of modifying an antibody have already been established in the art.

Further, the antibody to be used in the present invention may be a bispecific antibody. The bispecific antibody may have antigen-binding sites that recognize different epitopes on the C20orf102 molecule. Alternatively, one of which may recognize C20orf102, and the other may recognize a labeling substance or the like. The bispecific antibody can also be produced by ligating an HL pair of two types of antibodies, or by fusing hybridomas producing different monoclonal antibodies to provide a fusion cell producing the bispecific antibody. Furthermore, the bispecific antibody can also be produced by genetic engineering techniques.

Antibodies can be expressed from the antibody gene constructed as described above by a known method. In the case of a mammalian cell, the gene can be expressed by operably linking a conventional useful promoter, an antibody gene to be expressed and a poly A signal at the 3'-downstream of the gene. A promoter/enhancer includes, for example, a human cytomegalovirus immediate early promoter/enhancer.

Further, examples of the promoter/enhancer used for expressing antibodies to be used in the present invention include, for example, viral promoter/enhancers such as retrovirus, polyoma virus, adenovirus and simian virus 40 (SV40), mammalian promoter/enhancers such as human elongation factor 1α (HEF1α).

Antibodies can be readily expressed by the method of Mulligan et al. (Nature (1979) 277, 108) when SV40 promoter/enhancer is used, and by the method of Mizushima et al. (Nucleic Acids Res. (1990) 18, 5322) when HEF1α promoter/enhancer is used.

In the case of E. coli, the gene can be expressed by operably linking a conventional useful promoter, a signal sequence for antibody secretion and an antibody gene to be expressed. A promoter includes, for example, lacZ promoter and araB promoter. The gene can be expressed by the method of Ward et al. (Nature (1989) 341, 544-546; FASEB J. (1992) 6, 2422-2427) when the lacZ promoter is used, and by the method of Better et al. (Science (1988) 240, 1041-1043) when the araB promoter is used.

A signal sequence for antibody secretion may be used for producing the antibody in the periplasm of E. coli, such as pelB signal sequence (Lei, S. P. et al., J. Bacteriol. (1987) 169, 4379). After isolating the antibody produced in the periplasm, the antibody is appropriately refolded for use.

A replication origin may be derived from SV40, polyoma virus, adenovirus, bovine papilloma virus (BPV). To amplify the gene copy number in a host cell system, the expression vector may contain as a selection marker the aminoglycoside transferase (APH) gene, the thymidine kinase (TK) gene, the E. coli xanthine guaninephosphoribosyl transferase (Ecogpt) gene, the dihydrofolate reductase (dhfr) gene or the like.

Any expression system, for example, a eukaryotic cell or a prokaryotic cell can be used for producing the antibody to be used in the present invention. Examples of the eukaryotic cell include established animals cells such as mammalian cells, insect cells, filamentous fungus cells, and yeast cells and the like. Examples of the prokaryotic cell include bacteria cells such as E. coli cells.

The antibody to be used in the present invention is preferably expressed in a mammalian cell such as a CHO, COS, myeloma, BHK, Vero, or Hela cell.

Subsequently, the transformed host cell is cultured in vitro or in vivo to produce a desired antibody. The host cell may be cultured according to a known method. For example, DMEM, MEM, RPMI1640 and IMDM can be used as a culture medium, and a serum supplement such as fetal calf serum (FCS) may be used in combination.

The thus expressed and produced antibody can be isolated from the cell or the host animal and purified to homogeneity. The isolation and purification of the antibody to be used in the present invention can be carried out by using an affinity column. Examples of a Protein A column include Hyper D, POROS, Sepharose F. F. (manufactured by Pharmacia). Any other standard methods for isolation and purification of proteins may be used in the invention. For example, the antibody can be isolated and purified by appropriately selecting and combining chromatography columns, besides the above-mentioned affinity columns, filters, ultra filtration, salting-out, dialysis and the like (Antibodies A Laboratory Manual, Ed Harlow, David Lane, Cold Spring Harbor Laboratory, 1988).

### Cancer-associated gene of the present invention

A list of the cancer-associated genes identified in the present disclosure is shown in Table 1 with its name, a cancer tissue in which the expression of the gene is elevated, and SEQ ID NOs of the sequence of the gene and the sequence of a protein encoded by the gene.

**Table 1**

| No | Gene name | GenBank | Ref.ID | Cancer type in which expression is elevated | Gene SEQ ID NO | Amino acid SEQ ID NO |
|---|---|---|---|---|---|---|
| TEG1 | C20orf102 | AA206763 | NM_080607 | Lung cancer, Moderately differentiated liver cancer, Pancreatic cancer | 2 | 66 |
| TEG2 | ASCL2 | AI393930 | | Stomach cancer, Large bowel cancer, Lung cancer, Pancreatic cancer, Metastatic tissue of large bowel cancer (liver) | 3 | 67 |
| TEG3 | EST | BE645480 | | Stomach cancer, Moderately differentiated liver cancer, Large bowel cancer, Lung cancer, Pancreatic cancer, Metastatic tissue of large bowel cancer (liver) | 4 | |
| TEG4 | EST | AA447317 | | Stomach cancer, Large bowel cancer, Metastatic tissue of large bowel cancer (liver) | 5 | |
| TEG5 | EST | AI217375 | | Stomach cancer, Pancreatic cancer | 6 | |
| TEG6 | OK/SW-CL..30 | AI217375 | | Lung cancer, Stomach cancer, Large bowel cancer, Moderately differentiated liver cancer | 7 | 68 |
| TEG7 | DKFZp686L15 33 | BG492359 | | Lung cancer, Stomach cancer, Large bowel cancer, Moderately or poorly differentiated liver cancer, Metastatic tissue of large bowel cancer (liver) | 8 | |
| TEG8 | EST | BF825703 | | Stomach cancer, Poorly differentiated liver cancer, Lung cancer | 10 | 69 |
| TEG9 | LOC93082 | AL389981.1 | | Stomach cancer, Poorly differentiated liver cancer, Pancreatic cancer, Metastatic tissue of large bowel cancer (liver) | 11 | 70 |
| TEG10 | EST | BG285837 | | Stomach cancer, Moderately or poorly differentiated liver cancer, Lung cancer | 12 | |
| TEG11 | FLJ11041 | AI343467 | | Stomach cancer, Large bowel cancer, Moderately /differentiated liver cancer, Lung cancer, Pancreatic cancer, Metastatic tissue of large bowel cancer (liver) | 13 | 71 |
| TEG12 | EST | BF057073 | | Liver cancer | 15 | 72 |
| TEG13 | EST | H66658 | | Liver cancer | 16 | |
| TEG14 | ASPM | NM_018123.1 | | Stomach cancer, Large bowel cancer, Liver cancer, Lung cancer | 17 | 73 |
| TEG15 | Sp5 | AI380207 | | Stomach cancer, Large bowel cancer, Liver cancer, Lung cancer | 18 | 74 |
| TEG16 | IMAGE:29740 3 | AF339813.1 | | Liver cancer, Lung cancer, Pancreatic cancer, Metastatic tissue of large bowel cancer (liver) | 19 | |
| TEG17 | DKFZp434K24 35 | AL136855.1 | NM_032256 | Stomach cancer, Large bowel cancer, Liver cancer, Pancreatic cancer | 20 | 75 |
| TEG18 | CBRC7TM_24 9 | AI694413 | | Stomach cancer, Large bowel cancer, Moderately or poorly differentiated liver cancer, Pancreatic cancer, Metastatic tissue of large bowel cancer (liver) | 22 | 76 |
| TEG19 | MASS1/VLGR 1 | AF055084.1 | NM_032119 | Lung cancer, Pancreatic cancer | 1 | 77 |
| TEG20 | C20orf54 | AA903862 | NM_033409 | Stomach cancer, Large bowel cancer, Lung cancer, Metastatic tissue of large bowel cancer (liver) | 9 | 78 |
| TEG21 | RHBG | NM_020407.1 | NM_020407 | Liver cancer | 14 | 79 |
| TEG22 | COPG2 | AB047847.1 | NM_012133 | Large bowel cancer, Lung cancer | 21 | 80 |
| TEG23 | EST | | | Poorly differentiated liver cancer, Lung cancer | 64,65 | 81,82 |
| TEG24 | EST | BE670584 | | Stomach cancer, Lung cancer, Metastatic tissue of large bowel cancer (liver) | 23 | 83 |
| TEG25 | GPR49 | AL524520 | NM_003667 | Stomach cancer, Large bowel cancer, Moderately differentiated liver cancer, Lung cancer, Metastatic tissue of large bowel cancer (liver) | 24 | 84 |
| TEG26 | MUC17 | AK026404.1 | | Stomach cancer, Pancreatic cancer | 25 | 85 |
| TEG27 | EphB2 | AF025304.1 | NM_004442 | Stomach cancer, Large bowel cancer, Lung cancer, Metastatic tissue of large bowel cancer (liver) | 26 | 86 |
| TEG28 | FLJ11856/GP CR41 | AK021918.1 | NM_024531 | Stomach cancer, Large bowel cancer, Lung cancer, Metastatic tissue of large bowel cancer (liver), Pancreatic cancer | 27 | 87 |
| TEG29 | HS6ST2 | AI767756 | | Lung cancer, Large bowel cancer, poorly differentiated liver cancer, Pancreatic cancer | 28 | 88 |
| TEG30 | PCDHB2 | NM_018936.1 | MM_018936 | Lung cancer, Pancreatic cancer | 29 | 89 |
| TEG31 | WFDC3 | AL050348 | | Lung cancer, Pancreatic cancer | 30 | 90 |
| TEG32 | C20orf42 | NM_017671.1 | NM_017671 | Lung cancer, Stomach cancer, Large bowel cancer, Metastatic tissue of large bowel cancer (liver), | 31 | 91 |
| TEG33 | PIGR | NM_002644.1 | NM_002644 | Lung cancer, Large bowel cancer | 32 | 92 |
| TEG34 | NFE2L3 | NM_004289.3 | NM_004289 | Stomach cancer, Large bowel cancer, Lung cancer, Metastatic tissue of large bowel cancer (liver), Pancreatic cancer | 33 | 93 |
| TEG35 | TRAG3 | NM_004909.1 | NM_004909 | Stomach cancer, Lung cancer, Pancreatic cancer | 34 | 94 |
| TEG36 | TRIM31 | NM_007028 | | Stomach cancer, Pancreatic cancer, Lung cancer | 35 | 95 |
| TEG37 | KIAA1359 | AB037780 | | Stomach cancer, Lung cancer, Large bowel cancer, Pancreatic cancer, Metastatic tissue of large bowel cancer (liver) | 36 | 96 |
| TEG38 | ubiquitinD | NM_006398 | | Stomach cancer, Large bowel cancer, Lung cancer, Moderately or poorly differentiated liver cancer, Lung cancer, Pancreatic cancer | 37 | 97 |
| TEG39 | Hephaestin | NM_014799.1 | NM_014799 | Stomach cancer, Metastatic tissue of large bowel cancer (liver), Pancreatic cancer | 38 | 98 |
| TEG40 | KIAA0152 | BC000371.1 | NM_014730 | Stomach cancer, Large bowel cancer, Glioblastoma, Lung cancer | 39 | 99 |
| TEG41 | KIAA0703 | NM_014861.1 | NM_014861 | Stomach cancer, Lung cancer, Metastatic tissue of large bowel cancer (liver) | 40 | 100 |
| TEG42 | MEST/PEG1 | NM_002402.1 | NM_002402 | Stomach cancer, Large bowel cancer, Lung cancer | 41 | 101 |
| TEG43 | KIAA1199 | AB033025.1 | | Stomach cancer, Lung cancer, Large bowel cancer, Pancreatic cancer | 42 | 102 |
| TEG44 | ELOVL2 | BF508639 | NM_017770 | Liver cancer, Glioblastoma, Lung cancer | 43 | 103 |
| TEG45 | ROBO1 | BF059159 | NM_133631 | Liver cancer, Giloblastoma, Lung cancer | 44 | 104 |
| TEG46 | FLJ10504/mis ato | BC002535.1 | NM_018116 | Liver cancer, Lung cancer, Pancreatic cancer | 45 | 105 |
| TEG47 | cystatin SN | NM_001898.1 | NM_001898 | Large bowel cancer, Lung cancer | 46 | 106 |
| TEG48 | LOC116238 | BE328850 | NM_138463 | Stomach cancer, Large bowel cancer, Lung cancer, Poorly differentiated liver cancer, Pancreatic cancer | 47 | 107 |
| TEG49 | MRPL50 | BG028213 | NM_019051 | Stomach cancer, Large bowel cancer, Moderately or poorly differentiated liver cancer, Glioblastoma, Lung cancer, Pancreatic cancer | 48 | 108 |
| TEG50 | TOP1MT | AW592604 | MM_052963 | Large bowel cancer, Poorly differentiated liver cancer, Metastatic tissue of large bowel cancer (liver), Pancreatic cancer | 49 | 109 |
| TEG51 | FKSG14 | BC005400.1 | NM_022145 | Stomach cancer, Large bowel cancer, Lung cancer, Pancreatic cancer | 50 | 110 |
| TEG52 | CDH3 | NM_001793.1 | NM_001793 | Lung cancer, Stomach cancer, Large bowel cancer, Pancreatic cancer | 51 | 111 |
| TEG53 | NRP2 | N90777 | NM_003872 | Lung cancer, Glioblastoma, Metastatic tissue of large bowel cancer (liver), Pancreatic cancer | 52 | 112 |
| TEG54 | CLDN3 | BE791251 | NM_001306 | Stomach cancer, Lung cancer, Large bowel cancer, Metastatic tissue of large bowel cancer (liver) | 53 | 113 |
| TEG55 | CLDN4 | NM_001305.1 | NM_001305 | Stomach cancer, Lung cancer, Large bowel cancer, Metastatic tissue of large bowel cancer (liver), Pancreatic cancer | 54 | 114 |
| TEG56 | SFRP4 | AW089415 | NM_003014 | Lung cancer, Stomach cancer, Glioblastoma, Pancreatic cancer | 55 | 115 |
| TEG57 | ASPSCR1 | NM_024083.1 | NM_024083 | Liver cancer, Lung cancer | 56 | 116 |
| TEG58 | GAGEC1 | NM_007003.1 | MM_007003 | Liver cancer | 57 | 117 |
| TEG59 | RHAMM | NM_012485.1 | NM_012484 | Stomach cancer, Large bowel cancer, Liver cancer, Pancreatic cancer | 58 | 118 |
| TEG60 | PEG10 | BE858180 | NM_015068 | Liver cancer, Lung cancer, Hepatoblastoma | 59 | 119 |
| TEG61 | PAEP | NM_02571.1 | NM_002571 | Lung cancer, Pancreatic cancer | 60 | 120 |
| TEG62 | MGC10981 | BC004397.1 | NM_032654 | Lung cancer, Pancreatic cancer | 61 | 121 |
| TEG63 | DUSP9 | NM_001395.1 | NM_001395 | Liver cancer | 62 | 122 |
| TEG64 | EST1B | AB029012.1 | | Liver cancer, Lung cancer, Pancreatic cancer | 63 | 123 |

TEG1 (SEQ ID NO: 2; SEQ ID NO: 66) encodes C20orf102. The GenBank accession number of the gene is AA206763 (reference sequence ID: NM_080607). It was found that the expression of the gene is elevated in lung cancer, moderately differentiated liver cancer and pancreatic cancer. It is not known that the expression of the gene is associated with cancer.

TEG2 (SEQ ID NO: 3; SEQ ID NO: 67) encodes EST (ASCL2). The GenBank accession number of the gene is AI393930. It was found that the expression of the gene is elevated in stomach cancer, large bowel cancer, lung cancer, pancreatic cancer and metastatic tissue of large bowel cancer (liver). It is not known that the expression of the gene is associated with cancer.

TEG3 (SEQ ID NO: 4) encodes EST (EPST1 isoform). The GenBank accession number of the gene is BE645480. It was found that the expression of the gene is elevated in stomach cancer, moderately differentiated liver cancer, large bowel cancer, lung cancer, pancreatic cancer and metastatic tissue of large bowel cancer (liver). It is not known that the expression of the gene is associated with cancer.

TEG4 (SEQ ID NO: 5) encodes EST. The GenBank accession number of the gene is AA447317. It was found that the expression of the gene is elevated in stomach cancer, large bowel cancer and metastatic tissue of large bowel cancer (liver). It is not known that the expression of the gene is associated with cancer.

TEG5 (SEQ ID NO: 6) encodes EST. The GenBank accession number of the gene is AI217375. It was found that the expression of the gene is elevated in stomach cancer and pancreatic cancer. It is not known that the expression of the gene is associated with cancer.

TEG6 (SEQ ID NO: 7; SEQ ID NO: 68) encodes OK/SW-CL30. The GenBank accession number of the gene is AI217375. It was found that the expression of the gene is elevated in lung cancer, stomach cancer, large bowel cancer and moderately differentiated liver cancer. It is not known that the expression of the gene is associated with cancer.

TEG7 (SEQ ID NO: 8) encodes DKFZp686L1533. The GenBank accession number of the gene is BG492359. It was found that the expression of the gene is elevated in lung cancer, stomach cancer, large bowel cancer, moderately or poorly differentiated liver cancer and metastatic tissue of large bowel cancer (liver). It is not known that the expression of the gene is associated with cancer.

TEG8 (SEQ ID NO: 10; SEQ ID NO: 69) encodes EST (Gene #30). The protein encoded by the gene is a membrane protein. The GenBank accession number of the gene is BF825703. It was found that the expression of the gene is elevated in stomach cancer, poorly differentiated liver cancer and lung cancer. It is not known that the expression of the gene is associated with cancer.

TEG9 (SEQ ID NO: 11; SEQ ID NO: 70) encodes BC012317. The GenBank accession number of the gene is AL389981.1. It was found that the expression of the gene is elevated in stomach cancer, poorly differentiated liver cancer, pancreatic cancer and metastatic tissue of large bowel cancer (liver). It is not known that the expression of the gene is associated with cancer.

TEG10 (SEQ ID NO: 12) encodes EST242881. The GenBank accession number of the gene is BG285837. It was found that the expression of the gene is elevated in stomach cancer, moderately or poorly differentiated liver cancer and lung cancer. It is not known that the expression of the gene is associated with cancer.

TEG11 (SEQ ID NO: 13; SEQ ID NO: 71) encodes FLJ11041. The GenBank accession number of the gene is AI343467. It was found that the expression of the gene is elevated in stomach cancer, large bowel cancer, moderately differentiated liver cancer, lung cancer, pancreatic cancer and metastatic tissue of large bowel cancer (liver). It is not known that the expression of the gene is associated with cancer.

TEG12 (SEQ ID NO: 15; SEQ ID NO: 72) encodes EST. The GenBank accession number of the gene is BF057073. As described in the following Examples, the full-length sequence of the gene was determined in the present invention. It was found that the expression of the gene is elevated in liver cancer. It is not known that the expression of the gene is associated with cancer.

TEG13 (SEQ ID NO: 16) encodes EST. The GenBank accession number of the gene is H66658. It was found that the expression of the gene is elevated in liver cancer. It is not known that the expression of the gene is associated with cancer.

TEG14 (SEQ ID NO: 17 ; SEQ ID NO: 73) encodes ASPM. The GenBank accession number of the gene is NM_018123.1. It was found that the expression of the gene is elevated in stomach cancer, large bowel cancer, liver cancer and lung cancer. It is not known that the expression of the gene is associated with cancer.

TEG15 (SEQ ID NO: 18; SEQ ID NO: 74) encodes Sp5. The GenBank accession number of the gene is AI380207. It was found that the expression of the gene is elevated in stomach cancer, large bowel cancer, liver cancer and lung cancer. It is not known that the expression of the gene is associated with cancer.

TEG16 (SEQ ID NO: 19) encodes IMAGE: 297403. The GenBank accession number of the gene is AF339813.1. It was found that the expression of the gene is elevated in liver cancer, lung cancer, pancreatic cancer and metastatic tissue of large bowel cancer (liver). It is not known that the expression of the gene is associated with cancer.

TEG17 (SEQ ID NO: 20; SEQ ID NO: 75) encodes DKFZp434k2435. The protein encoded by the gene is a membrane protein. The GenBank accession number of the gene is AL136855.1 (reference sequence ID: NM_032256). It was found that the expression of the gene is elevated in stomach cancer, large bowel cancer, lung cancer and pancreatic cancer. It is not known that the expression of the gene is associated with cancer.

TEG18 (SEQ ID NO: 22; SEQ ID NO: 76) encodes CBRC7TM_249. The protein encoded by the gene is a membrane protein. The GenBank accession number of the gene is AI694413. It was found that the expression of the gene is elevated in stomach cancer, large bowel cancer, moderately or poorly differentiated liver cancer, pancreatic cancer and metastatic tissue of large bowel cancer (liver). It is not known that the expression of the gene is associated with cancer.

TEG19 (SEQ ID NO: 1; SEQ ID NO: 77) encodes VLGR1. The protein encoded by the gene is a membrane protein. The GenBank accession number of the gene is AF055084.1 (reference sequence ID: NM_032119). It was found that the expression of the gene is elevated in lung cancer and pancreatic cancer. It is not known that the expression of the gene is associated with cancer.

TEG20 (SEQ ID NO: 9; SEQ ID NO: 78) encodes C20orf54. The protein encoded by the gene is a membrane protein. The GenBank accession number of the gene is AA903862 (reference sequence ID: NM_033409). It was found that the expression of the gene is elevated in stomach cancer, large bowel cancer, lung cancer and metastatic tissue of large bowel cancer (liver). It is not known that the expression of the gene is associated with cancer.

TEG21 (SEQ ID NO: 14; SEQ ID NO: 79) encodes RHBG. The protein encoded by the gene is a membrane protein. The GenBank accession number of the gene is NM_020407.1 (reference sequence ID: NM_020407). It was found that the expression of the gene is elevated in liver cancer. It is not known that the expression of the gene is associated with cancer.

TEG22 (SEQ ID NO: 21; SEQ ID NO: 80) encodes COPG2. The GenBank accession number of the gene is AB047847.1 (reference sequence ID: NM_012133). It was found that the expression of the gene is elevated in large bowel cancer and lung cancer. It is not known that the expression of the gene is associated with cancer.

TEG23 (SEQ ID NOs : 64 and 65; SEQ ID NOs : 81 and 82) encodes EST. The GenBank accession number of the gene is AL039884. As described in the following Examples, the full-length sequence of this gene was determined in the present invention. It was found that the expression of the gene is elevated in poorly differentiated liver cancer and lung cancer. It is not known that the expression of the gene is associated with cancer.

TEG24 (SEQ ID NO: 23; SEQ ID NO: 83) encodes BE670584. The GenBank accession number of the gene is BE670584. It was found that the expression of the gene is elevated in stomach cancer, lung cancer and metastatic tissue of large bowel cancer (liver). It is not known that the expression of the gene is associated with cancer.

TEG25 (SEQ ID NO: 24; SEQ ID NO: 84) encodes GRP49. The protein encoded by the gene is a membrane protein. The GenBank accession number of the gene is AL524520 (reference sequence ID: NM_003667). It was found that the expression of the gene is elevated in stomach cancer, large bowel cancer, moderately differentiated liver cancer, lung cancer and metastatic tissue of large bowel cancer (liver). It is not known that the expression of the gene is associated with cancer.

TEG26 (SEQ ID NO: 25; SEQ ID NO: 85) encodes MUC17. The protein encoded by the gene is a membrane protein. The GenBank accession number of the gene is AK026404.1. It was found that the expression of the gene is elevated in stomach cancer and pancreatic cancer. It is not known that the expression of the gene is associated with stomach cancer.

TEG27 (SEQ ID NO: 26; SEQ ID NO: 86) encodes EPHB2. The protein encoded by the gene is a membrane protein. The GenBank accession number of the gene is AF025304.1 (reference sequence ID: NM_004442). It was found that the expression of the gene is elevated in stomach cancer, large bowel cancer, lung cancer and metastatic tissue of large bowel cancer (liver). It is not known that the expression of the gene is associated with large bowel cancer.

TEG28 (SEQ ID NO: 27; SEQ ID NO: 87) encodes GPCR41 (FLJ11856). The protein encoded by the gene is a membrane protein. The GenBank accession number of the gene is AK021918.1 (reference sequence ID: NM_024531). It was found that the expression of the gene is elevated in stomach cancer, large bowel cancer, lung cancer, metastatic tissue of large bowel cancer (liver) and pancreatic cancer. It is not known that the expression of the gene is associated with stomach cancer.

TEG29 (SEQ ID NO: 28; SEQ ID NO: 88) encodes HS6ST2. The protein encoded by the gene is a membrane protein. The GenBank accession number of the gene is AI767756. It was found that the expression of the gene is elevated in lung cancer, large bowel cancer, poorly differentiated liver cancer and pancreatic cancer. It is not known that the expression of the gene is associated with lung cancer.

TEG30 (SEQ ID NO: 29; SEQ ID NO: 89) encodes PCDHB2. The protein encoded by the gene is a membrane protein. The GenBank accession number of the gene is NM_018936.1 (reference sequence ID: NM_018936). It was found that the expression of the gene is elevated in lung cancer and pancreatic cancer. It is not known that the expression of the gene is associated with lung cancer.

TEG31 (SEQ ID NO: 30; SEQ ID NO: 90) encodes WFDC3 (C20orf167). The GenBank accession number of the gene is AL050348. It was found that the expression of the gene is elevated in lung cancer and pancreatic cancer. It is not known that the expression of the gene is associated with lung cancer.

TEG32 (SEQ ID NO: 31; SEQ ID NO: 91) encodes C20orf42. The GenBank accession number of the gene is NM_017671.1 (reference sequence ID: NM_017671). It was found that the expression of the gene is elevated in lung cancer, stomach cancer, large bowel cancer and metastatic tissue of large bowel cancer (liver). It is not known that the expression of the gene is associated with lung cancer.

TEG33 (SEQ ID NO: 32; SEQ ID NO: 92) encodes PIGR. The protein encoded by the gene is a membrane protein. The GenBank accession number of the gene is NM_002644.1 (reference sequence ID: NM_002644). It was found that the expression of the gene is elevated in lung cancer and large bowel cancer. It is not known that the expression of the gene is associated with lung cancer.

TEG34 (SEQ ID NO: 33; SEQ ID NO: 93) encodes 2FE2L3. The GenBank accession number of the gene is NM_004289.3 (reference sequence ID: NM_004289). It was found that the expression of the gene is elevated in stomach cancer, large bowel cancer, lung cancer, metastatic tissue of large bowel cancer (liver) and pancreatic cancer. It is not known that the expression of the gene is associated with stomach cancer.

TEG35 (SEQ ID NO: 34; SEQ ID NO: 94) encodes TRAG3. The GenBank accession number of the gene is NM_004909.1 (reference sequence ID: NM_004909). It was found that the expression of the gene is elevated in stomach cancer, lung cancer and pancreatic cancer. It is not known that the expression of the gene is associated with stomach cancer.

TEG36 (SEQ ID NO: 35; SEQ ID NO: 95) encodes TRIM31. The GenBank accession number of the gene is NM_007028. It was found that the expression of the gene is elevated in stomach cancer, pancreatic cancer and lung cancer. It is not known that the expression of the gene is associated with stomach cancer.

TEG37 (SEQ ID NO: 36; SEQ ID NO: 96) encodes KIAA1359. The GenBank accession number of the gene is AB037780. It was found that the expression of the gene is elevated in stomach cancer, lung cancer, large bowel cancer, pancreatic cancer and metastatic tissue of large bowel cancer (liver). It is not known that the expression of the gene is associated with stomach cancer.

TEG38 (SEQ ID NO: 37; SEQ ID NO: 97) encodes ubiquitin D. The GenBank accession number of the gene is NM_006398. It was found that the expression of the gene is elevated in stomach cancer, large bowel cancer, lung cancer, moderately or poorly differentiated liver cancer, lung cancer and pancreatic cancer. It is not known that the expression of the gene is associated with stomach cancer.

TEG39 (SEQ ID NO: 38; SEQ ID NO: 98) encodes Hephaestin. The protein encoded by the gene is a membrane protein. The GenBank accession number of the gene is NM_014799.1 (reference sequence ID: NM_014799). It was found that the expression of the gene is elevated in stomach cancer, metastatic tissue of large bowel cancer (liver) and pancreatic cancer. It is not known that the expression of the gene is associated with stomach cancer.

TEG40 (SEQ ID NO: 39; SEQ ID NO: 99) encodes KIAA0152. The protein encoded by the gene is a membrane protein. The GenBank accession number of the gene is BC000371.1 (reference sequence ID: NM_014730). It was found that the expression of the gene is elevated in stomach cancer, large bowel cancer, glioblastoma and lung cancer. It is not known that the expression of the gene is associated with stomach cancer.

TEG41 (SEQ ID NO: 40; SEQ ID NO: 100) encodes KIAA0703. The protein encoded by the gene is a membrane protein. The GenBank accession number of the gene is NM_014861.1 (reference sequence ID: NM_014861). It was found that the expression of the gene is elevated in stomach cancer, lung cancer and metastatic tissue of large bowel cancer (liver). It is not known that the expression of the gene is associated with stomach cancer.

TEG42 (SEQ ID NO: 41; SEQ ID NO: 101) encodes MEST/PEG1. The GenBank accession number of the gene is NM_002402.1 (reference sequence ID: NM_002402). It was found that the expression of the gene is elevated in stomach cancer, large bowel cancer and lung cancer. It is not known that the expression of the gene is associated with stomach cancer.

TEG43 (SEQ ID NO: 42; SEQ ID NO: 102) encodes KIAA1199. The GenBank accession number of the gene is AB033025.1. It was found that the expression of the gene is elevated in stomach cancer, lung cancer, large bowel cancer and pancreatic cancer. It is not known that the expression of the gene is associated with stomach cancer.

TEG44 (SEQ ID NO: 43; SEQ ID NO: 103) encodes ELOVL2. The GenBank accession number of the gene is BF508639 (reference sequence ID: NM_017770). It was found that the expression of the gene is elevated in liver cancer, glioblastoma and lung cancer.

TEG45 (SEQ ID NO: 44; SEQ ID NO: 104) encodes ROBO1. The protein encoded by the gene is a membrane protein. The GenBank accession number of the gene is BF059159 (reference sequence ID: NM_133631). It was found that the expression of the gene is elevated in liver cancer, glioblastoma and lung cancer.

TEG46 (SEQ ID NO: 45; SEQ ID NO: 105) encodes FLJ10504 MISATO. The GenBank accession number of the gene is BC002535.1 (reference sequence ID: NM_018116). It was found that the expression of the gene is elevated in liver cancer, lung cancer and pancreatic cancer. It is not known that the expression of the gene is associated with liver cancer.

TEG47 (SEQ ID NO: 46; SEQ ID NO: 106) encodes cystatin SN. The GenBank accession number of the gene is MM_001898.1 (reference sequence ID: NM_001898). It was found that the expression of the gene is elevated in large bowel cancer and lung cancer. It is not known that the expression of the gene is associated with large bowel cancer.

TEG48 (SEQ ID NO: 47 ; SEQ ID NO: 107) encodes LOC116238. The GenBank accession number of the gene is BE328850 (reference sequence ID: NM_138463). It was found that the expression of the gene is elevated in stomach cancer, large bowel cancer, lung cancer, poorly differentiated liver cancer and pancreatic cancer.

TEG49 (SEQ ID NO: 48; SEQ ID NO: 108) encodes MRPL50. The GenBank accession number of the gene is BG028213 (reference sequence ID: NM_019051). It was found that the expression of the gene is elevated in stomach cancer, large bowel cancer, moderately or poorly differentiated liver cancer, glioblastoma, lung cancer and pancreatic cancer.

TEG50 (SEQ ID NO: 49; SEQ ID NO: 109) encodes TOP1mt. The GenBank accession number of the gene is AW592604 (reference sequence ID: NM_052963). It was found that the expression of the gene is elevated in large bowel cancer, poorly differentiated liver cancer, metastatic tissue of large bowel cancer (liver) and pancreatic cancer. It is not known that the expression of the gene is associated with large bowel cancer.

TEG51 (SEQ ID NO: 50; SEQ ID NO: 110) encodes FKSG14. The GenBank accession number of the gene is B005400.1 (reference sequence ID: NM_022145). It was found that the expression of the gene is elevated in stomach cancer, large bowel cancer, lung cancer and pancreatic cancer. It is not known that the expression of the gene is associated with large bowel cancer.

TEG52 (SEQ ID NO: 51; SEQ ID NO: 111) encodes CDH3. The protein encoded by the gene is a membrane protein. The GenBank accession number of the gene is NM_001793.1 (reference sequence ID: MM_001793). It was found that the expression of the gene is elevated in lung cancer, stomach cancer, large bowel cancer and pancreatic cancer.

TEG53 (SEQ ID NO: 52; SEQ ID NO: 112) encodes NRP2. The protein encoded by the gene is a membrane protein. The GenBank accession number of the gene is N90777 (reference sequence ID: NM_003872). It was found that the expression of the gene is elevated in lung cancer, glioblastoma, metastatic tissue of large bowel cancer (liver) and pancreatic cancer.

TEG54 (SEQ ID NO: 53; SEQ ID NO: 113) encodes CLDN3. The protein encoded by the gene is a membrane protein. The GenBank accession number of the gene is BE791251 (reference sequence ID: NM_001306). It was found that the expression of the gene is elevated in stomach cancer, lung cancer, large bowel cancer and metastatic tissue of large bowel cancer (liver). It is not known that the expression of the gene is associated with stomach cancer.

TEG55 (SEQ ID NO: 54; SEQ ID NO: 114) encodes CLDN4. The protein encoded by the gene is a membrane protein. The GenBank accession number of the gene is NM_001305.1 (reference sequence ID: NM_001305). It was found that the expression of the gene is elevated in stomach cancer, lung cancer, large bowel cancer, metastatic tissue of large bowel cancer (liver) and pancreatic cancer. It is not known that the expression of the gene is associated with stomach cancer.

TEG56 (SEQ ID NO: 55 ; SEQ ID NO: 115) encodes sfrp4. The GenBank accession number of the gene is AW089415 (reference sequence ID: NM_003014). It was found that the expression of the gene is elevated in lung cancer, stomach cancer, glioblastoma and pancreatic cancer. It is not known that the expression of the gene is associated with stomach cancer.

TEG57 (SEQ ID NO: 56; SEQ ID NO: 116) encodes ASPSCR1. The GenBank accession number of the gene is NM_024083.1 (reference sequence ID: NM_024083). It was found that the expression of the gene is elevated in liver cancer and lung cancer. It is not known that the expression of the gene is associated with liver cancer.

TEG58 (SEQ ID NO: 57; SEQ ID NO: 117) encodes GAGEC1. The GenBank accession number of the gene is NM_007003.1 (reference sequence ID: NM_007003). It was found that the expression of the gene is elevated in liver cancer. It is not known that the expression of the gene is associated with liver cancer.

TEG59 (SEQ ID NO: 58; SEQ ID NO: 118) encodes RHAMM. The protein encoded by the gene is a membrane protein. The GenBank accession number of the gene is NM_012485.1 (reference sequence ID: NM_012484). It was found that the expression of the gene is elevated in stomach cancer, large bowel cancer, liver cancer and pancreatic cancer. It is not known that the expression of the gene is associated with liver cancer.

TEG60 (SEQ ID NO: 59; SEQ ID NO: 119) encodes PEG10. The GenBank accession number of the gene is BE858180 (reference sequence ID: NM_015068). It was found that the expression of the gene is elevated in liver cancer, lung cancer and hepatoblastoma.

TEG61 (SEQ ID NO: 60 ; SEQ ID NO: 120) encodes PAEP. The GenBank accession number of the gene is NM_002571.1 (reference sequence ID: NM_002571). It was found that the expression of the gene is elevated in lung cancer and pancreatic cancer.

TEG62 (SEQ ID NO: 61; SEQ ID NO: 121) encodes MGC10981. The GenBank accession number of the gene is BC004397.1 (reference sequence ID: NM_032654). It was found that the expression of the gene is elevated in lung cancer and pancreatic cancer.

TEG63 (SEQ ID NO: 62; SEQ ID NO: 12.2) encodes DUSP9. The GenBank accession number of the gene is MM_001395.1 (reference sequence ID: NM_001395). It was found that the expression of the gene is elevated in liver cancer.

TEG64 (SEQ ID NO: 63 ; SEQ ID NO: 123) encodes KIAA1089. The GenBank accession number of the gene is AB029012.1. It was found that the expression of the gene is elevated in liver cancer, lung cancer and pancreatic cancer.

### Examples

Hereinafter, the present invention will be described in more detail with reference to Examples.

### Example 1

### Identification of gene whose expression is elevated in human cancer tissue

In order to identify a gene whose expression is elevated in each type of human cancer tissue (lung adenocarcinoma, stomach cancer, large bowel cancer, hepatocellular carcinoma and brain tumor) compared with that in normal tissue, an expression analysis of mRNA was carried out in each type of excised human cancer tissue using GeneChip (GeneChip^{™} HG-133A, B Target; manufactured by Affymetryx).

### 1.1. Identification of gene whose expression is elevated in human lung adenocarcinoma

In order to identify a gene whose expression is elevated in human lung adenocarcinoma compared with that in normal human lung tissue, an expression analysis of mRNA was carried out as follows.

First, total RNA was prepared from the cancerous part of 12 cases of excised lung adenocarcinoma tissue including different differentiation degrees and stages, and one case of normal lung using ISOGEN (Nippon Gene) according to the attached method. Then, mRNA expression in the lung adenocarcinoma and normal lung was analyzed using a Genechip^{™} HG-U133A, B (manufactured by Affymetryx). More specifically, 5 µg of a material obtained by mixing the respective total RNA prepared from the 12 cases in an equal amount as for the cancerous part, or 5 µg of the total RNA prepared from the one case of normal lung as a control was analyzed for gene expression according to Expression Analysis Technical Manual (Affymetryx). The mean value of expression scores of all genes in each analysis was defined as 100 and the expression level of each gene was indicated as a relative value.

### 1.2. Identification of gene whose expression is elevated in human stomach cancer

In order to identify a gene whose expression is elevated in human stomach cancer compared with that in normal human stomach tissue, an expression analysis of mRNA was carried out in the same manner as described above.

Total RNA was prepared from 3 cases of excised stomach cancer tissue and one case of normal stomach in the same manner as described above. Five micrograms of a material obtained by mixing the respective total RNA prepared from the 3 cases in an equal amount as for the cancerous part, or 5 µg of the total RNA prepared from the one case of normal stomach as a control was analyzed for mRNA expression using a Genechip^{™} HG-U133A, B (manufactured by Affymetryx). The mean value of expression scores of all genes in each analysis was defined as 100 and the expression level of each gene was indicated as a relative value.

### 1.3. Identification of gene whose expression is elevated in human large bowel cancer

Identification of a gene whose expression is elevated in human large bowel cancer compared with that in normal human large bowel tissue was carried out in the same manner as described above.

Total RNA was prepared from the cancerous part of 3 cases of excised large bowel cancer tissue and one case of normal large bowel tissue in the same manner as described above. Five micrograms of a material obtained by mixing the respective total RNA prepared from the 3 cases in an equal amount as for the cancerous part, or 5 µg of the total RNA prepared from the one case of normal large bowel as a control was analyzed for mRNA expression using a GeneChip^{™} HG-U133A, B Target (manufactured by Affymetryx). The mean value of expression scores of all genes in each analysis was defined as 100 and the expression level of each gene was indicated as a relative value.

### 1.4. Identification of gene whose expression is elevated in human hepatocellular carcinoma

Identification of a gene whose expression is elevated in human hepatocellular carcinoma compared with that in normal human liver was carried out in the same manner as described above.

Total RNA was prepared from the cancerous part of 3 cases of hepatitis C virus infected moderately differentiated hepatocellular carcinoma, 3 cases of hepatitis C virus infected poorly differentiated hepatocellular carcinoma and one case of normal liver tissue in the same manner as described above . Five micrograms of a material obtained by mixing the respective total RNA prepared from the 3 cases in an equal amount as for each cancerous part of the different differentiation degrees, or 5 µg of the total RNA prepared from the one case of the normal liver as a control was analyzed for mRNA expression using a GeneChip^{™} HG-U133A, B (manufactured by Affymetryx). The mean value of expression scores of all genes in each analysis was defined as 100 and the expression level of each gene was indicated as a relative value.

### 1.5. Identification of gene whose expression is elevated in human glioblastoma

Identification of a gene whose expression is elevated in human glioblastoma compared with that in normal human brain tissue was carried out in the same manner as described above.

Total RNA was prepared from the cancerous part of 5 cases of excised glioblastoma tissue and one case of normal brain tissue in the same manner as described above. Five micrograms of a material obtained by mixing the respective total RNA prepared from the 5 cases in an equal amount as for the cancerous part, or 5 µg of the total RNA prepared from the one case of the normal brain tissue as a control was analyzed for mRNA expression using a Genechip^{™} HG-U133A, B (manufactured by Affymetryx). The mean value of expression scores of all genes in each analysis was defined as 100 and the expression level of each gene was indicated as a relative value.

From the results of the above analyses, it was found that mRNA expression of the genes shown in Table 2 is elevated compared with that in each of the corresponding normal tissue.

**Table 2**

| No. | Name | Cancer type in which expression is elevated | Gene chip analysis results | | | | | | | | | | |
|---|---|---|---|---|---|---|---|---|---|---|---|---|---|
| | | | Lung | Lung cancer | Stomach | Stomach cancer | Large bowel | Large bowel cancer | Liver | Moderately differentiated liver cancer | Poorly differentiated liver cancer | Brain | Glioblastoma |
| TEG1 | C20orf102 | Lung cancer, Moderately differentiated liver cancer | 32 | 299.3 | 98.6 | 50.3 | 95.2 | 18.4 | 39.1 | 104.9 | 13 | 834.4 | 90.8 |
| TEG2 | ASCL2 | Stomach cancer, Large bowel cancer | 66.1 | 27.4 | 6 | 406.9 | 79.1 | 738.8 | 19.5 | 5.9 | 31.2 | 3.6 | 10.7 |
| TEG3 | EST | Stomach cancer, Moderately differentiated liver cancer | 65.1 | 74.6 | 92.1 | 440.8 | 112.9 | 107.6 | 142.3 | 216 | 164.4 | 53.2 | 86.9 |
| TEG4 | EST | Stomach cancer, Large bowel cancer | 50.9 | 25.1 | 41.4 | 117.2 | 52.5 | 106.8 | 12 | 38.5 | 12.5 | 31.2 | 61.1 |
| TEG5 | EST | Stomach cancer | 79.7 | 85 | 58.7 | 248.2 | 73.9 | 63.5 | 11.3 | 59.7 | 96.9 | 44 | 87.2 |
| TEG6 | OK/SW-CL.. 30 | Lung cancer, Stomach cancer, Large bowel cancer, Moderately differentiated liver cancer | 84.1 | 118.9 | 55.6 | 537.1 | 98.5 | 734.1 | 157.7 | 1781. 4 | 160.8 | 78.7 | 106 |
| TEG7 | DKFZp686L 1533 | Lung cancer, Stomach cancer, Large bowel cancer, Moderately or poorly differentiated liver cancer | 79.2 | 173.3 | 14.6 | 588.5 | 89.2 | 750.3 | 22.7 | 158 | 309.6 | 15.5 | 87.1 |
| TEG8 | EST | Stomach cancer, Poorly differentiated liver cancer | 59.1 | 50.8 | 37.5 | 260.7 | 36.3 | 22.7 | 58.7 | 26.8 | 120 | 68.7 | 17.4 |
| TEG9 | LOC93082 | Stomach cancer, Poorly differentiated liver cancer | 107.3 | 34.5 | 14.5 | 1030. 1 | 89.2 | 21.9 | 130 | 155.6 | 448.1 | 18.5 | 105.4 |
| TEG10 | EST | Stomach cancer, Moderately or poorly differentiated liver cancer | 38.1 | 37.8 | 32.8 | 385.8 | 20.3 | 20.5 | 28.2 | 103.9 | 356.5 | 60.2 | 63.5 |
| TEG11 | FLJ11041 | Stomach cancer, Large bowel cancer, Moderately differentiated liver cancer | 607.1 | 481.8 | 16.9 | 261.5 | 19.2 | 522.1 | 97.9 | 128.1 | 56.2 | 43.2 | 49.2 |
| TEG12 | EST | Liver cancer | 60.8 | 65.2 | 91 | 38.6 | 44.5 | 62.2 | 16.2 | 194.3 | 527 | 66.2 | 47.7 |
| TEG13 | EST | Liver cancer | 38 | 10.3 | 35.5 | 17 | 16.3 | 4.6 | 26.1 | 493.7 | 177.7 | 4.6 | 14.7 |
| TEG14 | ASPM | Stomach cancer, Large bowel cancer, Liver cancer | 1.3 | 45.1 | 3.8 | 107.3 | 18.2 | 99.6 | 3.6 | 111.3 | 246.1 | 1.5 | 83.8 |
| TEG15 | Sp5 | Stomach cancer, Large bowel cancer, Liver cancer | 8 | 15.8 | 57.9 | 219.2 | 14.5 | 270.1 | 11.2 | 288.7 | 219.2 | 12 | 6.8 |
| TEG16 | IMAGE:2974 03 | Liver cancer | 5.7 | 12.7 | 25 | 11.5 | 20.2 | 17.8 | 34.4 | 273.1 | 159.7 | 16.2 | 66.8 |
| TEG17 | DKFZp434K 2435 | Stomach cancer, Large bowel cancer | 11.1 | 5.9 | 16.1 | 183.1 | 16.6 | 98.1 | 8.4 | 17.3 | 9.5 | 14.5 | 18.8 |
| TEG18 | CBRC7TM 249 | Stomach cancer, Large bowel cancer, Moderately or poorly differentiated liver cancer | 13.6 | 86.6 | 45 | 240.4 | 19.6 | 175.4 | 158.8 | 669 | 949 | 13.5 | 47.9 |
| TEG19 | MASS1/VLG R1 | Lung cancer | 23.6 | 254.4 | 17.1 | 5.3 | 18 | 4.3 | 133.6 | 77.4 | 21.2 | 21.8 | 111.8 |
| TEG20 | C20orf54 | Stomach cancer, Large bowel cancer, Liver cancer | 21.7 | 69.6 | 22,8 | 261.1 | 22.4 | 50.5 | 8.4 | 8.2 | 24.4 | 6.6 | 15.5 |
| TEG21 | RHBG | Liver cancer | 8.7 | 13.4 | 19.1 | 5.4 | 15.6 | 8.6 | 17.4 | 792.6 | 57.1 | 15.5 | 9.3 |
| TEG22 | COPG2 | Large bowel cancer | 77 | 66.9 | 83.1 | 47.5 | 21.7 | 178.4 | 52.8 | 8.7 | 22.6 | 40.9 | 78.7 |
| TEG23 | EST | Poorly differentiated liver cancer | 35.1 | 81.2 | 2 | 21.1 | 28 | 15.9 | 9.3 | 33.7 | 539.9 | 22.9 | 42.2 |
| TEG24 | EST | Stomach cancer | 28.9 | 19.4 | 35.6 | 197.1 | 44.8 | 80.1 | 5.2 | 15.5 | 31.1 | 57.6 | 58.8 |
| TEG25 | GPR49 | stomach cancer, Large bowel cancer, Moderately differentiated liver cancer | 23.9 | 15.8 | 24.3 | 538.3 | 41.6 | 135.3 | 16.7 | 233.8 | 78.8 | 33.5 | 11.2 |
| TEG26 | MUC17 | Stomach cancer | 73.4 | 59.1 | 89.4 | 565.2 | 113.3 | 102.8 | 34.7 | 67.6 | 113.8 | 100 | 56.3 |
| TEG27 | EphB2 | Stomach cancer, Large bowel cancer | 23.2 | 47.5 | 6.8 | 218.7 | 62.8 | 189.4 | 6.6 | 55.1 | 13.6 | 28.7 | 49 |
| TEG28 | FLJ11856/G PCR41 | Stomach cancer, Large bowel cancer | 22.2 | 35.2 | 9.1 | 229.5 | 63.8 | 197.5 | 2.7 | 5.1 | 67.3 | 4.4 | 78.3 |
| TEG29 | HS6ST2 | Stomach cancer, Large bowel cancer, Poorly differentiated liver cancer | 20.8 | 472.6 | 3.6 | 2.3 | 37.2 | 164.9 | 4.5 | 6.5 | 191.4 | 104 | 69 |
| TEG30 | PCDHB2 | Lung cancer | 11.9 | 228.5 | 55.2 | 37.7 | 32.2 | 58.9 | 14.4 | 13.4 | 27.7 | 80.4 | 78 |
| TEG31 | WFDC3 | Lung cancer | 30.1 | 304.2 | 110.6 | 28.7 | 32.2 | 27.8 | 46.4 | 29.9 | 30.4 | 28.7 | 28.9 |
| TEG32 | C20orf42 | Lung cancer, Stomach cancer, Large bowel cancer | 11.6 | 43.8 | 127.7 | 365.4 | 175.8 | 535.2 | 7 | 17.3 | 44 | 23.5 | 15.4 |
| TEG33 | PIGR | Lung cancer | 63.2 | 382.6 | 129.9 | 149.3 | 520.1 | 423.7 | 102.3 | 101.8 | 96 | 65.2 | 77.7 |
| TEG34 | NFE2L3 | Stomach cancer, Large bowel cancer | 37 | 62.4 | 55.2 | 144.9 | 22 | 216.8 | 27.4. | 18.6 | 37.3 | 13.7 | 27.8 |
| TEG35 | TRAG3 | Stomach cancer | 1.8 | 1.7 | 1.9 | 74.4 | 1.2 | 1.3 | 1.7 | 1.6 | 1.4 | 1.4 | 1.9 |
| TEG36 | TRIM31 | Stomach cancer | 16.9 | 13.2 ' | 14.6 | 155.2 | 67.3 | 52.7 | 21 | 41.4 | 31 | 4.6 | 26.8 |
| TEG37 | KIAA1359 | Stomach cancer, Lung cancer, Large bowel cancer | 22.8 | 190.3 | 7.5 | 521.1 | 196.8 | 196.7 | 37.9 | 5.7 | 9.1 | 3.5 | 40.6 |
| TEG38 | ubiquitinD | Stomach cancer, Large bowel cancer, Lung cancer, Moderately or poorly differentiated liver cancer | 89.7 | 311.5 | 44.2 | 1172.8 | 60.1 | 605.7 | 269.2 | 1460.9 | 2542.8 | 42.1 | 69 |
| TEG39 | Hephaestin | Stomach cancer | 97.6 | 97.3 | 75.8 | 341.5 | 568.8 | 419.1 | 34.6 | 50.6 | 27 | 126.1 | 91.6 |
| TEG40 | KIAA0152 | Stomach cancer, Large bowel cancer, Glioblastoma | 32.5 | 82.1 | 36.2 | 214.9 | 58.2 | 233.5 | 25.1 | 45.8 | 94 | 22,6 | 109.4 |
| TEG41 | KIAA0703 | Stomach cancer | 84.6 | 46.3 | 20.1 | 214.3 | 195.3 | 77.4 | 13.1 | 3.5 | 4.7 | 24.9 | 5.9 |
| TEG42 | MEST/PEG1 | Stomach cancer, Large bowel cancer | 235.9 | 406.2 | 92.6 | 524.3 | 178.4 | 640.8 | 423 | 248.4 | 455.9 | 207.2 | 771.4 |
| TEG43 | KIAA1199 | Stomach cancer, Lung cancer, Large bowel cancer | 53.6 | 162.4 | 26.2 | 80.7 | 28.9 | 185 | 68.5 | 63.5 | 44.3 | 89.1 | 69.4 |
| TEG44 | ELOVL2 | Liver cancer, Glioblastoma | 10.1 | 0.8 | 2.8 | 3 | 15.5 | 1.9 | 68.8 | 224.9 | 233.5 | 76.5 | 121.2 |
| TEG45 | ROBO1 | Liver cancer, Glioblastoma | 58.5 | 49.1 | 32.4 | 38 | 21.4 | 123.2 | 9.1 | 236.4 | 563 | 64.3 | 152.3 |
| TEG46 | FLJ10504/ml sato | Liver cancer | 53.8 | 38.8 | 6.5 | 49.5 | 5.6 | 21.5 | 5.1 | 105.2 | 106.8 | 27.4 | 41.6 |
| TEG47 | cystatin SN | Large bowel cancer | 2.7 | 53.6 | 4.4 | 98.1 | 9.4 | 804.5 | 6.1 | 27.6 | 24.1 | 15.5 | 2.3 |
| TEG48 | LOC116238 | Stomach cancer, Large bowel cancer, Lung cancer, Poorly differentiated liver cancer | 6.9 | 159.3 | 45.6 | 122.8 | 10.1 | 136.9 | 43.3 | 63.2 | 220.2 | 80 | 60.5 |
| TEG49 | MRPL50 | Stomach cancer, Large bowel cancer, Moderately or poorly differentiated liver cancer, Glioblastoma | 77.8 | 86.1 | 98.1 | 191.2 | 43.8 | 256.5 | 72 | 155.3 | 200.8 | 47.7 | 100 |
| TEG50 | TOP1MT | Large bowel cancer, Poorly differentiated liver cancer | 16.5 | 30.8 | 19.1 | 49.7 | 31.3 | 206.4 | 24.9 | 31.9 | 306.2 | 25.5 | 19 |
| TEG51 | FKSG14 | Stomach cancer, Large bowel cancer | 23.1 | 38.1 | 11.1 | 114.8 | 32.2 | 165 | 14 | 37.8 | 31.9 | 2.6 | 82.6 |
| TEG52 | CDH3 | Lung cancer, Stomach cancer, Large bowel cancer | 24.1 | 172.5 | 5.8 | 64.5 | 5.4 | 131.3 | 4.1 | 3.7 | 2.3 | 14.1 | 5.9 |
| TEG53 | NRP2 | Lung cancer | 26.4 | 171.1 | 40.4 | 25.8 | 88 | 79.1 | 89.9 | 19.1 | 43.6 | 22.4 | 155.2 |
| TEG54 | CLDN3 | Stomach cancer, Lung cancer | 3.2 | 147.6 | 0.8 | 624.4 | 1206. 9 | 738.3 | 40.2 | 42.3 | 4.1 | 1.8 | 0.6 |
| TEG55 | CLDN4 | Stomach cancer, Lung cancer | 70.1 | 193.6 | 3.9 | 364.8 | 258.4 | 325.8 | 7.1 | 37.4 | 45.4 | 3.3 | 2.5 |
| TEG56 | SFRP4 | Lung cancer, Stomach cancer, Glioblastoma | 153.6 | 244.9 | 66.9 | 153.1 | 69.4 | 87.8 | 51.1 | 49.2 | 49.3 | 53.4 | 250.3 |
| TEG57 | ASPSCR1 | Liver cancer | 42.4 | 45.4 | 41.5 | 75.1 | 28.4 | 102.3 | 58.3 | 285.1 | 78.3 | 46.1 | 44.5 |
| TEG58 | GAGEC1 | Liver cancer | 6.1 | 17.9 | 31.7 | 4.2 | 4.8 | 11.6 | 5.8 | 2014. 7 | 45.9 | 8.2 | 12.1 |
| TEG59 | RHAMM | Stomach cancer, Large bowel cancer, Liver cancer | 19.6 | 46.1 | 35.6 | 115.3 | 36.2 | 158.6 | 10.6 | 103.2 | 84.5 | 7.4 | 55.4 |
| TEG60 | PEG10 | Liver cancer, Lung cancer, Hepatoblastoma | 42.9 | 216.9 | 45.7 | 21.4 | 28.6 | 36.7 | 40.6 | 389.8 | 174.7 | 80.9 | 64.5 |
| TEG61 | PAEP | Lung cancer | 4.1 | 96.4 | 9.6 | 7.5 | 6.4 | 5.5 | 4.4 | 6.2 | 6 | 6.5 | 4.4 |
| TEG62 | MGC10981 | Lung cancer | 58.1 | 459 | 59.7 | 44.9 | 91 | 71.6 | 98.6 | 87.7 | 8.1 | 56.8 | 34 |
| TEG63 | DUSP9 | Liver cancer | 20 | 33.7 | 25.9 | 28.9 | 30.9 | 24 | 46.4 | 212.7 | 687 | 49.4 | 24.1 |
| TEG64 | EST1B | Liver cancer | 52.6 | 18.7 | 20.8 | 34.9 | 24.3 | 25.5 | 16 | 82 | 83.2 | 24.2 | 42.3 |

In particular, as for TEG1 to TEG18, an elevation in their expression in any type of cancer cells had not been found so far and it was shown in this study that their expression is elevated in a certain type of cancer. In addition, as for each gene of TEG19 to TEG60, it was now found that their expression is elevated in another type of cancer other than those reported before.

### 1.6. Identification of gene whose expression is elevated in each type of cancer tissue

The expression analysis of each gene of TEG1 to TEG64 in each type of cancer was carried out using a GeneChip^{™} HG-U133A, B (manufactured by Affymetryx) and a GeneChip^{™} HG-U133 plus 2 (manufactured by Affymetryx). More specifically, total RNA was prepared in the same manner as described above for each of the specimens from 10 cases of lung small cell lung cancer, 5 cases of lung squamous cell carcinoma, 5 cases of lung adenocarcinoma, 7 cases of large bowel cancer, 8 cases of metastatic tissue in liver from large bowel cancer, 2 cases of renal cancer and 4 cases of pancreatic cancer. Then, 5 µg of the total RNA was analyzed for mRNA expression with a GeneChip^{™} HG-U133A, B. The lung small cell cancer, large bowel cancer and Some samples from metastatic tissue in liver from large bowel cancer were analyzed only by a U-133A chip. The mean value of expression scores of all genes in each analysis was defined as 100 and the expression level of each gene was indicated as a relative value. In addition, samples from 22 cases of small cell cancer and 27 cases of pancreatic cancer were analyzed in the same manner using a GeneChip^{™} HG-U133 plus 2.

The results are shown in Tables 3 and 4. It was found that the expression of each gene of TEG1 to TEG 64 is elevated also in each type of cancer.

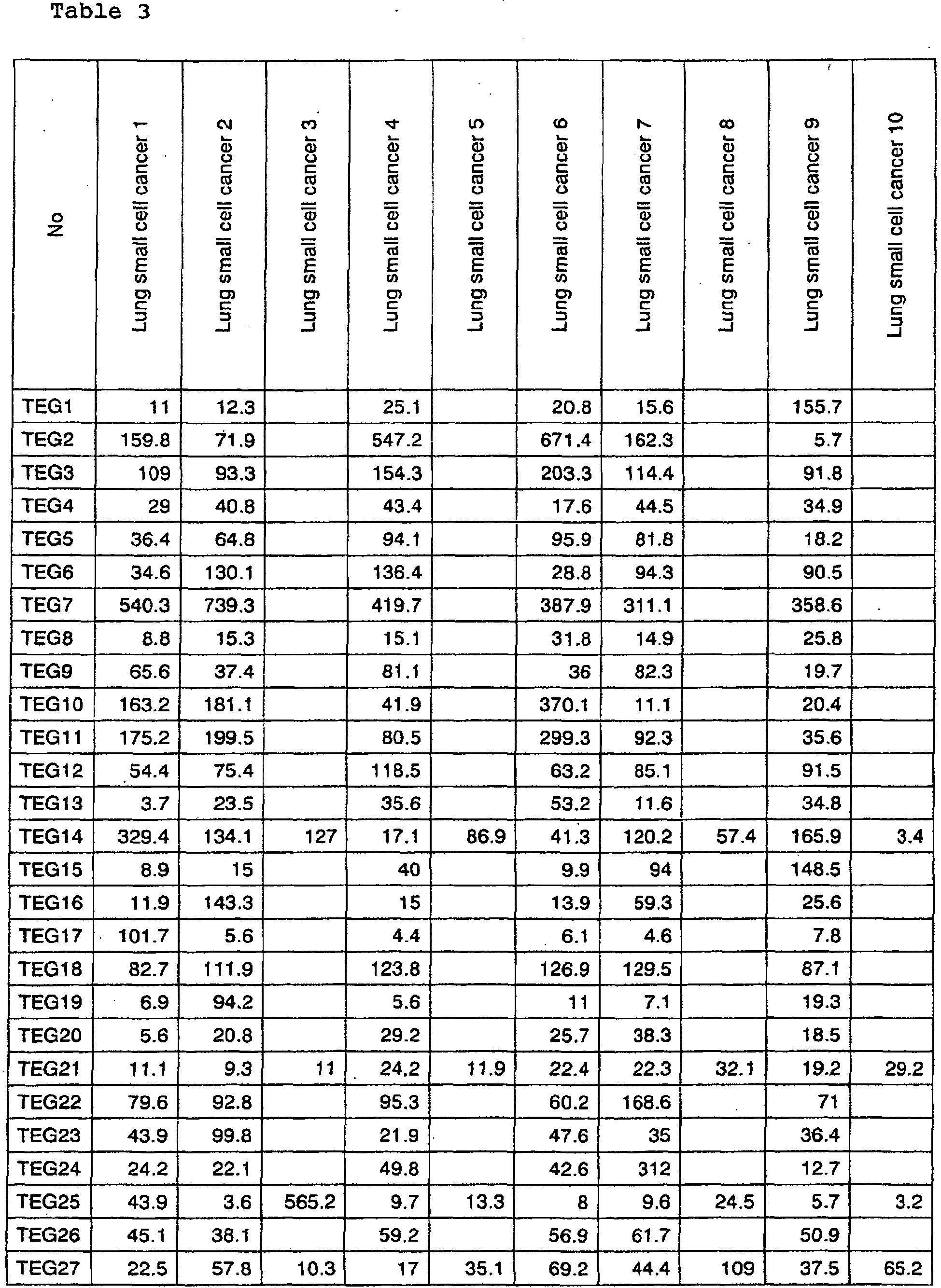

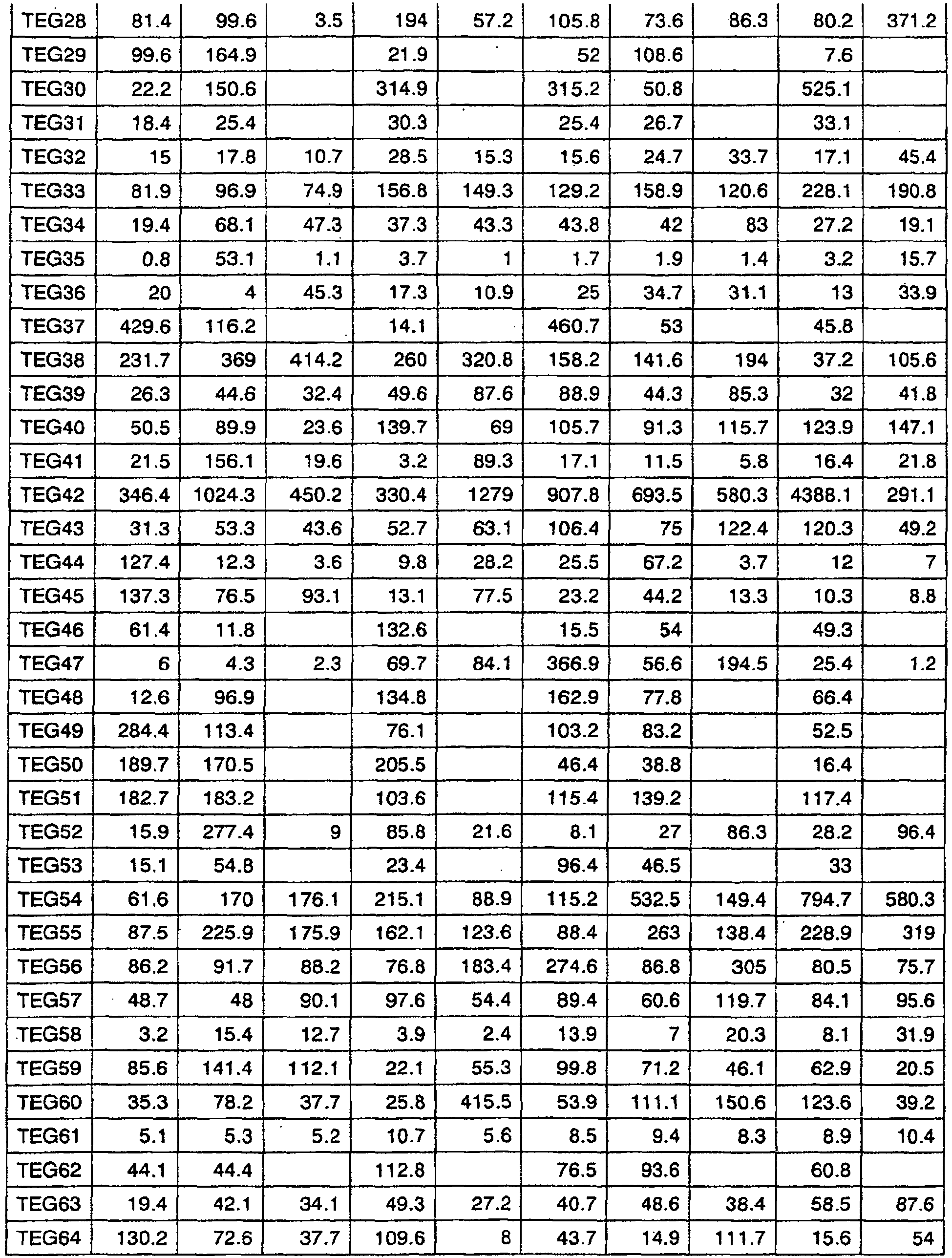

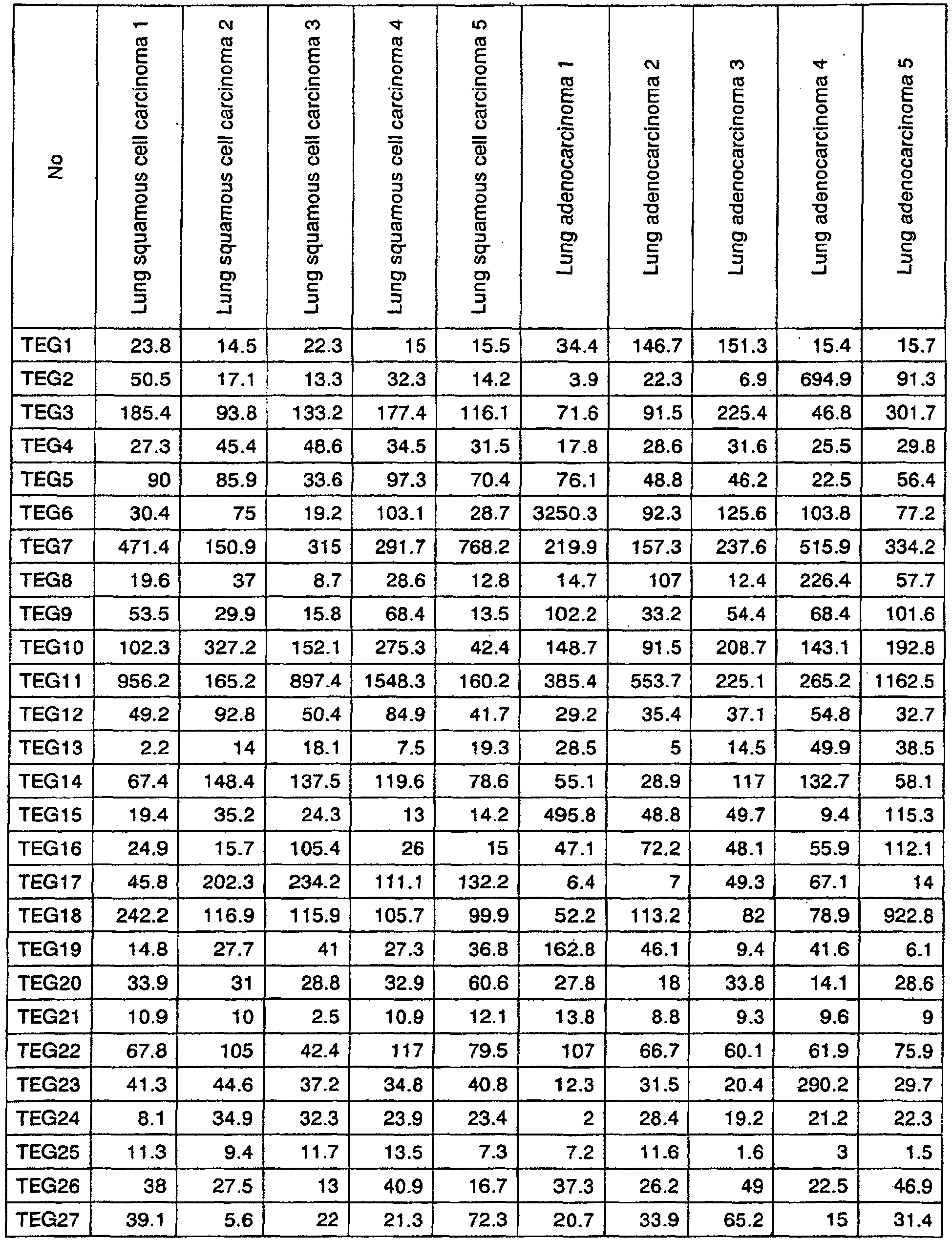

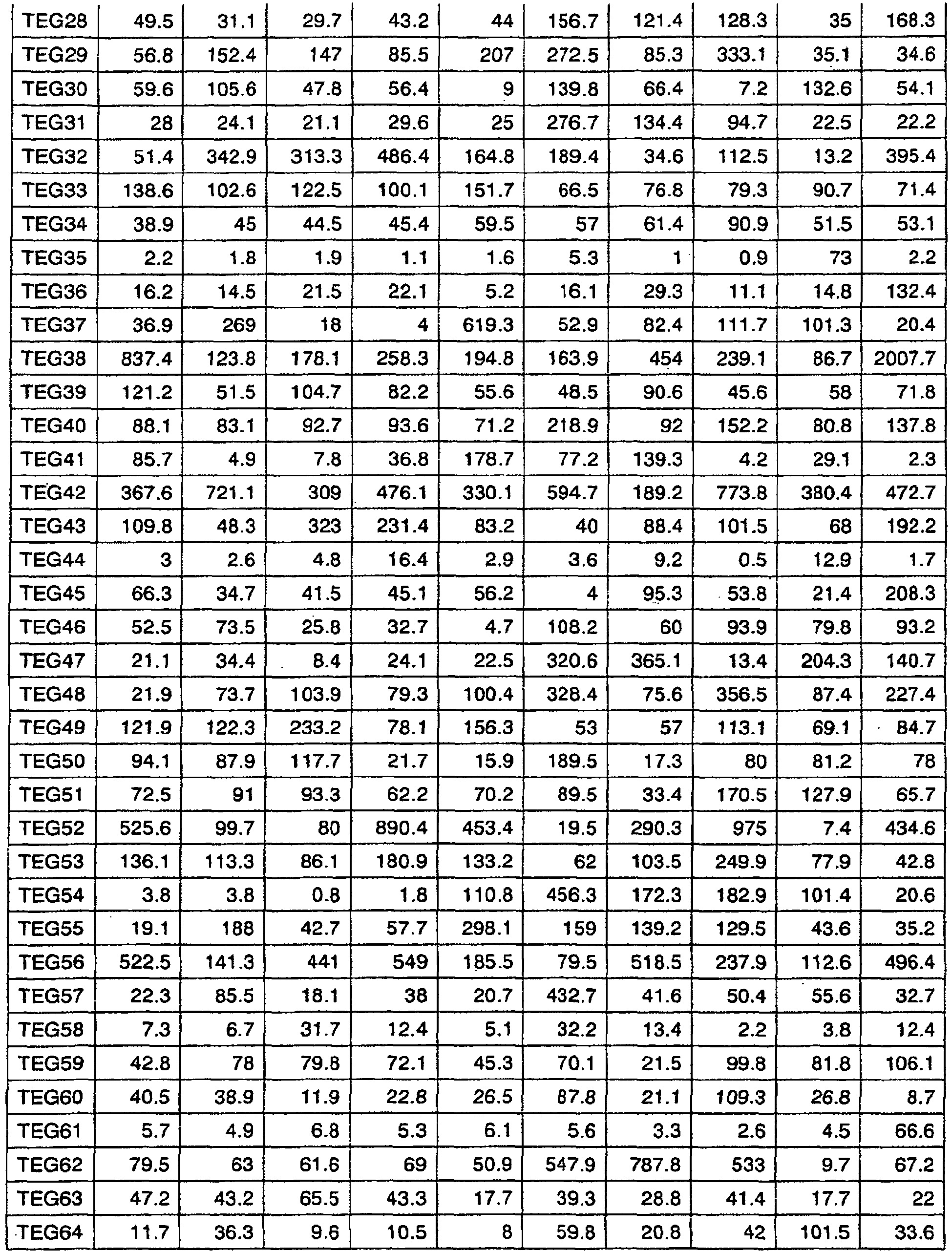

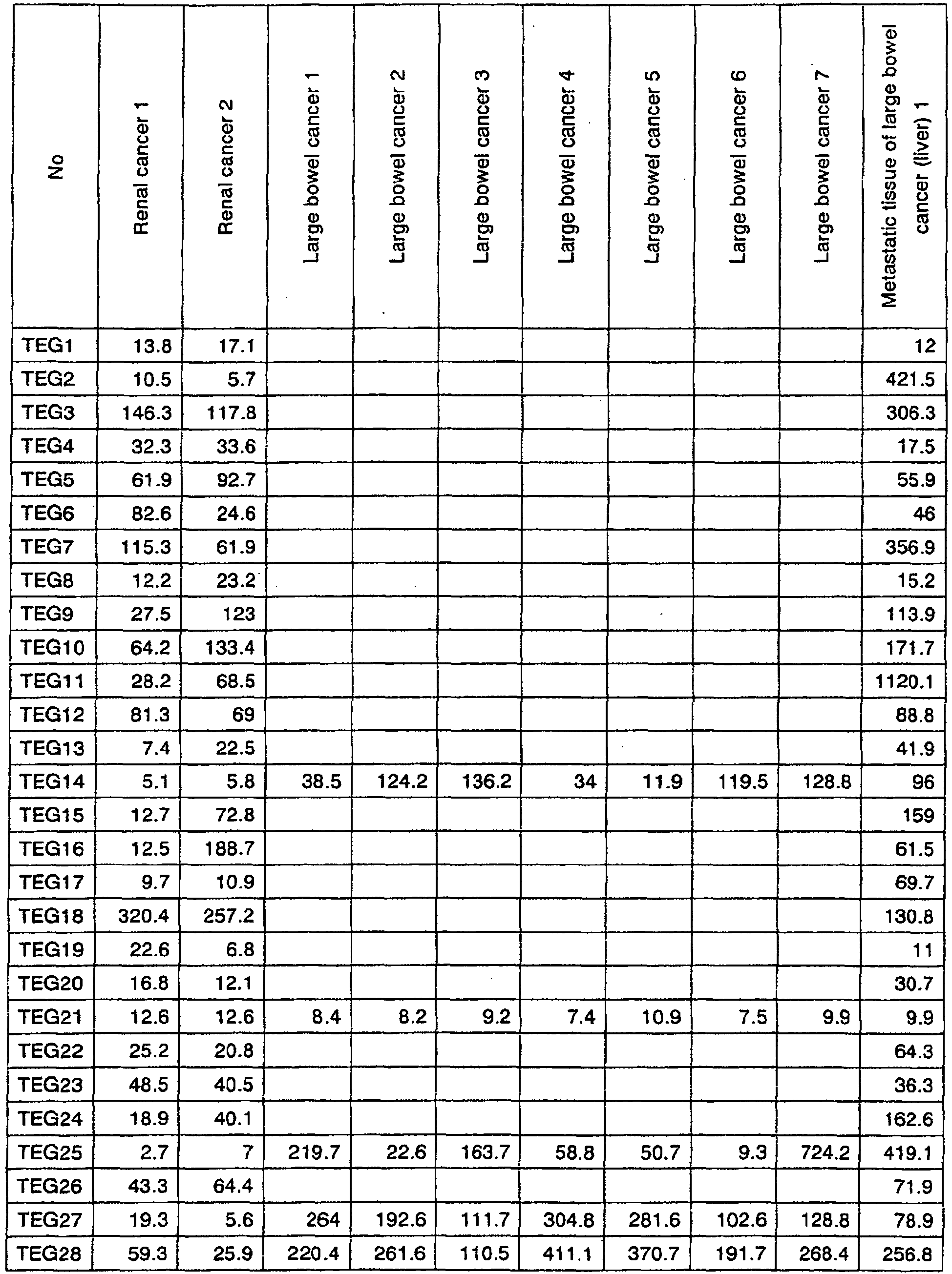

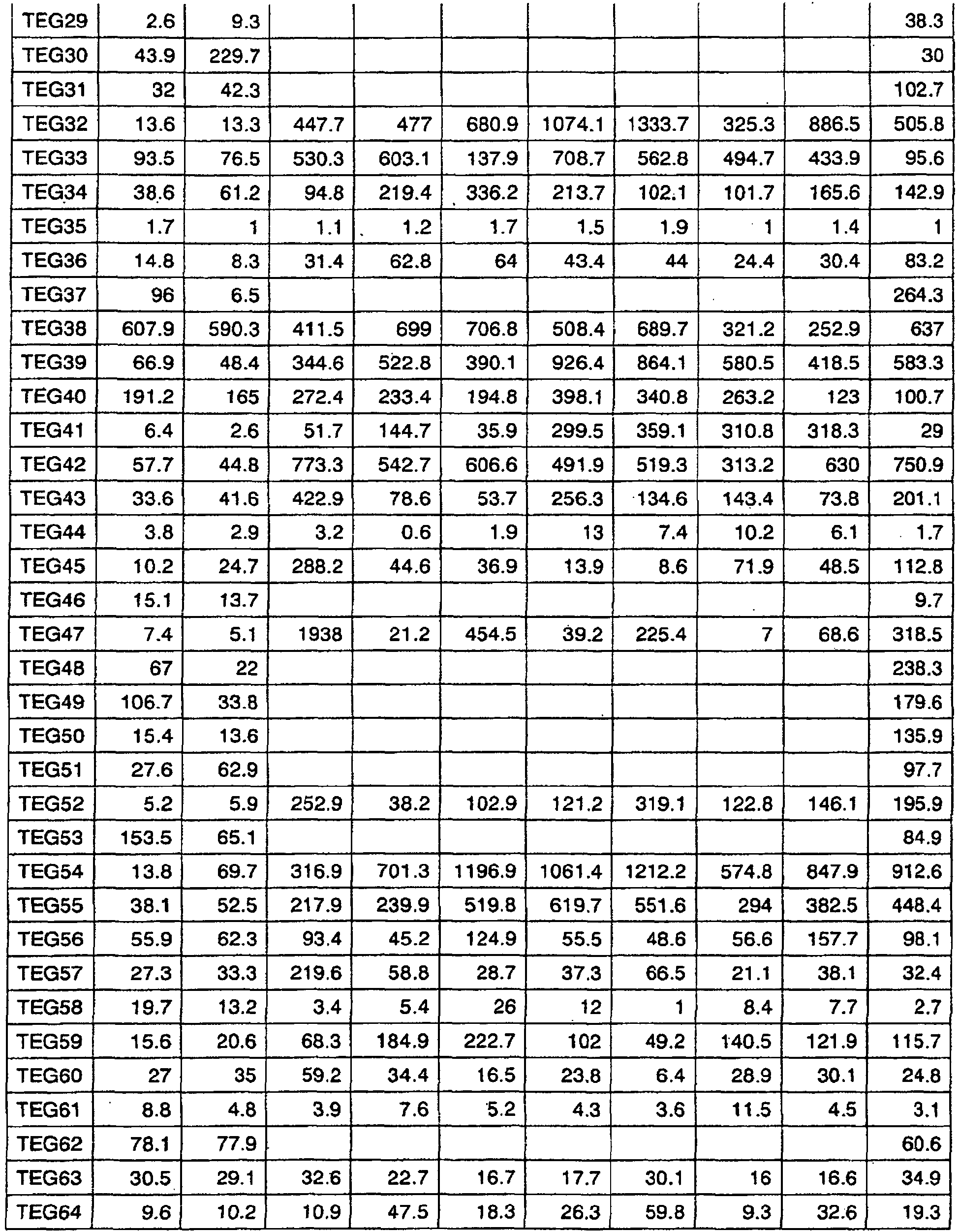

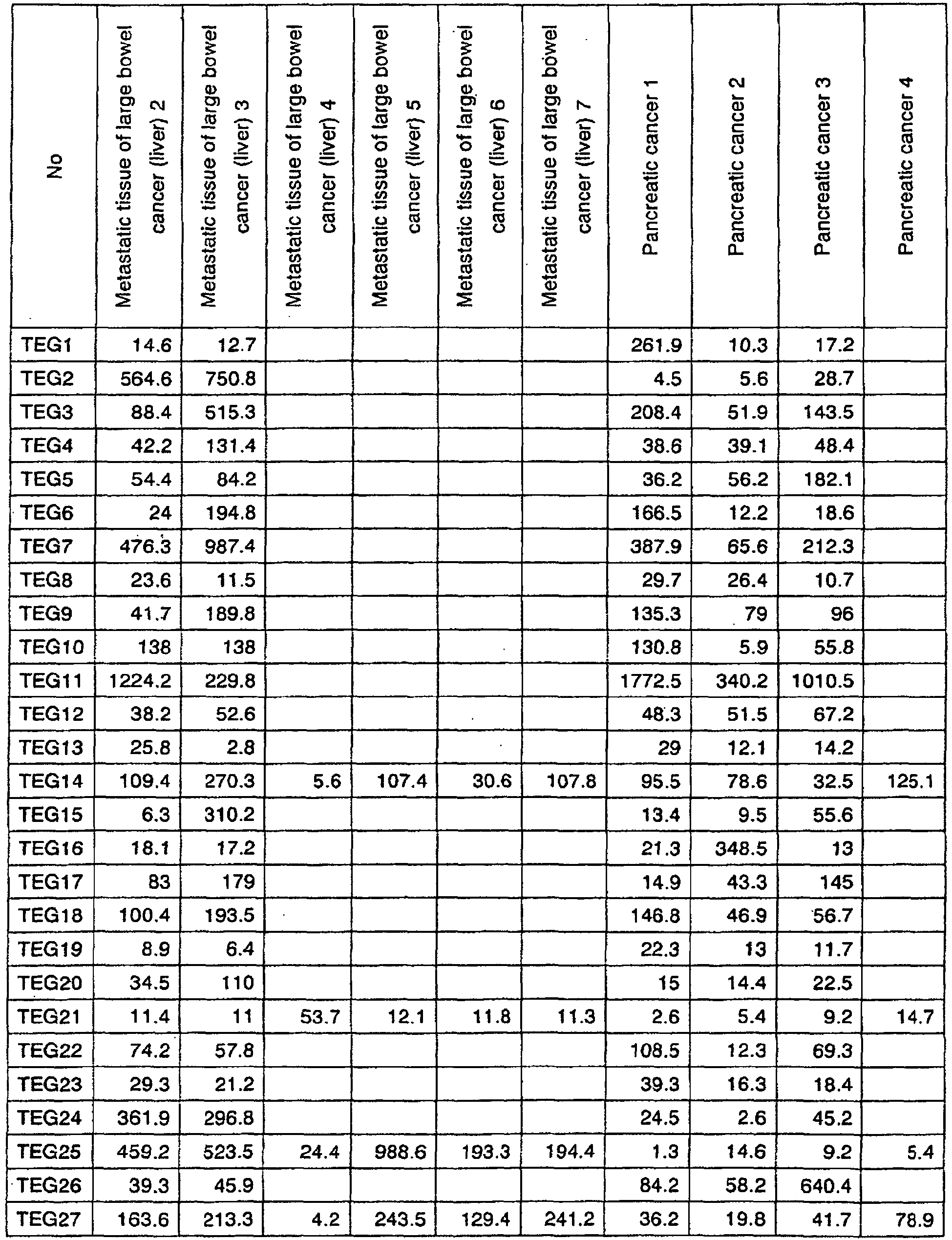

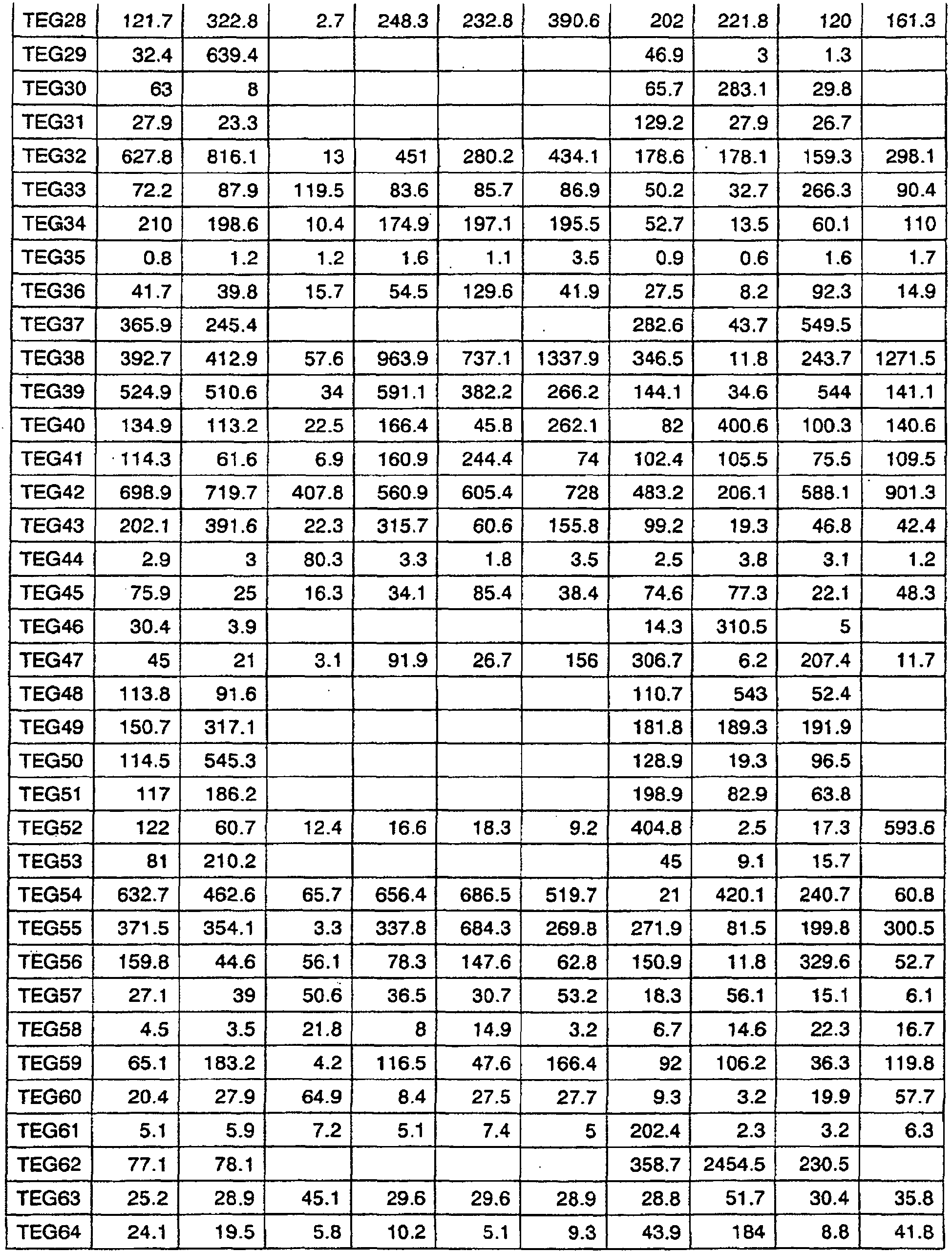

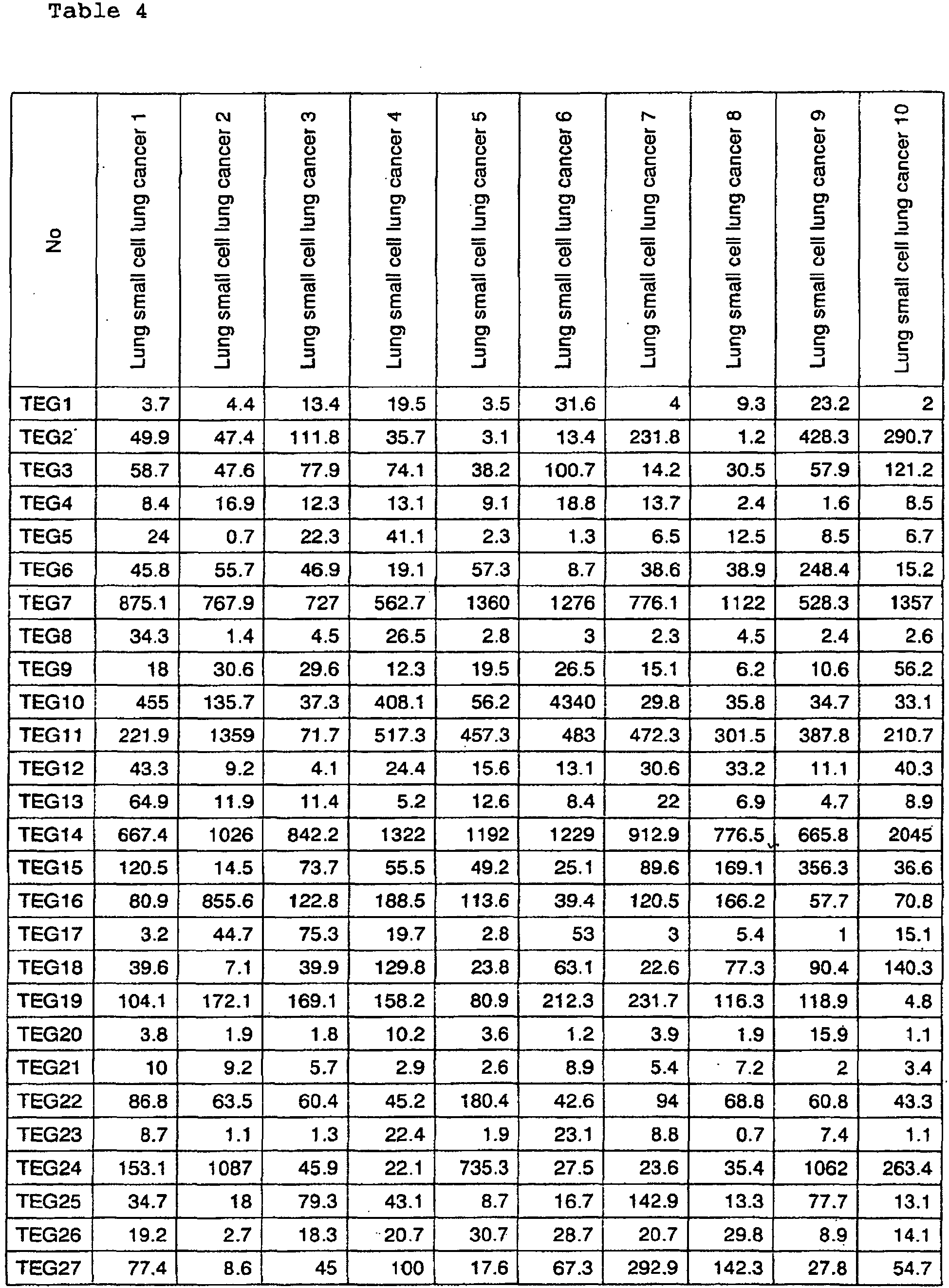

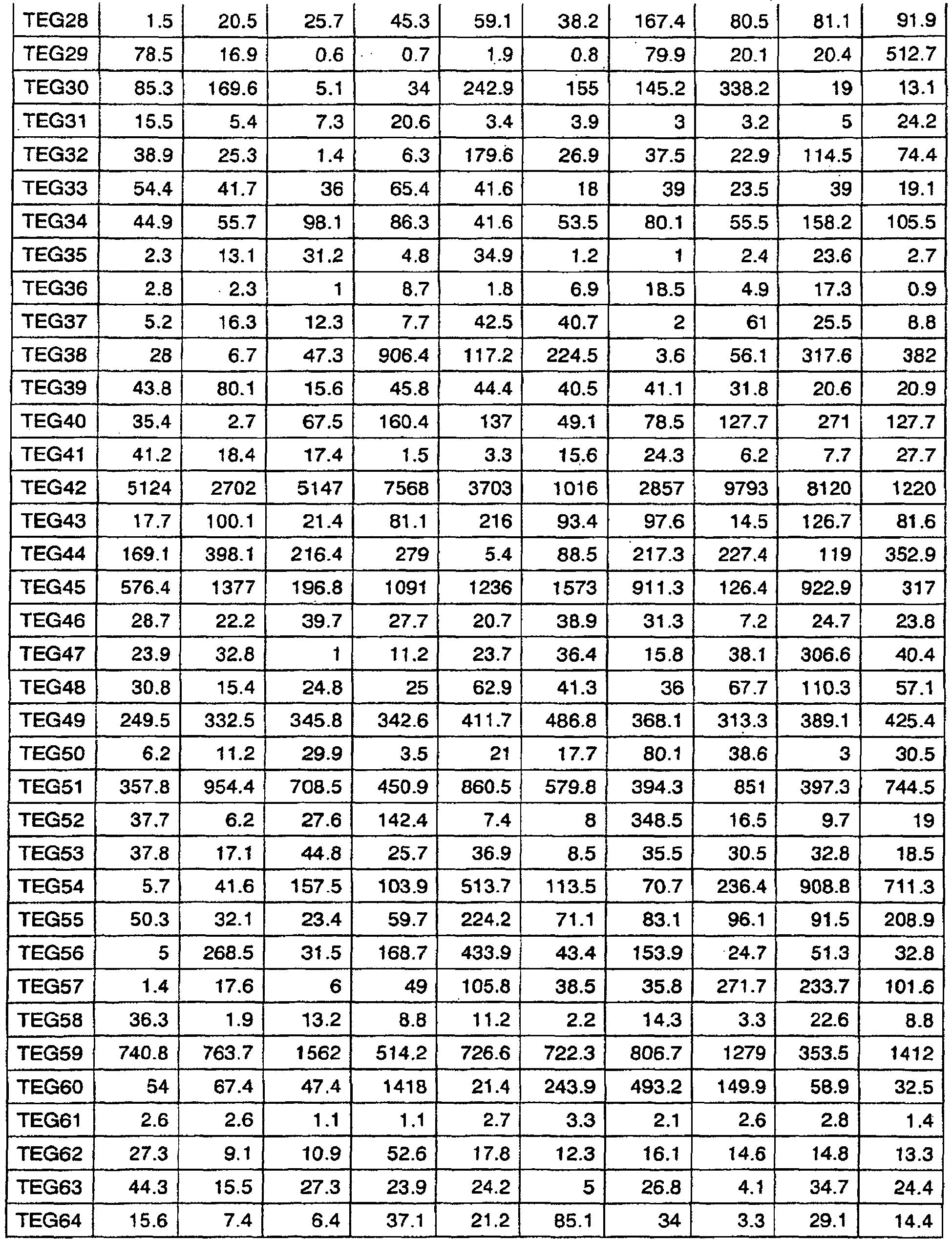

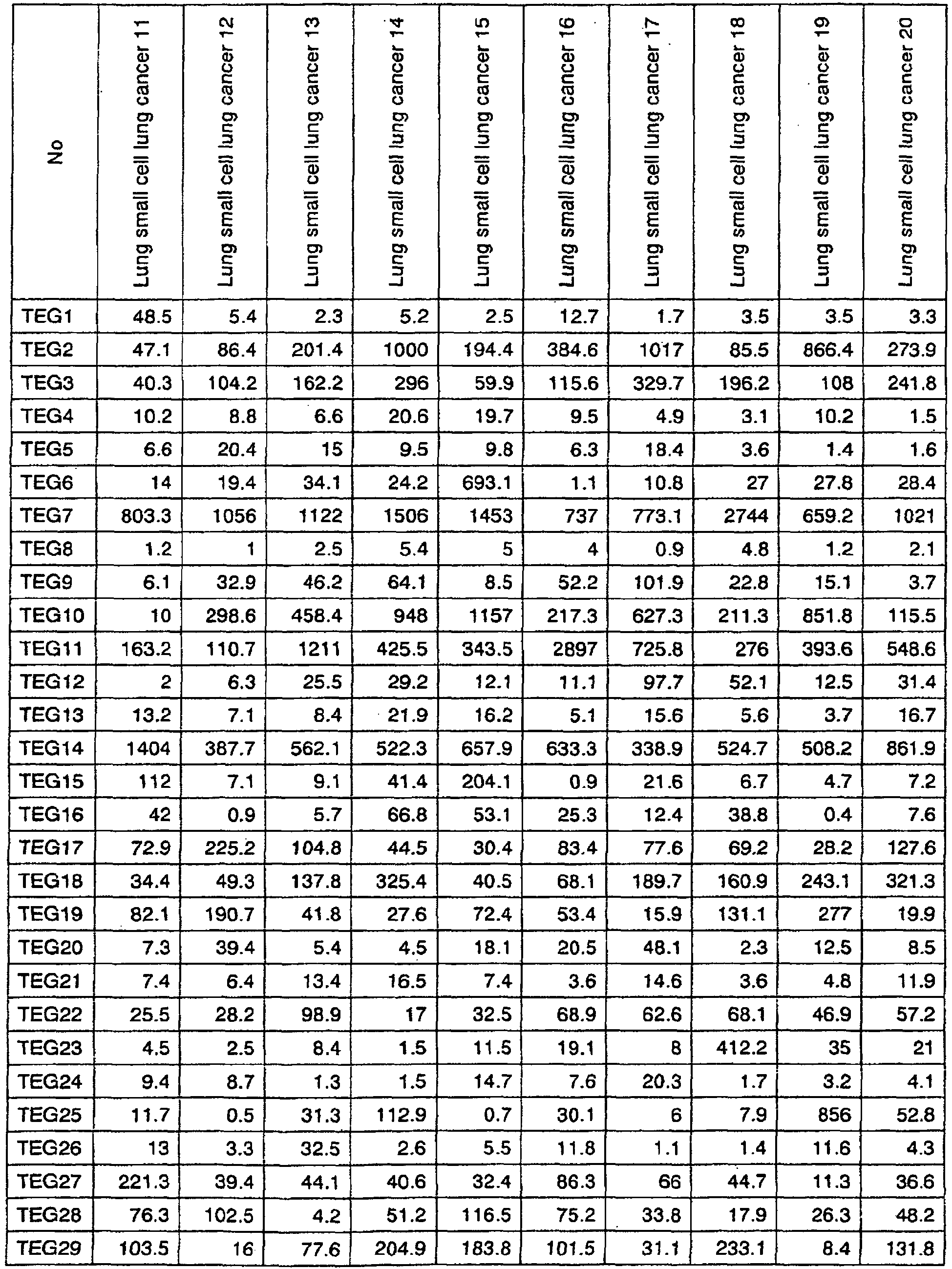

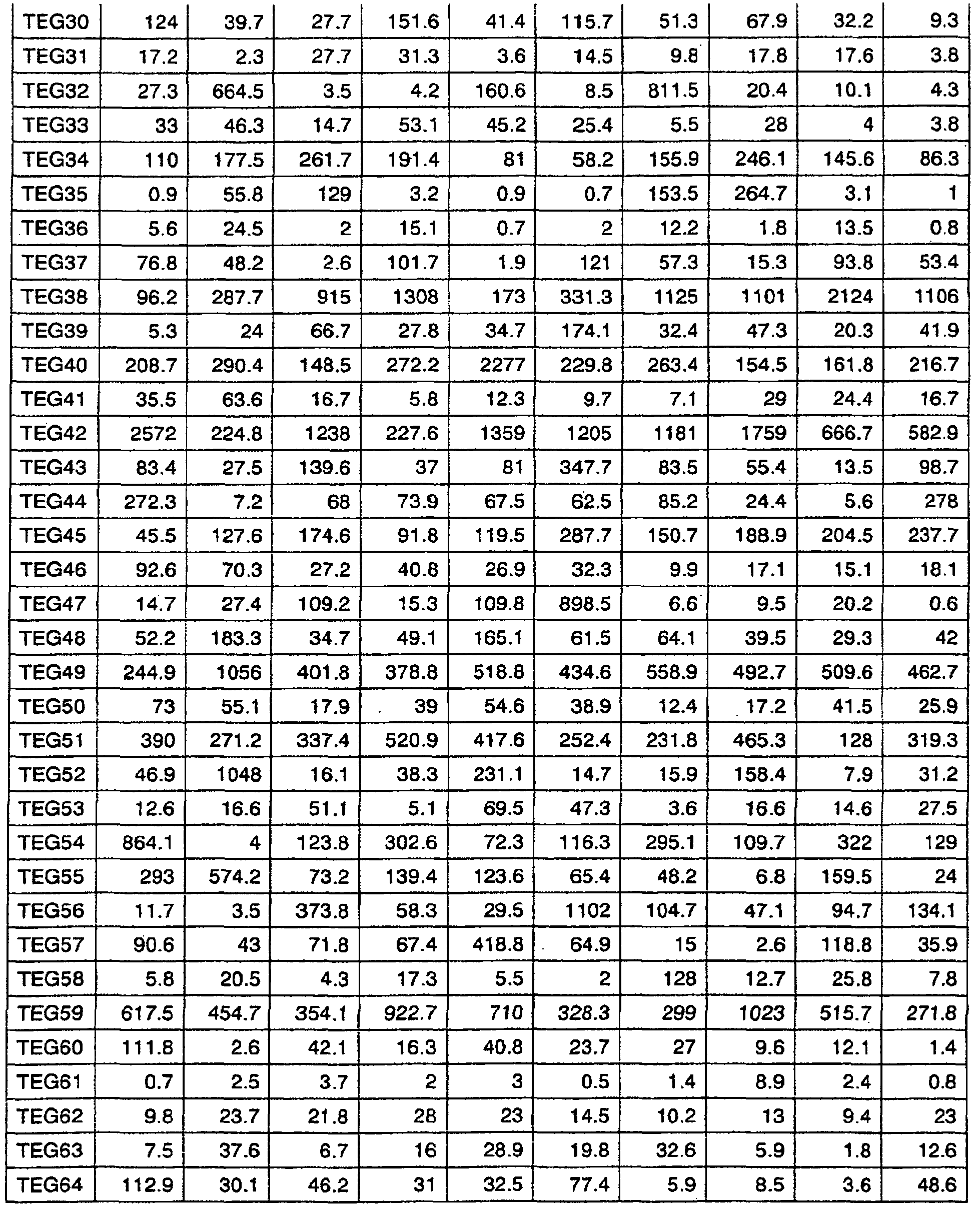

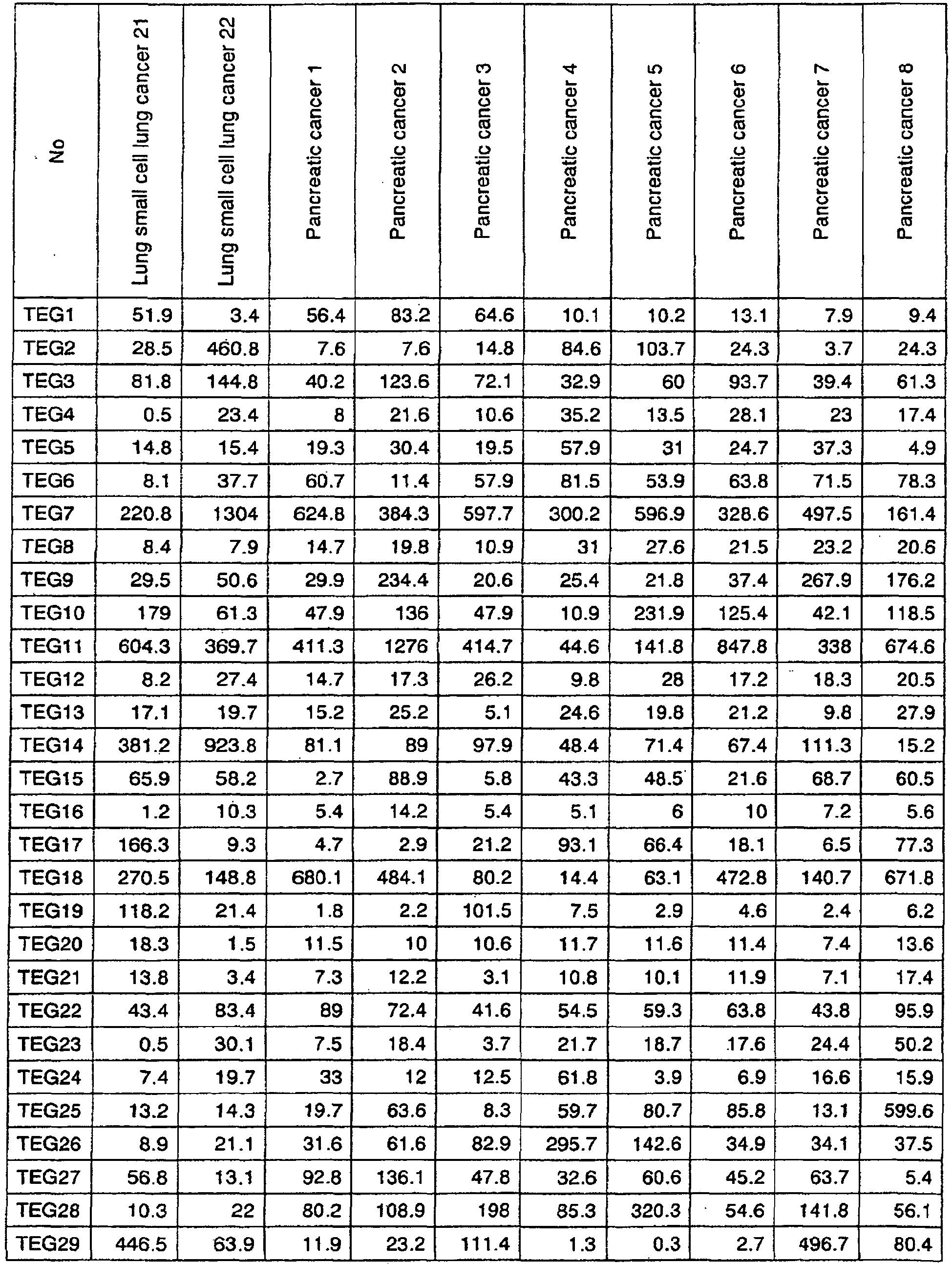

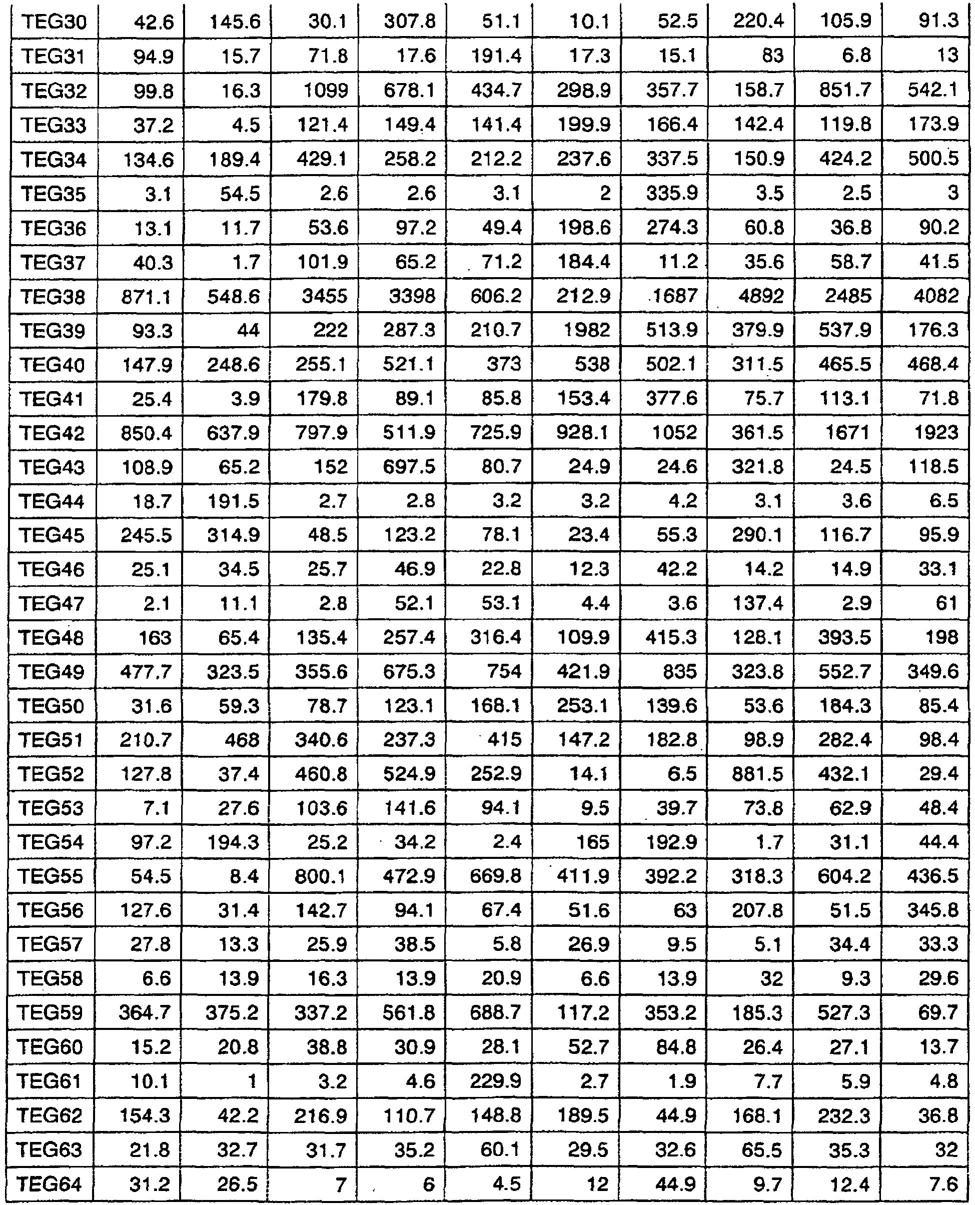

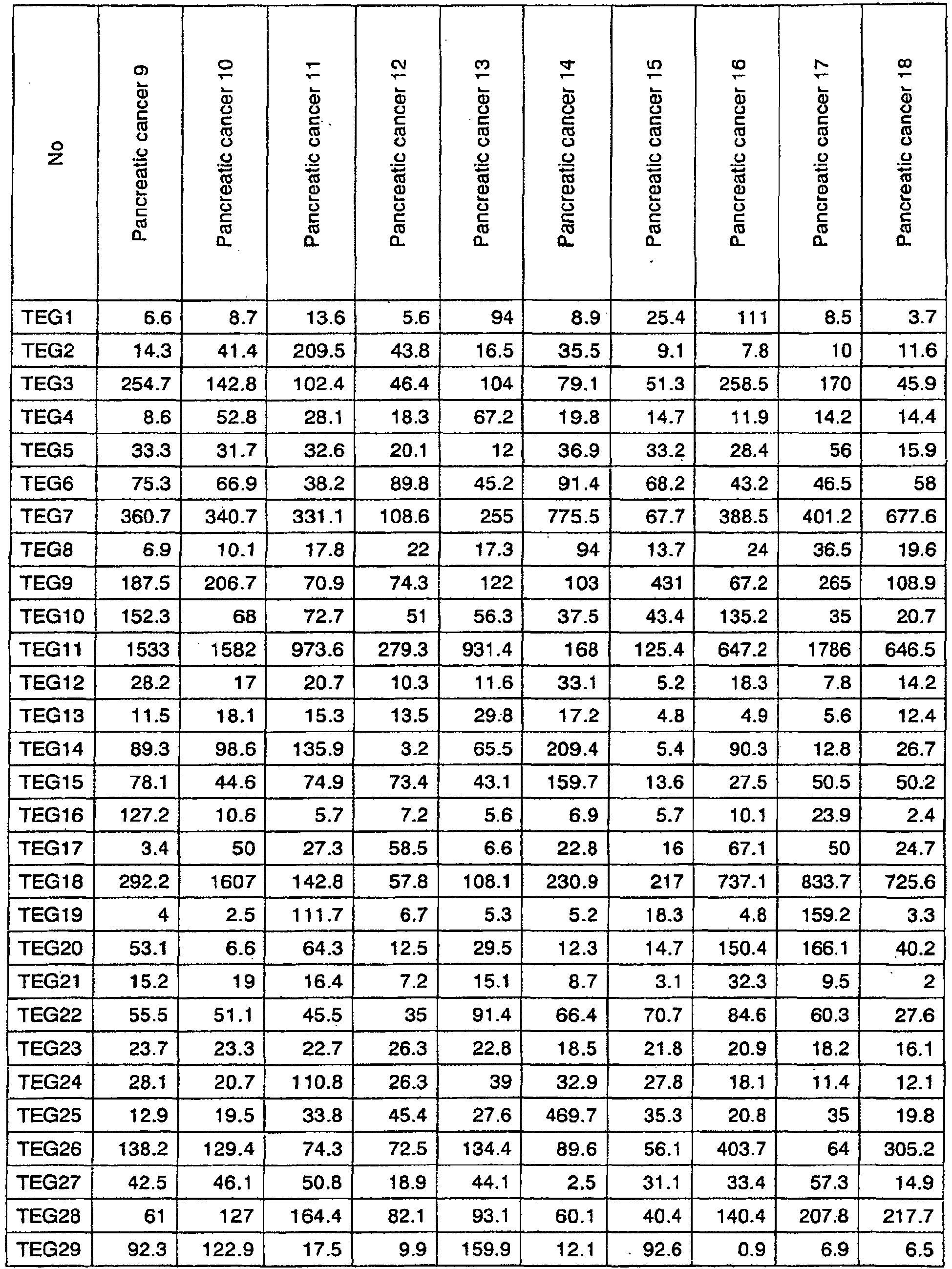

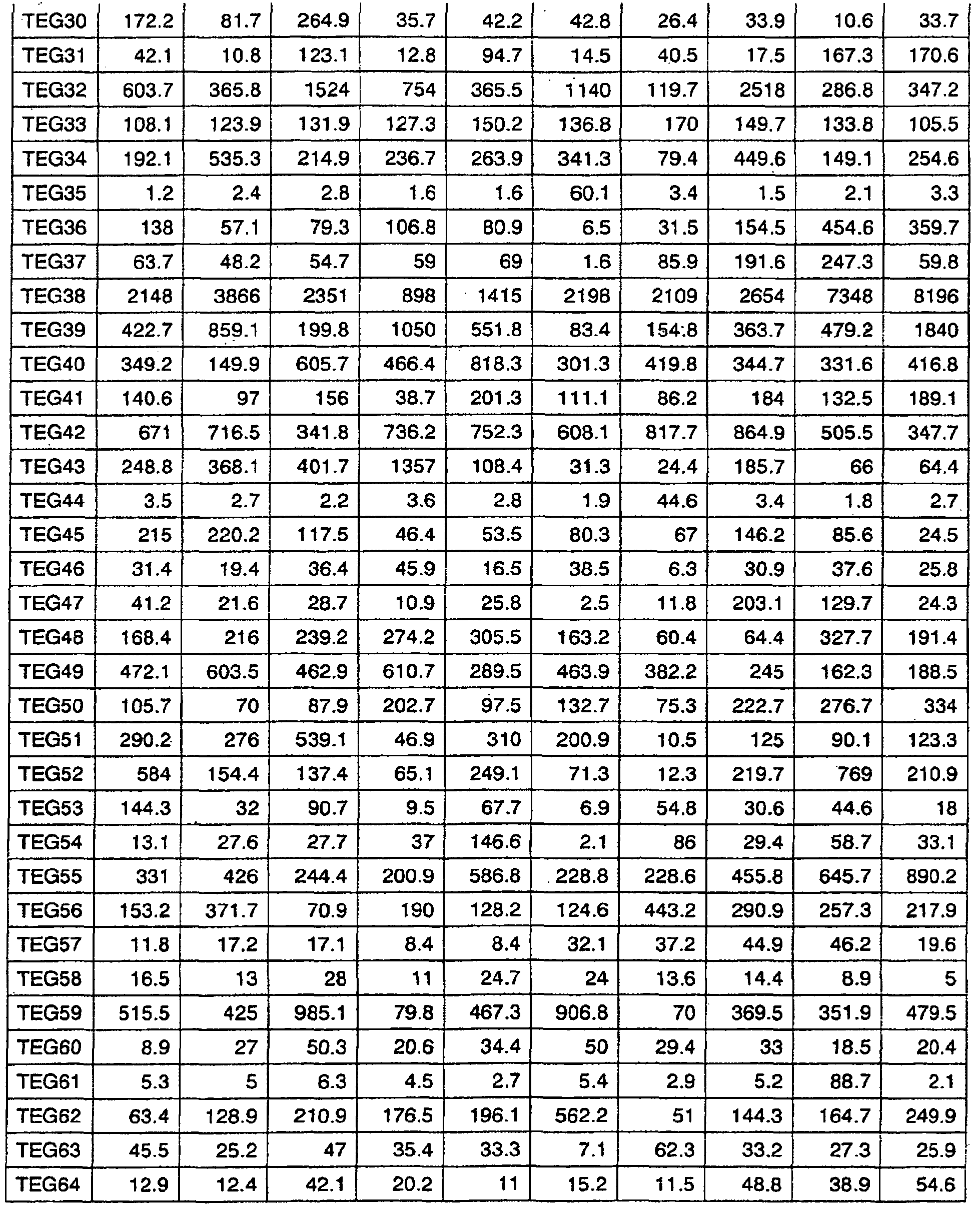

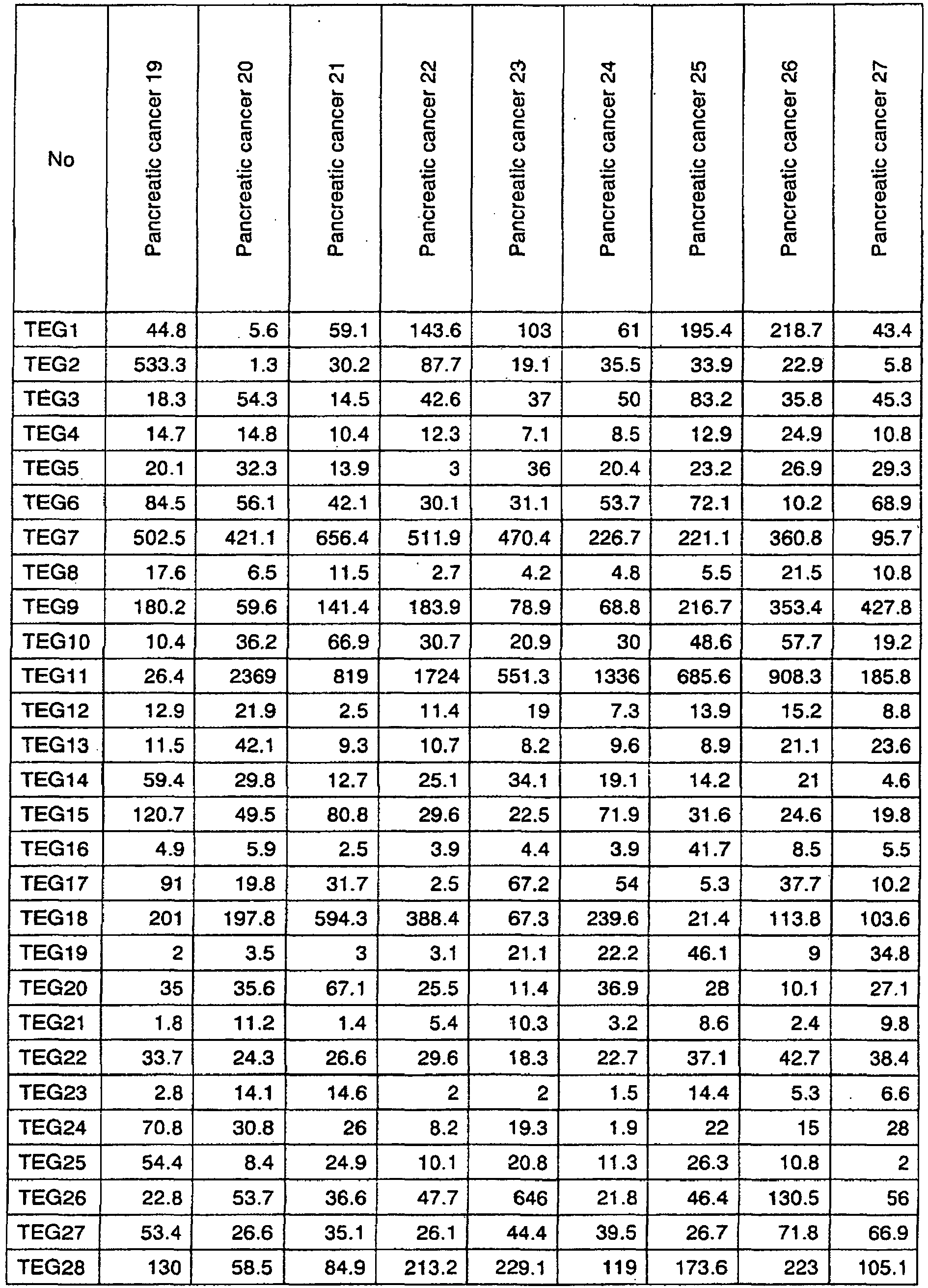

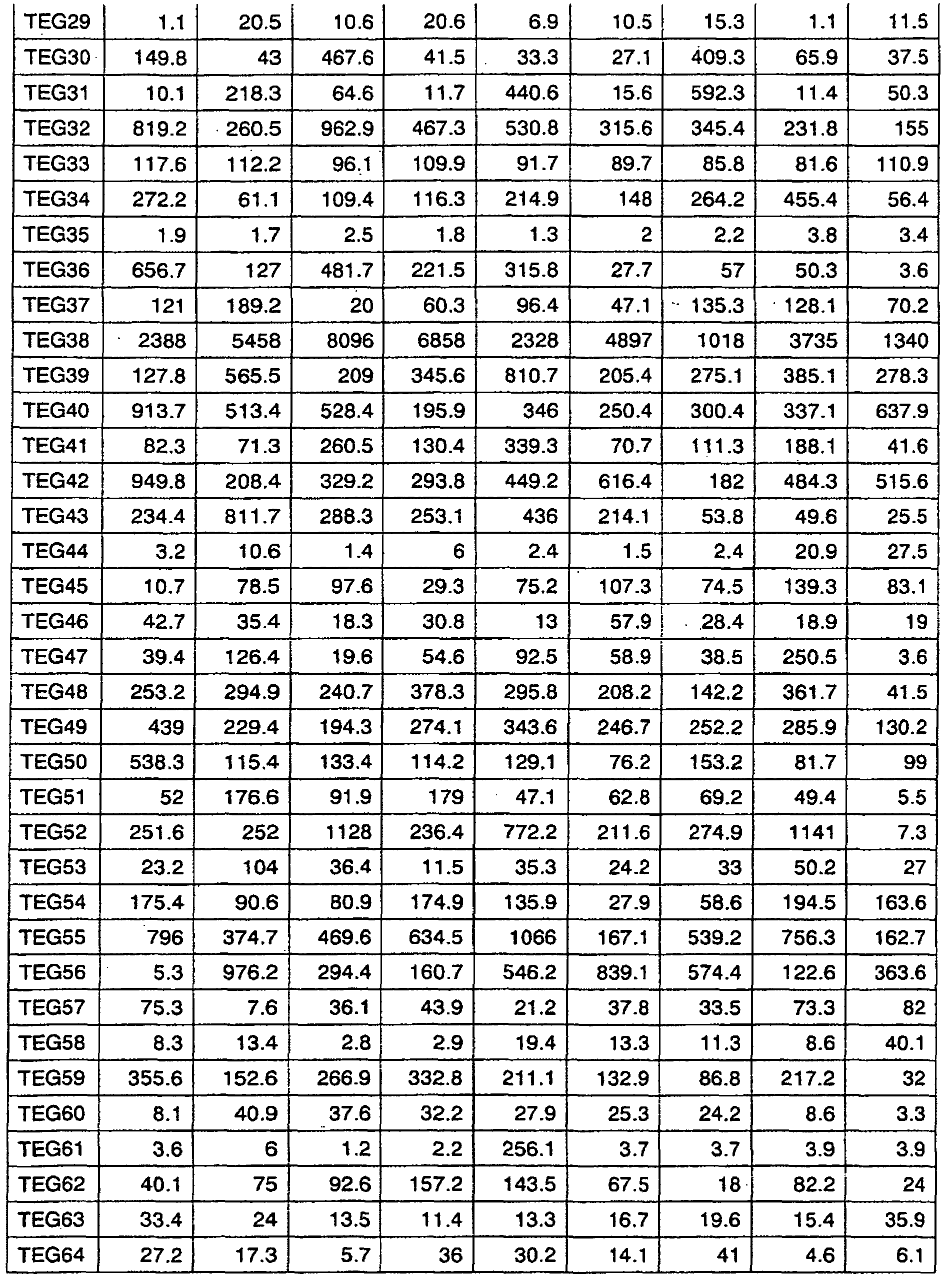

### Example 2

### Study of frequency of expression elevation using RT-PCR

In the above-mentioned Gene chip analysis, RNA prepared from each type of excised cancer tissue was analyzed as a whole. In order to verify the results from the Gene chip analysis, mRNA expression level of each gene in the respective cancer samples and the normal tissue in the non-cancerous part was analyzed by the RT-PCR method to examine the degree of expression elevation and the frequency of expression elevation.

### 2.1. Preparation of single-stranded cDNA from each type of cancer tissue

From each type of human cancer tissue and normal tissue, a single-stranded cDNA to be used as a template DNA in the PCR was prepared as follows.

Total RNA was prepared in the same manner as described above from 12 cases of lung adenocarcinoma tissue and 4 cases of normal lung tissue for lung adenocarcinoma, 10 cases of human large bowel cancer tissue and normal large bowel tissue in non-cancerous part of the same excised tissue for human large bowel cancer, 12 cases of excised human stomach cancer tissue and normal stomach tissue in non-cancerous part of the same excised tissue for human stomach cancer, and 9 cases of excised human liver cancer tissue and non-cancerous part of the same excised tissue for human liver cancer, respectively. Single-stranded cDNA was synthesized from the total RNA using a reverse transcriptase Superscript II (manufactured by GIBCO BRL). The single-stranded cDNA prepared in this manner was used as a template DNA in PCR described below.

### 2.2 Expression analysis by RT-PCR.

Subsequently, the expression level of mRNA corresponding to the genes shown in Table 2 was analyzed by the RT-PCR method. More specifically, 25 µL of a PCR solution was prepared containing 500 mM KCl, 100 mM Tris-HCl (pH 8.3), 20 mM MgCl₂, 0.1% gelatin, 1.25 mM of each dNTPs (dATP, DCTP, DGTP, dTTP). 1 µL of single-stranded cDNA, a set of sense and antisense primers that are specific for each gene in an amount of 5 pmole each, 0.75 µL of SYBR Green I (1000-fold diluted solution, manufactured by TAKARA) and 0.25 µL of recombinant Taq polymerase Mix (FG Pluthero, Rapid purification of high-activity Taq DNA polymerase, Nucl. Acids. Res. 1993 21: 4850-4851). The reaction consisted of initial denaturation at 94°C for 3 minutes, and 30 cycles of 94°C for 15 seconds, 57°C for 15 seconds and 72°C for 30 seconds. RT-PCR primers shown in Table 5 were designed and used in the analysis.

In addition, the expression level of human β-actin gene in each RNA was also analyzed in the same manner as described above using a sense primer (SEQ ID NO: 252: AGAAGGAGATCACTGCCCTGGCACC) and an antisense primer (SEQ ID NO: 253: CCTGCTTGCTGATCCACATCTGCTG) that are specific for human β-actin.

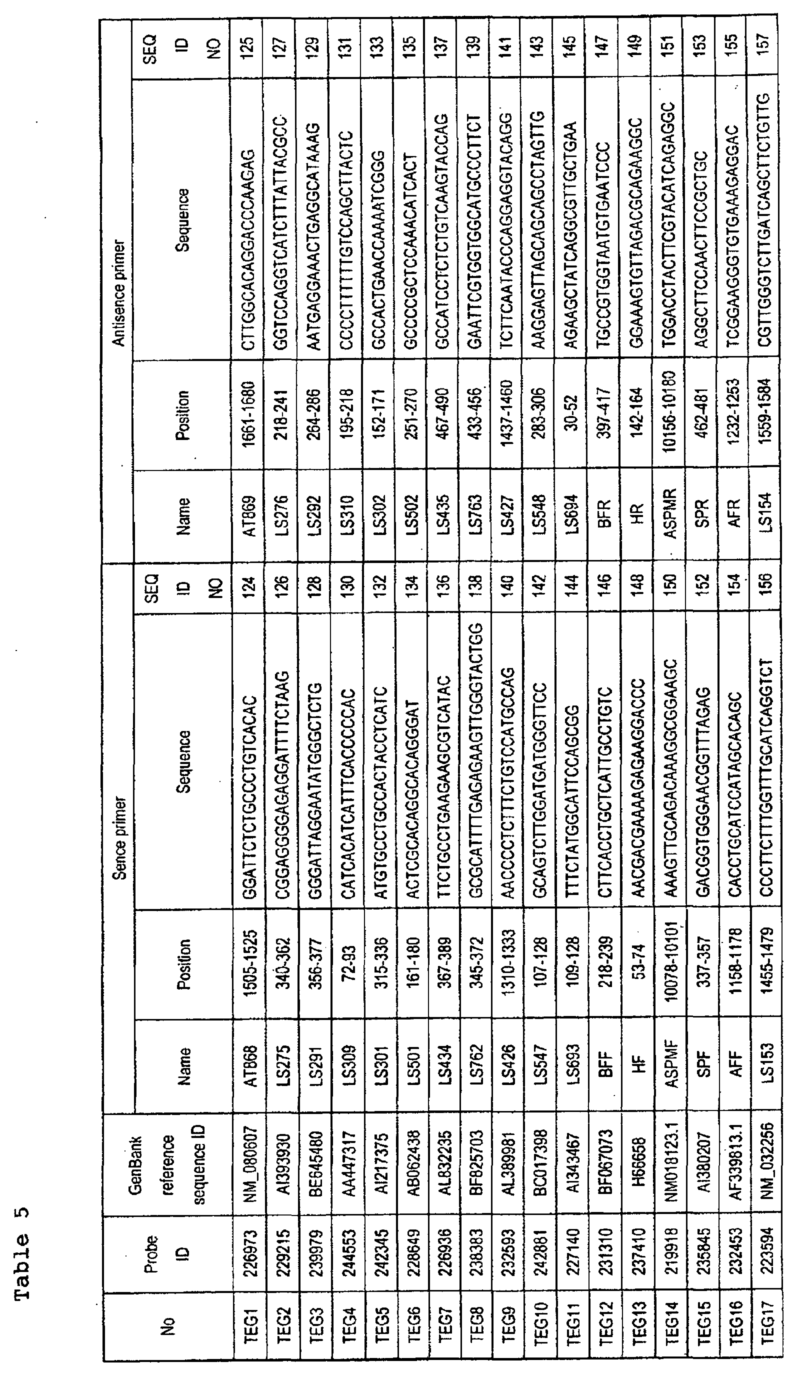

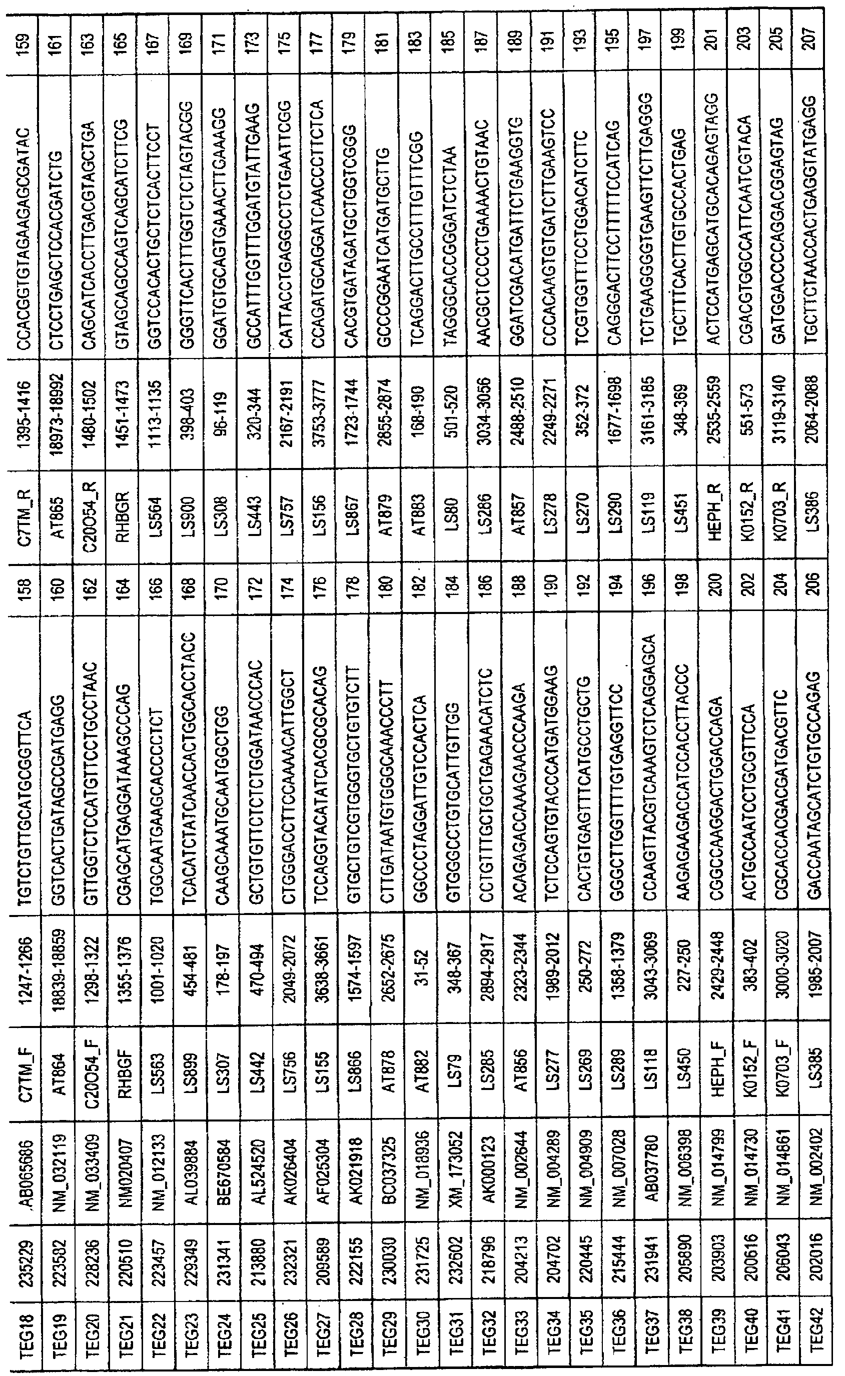

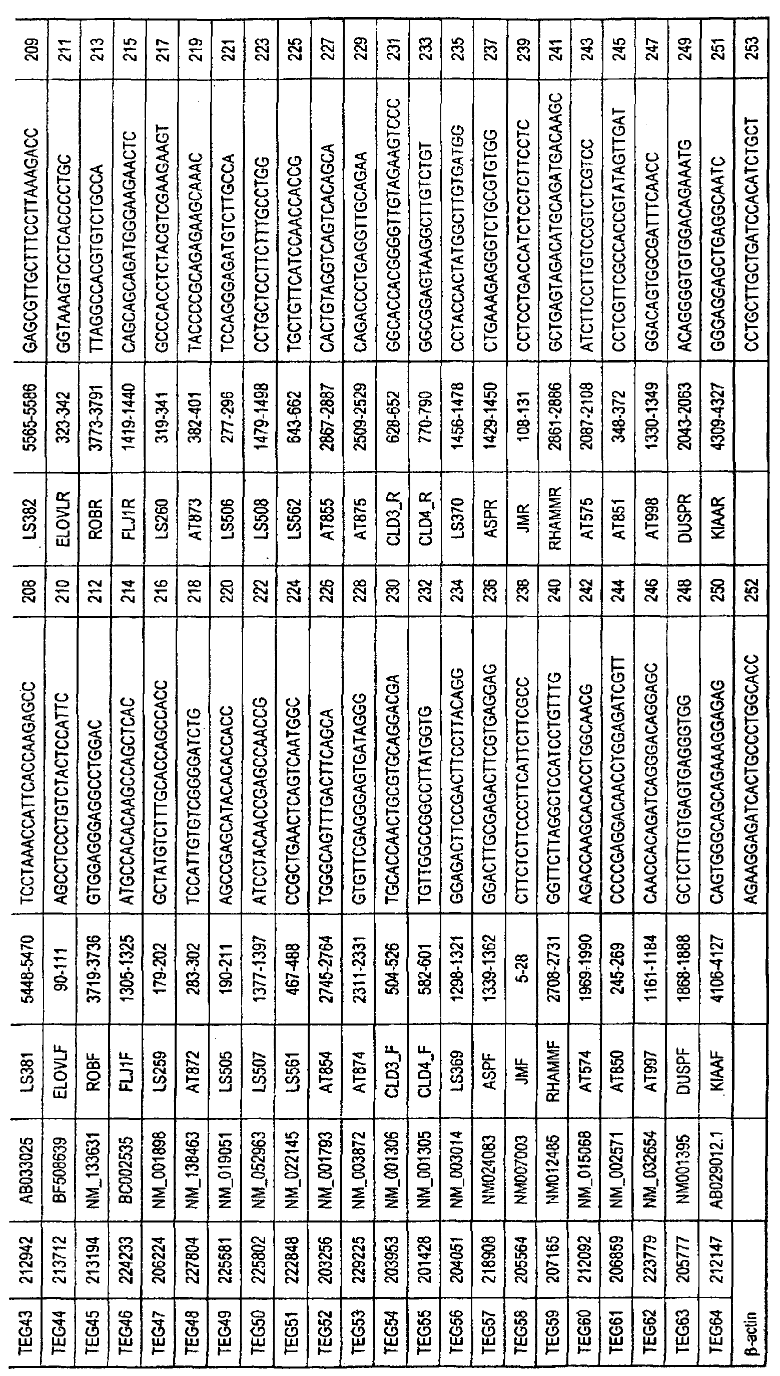

The product amplified by the PCR method was subjected to electrophoresis on 1.0% agarose gel and stained with ethidium bromide to observe the bands. The mRNA level was determined by iCyclerQ real time PCR analysis system (BIO-RAD).

### Expression analysis of TEG1

Gene expression was compared by a quantitative RT-PCR method using RNA prepared from 12 cases of lung adenocarcinoma tissue and 4 cases of normal lung tissue.

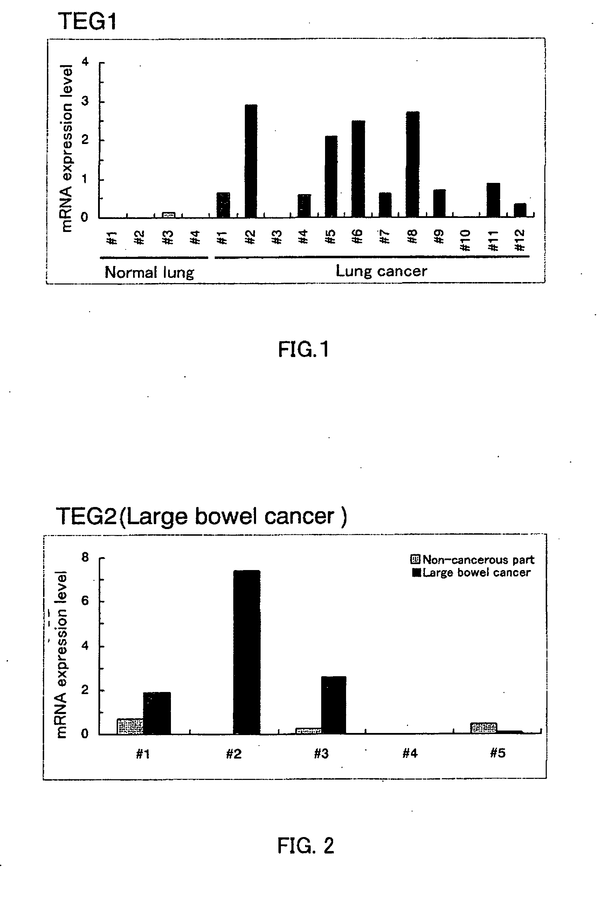

The PCR result showed that, while the expression of mRNA of TEG1 gene was not observed in the normal lung tissue, overexpression of TEG1 gene was observed clearly in 10 cases out of the analyzed 12 cases of lung adenocarcinoma tissue (Fig. 1).

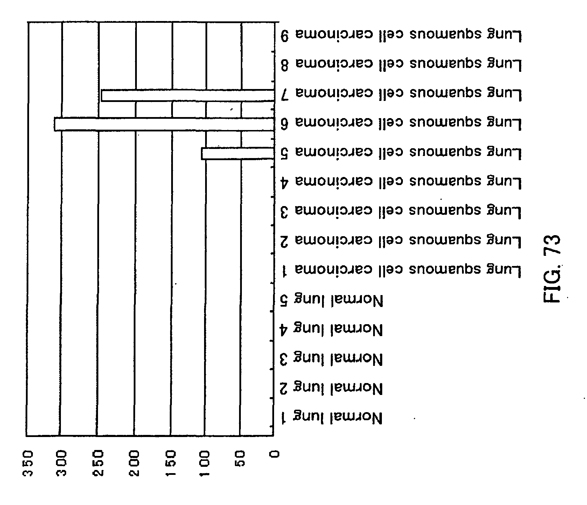

A quantitative PCR analysis was carried out for 5 cases of normal lung and 9 cases of lung squamous cell carcinoma in the same manner. The PCR result showed that, while the expression of mRNA of TEG1 gene was not observed in the normal lung tissue, an elevation in the expression of TEG1 gene was observed in 3 cases out of the analyzed 9 cases of lung squamous cell carcinoma tissue (Fig. 73).

### Expression analysis of TEG2

Gene expression was compared by an RT-PCR method using RNA prepared from 5 cases of large bowel cancer tissue and normal large bowel tissue in non-cancerous part of the same samples, and RNA prepared from 11 cases of stomach cancer tissue and normal stomach tissue in non-cancerous part of the same samples.

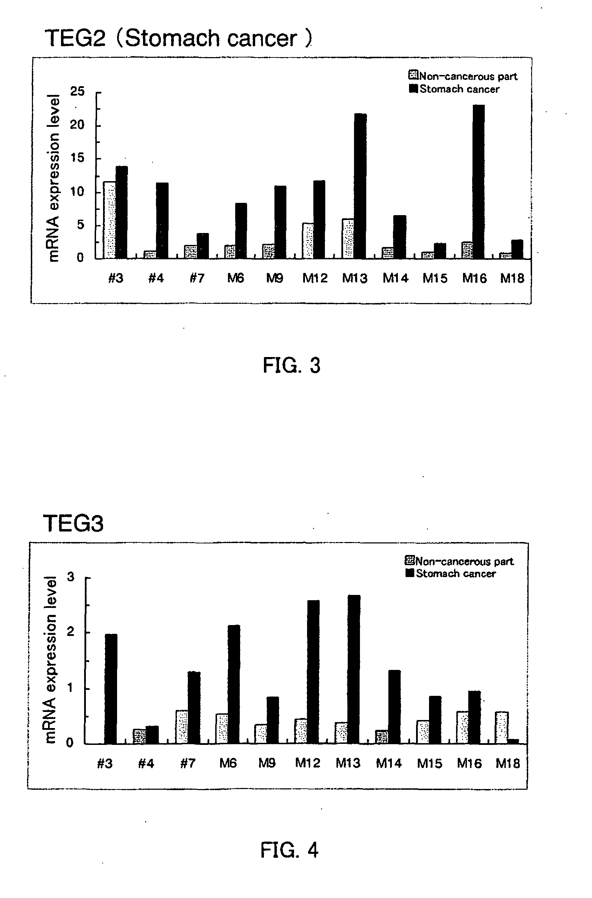

The PCR result showed that, an elevation in the expression of mRNA of TEG2 gene was observed in the cancerous part in 3 cases out of 5 cases of the analyzed large bowel cancer and in all the 11 cases of stomach cancer (Figs. 2 and 3).

### Expression analysis of TEG3

Gene expression was compared by an RT-PCR method using RNA prepared from 11 cases of stomach cancer tissue and normal stomach tissue in non-cancerous part of the same samples.

The PCR result showed that, an elevation in the expression of mRNA of TEG3 gene was observed clearly in the cancerous part in 9 cases out of 11 cases of the analyzed stomach cancer (Fig. 4).

### Expression analysis of TEG4

Gene expression was compared by an RT-PCR method using RNA prepared from 11 cases of stomach cancer tissue and normal stomach tissue in non-cancerous part of the same samples.

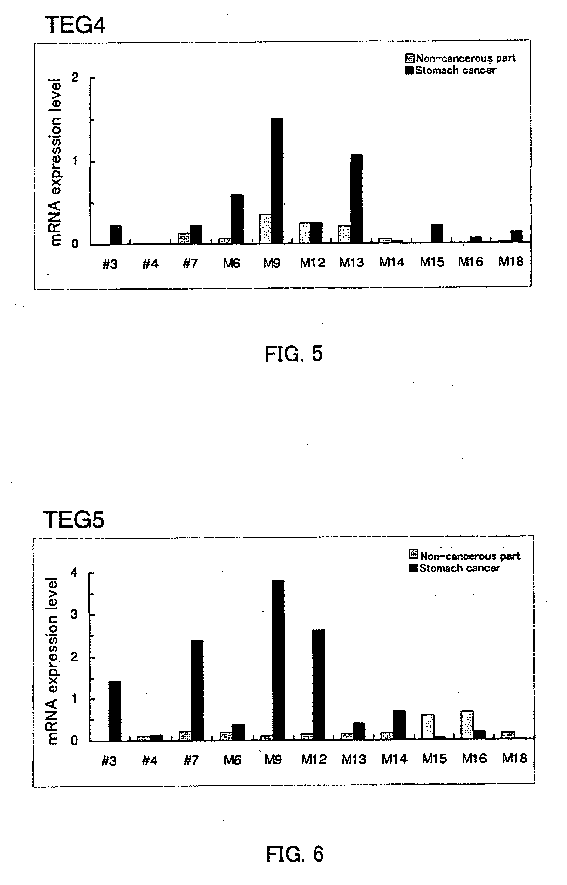

The PCR result showed that, an elevation in the expression of mRNA of TEG4 gene was observed clearly in the cancerous part in 7 cases out of 11 cases of the analyzed stomach cancer (Fig. 5).

### Expression analysis of TEG5

Gene expression was compared by an RT-PCR method using RNA prepared from 11 cases of stomach cancer tissue and normal stomach tissue in non-cancerous part of the same samples.

The PCR result showed that, an elevation in the expression of mRNA of TEG5 gene was observed clearly in the cancerous part in 7 cases out of 11 cases of the analyzed stomach cancer (Fig. 6).

### Expression analysis of TEG6

Gene expression was compared by an RT-PCR method using RNA prepared from 9 cases of large bowel cancer tissue and normal large bowel tissue in non-cancerous part of the same samples, and RNA prepared from 11 cases of stomach cancer tissue and normal stomach tissue in non-cancerous part of the same samples.

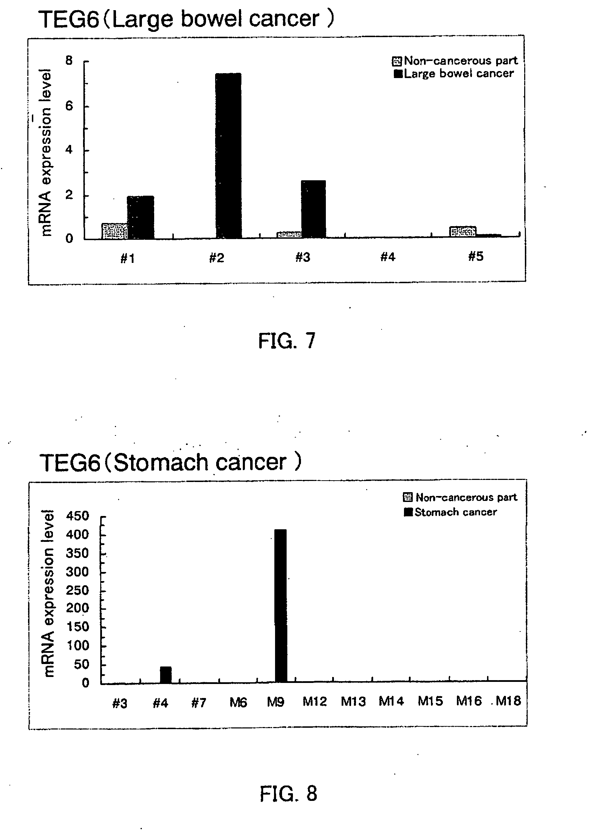

The PCR result showed that, an elevation in the expression of mRNA of TEG6 gene was observed clearly in the cancerous part in 3 cases out of 9 cases of the analyzed large bowel cancer. As for the stomach cancer, while no expression of mRNA was observed in all the analyzed normal stomach, very strong expression of mRNA was observed in 2 cases (Figs. 7 and 8).

### Expression analysis of TEG7

Gene expression was compared by an RT-PCR method using RNA prepared from 11 cases of stomach cancer tissue and normal stomach tissue in non-cancerous part of the same samples.

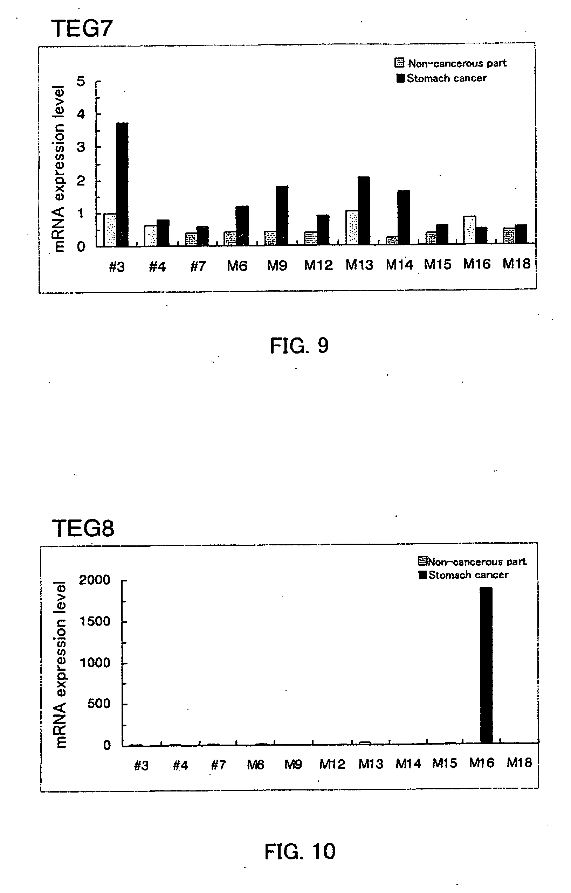

The PCR result showed that, an elevation in the expression of mRNA of TEG7 gene was observed clearly in the cancerous part in 6 cases out of 11 cases of the analyzed stomach cancer (Fig. 9).

### Expression analysis of TEG8

Gene expression was compared by an RT-PCR method using RNA prepared from 11 cases of stomach cancer tissue and normal stomach tissue in non-cancerous part of the same samples.

The PCR result showed that, while very little expression of mRNA of TEG8 gene was observed in the analyzed normal stomach, significant expression of mRNA was observed in 1 case out of 11 cases of stomach cancer (Fig. 10).

### Expression analysis of TEG9

Gene expression was compared by an RT-PCR method using RNA prepared from 11 cases of stomach cancer tissue and normal stomach tissue in non-cancerous part of the same samples.

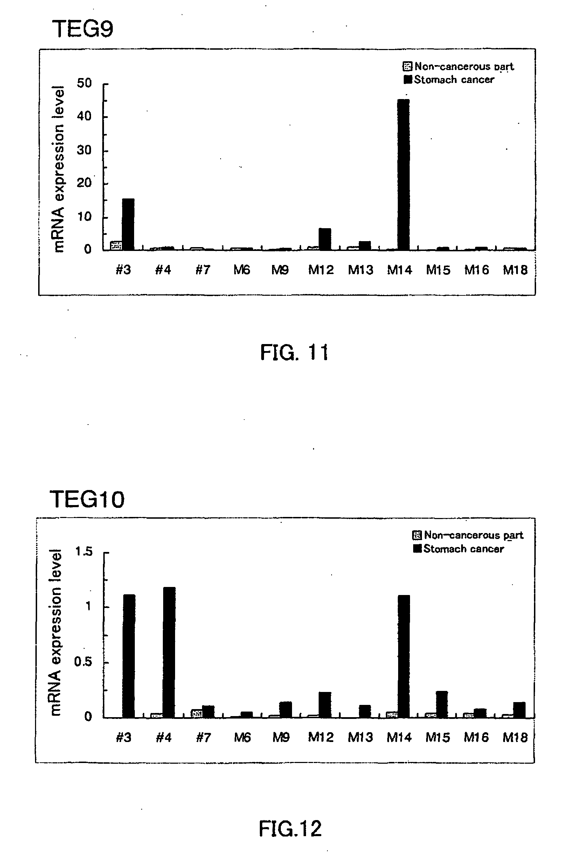

The PCR result showed that, an elevation in the expression of mRNA of TEG9 gene was observed clearly in the cancerous part in 6 cases out of 11 cases of the analyzed stomach cancer (Fig. 11).

### Expression analysis of TEG10

Gene expression was compared by an RT-PCR method using RNA prepared from 11 cases of stomach cancer tissue and normal stomach tissue in non-cancerous part of the same samples.

The PCR result showed that, an elevation in the expression of mRNA of TEG10 gene was observed clearly in the cancerous part in 10 cases out of 11 cases of the analyzed stomach cancer (Fig. 12).

### Expression analysis of TEG11

Gene expression was compared by an RT-PCR method using RNA prepared from 11 cases of stomach cancer tissue and normal stomach tissue in non-cancerous part of the same samples.

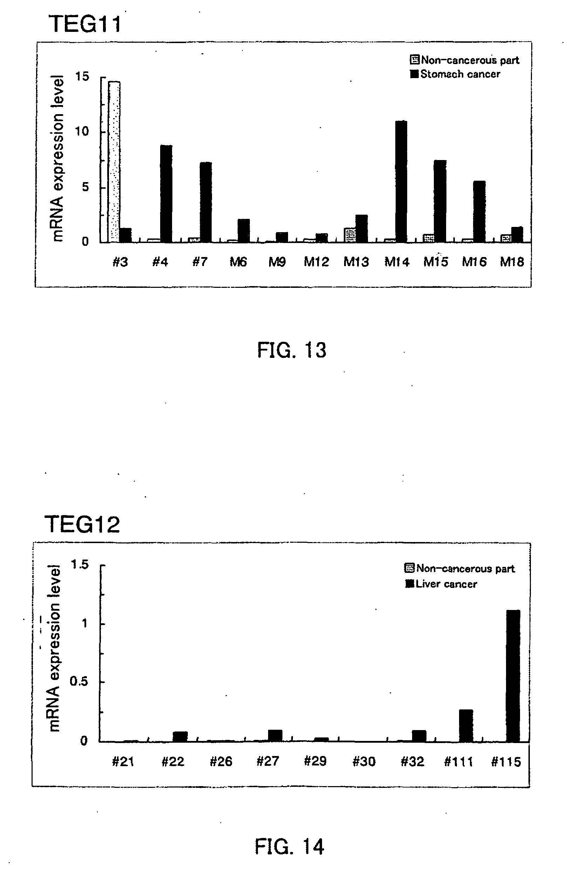

The PCR result showed that, an elevation in the expression of mRNA of TEG11 gene was observed clearly in the cancerous part in 10 cases out of 11 cases of the analyzed stomach cancer (Fig. 13).

### Expression analysis of TEG12

Gene expression was compared by an RT-PCR method using RNA prepared from 9 cases of liver cancer tissue and non-cancerous part of the same samples.

The PCR result showed that, an elevation in the expression of mRNA of TEG12 gene was observed clearly in the cancerous part in 6 cases out of 9 cases of the analyzed liver cancer, and in particular, a significant elevation in the expression of mRNA was observed in moderately differentiated liver cancer (#21, 29, 32) and poorly differentiated liver cancer (#22, 111, 115) (Fig. 14).

### Expression analysis of TEG13

Gene expression was compared by an RT-PCR method using RNA prepared from 9 cases of liver cancer tissue and non-cancerous part of the same samples.

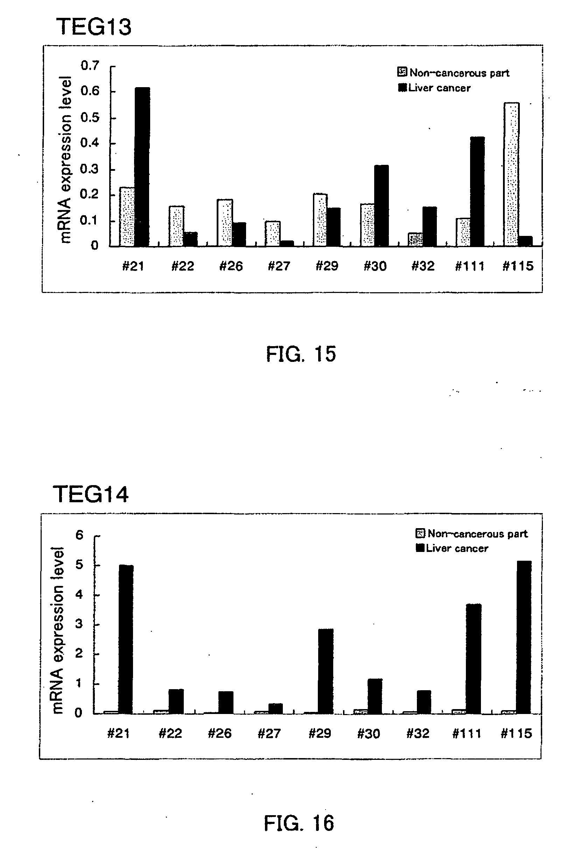

The PCR result showed that, an elevation in the expression of mRNA of TEG13 gene was observed clearly in the cancerous part in 4 cases out of 9 cases of the analyzed liver cancer (Fig. 15).

### Expression analysis of TEG14

Gene expression was compared by an RT-PCR method using RNA prepared from 9 cases of liver cancer tissue and non-cancerous part of the same samples.

The PCR result showed that, a significant elevation in the expression of mRNA of TEG14 gene was observed in the cancerous part in all the 9 cases of the analyzed liver cancer (Fig. 16).

### Expression analysis of TEG15

Gene expression was compared by an RT-PCR method using RNA prepared from 9 cases of liver cancer tissue and non-cancerous part of the same samples.

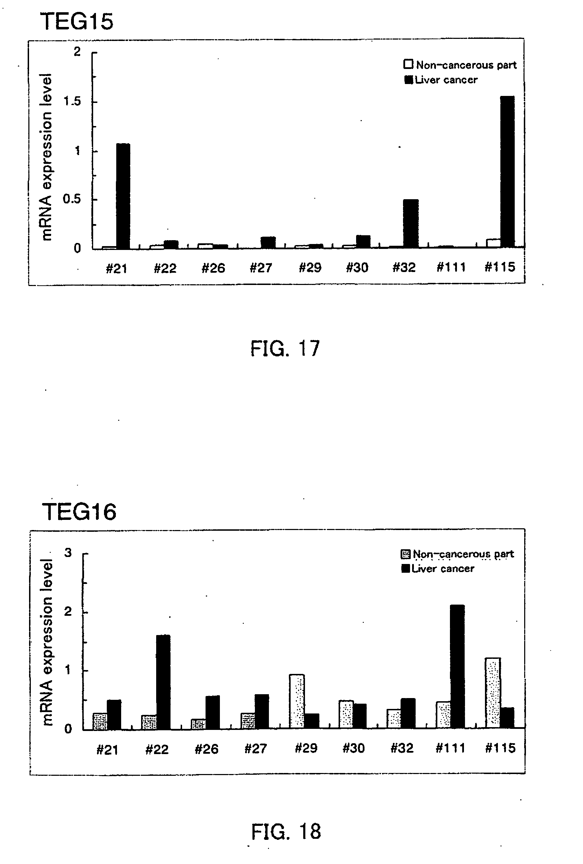

The PCR result showed that, a significant elevation in the expression of mRNA of TEG15 gene was observed in the cancerous part in 6 cases out of 9 cases of the analyzed liver cancer (Fig. 17).

### Expression analysis of TEG16

Gene expression was compared by an RT-PCR method using RNA prepared from 9 cases of liver cancer tissue and non-cancerous part of the same samples.

The PCR result showed that, a significant elevation in the expression of mRNA of TEG16 gene was observed in the cancerous part in 5 cases out of 9 cases of the analyzed liver cancer (Fig. 18).

### Expression analysis of TEG17

Gene expression was compared by an RT-PCR method using RNA prepared from 10 cases of large bowel cancer tissue and normal large bowel tissue in non-cancerous part of the same samples.

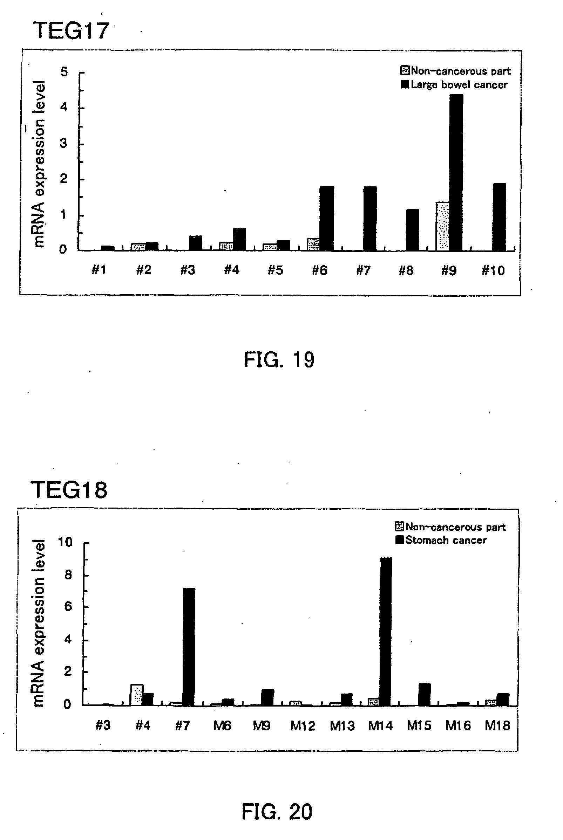

The PCR result showed that, an elevation in the expression of mRNA of TEG17 gene was observed in the cancerous part in all the analyzed 10 cases, and in particular, the gene was clearly overexpressed in 5 cases (Fig. 19).

### Expression analysis of TEG18

Gene expression was compared by an RT-PCR method using RNA prepared from 11 cases of stomach cancer tissue and normal stomach tissue in non-cancerous part of the same samples.

The PCR result showed that, an elevation in the expression of mRNA of TEG18 gene was observed clearly in the cancerous part in 7 cases out of 11 cases of the analyzed stomach cancer (Fig. 20).

### Expression analysis of TEG19

Gene expression was compared by a quantitative RT-PCR method using RNA prepared from 12 cases of lung adenocarcinoma tissue and 4 cases of normal lung tissue.

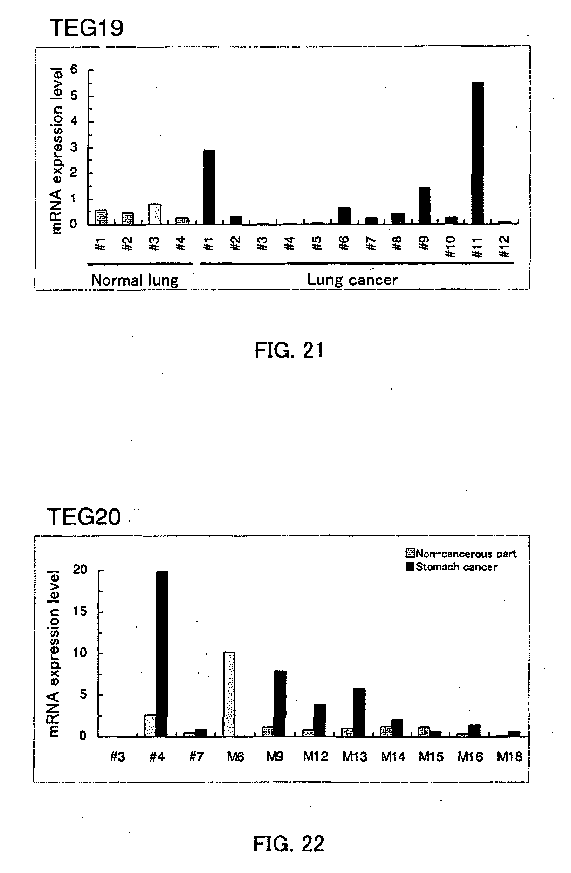

The PCR result showed that, while the expression of mRNA of TEG19 gene was not observed in the normal lung tissue, it was found that the expression of mRNA is clearly elevated in 3 cases out of the analyzed 12 cases of lung adenocarcinoma tissue (Fig. 21).

### Expression analysis of TEG20

Gene expression was compared by an RT-PCR method using RNA prepared from 11 cases of stomach cancer tissue and normal stomach tissue in non-cancerous part of the same samples.

The PCR result showed that, an elevation in the expression of mRNA of TEG20 gene was observed clearly in the cancerous part in 6 cases out of 11 cases of the analyzed stomach cancer (Fig. 22).

### Expression analysis of TEG21

Gene expression was compared by an RT-PCR method using RNA prepared from 9 cases of liver cancer tissue and non-cancerous part of the same samples.

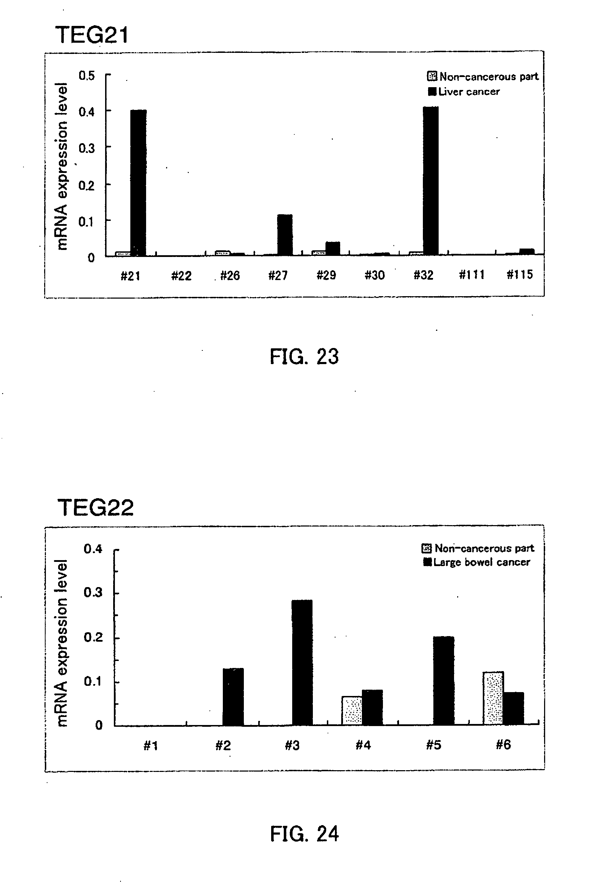

The PCR result showed that, an elevation in the expression of mRNA of TEG21 gene was observed clearly in the cancerous part in 5 cases out of 9 cases of the analyzed liver cancer, and in particular, a significant elevation in the expression of mRNA was observed in moderately differentiated liver cancer (#21, 27, 29, 32) (Fig. 23).

### Expression analysis of TEG22

Gene expression was compared by a quantitative RT-PCR method using RNA prepared from 6 cases of large bowel cancer tissue and normal large bowel tissue in non-cancerous part of the same samples.

The PCR result showed that, an elevation in the expression of mRNA of TEG22 gene was observed in the cancerous part in 3 cases out of the analyzed 6 cases (Fig. 24).

### Expression analysis of TEG23

Gene expression was compared by an RT-PCR method using RNA prepared from 9 cases of liver cancer tissue and non-cancerous part of the same samples.

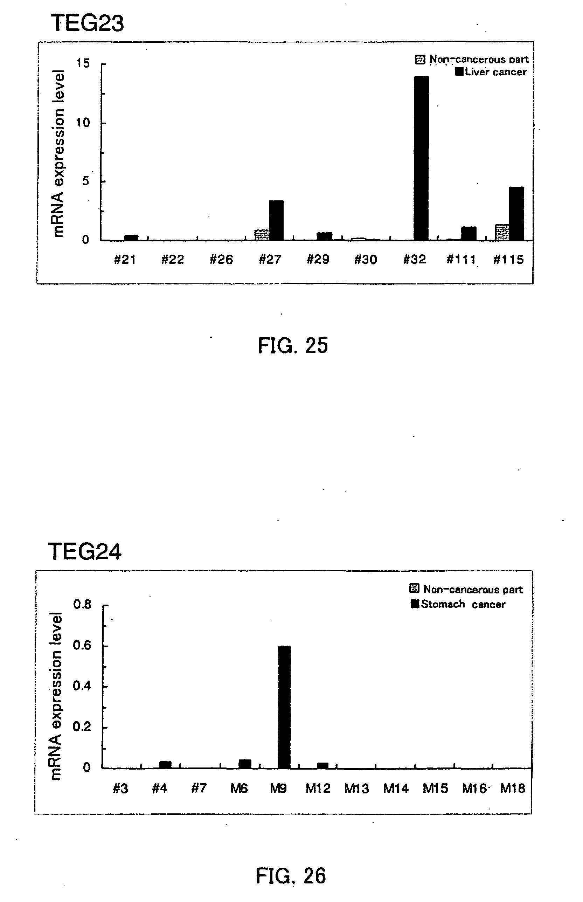

The PCR result showed that, an elevation in the expression of mRNA of TEG23 gene was observed clearly in the cancerous part in 6 cases out of 9 cases of the analyzed liver cancer (Fig. 25).

### Expression analysis of TEG24

Gene expression was compared by an RT-PCR method using RNA prepared from 11 cases of stomach cancer tissue and normal stomach tissue in non-cancerous part of the same samples.

The PCR result showed that, an elevation in the expression of mRNA of TEG24 gene was observed clearly in the cancerous part in 5 cases out of 11 cases of the analyzed stomach cancer (Fig. 26).

### Expression analysis of TEG25

Gene expression was compared by an RT-PCR method using RNA prepared from 11 cases of stomach cancer tissue and normal stomach tissue in non-cancerous part of the same samples.

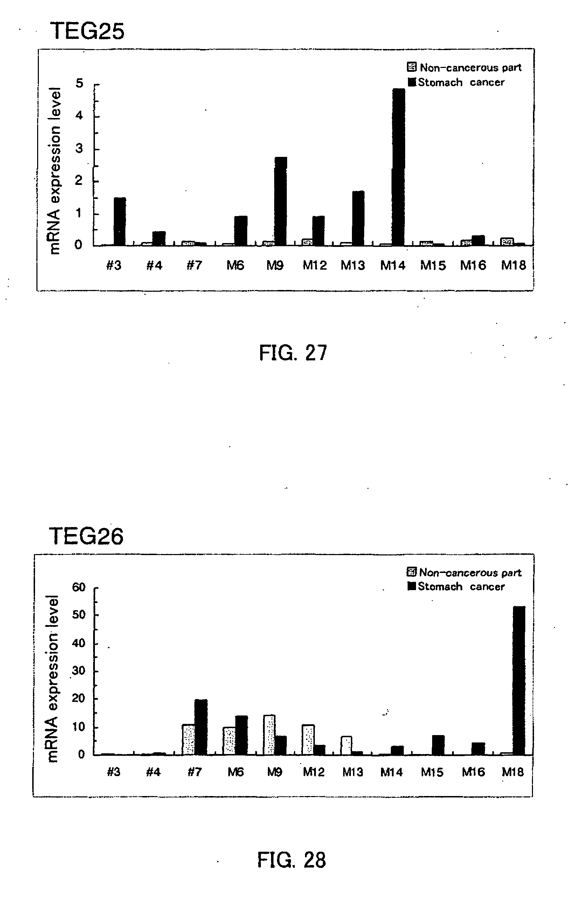

The PCR result showed that, an elevation in the expression of mRNA of TEG25 gene was observed clearly in the cancerous part in 7 cases out of 11 cases of the analyzed stomach cancer (Fig. 27).

### Expression analysis of TEG26

Gene expression was compared by an RT-PCR method using RNA prepared from 11 cases of stomach cancer tissue and normal stomach tissue in non-cancerous part of the same samples.

The PCR result showed that, an elevation in the expression of mRNA of TEG26 gene was observed clearly in the cancerous part in 4 cases out of 11 cases of the analyzed stomach cancer (Fig. 28).

### Expression analysis of TEG27

Gene expression was compared by an RT-PCR method using RNA prepared from 11 cases of stomach cancer tissue and normal stomach tissue in non-cancerous part of the same samples.

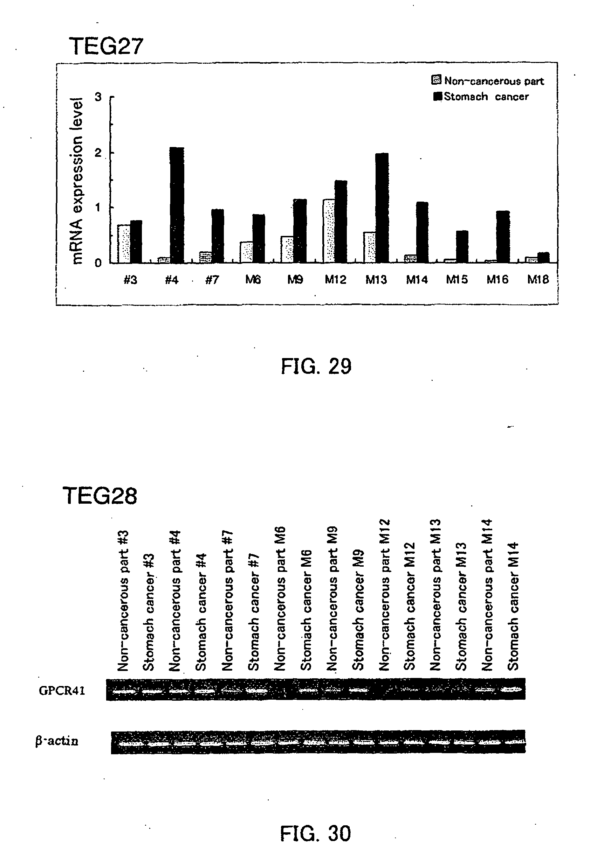

The PCR result showed that, an elevation in the expression of mRNA of TEG27 gene was observed clearly in the cancerous part in 8 cases out of 11 cases of the analyzed stomach cancer (Fig. 29).

### Expression analysis of TEG28

Gene expression was compared by an RT-PCR method using RNA prepared from 8 cases of stomach cancer tissue and normal stomach tissue in non-cancerous part of the same samples.

The PCR result showed that, an elevation in the expression of mRNA of TEG28 gene was observed clearly in the cancerous part in 5 cases out of 8 cases of the analyzed stomach cancer (Fig. 30).

### Expression analysis of TEG29

Gene expression was compared by a quantitative RT-PCR method using RNA prepared from 8 cases of lung adenocarcinoma tissue and 4 cases of normal lung tissue.

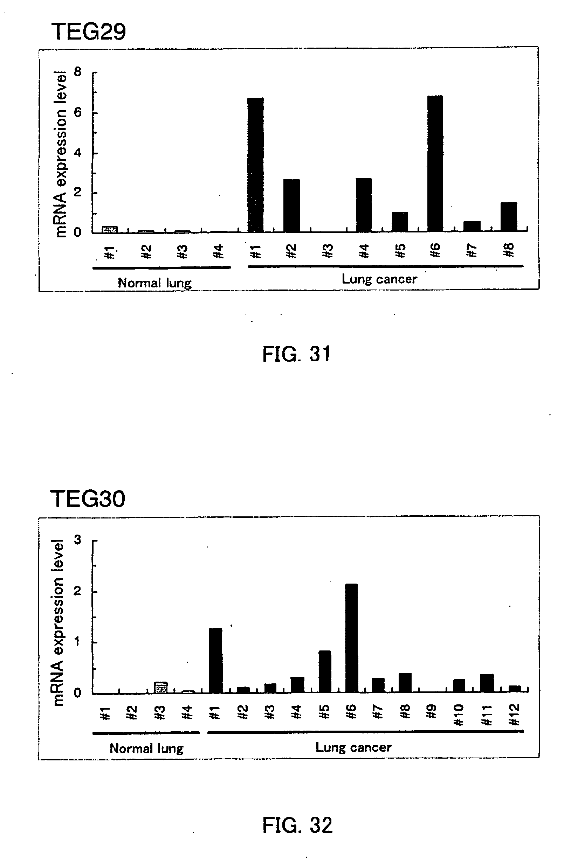

The PCR result showed that, while the expression of mRNA of TEG29 gene was not observed in the normal lung tissue, it was found that the expression of mRNA is clearly elevated in 7 cases out of the analyzed 8 cases of lung adenocarcinoma tissue (Fig. 31).

### Expression analysis of TEG30

Gene expression was compared by a quantitative RT-PCR method using RNA prepared from 12 cases of lung adenocarcinoma tissue and 4 cases of normal lung tissue.

The PCR result showed that, while very little expression of mRNA of TEG30 gene was observed in the normal lung tissue, expression of mRNA was observed in 11 cases out of the analyzed 12 cases of lung adenocarcinoma tissue, and moreover, it was found that the expression of mRNA is clearly elevated in 4 cases out of the 11 cases compared with that of the normal lung (Fig. 32).

### Expression analysis of TEG31

Gene expression was compared by a quantitative RT-PCR method using RNA prepared from 12 cases of lung adenocarcinoma tissue and 4 cases of normal lung tissue.

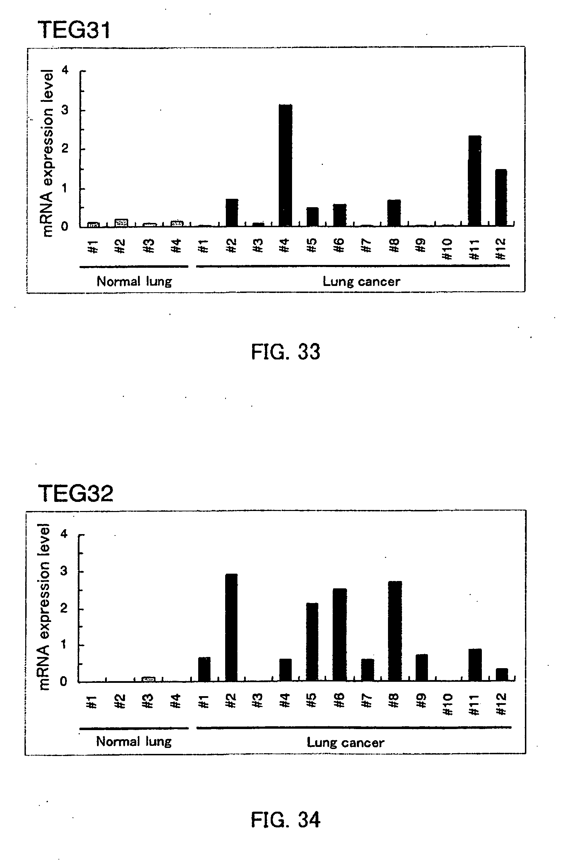

The PCR result showed that, while very little expression of mRNA of TEG31 gene was observed in the normal lung tissue, indicating that the expression of mRNA is clearly elevated in 7 cases out of the analyzed 12 cases of lung adenocarcinoma tissue (Fig. 33).

### Expression analysis of TEG32

Gene expression was compared by a quantitative RT-PCR method using RNA prepared from 12 cases of lung adenocarcinoma tissue and 4 cases of normal lung tissue.

The PCR result showed that, while the expression of mRNA of TEG32 gene was not observed in the normal lung tissue, indicating that the expression of mRNA is clearly elevated in 4 cases out of the analyzed 12 cases of lung adenocarcinoma tissue (Fig. 34).

### Expression analysis of TEG33

Gene expression was compared by a quantitative RT-PCR method using RNA prepared from 12 cases of lung adenocarcinoma tissue and 4 cases of normal lung tissue.

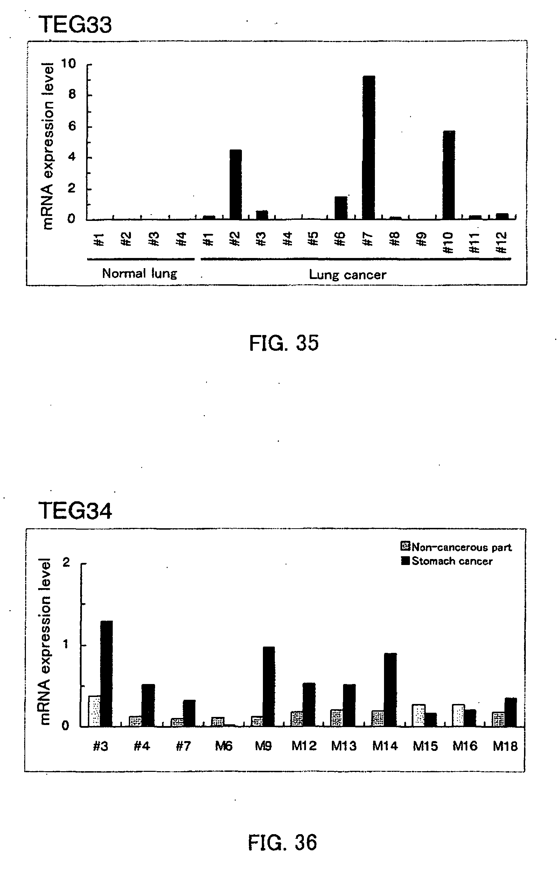

The PCR result showed that, while the expression of mRNA of TEG33 gene was not observed in the normal lung tissue, the expression of mRNA was observed in 9 cases out of the analyzed 12 cases of lung adenocarcinoma tissue, and in particular, extremely high expression of mRNA was observed in 4 cases (Fig. 35).

### Expression analysis of TEG34

Gene expression was compared by an RT-PCR method using RNA prepared from 11 cases of stomach cancer tissue and normal stomach tissue in non-cancerous part of the same samples.

The PCR result showed that, an elevation in the expression of mRNA of TEG34 gene was observed clearly in the cancerous part in 8 cases out of the analyzed 11 cases (Fig. 36).

### Expression analysis of TEG35

Gene expression was compared by an RT-PCR method using RNA prepared from 11 cases of stomach cancer tissue and normal stomach tissue in non-cancerous part of the same samples.

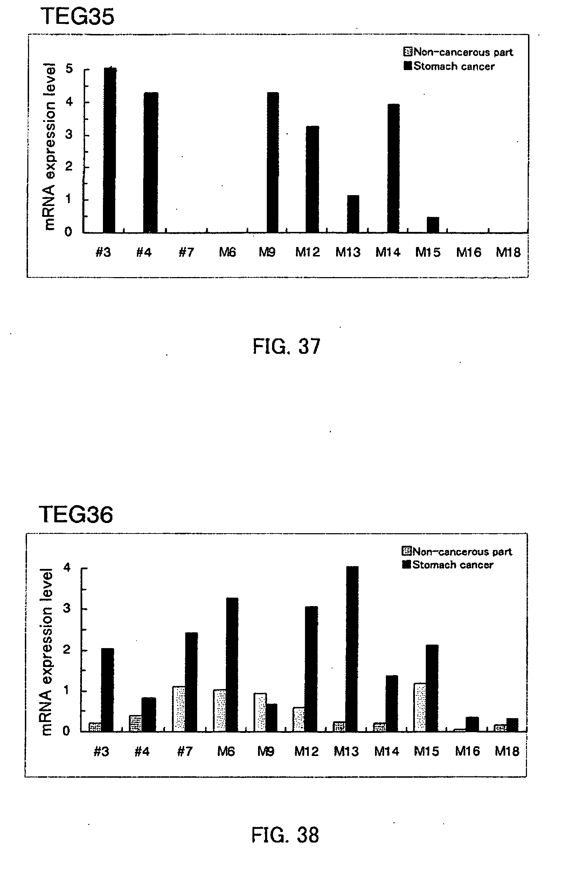

The PCR result showed that, an elevation in the expression of mRNA of TEG35 gene was observed clearly in the cancerous part in 7 cases out of the analyzed 11 cases (Fig. 37).

### Expression analysis of TEG36

Gene expression was compared by an RT-PCR method using RNA prepared from 11 cases of stomach cancer tissue and normal stomach tissue in non-cancerous part of the same samples.

The PCR result showed that, an elevation in the expression of mRNA of TEG36 gene was observed clearly in the cancerous part in 8 cases out of the analyzed 11 cases (Fig. 38).

### Expression analysis of TEG37

Gene expression was compared by an RT-PCR method using RNA prepared from 11 cases of stomach cancer tissue and normal stomach tissue in non-cancerous part of the same samples.

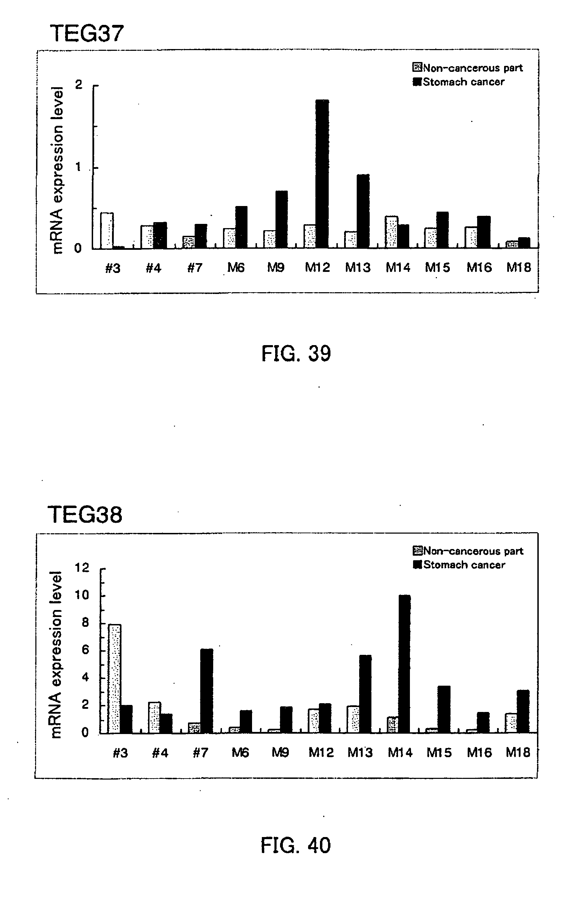

The PCR result showed that, an elevation in the expression of mRNA of TEG37 gene was observed clearly in the cancerous part in 7 cases out of the analyzed 11 cases (Fig. 39).

### Expression analysis of TEG38

Gene expression was compared by an RT-PCR method using RNA prepared from 11 cases of stomach cancer tissue and normal stomach tissue in non-cancerous part of the same samples.

The PCR result showed that, an elevation in the expression of mRNA of TEG38 gene was observed clearly in the cancerous part in 8 cases out of the analyzed 11 cases (Fig. 40).

### Expression analysis of TEG39

Gene expression was compared by an RT-PCR method using RNA prepared from 11 cases of stomach cancer tissue and normal stomach tissue in non-cancerous part of the same samples.

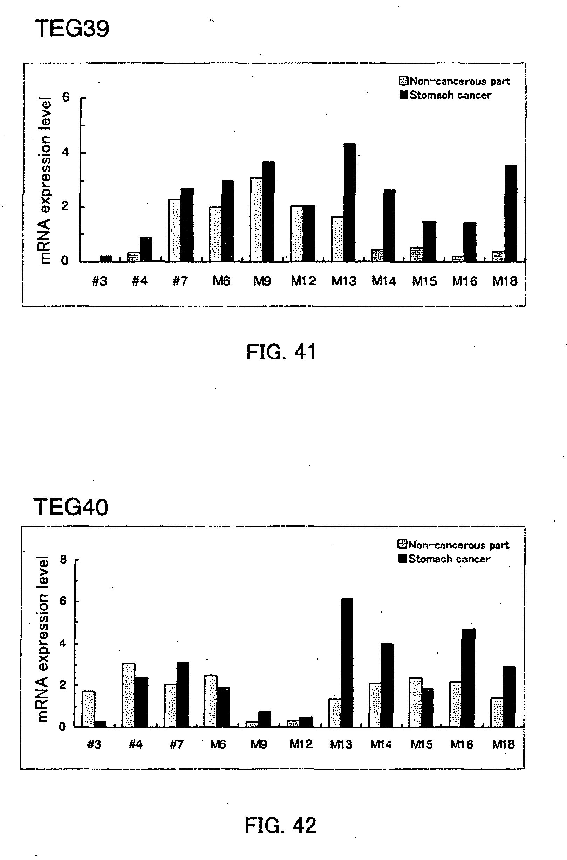

The PCR result showed that, a tendency that mRNA of TEG39 gene is overexpressed in the analyzed cancerous part was observed as a whole, and in particular, an elevation in the expression of mRNA was observed in the cancerous part in 6 cases out of 11 cases (Fig. 41).

### Expression analysis of TEG40

Gene expression was compared by an RT-PCR method using RNA prepared from 11 cases of stomach cancer tissue and normal stomach tissue in non-cancerous part of the same samples.

The PCR result showed that, an elevation in the expression of mRNA of TEG40 gene was observed clearly in the cancerous part in 4 cases out of the analyzed 11 cases (Fig. 42).

### Expression analysis of TEG41

Gene expression was compared by an RT-PCR method using RNA prepared from 11 cases of stomach cancer tissue and normal stomach tissue in non-cancerous part of the same samples.

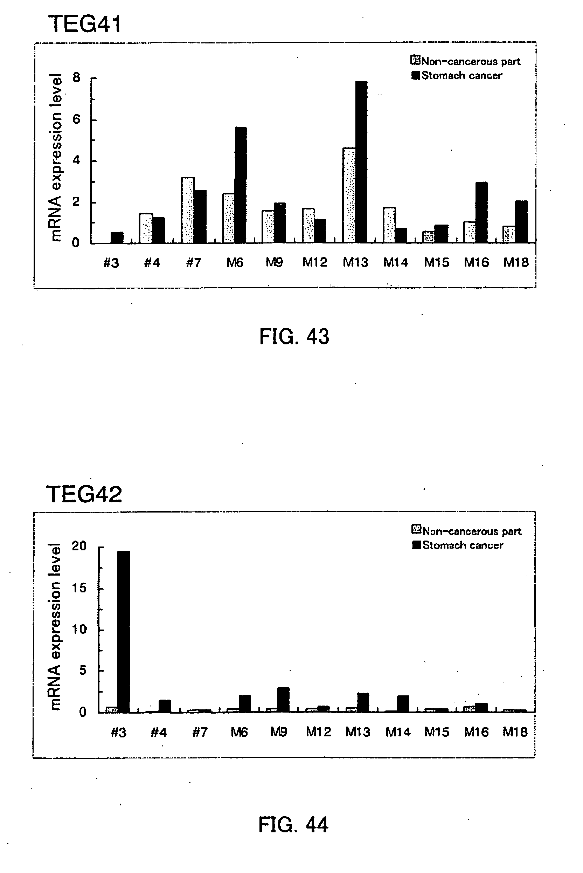

The PCR result showed that, an elevation in the expression of mRNA of TEG41 gene was observed clearly in the cancerous part in 4 cases out of the analyzed 11 cases (Fig. 43).

### Expression analysis of TEG42

Gene expression was compared by an RT-PCR method using RNA prepared from 11 cases of stomach cancer tissue and normal stomach tissue in non-cancerous part of the same samples.

The PCR result showed that, while the expression of mRNA of TEG42 gene was low as a whole in the normal stomach, an elevation in the expression of mRNA was observed clearly in the cancerous part in 6 cases out of the analyzed 11 cases (Fig. 44).

### Expression analysis of TEG43

Gene expression was compared by an RT-PCR method using RNA prepared from 11 cases of stomach cancer tissue and normal stomach tissue in non-cancerous part of the same samples.

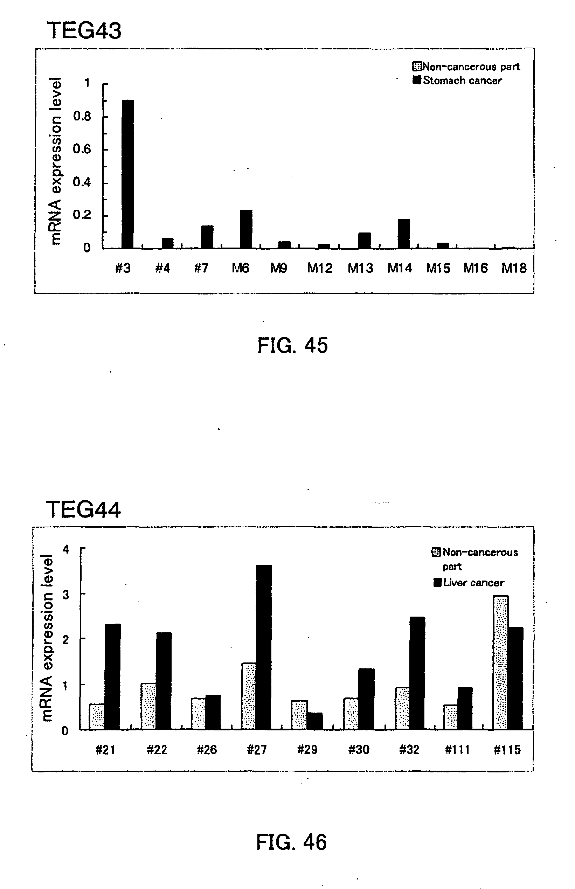

The PCR result showed that, while very little expression of mRNA of TEG43 gene was observed in the normal stomach, the expression of mRNA was observed in the cancerous part in 9 cases out of the analyzed 11 cases (Fig. 45).

### Expression analysis of TEG44

Gene expression was compared by an RT-PCR method using RNA prepared from 9 cases of liver cancer tissue and non-cancerous part of the same samples.

The PCR result showed that, an elevation in the expression of mRNA of TEG44 gene was observed clearly in the cancerous part in 5 cases out of 9 cases of the analyzed liver cancer (Fig. 46).

### Expression analysis of TEG45

Gene expression was compared by an RT-PCR method using RNA prepared from 11 cases of liver cancer tissue and non-cancerous part of the same samples.

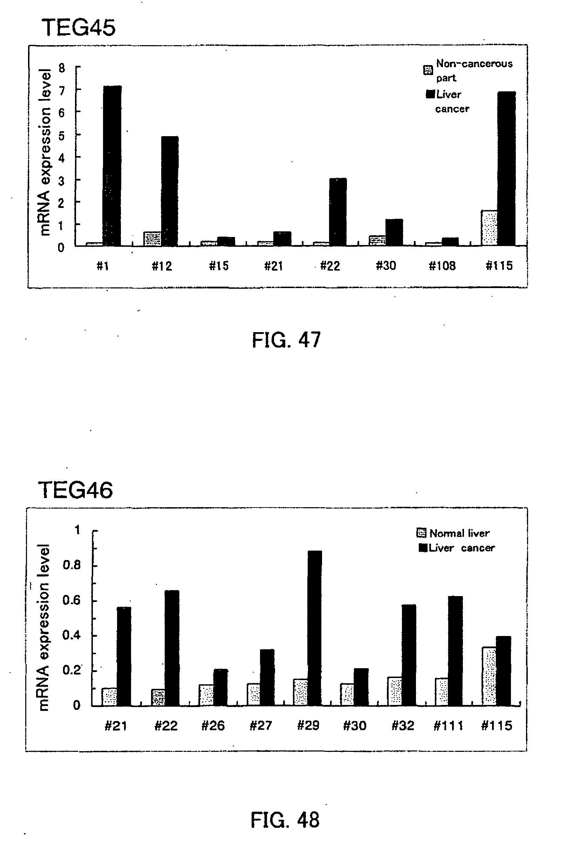

The PCR result showed that, an elevation in the expression of mRNA of TEG45 gene was observed clearly in the cancerous part in 7 cases out of 11 cases of the analyzed liver cancer (Fig. 47).

### Expression analysis of TEG46

Gene expression was compared by an RT-PCR method using RNA prepared from 9 cases of liver cancer tissue and non-cancerous part of the same samples.

The PCR result showed that, the expression level of mRNA of TEG46 gene showed a higher value in the cancerous part in all the 9 cases of the analyzed liver cancer and in particular, a significant elevation in the expression of mRNA was observed in the cancerous part in 6 cases (Fig. 48).

### Expression analysis of TEG47

Gene expression was compared by a quantitative RT-PCR method using RNA prepared from 10 cases of large bowel cancer tissue and normal large bowel tissue in non-cancerous part of the same samples.

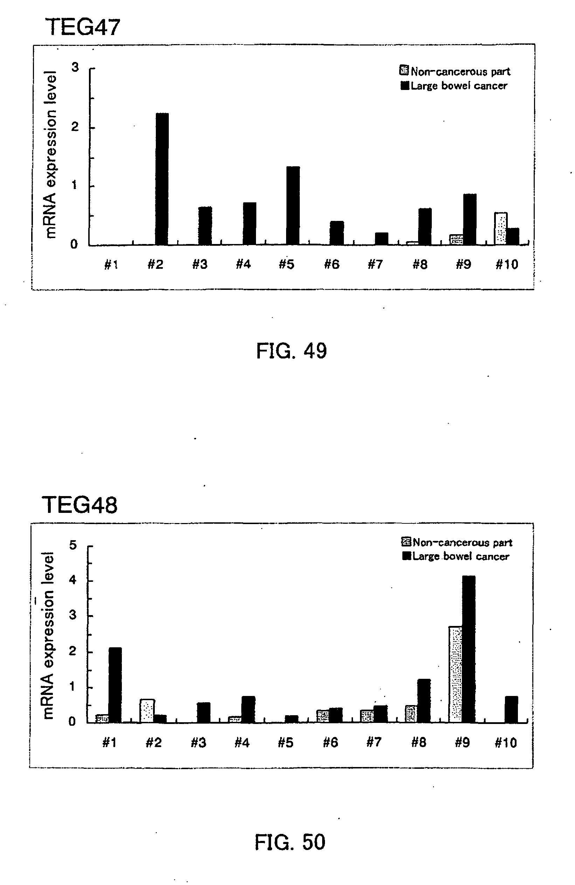

The PCR result showed that, an elevation in the expression of mRNA of TEG47 gene was observed clearly in the cancerous part compared with that in the normal large bowel tissue in the samples of 8 cases out of the analyzed 10 cases (Fig. 49).

### Expression analysis of TEG48

Gene expression was compared by an RT-PCR method using RNA prepared from 10 cases of large bowel cancer tissue and normal large bowel tissue in non-cancerous part of the same samples.

The PCR result showed that, an elevation in the expression of mRNA of TEG48 gene was observed in the cancerous part in 9 cases out of the analyzed 10 cases (Fig. 50).

### Expression analysis of TEG49

Gene expression was compared by a quantitative RT-PCR method using RNA prepared from 6 cases of large bowel cancer tissue and normal large bowel tissue in non-cancerous part of the same samples.

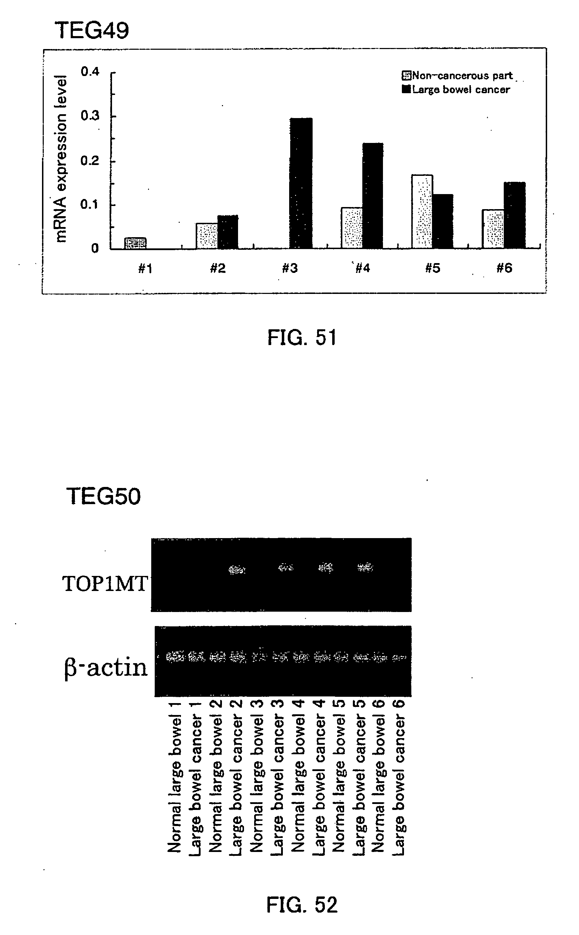

The PCR result showed that, the expression of mRNA of TEG49 gene is elevated in the cancerous part compared with that in the non-cancerous part in 3 cases out of the analyzed 6 cases (Fig. 51).

### Expression analysis of TEG50

Gene expression was compared by an RT-PCR method using RNA prepared from 6 cases of large bowel cancer tissue and normal large bowel tissue in non-cancerous part of the same samples.

The PCR result showed that, while amplification of a band derived from TEG50 was not observed in the normal large bowel tissue in all the analyzed 6 cases, amplification of the band was observed in the cancerous part in 4 cases out of 6 cases, indicating that the expression of mRNA is elevated in the cancerous part (Fig. 52).

### Expression analysis of TEG51

Gene expression was compared by an RT-PCR method using RNA prepared from 6 cases of large bowel cancer tissue and normal large bowel tissue in non-cancerous part of the same samples.

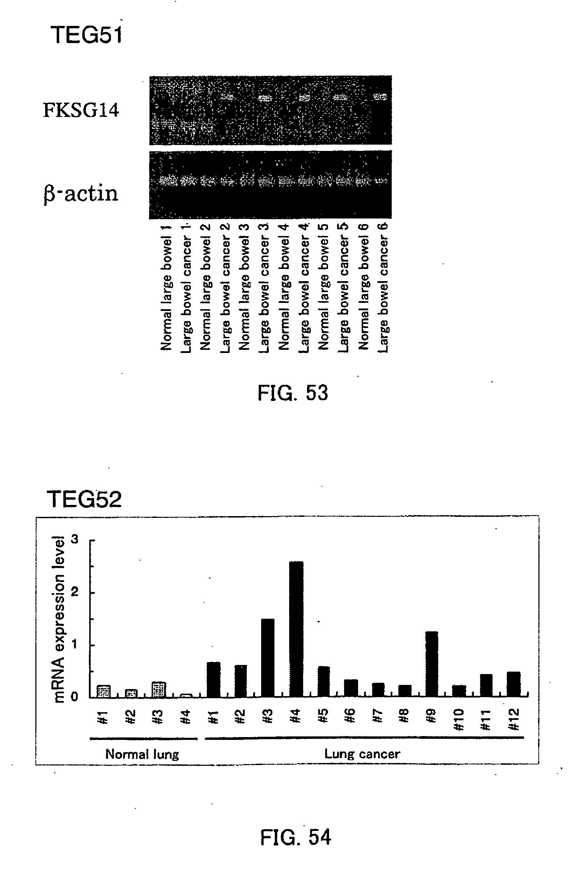

The PCR result showed that, while amplification of mRNA of TEG51 gene by PCR was not observed in any of the normal large bowel tissue, amplification of TEG51 gene was clearly observed in 5 cases out of the analyzed 6 cases of large bowel tissue, indicating that the expression of mRNA is elevated in large bowel cancer (Fig. 53).

### Expression analysis of TEG52

Gene expression was compared by a quantitative RT-PCR method using RNA prepared from 12 cases of lung adenocarcinoma tissue and 4 cases of normal lung tissue.

The PCR result showed that, the expression of mRNA of TEG52 gene is elevated clearly in lung cancer in 7 cases out of the analyzed 12 cases compared with that in the normal lung tissue (Fig. 54).

### Expression analysis of TEG53

Gene expression was compared by a quantitative RT-PCR method using RNA prepared from 8 cases of lung adenocarcinoma tissue and 4 cases of normal lung tissue.

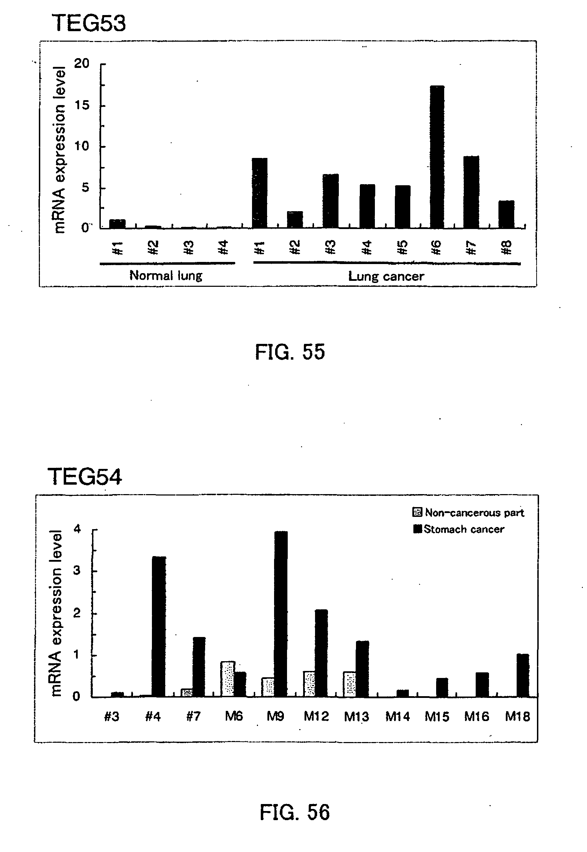

The PCR result showed that while the expression of mRNA of TEG53 gene was not observed in the normal lung tissue, an elevation in the expression of mRNA was observed in all the analyzed 8 cases of lung adenocarcinoma tissue (Fig. 55).

### Expression analysis of TEG54

Gene expression was compared by an RT-PCR method using RNA prepared from 11 cases of stomach cancer tissue and normal stomach tissue in non-cancerous part of the same samples.

The PCR result showed that, an elevation in the expression of mRNA of TEG54 gene was observed clearly in the cancerous part in 9 cases out of the analyzed 11 cases (Fig. 56).

### Expression analysis of TEG55

Gene expression was compared by an RT-PCR method using RNA prepared from 11 cases of stomach cancer tissue and normal stomach tissue in non-cancerous part of the same samples.

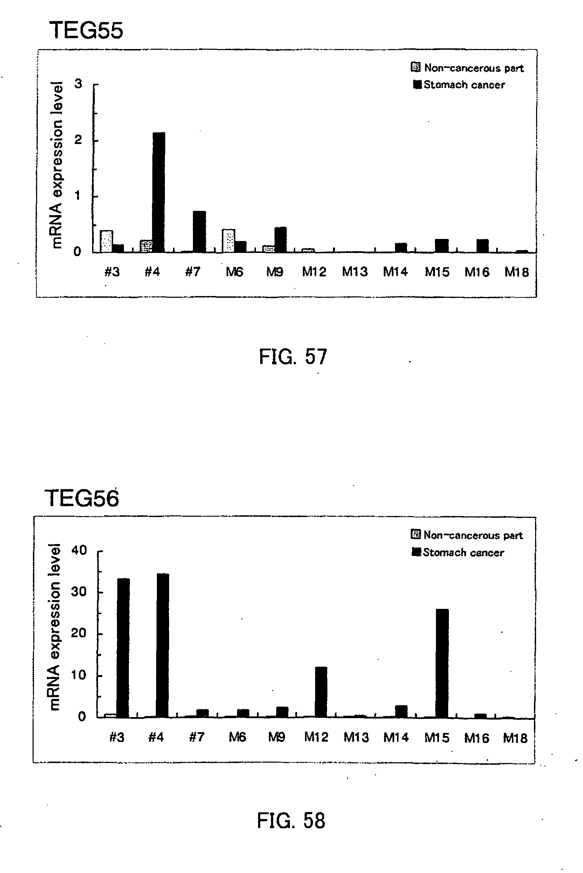

The PCR result showed that, an elevation in the expression of mRNA of TEG55 gene was observed clearly in the cancerous part in 6 cases out of the analyzed 11 cases (Fig. 57).

### Expression analysis of TEG56

Gene expression was compared by an RT-PCR method using RNA prepared from 11 cases of stomach cancer tissue and normal stomach tissue in non-cancerous part of the same samples.

The PCR result showed that, while the expression of mRNA of TEG56 gene was low as a whole in the normal stomach, an elevation in the expression of mRNA was observed clearly in the cancerous part in 9 cases out of the analyzed 11 cases (Fig. 58).

### Expression analysis of TEG57

Gene expression was compared by an RT-PCR method using RNA prepared from 9 cases of liver cancer tissue and non-cancerous part of the same samples.

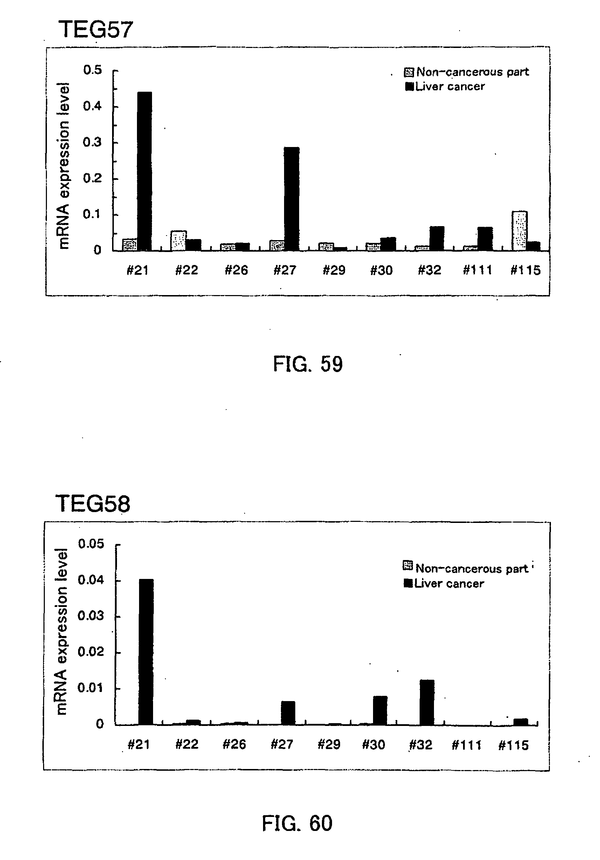

The PCR result showed that, an elevation in the expression of mRNA of TEG57 gene was observed clearly in the cancerous part in 5 cases out of the analyzed 9 cases of liver cancer (Fig. 59).

### Expression analysis of TEG58

Gene expression was compared by an RT-PCR method using RNA prepared from 9 cases of liver cancer tissue and non-cancerous part of the same samples.

The PCR result showed that, an elevation in the expression of mRNA of TEG58 gene was observed clearly in the cancerous part in 5 cases out of the analyzed 9 cases of liver cancer (Fig. 60).

### Expression analysis of TEG59

Gene expression was compared by an RT-PCR method using RNA prepared from 9 cases of liver cancer tissue and non-cancerous part of the same samples.

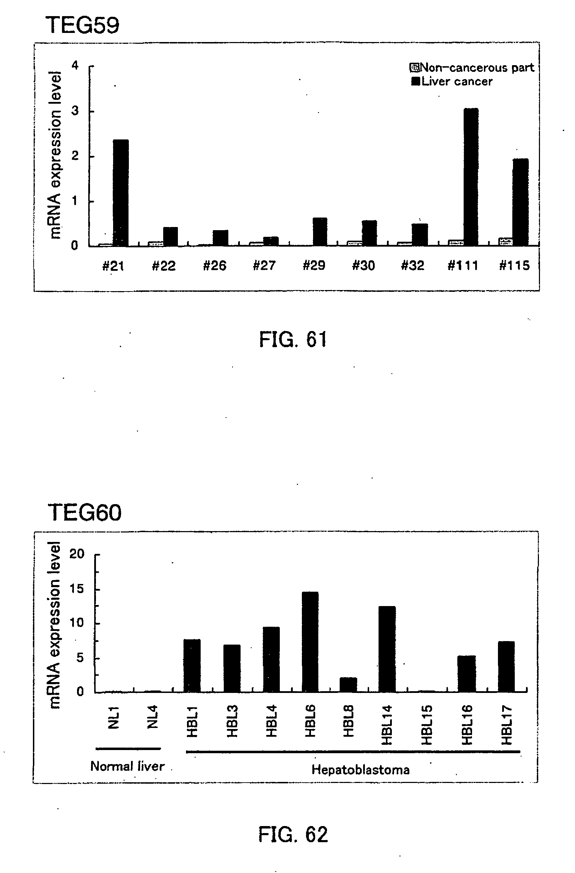

The PCR result showed that, while the expression level of mRNA of TEG59 gene was little as a whole in 9 cases of the analyzed non-cancerous part, an elevation in the expression of mRNA was observed clearly in the cancerous part in all the analyzed 9 cases of liver cancer (Fig. 61).

### Expression analysis of TEG60

Gene expression was compared by an RT-PCR method using RNA prepared from 9 cases of hepatoblastoma tissue and 2 cases of normal liver.

The PCR result showed that, while very little expression of mRNA of TEG60 gene was observed in the analyzed normal liver, an elevation in the expression of mRNA was observed clearly in 8 cases out of the analyzed 9 cases of hepatoblastoma (Fig. 62).

### Expression analysis of TEG61

Gene expression was compared by a quantitative RT-PCR method using RNA prepared from 12 cases of lung adenocarcinoma tissue and 4 cases of normal lung tissue.

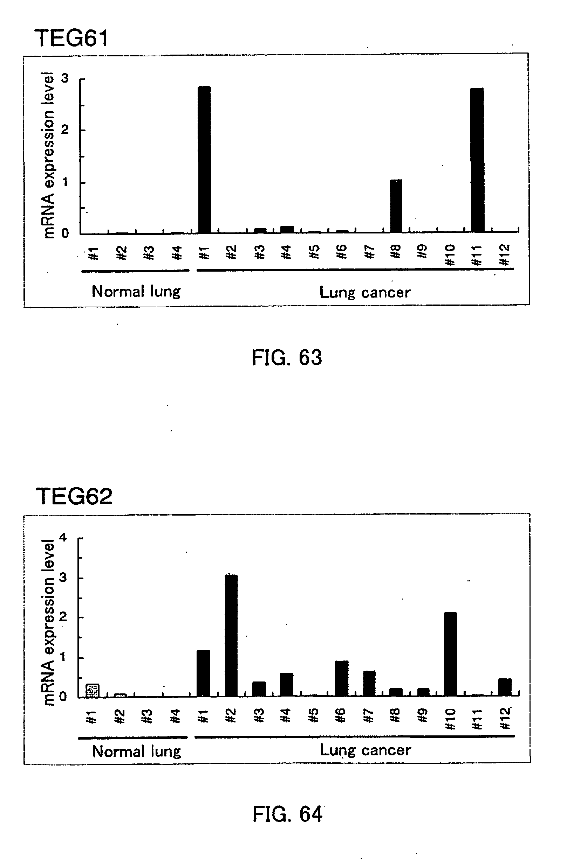

The result showed that, while the expression of mRNA of TEG61 gene was not observed in the normal lung tissue, overexpression of the PAEP gene was observed in 3 cases out of the analyzed 12 cases of lung adenocarcinoma tissue (Fig. 63).

### Expression analysis of TEG62

Gene expression was compared by a quantitative RT-PCR method using RNA prepared from 12 cases of lung adenocarcinoma tissue and 4 cases of normal lung tissue.

The PCR result showed that, the expression of mRNA of TEG62 gene is clearly elevated in 8 cases out of the analyzed 12 cases of lung adenocarcinoma tissue compared with the expression in the normal lung tissue (Fig. 64).

### Expression analysis of TEG63

Gene expression was compared by an RT-PCR method using RNA prepared from 9 cases of liver cancer tissue and non-cancerous part of the same samples.

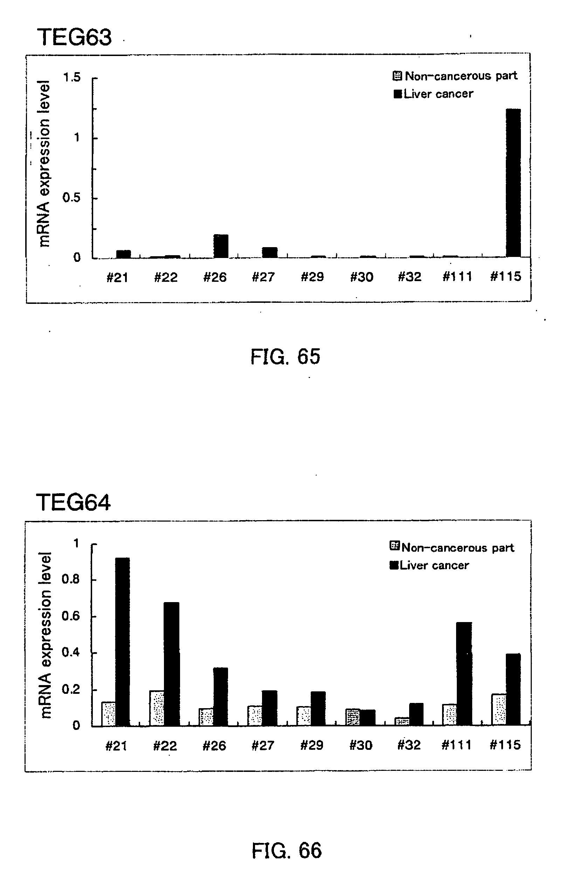

The PCR result showed that, while very little expression of mRNA of TEG63 gene was observed in the non-cancerous part in the analyzed 9 cases, an elevation in the expression of mRNA was observed clearly in 8 cases out of the analyzed 9 cases of liver cancer (Fig. 65).

### Expression analysis of TEG64

Gene expression was compared by an RT-PCR method using RNA prepared from 9 cases of liver cancer tissue and non-cancerous part of the same samples.

The PCR result showed that, an elevation in the expression of mRNA of TEG64 gene was observed clearly in the cancerous part in 8 cases out of the analyzed 9 cases of liver cancer (Fig. 66).

The above results revealed that these genes can be used in diagnosis of cancer by measuring the expression level of the genes or proteins.

### Example 3

### Isolation and identification of full-length cDNA for TEG12 gene expressed in liver cancer

cDNA was isolated and identified to determine the cDNA sequence of TEG12 whose expression was found to be elevated in liver cancer in the above-mentioned Gene chip analysis and RT-PCR analysis.

More specifically, the sequence of the EST (GenBank; BU844373) located near the EST (GenBank; BF057073: SEQ ID NO: 254), used as the origin of the probe sequence in the Gene chip analysis, was obtained from GenBank. Primers to be hybridized to each EST were designed and cDNA was amplified by PCR. PCR was carried out by using a single-stranded cDNA prepared from an equal amount of each RNA prepared from Hep3B, HuH6 and HepG2, which are human liver cancer cell lines, as a template and each 5 pmole of PCR primers LS557 (ATCCGCCAGG TGAAAGCCAA GTC: SEQ ID NO: 255) and LS589 (GGGATTCACA TTACCACGGC AGTGC: SEQ ID NO: 256). PCR was carried out by using an LA-PCR kit (manufactured by TAKARA) for 35 cycles of 94°C for 30 seconds, 63°C for 30 seconds and 72°C for 5 minutes. A band of about 2000 bp was amplified. The PCR amplification product was inserted into pGEM-T Easy vector (manufactured by Promega), and the nucleotide sequence of the amplified gene was analyzed by a standard method. It was found that it contains a sequence of the 5' upstream region from the DNA sequence of the original EST (BF057073). The DNA sequence amplified by PCR is shown in SEQ ID NO: 257.

Subsequently, PCR was carried out by using PCR primers designed based on the sequence of another EST sequence (BU859386) that is thought to be located near BF057073 and the sequence of the gene isolated and identified as described above. Five pmole of each of LS858 (ATGGCTTCGT TCCCCGAGAC CGATTC: SEQ ID NO: 258) and LS859 (GAAGACGAGG ATTCGATTGT TGCCAAAGT CCACC: SEQ ID NO: 259) was used as PCR primers, and PCR was carried out in the same conditions as described above except for performing 35 cycles of a reaction consisting of 95°C for 30 seconds and 68°C for 3 minutes. A band of about 2,500 bp was amplified. After the PCR amplification product was inserted into pGEM-T Easy vector in the same manner as described above, the nucleotide sequence was identified. It was found that the product further contains a sequence of further 5' upstream. The DNA sequence amplified by PCR is shown in SEQ ID NO: 260.

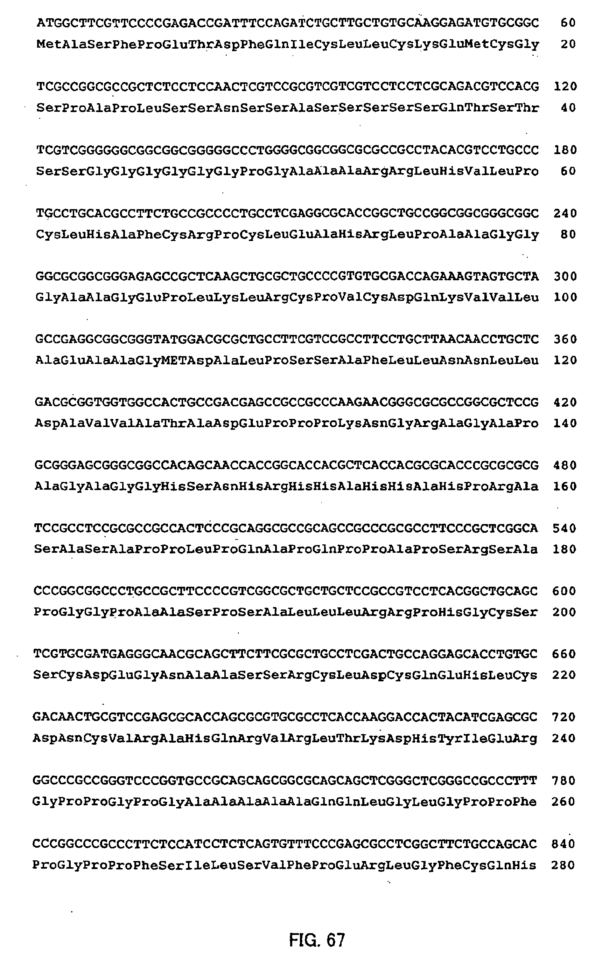

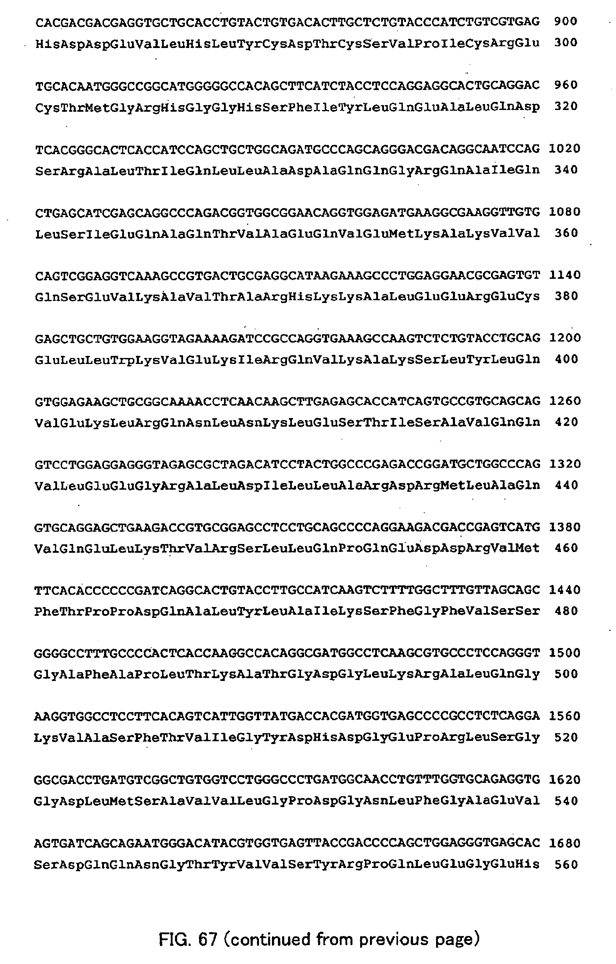

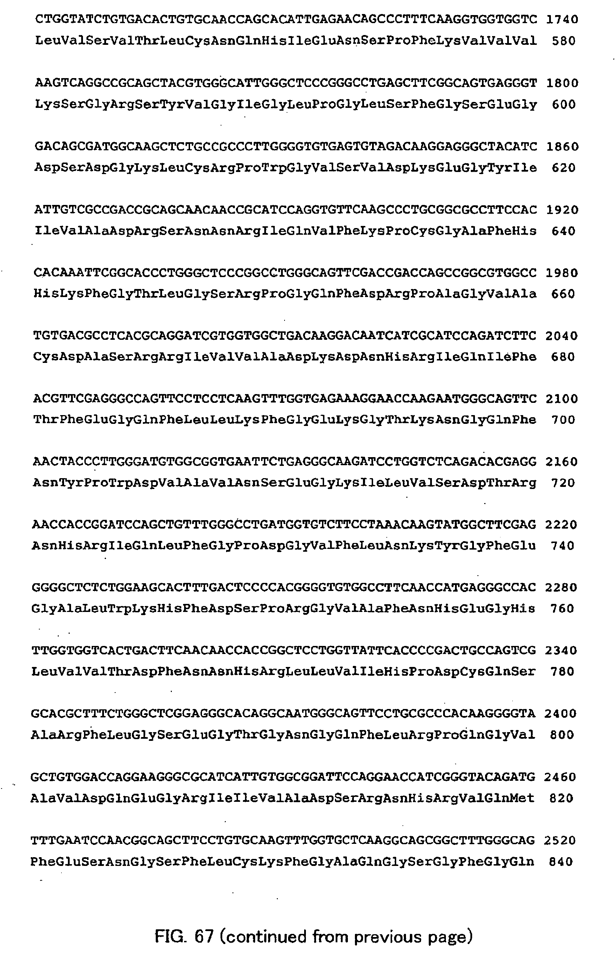

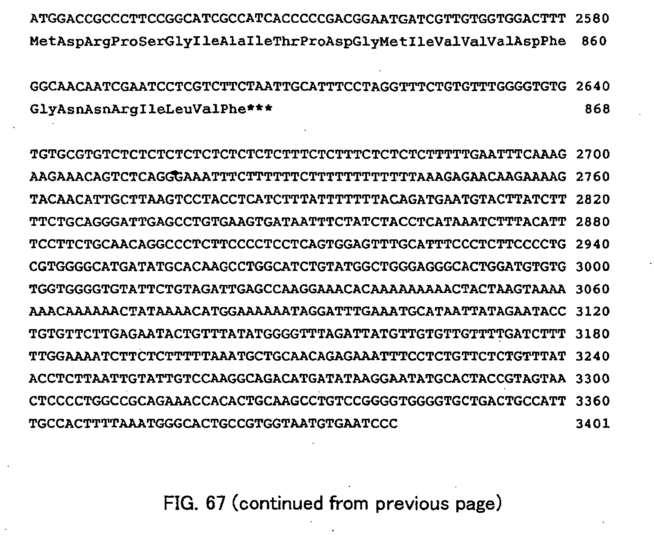

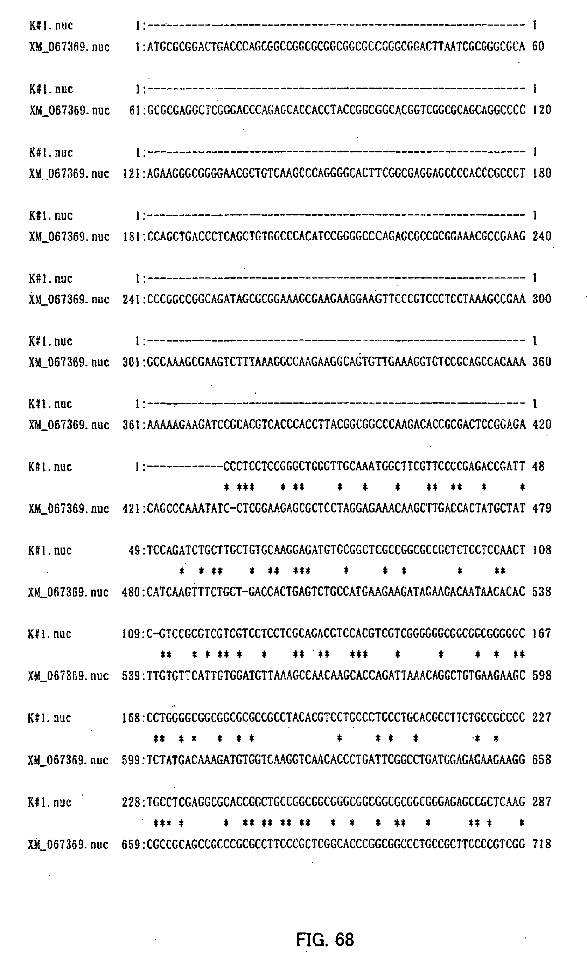

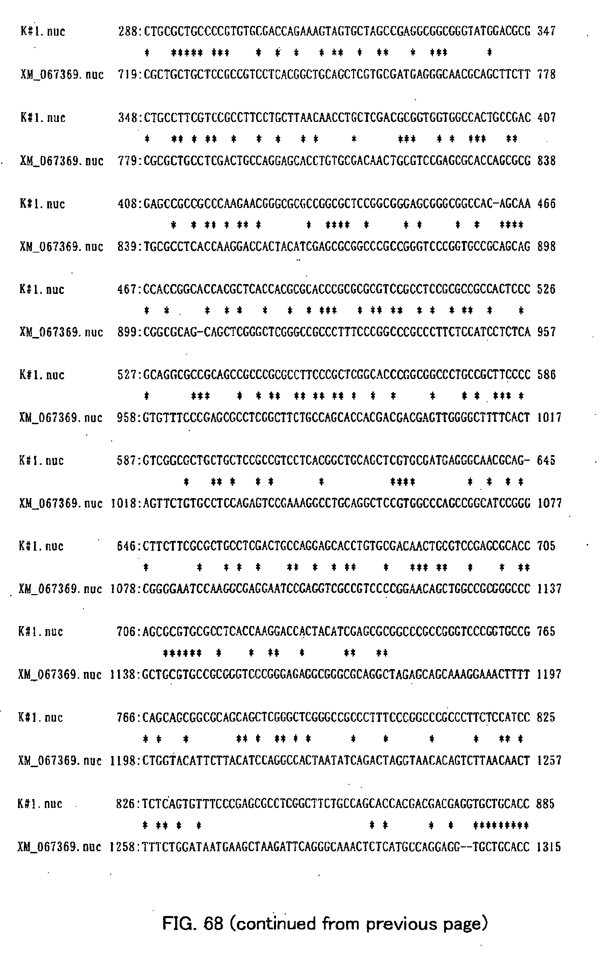

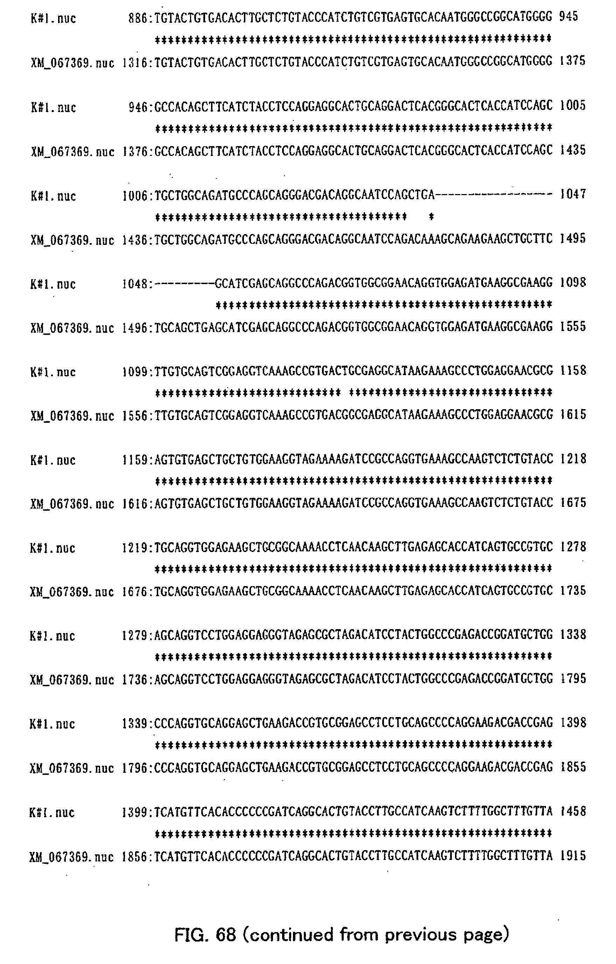

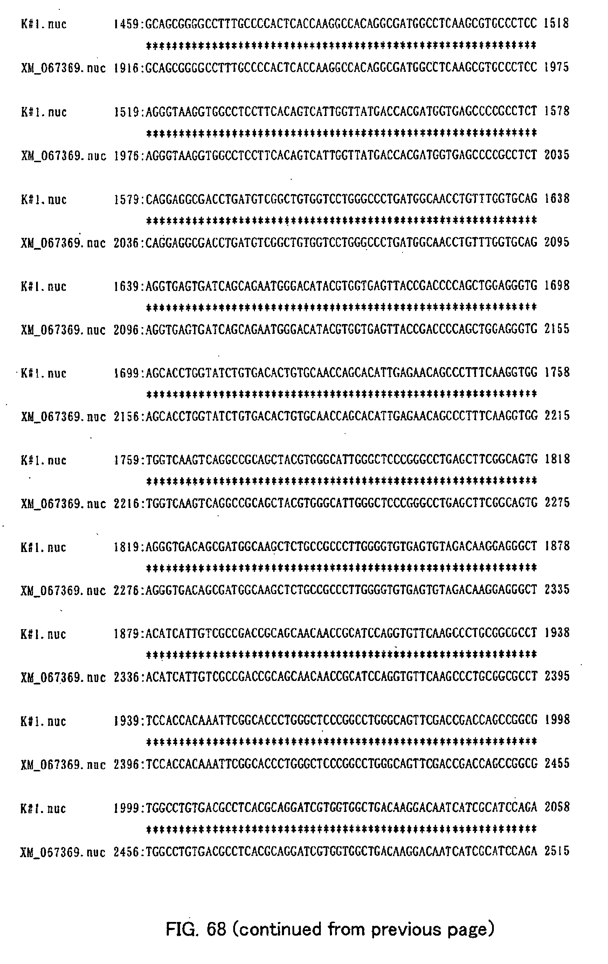

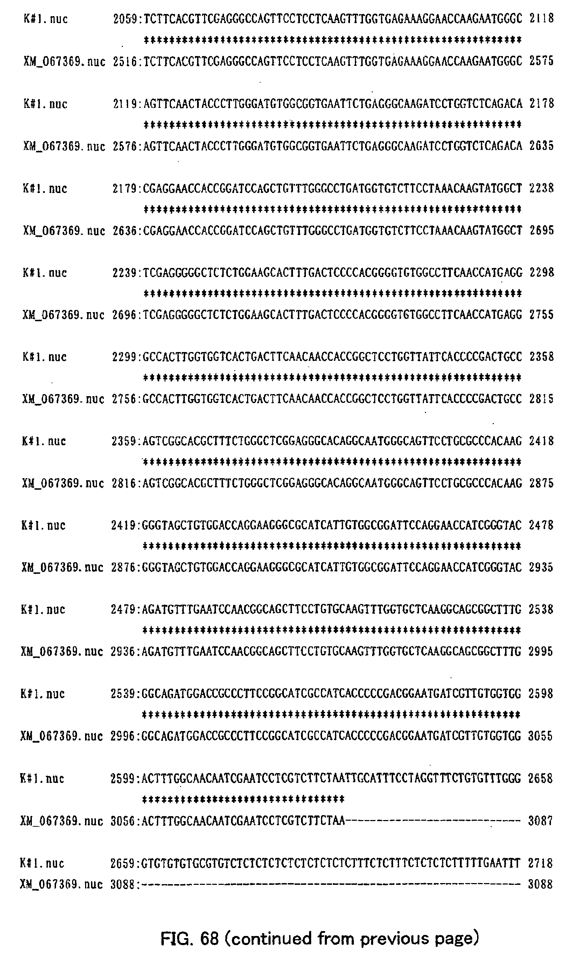

Based on the sequence of the two amplified products obtained by the PCR method described above, a novel cDNA consisting of the total length of 3, 401 bp was identified, which contains one open reading frame (Fig. 67). Its nucleotide sequence and the amino acid sequence deduced from the nucleotide sequence are shown in SEQ ID NOs: 15 and 72, respectively. A Blast search was conducted based on the sequence isolated and identified in this study. It was found that the gene shows homology to GenBank No. XM_067369 (SEQ ID NO: 263), but contains a region having a partially different sequence (Fig. 68). In this way, a novel gene whose expression is elevated specifically in a liver cancer cell was isolated, identified and named K#1.

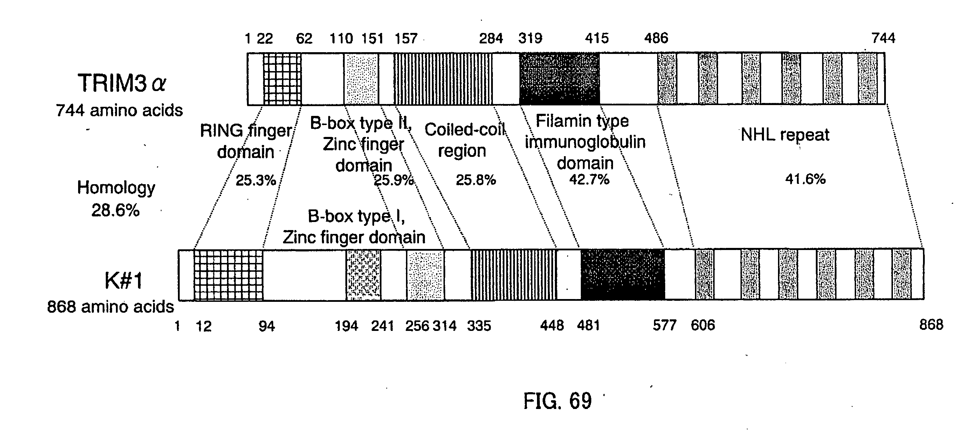

A homology search was conducted based on the amino acid sequence deduced from the nucleotide sequence isolated in this study against known proteins. The sequence showed a homology of 28.6% to human TRIM3α (tripartite motif-containing 3, GenBank No: NM_006458) and a homology of 27.5% to human TRIM2. It has been reported that there are 37 types of genes belonging to TRIM family, and it is known that they have several characteristic motifs (Reymond A., et al., EMBO J. (2001) 20. 2140-2151). Thus, a motif analysis was carried out based on the amino acid sequence of K#1. The novel gene was found to have a motif structure which is relatively similar to that of TRIM3 or TRIM2 as well as the homology of the amino acid sequence. In fact, as shown in Fig. 69, characteristic motifs similar to TRIM3α were found to be conserved. However, since a molecule having exactly the same structure as the motif structure of K#1 does not exist in the known TRIM family, it was strongly suggested that K#1 isolated and identified in this study is a novel TRIM molecule that is relatively similar to TRIM2 and TRIM3. Further, it has been suggested that rat BERP having a structure similar to that of TRIM3 is localized in a cell, binds to myosin V or the like, and is involved in the intracellular transport of protein or is involved in neurite outgrowth as in the case of K#1 (El-Husseini, A. et al. , Biochem. Biophys. Res. Commun. 267, 906-911, 2000, El-Husseini, A. et al., J. Biol. Chem. 274, 19771-19777, 1999). Accordingly, it is believed that the K#1 protein identified in this study belongs to the TRIM family and may be involved in morphogenesis or growth of cells by playing a role in intracellular protein transport in the same manner as rat BERP. It was also suggested that the protein may play an important role in a disease in which its expression is elevated, such as liver cancer, and may serve as a target molecule of a drug.

### Example 4

### 4-1. Isolation and identification of full-length cDNA for gene expressed in liver cancer (TEG23)

cDNA was isolated and identified by RACE (rapid amplification of cDNA ends) method to determine the full-length cDNA sequence of TEG23 whose expression was found to be elevated in liver cancer in the above-mentioned Gene chip analysis and RT-PCR analysis.

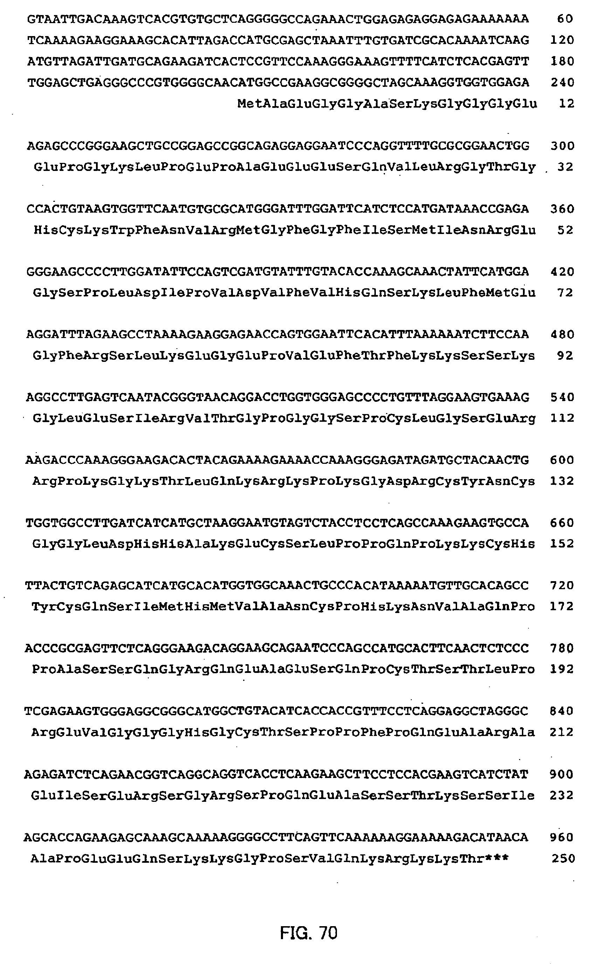

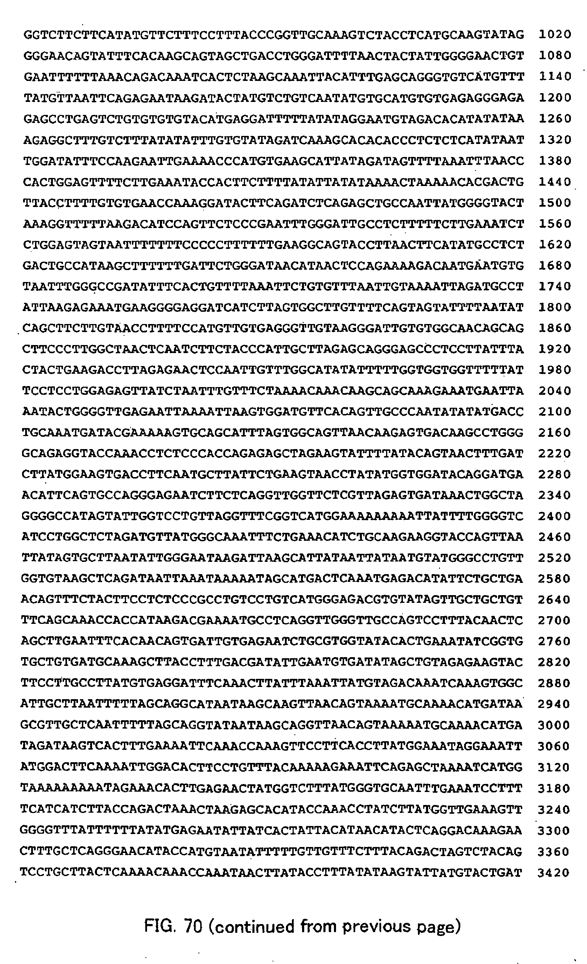

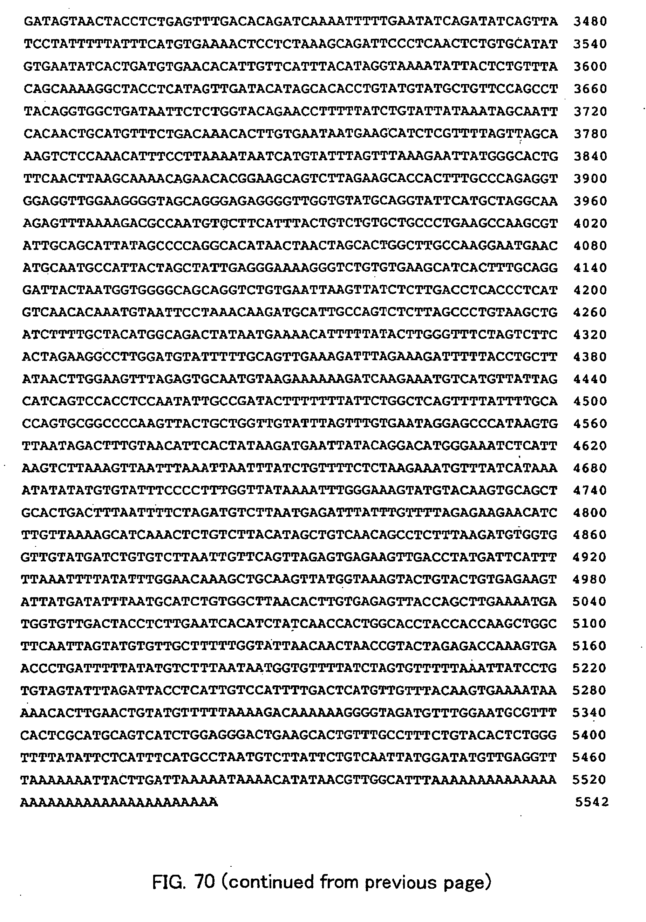

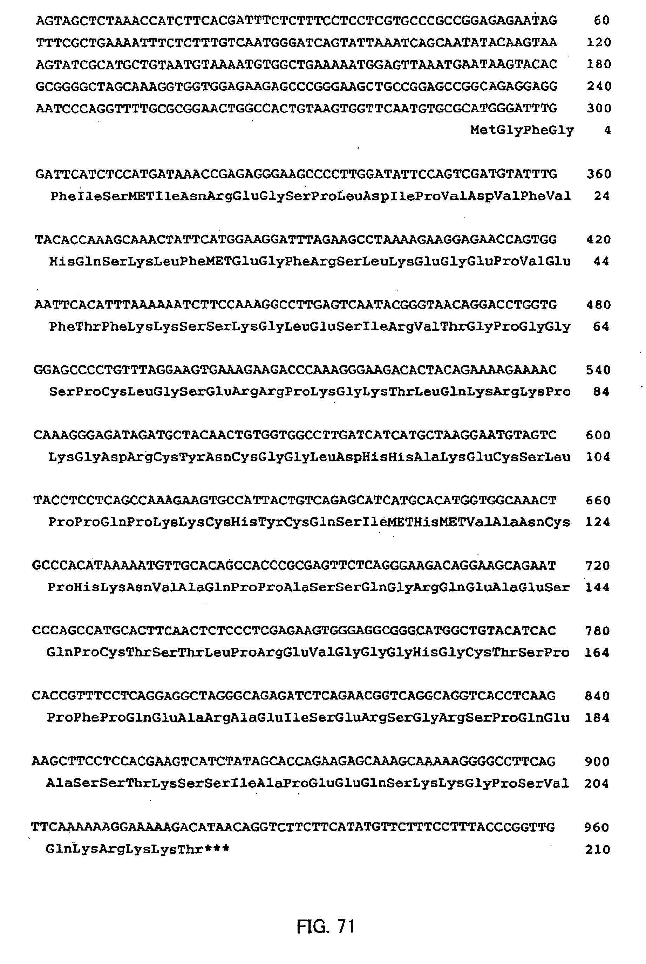

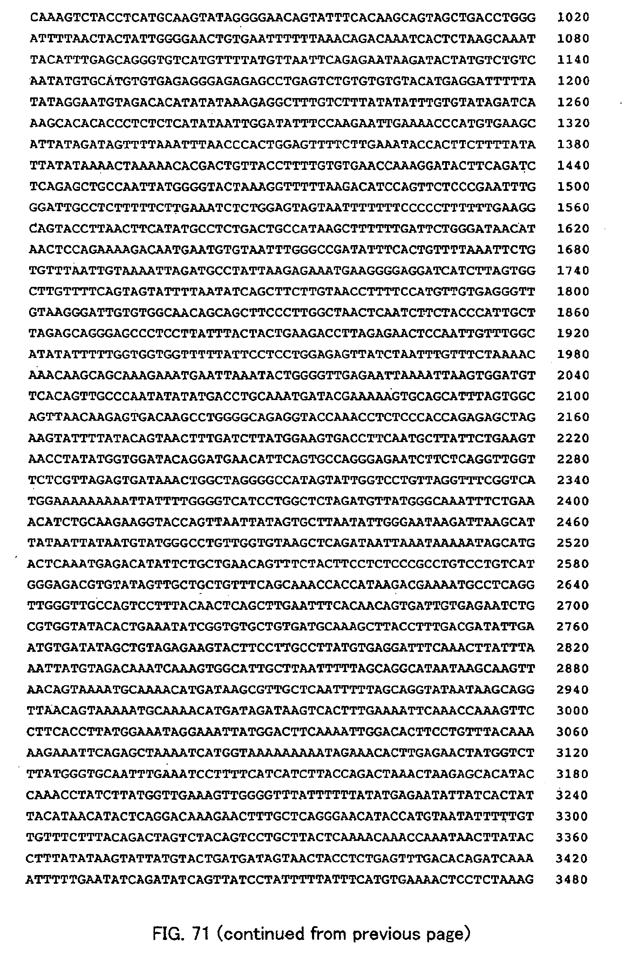

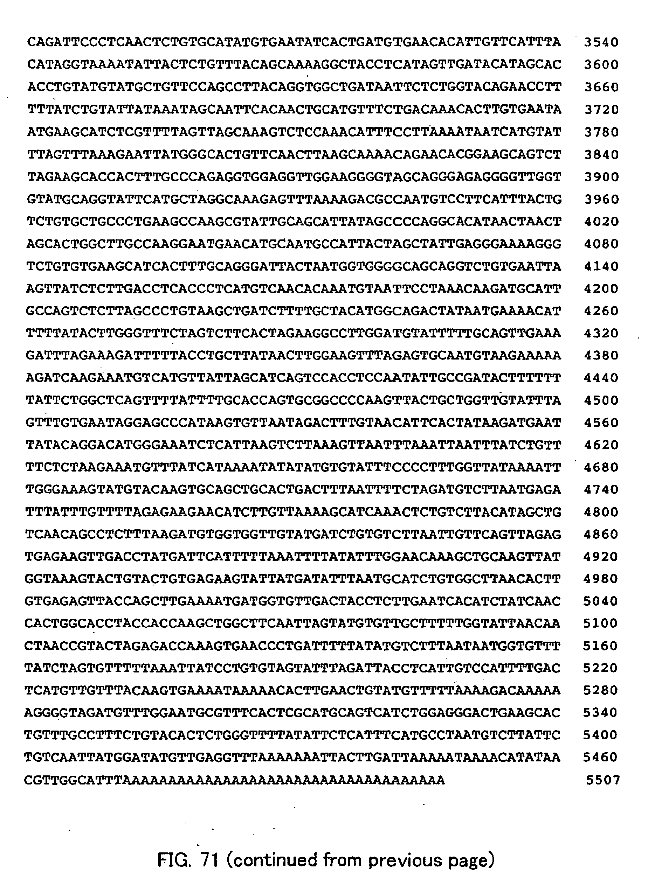

More specifically, a 5'-RACE analysis was carried out with a SMART RACE cDNA amplification kit (manufactured by Clontech) to identify the sequence at the 5' side from the probe sequence (229349_at_u133B) used in the Gene chip analysis. First, total RNA was obtained by mixing equal amount of each total RNA prepared from HepG2, HuH6 and Hep3B, human liver cancer cell lines, and a single-stranded cDNA was synthesized according to the method attached to the kit from 1000 ng of the total RNA using a primer LS900 (SEQ ID NO: 262: GGGTTCACTT TGGTCTCTAG TACGG) designed based on the sequence of the human EST (GenBank Accession No. AL039884: SEQ ID NO: 261) used as an origin of the probe sequence. Then, cDNA containing the sequence at the 5' side was amplified by PCR by using the synthesized single-stranded cDNA as a template. More specifically, by using 1.25 µL of the single-stranded cDNA and 5 pmole of LS900 as a PCR primer, PCR reaction was carried out according to the method attached to the kit. The PCR was carried out as follows: denaturation at 94°C for 1 minute, 35 cycles of 98°C for 10 seconds and 68°C for 3 minutes, and incubation at 72°C for 5 minutes. The PCR product of about 5,000 bp was inserted into pGEM-T Easy vector (manufactured by Promega), E. coli DH5α (manufactured by Toyobo) was transformed with the vector by a standard method, and then plasmid DNA was prepared from the obtained transformants. The nucleotide sequence of the gene inserted in the plasmid DNA was analyzed to isolate two clones, clone 11 and clone 18, which have different nucleotide sequence. The nucleotide sequences are shown in SEQ ID NO: 64 and SEQ ID NO: 65, respectively with the sequence of human EST (GenBank Accession No. AL039884) attached to the 3' side. It was found that both of the two types of clones isolated in this study have an open reading frame encoding 250 amino acids (clone 11) or 210 amino acids (clone 18), respectively (Figs. 70 and 71). The amino acid sequences deduced from clone 11 and clone 18 are shown in SEQ ID NOs : 81 and 82, respectively. When the amino acid sequences deduced from the two types of clones obtained this time were compared, clone 11 is longer than clone 18 by 40 amino acids at the N-terminal side, suggesting that the two types of clones isolated in this study may be splicing variants having different exons used at the 5' side. In this way, a novel gene whose expression is elevated specifically in a liver cancer cell was isolated, identified and named K#2.

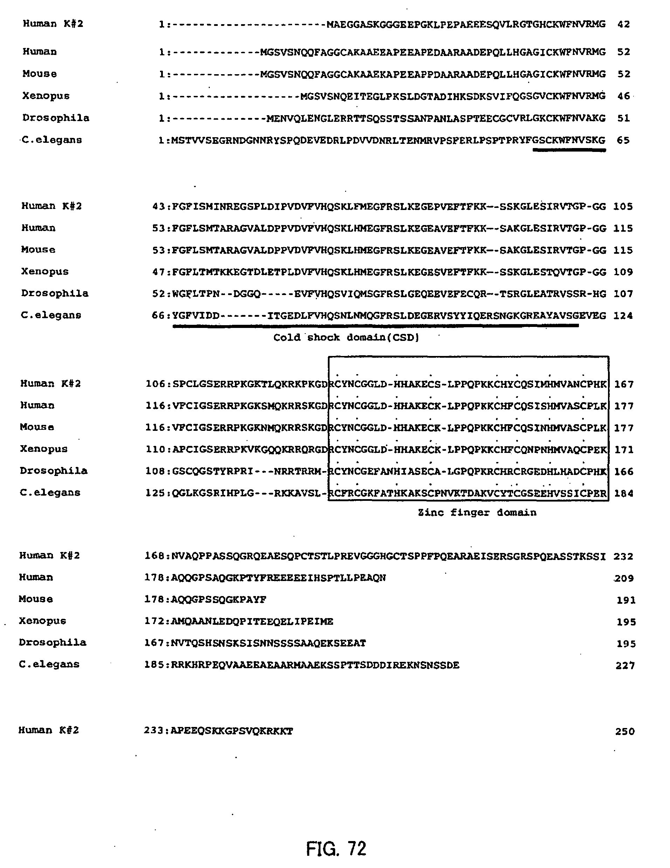

A Blast search was carried out based on the amino acid sequence of K#2 (clone 11) to identify homologous proteins. The novel gene was found to have a homology of 71.8% to human LIN-28 (GenBank No. NM_024674) (SEQ ID NO: 264) and a homology of 33.1% to Caenorhabditis elegans LIN-28 (GenBank No. NM_059880) (SEQ ID NO: 265). Since LIN-28 homologue is a protein also conserved in a higher organism such as mouse and human, as well as Caenorhabditis elegans and Drosophila (Moss, E. G. et al. , Dev. Biol., 258, 432-442, 2003), the amino acid sequences of Xenopus laevis LIN-28 (GenBank No. AF521098) (SEQ ID NO: 266), Drosophila LIN-28 (GenBank No. AF521096) (SEQ ID NO: 267), mouse LIN-28 (GenBank No. NM_145833) (SEQ ID NO: 268) as well as human LIN-28 and Caenorhabditis elegans LIN-28 were compared. It was found that every sequence contains a cold shock domain and a zinc finger domain (Fig. 72), strongly suggesting that K#2 isolated and identified in this study may possibly be a human LIN-28 homologue. Incidentally, it has been revealed that LIN-28 protein plays a role in regulation of cell fate in the developmental stage by binding to mRNA and participating in the translation from mRNA or the stability of mRNA (Moss, E. G. et al., Cell, 88, 637-646, 1997). Accordingly, it is believed that K#2 protein may have a function similar to that of LIN-28. K#2 is predicted to be involved in the regulation in human developmental stage, the development or growth of cancer cells, the replication of viruses such as hepatitis virus or the like.

### 4-2. Production of anti-K#2 antibody

In order to test whether cancer can be detected by using an anti-K#2 antibody, an anti-K#2 antibody was prepared.

As an antigen for immunization against K#2, a recombinant GST fusion protein was prepared using a partial sequence of the amino acids (1-210 aa) of K#2 (clone 18). More specifically, a gene encoding K#2 (1-210 aa) was amplified by the PCR method using K#2 cDNA clone 18 as a template and using primer F (SEQ ID NO: 278) and primer R (SEQ ID NO: 279), and the amplified gene was inserted into pGEM-Te vector (manufactured by Promega). After the nucleotide sequence was confirmed by a standard method, the vector was digested with restriction enzymes EcoRI and NotI, and the digested gene fragment was inserted into pDEST15 (manufactured by Invitrogen), to construct an expression vector pDEST15-K#2.
SEQ ID NO: 278 (F): CACCATGGGATTTGGATTCATCTCCATGAT
SEQ ID NO: 279 (R): TGTCTTTTTCCTTTTTTGAACTGAAGGCCCC

Then, by using the expression vector pDEST15-K#2, a GST-binding antigen protein (k#2 (1-210 aa)) was prepared in the same manner as described above. In order to produce a K#2 polyclonal antibody, a rabbit was immunized with the k#2 (1-210 aa)-GST fusion protein, and the antiserum was prepared. More specifically, in the initial immunization the K#2_GST fusion protein suspended in PBS (100 µg/0.5 mL/rabbit) was mixed with 0.5 mL of Freund's complete adjuvant (DIFCO) to obtain an emulsion and administered to a New Zealand white rabbit (10 weeks of age, female, purchased from Clea Japan) by subcutaneous injection. Thereafter, an emulsion obtained by mixing the K#2_GST fusion protein suspended in PBS (100 µg/0.5 mL/rabbit) with 0.5 mL of Freund's incomplete adjuvant was administered by subcutaneous injection at two weeks intervals for a total of 4 times immunization. Blood was collected before each immunization and after the third and fourth immunization, and an increase in the antibody titer against the K#2_GST fusion protein was measured by ELISA. After an increase in the antibody titer was observed, whole blood was collected, and K#2-immune rabbit antiserum was obtained, which was used as a K#2 polyclonal antibody.

### 4-3. Detection of K#2 protein molecule using anti-K#2 polyclonal antibody

In order to test the reactivity of the K#2-immune rabbit antiserum prepared as described above, K#2 was measured in cell lysates from a cell line forcibly expressing K#2 and a variety of cancer cell lines.

An animal cell expression vector for expressing K#2 was prepared by inserting the above-mentioned cDNA encoding K#2 into pcDNA3.1 to construct a K#2 gene expression vector pcDNA3.1-K#2. Then, 1 µg of the expression vector pcDNA3.1-K#2 was introduced into 2 x 10⁵ HEK293 cells by using FuGene 6 reagent (manufactured by Roche Diagnostics) and the cells were made to transiently express K#2. The cells at three days after introduction of the expression vector were collected, and the cultured cells were solubilized in RIPA buffer (150 mM sodium chloride, 1% NP-40, 0.5% deoxycholic acid, 0.1% SDS, 50 mM tris hydroxyaminomethane hydrochloride (pH 8.0)), whereby a cell lysate was prepared. Each lysate in an amount corresponding to 3 mg of protein was loaded on a SDS-polyacrylamide gel and the protein was separated by SDS-PAGE and transferred to Hybond-P (manufactured by Amersham Biosciences). Then, K#2 was detected by ECL plus (manufactured by Amersham Biosciences) using the anti-K#2 polyclonal antibody (1:5,000 dilution of antiserum) as a primary antibody and an HRP-labeled anti-rabbit IgG antibody (manufactured by Jackson) as a secondary antibody. A band was detected, which is considered to be K#2.

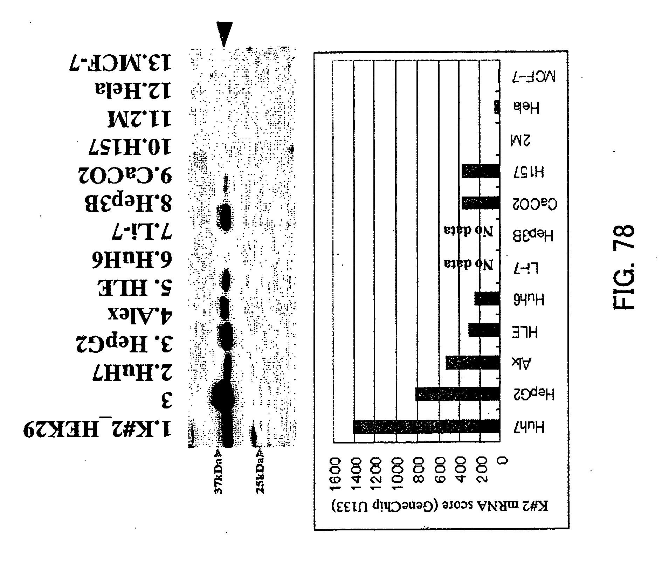

In parallel, the cell lysates from a variety of cancer cell lines was analyzed by Western blot analysis in the same way. The result agrees with the analysis results of GeneChip U133, and a band with a molecular weight of about 27 kDa, which is considered to be the full-length K#2, was successfully detected only in a cell line which showed a high mRNA expression score (Fig. 78). No GeneChip data was available for Li-7 cell and Hep3B cell.

### 4-4. Expression analysis of K#2 protein in liver cancer tissue using anti-K#2 polyclonal antibody

Extract from K#2 cancer tissue was analyzed by Western blot analysis using the anti-K#2 polyclonal antibody. Human tissue extract was prepared by adding RIPA buffer (150 mM sodium chloride, 1% NP-40, 0.5% deoxycholic acid, 0.1% SDS, 50 mM tris hydroxyaminomethane hydrochloride (pH 8.0)) to tissue sections, followed by homogenization by sonication, and then collecting the supernatant fraction by centrifugation. The protein concentration was determined by the Bradford method for each extract sample, and the sample was adjusted at a concentration of 4 mg/mL. Then, the sample was mixed with an equal amount of SDS-sample buffer and heated at 95°C for 5 minutes. Ten mg of each of the extract samples was applied to 15% polyacrylamide gel and subjected to SDS-PAGE.

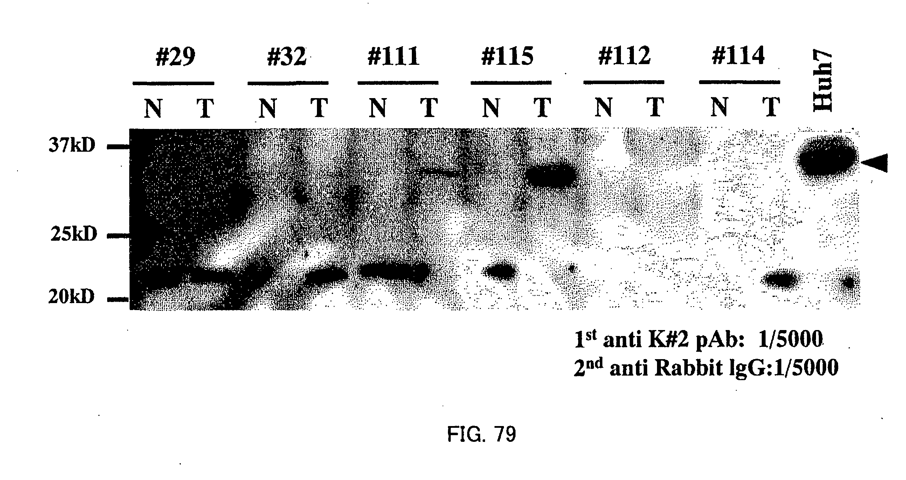

The sample was analyzed by Western blot analysis using the anti-K#2 polyclonal antibody in the same manner as described above. A specific band near K#2 was specifically detected in the extract from the cancerous part (Fig. 79).

From the above results, it was found that the TEG23: K#2 molecule is highly expressed specifically in the cancerous part even at the protein level and secreted in a cancer cell line, suggesting that the molecule is useful for diagnosis of cancer with an anti-K#2 antibody using tissue and serum specimens.

### Example 5

### Production of anti-TEG1: C20orf102 monoclonal antibody

In order to determine whether cancer can be detected using an anti-C20orf102 antibody, an anti-C20orf102 monoclonal antibody was prepared.

### 5-1. Isolation of C20orf102 cDNA

In order to express C20orf102, C20orf102 cDNA was first isolated as follows. A single-stranded cDNA was prepared from lung adenocarcinoma tissue according to the above-mentioned method. Then, PCR was carried out using the single-stranded cDNA as a template and primers F (SEQ ID NO: 269) and R (SEQ ID NO: 270) with a restriction enzyme site for EcoRI or XhoI. A band near about 615 bp was successfully detected, which agrees with that of the predicted sequence of C20orf102. Advantage HF Polymerase Mix (manufactured by Clontech), Advantage HF PCR buffer, 200 µM deoxynucleotide triphosphate and 0.2 µM primer were used as the enzymes and reagents for PCR, and PCR (35 cycles of 94°C for 30 seconds, 68°C for 30 seconds and 72°C for 3 minutes) was carried out using 1 µL of the cDNA as a template. The specifically amplified fragment obtained by PCR was inserted into pGEM-T Easy vector (manufactured by Promega) using a DNA ligation kit (manufactured by Takara). The nucleotide sequence was checked by a standard method, and the isolated cDNA was found to correspond to C20orf102. The primers F and R were designed to hybridize with the 5' end and 3' end of C20orf102 gene (GenBank: NM_080607), respectively.
SEQ ID NO: 269 (F): CGAATTCATGGGGGCCCCGCTCGCCGTAGC
SEQ ID NO: 270 (R): CCTCGAGGAGGCTGCAGGCCTCCTGGTCCA

### 5-2. Preparation of antigen for immunization against C20orf102

The pGEM-T Easy vector incorporated with the PCR product was transformed into a competent cell XL-1 Blue (manufactured by Stratagene), and the vector bearing the PCR product was selected by color selection using 5-bromo-4-chloro-3-indolyl-β-galactopyranoside (X-gal). As for the transformation, 10 µL of a ligation reaction product was added to the competent cells, the mixture was cooled on ice for 30 minutes, and the cells were subjected to heat shock at 42°C for 45 seconds and cooled on ice for 2 minutes thereby inducing transformation. Further, in order to allow an antibiotic resistance gene to be expressed, 900 µL of LB medium without antibiotics was added and the mixture was mildly stirred at 37°C for 30 minutes. Then, the cells were collected by centrifugation and plated on an LB plate containing ampicillin to which 20 µL of 20 mg/mL X-gal had been sprayed, and the cultivation was carried out at 37°C for 16 hours. Among the colonies grown on the plate, 5 colonies without developing color (i.e. the PCR product is expected to be incorporated into the vector) were selected, and the cells were grown in 5 mL of LB medium containing ampicillin at a final concentration of 100 µg/mL at 37°C for 16 hours with vigorous stirring. Plasmid DNA was collected by phenol/chloroform extraction from a portion of the grown cells, then 0.5 µL of EcoRI (8 U/µL), 2 µL of a 10×H buffer and 7.5 µL of distilled water were added thereto, and the plasmid was digested at 37°C for 1 hour. The size of the digested fragment was confirmed to be the same as that of the PCR product by electrophoresis on a 0.8% agarose gel. The plasmid DNA into which C20orf102 gene is considered to have been inserted was collected using a Quantum Prep Plasmid Miniprep Kit (manufactured by BioRad). The plasmid was eluted with distilled water. After the nucleotide sequence was determined by a standard method, DNA was digested with restriction enzymes EcoRI and XhoI. Then the digested fragment was inserted into an E. coli protein expression vector, pET41a vector (manufactured by Novagen). The gene incorporated into pET41 is translated as a GST fusion protein.

pET41 was digested with restriction enzymes (EcoRI and XhoI), subjected to electrophoresis and purified by using a Qiaquick Gel Extraction Kit. A fragment having the sequence of C20orf102 was amplified by pGEM-T Easy and was inserted into pET41 using a DNA ligation kit.

To 4 µL of the C20orf102 fragment purified from pGEM-T Easy, 5 µL of a ligation buffer and 1 µL of pET41 were added and the mixture was incubated at 16°C for 30 minutes.

After finishing the ligation reaction, the plasmid DNA was transformed into XL-1 Blue, the cells were grown with shaking in LB medium containing kanamycin for 16 hours. From the grown E. coli cells, the plasmid was purified using a Quantum Prep Plasmid Miniprep Kit. In order to confirm the insertion of C20orf102 into pET41, the plasmid was sequenced with a primer pair (SEQ ID NOs : 271 and 272) corresponding to the sequence of pET.
SEQ ID NO: 271: TTCGAACGCCAGCACATGGAC
SEQ ID NO: 272: GCTAGTTATTGCTCAGCGGTG

The pET41 vector bearing C20orf102 was transformed into a competent cell of BL21 Codon PLUS RIL (manufactured by Novagen) having T7 promoter.

Transformation was carried out according to the following procedure. To 100 µL of BL21 Codon PLUS RIL, 1 µL of pET-C20orf102-FL was added at a concentration of 1 µg/µL and the mixture was cooled on ice for 5 minutes. Then, the mixture was placed in a thermostat bath at 42°C for 20 seconds, and the cells were subjected to heat shock. Then, the mixture was cooled on ice for 2 minutes, and 900 µL of LB without antibiotics was added. Then, the cells were incubated at 37°C for 10 minutes, and centrifuged (1000 x g, 5 min). After the supernatant was discarded, the competent cells were resuspended and plated on an LB plate containing kanamycin, and then selection culture was carried out at 37°C for 16 hours.

The GST fusion protein of C20orf102 expressed in E. coli was purified by affinity purification utilizing the binding of GST to glutathione. First, the culture solution was centrifuged at 6000 x g for 10 minutes at 4°C to collect E. coli cells. A cell lysis buffer (50 mM sodium chloride, 1 mM EDTA, 1 mM dithiothreitol (DTT), 50 mM tris hydroxyaminomethane hydrochloride, pH 8.0) was added and the mixture was sonicated on ice. Triton X-100 was added at a final concentration of 1%, and centrifuged at 13400 x g for 45 minutes at 4°C to collect the supernatant. To the supernatant, 500 µL of glutathione sepharose (manufactured by Amersham Biosciences) was added, and end-over-end mixing was carried out at 4°C for 1 hour to allow the GST-C20orf102 fusion protein to be adsorbed.

Glutathione sepharose was collected by centrifugation (3000 x g, 4°C, 5 min) and washed with 10 mL of PBS-T (PBS containing 0.5% Triton X-100). An elution buffer (50 mM reduced glutathione, 200 mM sodium chloride, 1 mM EDTA, 1 mM DTT, 200 mM Tris-HCl, pH 8.0) was added and mixed end-over-end at 4°C for 1 hour to elute the GST fusion protein. The glutathione sepharose was removed by centrifugation (3000 x g, 4°C, 5 min) to obtain a purified protein of GST-fused C20orf102. A PBS solution of the fusion protein was prepared using a PD-10 column (manufactured by Amersham Biosciences), and the protein concentration was determined by the Bradford method. The purity of the protein was assayed by SDS-PAGE to confirm that the obtained protein satisfies the amount and purity required for immunization. The protein was used as an immunogen to produce a monoclonal antibody as described below.

### 5-3. Production of C20orf102 monoclonal antibody

A purified product (E. coli-expressed protein) of the GST fusion protein of the full-length human C20orf102 was used as an immunogen. Mice (BALB/c female at 6 weeks of age) were immunized three times with the immunogen at 50 µg/mouse, and the antibody titer in the serum was assayed. The antibody titer assay was carried out by an immunogen solid-phase ELISA method. A dilution series of the immunized mouse serum pretreated with GST protein to absorb the anti-GST antibody was reacted with the immunogen immobilized on an ELISA plate in an amount of 0.5 µg/well. It was reacted with an HRP-labeled anti-mouse antibody, and then the absorbance at 450 nm was measured for the developed color upon addition of a substrate.

Mice showing an increase in the antibody titer received a final immunization with the antigen at 25 µg/mouse. Spleen cells were collected at 72 hours after the final immunization, and fused with myeloma cells (P3/NSI-1-Ag4-1) (Kohler, G, Milstein, C: Nature, 256: 495 (1975)). The cells were cultured in HAT selection medium to obtain hybridomas. The culture supernatant of the hybridomas was pre-absorbed with GST protein and assayed by an immunogen solid-phase ELISA to primarily select those reacting with C20orf102 (actual product). The immunogen ELISA positive hybridoma was assayed for the specificity of the antibody by Western blotting using a protein extract solution from COS7 cells forcibly expressing C20orf102. A positive hybridoma was cloned by a limiting dilution method, whereby a monoclonal antibody-producing cell line was established. The antibody- producing hybridoma was inoculated into BALB/c mice, and mouse ascites was obtained. The monoclonal antibody in the ascites was purified by an ammonium sulfate precipitation method to obtain a purified antibody preparation. In this way, an anti-C20orf102 antibody H9615 was prepared.

### Example 6

### Detection of C20orf102 protein molecule using anti-C20orf102 monoclonal antibody

In order to test the reactivity of the anti-C20orf102 monoclonal antibody H9615 prepared as described above, C20orf102 protein was measured in cell lysates of a cell line forcibly expressing C20orf102 and cell lysates of a variety of cancer cell lines.

First, the reactivity of the anti-C20orf102 monoclonal antibody H9615 was examined by Western blot analysis using a COS7 cell line forcibly expressing C20orf102. A C20orf102 gene expression vector was constructed by inserting the C20orf102-encoding cDNA into pcDNA4Mys-His (manufactured by Invitrogen) and used as an animal cell expression vector. More specifically, 1 µg of the expression vector was introduced into 5 x 10⁴ COS7 cells using FuGene 6 reagent (manufactured by Roche Diagnostics) and the cells were made to transiently express C20orf102. Three days after introduction of the expression vector, the cells were collected and were solubilized with RIPA buffer (150 mM sodium chloride, 1% NP-40, 0. 5% deoxycholic acid, 0.1% SDS, 50 mM tris hydroxyaminomethane hydrochloride (pH 8.0)) to prepare a cell lysate. Each lysate in an amount corresponding to 10 µg of proteins was loaded on a SDS-polyacrylamide gel and the protein was separated by SDS-PAGE and transferred to Hybond-P (manufactured by Amersham Biosciences). The protein was detected by ECL plus (manufactured by Amersham Biosciences) using the anti-C20orf102 monoclonal antibody H9615 (1 µg/mL) and HRP-labeled anti-mouse IgG antibody (manufactured by Jackson) as a secondary antibody. A specific band was detected near the theoretical molecular weight of 22.5 kDa, which is considered to be the C20orf102 protein.

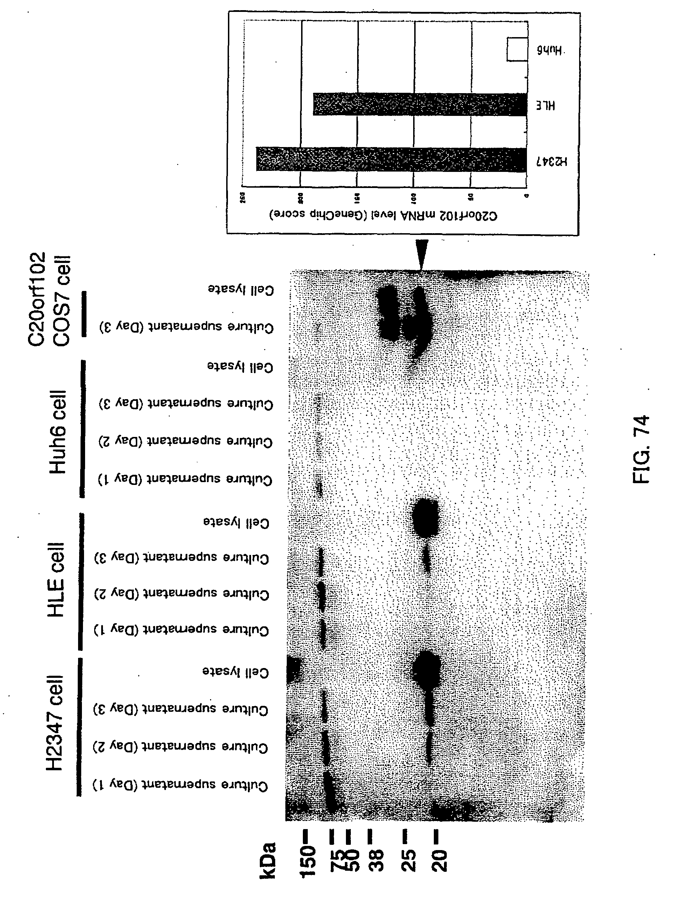

In parallel, the cell lysates from a variety of cancer cell lines were assayed by Western blot analysis in the same way as above. The result was consistent with the analysis results from GeneChip U133, namely, a band which is considered to be the full-length C20orf102 with a molecular weight of about 22.5 kDa was successfully detected only in the cell lines which showed a high mRMA expression score (Fig. 74).

Further, since C20orf102 gene has a secretory signal in the predicted-sequence, it was examined whether a secretory form of C20orf102 can be detected in the culture supernatant of a cancer cell line expressing C20orf102. A band with the same molecular weight as that in the culture supernatant of the cell line forcibly expressing C20orf102 was also detected in the culture supernatant of the cancer cell lines overexpressing C20orf102 by the anti-C20orf102 monoclonal antibody (Fig. 74).

From the above results, it was found that the anti-C20orf102 monoclonal antibody H9615 can specifically detect C20orf102 and that the mRNA expression level from the GeneChip analysis agrees with the C20orf102 protein expression level. Further, from the study using the anti-C20orf102 monoclonal antibody, it was found that secretory C20orf102 is present in the culture supernatant of C20orf102-expressing cells, strongly suggesting that the presence or absence of cancer cells may possibly be determined by detecting the secretory form of C20orf102.

### Example 7

### Expression analysis of C20orf102 protein in lung adenocarcinoma tissue using anti-C20orf102 monoclonal antibody

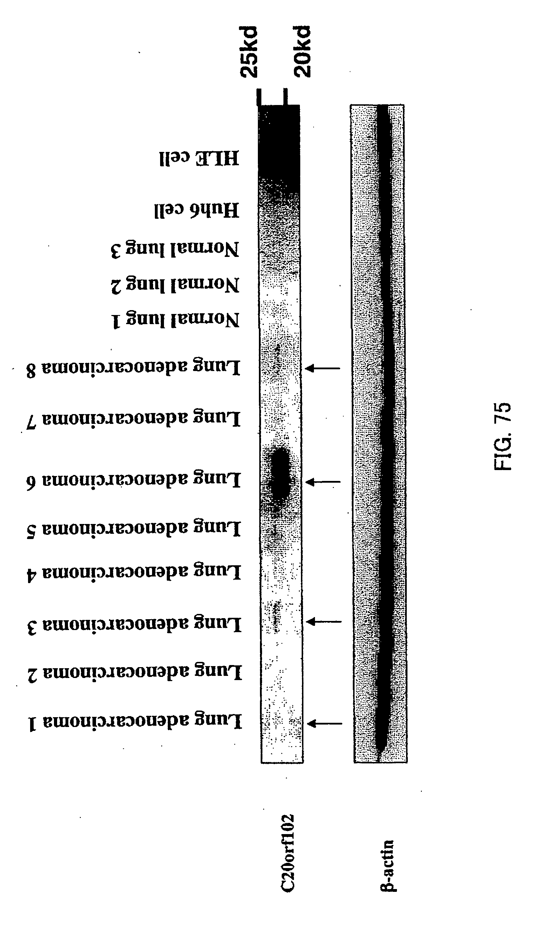

Tissue extract from lung adenocarcinoma was analyzed by Western blot analysis using the anti- C20orf102 monoclonal antibody H9615. Human tissue extract was prepared by adding RIPA buffer (150 mM sodium chloride, 1% NP-40, 0.5% deoxycholic acid, 0.1% SDS, 50 mM tris hydroxyaminomethane hydrochloride (pH 8.0)) to tissue fragments, followed by homogenization by sonication, and then collecting the supernatant fraction by centrifugation. The protein concentration was determined by the Bradford method for each extract sample, and the sample was adjusted at a concentration of 4 mg/mL. Then, the sample was mixed with an equal amount of SDS-sample buffer and heated at 95°C for 5 minutes. Ten µg of each of the extract samples was applied to a 15% polyacrylamide gel and subjected to SDS-PAGE. The sample was analyzed by Western blot analysis with the anti-C20orf102 monoclonal antibody H9615 in the same manner as described above. A specific band near about 22.5 kDa was detected specifically in the cancerous part (Fig. 75).

From the above results, it was found that the TEG1: C20orf102 molecule is highly expressed specifically in the cancerous part even at the protein level and is secreted in a cancer cell line, suggesting that the molecule is useful for diagnosis of cancer by a monoclonal antibody using tissue and serum specimens.

### Example 8

### Production of anti-OK/SW-CL..30 antibody

As for TEG6: OK/SW-CL..30, in order to determine whether cancer can be detected by using an anti-OK/SW-CL..30 antibody, an anti-OK/SW-CL..30 antibody was prepared.

### 8-1. Isolation of hNotum cDNA

From a public database (UCSC and GenBank) search, it was found that the cDNA sequence of OK/SW-CL..30 is a partial sequence, and in fact, a putative protein LOC147111 (GenBank: NM_178493, SEQ ID NOs: 273 and 274) containing the entire sequence of OK/SW-CL..30 and further contains the 5' region may represent a full-length ORF gene. The sequence contains a signal sequence and has a homology of 42.7% to fly Notum (No_168642), therefore, it is named hNotum as a novel gene and further analyzed. First, hNotum cDNA was isolated as follows. A single-stranded cDNA was prepared from a HepG2 cell according to the above-mentioned method. Then, PCR was carried out using the prepared single-stranded cDNA as a template and primers WT164 (SEQ ID NO: 275) and LS746 (SEQ ID NO: 276). A band near about 1.5 kbp was successfully detected, which agrees with the predicted sequence of hNotum. In the PCR method, DMSO was added to a reaction mixture prepared according to the protocol of a KOD plus kit (manufactured by TOYOBO) in an amount corresponding to 5% of the total amount of the reaction mixture. The reaction consisted of an initial denaturation at 95°C for 2 minutes and 35 cycles of 94°C for 15 seconds and 68°C for 90 seconds. The specifically amplified fragment obtained by PCR was inserted into pENTR (manufactured by Invitrogen) by the TOPO cloning method, and the nucleotide sequence was checked by a standard method. The isolated cDNA was confirmed to be hNotum.

The primers WT164 and LS746 were designed to hybridize with the 5' end and 3' end of hNotum gene (GenBank: NM_178493), respectively.
SEQ ID NO: 275 (WT164): CACCGAATTCATGGGCCGAGGGGTGCGCGTG
SEQ ID NO: 276 (LS746): CTCGAGGCTTCCGTTGCTCAGCATCCCCAG

### 8-2. Preparation of antigen for immunization against hNotum

To use as an antigen for immunization against hNotum, a recombinant protein was prepared as a GST-binding protein using a partial sequence of the amino acids (143 aa to 496 aa) of hNotum. More specifically, a gene encoding hNotum (from 143 aa to 496 aa) was amplified by PCR using the above-mentioned hNotum cDNA as a template and primers LS695 (SEQ ID NO: 277) and LS746 (SEQ ID NO: 276), and the amplified gene was inserted into pGEM-T Easy vector (manufactured by Promega). After the nucleotide sequence was checked by a standard method, the vector was digested with restriction enzymes EcoRI and XhoI, and the digested fragment was inserted into pET41a vector (manufactured by Novagen) to construct an expression vector.
SEQ ID NO: 277 (LS695): GAATTCATGCGGCGCCTCATGAGCTCCCGGGA

A GST fusion antigen protein (containing hNotum from 143 aa to 496 aa) was prepared and used for immunizing mice to produce a monoclonal antibody in the same manner as described above, whereby an hNotum monoclonal antibody H9541 was prepared.

### Example 9

### Detection of hNotum protein molecule using anti-hNotum antibody

In order to test reactivity of the produced monoclonal antibody, hNotum was measured in cell lysates from a cell line forcibly expressing hNotum and a variety of cancer cell lines. A vector obtained by inserting the antigen region (from 143 aa to 496 aa) used in the above into pcDNA4 was used as a control. The expected molecular weight is 39.9 kDa. Western blot analysis was carried out in the same manner as above, except that the primary antibody H9541 was used at a final concentration of 100 µg/mL.

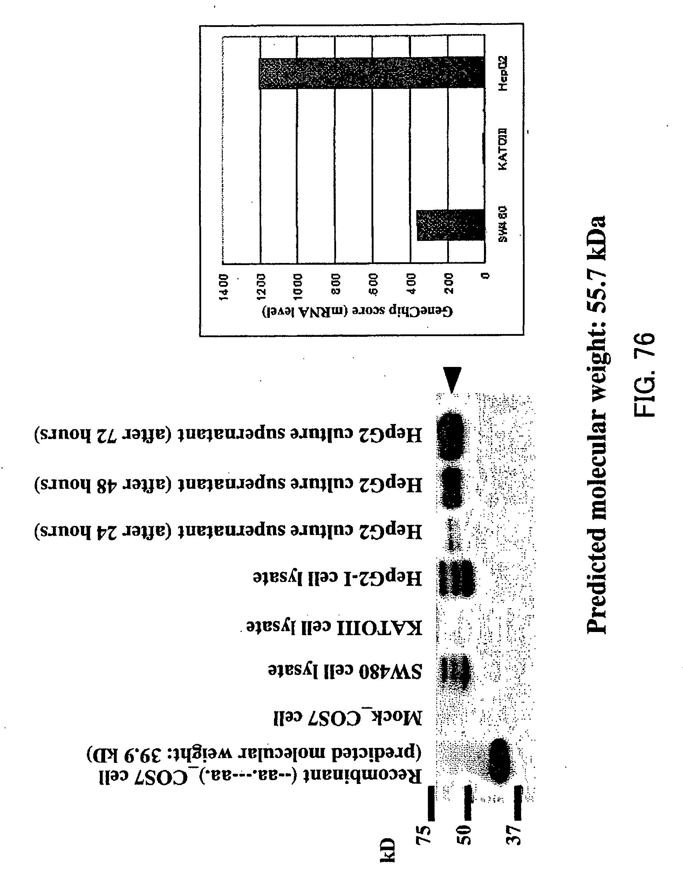

As shown in Fig. 76, a specific band was detected near the position of the 37 kDa marker, which is considered to be hNotum (from 143 aa to 496 aa).

The cell lysates from a variety of cancer cell lines were analyzed by Western blot analysis in the same manner. The result was consistent with the analysis results from GeneChip U133, namely, a band which is considered to be the full-length hNotum with a molecular weight of about 55 kDa was successfully detected only in the cell line which showed a high mRNA expression score (Fig. 76).

Further, since hNotum gene has a secretory signal in the predicted sequence, it was examined whether a secretory form of hNotum can be detected in the culture supernatant of the cancer cell line expressing hNotum. A band with the same molecular weight as that in the culture supernatant of the cell line forcibly expressing hNotum was also detected by the anti-hNotum antibody in the culture supernatant of the cancer cell lines overexpressing hNotum (Fig. 76).

From the above results, it was found that the hNotum monoclonal antibody H9541 can specifically detect hNotum and that the mRNA expression level from GeneChip analysis agrees with the hNotum protein expression level. Further, from the study using the anti-hNotum antibody, it was found that secretory form of hNotum is present in the culture supernatant of hNotum-expressing cells, strongly suggesting that the presence or absence of cancer cells can be determined by detecting secretory form of hNotum.

### Example 10

### Expression analysis of hNotum protein in liver cancer tissue using hNotum antibody

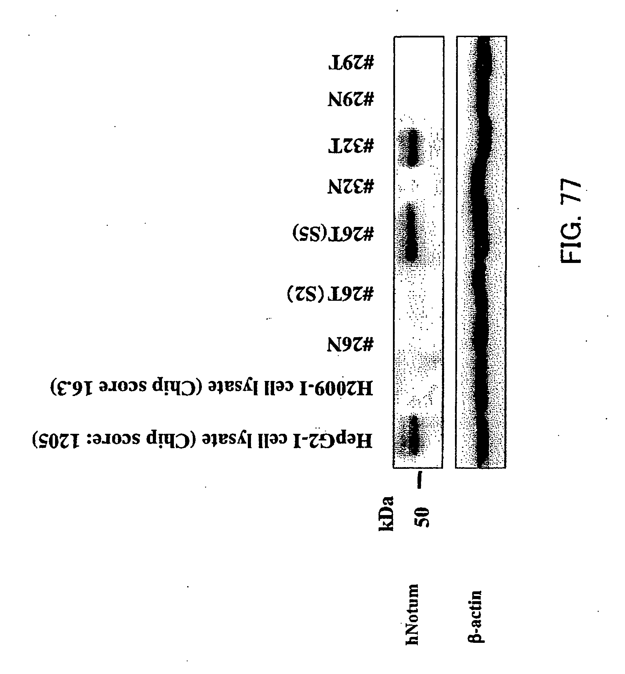

Tissue extract from liver cancer was analyzed by Western blot analysis using the anti-hNotum antibody. The Western blot analysis was carried out with the hNotum antibody in the same manner as described above. A specific band near hNotum was detected specifically in the cancerous part (Fig. 77). Two samples were positive out of three samples tested. In addition, in Sample #26 containing hepatocellular carcinoma tissues obtained from two sites (S2 and S5) of the same patient, one tissue was positive for hNotum.

From the above results, it was found that the TEG6: hNotum (OK/SW-CL..30) molecule is highly expressed specifically in the cancerous part even at the protein level and is secreted in a cancer cell line, suggesting that the molecule is useful in diagnosis of cancer with a monoclonal antibody using tissue and serum specimens.

### Example 11

### 11-1. Production of anti-KIAA1359 antibody

As for TEG37: KIAA1359, in order to determine whether cancer can be detected by using an anti-KIAA1359 antibody, an anti-KIAA1359 antibody was prepared. More specifically, a peptide was synthesized by a standard method with a partial sequence of the amino acids (from 76 aa to 88 aa) of KIAA1359 to be used an antigen for immunization against KIAA1359. C:cysteine residue was added at the N-terminus of the peptide and the peptide was conjugated to Keyhole limpet hemocyanin (KLH), which was used as an immunogen. A monoclonal antibody was produced in the same manner as described above and a monoclonal antibody A8409A was successfully isolated. Peptide sequence: PEAETRGAKRISPA (SEQ ID NO: 280)

### 11-2. Isolation of KIAA1359 cDNA

In order to express KIAA1359, KIAA1359 cDNA was first isolated as follows. A single-stranded cDNA was prepared from MKN74 cell expressing KIAA1359 according to the above-mentioned method. Then, PCR was carried out using the prepared single-stranded cDNA as a template and primers F (SEQ ID NO: 281) and R (SEQ ID NO: 282). A band near about 1.6 kbp was successfully detected, which agrees with that of the predicted sequence of KIAA1359. In the PCR method, a reaction mixture was prepared according to the protocol of an Advantage HF2 kit (manufactured by Clontech). The reaction was carried out under the following conditions: an initial denaturation at 95°C for 1 minute and 35 cycles of 94°C for 15 seconds, 63°C for 30 seconds and 68°C for 2 minutes, and a final extension reaction at 68°C for 6 minutes. The specifically amplified fragment obtained by PCR was inserted into pGEM-T Easy (manufactured by Promega) by the TA cloning method, and the nucleotide sequence was checked by a standard method. The isolated cDNA was confirmed to be KIAA1359. Then the cDNA was inserted into pcDNA4/myc-His A (manufactured by Invitrogen), which was used as a KIAA1359 gene expression vector. The primers F and R were designed to hybridize with the 5' end and 3' end of KIAA1359 gene (GenBank: NM_152673), respectively.
SEQ ID NO: 281 (F): GGATCCATGGGCTGTCTCTGGGGTCTGGCTCTGC
SEQ ID NO: 282 (R): CTCGAGGCCTCTCCTGACACGCAGTAAGGAGACC

### 11-3. Detection of KIAA1359 protein molecule using anti-KIAA1359 antibody A8409A

In order to test the reactivity of the anti- KIAA1359 antibody A8409A prepared as above, KIAA1359 was detected in cell lysates from a cell line forcibly expressing KIAA1359 and a variety of cancer cell lines.

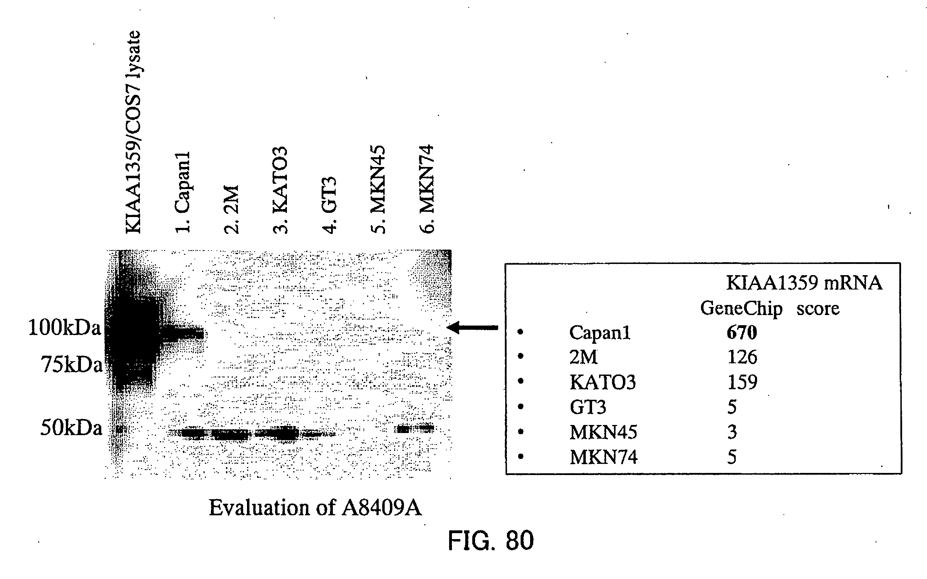

Cell lysates of a variety of cancer cell lines were analyzed by Western blot analysis in the same manner using a lysate of COS7 forcibly expressing KIAA1359 as a control. The A8409A antibody was used at a concentration of 100 µg/mL., a band at about 100 kDa was successfully detected in Capan1 which showed a high score of the GeneChip U133 analysis (Fig. 80). This band is the same as that of KIAA1359 forcibly expressed in the control and is considered to be a KIAA1359 molecule.

### 11-4. Expression analysis of KIAA1359 protein in stomach cancer tissue using anti-KIAA1359 antibody A8409A

Tissue extract from stomach cancer was analyzed by Western blot analysis with the anti-KIAA1359 antibody A8409A. Human tissue extract was prepared by adding RIPA buffer (150 mM sodium chloride, 1% NP-40, 0.5% deoxycholic acid, 0.1% SDS, 50 mM tris hydroxyaminomethane hydrochloride (pH 8.0)) to tissue fragments, followed by homogenization by sonication, and then collecting the supernatant fraction by centrifugation. The protein concentration was determined by the Bradford method for each extract sample, and the sample was adjusted at a concentration of 4 mg/mL. Then, the sample was mixed with an equal amount of SDS-sample buffer and heated at 95°C for 5 minutes. Ten 10 mg of each of the extract samples was applied on a 10% polyacrylamide gel and subjected to SDS-PAGE.

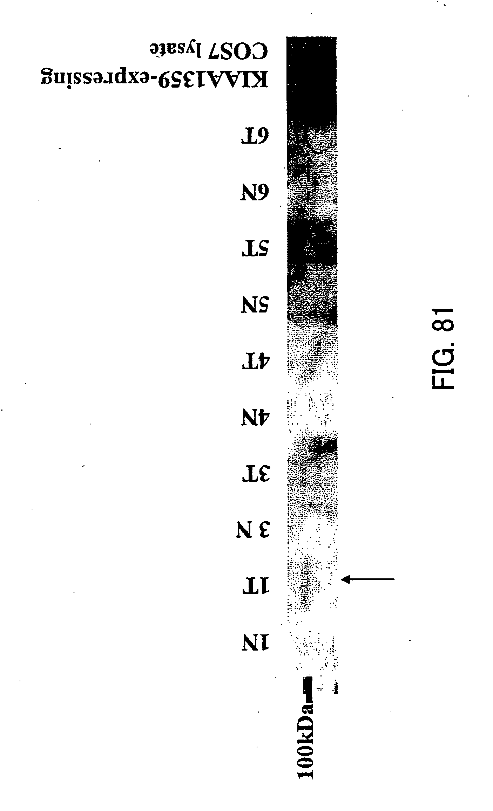

The sample was analyzed by Western blot analysis with the anti-KIAA1359 antibody A8409A in the same manner as described above. A specific band near 100 kDa was detected specifically in the cancerous part (Fig. 81).

From the above results, it was found that the TEG37: KIAA1359 molecule is highly expressed specifically in the cancerous part even at the protein level and is highly expressed in the cancer cell lines, suggesting that the molecule is useful for diagnosis of cancer with a monoclonal antibody using tissue and serum specimens.

### Example 12

### 12-1. Production of anti-PEG10 antibody

It has been suggested that there are two ORFs in TEG60: PEG10; ORF1 which is translated using standard codon usage and ORF2 which is newly translated by the occurrence of frameshift at the stop codon region in the ORF1 according to the report of mouse PEG10 (Shigemoto et al., Nucleic Acids Research, 29, 4079-4088, 2001), and the prediction from the genome sequence of human PEG10 (Ono et al., Genomics, 73, 232-237, 2001). However, the presence of ORF2 of human PEG10 has not been experimentally demonstrated. Accordingly, in order to demonstrate whether or not the frameshift in the ORF2 region actually occurs and whether or not the newly translated region is present in cancer tissue, an anti-PEG10/ORF2 monoclonal antibody was prepared based on the predicted ORF2 amino acid sequence.
ORF2 amino acid sequence (SEQ ID NO: 283)

### 12-2. Isolation of PEG10 cDNA

In order to express PEG10, PEG10 cDNA was first isolated as follows. A single-stranded cDNA was prepared from human fetal liver tissue according to the above-mentioned method. Then, PCR was carried out using the single-stranded cDNA as a template and primers F1 (SEQ ID NO: 284) and R1 (SEQ ID NO: 285). A band near about 2200 kbp was successfully detected, which agrees with that of the predicted sequence of PEG10. In the PCR method, a reaction mixture was prepared according to the protocol of an Advantage 2 cDNA PCR kit (manufactured by Clontech) and the reaction was carried out under the following conditions: an initial denaturation at 94°C for 1 minute, 35 cycles of 94°C for 30 seconds and 68°C for 3 minutes, and a final extension reaction at 68°C for 10 minutes. The specifically amplified fragment obtained by PCR was inserted into pGEM-T Easy (manufactured by Promega) by the TA cloning method, and the nucleotide sequence was checked by a standard method. The isolated cDNA was confirmed to be PEG10.

The primers F1 and R1 were designed to hybridize to the 5' end and 3' end of PEG10 gene (GenBank: AB049834), respectively.
SEQ ID NO: 284 (F1): GGATCCATGACCGAACGAAGAAGGGACGAG
SEQ ID NO: 285 (R1): TCTAGACAGGGTACTGTAAGATGGAGGCGG

### 12-3. Preparation of antigen for immunization against PEG10/ORF2 and Production of monoclonal antibody

To use as an antigen for immunization against PEG10/ORF2, a recombinant protein was prepared as a GST-binding protein using a partial sequence of the amino acids (ORF2/ 51 aa-251 aa).

More specifically, a gene encoding PEG10 (ORF2/ 51 aa-251 aa) was amplified by PCR using the above-mentioned PEG10 cDNA as a template and primers F2 (SEQ ID NO: 286) and R2 (SEQ ID NO: 287), and the amplified gene was inserted into pGEM-T Easy vector (manufactured by Promega). After the nucleotide sequence was confirmed by a standard method, the vector was digested with restriction enzymes BamHI and XhoI, and the digested gene fragment was inserted into pET41c vector (manufactured by Novagen) to construct an expression vector pETc_PEG10_ORF2.
SEQ ID NO: 286 (F2): GGATCCATCTTCCGGGCAGACACACCCT
SEQ ID NO: 287 (R2): CTCGAGTGCCATTTCAGGTTCGGACAGTG

A GST-binding PEG10_ORF2 protein was prepared using the expression vector pETc_PEG10_ORF2 and used to immunize mice to obtain a monoclonal antibody in the same manner as described above, whereby a monoclonal antibody H4128 against PEG10_ORF2 was prepared.

### 12-4. Detection of PEG10 protein molecule using anti-PEG10/ORF2 antibody

In order to test the reactivity of the anti- PEG10/ORF2 antibody H4128 prepared as described above, PEG10 was measured in cell lysates from a cell line forcibly expressing PEG10 and a variety of cancer cell lines.

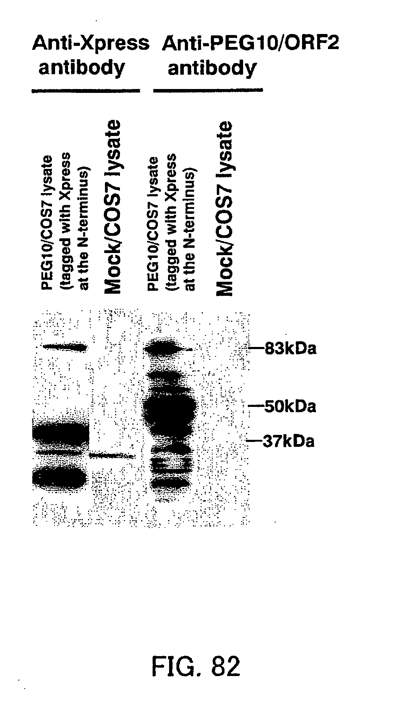

First, the reactivity of the anti-PEG10/ORF2 antibody B0000A was examined by Western blot analysis using a COS7 cell forcibly expressing PEG10. A PEG10 gene expression vector pcDNA4/HisMax_PEG10_Full was obtained by inserting the cDNA encoding the full-length PEG10 into pcDNA4HisMaxC (manufactured by Invitrogen) and was used as an animal cell expression vector. This is a construct comprising an Xpress tag sequence inserted at the N-terminus of PEG10. More specifically, 1 µg of the expression vector pcDNA4/HisMax_PEG10_Full or pcDNA4 (Mock) as a negative control was introduced into 5 x 10⁴ COS7 cells and Hep3B cells using FuGene 6 reagent (manufactured by Roche Diagnostics) and the cells were made to transiently express PEG10. Three days after introduction of the expression vector, the cells were collected and solubilized in RIPA buffer (150 mM sodium chloride, 1% NP-40, 0.5% deoxycholic acid, 0.1% SDS, 50 mM tris hydroxyaminomethane hydrochloride (pH 8.0)) to prepare a cell lysate. Each lysate in an amount corresponding to 5 mg of proteins was loaded on a SDS-polyacrylamide gel and the protein was separated by SDS-PAGE and transferred to Hybond-P (manufactured by Amersham Biosciences). The protein was detected by ECL plus (manufactured by Amersham Biosciences) using the anti-Xpress antibody (1:5,000 dilution) (manufactured by Invitrogen) or the PEG10/ORF2 antibody H4128 (2 µg/mL) as a primary antibody and an HRP-labeled anti-mouse IgG antibody (manufactured by Amersham Biosciences) as a secondary antibody. Specific bands were detected near 83 kDa and 50 kDa, which are considered to be the PEG10 protein were specifically detected by the H4128 antibody, but not in the negative control (Fig. 82). In addition, a band near about 83 kDa was specifically detected in the same manner by the Xpress-tagged antibody labeled at the N-terminus. It is considered that the band near about 83 kDa may represent a full-length protein generated by the occurrence of frameshift downstream from ORF1 and being fused with ORF2. Further, since the amino acid sequence of the ORF2 region which was used as an antigen is not translated from a common frame, it was found by anti-PEG10/ORF2 antibody H4128 that frameshift occurs in human PEG10.

### 12-5. Expression analysis of PEG10 protein in hepatocellular carcinoma tissue using anti-PEG10 antibody H4128

Tissue extract from hepatocellular carcinoma and hepatoblastoma was analyzed by Western blot analysis with the anti-PEG10 antibody. Human tissue extract was prepared by adding RIPA buffer (150 mM sodium chloride, 1% NP-40, 0.5% deoxycholic acid, 0.1% SDS, 50 mM tris hydroxyaminomethane hydrochloride (pH 8.0)) to tissue fragments, followed by homogenization by sonication, and then collecting the supernatant fraction by centrifugation. The protein concentration was determined by the Bradford method for each extract sample, and the sample was adjusted at a concentration of 4 mg/mL. Then, the sample was mixed with an equal amount of SDS-sample buffer and heated at 95°C for 5 minutes. Ten mg of each of the extract samples was applied to a 12% polyacrylamide gel and subjected to SDS-PAGE.

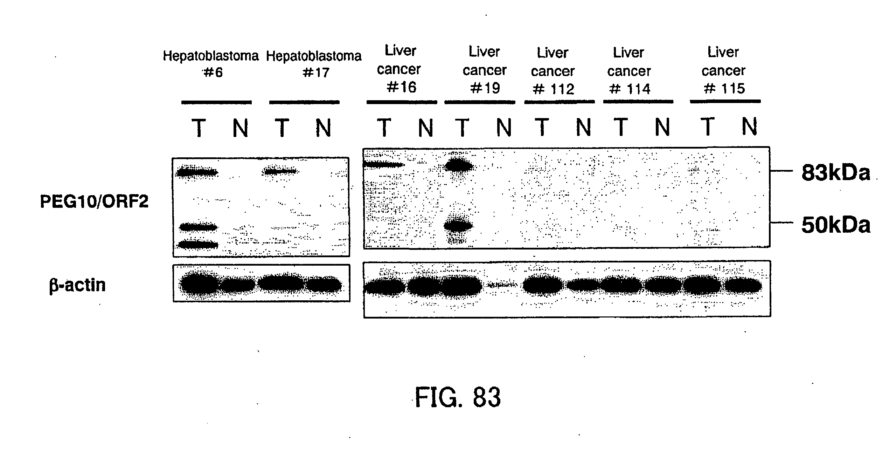

The sample was analyzed by Western blot analysis with the anti-PEG10 antibody H4128 in the same manner as described above. Specific bands near 83 kDa and 50 kDa were detected specifically in the cancerous part (Fig. 83), demonstrating that not only forcibly expressed PEG10, but also PEG10/ORF2 are present in hepatocellular carcinoma and hepatoblastoma tissue.

From the above results, it was found that the TEG60: PEG10 molecule is highly expressed specifically in the cancerous part even at the protein level, suggesting that the molecule is useful in diagnosis of cancer with a monoclonal antibody using tissue and serum specimens.

### Example 13

### 13-1. Preparation of antigen for immunization against DUSP9 and production of monoclonal antibody

As for TEG63: DUSP9, in order to determine whether cancer can be detected by using a monoclonal antibody, an anti-DUSP9 antibody was prepared.

To use as an antigen for immunization against DUSP9, a recombinant protein was prepared as a GST fusion protein using the full-length sequence of DUSP9. More specifically, a gene encoding DUSP9 (385 aa) was amplified by PCR using HepG2 cDNA as a template and primers Ls772 (SEQ ID NO: 288) and Ls773 (SEQ ID NO: 289), and the amplified gene was inserted into pGEM-Te vector (manufactured by Promega). After the nucleotide sequence was confirmed by a standard method, the vector was digested with restriction enzymes EcoRI and HindIII, and the digested fragment was inserted into pET41a vector (manufactured by Novagen) to construct an expression vector pET41a-DUSP9.
SEQ ID NO: 288 (F): GAATTCATGGAGGGTCTGGGCCGCTC
SEQ ID NO: 289 (R): CTCGAGGGTGGGGGCCAGCTCGAAG

A GST-fused DUSP9 (1-385 aa) protein was prepared using the expression vector pET41a-DUSP9 in the same manner as described above and used for immunizing mice to produce a monoclonal antibody, whereby an anti-DUSP9 antibody #8901 was prepared.

### 13-2. Detection of DUSP9 protein molecule using anti- DUSP9 antibody

In order to test the reactivity of the anti-DUSP9 antibody #8901 produced as described above, DUSP9 was measured in cell lysates of from a cell line forcibly expressing DUSP9 and a variety of cancer cell lines.

First, the reactivity of the anti-DUSP9 antibody #8901 was examined by Western blot analysis using a COS7 cell line forcibly expressing DUSP9. A DUSP9 gene expression vector pcDNA4-DUSP9 was constructed by inserting the cDNA encoding DUSP9 into pcDNA4Mys-His (manufactured by Invitrogen) was used as an animal cell expression vector. More specifically, 1 µg of the expression vector pcDNA4-DUSP9 was introduced into 5 x 10⁴ COS7 cells by using FuGene 6 reagent (manufactured by Roche Diagnostics) and the cells were made to transiently express DUSP9. Three days after introduction of the expression vector, the cells were collected and were solubilized in RIPA buffer (150 mM sodium chloride, 1% NP-40, 0.5% deoxycholic acid, 0.1% SDS, 50 mM tris hydroxyaminomethane hydrochloride (pH 8.0)) to prepare a cell lysate. Each lysate in an amount corresponding to 3 mg of proteins was loaded on a SDS-polyacrylamide gel and the protein were separated by SDS-PAGE and transferred to Hybond-P (manufactured by Amersham Biosciences). The protein was detected by ECL plus (manufactured by Amersham Biosciences) using the DUSP9 antibody (1 µg/mL) as a primary antibody and an HRP-labeled anti-mouse IgG antibody (manufactured by Jackson) as a secondary antibody. A specific band was detected near about 42 kDa, which is considered to be the DUSP9 protein.

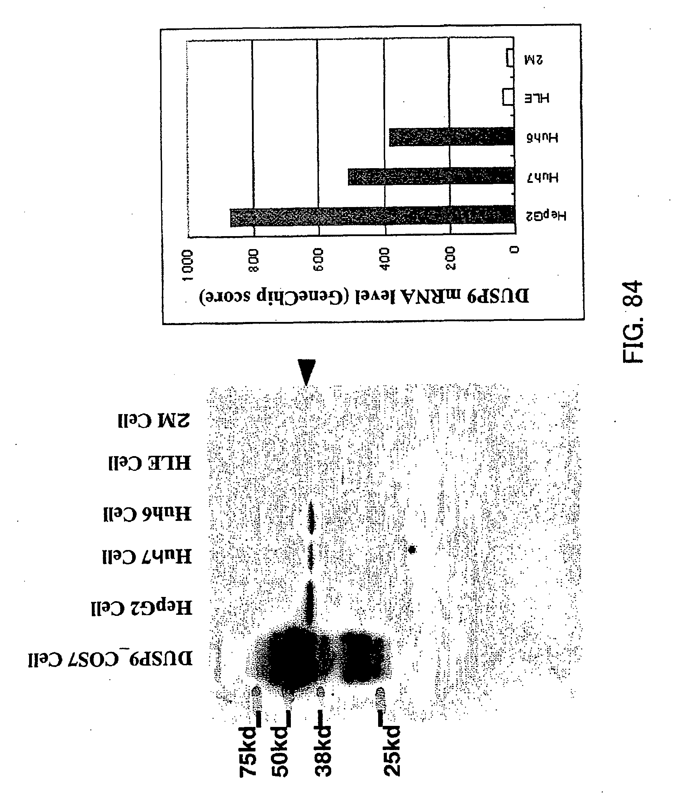

In parallel, Western blot analysis was carried out for the cell lysates from a variety of cancer cell lines in the same manner. The result was consistent with the analysis results from GeneChip U133, namely, a band which is considered to be the full-length DUSP9 with a molecular weight of about 42 kDa was specifically and successfully detected only in the cell lines which showed a high mRNA expression score (Fig. 84).

### 13-3. Expression analysis of DUSP9 protein in hepatocellular carcinoma tissue using anti-DUSP9 antibody

Tissue extract from hepatocellular carcinoma was analyzed by Western blot analysis with the anti-DUSP9 antibody #8901. Human tissue extract was prepared by adding RIPA buffer (150 mM sodium chloride, 1% NP-40, 0.5% deoxycholic acid, 0.1% SDS, 50 mM tris hydroxyaminomethane hydrochloride (pH 8.0)) to tissue fragments, followed by homogenization by sonication, and then collecting the supernatant fraction by centrifugation. The protein concentration was determined by the Bradford method for each extract sample, and the sample was adjusted at a concentration of 4 mg/mL. Then, the sample was mixed with an equal amount of SDS-sample buffer and heated at 95°C for 5 minutes. Ten mg of each of the extract samples was applied to a 12% polyacrylamide gel and subjected to SDS-PAGE.

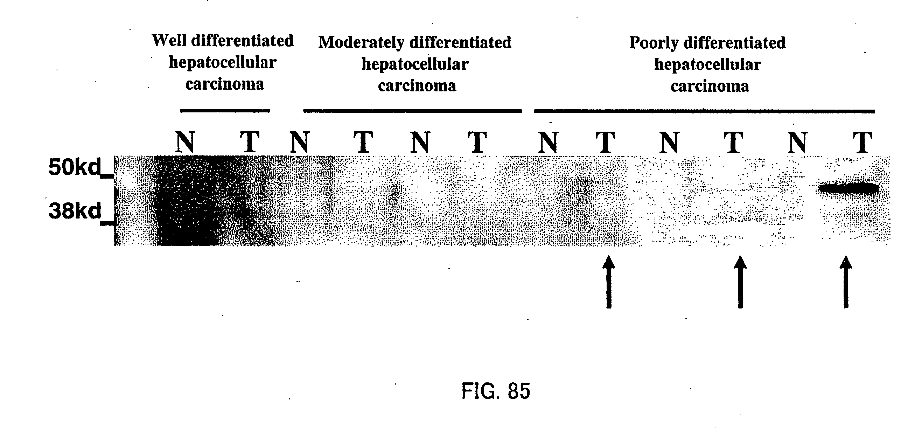

The sample was analyzed by Western blot analysis with the anti-DUSP9 antibody #8901 in the same manner as described above. A specific band near 42 kDa was detected specifically in the cancerous part (Fig. 85). Particularly, the band was detected in 3 cases out of 3 cases of the poorly differentiated hepatocellular carcinoma.

From the above results, it was demonstrated that the TEG63: DUSP9 molecule is highly expressed in the cancerous part. Moreover, its expression is elevated in the cancerous part as well as in the cancer cell lines even at the protein level as detected by the monoclonal antibody, suggesting that the molecule is useful for diagnosis of cancer with a monoclonal antibody using tissue and serum specimens.

### Example 14

### 14-1. Production of anti-cystatin SN antibody

As for TEG47: cystatin SN, in order to determine whether cancer can be detected by using an anti-cystatin SN antibody, an anti-cystatin SN antibody was prepared.

More specifically, a peptide was synthesized by a standard method with a partial sequence of the amino acids (from 60 aa to 75 aa) of cystatin SN to be used as an antigen for immunization against cystatin SN (see GenBank No. NM_001898). C: cysteine residue was added at the N-terminus of the peptide, and the peptide was conjugated to Keyhole limpet hemocyanin (KLH), which was used as an immunogen. A monoclonal antibody was produced in the same manner as described above and a monoclonal antibody was successfully isolated.
Peptide sequence: C-KDDYYRRPLRVLRARQ (SEQ ID NO: 290)

### 14-2. Expression analysis of cystatin SN protein in large bowel cancer tissue using anti-cystatin SN antibody

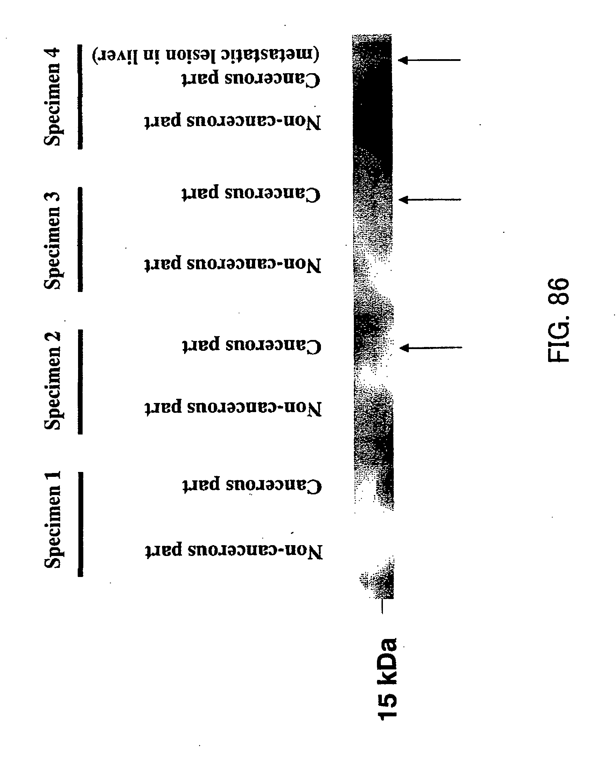

Tissue extract from large bowel cancer was analyzed by Western blot analysis with the anti-cystatin SN antibody. Human tissue extract was extracted in the same manner as described above. The samples was analyzed by Western blot analysis with the anti-cystatin SN antibody (4 µg/mL). A specific band near 15 kDa was detected specifically in the cancerous part (Fig. 86). Since the predicted molecular weight of cystatin SN is about 16 kDa, it was found that the expression of cystatin SN is elevated specifically in the cancerous part.

From the above results, it was found that the TEG47: cystatin SN molecule is highly expressed specifically in the cancerous part at the protein level, suggesting that the molecule is useful for diagnosis of cancer with a monoclonal antibody using tissue and serum specimens.

### Example 15

### Production of anti-SFRP4 antibody

As for TEG56 : SFRP4, in order to determine whether cancer can be detected by using an anti-SFRP4 antibody, an anti-SFRP4 antibody was prepared.

### 15-1. Isolation of SFRP4 cDNA

In order to express SFRP4, SFRP4 cDNA was first isolated as follows. A single-stranded cDNA was prepared from stomach cancer tissue according to the above-mentioned method. Then, PCR was carried out using the single-stranded cDNA as a template and primers GC898 (SEQ ID NO: 291) and GC899 (SEQ ID NO: 292) with a restriction enzyme site for EcoRI or XhoI. A band near about 1000 bp corresponding to a desired size was successfully detected. The enzymes and reagents used in PCR included Advantage HF Polymerase Mix (manufactured by Clontech), Advantage HF PCR buffer, 200 µM deoxynucleotide triphosphate and 0.2 µM primer. PCR was carried out using 1 µL of the cDNA as a template (35 cycles of 94°C for 30 seconds, 68°C for 30 seconds and 72°C for 3 minutes). The specifically amplified fragment obtained by PCR was inserted into pGEM-T Easy vector (manufactured by Promega) using a DNA ligation kit (manufactured by Takara), and the nucleotide sequence was checked by a standard method. The isolated cDNA was found to correspond to SFRP4.

The primers GC898 and GC899 were designed to hybridize with the 5' end and 3' end of SFR4_ORF gene (GenBank: NM_003014), respectively.
SEQ ID NO: 291 (GC898): CGGGATCCATGTTCCTCTCCATCCTAGTGG
SEQ ID NO: 292 (GC899): CGCTCGAGACACTCTTTTCGGGTTTGTTC

### 15-2. Preparation of antigen for immunization against SFRP4

To use as an antigen for immunization against SFRP4, a recombinant protein was prepared as a GST-binding protein using the full-length SFRP4 sequence. More specifically, the above-mentioned SFRP4 sequence was inserted into pGEM-T and digested with restriction enzymes EcoRI and XhoI. Then, the digested gene fragment was inserted into pET41a vector (manufactured by Novagen) to construct an expression vector GST-SFRP4.

Preparation of a GST fusion antigen protein and production of a monoclonal antibody by immunizing mice were carried out in the same manner as described above, whereby an anti-SFRP4 monoclonal antibody A7113 was prepared.

### 15-3. Expression analysis of SFRP4 protein in stomach tissue using anti-SFRP4 antibody

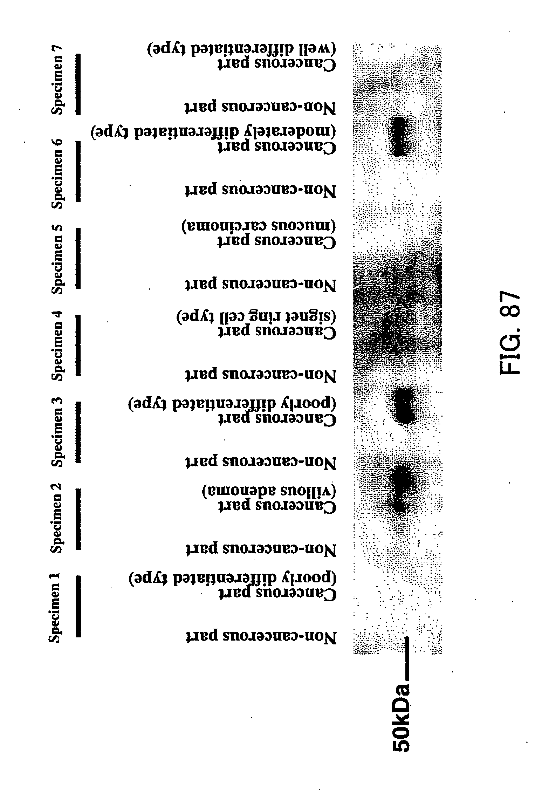

Tissue extract from stomach cancer was analyzed by Western blot analysis with the anti-SFRP4 antibody. The Western blot analysis with the anti-SFRP4 antibody A7113 (40 µg/mL) was carried out in the same manner as described above. A specific band near about 50 kDa was detected in the cancerous part (Fig. 87).

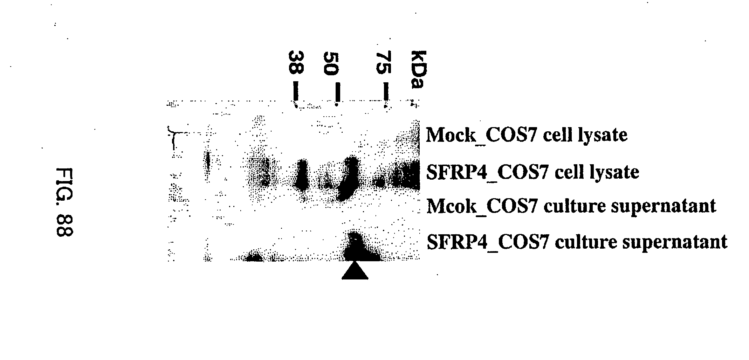

In parallel, the SFRP4 sequence cloned as described above was inserted into an expression vector to prepare an expression vector SFRP4_pcDNA4His-Myc (manufactured by Invitrogen). COS7 cells were forcibly made to express the vector and a lysate of the COS7 cells was analyzed by Western blot analysis with the anti-Myc antibody (1:5,000 dilution, Invitrogen). A band with the same size as the one detected in a clinical specimen was detected (Fig. 88). Accordingly, the band of 50 kDa detected by the anti-SFRP4 monoclonal antibody in the clinical specimen is considered to be SFRP4. It was found that an elevation in the expression of SFRP4 in the cancerous part was specifically detected by the monoclonal antibody. Further, the culture supernatant of COS7 cells forcibly made to express SFRP4 was analyzed and it was found that SFRP4 having a signal sequence is secreted in the culture supernatant (Fig. 88).

From the above results, it was shown that the TEG56 : SFRP4 molecule is highly expressed specifically in the cancerous part even at the protein level, and is secreted in the cancer cell, suggesting that the molecule is useful for diagnosis of cancer with a monoclonal antibody using tissue and serum specimens.

### Industrial Applicability

The gene, protein, and antibody of the present invention can be used in diagnosis and treatment of liver cancer, as well as development of a therapeutic drug of liver cancer.
Furthermore, also described are the following items:
1. A protein encoded by a gene having a nucleotide sequence represented by any of SEQ ID NOs : 1 to 65 or a fragment thereof.
2. A protein encoded by a gene having a nucleotide sequence represented by any of SEQ ID NOs: 1, 2, 28, 29, 30, 31, 32, 51, 52, 60 and 61 or a fragment thereof.
3. A composition for diagnosing or treating lung cancer comprising the protein or fragment thereof according to item 2.
4. A protein encoded by a gene having a nucleotide sequence represented by any of SEQ ID NOs : 3, 4, 5, 6, 7, 8, 9, 10, 11, 12, 13, 22, 23, 24, 25, 26, 27, 33, 34, 35, 36, 37, 38, 39, 40, 41, 42, 53, 54 and 55 or a fragment thereof.
5. A composition for diagnosing or treating stomach cancer comprising the protein or fragment thereof according to item 4.
6. A protein encoded by a gene having a nucleotide sequence represented by any of SEQ ID NOs: 3, 7, 20, 21, 46, 47, 48, 49 and 50 or a fragment thereof.
7. A composition for diagnosing or treating large bowel cancer comprising the protein or fragment thereof according to item 6.
8. A protein encoded by a gene having a nucleotide sequence represented by any of SEQ ID NOs: 14, 15, 16, 17, 18, 19, 43, 44, 45, 56, 57, 58, 59, 62, 63, 64 and 65 or a fragment thereof.
9. A composition for diagnosing or treating liver cancer comprising the protein or fragment thereof according to item 8.
10. The protein or fragment thereof according to item 1, wherein the gene has a nucleotide sequence represented by any of SEQ ID NOs: 1, 9, 10, 14, 20, 22, 24, 25, 26, 27, 28, 29, 32, 38, 39, 40, 44, 51, 52, 53, 54 and 58.
11. The protein or fragment thereof according to item 1, wherein the gene has a nucleotide sequence represented by any of SEQ ID NOs: 1, 9, 10, 14, 20, 22, 24, 25 and 26.
12. The protein or fragment thereof according to item 1. having an amino acid sequence represented by any of SEQ ID NOs: 66 to 123.
13. An antibody recognizing the protein or fragment thereof according to any one of items 1, 2, 4, 6, 8, 10, 11 and 12.
14. A polynucleotide having a nucleotide sequence represented by any of SEQ ID NOs: 1 to 65 or a nucleotide sequence complementary thereto, and a polynucleotide hybridizing under high stringent conditions to any of said polynucleotides.
15. A polynucleotide having a sequence comprising at least 12 consecutive nucleotides of a nucleotide sequence represented by any of SEQ ID NOs: 1 to 65 or a nucleotide sequence complementary thereto, and a polynucleotide having at least 12 nucleotides hybridizing under high stringent conditions to any of said polynucleotides.
16. The polynucleotide according to item 14 or 15, having a nucleotide sequence represented by any of SEQ ID NOs: 1, 2, 28, 29, 30, 31, 32, 51, 52, 60 and 61.
17. A composition for diagnosing or treating lung cancer comprising the polynucleotide according to item 16.
18. The polynucleotide according to item 14 or 15, having a nucleotide sequence represented by any of SEQ ID NOs: 3, 4, 5, 6, 7, 8, 9, 10, 11, 12, 13, 22, 23, 24, 25, 26, 27, 33, 34, 35, 36, 37, 38, 39, 40, 41, 42, 53, 54 and 55.
19. A composition for diagnosing or treating stomach cancer comprising the polynucleotide according to item 18.
20. The polynucleotide according to item 14 or 15, having a nucleotide sequence represented by any of SEQ ID NOs: 3, 7, 20, 21, 46, 47, 48, 49 and 50.
21. A composition for diagnosing or treating large bowel cancer comprising the polynucleotide according to item 20.
22. The polynucleotide according to item 14 or 15, having a nucleotide sequence represented by any of SEQ ID NOs: 14, 15, 16, 17, 18, 19, 43, 44, 45, 56, 57, 58, 59, 62, 63, 64 and 65.
23. A composition for diagnosing or treating lung cancer comprising the polynucleotide according to item 22.
24. The polynucleotide according to item 14 or 15, having a nucleotide sequence represented by any of SEQ ID NOs: 1, 9, 10, 14, 20, 22, 24, 25, 26, 27, 28, 29, 32, 38, 39, 40, 44, 51, 52, 53, 54 and 58.
25. A vector comprising the polynucleotide according to any one of items 14, 15, 16, 18, 20, 22 and 24.
26. A cell comprising the vector according to item 25.
27. A method of identifying a compound having an anticancer activity comprising the steps of:
   bringing a cultured human cell into contact with a test compound; and
   identifying a compound that causes a change in the expression level of a gene containing a nucleotide sequence represented by any of SEQ ID NOs: 1 to 65 in the cell as a compound having an anticancer activity.
28. A method of diagnosing cancer comprising measuring the expression level of the protein according to any one of items 1, 2, 4, 6, 8, 10, 11 and 12 or the polynucleotide according to any one of items 14, 15, 16, 18, 20, 22 and 24.
29. A method of diagnosing cancer comprising detecting C20orf102 protein.
30. The method according to item 29, wherein the cancer is lung cancer, liver cancer or pancreatic cancer.
31. The method according to item 29, wherein a secretory form of C20orf102 protein is detected.
32. The method according to item 29 wherein C20orf102 protein is detected by an antibody recognizing C20orf102 protein.
33. The method according to item 29, wherein C20orf102 protein present in blood, serum or plasma is detected.
34. A method of diagnosing cancer comprising the steps of:
   (a) collecting a sample from a subject; and
   (b) detecting C20orf102 protein contained in the collected sample.
35. The method according to item 34, wherein the sample collected from the subject is blood, serum or plasma.
36. The method according to item 34, wherein an extracellular domain of C20orf102 protein is detected.
37. The diagnostic method according to item 34, wherein C20orf102 protein is detected by an antibody recognizing C20orf102 protein.

## Claims

1. A protein encoded by a gene having a nucleotide sequence represented by SEQ ID NO: 15.

2. The protein having an amino acid sequence represented by SEQ ID NO: 72.

3. An antibody for use in in vivo diagnosing or in treating liver cancer, wherein the antibody recognizes the protein according to Claim 1 or 2.

4. A polynucleotide having a nucleotide sequence represented by SEQ ID NO: 15 or a nucleotide sequence complementary thereto.

5. A composition for use in in vivo diagnosing or in treating liver cancer comprising the polynucleotide according to Claim 4.

6. A vector comprising the polynucleotide according to Claim 4.

7. A cell comprising the vector according to Claim 6.

8. A method of identifying a compound having an anticancer activity comprising the steps of:
bringing a cultured human cell into contact with a test compound; and
identifying a compound that causes a change in the expression level of a gene containing a nucleotide sequence represented by SEQ ID NO: 15 in the cell as a compound having an anticancer activity.

9. An in vitro method of diagnosing liver cancer comprising measuring the expression level of the protein according to Claim 1 or 2, or the polynucleotide according to Claim 4 in a sample from a patient.

10. The in vitro method of diagnosing liver cancer of claim 9, wherein the expression level of the protein is detected by an antibody recognizing the protein according to Claim 1 or 2.

11. The method according to Claim 10, wherein the antibody is a polyclonal antibody.

12. The method according to Claim 10, wherein the antibody is a monoclonal antibody.

13. The antibody for use in in vivo diagnosing or in treating liver cancer according to Claim 3, wherein the antibody is a polyclonal antibody.

14. The antibody for use in in vivo diagnosing or in treating liver cancer according to Claim 3, wherein the antibody is a monoclonal antibody.

15. A composition for use in in vivo diagnosing or in treating liver cancer comprising an antibody recognizing the protein according to Claim 1 or 2.

## Patentansprüche

1. Protein, das von einem Gen codiert wird, das eine Nucleotidsequenz wie in SEQ ID NO:15 dargestellt aufweist.

2. Protein, das eine Aminosäuresequenz wie in SEQ ID NO:72 dargestellt aufweist.

3. Antikörper zur Verwendung bei der *in vivo*-Diagnose oder bei der Behandlung von Leberkrebs, wobei der Antikörper das Protein nach Anspruch 1 oder 2 erkennt.

4. Polynucleotid, das eine Nucleotidsequenz wie in SEQ ID NO:15 dargestellt oder eine dazu komplementäre Nucleotidsequenz aufweist.

5. Zusammensetzung zur Verwendung bei der *in vivo*-Diagnose oder bei der Behandlung von Leberkrebs, die das Polynucleotid nach Anspruch 4 umfasst.

6. Vektor, der das Polynucleotid nach Anspruch 4 umfasst.

7. Zelle, die den Vektor nach Anspruch 6 umfasst.

8. Verfahren zum Identifizieren einer Verbindung, die eine Antikrebsaktivität aufweist, umfassend die Schritte:
des Inkontaktbringens einer gezüchteten menschlichen Zelle mit einer Testverbindung; und
des Identifizierens einer Verbindung, die eine Veränderung im Expressionsspiegel eines Gens bewirkt, das eine Nucleotidsequenz wie in SEQ ID NO:15 dargestellt enthält, in der Zelle als eine Verbindung mit Antikrebsaktivität.

9. *In vitro*-Verfahren zum Diagnostizieren von Leberkrebs, umfassend das Messen des Expressionsspiegels des Proteins nach Anspruch 1 oder 2 oder des Polynucleotids nach Anspruch 4 in einer Probe eines Patienten.

10. *In vito*-Verfahren zum Diagnostizieren von Leberkrebs nach Anspruch 9, wobei der Expressionsspiegel des Proteins mittels eines Antikörpers nachgewiesen wird, der das Protein nach Anspruch 1 oder 2 erkennt.

11. Verfahren nach Anspruch 10, wobei der Antikörper ein polyclonaler Antikörper ist.

12. Verfahren nach Anspruch 10, wobei der Antikörper ein monoclonaler Antikörper ist.

13. Antikörper zur Verwendung bei der *in vivo*-Diagnose oder bei der Behandlung von Leberkrebs nach Anspruch 3, wobei der Antiköper ein polyclonaler Antikörper ist.

14. Antikörper zur Verwendung bei der *in vivo*-Diagnose oder bei der Behandlung von Leberkrebs nach Anspruch 3, wobei der Antiköper ein monoclonaler Antikörper ist.

15. Zusammensetzung zur Verwendung bei der *in vivo*-Diagnose oder bei der Behandlung von Leberkrebs, umfassend einen Antikörper, der das Protein nach Anspruch 1 oder 2 erkennt.

## Revendications

1. Protéine codée par un gène ayant une séquence nucléotidique représentée par SEQ ID NO : 15.

2. Protéine ayant une séquence d'acides aminés représentée par SEQ ID NO : 72.

3. Anticorps pour une utilisation dans le diagnostic *in vivo* ou dans le traitement d'un cancer du foie, où l'anticorps reconnaît la protéine selon la revendication 1 ou 2.

4. Polynucléotide ayant une séquence nucléotidique représentée par SEQ ID NO : 15 ou une séquence nucléotidique qui est complémentaire de celle-ci.

5. Composition pour une utilisation dans le diagnostic *in vivo* ou dans le traitement d'un cancer du foie comprenant le polynucléotide selon la revendication 4.

6. Vecteur comprenant le polynucléotide selon la revendication 4.

7. Cellule comprenant le vecteur selon la revendication 6.

8. Méthode d'identification d'un composé ayant une activité anticancéreuse comprenant les étapes suivantes :
la mise en contact d'une cellule humaine en culture avec un composé à tester ; et
l'identification d'un composé qui provoque un changement dans le niveau d'expression d'un gène contenant une séquence nucléotidique représentée par SEQ ID NO : 15 dans la cellule en tant que composé ayant une activité anticancéreuse.

9. Méthode *in vitro* de diagnostic d'un cancer du foie comprenant la mesure du niveau d'expression de la protéine selon la revendication 1 ou 2, ou du polynucléotide selon la revendication 4 dans un échantillon provenant d'un patient.

10. Méthode *in vitro* de diagnostic d'un cancer du foie selon la revendication 9, dans laquelle le niveau d'expression de la protéine est détecté par un anticorps reconnaissant la protéine selon la revendication 1 ou 2.

11. Méthode selon la revendication 10, dans laquelle l'anticorps est un anticorps polyclonal.

12. Méthode selon la revendication 10, dans laquelle l'anticorps est un anticorps monoclonal.

13. Anticorps pour une utilisation dans le diagnostic *in vivo* ou dans le traitement d'un cancer du foie selon la revendication 3, où l'anticorps est un anticorps polyclonal.

14. Anticorps pour une utilisation dans le diagnostic *in vivo* ou dans le traitement d'un cancer du foie selon la revendication 3, où l'anticorps est un anticorps monoclonal.

15. Composition pour une utilisation dans le diagnostic *in vivo* ou dans le traitement d'un cancer du foie comprenant un anticorps reconnaissant la protéine selon la revendication 1 ou 2.


### SEQUENCE LISTING

<110> ABURATANI, Hiroyuki; PERSEUS PROTEOMICS INC.; CHUGAI SEIYAKU KABUSHIKI
   KAISHA
<120> Genes Highly Expressed in Cancers
<130> PCG9001WO
<150> JP 2003-290704
   <151> 2003-08-08
<160> 292
<170> PatentIn version 3.1
<210> 1
   <211> 19341
   <212> DNA
   <213> homo sapiens
<400> 1
   agtaagaatc agcagcgcgg gcaaggagta cggacgggag tcagaggcag agcgagggtg     60
   tgtggagggc cggcggggac cgccgggagc gcgcggatgt cggtgttcct ggggccaggg    120
   atgccctctg catctttatt agtaaatctt ctttcagctt tactcatcct atttgtgttt    180
   ggagaaacag aaataagatt tactggacaa actgaatttg ttgttaatga aacaagtaca    240
   acagttattc gtcttatcat tgaaaggata ggagagccag caaatgttac tgcaattgta    300
   tcgctgtatg gagaggacgc tggtgacttt tttgacacat atgctgcagc ttttatacct    360
   gccggagaaa caaacagaac agtgtacata gcagtatgtg atgatgactt accagggcct    420
   gacgaaactt ttatttttca cttaacatta cagaaacctt cagcaaatgt gaagcttgga    480
   tggccaagga ctgttactgt gacaatatta tcaaatgaca atgcatttgg aattatttca    540
   tttaatatgc ttccctcaat cgcagtgagt gagcccaagg gcagaaatga gtctatgcct    600
   cttactctca tcagggaaaa gggaacctat ggaatggtca tggtgacttt tgaggtagag    660
   ggtggcccaa atccccctga tgaagatttg agtccagtta aaggaaatat cacctttccc    720
   cctggcagag caacagtaat ttataacttg gcagtactcg atgacgaggt accagaaaat    780
   gatgaaatat ttttaattca actgaaaagt gtagaaggag gagctgagat taacacctct    840
   aggaattcca ttgagatcat cattgagaaa aatgatagtc ccgtgagatt ccttcagagt    900
   atttatttgg ttcctgagga agaccacata ctcataattc cagtagttcg tggaaaggac    960
   aacaatggaa atctgattgg atctgatgaa tatgaggttt caatcagtta tgctgtcaca   1020
   actgggaatt ccacagcaca tgcccagcaa aatctggact tcattgatct tcagccaaac   1080
   acaactgttg tttttccacc ttttattcat gaatctcact tgaaatttca aatagttgat   1140
   gacaccatac cggagattgc tgaatcgttt cacattatgt tactaaaaga taccttacag   1200
   ggagatgctg tgctaataag cccttctgtt gtacaagtca ccattaagcc aaatgataaa   1260
   ccttatggag tcctttcatt caacagtgtt ttgtttgaaa ggacagttat aattgatgaa   1320
   gatagaatat caagatatga agaaatcaca gtggttagaa atggaggaac ccatgggaat   1380
   gtctctgcga attgggtggt gacaccgaac agcactgatc cctcaccagt aacagcagat   1440
   atcagaccga gctctggagt tcttcatttt gcacaagggc agatgttggc aacaattcct   1500
   cttactgggg gtgatgatga tcttccagaa gaggcagaag cttatctact tcaaattctg   1560
   cctcatacaa tacgaggagg tgcagaagtg agcgagccag cggagctttt gttctacatt   1620
   caggatagtg atgatgtcta tggcctaata acattttttc ctatggaaaa ccagaagatt   1680
   gaaagcagcc caggtggacg atacttatcc ttgagtttta caagactagg agggactaaa   1740
   ggagatgtga ggttgcttta ttctgtactt tacattcctg ctggagctgt ggaccccttg   1800
   caagcaaaag aaggcatctt aaatatatca aggagaaatg acctcatttt tccagagcaa   1860
   aaaactcaag tcactacaaa attaccaata agaaatgatg cattccttca aaatggagct   1920
   cactttctag tacagttgga aactgtggag ttgttaaaca taattcctct aatcccaccc   1980
   ataagcccta gatttgggga aatctgcaat atttctttac tggttactcc agccattgca   2040
   aatggagaaa ttggctttct cagcaatctt ccaattattt tgcatgaacc agaagatttt   2100
   gctgctgaag tggtatacat tcccttacat cgggatggaa ctgatggcca ggctactgtc   2160
   tactggagtt tgaagccctc tggctttaat tcaaaagcag tgaccccgga tgatataggc   2220
   ccctttaatg gctctgtttt gtttttatct gggcaaagtg acacaacaat caacattact   2280
   atcaaaggtg atgacatacc ggaaatgaat gaaactgtaa cactttctct agactgggtt   2340
   aacgtggaaa accaagtgct gaaatctgga tatactagcc gtgacctaat tattttggaa   2400
   aatgatgacc ctgggggagt tttgaatttt tctcctgctt ccagaggacc ctatgttata    2460
   aaagaaggag aatctgtaga gctccacatc atccgatcaa gggggtccct tgttaagcag   2520
   tttctacact accgagtaga gccaagagat agcaatgaat tctatggaaa cacgggagta   2580
   ctagaattta aacctggaga aagggagata gtgatcacct tgctagcaag attggatggg   2640
   ataccagagt tggatgaaca ctactgggtg gtcctcagca gccacggaga acgggaaagc   2700
   aagttgggaa gtgccaccat tgtcaatata acgattctga aaaatgatga tcctcatggc   2760
   attatagaat ttgtttctga tggtctaatt gtgatgataa atgaaagcaa aggagatgct   2820
   atctatagtg ctgtttatga tgtagtaaga aatcgaggca actttggtga tgttagtgta   2880
   tcatgggtgg ttagtccaga ctttacacaa gatgtatttc ctgtacaagg gactgttgtc   2940
   tttggagatc aggaattttc aaaaaatatc accatttact cccttccaga tgagattcca   3000
   gaagaaatgg aagaatttac cgttatccta ctgaatggca ctggaggagc taaagtggga   3060
   aatagaacaa ctgcaactct gaggattaga agaaatgatg accccattta ttttgcagaa   3120
   cctcgtgtag tgagggttca agaaggggag actgccaact ttacagttct cagaaatgga   3180
   tctgttgatg tgacttgcat ggtccagtat gctaccaagg atgggaaggc tactgcaaga   3240
   gagagagatt tcattcctgt tgaaaaagga gaaacgctca tttttgaggt tggaagtaga   3300
   cagcagagca tatccatatt tgttaatgaa gatggtatcc cggaaacaga tgagcccttt   3360
   tatataatcc tcttgaattc accaggtgat ccagtagtat atcaatatgg agtagctaca   3420
   gtaataattg aagctaatga tgacccaaat ggcatttttt ctctggagcc catagacaaa   3480
   gcagtggaag aaggaaagac taatgcattt tggattttga ggcaccgagg atactttggt   3540
   agtgtttctg tatcttggca gctctttcag aatgattctg ctttgcagcc tgggcaggag   3600
   ttctatgaaa cttcaggaac tgttaacttc atggatggag aagaagcaaa accaatcatt   3660
   ctccatgctt ttccagataa aattcctgaa ttcaatgaat tttatttcct aaaacttgta   3720
   aacatttcag gtggatcccc aggtcctggg ggccagctag cagaaaccaa cctccaggtg   3780
   acagtaatgg ctccattcaa tgatgatccc tttggagttt ttatcttgga tccagagtgt   3840
   ttagagagag aagtggcaga agatgtcctg tctgaagatg atatgtctta tattaccaac   3900
   ttcaccattt tgaggcagca gggtgtgttt ggtgatgtac aactgggctg ggaaatactg   3960
   tccagtgagt tccctgctgg tctgccacca atgatagatt ttttactggt tggaattttc   4020
   cccaccaccg tgcatttaca acagcacatg cggggtcacc acagtggaac ggatgctttg   4080
   tactttaccg gactagaagg tgcatttggg actggtaatt caaaatacca tcccttcagg   4140
   aataatacaa ttgccaactt tacattttca gcttgggtaa tgcccaatgc caatacgaat   4200
   ggattcatta tagcgaagga tgacggtaat ggaagcatct actacggggt aaaaatacaa   4260
   acaaacgaat cccatgtgac actttccctt cattataaaa ccttgggttc caatgctaca   4320
   tacattgcca agacaacagt catgaaatat ttagaagaaa gtgtttggct tcatctacta   4380
   attatcctgg aggatggtat aatcgaattc tacctggatg gaaatgcaat gcccagggga   4440
   atcaagagtc tgaaaggaga agccattact gacggtcctg ggatactgag aattggagca   4500
   gggataaatg gcaatgacag atttacaggt ctgatgcagg atgtgaggtc ctatgagcgg   4560
   aaactgacgc ttgaagaaat ttatgaactt catgccatgc ccgcaaaaag tgatttacac   4620
   ccaatttctg gatatctgga gttcagacag ggagaaacta acaaatcatt cattatttct   4680
   gcaagagatg acaatgacga ggaaggagaa gaattattca ttcttaaact agtttctgta   4740
   tatggaggag ctcgtatttc ggaagaaaat actactgcaa gattaacaat acaaaaaagt   4800
   gacaatgcaa atggcttgtt tggtttcaca ggagcttgta taccagagat tgcagaggag   4860
   ggatcaacca tttcttgtgt ggttgagaga accagaggag ctctggatta tgtgcatgtt   4920
   ttttacacca tttcacagat tgaaactgat ggcattaatt accttgttga tgactttgct   4980
   aatgccagtg gaactattac attccttcct tggcagagat cagaggttct gaatatatat   5040
   gttcttgatg atgatattcc tgaacttaat gagtatttcc gtgtgacatt ggtttctgca   5100
   attcctggag atgggaagct aggctcaact cctaccagtg gtgcaagcat agatcctgaa   5160
   aaggaaacga ctgatatcac catcaaagct agtgatcatc catatggctt gctgcagttc   5220
   tccacagggc tgcctcctca gcctaaggac gcaatgaccc tgcctgcaag cagcgttcca   5280
   catatcactg tggaggagga agatggagaa atcaggttat tggtcatccg tgcacaggga   5340
   cttctgggaa gggtgactgc ggaatttaga acagtgtcct tgacagcatt cagtcctgag   5400
   gattaccaga atgttgctgg cacattagaa tttcaaccag gagaaagata taaatacatt   5460
   ttcataaaca tcactgataa ttctattcct gaactggaaa aatcttttaa agttgagttg   5520
   ttaaacttgg aaggaggagt agctgaactc tttagggttg atggaagtgg tagtggtgat   5580
   ggggacatgg aattcttcct tccaactatt cacaaacgtg ccagtctagg agtggcttcc   5640
   caaattctag tgacaattgc agcctctgac cacgctcatg gcgtatttga atttagccct   5700
   gagtcactct ttgtcagtgg aactgaacca gaagatgggt atagcactgt tacattaaat   5760
   gttataagac atcatggaac tctgtctcca gtgactttgc attggaacat agactctgat   5820
   cctgatggtg atctcgcctt cacctctggc aacatcacat ttgagattgg gcagacgagc   5880
   gccaatatca ctgtggagat attgcctgac gaagacccag aactggataa ggcattctct   5940
   gtgtcagtcc tcagtgtttc cagtggttct ttgggagctc atattaatgc cacgttaaca   6000
   gttttggcta gtgatgatcc atatgggata ttcatttttt ctgagaaaaa cagacctgtt   6060
   aaagttgagg aagcaaccca gaacatcaca ctatcaataa taaggttgaa aggcctcatg   6120
   ggaaaagtcc ttgtctcata tgcaacacta gatgatatgg aaaaaccacc ttattttcca   6180
   cctaatttag cgagagcaac tcaaggaaga gactatatac cagcttctgg atttgctctt   6240
   tttggagcta atcagagtga ggcaacaata gctatttcaa ttttggatga tgatgagcca   6300
   gaaaggtccg aatctgtctt tatcgaacta ctcaactcta ctttagtagc gaaagtacag   6360
   agtcgttcaa ttccaaattc tccacgtctt gggcctaagg tagaaactat tgcgcaacta   6420
   attatcattg ccaatgatga tgcatttgga actcttcagc tctcagcacc aattgtccga   6480
   gtggcagaaa atcatgttgg acccattatc aatgtgacta gaacaggagg agcatttgca   6540
   gatgtctctg tgaagtttaa agctgtgcca ataacagcaa tagctggtga agattatagt   6600
   atagcttcat cagatgtggt cttgctagaa ggggaaacca gtaaagccgt gccaatatat   6660
   gtcattaatg atatctatcc tgaactggaa gaatcttttc ttgtgcaact gatgaatgaa   6720
   acaacaggag gagccagact aggggcttta acagaggcag tcattattat tgaggcctct   6780
   gatgacccct atggattatt tggttttcag attactaaac ttattgtaga ggaacctgag   6840
   tttaactcag tgaaggtaaa cctgccaata attcgaaatt ctgggacact cggcaatgtt   6900
   accgttcagt gggttgccac cattaatgga cagcttgcta ctggcgacct gcgagttgtc   6960
   tcaggtaatg tgacctttgc ccctggggaa accattcaaa ccttgttgtt agaggtcctg   7020
   gctgacgacg ttccggagat tgaagaggtt atccaagtgc aactaactga tgcctctggt   7080
   ggaggtacta ttgggttaga tcgaattgca aatattatta ttcctgccaa tgatgatcct   7140
   tatggtacag tagcctttgc tcagatggtt tatcgtgttc aagagcctct ggaaagaagt   7200
   tcctgtgcta atataactgt caggcgaagc ggagggcact ttggtcggct gttgttgttc   7260
   tacagtactt ccgacattga tgtagtggct ctggcaatgg aggaaggtca agatttactg   7320
   tcctactatg aatctccaat tcaaggggtg cctgacccac tttggagaac ttggatgaat   7380
   gtctctgccg tgggggagcc cctgtatacc tgtgccactt tgtgccttaa ggaacaagct   7440
   tgctcagcgt tttcattttt cagtgcttct gagggtcccc agtgtttctg gatgacatca   7500
   tggatcagcc cagctgtcaa caattcagac ttttggacct acaggaaaaa catgaccagg   7560
   gtagcatctc tttttagtgg tcaggctgtg gctgggagtg actatgagcc tgtgacaagg   7620
   caatgggcca taatgcagga aggtgatgaa ttcgcaaatc tcacagtgtc tattcttcct   7680
   gatgatttcc cagagatgga tgagagtttt ctaatttctc tccttgaagt tcacctcatg   7740
   aacatttcag ccagtttgaa aaatcagcca accataggac agccaaatat ttctacagtt   7800
   gtcatagcac taaatggtga tgcctttgga gtgtttgtga tctacaatat tagtcccaat   7860
   acttccgaag atggcttatt tgttgaagtt caggagcagc cccaaacctt ggtggagctg   7920
   atgatacaca ggacaggggg cagcttaggt caagtggcag tcgaatggcg tgttgttggt   7980
   ggaacagcta ctgaaggttt agattttata ggtgctggag agattttgac ctttgctgaa   8040
   ggtgaaacca aaaagacagt cattttaacc atcttggatg actctgaacc agaggatgac   8100
   gaaagtatca tagttagttt ggtgtacact gaaggtggaa gtagaatttt gccaagctcc   8160
   gacactgtta gagtgaacat tttggccaat gacaatgtgg caggaattgt tagctttcag   8220
   acagcttcca gatctgtcat aggtcatgaa ggagaaattt tacaattcca tgtgataaga   8280
   actttccctg gtcgaggaaa tgttactgtt aactggaaaa ttattgggca aaatctagaa   8340
   ctcaattttg ctaactttag cggacaactt ttctttcctg aggggtcgtt gaatacaaca   8400
   ttgtttgtgc atttgttgga tgacaacatt cctgaggaga aagaagtata ccaagtcatt   8460
   ctgtatgatg tcaggacaca aggagttcca ccagccggaa tcgccctgct tgatgctcaa   8520
   ggatatgcag ctgtcctcac agtagaagcc agtgatgaac cacatggagt tttaaatttt   8580
   gctctttcat caagatttgt gttactacaa gaggctaaca taacaattca gcttttcatc   8640
   aacagagaat ttggatctct aggagctatc aatgtcacat ataccacggt tcctggaatg   8700
   ctgagtctga agaaccaaac agtaggaaac ctagcagagc cagaagttga ttttgtccct   8760
   atcattggct ttctgatttt agaagaaggg gaaacagcag cagccatcaa cattaccatt   8820
   cttgaggatg atgtaccaga gctagaagaa tatttcctgg tgaatttaac ttacgtggga   8880
   cttaccatgg ctgcttcaac ttcatttcct cccagactag attcagaagg tttgactgca   8940
   caagttatta ttgatgccaa tgatggggcc cgaggtgtaa ttgaatggca acaaagcagg   9000
   tttgaagtaa atgaaaccca tggaagttta acattggtag cccagaggag cagagaacct   9060
   cttggccatg tttccttatt tgtgtatgct cagaatttgg aagcacaagt ggggctggat   9120
   tatatcttca ccccaatgat tcttcatttt gctgatggag aaaggtataa aaatgtcaat   9180
   atcatgattc ttgatgatga cattccagaa ggagatgaaa aatttcagct gattttaaca   9240
   aatccttctc ctggactaga gctagggaaa aatacaatag ccttaattat tgtccttgct   9300
   aatgatgacg gccctggagt tctatcattt aacaacagtg agcacttttt cctaagagag   9360
   ccaacagctc tctacgtcca ggagagtgtt gcagtattgt acattgttcg ggaacctgca   9420
   caaggattgt ttggaacagt gacagttcag ttcattgtga cagaagtgaa ttcctcaaat   9480
   gaatctaaag atctgactcc ttccaaaggc tatattgttt tagaagaagg tgttcgattc   9540
   aaggccctac aaatatctgc catattagac acggaaccag aaatggatga gtattttgtt   9600
   tgcaccttgt ttaatccaac tggaggtgct agactagggg tgcatgttca aaccctgata   9660
   acagttttgc aaaaccaggc ccctttgggg ctattcagta tctctgcagt tgaaaataga   9720
   gccacctcca tagacatcga agaagccaat aggaccgtgt atttaaatgt atctcgaact   9780
   aatgacattg atttggctgt gagtgtgcag tgggagacag tatctgaaac agcctttggc   9840
   atgaggggaa tggatgttgt gttttccgta tttcaaagtt ttttggatga atcagcttct   9900
   ggctggtgtt tctttacttt ggaaaattta atatatggta taatgttaag aaaatcatct   9960
   gttactgttt accgatggca ggggattttt attccagttg aggatttaaa tatagaaaat   10020
   cctaaaactt gtgaggcctt taatattggt ttttctccct actttgtgat tactcatgaa   10080
   gaaagaaatg aagaaaagcc ttctcttaac agtgtgttta cattcacatc tggatttaaa   10140
   ttattcctgg tacaaacaat cattattctg gaaagttctc aagtaagata ttttacttca   10200
   gacagccaag attatttaat cattgcaagt caaagagatg attccgaatt aactcaggtc   10260
   ttcaggtgga atggaggaag cttcgtgttg catcaaaaac tccctgtccg aggtgtgctg   10320
   accgtggcct tgttcaacaa gggaggctct gtgttcttag ccatttccca ggctaatgcc   10380
   aggctaaact cccttttatt cagatggtct ggcagtgggt ttattaactt tcaagaggtg   10440
   cctgtcagtg ggacaacaga agttgaggct ttgtcttcag ccaatgatat ttacctaata 10500
   tttgccgaaa atgtctttct aggagatcag aattcaattg atattttcat ctgggagatg 10560
   ggacagtctt ccttcaggta ttttcagtct gtagattttg ctgctgttaa cagaatccac 10620
   tccttcacac cagcctcagg aatagcccac atacttctta ttgggccaag atatgtctac 10680
   tcttttactg ctggaaattc ggagcgtaat caattctctt ttgttctgga agtaccttct 10740
   gcttatgatg tggtttctgt tacagtaaag tcccttaatt caagcaagaa tttaatagct 10800
   ctagtgggag ctcattcaca tatatatgag ctagcctaca tttccagcca ttctgacttt 10860
   attcctagtt caggtgaact gatatttgaa cctggtgaga gagaagctac aatagcagta 10920
   aatatccttg atgatacagt tccagaaaaa gaagaatcct tcaaagttca acttaaaaat 10980
   cccaaaggag gagcagagat tggcattaat gattctgtaa caataaccat tctgtctaat 11040
   gatgatgcct atggaattgt tgcatttgct cagaattcat tatataagca agtggaagaa 11100
   atggagcaag atagcctagt aaccttgaac gttgaacgct taaaaggaac atatggccgt 11160
   ataaccatag catgggaagc tgatggaagt attagtgata tatttcctac ctcaggagtg 11220
   attttattta ctgaaggcca ggtactgtca acaatcactc taactattct tgctgataat 11280
   ataccagagt tatcagaggt tgtgattgta accctcaccc gtatcaccac agaaggggtt 11340
   gaggactcat acaaaggtgc tactattgat caggacagaa gcaagtctgt tataacaact 11400
   ttgcccaatg actcaccttt tggcttggtg ggctggcgtg ctgcgtctgt cttcattaga 11460
   gtagcagagc ctaaagaaaa caccaccact cttcagttac aaatagctcg agataaagga 11520
   ctacttgggg atattgccat tcacttgaga gctcaaccca atttcttact gcatgtcgat 11580
   aatcaagcta ctgagaatga agattatgta ttgcaagaaa caataataat aatgaaagaa 11640
   aacataaaag aagctcatgc cgaagtttcc attttgccgg atgaccttcc tgaattggag 11700
   gaaggattta ttgtcactat cactgaggtg aacctggtga actctgactt ctctacagga 11760
   cagccaagtg tgcggaggcc cggaatggaa atagctgaga taatgataga agaaaatgac 11820
   gatcccagag gaatttttat gtttcatgtt actagaggcg ctggggaagt tattactgcc 11880
   tatgaggtgc ctccacccct gaacgttctt caagttcctg tagtccggct ggctggaagc 11940
   tttggggcag taaatgttta ttggaaagca tcaccagaca gtgctggcct ggaagacttt 12000
   aaaccatctc atgggattct tgaatttgca gataaacagg ttactgcaat gatagaaatc 12060
   accataattg atgatgctga atttgaattg acagagacgt tcaatatttc cttgatcagt 12120
   gttgctggag gtggcagact tggtgatgat gttgtggtaa ctgttgttat tccacaaaat 12180
   gattctccat ttggagtatt tggatttgaa gaaaagactg taatgattga tgaatccctt 12240
   tcatccgatg accctgattc atatgtgaca ttgacggttg tccggtcccc aggaggaaaa 12300
   ggaaccgtcc gacttgagtg gaccatagat gagaaggcta aacataacct tagtcctttg 12360
   aatgggaccc ttcattttga tgagactgag tcccagaaga ccattgtgtt gcacacactt 12420
   caagacacag tgttggagga ggacaggcgt ttcaccattc agctgatatc aattgatgag 12480
   gtagaaatat ctccagtaaa aggtagtgca tcaataatta ttcggggtga taagcgagca 12540
   tcaggagaag ttgggatagc tccgtcatct aggcacatcc tcattgggga accctcagca 12600
   aaatataatg gtaccgctat tatcagcctt gttcgaggcc cagggatttt gggggaggtc 12660
   acagtgttct ggaggatatt ccctccttcc gtgggggaat ttgctgaaac atcaggaaaa 12720
   ctgacaatgc gagacgaaca gtctgcagtc attgtagtaa tacaggcttt gaacgatgac 12780
   attcccgagg aaaaaagctt ctatgagttt cagctcactg cagtcagtgg gggaggagtt 12840
   ctgagtgaat ccagcagcac tgccaacatc acggtggtgg ccagcgactc tccctatggc 12900
   cgatttgcct tttcacatga gcaacttcga gtgtcagaag cacagagggt taacatcaca 12960
   atcatccgtt ccagtggaga ttttggccat gtgcgactct ggtacaagac gatgagcggg 13020
   acagcggaag caggcttgga ttttgttcct gcagcagggg agctcctctt tgaagcaggg 13080
   gagatgagga aaagtctgca tgttgaaatc cttgatgatg actatcctga aggcccagag 13140
   gaattttctc taacaattac aaaggtggaa ctccagggaa gagggtatga ttttaccatt 13200
   caagaaaatg gacttcagat agatcaacct cctgaaatag gaaacatctc cattgttcgc 13260
   atcataataa tgaaaaatga taacgcagaa ggcatcattg aatttgaccc aaagtatact 13320
   gccttcgaag tggaggaaga tgttgggctg atcatgatcc cagtggtgag gctacatgga 13380
   acttatggct atgtgacagc tgatttcatc tctcagagct cctctgccag tcccggaggt 13440
   gttgattaca ttttgcatgg cagtacagtc acctttcagc atgggcaaaa cttaagtttt 13500
   ataaatatct ccatcattga tgacaatgaa agtgaatttg aggagcccat tgaaattcta 13560
   ctcactggag ctactggagg agcggtcctt gggcgccacc tagtgagcag aatcataata 13620
   gctaagagtg actctccctt tggagttata aggtttctca atcaaagcaa aatttctatt 13680
   gctaatccca attccacaat gattttatca ctggtgctgg agcggactgg aggactcttg 13740
   ggagagattc aggtgaactg ggagacagta ggacccaact ctcaagaagc cttactgcca 13800
   cagaatagag acattgcaga cccagtgagc gggttgttct attttggaga aggagaagga 13860
   ggagtgagaa ccataattct gacaatctat cctcatgaag aaattgaagt tgaagagaca 13920
   ttcattatta aacttcatct tgtgaaagga gaagctaaat tagactccag agctaaagat 13980
   gttacattaa ccatacaaga gtttggtgac ccaaatggag ttgttcagtt tgctcctgaa 14040
   actttgtcta agaagactta ttcagagcct ctggctctgg aagggcccct gctcattacc 14100
   ttctttgtca gaagagtcaa gggcaccttt ggagagatta tggtttactg ggaattaagt 14160
   agtgagtttg acattactga agactttctt tccaccagtg gatttttcac cattgctgat 14220
   ggagagagtg aagctagctt tgatgttcat ttgctaccag atgaggtacc tgagatagag 14280
   gaagattatg tgatccagct tgtttctgta gagggaggag ccgaactgga tctggagaag 14340
   agtatcacat ggttctctgt ttatgcaaat gatgacccac atggagtatt tgccctgtat 14400
   tcggatcgcc agtcaatact tattgggcag aaccttatta gatccatcca aattaacata 14460
   acccggcttg ctggaacatt tggagatgtg gctgttgggc ttcgaatatc atcggatcat 14520
   aaagaacagc cgattgttac cgaaaatgca gagaggcagc tggtggtcaa agatggtgcc 14580
   acatataaag tggacgtggt gccaataaag aatcaggtct tcctatcact gggctctaat 14640
   ttcactttgc aactggtgac tgtgatgctt gtcggtggac gtttctatgg aatgccaaca 14700
   attcttcagg aagcaaaatc tgctgtcctt ccagtctctg agaaagctgc caattctcag 14760
   gtcggatttg aatccactgc ttttcaactc atgaacatca ctgctggcac aagccacgtt 14820
   atgatttcta ggagaggcac atatggagct ctctcggttg cctggaccac tggatatgct 14880
   cctgggttag aaattcctga attcattgtt gttggcaaca tgaccccaac actggggagc 14940
   ctttcatttt cccacggtga acaaaggaaa ggagttttcc tgtggacgtt tcctagccct 15000
   ggttggccag aggcctttgt tcttcaccta tcaggagtgc agagcagtgc tcctggcgga 15060
   gctcaactcc gatcaggttt cattgttgct gaaattgaac caatgggcgt cttccaattt 15120
   tccactagct caagaaatat catagtgtca gaagatacac agatgatcag attacatgta 15180
   caaagactat ttgggttcca cagcgatctt attaaagttt cttatcagac cactgcagga 15240
   agcgccaagc cactggaaga ttttgagcct gttcagaatg gggaactgtt ttttcaaaaa 15300
   ttccaaactg aggttgattt tgaaataacc attattaatg atcagctttc tgagatagaa 15360
   gaattttttt acattaacct tacttcagta gaaattaggg gattacaaaa gtttgatgtt 15420
   aattggagcc cacgcctgaa tctagatttc agtgttgcag tgattacaat attggataat 15480
   gatgacctgg caggaatgga tatttccttc cccgagacaa ctgtggctgt agcagttgac 15540
   acaactctca ttcctgtaga aactgaatcc accacatacc tcagcacaag caagacgact 15600
   accattctgc agccaaccaa cgtggttgcc attgttactg aggcaactgg tgtatctgcc 15660
   atccctgaga aacttgtcac ccttcatggc acacctgctg tgtctgaaaa gcctgatgtg 15720
   gccactgtaa ctgccaatgt ttccattcat ggaacattca gccttgggcc atccattgtt 15780
   tatattgaag aggagatgaa gaatggcaca ttcaacactg cagaagttct tatccgaaga 15840
   actggtgggt ttactggcaa tgtcagcata acagttaaaa ctttcggtga aagatgtgct 15900
   cagatggaac caaatgcatt gccctttcgt ggtatctatg ggatttccaa cctaacatgg 15960
   gcagttgaag aagaagactt tgaagaacaa actcttaccc ttatattcct agatggagaa 16020
   agagaacgta aagtatcagt tcaaattttg gatgatgatg agcctgaggg gcaggaattc 16080
   ttctacgtgt ttctcacaaa ccctcaaggg ggagcacaga ttgtggaggg gaaggatgat 16140
   actggatttg cagcttttgc catggttatt attacaggga gtgaccttca caatggcatc 16200
   ataggattca gtgaggagtc ccagagtgga ctagaactca gggaaggagc tgttatgaga 16260
   agattgcacc ttattgtcac aagacagcca aacagggcct ttgaagatgt caaggtcttt 16320
   tggcgagtca cacttaacaa aacagtcgtc gtgctccaga aggatggggt aaacctgatg 16380
   gaggaacttc agtctgtgtc agggaccaca acctgtacaa tgggtcaaac aaaatgcttt 16440
   atcagcattg aactcaaacc agaaaaggta ccacaggttg aagtgtattt ttttgtggaa 16500
   ctatatgaag ctactgctgg agcagcaata aacaacagtg ccagattcgc acagattaaa 16560
   atcttagaaa gtgatgaatc tcaaagcctt gtgtattttt ctgtgggttc tcggctggca 16620
   gtggctcaca agaaggccac tttaatcagt ctgcaggtgg ccagagattc tgggacagga 16680
   ctaatgatgt ctgttaactt tagtacccag gagttgagga gtgctgaaac aattggtcgt 16740
   accatcatat ctccagctat ttctggaaag gattttgtga taactgaagg cacattggtc 16800
   tttgaacctg gccagagaag cactgtattg gatgtcatcc taacgccaga gacaggatct 16860
   ttaaattcat ttcctaaacg cttccagatt gtcctttttg acccaaaagg tggtgccaga 16920
   attgataaag tgtatgggac tgccaacatc actcttgtct cagatgcaga ttcgcaggcc 16980
   atttgggggc ttgcagatca gctacatcag cctgtgaatg atgatattct caacagagtg 17040
   ctccatacca tcagcatgaa agtggccaca gaaaacacag atgaacaact cagtgccatg 17100
   atgcatctaa tagaaaagat aactactgaa ggaaaaattc aagctttcag tgttgccagc 17160
   cgaactcttt tctatgagat tctttgttct cttattaacc caaagcgcaa ggacactagg 17220
   ggattcagtc actttgctga agtgactgag aattttgcct tttctctgct gactaatgtt 17280
   acttgcggct ctcctggtga aaaaagcaaa accatccttg atagttgccc atatttgtca 17340
   atattggctc ttcactggta tcctcagcaa atcaatggac acaagtttga aggaaaggaa 17400
   ggagattaca ttcgaattcc agagaggcta ctggatgtcc aggatgcaga aataatggct 17460
   gggaaaagta catgtaaatt agtccagttt acagagtata gcagccaaca gtggtttata 17520
   agtggaaaca atcttcctac cctaaaaaat aaggtattat ctttgagtgt gaaaggtcag 17580
   agttcacaac tcctgactaa tgacaatgag gttctctaca ggatttatgc tgctgagcct 17640
   agaattattc ctcagacatc tctgtgtctc ctttggaatc aggctgctgc aagctggttg 17700
   tctgacagtc agttttgcaa agtgattgag gaaactgcag actatgtgga atgtgcctgt 17760
   tcacacatgt ctgtgtatgc tgtctatgct cggactgaca acttgtcttc atacaatgaa 17820
   gccttcttca cttctggatt tatatgtatc tcaggtcttt gcttggctgt tctttcccat 17880
   atcttctgtg ccaggtactc catgtttgca gctaaacttc tgactcacat gatggcagcc 17940
   agcttaggta cacagattct gtttctggcg tctgcatacg caagtcccca actcgctgag 18000
   gagagctgtt cagctatggc tgctgtcaca cattacctgt atctttgcca gtttagctgg  18060
   atgctcattc agtctgtgaa tttctggtac gtgctggtga tgaatgatga gcacacagag  18120
   aggcgatatc tgctgttttt ccttctgagt tggggactac cagcttttgt ggtgattctc  18180
   ctcatagtta ttttgaaagg aatctatcat cagagcatgt cacagatcta tggactcatt  18240
   catggtgacc tgtgttttat tccaaacgtc tatgctgctt tgttcactgc agctcttgtt  18300
   cctttgacgt gcctcgtggt ggtgttcgtg gtgttcatcc atgcctacca ggtgaagcca  18360
   cagtggaaag catatgatga tgtcttcaga ggaaggacaa atgctgcaga aattccactg  18420
   attttatatc tctttgctct gatttccgtg acatggcttt ggggaggact acacatggcc  18480
   tacagacact tctggatgtt ggttctcttt gtcattttca acagtctgca gggactttat  18540
   gttttcatgg tttatttcat tttacacaac caaatgtgtt gccctatgaa ggccagttac  18600
   actgtggaaa tgaatgggca tcctggaccc agcacagcct ttttcacgcc  cgggagtgga  18660
   atgcctcctg ctggagggga aatcagcaag tccacccaga atctcatcgg tgctatggag  18720
   gaggtgccac ctgactggga gagagcatcc ttccaacagg gcagtcaggc cagccctgat  18780
   ttaaagccaa gtccacaaaa tggagccacg ttcccgtcct ctggaggata tggccagggg  18840
   tcactgatag ccgatgagga gtcccaggag tttgatgatt taatatttgc attaaaaact  18900
   ggtgctggtc tcagtgtcag tgataatgaa tctggtcaag gcagccagga ggggggcacc   18960
   ttgactgact cccagatcgt ggagctcagg aggataccca tcgccgacac tcacctgtag  19020
   cacctcacta accattcgac tgagcacact ttcatatttg tatcagcttt tgtgctaaaa  19080
   ctctctaagt acatccacct gtgtaatagg aacctgtgaa ttgtactgga tgattaatac  19140
   aaacgtgatt gttgtatttg gagtataaat tactgattgt atgtgacctg aaaattcact  19200
   gctataagaa aggtggagtc agtttgtatc agttaatagg atgttcatat tccaaggata  19260
   ttagttgttt ttttaatcat cctatatggc taacattgtt taatgaaagt aataatcaat  19320
   aaagcaatag aatctaaaaa a                                            19341
<210> 2
   <211> 1952
   <212> DNA
   <213> homo sapiens
<400> 2
   cttccgcgga agggaagagt cccgcagtcg gaggcggccg gctgggcgtg cgctcgctcc    60
   ccgaagccgg ggctgggccg gagccgggcg agggctggga gctgggccgg gtccggggac   120
   agcgggcgag gggcagctgc cggagccggg cagccaggcc gctcagggca ggggacagct   180
   ggcgccggtt ctgcggtctc cggggcccag atgtgaggcg gcggcgcccc cggcccgaga   240
   gcgcacgatg ggggccccgc  tcgccgtagc gctgggcgcc ctccactacc tggcactttt   300
   cctgcaactc ggcggcgcca cgcggcccgc cggccacgcg ccctgggaca accacgtctc   360
   cggccacgcc ctgttcacag agacacccca tgacatgaca gcacggacgg gcgaggacgt   420
   ggagatggcc tgctccttcc gcggcagcgg ctccccctcc tactcgctgg agatccagtg   480
   gtggtatgta cggagccacc gggactggac cgacaagcag gcgtgggcct cgaaccagct   540
   aaaagcatct cagcaggaag acgcagggaa ggaggcaacc aaaataagtg tggtcaaggt   600
   ggtgggcagc aacatctccc acaagctgcg cctgtcccgg gtgaagccca cggacgaagg   660
   cacctacgag tgccgcgtca tcgacttcag cgacggcaag gcccggcacc acaaggtcaa   720
   ggcctacctg cgggtgcagc caggggagaa ctccgtcctg catctgcccg aagcccctcc   780
   cgccgcgccc gccccgccgc cccccaagcc aggcaaggag ctgaggaagc gctcggtgga   840
   ccaggaggcc tgcagcctct agactgatgc ccctgccccc gcccatccgc ccccacgctg   900
   tacagagtgc atgaggagcc gccggaccac cggggaccga ctgcctgcgt ccagccgcgc   960
   cccatccccg aggccgcctg tggccaccat gtcggccctc tttccaccac cccttgctca  1020
   gcatgtaagc cccacccacc cctgcccttt cagacccctg cggtgacctg gctcggagaa  1080
   ggtggccctg ggcaccaagg ggccaaccgc cctgaacact ggggcaggga ccatgctggg  1140
   gcccggggcc acccccttcc tgtcaccagc ttctgtggag tccagtgttt tgctttgctt  1200
   gcttgtcccc catcctgtcc tgagccgggg ccccccagcc tcgcctccct cctcctacca  1260
   tccctcactt ggacctgggg gtgtggacag tgacccctcc ctgaatatgg acttgaatct  1320
   tctgagcaga actagggcct ctcccctggt gaagacccag ggaacccagg agggcccttc  1380
   tggggcagtg gctctgcagg gtcactcatg gaggcctagg ggaacagcga gatgccccac  1440
   cacctcctgg cgagtccttc ctgttcagct ccctgtgcga ccctccaggg atgcagggga  1500
   tccaggattc tctgccctgt cacacggcga gtcagaaggg aggggccttt ccctcggacc  1560
   catggcccca ggcagagttt tgcaccagca ggaccccttt gagggccttc aaggctctcc  1620
   caggagtccc cctctgccgg ccccccaatg ccccagctcc ctcttgggtc ctgtgccaag  1680
   tccgccccag ggcctggggc tgttgggagc caagggcccc ctggtactca gttccctcac  1740
   gattcccgat cacgggcaca cctgccccct ggttatttgt aaatatttct attggaccca  1800
   attctcctcg gaattggctg gcacctctgg ttgccacagc tcagtgatga cgtgggggag  1860
   gtgggagagg ccgagggctt tgcctagggg tgggttgccc tgtatacatg atccagtctg  1920
   tgactaccag ccaacctgaa taaagcggtt tt                                1952
<210> 3
   <211> 932
   <212> DNA
   <213> homo sapiens
<400> 3
   gggcgtgaga aaggcgacgg cggcggcgcg gaggagggtt atctatacat ttaaaaacca     60
   gccgcctgcg ccgcgcctgc ggagacctgg gagagtccgg ccgcacgcgc gggacacgag    120
   cgtcccacgc tccctggcgc gtacggcctg ccaccactag gcctcctatc cccgggctcc    180
   agacgaccta ggacgcgtgc cctggggagt tgcctggcgg cgccgtgcca gaagccccct    240
   tggggcgcca cagttttccc cgtcgcctcc ggttcctctg cctgcacctt cctgcggcgc    300
   gccgggacct ggagcgggcg ggtggatgca ggcgcgatgg acggcggcac actgcccagg    360
   tccgcgcccc ctgcgccccc cgtccctgtc ggctgcgctg cccggcggag acccgcgtcc    420
   ccggaactgt tgcgctgcag ccggcggcgg cgaccggcca ccgcagagac cggaggcggc    480
   gcagcggccg tagcgcggcg caatgagcgc gagcgcaacc gcgtgaagct ggtgaacttg    540
   ggcttccagg cgctgcggca gcacgtgccg cacggcggcg ccagcaagaa gctgagcaag    600
   gtggagacgc tgcgctcagc cgtggagtac atccgcgcgc tgcagcgcct gctggccgag    660
   cacgacgccg tgcgcaacgc gctggcggga gggctgaggc cgcaggccgt gcggccgtct    720
   gcgccccgcg ggccgccagg gaccaccccg gtcgccgcct cgccctcccg cgcttcttcg    780
   tccccgggcc gcgggggcag ctcggagccc ggctccccgc gttccgccta ctcgtcggac    840
   gacagcggct gcgaaggcgc gctgagtcct gcggagcgcg agctactcga cttctccagc    900
   tggttagggg gctactgagc gccctcgacc ta                                  932
<210> 4
   <211> 459
   <212> DNA
   <213> homo sapiens
<400> 4
   ggagagacaa gggactccct atgttactcc tggactaaag caatcctccc acattggctt     60
   tccaaagtgc tgaggtcaca ggcacaagcc cctgcgcccg accacaagta ggtgttttaa    120
   accagtgttt ttttttaaca aggcacaaac attcgactta agggtgacag catagtactt    180
   taccaggaat aaagttatgt tttacacata tacacgtgga aaaaattaaa accctatgaa    240
   gttggtattg tcttatatta agtaatgagg aaactgaggc ataaagtagc taaggatttt    300
   gttcaaagag caagtgatgg cataatcaga acttgaaccc aggtctaccc agcatcagag    360
   cccatattcc taatccccac actggggctg caggaggaaa ttaatggaag gattcccaca    420
   aaacaagaca aatcttttac catataaata aattcacat                           459
<210> 5
   <211> 667
   <212> DNA
   <213> homo sapiens
<220>
   <221> misc_feature
   <222> (435)..(435)
   <223> n is a, g, c or t
<220>
   <221> misc_feature
   <222> (652)..(652)
   <223> n is a, g, c or t
<400> 5
   tcgacccacg cgtccgtatt atccacttcc tctctctcta tctttagtat tttaaagtaa     60
   atcccagata gcatcacatc atttcacccc caccatagga tttcaaagat ctgttatatt    120
   tcaagattga gtaaaagggc ttgaaattgg gttattgcaa tgaaactcta gaaaaagctt    180
   gagggttcac ccaggagtaa gctggacaaa aaaggggttt gaggggtgga cccatcttgc    240
   ctaaaaatct tgtctcatct ttctaaaaat tacatatgaa agaggaagat ttatgttact    300
   tttttatatg agagaatcgt cctttaatag aaaatttcta ttgctgcatc agaattatgg    360
   aggaacacaa aaaacatacc tcagtcctta gtgtgtccta aattaacaca tattcactta    420
   ttagtgggta aatgnctata tttcatttca gcacaacttc tcccctggta gaaactcaaa    480
   agaaatttct aatgattaaa ctagggaagt tgcactggaa ttggatggct tatcagagca    540
   accgcagttt tccaggaagg aaattccaat ggccatgcgg ttggaaaatt ccccctagca    600
   aataagggat taatttttaa aaaaggaagg ataaaggagg tctgggttct tntggtttta    660
   aaaaaaa                                                              667
<210> 6
   <211> 418
   <212> DNA
   <213> homo sapiens
<400> 6
   ttttatagtg ctgtatttgt attgggtgaa tatgtggaaa ttagggagtt ctatgctttt     60
   gatagagaca ggccaattta cttgctcatc cttgaatgca ggtttcttga cattcctttt    120
   cactgttgaa tctatttcct gagccattac agccactgaa ccaaaatcgg gcacaagagt    180
   tgacctgttt gtcataatac catcgaacca catattcacc acagtttcca ggcttcaagg    240
   cttccaaaca tctaggatcc tctgccccat ccacaggggt gctgagcagt ggcctggggg    300
   tggtggtggc ctcagatgag gtagtggcag gcacatcatc agcccacgtt ggctctggag    360
   ccttatcatc ttcatcctgg tctctggtga cactcaatga ctcagaaatt tctttttg      418
<210> 7
   <211> 1665
   <212> DNA
   <213> homo sapiens
<400> 7
   caacgacggc agccccgccg gctactacct gaaggagtcc aggggcagcc ggcggtggct     60
   cctcttcctg gaaggcggct ggtactgctt caaccgcgag aactgcgact ccagatacga    120
   caccatgcgg cgcctcatga gctcccggga ctggccgcgc actcgcacag gcacagggat    180
   cctgtcctca cagccggagg agaaccccta ctggtggaac gcaaacatgg tcttcatccc    240
   ctactgctcc agtgatgttt ggagcggggc ttcatccaag tctgagaaga acgagtacgc    300
   cttcatgggc gccctcatca tccaggaggt ggtgcgggag cttctgggca gagggctgag    360
   cggggccaag gtgctgctgc tggccgggag cagcgcgggg ggcaccgggg tgctcctgaa    420
   tgtggaccgt gtggctgagc agctggagaa gctgggctac ccagccatcc aggtgcgagg    480
   cctggctgac tccggctggt tcctggacaa caagcagtat cgccacacag actgcgtcga    540
   cacgatcacg tgcgcgccca cggaggccat ccgccgtggc atcaggtact ggaacggggt    600
   ggtcccggag cgctgccgac gccagttcca ggagggcgag gagtggaact gcttctttgg    660
   ctacaaggtc tacccgaccc tgcgctgccc tgtgttcgtg gtgcagtggc tgtttgacga    720
   ggcacagctg acggtggaca acgtgcacct gacggggcag ccggtgcagg agggcctgcg    780
   gctgtacatc cagaacctcg gccgcgagct gcgccacaca ctcaaggacg tgccggccag    840
   ctttgccccc gcctgcctct cccatgagat catcatccgg agccactgga cggatgtcca    900
   ggtgaagggg acgtcgctgc cccgagcact gcactgctgg gacaggagcc tccatgacag    960
   ccacaaggcc agcaagaccc ccctcaaggg ctgccccgtc cacctggtgg acagctgccc   1020
   ctggccccac tgcaacccct catgccccac cgtccgagac cagttcacgg ggcaagagat   1080
   gaacgtggcc cagttcctca tgcacatggg cttcgacatg cagacggtgg cccagccgca   1140
   gggactggag cccagtgagc tgctggggat gctgagcaac ggaagctagg cagactgtct   1200
   ggaggaggag ccggcactga ggggcccaga cacccgctgc cccagtgcca cctcaccccc   1260
   caccagcagg ccctcccgtc tcttcgggac agggccccag ccgtcccccc tgtctgggtc   1320
   tgcccactgc cctcctgccc cggctttccc tgcccctctc ccacagccca gccagagaca   1380
   agggacctgc tgtcatcccc atctgtggcc tgggggtcct tcctgacaac gagggggtag   1440
   ccagaagaga agcactggat tcctcagtcc accagctcag acagcaccca ccggccccac   1500
   ccatcaagcc cttttatatt attttataaa gtgacttttt tattacttta attttttaaa    1560
   aaaaggaaaa taagaatata tgatgaatga tattgttttg taacttttta aaaatgattt    1620
   taaagagaca aaaaagaaaa aaaaaaaaaa aaaaaaaaaa aaaaa                   1665
<210> 8
   <211> 3561
   <212> DNA
   <213> homo sapiens
<400> 8
   atggcattag tgctgtcgga atcctgataa aacatcacaa acttctgttc gttggtatta     60
   gggacagtat agagtgagtg cttgaagaac tgccttggct taccaatctc tctctccaca    120
   acttccaatc atctctaggg aacctagcag aaacacttcc acagagcaaa agttataata    180
   cagaaagtga tgaaaggagg acgcaggctc tcaatgacgc caggtattcc cgggacccgc    240
   ccaccgtggg cgttttccac ctacaggcag gcgtctccgg gggcggggct tgctcagggt    300
   taacgtcact actgagcgcc gggcgcgttc cgttggcggc ggattcgaac gttcggactg    360
   aggtttttct gcctgaagaa gcgtcatacg gaccggattg ttttcgctgg cccagtgtcc    420
   ccggagcttg tgtgcgatac agagagcacc tcggaagctg aggcagctgg tacttgacag    480
   agaggatggc gctgtcgacc atagtctccc agaggaagca gataaagcgg aaggctcccc    540
   gtggctttct aaagcgagtc ttcaagcgaa agaagcctca acttcgtctg gagaaaagtg    600
   gtgacttatt ggtccatctg aactgtttac tgtttgttca tcgattagca gaagagtcca    660
   ggacaaacgc ttgtgcgagt aaatgtagag tcattaacaa ggagcatgta ctggccgcag    720
   caaaggtatc tgaaaactga agagtggaga atatgttcag cagggaacaa gaggattctt    780
   ttagaaataa gaggtagaag gtcacctcaa ataccacaaa atgaatggga agatgaacac    840
   tatgcaagta ctcattaaaa acatcccaaa atgctggagg aaatgccgtg gaattaagga    900
   atgactcttt tgtggtagaa aatttttatt tcagagtata gacactgctt gtgctgggtc    960
   tcttaatttt actggatatt ataattttct tggactgtta gattttctca gtgcaacatt   1020
   tctgagagca aatatgttct gctcaattaa atactttcta aagctagata aatgccttcc   1080
   agtaagcaca ttaacattca atatgttata ttttaccaga ggtaaaacat ttatattcta   1140
   aaacattata ttctaaaatt ttttacccca gggaaaataa aatgccaaaa atctcaacta    1200
   ccacacatag ctttgtaata agatcaatgg gcaaatggaa tcagcttcag tttgcactaa    1260
   tcctacatat atgtattaag gatatgtgta atgaactttg gccataataa attataacta    1320
   atttatttca tcagttcact actatatttc tttcttgttt gcttctcctc ttgtcgtctc    1380
   ttcatatttg gtagaatata ttttattgaa tatctagttt cagattacct gagcttcaga    1440
   ttacatggaa ttttggactg ttgaatactt ttgattgtgt agtggatata actaagcaat    1500
   tttttttttg gggtgcatac ttagaacgta atttctggat cttttgatag gcatattgtt    1560
   ttagtttgtt ttctgcttct ataacataat actgtagact gggtaatgta taaagaaaag    1620
   agatttattt ccctcagagt tctggagatt gtccaagatc aaggggctgc atctggtgaa    1680
   atccttcttg ctgtgtccta tatggtggaa agcatcacat ggcaaggaag catgcttgtg    1740
   acagagagaa tgagagctga acttcatctt tttatcagca accccctcca gcaataatta    1800
   acccaatccc acaataatgg ccttaatgta ttcatgtggg cagagtcctt ataactcagt    1860
   cacctcttga aggtcccact cttaatgctg tcgcagtagc aattacattt caacatgagt    1920
   tttggaaggg atatttaaac catagcatac atgtttgcta taaaaacgcc agttttccaa    1980
   gtgaatgtac ccattttaaa cacccaccag aatcatttgt gagatctaat tgttcattca    2040
   atcttactat acttggtatg gtcaattatt ttaattttag caccataatg aatatgtagt    2100
   gatattaaat ttggtattac tttgcattaa tgactaaaaa ttagataaca aatgatattg    2160
   aacttatttc cacatgctta ttgatcattt ggatatatta ctttgtgaag ttcctttttg    2220
   aagccttttg cccatttttc tttgtgttgt atttattttc catactcatc aaaaacatcc    2280
   caaaatgcta gaggaaatgc tctagaattc aggaataact tcttttgtgg tagagaattt    2340
   ttatttcaga gtatagacac tgtttgtgct gggtttctta attttactgg atatcatcat    2400
   tttcttgggc tgtttgatca agtttgattt tcccagtgca acatctctga gagtaaatat    2460
   gatctgctta ataaaatatg ttctaaagcc agataagtgc tttccagcaa gtacaatttt    2520
   ccttccagca agtataatac ctggtttata agcattacat atattaatat tattattttg    2580
   tcagatagat ataataaaag tttaatattt caatctttta tggtaagtgc tatttatata    2640
   ctgtttaaga aagaggcacc catcatgaca ataatgttct atattttcct atgaaagtct    2700
   tgttttatat ttcacattta ggttttgatg gtccttaaat ataagatttt tatacatgat    2760
   attatgtatg gatttaggtt aatttatttt tatatggata gccaactaac ccagaaccac    2820
   ttatcaaaaa gacatccttt atacattgaa ttgcaatagt gttttatcaa aaccagctaa    2880
   gtatttttga gtgagtgtat ttctgaactc tctaatttgt ttcatttgtt taccttttgt    2940
   tgtattattt cctcaccatc ttaattattt ttagccttat aaaagctttg atatttggta    3000
   gtataagttt tcctgtttag tttttcttct gtaatatttt cttgactata taaagtcctt    3060
   gcatttctat gtacatttta gaataaaact gtcaatttgc acaaaataac aggctgggat    3120
   ttggatagag attatgttga tttgagggag attggcattt tatcaatatt gtgtctttca    3180
   gtcaatatac agagtatatc tatttagtta ttttgatatt aatttttctc accaatgttt    3240
   tatggtcctt aaggaaaaag ccttgcaatg tttctatata tgtgatccta ggtagtggac    3300
   ccttttagat gttattataa atgattttgc tttttaatta aatttcattt ttcagttatt    3360
   tctagtataa agaaatggag gtgactgatt attggaaaca tttatttgta aattttctta    3420
   tttttaattt ttttgtagat tcttgtaaat tttctgcaga aaatttatat ttttttctgg    3480
   ctttattgaa ctgctttgaa actccagtga aatgttgaat aaaagtgctg ataattgata    3540
   ttaaaaaaaa aaaaaaaaaa a                                              3561
<210> 9
   <211> 2628
   <212> DNA
   <213> homo sapiens
<400> 9
   ggcacgagga ctagaaggaa gaagtatgga gttaaagact gcagcgtgaa ctgaggagtc     60
   ccggacaggc cgcttgctgc agaggatcca gtccagatcc caggagagcc cctctgcccc    120
   ttcggacctc gtctcccatc tacaaaacgt gaagattggc ccagttggcg tgtctctaca    180
   aaaaggtgca tataccactg ccccgctgca ggctgatctg agaaagcctc tggcccaggg    240
   cagataccgc catggccttc ctgatgcacc tgctggtctg cgtcttcgga atgggctcct    300
   gggtgaccat caatgggctc tgggtagagc tgcccctgct ggtgatggag ctgcccgagg    360
   gctggtacct gccctcctac ctcacggtgg tcatccagct ggccaacatc gggcccctcc    420
   tggtcaccct gctccatcac ttccggccca gctgcctttc cgaagtgccc atcatcttca    480
   ccctgctggg cgtgggaacc gtcacctgca tcatctttgc cttcctctgg aatatgacct    540
   cctgggtgct ggacggccac cacagcatcg ctttcttggt cctcaccttc ttcctggccc    600
   tggtggactg cacctcttca gtgaccttcc tgccgttcat gagccggctg cccacctact    660
   acctcaccac cttctttgtg ggtgaaggac tcagcggcct cttgcccgcc ctggtggctc    720
   ttgcccaggg ctccggtctc actacctgcg tcaatgtcac tgagatatca gacagcgtac    780
   caagccctgt acccacgagg gagactgaca tcgcacaggg agttcccaga gctttggtgt    840
   ccgccctccc cggaatggaa gcacccttgt cccacctgga gagccgctac cttcccgccc    900
   acttctcacc cctggtcttc ttcctcctcc tatccatcat gatggcctgc tgcctcgtgg    960
   cgttctttgt cctccagcgt caacccaggt gctgggaggc ttccgtggaa gacctcctca   1020
   atgaccaggt caccctccac tccatccggc tgcgggaaga gaatgacttg ggccctgcag   1080
   gcatggtgga cagcagccag ggccaggggt atctagagga gaaagcagcc ccctgctgcc   1140
   cggcgcacct ggccttcgtc tataccctgg tggccttcgt caacgcgctc accaacggca   1200
   tgctgccctc tgtgcagacc tactcctgcc tgtcctatgg gccagttgcc taccacctgg   1260
   ctgccaccct cagcattgtg gccaaccctc ttgcctcgtt ggtctccatg ttcctgccta   1320
   acaggtctct gctgttcctg ggggtcctct ccgtgcttgg gacctgcttt gggggctaca   1380
   acatggccat ggcggtgatg agcccctgcc ccctcttgca gggccactgg ggtggggaag   1440
   tcctcattgt ggcctcgtgg gtgcttttca gcggctgcct cagctacgtc aaggtgatgc   1500
   tgggcgtggt cctgcgcgac ctcagccgca gcgccctctt gtggtgcggg gcggcggtgc   1560
   agctgggctc gctgctcgga gcgctgctca tgttccctct ggtcaacgtg ctgcggctct   1620
   tctcgtccgc ggacttctgc aatctgcact gtccagccta ggcaggccgc cgaccccgcc   1680
   cccatcgctc acggacggaa ctggggtcca gagaggccag gtcacagagc aaggggcagg   1740
   aacagagaga cagagcctga gtaattgaat catgaacgca agtgcccact ggggactgtg   1800
   gggaagatgg cacctggaaa tgcaaggtgc ggctctatcc ccaactctgt gtcacactac   1860
   ctgtgacgac cagctcagat ctcctttgct ttgactctca agagaggact gatttgcagc   1920
   atctagctgg aggcaggccc aagggtgtta gaagggaaac agctgggaca gccggctgtc   1980
   ccttcaggct gtgtgacctt gggaaagtca tttggcttct ctgtgcctgt ttcttcatgc   2040
   atgcagtggg gattccagta agtaccaact acctcacagg catggcacgg aggcaaaagg   2100
   aaaaagcagc ccgcatcaag caagccctcc tgggccacct gctgatctga cagtccatcg   2160
   tagtaacaag agtggcagtc tgcacaacct agaagtggcc agaagggttg agacacgccc   2220
   ctgccctctc tcctttgccc ctcagtctca cagaggggct tctacaagac aagcagataa   2280
   cgatagaatc ttgggcatct tggctttcgg attctcagtg tggagggacg tagtacccca   2340
   cacacccctt cctgtcatcc ttcctggccc ataaagccca ctagttggag agtaagtacc   2400
   ctcctggaag cagggagaga tgatttgctg gtggggctgg ggaaggccca tccctgagcc   2460
   tctgaaagtg aactccccga ccaggttggg gaccagacat gcagagcccc tggaagtatt   2520
   ctctcaaatg gaggcaacag aggtgattgt tattttgttt tagtttctgt ttttcatttt    2580
   tttaaataaa ggcattccct gcttttaaaa aaaaaaaaaa aaaaaaaa                 2628
<210> 10
   <211> 570
   <212> DNA
   <213> homo sapiens
<400> 10
   cgtacataca tagtctgatg acagttggtg ggctgatttg aattatttct gtcttcatta     60
   ccctttctgc tgcggttgat tctggtgtaa tggggatatc atcagaccaa gtcaggcttt    120
   tgcccccacc caagaatgag aggaagtttt gttatgatgt ttctagctgt cgttcatcct    180
   tccctgagac aatgaacaag tggaacacct tttaccagta tttgcagtca ccttttagta    240
   agtttgatga tctgttgaag tacttatggg ctgcacacac ttcaaccttg gcagataata    300
   tcaaaagttt tgaagacaga tatgattatt attctaaagc agaagcgcat tttgagagaa    360
   gttgggtact ggctgtggat catttagctg cagtcctctt tcctacaacc ttgattagat    420
   catataagtt ccagaagggc atgccaccac gaattcttct taatactgat gtagcccctt    480
   tcatcagtga ctttactgct tttcagaatg tagtcctggt tcttctaaat atgcttgaca    540
   atgtggataa atctataggt tatctttgta                                      570
<210> 11
   <211> 1704
   <212> DNA
   <213> homo sapiens
<400> 11
   gcctcaacct gacactcagc agcaagttag gcctatctac aaatctcctg ctctgtgccc     60
   gcgcagtcca gtggaggaga tattcctaat tagggggcac ttccaagggg cggagtctcc    120
   gcggcgagtg gccgtggagg ggcgcaaggc agacggtctc cagccacgat acctgggcca    180
   atcccagcct gtcacgtcat gcctagtgag ctgaacaagc tgcgtaactg tcgtgtgcct    240
   cagtttcccc acgtgtataa tggaaatgat tacaataaac aggacctttt tgaggagttg    300
   ccatgaggac tgtcagacgc caaggcgccc cgagaggcac ttcgcttcca tgccgaggcc    360
   aagggcgcac aggtgcgtct ggacacgcgt ggctgcatcg cgcacaggcg caccacgttc    420
   cacgacggca tcgtgttcag ccagcggccg gtgcgcctgg gcgagcgtgt ggcgctgcga    480
   gtgctgcggg aggagagcgg ctggtgcggc ggcctccgcg tgggcttcac gcgcctggac    540
   cccgcgtgcg tgtccgtgcc cagcctgccg cccttcctgt gccccgacct ggaggagcag    600
   agcccgacgt gggcggccgt gctgcctgag ggctgcgcgc tcactgggga cttggtccgc    660
   ttctgggtgg accgccgcgg ctgcctcttc gccaaggtca acgccggctg ccggctcctg    720
   ctgcgtgagg gcgtgcccgt cggcgccccg ctctgggccg tgatggacgt gtatgggacc    780
   actaaggcca tcgagctgct ggatcccaca gccagccggc tcccaacacc catgccatgg    840
   gacctcagca acaaggctgt gcctgagccc aaagccacac caggagagga gtgtgccatc    900
   tgcttctatc acgctgccaa cacccgcctt gtgccctgcg gccacacata cttctgcaga    960
   tactgtgcct ggcgggtctt cagcgatacg gccaagtgcc ctgtgtgccg ctggcagata   1020
   gaggcggtag cccctgcgca gggccctcct gctctgaggg ttgaggaagg ctcatgaaag   1080
   gaggcttccc agtatgagtg gcagcccggg cctagatctg agtctggccc ctgcagagag   1140
   gaaggaggcg cagccctacc ttctttctgg ggaagagtca gaaaggctga ttagcaagag   1200
   gtgcggcaga gagaggaagg cagggaggtg ctgtctgctg cctccacctg ttccccaaca   1260
   ggatagcaag gaaaaacccc tctttctgtc catgccagaa ctatccttct gatggggtgc   1320
   tttgtttaga gatggggtgg cccaatcccc aatcagtttt acatctgagg gagttcaggt   1380
   atctgttgtg actggtgaag ccctgtacct cctgggtatt gaagaacctg gacttgaagc   1440
   aggaggtatc tgcaaggaat gtataaagtt ccacatggaa gctgggttgc ctcccacaag   1500
   tccccagtag agtggatctg tagttacccg ccctgcctcc ctttcagggt ggtcatgagg   1560
   tcccagagag accatgcatc tgaagatgat ttaaaacatg aaagtgtatt gttgtcactg   1620
   tggtaatttc cttgccagtt tctgagatat caaaataaag tttgtgtttc ctgaaaaaaa   1680
   aaaaaaaaaa aaaaaaaaaa aaaa                                            1704
<210> 12
   <211> 1329
   <212> DNA
   <213> homo sapiens
<400> 12
   gggagctaca atgttttgtc attattcact ctgatgtgaa aaaggcagtg aatttaatag     60
   aaaataactt cgtagagcaa aatctcaggt gtgttttttt agtgccgcag tcttggatga    120
   tgggttccta gaagctctca acatctcttc ttaattggag aaagtgttaa gccccaaagt    180
   agctggagca gtacatcttc aatttttgac aagaaaacag gaacttgatt actttgagtg    240
   ctattcatta gtttctgctt tcattgagaa tgcaacaaaa gccaactagg ctgctgctaa    300
   ctccttgctg gacttcttct gccactgtca caggaactgt aatctcactg gacaattaac    360
   tagggagtct ttcatcttga gtgactgctg cacaaatgat cttcaaagca ttttagccac    420
   cagaggaatt ctcttgaaat acccaaaatc catcagtatc ttgaatcatg ctggattttg    480
   aagaattctt aacaagccat gtaaaggggg ctctctggcc ttgaaatagt gatgtttttt    540
   atacagaaag gagaatgcag aatggtcaga ctaccatgca ctgttaaatt tgatttcaag    600
   aaattacagg aaaactttcc aaagttccat ctcacagaaa ttatttttac aaagaattcc    660
   aagataagtt tagttttatg gaagactttt atgtggtttt tactcactct tcatctcaga    720
   catcaacaga tgattacatc acttatttag ctagtaaatt tattaatata aaaactcaga    780
   gacattccaa tatccacatt gcttacacca ttaggcatag attcagtgtc agctatgaca    840
   attgaaaata agctgttttg tgatttaaag gtttaaattt ctctaaccaa actgcttgat    900
   ccagatgcag gactgcaaat gttaatattt gttctggaag aacaatcaaa taagacttaa    960
   gaggaaaagg aatggccaca atccacctga aatttttttt taaaaagtgt gcagcctact   1020
   aaatcagaat gaaaatagaa gtacaagatt ataaacaaaa tgcaatcaaa cttttcttaa   1080
   gcttacctaa agttatttca tctgaaaatt tcaagcaact ttgttcaaca ttaaattgac   1140
   aatctaaact aacaagtctt ttgaatttat gcatggtagt aaacattctc tctattaact   1200
   gtattaccta aggctaaacc taaaattttt aagcaaaatt agaaaaatag tcttcactca   1260
   tcaaaaaata aagtttgtta catttagtat tttcccaaga aaaaaaaaaa aaaaaaaaaa   1320
   aaaaaaaaa                                                           1329
<210> 13
   <211> 560
   <212> DNA
   <213> homo sapiens
<400> 13
   acaggtcttt ttatttaaca taaggccaaa gaagctatca ggcgttgctg aatactgtcc     60
   actaactgta caaaatattg actgcatgcc tcgcaaacac caaaatatcc gctggaatgc    120
   catagaaata aataacttct gctataaaca catgaaaaca tatcaaactg ttatctcttt    180
   aaacatattg taaataaaaa aattaccagt acttctacac aataaatatt aagaaaccat    240
   tgacatagtt gaaatgcact catataaatt aacaacttta attacattag ccaaacagac    300
   attggttaaa gaactgcatg tagtatgcaa aacaaaacaa aacaaaacaa aaaacaaagt    360
   aaaaaaccaa caaaatagaa acaaacaaac aaacaacatc aaccacagaa cataaaaagt    420
   tttaaaataa aacaggcttc agattatctt ggctttcata attatatttt tcttttaaag    480
   aaaaatatca acccattgtc aatgcactgt ttttcaaagc atttaaatag agggtaaaac    540
   cctttggaaa ttaatacaga                                               560
<210> 14
   <211> 1805
   <212> DNA
   <213> homo sapiens
<400> 14
   aaagcctgcg agcgccagcc gagatcgcag cccaacccat ggccgggtct cctagccgcg     60
   ccgcgggccg gcgactgcag cttcccctgc tgtgcctctt cctccagggc gccactgccg    120
   tcctctttgc tgtctttgtc cgctacaacc acaaaaccga cgctgccctc tggcaccgga    180
   gcaaccacag taacgcggac aatgaatttt actttcgcta cccaagcttc caggacgtgc    240
   atgccatggt cttcgtgggc tttggcttcc tcatggtctt cctgcagcgt tacggcttca    300
   gcagcgtggg cttcaccttc ctcctggccg cctttgccct gcagtggtcc acactggtcc    360
   agggctttct ccactccttc cacggtggcc acatccatgt tggcgtggag agcatgatca    420
   atgctgactt ttgtgcgggg gccgtgctca tctcctttgg tgccgtcctg ggcaagaccg    480
   ggcctaccca gctgctgctc atggccctgc tggaggtggt gctgtttggc atcaatgagt    540
   ttgtgctcct tcatctcctg ggggtgagag atgccggagg ctccatgact atccacacct    600
   ttggtgccta cttcgggctc gtcctttcgc gggttctgta caggccccag ctggagaaga    660
   gcaagcaccg ccagggctcc gtctaccatt cagacctctt cgccatgatt gggaccatct    720
   tcctgtggat cttctggcct agcttcaatg ctgcactcac agcgctgggg gctgggcagc    780
   atcggacggc cctcaacaca tactactccc tggctgccag cacccttggc acctttgcct    840
   tgtcagccct tgtaggggaa gatgggaggc ttgacatggt ccacatccaa aatgcagcgc    900
   tggctggagg ggttgtggtg gggacctcaa gtgaaatgat gctgacaccc tttggggctc    960
   tggcagctgg cttcttggct gggactgtct ccacgctggg gtacaagttc ttcacgccca   1020
   tccttgaatc aaaattcaaa gtccaagaca catgtggagt ccacaacctc catgggatgc   1080
   cgggggtcct gggggccctc ctgggggtcc ttgtggctgg acttgccacc catgaagctt   1140
   acggagatgg cctggagagt gtgtttccac tcatagccga gggccagcgc agtgccacgt   1200
   cacaggccat gcaccagctc ttcgggctgt ttgtcacact gatgtttgcc tctgtgggcg   1260
   ggggccttgg agggctcctg ctgaagctac cctttctgga ctccccccca gactcccagc   1320
   actacgagga ccaagttcac tggcaggtgc ctggcgagca tgaggataaa gcccagagac   1380
   ctctgagggt ggaggaggca gacactcagg cctaacccac tgccagcccc tgagaggaca   1440
   cgctcctttt cgaagatgct gactggctgc tactaggaag ttctttttga gctcccattc   1500
   ctccagctgc aagaagggag ccatgagcca gaaggaggcc cctttccaca ggcagcgtct   1560
   ccacagggag aggggcaaca ggaggctggg aaatggtggg gagtggggcc gtaactgggt   1620
   acaatagggg gaacctcacc agatgcccaa cccgactgcc ctaccagcct gcacatgggt   1680
   agaagaggcc aaattgaggc acccaagtga tccactggcc ccacgtcaca cagttacagt   1740
   gaagcccaag ccaggcctgg ttgagggtga taaacgccac tgtctctaaa aaaaaaaaaa   1800
   aaaaa                                                               1805
<210> 15
   <211> 3401
   <212> DNA
   <213> homo sapiens
<400> 15
   atggcttcgt tccccgagac cgatttccag atctgcttgc tgtgcaagga gatgtgcggc      60
   tcgccggcgc cgctctcctc caactcgtcc gcgtcgtcgt cctcctcgca gacgtccacg     120
   tcgtcggggg gcggcggcgg gggccctggg gcggcggcgc gccgcctaca cgtcctgccc     180
   tgcctgcacg ccttctgccg cccctgcctc gaggcgcacc ggctgccggc ggcgggcggc     240
   ggcgcggcgg gagagccgct caagctgcgc tgccccgtgt gcgaccagaa agtagtgcta     300
   gccgaggcgg cgggtatgga cgcgctgcct tcgtccgcct tcctgcttaa caacctgctc     360
   gacgcggtgg tggccactgc cgacgagccg ccgcccaaga acgggcgcgc cggcgctccg     420
   gcgggagcgg gcggccacag caaccaccgg caccacgctc accacgcgca cccgcgcgcg     480
   tccgcctccg cgccgccact cccgcaggcg ccgcagccgc ccgcgccttc ccgctcggca     540
   cccggcggcc ctgccgcttc cccgtcggcg ctgctgctcc gccgtcctca cggctgcagc     600
   tcgtgcgatg agggcaacgc agcttcttcg cgctgcctcg actgccagga gcacctgtgc     660
   gacaactgcg tccgagcgca ccagcgcgtg cgcctcacca aggaccacta catcgagcgc     720
   ggcccgccgg gtcccggtgc cgcagcagcg gcgcagcagc tcgggctcgg gccgcccttt     780
   cccggcccgc ccttctccat cctctcagtg tttcccgagc gcctcggctt ctgccagcac     840
   cacgacgacg aggtgctgca cctgtactgt gacacttgct ctgtacccat ctgtcgtgag     900
   tgcacaatgg gccggcatgg gggccacagc ttcatctacc tccaggaggc actgcaggac     960
   tcacgggcac tcaccatcca gctgctggca gatgcccagc agggacgaca ggcaatccag    1020
   ctgagcatcg agcaggccca gacggtggcg gaacaggtgg agatgaaggc gaaggttgtg    1080
   cagtcggagg tcaaagccgt gactgcgagg cataagaaag ccctggagga acgcgagtgt    1140
   gagctgctgt ggaaggtaga aaagatccgc caggtgaaag ccaagtctct gtacctgcag    1200
   gtggagaagc tgcggcaaaa cctcaacaag cttgagagca ccatcagtgc cgtgcagcag    1260
   gtcctggagg agggtagagc gctagacatc ctactggccc gagaccggat gctggcccag    1320
   gtgcaggagc tgaagaccgt gcggagcctc ctgcagcccc aggaagacga ccgagtcatg    1380
   ttcacacccc ccgatcaggc actgtacctt gccatcaagt cttttggctt tgttagcagc    1440
   ggggcctttg ccccactcac caaggccaca ggcgatggcc tcaagcgtgc cctccagggt    1500
   aaggtggcct ccttcacagt cattggttat gaccacgatg gtgagccccg cctctcagga    1560
   ggcgacctga tgtcggctgt ggtcctgggc cctgatggca acctgtttgg tgcagaggtg    1620
   agtgatcagc agaatgggac atacgtggtg agttaccgac cccagctgga gggtgagcac    1680
   ctggtatctg tgacactgtg caaccagcac attgagaaca gccctttcaa ggtggtggtc    1740
   aagtcaggcc gcagctacgt gggcattggg ctcccgggcc tgagcttcgg cagtgagggt    1800
   gacagcgatg gcaagctctg ccgcccttgg ggtgtgagtg tagacaagga gggctacatc    1860
   attgtcgccg accgcagcaa caaccgcatc caggtgttca agccctgcgg cgccttccac    1920
   cacaaattcg gcaccctggg ctcccggcct gggcagttcg accgaccagc cggcgtggcc    1980
   tgtgacgcct cacgcaggat cgtggtggct gacaaggaca atcatcgcat ccagatcttc    2040
   acgttcgagg gccagttcct cctcaagttt ggtgagaaag gaaccaagaa tgggcagttc    2100
   aactaccctt gggatgtggc ggtgaattct gagggcaaga tcctggtctc agacacgagg    2160
   aaccaccgga tccagctgtt tgggcctgat ggtgtcttcc taaacaagta tggcttcgag    2220
   ggggctctct ggaagcactt tgactcccca cggggtgtgg ccttcaacca tgagggccac    2280
   ttggtggtca ctgacttcaa caaccaccgg ctcctggtta ttcaccccga ctgccagtcg    2340
   gcacgctttc tgggctcgga gggcacaggc aatgggcagt tcctgcgccc acaaggggta    2400
   gctgtggacc aggaagggcg catcattgtg gcggattcca ggaaccatcg ggtacagatg    2460
   tttgaatcca acggcagctt cctgtgcaag tttggtgctc aaggcagcgg ctttgggcag    2520
   atggaccgcc cttccggcat cgccatcacc cccgacggaa tgatcgttgt ggtggacttt    2580
   ggcaacaatc gaatcctcgt cttctaattg catttcctag gtttctgtgt ttggggtgtg    2640
   tgtgcgtgtc tctctctctc tctctctctt tctctttctc tctctttttg aatttcaaag   2700
   aagaaacagt ctcagggaaa tttctttttt cttttttttt tttaaagaga acaagaaaag    2760
   tacaacattg cttaagtcct acctcatctt tattttttta cagatgaatg tacttatctt    2820
   ttctgcaggg attgagcctg tgaagtgata atttctatct acctcataaa tctttacatt    2880
   tccttctgca acaggccctc ttcccctcct cagtggagtt tgcatttccc tcttcccctg    2940
   cgtggggcat gatatgcaca agcctggcat ctgtatggct gggagggcac tggatgtgtg    3000
   tggtggggtg tattctgtag attgagccaa ggaaacacaa aaaaaaacta ctaagtaaaa    3060
   aaacaaaaaa ctataaaaca tggaaaaaat aggatttgaa atgcataatt atagaatacc    3120
   tgtgttcttg agaatactgt ttatatgggg tttagattat gttgtgttgt tttgatcttt    3180
   ttggaaaatc ttctcttttt aaatgctgca acagagaaat ttcctctgtt ctctgtttat    3240
   acctcttaat tgtattgtcc aaggcagaca tgatataagg aatatgcact accgtagtaa    3300
   ctcccctggc cgcagaaacc acactgcaag cctgtccggg gtggggtgct gactgccatt    3360
   tgccactttt aaatgggcac tgccgtggta atgtgaatcc c                        3401
<210> 16
   <211> 474
   <212> DNA
   <213> homo sapiens
<220>
   <221> misc_feature
   <222> (1)..(1)
   <223> n is a, g, c or t
<220>
   <221> misc_feature
   <222> (3)..(3)
   <223> n is a, g, c or t
<220>
   <221> misc_feature
   <222> (5)..(5)
   <223> n is a, g, c or t
<220>
   <221> misc_feature
   <222> (416)..(416)
   <223> n is a, g, c or t
<220>
   <221> misc_feature
   <222> (424)..(424)
   <223> n is a, g, c or t
<220>
   <221> misc_feature
   <222> (463)..(463)
   <223> n is a, g, c or t
<220>
   <221> misc_feature
   <222> (474)..(474)
   <223> n is a, g, c or t
<400> 16
   ntnantttgt cagtgaaacc atgtgtaagt ttattagaaa gttggatttt ttaacgacga     60
   aaagagaagg acccacacac cttaattttg tacctataag cttgcgttct gtctgcataa    120
   catagattta gcccagtctt agccttctgc gtctaacact ttcctaacta ttcattttaa    180
   gtctataagc atgggttaaa tgattacctc gtgcctgggc cttgtgttgg gggctctgag    240
   gaactctgca atctttttat tttcattttt tgagacaggg atcttgctct gtcacacagg    300
   gctgggagtg cagtgggtac catcacaggc tcactacagg cctcaacctc gtggggctca    360
   attgatccct cccacctcag gcctcttcaa gtaggctgag gttacagggc acatgncacc    420
   atgncggggg gtaatttttt gtaggttttt gtaggagaca ggntttcacc aagn          474
<210> 17
   <211> 10434
   <212> DNA
   <213> homo sapiens
<400> 17
   atggcgaacc ggcgagtggg gcgaggctgc tgggaagtga gcccgaccga gcggaggccg     60
   cccgcggggc tgcggggccc cgcggccgag gaggaggcgt cttccccgcc ggtcctgtct    120
   ctcagccact tctgcaggtc tcctttcctt tgcttcgggg acgttctcct gggagcctca    180
   cggacgctgt ctctggccct agacaaccct aacgaggagg tggcagaagt gaagatctcc    240
   cacttcccgg ccgcggacct gggcttcagt gtgtcgcagc gctgtttcgt gttgcagcct    300
   aaagagaaaa ttgttatttc tgttaactgg acaccactca aagaaggccg agtaagagag    360
   attatgacat ttcttgtaaa tgatgttctg aaacaccaag ctatattact aggaaatgca    420
   gaagagcaga aaaagaaaaa gaggagtctt tgggatacca ttaaaaagaa gaaaatttca    480
   gcctctacaa gtcacaacag aagggtttca aatattcaga atgttaataa aacatttagt    540
   gtttcccaaa aagttgacag agttaggagc ccactacaag cttgtgaaaa cttggctatg    600
   aatgaaggcg gtcccccaac agaaaacaat tctttaatac ttgaagaaaa taaaataccc    660
   atatcaccta ttagccctgc tttcaatgaa tgccatggtg caacttgctt gccactctct    720
   gtacgtcgat ctactaccta ctcatctctt catgcatcag aaaataggga actattaaat    780
   gtacacagtg ccaacgtttc aaaagtttct tttaatgaga aagctgtaac tgaaacttcc    840
   tttaattctg taaatgttaa tggccaaaga ggagagaata gtaaacttag tcttaccccc    900
   aactgttctt caactttgaa cattacacaa agccaaatac attttctaag tccagattct    960
   tttgtaaata atagtcatgg agctaataat gaactagaat tagtaacatg tctttcatca   1020
   gatatgttta tgaaagataa ttcacagcct gtgcatttgg aatcaacaat tgcacatgaa   1080
   atttatcaga aaattttaag tccagattct ttcataaaag ataattatgg actaaatcag   1140
   gatctagaat cagagtcagt taatcctatt ttatccccta atcaattttt aaaagataac   1200
   atggcatata tgtgtacatc tcagcaaaca tgtaaagtac cattatcaaa tgaaaattct   1260
   caagtcccac agtctcctga agattggaga aaaagtgaag tttcgccacg tattcctgaa   1320
   tgtcagggtt caaaatctcc caaagctatt tttgaagaac tagtagaaat gaagtcaaat   1380
   tactacagtt ttataaaaca aaataatcct aaattttctg cagttcagga tatttctagt   1440
   catagccaca ataaacaacc taagagacgt ccaatacttt ctgccactgt tactaaaagg   1500
   aaggccacct gtaccagaga aaaccaaact gagattaata aaccaaaagc aaaaagatgt   1560
   ctcaacagtg cagtgggtga acatgaaaaa gtaataaata atcaaaagga aaaagaagat   1620
   tttcattctt atcttccaat tatagatcca atattaagta aatctaagag ttataaaaac   1680
   gaggtaacac cctcttcgac aacagcttca gttgctcgga aaagaaagag cgatggaagc   1740
   atggaagatg caaatgtgag agttgcaatt acagaacata cagaagtgcg agaaatcaaa   1800
   agaatccatt tttctccctc agagcctaaa acatcagctg ttaagaaaac aaaaaatgtg   1860
   acaacaccca tctcaaaacg tattagcaac agagagaaat taaacctgaa gaagaaaact   1920
   gatttatcaa tattcagaac tccaatttct aaaacaaaca aaaggacaaa acccattatc   1980
   gctgtggcac agtccagttt gaccttcata aaaccattaa aaacagatat tcccagacac   2040
   ccgatgccat ttgctgcaaa aaacatgttt tatgatgaac gctggaagga aaagcaggaa   2100
   cagggcttca cttggtggtt aaattttata ttaacccctg atgacttcac tgtaaaaaca   2160
   aatatttctg aagtaaatgc tgctactctt cttttgggaa tagagaatca acataaaata   2220
   agtgttccta gagcacctac aaaagaggaa atgtctctca gagcttatac tgctcggtgt   2280
   aggttaaaca gactacgtcg tgcagcatgc cgtttgttta cttctgaaaa aatggttaaa   2340
   gctattaaaa agcttgaaat tgaaattgaa gctaggcggt taattgttcg aaaagataga   2400
   cacctatgga aagatgtggg agaacgtcag aaagtcctga attggctgtt gtcctacaat   2460
   cctttgtggc ttcgaattgg tctagagaca acttatggag aactcatatc tttggaagat   2520
   aacagtgatg tcacagggtt ggctatgttt attctgaatc gcctactttg gaatcctgat   2580
   atagcagctg agtatagaca ccccactgtt cctcacctgt atagagatgg tcatgaagaa   2640
   gctttgtcca agtttacatt gaaaaagtta ttgttgttgg tctgttttct tgattatgct   2700
   aaaatttcca gactcattga tcatgatcct tgtctcttct gtaaagatgc cgaattcaag   2760
   gctagtaaag aaatcctttt ggctttttca cgagatttcc taagtggtga aggtgacctt   2820
   tcccgtcacc ttggcttatt gggattacct gttaaccatg ttcagacacc atttgatgaa   2880
   tttgattttg ccgttacaaa tcttgccgta gacttgcaat gtggagtgcg ccttgtgcga   2940
   accatggaac ttctcacaca gaactgggac ctctcaaaga aactcaggat tccggcaata   3000
   agtcgtcttc aaaagatgca caatgttgac attgttcttc aagttcttaa atcacgagga   3060
   attgaattaa gtgatgagca tggaaataca attctatcta aggatattgt ggataggcac   3120
   agagaaaaaa ctctcaggtt gctttggaaa atagcgtttg cttttcaggt ggatatttcc 3180
   cttaacttag atcaattaaa ggaagaaatt gcctttctaa aacacacaaa gagtataaag 3240
   aaaacaatat ctctactatc atgccattct gatgatctta ttaataagaa aaaaggcaaa 3300
   agggatagtg gttcctttga acaatatagt gaaaacataa agttattgat ggattgggta 3360
   aatgctgttt gtgccttcta taataaaaag gtggagaatt ttacagtgtc tttctcagac 3420
   ggccgtgtgt tatgttacct gatccaccat taccatcctt gctatgtgcc atttgacgct 3480
   atatgtcagc gtactactca aactgtggaa tgtacgcaaa ctggttcagt ggtattaaat 3540
   tcatcatctg aatctgatga cagttctctg gatatgtcac ttaaagcatt tgatcatgaa 3600
   aatacttcag agctatacaa agagctccta gaaaatgaaa agaaaaattt tcacttggtt 3660
   aggtctgcag ttagagacct tggtggaata cctgctatga ttaatcattc agatatgtca 3720
   aatacaattc cagatgaaaa ggtggttatt acctatttgt catttctttg tgcaaggctt 3780
   ttggatcttc gtaaagaaat aagagctgct cgactcatac aaacaacatg gagaaaatat 3840
   aaactaaaaa cagatctcaa acgccatcag gagagagaga aagctgcaag aattattcaa 3900
   ttggctgtaa tcaattttct agcaaaacaa agattgagaa aaagagttaa tgcagcactc 3960
   gtcattcaga aatattggcg aagagtctta gcacagagaa aattattaat gttaaaaaag 4020
   gaaaagctgg aaaaagttca aaataaagca gcatcactta ttcagggata ttggagaaga 4080
   tattccacta gacaaagatt tctgaaattg aaatattatt caatcatcct gcaatctagg 4140
   ataagaatga taattgctgt tacatcttat aaacgatatc tttgggctac agttacaatt 4200
   cagaggcatt ggcgtgctta tttaagaaga aaacaagatc aacaaagata tgaaatgcta 4260
   aaatcatcaa ctcttataat ccaatctatg ttcagaaaat ggaagcaacg taaaatgcaa 4320
   tcacaagtaa aagctacagt aatattgcaa agagctttta gagaatggca tttaagaaaa 4380
   caagctaaag aagaaaattc tgctattatc atacaatcat ggtatagaat gcataaagaa 4440
   ttacggaagt atatttatat tagatcttgt gttgttatca ttcagaaaag atttcggtgc 4500
   tttcaagccc aaaagttata taaaagaaga aaagagtcca tactaaccat ccagaagtac 4560
   tacaaagcat atctgaaagg aaagattgag cgcaccaact atttgcagaa acgagctgca 4620
   gccattcaat tacaagctgc ttttaggaga ctgaaagctc ataatttatg tagacaaatt 4680
   agagctgctt gtgttattca gtcatactgg agaatgagac aagacagagt tcgattttta 4740
   aaccttaaga agactattat caaatttcag gcacatgtaa gaaaacatca acaacgacag 4800
   aaatataaga agatgaagaa agcagctgtt ataattcaga ctcatttccg agcttatatt 4860
   tttgccatga aagttctagc atcttaccag aaaacacgct ctgctgtcat tgtgctgcag 4920
   tctgcatata gagggatgca agccaggaaa atgtatattc acatcctcac atctgttata 4980
   aagattcaat catattatcg tgcttatgtt tctaaaaagg aatttttgag cctaaaaaat 5040
   gctacaataa aattgcagtc aactgttaag atgaaacaaa cacgtaaaca atatttgcat 5100
   ttaagagcag ctgcactatt tatccagcaa tgttaccgtt ccaaaaaaat agctgcacaa 5160
   aagagagaag agtatatgca gatgcgggaa tcttgtatca aactgcaagc atttgttaga 5220
   ggataccttg tccgaaagca gatgaggtta caaagaaaag ctgttatttc actacagtct 5280
   tatttcagaa tgagaaaggc tcggcagtat tatctgaaaa tgtataaagc aattattgtc 5340
   attcagaatt actatcatgc atacaaagca caggtcaatc agaggaagaa cttcttgcaa 5400
   gtcaaaaaag cagctacttg cttgcaagca gcttacagag gttataaagt acgccagcta 5460
   atcaaacaac aatctatagc tgctcttaaa attcagtctg cttttagagg ctataataaa 5520
   agggtaaaat atcaatctgt gcttcaatct ataataaaga ttcagagatg gtacagggcg 5580
   tacaagactc ttcatgatac aagaacacat tttttgaaga caaaggcagc tgtgatttcc 5640
   ctccagtctg cttatcgtgg ctggaaggtt cggaaacaga ttagaaggga acatcaagct 5700
   gccttgaaga ttcagtctgc ttttagaatg gccaaggccc agaaacagtt tagattgttt 5760
   aaaacagcag cattagtcat ccagcaaaat ttcagagcat ggactgcagg aaggaagcaa 5820
   tgtatggagt atattgaact ccgtcatgcg gtactggtgc ttcaatctat gtggaaggga 5880
   aaaacactga gaagacagct tcaaaggcaa cataaatgtg ctatcatcat acagtcatac 5940
   tatagaatgc atgtgcaaca aaagaagtgg aaaatcatga aaaaagctgc tcttctgatt 6000
   caaaagtatt atagggctta cagtattgga agagaacaga atcatttata tttgaaaaca 6060
   aaagcagctg tagtaacttt acagtcagct tatcgtggta tgaaagtgag aaaaagaata 6120
   aaggattgca acaaagcagc agtcactata cagtctaaat acagagctta caaaaccaaa 6180
   aagaaatatg caacctatag agcttcagct attataattc agagatggta tcgaggtatt 6240
   aaaattacaa accatcagca taaggagtat cttaatttga agaagacagc aattaaaatc 6300
   caatctgttt atagaggtat tagagttaga agacatattc aacacatgca cagggcagcc 6360
   acttttatta aagccatgtt taaaatgcat cagtcaagaa taagttacca tacaatgaga 6420
   aaagcagcta ttgttattca agtaagatgt agagcatatt atcaaggtaa aatgcagcgt 6480
   gaaaagtacc tgacaatttt gaaagctgtt aaagtccttc aggcaagttt tagaggagta 6540
   agagttagac ggactcttag aaagatgcag actgcagcaa cactcattca gtcaaactac 6600
   agaagataca gacagcaaac atactttaat aagttaaaga aaataacaaa aacagtacag 6660
   caaagatact gggcaatgaa agaaagaaac atacaatttc aaaggtataa caaactgagg 6720
   cattctgtaa tatacattca ggctattttt aggggaaaga aagctagaag acatttaaaa 6780
   atgatgcata tagccgcaac tctcattcag aggagattta gaactctaat gatgagaaga 6840
   agattcctct ctctcaagaa aactgctatt ttgattcaga gaaaatatcg ggcacatctt 6900
   tgtacaaagc atcacttaca gttccttcag gtacaaaatg cagttattaa aatccagtca    6960
   tcatacagaa gatggatgat aaggaaaagg atgcgagaga tgcacagggc tgctactttc    7020
   atccagtcta ctttcagaat gcacagatta catatgagat atcgagcttt gaaacaggcc    7080
   tccgttgtga tccaacagca ataccaagca aatagagctg caaaactgca gaggcagcat    7140
   tatctcagac aaagacactc tgctgtgatc cttcaggctg cattcagggg tatgaaaact    7200
   agaagacatt tgaagagtat gcattcctct gcaaccctta ttcagagtag gtttagatca    7260
   ttactggtga ggagaagatt catttccctc aaaaaagcta ctatttttgt tcagaggaaa    7320
   tatcgagcca ccatttgtgc caaacataaa ttgtaccaat tcttgcactt aagaaaggca    7380
   gccattacaa tacagtcatc ttacagaaga ctgatggtaa agaagaagtt acaagaaatg    7440
   caaagggctg cagttctcat tcaggctact ttcaggatgc acagaacata tattacattt    7500
   cagacttgga aacatgcttc aattctaatt cagcaacatt atcgaacata tagagctgca    7560
   aaattgcaaa gagaaaatta tatcagacaa tggcattctg ctgtggttat tcaggctgca    7620
   tataaaggaa tgaaagcaag acaactttta agggaaaaac acaaagcttc tatcgtaata    7680
   caaagcacct acagaatgta taggcagtat tgtttctacc aaaagcttca gtgggctaca    7740
   aaaatcatac aagaaaaata tagagcaaat aaaaagaaac agaaagtatt tcaacacaat    7800
   gaacttaaga aagagacttg tgttcaggca ggttttcagg acatgaacat aaaaaaacag    7860
   attcaggaac agcaccaggc tgccattatt attcagaagc attgtaaagc ctttaaaata    7920
   aggaagcatt atctccacct tagagcaaca gtagtttcta ttcaaagaag atacagaaaa    7980
   ctaactgcag tgcgtaccca agcagttatt tgtatacagt cttattacag aggctttaaa    8040
   gtacgaaagg atattcaaaa tatgcaccgg gctgccacac taattcagtc attctatcga    8100
   atgcacaggg ccaaagttga ttatgaaaca aagaaaactg caattgtggt tatacagaat    8160
   tattataggt tgtatgttag agtaaaaaca gaaagaaaaa actttttagc agttcagaaa    8220
   tctgtacgaa ctattcaggc tgcttttaga ggcatgaaag ttagacaaaa attgaaaaat    8280
   gtatcagagg aaaagatggc agccattgtt aaccaatctg cactctgctg ttacagaagt    8340
   aaaactcagt atgaagctgt tcaaagtgaa ggtgttatga ttcaagagtg gtataaagct    8400
   tctggccttg cttgttcaca ggaagcagag tatcattctc aaagtagggc tgcagtaaca    8460
   attcaaaaag ctttttgtag aatggtcaca agaaaactgg aaacacagaa atgtgctgcc    8520
   ctacggattc agttcttcct tcagatggct gtgtatcgga gaagatttgt tcagcagaaa    8580
   agagctgcta tcactttaca gcattatttt aggacgtggc aaaccagaaa acagttttta    8640
   ctatatagaa aagcagcagt ggttttacaa aatcactaca gagcatttct gtctgcaaaa    8700
   catcaaagac aagtctattt acagatcaga agcagtgtta tcattattca agctagaagt    8760
   aaaggattta tacagaaacg gaagtttcag gaaattaaaa atagcaccat aaaaattcag    8820
   gctatgtgga ggagatatag agccaagaaa tatttatgta aagtgaaagc tgcctgcaag    8880
   attcaagcct ggtatagatg ttggagagca cacaaagaat atctagctat attaaaagct    8940
   gttaaaatta ttcaaggttg cttctatacc aaactagaga gaacacggtt tttgaatgtg    9000
   agagcatcag caattatcat tcagagaaaa tggagagcta tacttcctgc aaagatagct    9060
   catgaacact tcttaatgat aaaaagacat cgagctgctt gtttgatcca agcacattat    9120
   agaggatata aaggaaggca ggtctttctt cggcagaaat ctgctgcttt gatcatacaa    9180
   aaatatatac gagccaggga ggctggaaag catgaaagga taaaatatat tgaatttaaa    9240
   aaatctacag ttatcctaca agcactggtg cgtggttggc tagtacgaaa aagattttta    9300
   gaacagagag ccaaaattcg acttcttcac ttcactgcag ctgcatatta tcacctgaat    9360
   gctgttagaa ttcaaagagc ctataaactt tacctggctg tgaagaatgc taacaagcag    9420
   gttaattcag tcatctgtat tcagagatgg tttcgagcaa gattacaaga aaagagattt    9480
   attcagaaat atcatagcat caaaaagatt gagcatgaag gtcaagaatg tctgagccag    9540
   cgaaataggg ctgcatcagt aatacagaaa gcagtgcgcc attttctcct ccgtaaaaag    9600
   caggaaaaat tcactagtgg aatcattaaa attcaggcat tatggagagg ctattcttgg    9660
   aggaagaaaa atgattgtac aaaaattaaa gctatacgac taagtcttca agttgttaat    9720
   agggagattc gagaagaaaa caaactctac aaaagaactg cacttgcact tcattacctt    9780
   ttgacatata agcacctttc tgccattctt gaggccttaa aacacctaga ggtagttact    9840
   agattgtctc cactttgttg tgagaacatg gcccagagtg gagcaatttc taaaatattt    9900
   gttttgatcc gaagttgtaa tcgcagtatt ccttgtatgg aagtcatcag atatgctgtg    9960
   caagtcttgc ttaatgtatc taagtatgag aaaactactt cagcagttta tgatgtagaa   10020
   aattgtatag atatactatt ggagcttttg cagatatacc gagaaaagcc tggtaataaa   10080
   gttgcagaca aaggcggaag catttttaca aaaacttgtt gtttgttggc tattttactg   10140
   aagacaacaa atagagcctc tgatgtacga agtaggtcca aagttgttga ccgtatttac   10200
   agtctctaca aacttacagc tcataaacat aaaatgaata ctgaaagaat actttacaag   10260
   caaaagaaga attcttctat aagcattcct tttatcccag aaacacctgt aaggaccaga   10320
   atagtttcaa gacttaagcc agattgggtt ttgagaagag ataacatgga agaaatcaca   10380
   aatcccctgc aagctattca aatggtgatg gatacgcttg gcattcctta ttag          10434
<210> 18
   <211> 1925
   <212> DNA
   <213> homo sapiens
<400> 18
   agaccgcgcg cggggcgagc gagcggggcg cggcgagggg caagggcggg gagggccccg     60
   gcgctcagag caggcgccag ggaggcaggc tgggcggccc ttcgtcctcg ccttcgggtg    120
   tccatggccg cggtggccgt cctccggaac gactcgctgc aggcctttct ccaggaccgc    180
   acccccagcg cctccccgga cctgggcaag cactcgcccc tggcattgct ggccgccacc    240
   tgtagccgca tcggccagcc gggcgcggcg gcgcccccgg acttcctgca ggtgccctac    300
   gaccccgcgc tgggctcacc ctccaggctc ttccacccgt ggaccgccga catgccggcg    360
   cactcgccag gcgcactgcc gcccccgcat cccagcttgg ggctgacgcc gcagaagacg    420
   cacctgcagc cgtccttcgg ggctgcgcac gagcttcccc ttacaccccc cgccgacccc    480
   tcgtacccct acgagttctc gccggtcaag atgctgccct cgagcatggc ggctctgccc    540
   gccagctgcg cgcccgccta cgtgccctac gcggcgcagg ccgcgctgcc gccaggctac    600
   tccaacctgc tgcctccgcc gccgccaccg cccccgccgc ccacctgccg ccagttgtca    660
   cccaacccgg cccccgacga cctcccgtgg tggagcatcc cgcaggcggg cgccgggccg    720
   ggggcctccg gggttccggg aagcggcctc tccggcgcct gtgccggggc cccccacgcg    780
   ccccgcttcc ccgcctctgc ggccgctgct gctgcggccg ccgccgccct acaaagaggc    840
   ctggtgttgg gcccgtcgga ctttgcgcag taccagagcc agatcgccgc gctgctgcag    900
   accaaggccc ccctggcggc cacggccagg aggtgccgcc gctgccgctg tcccaactgc    960
   caggcggcgg gcggcgcccc cgaggcggag ccggggaaga agaagcagca cgtgtgccac   1020
   gtgccgggct gcggcaaggt gtacgggaag acgtcgcacc tgaaggcgca cctgcgctgg   1080
   cacacgggcg agcgaccctt cgtgtgcaac tggctcttct gcgggaagag cttcacgcgc   1140
   tcggacgagc tgcagcggca cctgcggact cacacgggcg agaagcgctt tgcctgtccc   1200
   gagtgcggca agcgcttcat gcgcagcgac cacctcgcga agcacgtcaa gactcaccag   1260
   aataagaagc tcaaagtcgc tgaggccggg gttaagcggg aggacgcgcg ggacctgtga   1320
   gccctcccgg aggtggaccc ccttcccagc acctctgcga gagatccggg gacctgtggg   1380
   cagctggcgg aggggagact cagcagacgg accctctccg ttgcctgcct cccaaaatgg   1440
   agccaggctt ccaacttccg ctgccttcgg acatagggac ccagttccca ggagcgggga   1500
   ggtagggttg gggctggggc atttggattg taattgggag ctctgccgta cgccagggcg   1560
   gttccaaact ctaaaccgtt cccaccgtca gggagaccta cagtttcggg ggaccaccct   1620
   ggtctggcct tgtatatagg aaatgctgct gaactgaata gaaaggaact tgggagattt   1680
   gaaacagtgc tcgggttttc gctaggaccg gtttgggctt tgtacaggtt atttaatagc   1740
   tttgttaaag ataattataa taattataac attaataaaa atgttgcttt tgtcttcagc   1800
   tccatgcaga gctacagcat gatatgtctc tgtaaagtga tcagcagttg cagcgtgaaa   1860
   ataaatactt taactcaggg gtcactacag gaagaccccg ttgaaaaaaa aaaaaaaaaa   1920
   aaaaa                                                               1925
<210> 19
   <211> 1638
   <212> DNA
   <213> homo sapiens
<400> 19
   aagaattcgg cacgagtgaa aatgtaagat tatctgagta atggacaatt tctaatattc     60
   attttttaaa gtagctaact ctgccttatt atccccttcc acttttcccc tggatgggtg    120
   atttatataa tggaatgcag tgtcattgat tgtgtttgaa ggaggcacac taaatagcac    180
   actattgtta tcttgaaaat ttgttttatt ttgtttttgc ccatttttta aatcatttca    240
   ttctattttt gcccatgtct cttgtaatga gctacacatg taattagtac acagagttct    300
   ggtgatgtat tcattcatga gtttaggaag tgagattttc agcttcattt ccttctaagc    360
   atctcaacta ggagcacagg ggagacaaaa aagattgcaa cctctttata cagttgtgtt    420
   tgacccttgg cactctattc ttacttcaat tatcgtccat tgtaatgatc ccttttcatg    480
   tctagcttaa ccctaaactg tgagttcctt gagaataaga actttgtcac agtgatgttt    540
   taatatattc tcaccacaca ataaaggatc taagtgtgtg ctcaataaat gggtatttgt    600
   tgggaaattt aaacaagaaa tagtgaatta ttttctattc actatttgga taccctttct    660
   ccaaagatat tttttatttg agagtatctt atttacagta aaaatcacaa atattatata    720
   tacaatttag ttttgacaaa tgtctacatt tctatccttt acccaggcct tcaataagaa    780
   aataataata atttttttcc caaagacata atctggttat ttggttattt ggctgaaaca    840
   atgaatatct ttgatacatg gattctttaa taatgaatct gttatttagt atttagtcat    900
   tacttcataa tgattccaaa tttcctaata acttcttccc ccactatcca cccccatccc    960
   ctgcccctgc aaaaaagaca aaacaaatct ccctttccct caggaagtag ttgatttggt   1020
   gcctgtgtaa ggtagttcat gcatttcctt cttctgtttt gttaccatac cgtatgcttg   1080
   gcactcagta caccaagaaa acaaagaggc attgcctgtc tcgaagttgc ttagagtcta   1140
   gtgggggaga taatgcacac ctgcatccat agcacagcaa tgtgtacata ataacaaaga   1200
   tctgaaaacc ccagtcagtt ttctatgttt tgtcctcttt cacacccttc cgagagttct   1260
   atctaatgta actatgaatg gtcactgtgt ctttattttt ggcccagact cttctcaatg   1320
   cttcagcctc acgtatccaa ctacctactg attattctgc ttggacatac atcagccatt   1380
   ttaaatttgg cctgagttga actcattctc tttctctccc acattcattg tgttttcatt   1440
   tgttttcttg aaatctcctg gttaagtcag ttcaggttgc tatagaaaaa aattttgggt   1500
   ctggcgtggt gtcttgtgcc tgtaatccca gaatgttggg aagcctaggt gggcagattg   1560
   cttgagcctc aggagttcga gaccagccag ggtgattatg aggaaactct gtctctgcaa   1620
   aaaaaaaaaa aaaaaaaa                                                 1638
<210> 20
   <211> 2706
   <212> DNA
   <213> homo sapiens
<400> 20
   ccgtgtgcag tcgccccgcg ccccgcgcga cccttcgggt aaactacgaa ctgggagttc     60
   tgaagaatgg gtaaagactt tcgttactat ttccagcatc cctggtctcg catgattgtg    120
   gcttacttgg tgatcttctt taacttctta atatttgcgg aggacccagt ttctcatagc    180
   caaacagaag ccaatgttat tgttgttgga aactgttttt catttgttac aaataaatac    240
   cctagaggag ttggctggag gattttgaag gtgcttctat ggctacttgc cattctcaca    300
   ggactaatag ctggcaaatt tctgttccat cagcgtttgt ttggtcagtt gctccgatta    360
   aaaatgtttc gagaagatca tgggtcgtgg atgacaatgt tcttcagcac aattctcttt    420
   ctcttcatat tttctcacat atacaacacg attcttctaa tggatgggaa catgggagca    480
   tatatcatta cagactatat gggcatccga aatgaaagtt tcatgaaatt agctgcagta    540
   gggacctgga tgggggactt tgtcacagct tggatggtca ctgatatgat gcttcaggac    600
   aaaccctatc ctgactgggg aaaatcagca agagctttct ggaagaaagg aaatgttagg    660
   atcactttat tctggacagt tctttttact ctgacgtctg tggttgtact tgtgattaca    720
   acggactgga tcagctggga caagctgaat cggggatttt tgcccagtga tgaagtttcc    780
   agagcattcc ttgcttcttt tatcttggtc tttgaccttc ttattgtgat gcaggactgg    840
   gaattcccac atttcatggg agatgttgat gtaaatctcc ctggtttgca cacccctcac    900
   atgcagttca agattccttt cttccagaaa atcttcaagg aggaatatcg tattcacata    960
   acaggcaaat ggtttaacta tggaattatc ttcctcgtct tgattttgga tcttaatatg   1020
   tggaagaacc aaatatttta taaacctcat gaatatgggc aatatatcgg cccggggcag   1080
   aagatatata cagtgaaaga ctcagaaagt ttaaaagatt tgaacagaac caagctatcc   1140
   tgggaatgga ggtccaatca cactaaccct cggactaata aaacatatgt tgagggagac   1200
   atgttcttac acagcaggtt cataggagcc agtcttgatg tcaagtgtct ggcctttgtt   1260
   ccaagcctga tagcctttgt gtggtttgga ttctttattt ggttctttgg acgatttttg   1320
   aaaaatgagc cacgcatgga gaatcaagac aaaacttaca ctcgcatgaa aagaaaatct   1380
   ccatcagaac atagcaaaga catgggaatc actcgagaaa acacccaggc ttcagtagaa   1440
   gaccccttga atgacccttc tttggtttgc atcaggtctg acttcaatga gatcgtctac   1500
   aagtcttccc acctaacctc ggaaaacttg agctcacagt tgaacgaatc tactagtgca   1560
   acagaagctg atcaagaccc aacgacttct aaaagtacac ctacgaacta gactcggaga   1620
   tagacttgga gataacacaa aaagcaacct tgagtgtaac tttaaaaatt tagtctttcc   1680
   ttttgtatat gtaaggttta cgtagtgtta ggtaaaaata tgaacaatgc cacaacggtg   1740
   ctcaacatgc tttttctagg attcattgtt ttctatttgt attataatac acgtgcctac   1800
   tgtatactca acagtcctct agagattgct tttcacaatt gcacaagcta ttactgactt   1860
   tacagcatag tggaagatta gctgatgacc catgtatctg atgttcaacc atagtggtgc   1920
   cttgagacat taaactgttt ttaactgtac cagaaatgaa gtgtggaaca gttacctaac   1980
   ctatttcaca tgggcgtttt gtatacaact attttgatct acacttgatg tctgagcaga   2040
   aaacagaaat agctaaatgt gactcaggaa gtatctcttg gtttcttatt cagcagcaga   2100
   gttggtgact ttgacaactg gactgcagag aaacatggtg atcacctttt aatttttatt   2160
   ggctgtctgc caaatataaa tacagatgca aaattcagta ataggagatc cataacccaa   2220
   catgggtcac tactcgtgaa atgtgacttt ctcccaccag taattgaaat gaggtgatga   2280
   tacctaatta tgttttccta attaaagata aattgctact tgattaaaaa tcctgccctt   2340
   cacctttggg aacaaaggtt aagagacaca gttgggcgaa ctctcaaatt tattggcatt   2400
   tacacaaagt cccagacaac caaggaactg aagttttcat catatgagag cagcacatcc   2460
   caccatttac aatattcgta tatctttctg caaatatggc tctggatagt gaaaattgaa   2520
   aaacatatgc caaccctgag caagggaact cctcaaaaaa tcatgcagcg gaaccttgtc   2580
   aggtagagaa gccgtgcatg aaagaatttg tttaatgtct tgttttgcgt atgtgttttt   2640
   tgtttttgtt ttttaagaac taaatattgc acattaataa ataagaatta tacagcaaaa   2700
   aaaaaa                                                              2706
<210> 21
   <211> 3110
   <212> DNA
   <213> homo sapiens
<400> 21
   tcgccggctg cggcgcctgg gacggttgcg gtgggtctgg gcgctgggaa gtcgtccaag     60
   atgattaaaa aattcgacaa gaaggacgag gagtctggta gtggctccaa tcctttccag    120
   catctggaga agagtgctgt tttacaggag gctcgtatat tcaatgaaac tccaatcaat    180
   ccaagaagat gtttgcatat tcttacaaag attctttact tactgaacca gggtgaacac    240
   tttggaacaa cggaagctac agaagccttc tttgcaatga cgcgattgtt tcaatctaat    300
   gatcaaacat tgaggagaat gtgctacctt accatcaaag aaatggctac catctctgag    360
   gatgtgataa ttgtcacaag cagtctgact aaagacatga ctggaaaaga agatgtatac    420
   cgaggcccgg ccatcagagc tctctgcagg atcaccgatg gaacaatgtt gcaagccatt    480
   gaaagataca tgaagcaggc cattgtggat aaagtttcca gtgtatccag ttcagcactg    540
   gtatcttccc tgcacatgat gaagataagc tatgatgtgg ttaagcgctg gatcaatgaa    600
   gcccaagaag ctgcatcaag tgataatatt atggtccagt accatgcatt gggagtcctg    660
   tatcacctta gaaagaatga tcgacttgct gtttccaaga tgttgaataa gtttactaaa    720
   tctggtctca agtcacagtt tgcttactgc atgctgatcc gaattgccag tcgcttacta    780
   aaagaaactg aggatggcca tgaaagtcca ctgtttgatt tcattgagag ctgcttgcga    840
   aataaacatg aaatggttat ttatgaagct gcttcagcta tcatccatct tcctaactgc    900
   actgcaagag agttggcacc tgctgtttca gttcttcaac ttttctgtag ttctcctaag    960
   ccagccttga gatatgcagc tgtgaggacc ttgaacaagg tggcaatgaa gcacccctct   1020
   gctgttactg cctgcaatct ggacttagaa aacttaatca cagactcaaa cagaagcatt   1080
   gctaccttag ccattactac actcctcaaa acaggaagtg agagcagtgt ggaccggctc   1140
   atgaagcaga tatcttcttt tgtgtctgaa atctcagatg agttcaaggt ggtggttgta   1200
   caggcaatta gtgctctctg tcagaaatac cctcgaaagc acagtgtcat gatgactttc   1260
   ctctccaaca tgctccgaga tgatggaggc tttgagtaca agcgggccat tgtggactgt   1320
   ataatcagca ttgtggaaga gaaccctgag agtaaagaag caggcctagc ccacctttgt   1380
   gaattcattg aggactgtga acacactgtt ctggctacta agattctaca cttgttgggc   1440
   aaagagggcc ctagaacgcc tgtcccctcc aaatatatcc gttttatttt taatagggtt   1500
   gtcctggaga atgaggctgt cagagctgct gctgtgagtg ctttggctaa atttggggct   1560
   cagaatgaga gtcttctccc aagcatcctt gtactcttac agaggtgtat gatggatact   1620
   gatgacgagg tacgagacag agctaccttc tatctgaatg tgctgcagca gaggcagatg   1680
   gcactaaatg ccacatatat ctttaatggt ttgacggtct ctgtaccagg gatggaaaaa   1740
   gccttacacc agtacacgtt ggagccttca gaaaaaccgt ttgacatgaa atcaattcct   1800
   cttgctatgg ctcctgtctt tgaacagaaa gcagaaatca cacttgtggc tactaagcca   1860
   gagaagttgg ctccttccag gcaagacatt ttccaagaac aattggctgc cattcctgag   1920
   tttctgaata taggaccctt gttcaagtct tctgagcctg ttcaacttac agaagcagag   1980
   acagaatatt ttgttcgatg tatcaagcac atgtttacca atcacatcgt gttccagttt   2040
   gactgcacca acactctcaa tgaccagctg ctggaaaaag tgacagtgca gatggagcca   2100
   tcagattcct atgaagtgct gtcttgtatc ccagccccca gccttcctta taaccaacca   2160
   ggaatatgtt acactcttgt tcgtttgcct gatgatgacc ctacagcagt tgcaggctcc   2220
   tttagctgca ccatgaagtt tacagtccgg gactgtgacc ctaacactgg agttccagat   2280
   gaggatgggt atgatgatga gtatgtgctg gaagatctcg aagtgactgt gtctgaccat   2340
   attcagaaag tactgaagcc taactttgct gctgcttggg aagaggtggg agataccttt   2400
   gagaaagagg aaacctttgc cctcagttct accaaaaccc ttgaagaggc tgtcaacaat   2460
   atcatcacat ttctgggcat gcagccatgt gagaggtccg ataaagtacc tgagaacaag   2520
   aattcccatt cgctctatct ggcaggtata ttcagaggtg gctatgattt attggtgagg   2580
   tccaggctgg ccttagccga tggagtgacc atgcaggtga ctgtcagaag taaagagaga   2640
   acacctgtag atgttatctt agcttctgtt ggataaatgc ttactggaca agaggaaact   2700
   gatgcacact acatggtcag tgggctttta ggctagtggc atcagtttcc cagaatcaga   2760
   cttttgaaga tgaatgactt tggagaagca aattaaacat ttggccctga gccagcagat   2820
   caagcaaatg tctatctttg cgcatgggtt gttttttttt tttttctttt tattctactt   2880
   ggtcagcttt gggacgatag tgcagctttg ggtgatcttg aaaatcaaat actatcctat   2940
   actccagctg cttaacttca ttttattctt taatgtgtac ctgaaagctc ctggcaatgc   3000
   tggaaaattt ttatcccaga ggggtggggg ggagggggga ggggaagcca gagtccactt   3060
   ttgtcacaat tcatttttat taatagaaaa taaacactta ttccagtttc              3110
<210> 22
   <211> 1723
   <212> DNA
   <213> homo sapiens
<400> 22
   tagataaaag caaagataat atttcattgg ttacagttat acagttacac agttatacag      60
   ttgccttatt tggtctatcc catgaggaag tcctagttac taattacgtt tttgttggct     120
   gcttctgatt ggttgagctt aagttctgtg tttctttaac ataggcattt acaagaaata     180
   ccacaaataa agtttcagac atgcttgcaa atcaagcaag gttaaggtca cttaggaggc     240
   ccaactggct ctgtctgctc aaggattctt ctggcctcgt ctccatttta catgaactgt     300
   tgcataaata aacacagagt acctgaaaca acggaggtga tcattctgcc taccgagtgt     360
   tggccacgcc aagcttggag tgttgctctt attcttaggg agtttatttt taagtaatct     420
   catctgtaaa tgggattaca atccacaaac tgaccttgta tatgattcca ttccttctcc     480
   cagcccagcc ccacactcca aggttttccc tttgcttata aggggtagtc accctttttt     540
   atttcgacct tccaaacatt ctgggagttt tcctccttta ggccaactac agcgcagagg     600
   agcgctttct cctgctgggt ttctccgact ggccttccct gcagccggtc ctcttcgccc     660
   ttgtcctcct gtgctacctc ctgaccttga cgggcaactc ggcgctggtg ctgctggcgg     720
   tgcgcgaccc gcgcctgcac acgcccatgt actacttcct ctgccacctg gccttggtag     780
   acgcgggctt cactactagc gtggtgccgc cgctgctggc caacctgcgc ggaccagcgc     840
   tctggctgcc gcgcagccac tgcacggccc agctgtgcgc atcgctggct ctgggttcgg     900
   ccgaatgcgt cctcctggcg gtgatggctc tggaccgcgc ggccgcagtg tgccgcccgc     960
   tgcgctatgc ggggctcgtc tccccgcgcc tatgtcgcac gctggccagc gcctcctggc    1020
   taagcggcct caccaactcg gttgcgcaaa ccgcgctcct ggctgagcgg ccgctgtgcg    1080
   cgccccgcct gctggaccac ttcatctgtg agctgccggc gttgctcaag ctggcctgcg    1140
   gaggcgacgg agacactacc gagaaccaga tgttcgccgc ccgcgtggtc atcctgctgc    1200
   tgccgtttgc cgtcatcctg gcctcctacg gtgccgtggc ccgagctgtc tgttgcatgc    1260
   ggttcagcgg aggccggagg agggcggtgg gcacgtgtgg gtcccacctg acagccgtct    1320
   gcctgttcta cggctcggcc atctacacct acctgcagcc cgcgcagcgc tacaaccagg    1380
   cacggggcaa gttcgtatcg ctcttctaca ccgtggtcac acctgctctc aacccgctca    1440
   tctacaccct caggaataag aaagtgaagg gggcagcgag gaggctgctg cggagtctgg    1500
   ggagaggcca ggctgggcag tgagtagttg gggaggggag aaagtattaa gccagaaccc    1560
   aaggatggaa atacccctta gtgagtcagt ttagacttca ggctgttcat ttttgtatga    1620
   taatctgcaa gatttgtcct aaggagtcca atgggggata tgttttcctc ccgtgaggaa    1680
   atgtttagtt cttgagggaa aaatccctaa atcctctata tac                       1723
<210> 23
   <211> 545
   <212> DNA
   <213> homo sapiens
<400> 23
   tttaatagtt agactcatac tttattttga caaatttaag atagaaaaat atcataatgt      60
   gaatatagca gttgctcttt ttgtaacatg gtttgggatg tgcagtgaaa cttgaaagga     120
   cttgctttac aggtggtccc tcttctggct gggtttcagt taattctgaa ttatattcca     180
   gccattgcat ttgcttgaaa gaatattgga cacagtaaaa aaaagaacag gtttggcatt     240
   caataataaa tattataaag caatgaacca aaacaacttt taaaataatt actgaaagca     300
   aacttcagac ttcatgatta aagctaagaa ctcatatttt caaaatagct ttaacagttt     360
   ctatcaatat ataatacaat agtaggacac ttatttttaa aaaacaagtg agtagaatca     420
   gagtaaatat gatatttcag atgactataa acagtaaaca tcaattcaat atatttatat     480
   atcatttcag caatatactc tgtgcccagc tggcgataaa aactgtagtt ctatcatcaa     540
   aaaat                                                                 545
<210> 24
   <211> 2880
   <212> DNA
   <213> homo sapiens
<400> 24
   tgctgctctc cgcccgcgtc cggctcgtgg ccccctactt cgggcaccat ggacacctcc     60
   cggctcggtg tgctcctgtc cttgcctgtg ctgctgcagc tggcgaccgg gggcagctct    120
   cccaggtctg gtgtgttgct gaggggctgc cccacacact gtcattgcga gcccgacggc    180
   aggatgttgc tcagggtgga ctgctccgac ctggggctct cggagctgcc ttccaacctc    240
   agcgtcttca cctcctacct agacctcagt atgaacaaca tcagtcagct gctcccgaat    300
   cccctgccca gtctccgctt cctggaggag ttacgtcttg cgggaaacgc tctgacatac    360
   attcccaagg gagcattcac tggcctttac agtcttaaag ttcttatgct gcagaataat    420
   cagctaagac acgtacccac agaagctctg cagaatttgc gaagccttca atccctgcgt    480
   ctggatgcta accacatcag ctatgtgccc ccaagctgtt tcagtggcct gcattccctg    540
   aggcacctgt ggctggatga caatgcgtta acagaaatcc ccgtccaggc ttttagaagt    600
   ttatcggcat tgcaagccat gaccttggcc ctgaacaaaa tacaccacat accagactat    660
   gcctttggaa acctctccag cttggtagtt ctacatctcc ataacaatag aatccactcc    720
   ctgggaaaga aatgctttga tgggctccac agcctagaga ctttagattt aaattacaat    780
   aaccttgatg aattccccac tgcaattagg acactctcca accttaaaga actaggattt    840
   catagcaaca atatcaggtc gatacctgag aaagcatttg taggcaaccc ttctcttatt    900
   acaatacatt tctatgacaa tcccatccaa tttgttggga gatctgcttt tcaacattta    960
   cctgaactaa gaacactgac tctgaatggt gcctcacaaa taactgaatt tcctgattta   1020
   actggaactg caaacctgga gagtctgact ttaactggag cacagatctc atctcttcct   1080
   caaaccgtct gcaatcagtt acctaatctc caagtgctag atctgtctta caacctatta   1140
   gaagatttac ccagtttttc agtctgccaa aagcttcaga aaattgacct aagacataat   1200
   gaaatctacg aaattaaagt tgacactttc cagcagttgc ttagcctccg atcgctgaat   1260
   ttggcttgga acaaaattgc tattattcac cccaatgcat tttccacttt gccatcccta   1320
   ataaagctgg acctatcgtc caacctcctg tcgtcttttc ctataactgg gttacatggt   1380
   ttaactcact taaaattaac aggaaatcat gccttacaga gcttgatatc atctgaaaac   1440
   tttccagaac tcaaggttat agaaatgcct tatgcttacc agtgctgtgc atttggagtg   1500
   tgtgagaatg cctataagat ttctaatcaa tggaataaag gtgacaacag cagtatggac   1560
   gaccttcata agaaagatgc tggaatgttt caggctcaag atgaacgtga ccttgaagat   1620
   ttcctgcttg actttgagga agacctgaaa gcccttcatt cagtgcagtg ttcaccttcc   1680
   ccaggcccct tcaaaccctg tgaacacctg cttgatggct ggctgatcag aattggagtg   1740
   tggaccatag cagttctggc acttacttgt aatgctttgg tgacttcaac agttttcaga   1800
   tcccctctgt acatttcccc cattaaactg ttaattgggg tcatcgcagc agtgaacatg   1860
   ctcacgggag tctccagtgc cgtgctggct ggtgtggatg cgttcacttt tggcagcttt   1920
   gcacgacatg gtgcctggtg ggagaatggg gttggttgcc atgtcattgg ttttttgtcc   1980
   atttttgctt cagaatcatc tgttttcctg cttactctgg cagccctgga gcgtgggttc   2040
   tctgtgaaat attctgcaaa atttgaaacg aaagctccat tttctagcct gaaagtaatc   2100
   attttgctct gtgccctgct ggccttgacc atggccgcag ttcccctgct gggtggcagc   2160
   aagtatggcg cctcccctct ctgcctgcct ttgccttttg gggagcccag caccatgggc   2220
   tacatggtcg ctctcatctt gctcaattcc ctttgcttcc tcatgatgac cattgcctac   2280
   accaagctct actgcaattt ggacaaggga gacctggaga atatttggga ctgctctatg   2340
   gtaaaacaca ttgccctgtt gctcttcacc aactgcatcc taaactgccc tgtggctttc   2400
   ttgtccttct cctctttaat aaaccttaca tttatcagtc ctgaagtaat taagtttatc   2460
   cttctggtgg tagtcccact tcctgcatgt ctcaatcccc ttctctacat cttgttcaat   2520
   cctcacttta aggaggatct ggtgagcctg agaaagcaaa cctacgtctg gacaagatca   2580
   aaacacccaa gcttgatgtc aattaactct gatgatgtcg aaaaacagtc ctgtgactca   2640
   actcaagcct tggtaacctt taccagctcc agcatcactt atgacctgcc tcccagttcc   2700
   gtgccatcac cagcttatcc agtgactgag agctgccatc tttcctctgt ggcatttgtc   2760
   ccatgtctct aattaatatg tgaaggaaaa tgttttcaaa ggttgagaac ctgaaaatgt   2820
   gagattgagt atatcagagc agtaattaat aagaagagct gaggtgaaac tcggtttaaa   2880
<210> 25
   <211> 3789
   <212> DNA
   <213> homo sapiens
<400> 25
   ctctcagtac cacgccggtg gccagttctg aggctagcac cctttcaaca actcctgttg 60
   acaccagcac acctgtgacc acttcttctc caaccaattc atctcctaca actgctgaag 120
   ttaccagcat gccaacatca actgctggtg aaggaagcac tccattaaca aatatgcctg 180
   tcagcaccac accggtggcc agttctgagg ctagcaccct ttcaacaact cctgttgact 240
   ccaacacttt tgttaccagt tctagtcaag ccagttcatc tccagcaact cttcaggtca 300
   ccactatgcg tatgtctact ccaagtgaag gaagctcttc attaacaact atgctcctca 360
   gcagcacata tgtgaccagt tctgaggcta gcacaccttc cactccttct gttgacagaa 420
   gcacacctgt gaccacttct actcagagca attctactcc tacacctcct gaagttatca 480
   ccctgccaat gtcaactcct agtgaagtaa gcactccatt aaccattatg cctgtcagca 540
   ccacatcggt gaccatttct gaggctggca cagcttcaac acttcctgtt gacaccagca 600
   cacctgtgat cacttctacc caagtcagtt catctcctgt gactcctgaa ggtaccacca 660
   tgccaatctg gacgcctagt gaaggaagca ctccattaac aactatgcct gtcagcacca 720
   cacgtgtgac cagctctgag ggtagcaccc tttcaacacc ttctgttgtc accagcacac 780
   ctgtgaccac ttctactgaa gccatttcat cttctgcaac tcttgacagc accaccatgt 840
   ctgtgtcaat gcccatggaa ataagcaccc ttgggaccac tattcttgtc agtaccacac 900
   ctgttacgag gtttcctgag agtagcaccc cttccatacc atctgtttac accagcatgt 960
   ctatgaccac tgcctctgaa ggcagttcat ctcctacaac tcttgaaggc accaccacca 1020
   tgcctatgtc aactacgagt gaaagaagca ctttattgac aactgtcctc atcagcccta 1080
   tatctgtgat gagtccttct gaggccagca cactttcaac acctcctggt gataccagca 1140
   cacctttgct cacctctacc aaagccggtt cattctccat acctgctgaa gtcactacca 1200
   tacgtatttc aattaccagt gaaagaagca ctccattaac aactctcctt gtcagcacca 1260
   cacttccaac tagctttcct ggggccagca tagcttcgac acctcctctt gacacaagca 1320
   caacttttac cccttctact gacactgcct caactcccac aattcctgta gccaccacca 1380
   tatctgtatc agtgatcaca gaaggaagca cacctgggac aaccattttt attcccagca 1440
   ctcctgtcac cagttctact gctgatgtct ttcctgcaac aactggtgct gtatctaccc 1500
   ctgtgataac ttccactgaa ctaaacacac catcaacctc cagtagtagt accaccacat 1560
   ctttttcaac tactaaggaa tttacaacac ccgcaatgac tactgcagct cccctcacat 1620
   atgtgaccat gtctactgcc cccagcacac ccagaacaac cagcagaggc tgcactactt 1680
   ctgcatcaac gctttctgca accagtacac ctcacacctc tacttctgtc accacccgtc 1740
   ctgtgacccc ttcatcagaa tccagcaggc cgtcaacaat tacttctcac accatcccac 1800
   ctacatttcc tcctgctcac tccagtacac ctccaacaac ctctgcctcc tccacgactg 1860
   tgaaccctga ggctgtcacc accatgacca ccaggacaaa acccagcaca cggaccactt 1920
   ccttccccac ggtgaccacc accgctgtcc ccacgaatac tacaattaag agcaacccca 1980
   cctcaactcc tactgtgcca agaaccacaa catgctttgg agatgggtgc cagaatacgg   2040
   cctctcgctg caagaatgga ggcacctggg atgggctcaa gtgccagtgt cccaacctct   2100
   attatgggga gttgtgtgag gaggtggtca gcagcattga catagggcca ccggagacta   2160
   tctctgccca aatggaactg actgtgacag tgaccagtgt gaagttcacc gaagagctaa   2220
   aaaaccactc ttcccaggaa ttccaggagt tcaaacagac attcacggaa cagatgaata   2280
   ttgtgtattc cgggatccct gagtatgtcg gggtgaacat cacaaagcta cgtcttggca   2340
   gtgtggtggt ggagcatgac gtcctcctaa gaaccaagta cacaccagaa tacaagacag   2400
   tattggacaa tgccaccgaa gtagtgaaag agaaaatcac aaaagtgacc acacagcaaa   2460
   taatgattaa tgatatttgc tcagacatga tgtgtttcaa caccactggc acccaagtgc   2520
   aaaacattac ggtgacccag tacgaccctg aagaggactg ccggaagatg gccaaggaat   2580
   atggagacta cttcgtagtg gagtaccggg accagaagcc atactgcatc agcccctgtg   2640
   agcctggctt cagtgtctcc aagaactgta acctcggcaa gtgccagatg tctctaagtg   2700
   gacctcagtg cctctgcgtg accacggaaa ctcactggta cagtggggag acctgtaacc   2760
   agggcaccca gaagagtctg gtgtacggcc tcgtgggggc aggggtcgtg ctgatgctga   2820
   tcatcctggt agctctcctg atgctcgttt tccgctccaa gagagaggtg aaacggcaaa   2880
   agtacagatt gtctcagtta tacaagtggc aagaagagga cagtggacca gctcctggga   2940
   ccttccaaaa cattggcttt gacatctgcc aagatgatga ttccatccac ctggagtcca   3000
   tctatagtaa tttccagccc tccttgagac acatagaccc tgaaacaaag agatccgaat   3060
   tcagaggcct caggtaatga cgacatcatt ttaaggcatg gagctgagaa gtctgggagt   3120
   gaggagatcc cagtccggct aagcttggtg gagcattttc ccattgagag ccttccatgg   3180
   gaactcaatg ttcccattgt aagtacagga aacaagccct gtacttacca aggagaaaga   3240
   ggagagacag cagtgctggg agattctcaa atagaaaccc gtggacgctc caatgggctt   3300
   gtcatgatat caggctaggc tttcctgctc atttttcaaa gacgctccag atttgagggt   3360
   actctgactg caacatcttt caccccattg atcgccagga ttgatttggt tgatctggct   3420
   gagcaggcgg gtgtccccgt cctccctcac tgccccatat gtgtccctcc taaagctgca   3480
   tgctcagttg aagaggacga gaggacgacc ttctctgata gaggaggacc acgcttcagt   3540
   caaaggcata caagtatcta tctggacttc cctgctagca cttccaaaca agctcagaga   3600
   tgttcctccc ctcatctgcc cgggttcagt accatggaca gcgccctcga cccgctgttt   3660
   acaaccatga ccccttggac actggactgc atgcacttta catatcacaa aatgctctca   3720
   taagaattat tgcataccat cttcatgaaa aacacctgta tttaaatata gagcatttac   3780
   cttttggta                                                           3789
<210> 26
   <211> 4711
   <212> DNA
   <213> homo sapiens
<400> 26
   gccccgggaa gcgcagccat ggctctgcgg aggctggggg ccgcgctgct gctgctgccg     60
   ctgctcgccg ccgtggaaga aacgctaatg gactccacta cagcgactgc tgagctgggc    120
   tggatggtgc atcctccatc agggtgggaa gaggtgagtg gctacgatga gaacatgaac    180
   acgatccgca cgtaccaggt gtgcaacgtg tttgagtcaa gccagaacaa ctggctacgg    240
   accaagttta tccggcgccg tggcgcccac cgcatccacg tggagatgaa gttttcggtg    300
   cgtgactgca gcagcatccc cagcgtgcct ggctcctgca aggagacctt caacctctat    360
   tactatgagg ctgactttga ctcggccacc aagaccttcc ccaactggat ggagaatcca    420
   tgggtgaagg tggataccat tgcagccgac gagagcttct cccaggtgga cctgggtggc    480
   cgcgtcatga aaatcaacac cgaggtgcgg agcttcggac ctgtgtcccg cagcggcttc    540
   tacctggcct tccaggacta tggcggctgc atgtccctca tcgccgtgcg tgtcttctac    600
   cgcaagtgcc cccgcatcat ccagaatggc gccatcttcc aggaaaccct gtcgggggct    660
   gagagcacat cgctggtggc tgcccggggc agctgcatcg ccaatgcgga agaggtggat    720
   gtacccatca agctctactg taacggggac ggcgagtggc tggtgcccat cgggcgctgc    780
   atgtgcaaag caggcttcga ggccgttgag aatggcaccg tctgccgagg ttgtccatct    840
   gggactttca aggccaacca aggggatgag gcctgtaccc actgtcccat caacagccgg    900
   accacttctg aaggggccac caactgtgtc tgccgcaatg gctactacag agcagacctg    960
   gaccccctgg acatgccctg cacaaccatc ccctccgcgc cccaggctgt gatttccagt   1020
   gtcaatgaga cctccctcat gctggagtgg acccctcccc gcgactccgg aggccgagag   1080
   gacctcgtct acaacatcat ctgcaagagc tgtggctcgg gccggggtgc ctgcacccgc   1140
   tgcggggaca atgtacagta cgcaccacgc cagctaggcc tgaccgagcc acgcatttac   1200
   atcagtgacc tgctggccca cacccagtac accttcgaga tccaggctgt gaacggcgtt   1260
   actgaccaga gccccttctc gcctcagttc gcctctgtga acatcaccac caaccaggca   1320
   gctccatcgg cagtgtccat catgcatcag gtgagccgca ccgtggacag cattaccctg   1380
   tcgtggtccc agccggacca gcccaatggc gtgatcctgg actatgagct gcagtactat   1440
   gagaaggagc tcagtgagta caacgccaca gccataaaaa gccccaccaa cacggtcacc   1500
   gtgcagggcc tcaaagccgg cgccatctat gtcttccagg tgcgggcacg caccgtggca   1560
   ggctacgggc gctacagcgg caagatgtac ttccagacca tgacagaagc cgagtaccag   1620
   acaagcatcc aggagaagtt gccactcatc atcggctcct cggccgctgg cctggtcttc   1680
   ctcattgctg tggttgtcat cgccatcgtg tgtaacagaa gacgggggtt tgagcgtgct   1740
   gactcggagt acacggacaa gctgcaacac tacaccagtg gccacatgac cccaggcatg   1800
   aagatctaca tcgatccttt cacctacgag gaccccaacg aggcagtgcg ggagtttgcc   1860
   aaggaaattg acatctcctg tgtcaaaatt gagcaggtga tcggagcagg ggagtttggc   1920
   gaggtctgca gtggccacct gaagctgcca ggcaagagag agatctttgt ggccatcaag   1980
   acgctcaagt cgggctacac ggagaagcag cgccgggact tcctgagcga agcctccatc   2040
   atgggccagt tcgaccatcc caacgtcatc cacctggagg gtgtcgtgac caagagcaca   2100
   cctgtgatga tcatcaccga gttcatggag aatggctccc tggactcctt tctccggcaa   2160
   aacgatgggc agttcacagt catccagctg gtgggcatgc ttcggggcat cgcagctggc   2220
   atgaagtacc tggcagacat gaactatgtt caccgtgacc tggctgcccg caacatcctc   2280
   gtcaacagca acctggtctg caaggtgtcg gactttgggc tctcacgctt tctagaggac   2340
   gatacctcag accccaccta caccagtgcc ctgggcggaa agatccccat ccgctggaca   2400
   gccccggaag ccatccagta ccggaagttc acctcggcca gtgatgtgtg gagctacggc   2460
   attgtcatgt gggaggtgat gtcctatggg gagcggccct actgggacat gaccaaccag   2520
   gatgtaatca atgccattga gcaggactat cggctgccac cgcccatgga ctgcccgagc   2580
   gccctgcacc aactcatgct ggactgttgg cagaaggacc gcaaccaccg gcccaagttc   2640
   ggccaaattg tcaacacgct agacaagatg atccgcaatc ccaacagcct caaagccatg   2700
   gcgcccctct cctctggcat caacctgccg ctgctggacc gcacgatccc cgactacacc   2760
   agctttaaca cggtggacga gtggctggag gccatcaaga tggggcagta caaggagagc   2820
   ttcgccaatg ccggcttcac ctcctttgac gtcgtgtctc agatgatgat ggaggacatt   2880
   ctccgggttg ggtcactttt ggctggccac cagaaaaaaa tcctgaacag tatccaggtg   2940
   atgcgggcgc agatgaacca gattcagtct gtggaggttt gacattcacc tgcctcggct   3000
   cacctcttcc tccaagcccc gccccctctg ccccacgtgc cggccctcct ggtgctctat   3060
   ccactgcagg gccagccact cgccaggagg ccacgggcca cgggaagaac caagcggtgc   3120
   cagccacgag acgtcaccaa gaaaacatgc aactcaaacg acggaaaaaa aaagggaatg   3180
   ggaaaaaaga aaacagatcc tgggaggggg cgggaaatac aaggaatatt ttttaaagag   3240
   gattctcata aggaaagcaa tgactgttct tgcgggggat aaaaaagggc ttgggagatt   3300
   catgcgatgt gtccaatcgg agacaaaagc agtttctctc caactccctc tgggaaggtg   3360
   acctggccag agccaagaaa cactttcaga aaaacaaatg tgaaggggag agacaggggc   3420
   cgcccttggc tcctgtccct gctgctcctc taggcctcac tcaacaacca agcgcctgga   3480
   ggacgggaca gatggacaga cagccaccct gagaacccct ctgggaaaat ctattcctgc   3540
   caccactggg caaacagaag aatttttctg tctttggaga gtattttaga aactccaatg   3600
   aaagacactg tttctcctgt tggctcacag ggctgaaagg ggcttttgtc ctcctgggtc   3660
   agggagaacg cggggacccc agaaaggtca gccttcctga ggatgggcaa cccccaggtc   3720
   tgcagctcca ggtacatatc acgcgcacag cctggcagcc tggccctcct ggtgcccact   3780
   cccgccagcc cctgcctcga ggactgatac tgcagtgact gccgtcagct ccgactgccg   3840
   ctgagaaggg ttgatcctgc atctgggttt gtttacagca attcctggac tcgggggtat   3900
   tttggtcaca gggtggtttt ggtttagggg gtttgtttgt tgggttgttt tttgtttttt   3960
   ggtttttttt aatgacaatg aagtgacact ttgacatttc ctaccttttg aggacttgat   4020
   ccttctccag gaagaaggtg ctttctgctt actgacttag gcaatacacc aagggcgaga   4080
   ttttatatgc acatttctgg atttttttat acggttttca ttgacactct tccctcctcc   4140
   cacctgccac caggcctcac caaagcccac tgccatgggg ccatctgggc cattcagaga   4200
   ctggagtgag atttgggtgt ggagggggag gcgccaaggt ggaggagctt cccactccag   4260
   gactgttgat gaaagggaca gattgaggag gaagtgggct ctgaggctgc agggctggaa   4320
   gtccttgccc acttcccact ctcctgcccc aatctatcta gtacttccca ggcaaatagg   4380
   cccctttgag gctcctgagt gccctcagat ggtcaaaacc cagttttccc tctgggagcc   4440
   taaaccaggc tgcatcggag gccaggaccc ggatcattca ctgtgatacc ctgccctcca   4500
   gagggtgcgc tcagagacac gggcaagcat gcctcttccc ttccctggag agaaagtgtg   4560
   tgatttctct cccacctcct tccccccacc agacctttgc tgggcctaaa ggtcttggcc   4620
   atggggacgc cctcagtcta gggatctggc cacagactcc ctcctgtgaa ccaacacaga   4680
   cacccaagca gagcaatcag ttagtgaatt g                                  4711

<210> 27
   <211> 1853
   <212> DNA
   <213> homo sapiens
<400> 27
   ggcacgaggg tccctgggcc ggacggcggt gtcccggcgt ggcgggaagc cggcactgga     60
   gcgggagcgc actgggcgcg ggaccgggag gcgcagggac cggacggctc ccgagtcgcc    120
   cacctgacgc tagaagaagt cttcacttcc caggagagcc aaagcgtgtc tggccctagg    180
   tgggaaaaga actggctgtg acctttgccc tgacctggaa gggcccagcc ttgggctgaa    240
   tggcagcacc cacgcccgcc cgtccggtgc tgacccacct gctggtggct ctcttcggca    300
   tgggctcctg ggctgcggtc aatgggatct gggtggagct acctgtggtg gtcaaagagc    360
   ttccagaggg ttggagcctc ccctcttacg tctctgtgct tgtggctctg gggaacctgg     420
   gtctgctggt ggtgaccctc tggaggaggc tggccccagg aaaggacgag caggtcccca    480
   tccgggtggt gcaggtgctg ggcatggtgg gcacagccct gctggcctct ctgtggcacc    540
   atgtggcccc agtggcagga cagttgcatt ctgtggcctt cttagcactg gcctttgtgc    600
   tggcactggc atgctgtgcc tcgaatgtca ctttcctgcc cttcttgagc cacctgccac    660
   ctcgcttctt acggtcattc ttcctgggtc aaggcctgag tgccctgctg ccctgcgtgc    720
   tggccctagt gcagggtgtg ggccgcctcg agtgcccgcc agcccccatc aacggcaccc    780
   ctggcccccc gctcgacttc cttgagcgtt ttcccgccag caccttcttc tgggcactga    840
   ctgcccttct ggtcgcttca gctgctgcct tccagggtct tctgctgctg ttgccgccac    900
   caccatctgt acccacaggg gagttaggat caggcctcca ggtgggagcc ccaggagcag    960
   aggaagaggt ggaagagtcc tcaccactgc aagagccacc aagccaggca gcaggcacca   1020
   cccctggtcc agaccctaag gcctatcagc ttctatcagc ccgcagtgcc tgcctgctgg   1080
   gcctgttggc cgccaccaac gcgctgacca atggcgtgct gcctgccgtg cagagctttt   1140
   cctgcttacc ctacgggcgt ctggcctacc acctggctgt ggtgctgggc agtgctgcca   1200
   atcccctggc ctgcttcctg gccatgggtg tgctgtgcag gtccttggca gggctgggcg   1260
   gcctctctct gctgggcgtg ttctgtgggg gctacctgat ggcgctggca gtcctgagcc   1320
   cctgcccgcc cctggtgggc acctcggcgg gggtggtcct cgtggtgctg tcgtgggtgc   1380
   tgtgtcttgg cgtgttctcc tacgtgaagg tggcagccag ctccctgctg catggcgggg   1440
   gccggccggc attgctggca gccggcgtgg ccatccaggt gggctctctg ctcggcgctg   1500
   ttgctatgtt ccccccgacc agcatctatc acgtgttcca cagcagaaag gactgtgcag   1560
   acccctgtga ctcctgagcc tgggcaggtg gggaccccgc tccccaacac ctgtctttcc   1620
   ctcaatgctg ccaccatgcc tgagtgcctg cagcccagga ggcccgcaca ccggtacact   1680
   cgtggacacc tacacactcc ataggagatc ctggctttcc agggtgggca agggcaagga   1740
   gcaggcttgg agccagggac cagtgggggc tgtagggtaa gcccctgagc ctgggaccta   1800
   catgtggttt gcgtaataaa acatttgtat ttaaaaaaaa aaaaaaaaaa aaa          1853
<210> 28
   <211> 2564
   <212> DNA
   <213> homo sapiens
<400> 28
   actgttccgc gggcaccggc agcgcagcgt ctccgatagt aagtcgggct gccggccggc     60
   tcattccccc agggtaactc tgagcccccg gctccgagct ccctcgaggc cgcctaccgg    120
   cgtcgggaac atggatgaga aatccaacaa gctgctgcta gctttggtga tgctcttcct    180
   atttgccgtg atcgtcctcc aatacgtgtg ccccggcaca gaatgccagc tcctccgcct    240
   gcaggcgttc agctccccgg tgccggaccc gtaccgctcg gaggatgaga gctccgccag    300
   gttcgtgccc cgctacaatt tcacccgcgg cgacctcctg cgcaaggtag acttcgacat    360
   caagggcgat gacctgatcg tgttcctgca catccagaag accgggggca ccactttcgg    420
   ccgccacttg gtgcgtaaca tccagctgga gcagccgtgc gagtgccgcg tgggtcagaa    480
   gaaatgcact tgccaccggc cgggtaagcg ggaaacctgg ctcttctcca ggttctccac    540
   gggctggagc tgcgggttgc acgccgactg gaccgagctc accagctgtg tgccctccgt    600
   ggtggacggc aagcgcgacg ccaggctgag accgtccagg aacttccact acatcaccat    660
   cctccgagac ccagtgtccc ggtacttgag tgagtggagg catgtccaga gaggggcaac    720
   atggaaagca tccctgcatg tctgcgatgg aaggcctcca acctccgaag agctgcccag    780
   ctgctacact ggcgatgact ggtctggctg ccccctcaaa gagtttatgg actgtcccta    840
   caatctagcc aacaaccgcc aggtgcgcat gctctccgac ctgaccctgg taggctgcta    900
   caacctctct gtcatgcctg aaaagcaaag aaacaaggtc cttctggaaa gtgccaagtc    960
   aaatctgaag cacatggcgt tcttcggcct cactgagttt cagcggaaga cccaatatct   1020
   gtttgagaaa accttcaaca tgaactttat ttcgccattt acccagtata ataccactag   1080
   ggcctctagt gtagagatca atgaggaaat tcaaaagcgt attgagggac tgaattttct   1140
   ggatatggag ttgtacagct atgccaaaga cctttttttg cagaggtacc agtttatgag   1200
   gcagaaagag catcaggagg ccaggcgaaa gcgtcaggaa caacgcaaat ttctgaaggg   1260
   aaggctcctt cagacccatt tccagagcca gggtcagggc cagagccaga atccgaatca   1320
   gaatcagagt cagaacccaa atccgaatgc caatcagaac ctgactcaga atctgatgca   1380
   gaatctgact cagagtttga gccagaagga gaaccgggaa agcccgaagc agaactcagg   1440
   caaggagcag aatgataaca ccagcaatgg caccaacgac tacataggca gtgtagagaa   1500
   atggcgttaa atggctcaaa aaggcctgta catacttctc ccaaagcgcc actgaaaaga   1560
   tggcatagct taaaagatga aagtgtccaa acacatcctg cttccttcat tggggaagtt   1620
   ttaaaaaaaa gtttagatgt tgcctttaca gttgcctttc aattcagtgt tatactgtgt   1680
   gtaggtaaaa caaatctcaa tatggaatta aattgtcttt ttggggttgg actaaatatg   1740
   aaatccgaaa gccaaaccag actcaccaga aattgctgtt tagatatttt aagaagttct   1800
   taaattagtt atggagacaa agtgaaaaca taaaatgtga ccatttaact tatggctaag   1860
   aaatggactt taaattattc atgatacact gttaaaaccc aatcttggaa tcaaatattt   1920
   tttccagggg tgagaataag tataaacata aagcaactaa aatgaaacat aaaacctttt   1980
   attttcttct gattttaaca aggaatctat ttaaatagaa taacaactga tggtgaatct   2040
   taccgagctg tagaaaataa aaaattcctc tccaaacatg ggtagtttta tgtcaaaata   2100
   ttggcttttc aagaacagga ctcatatctt gatatttaag agatgtttaa aattttaaac   2160
   tttttctacc ttctactgtt taaaggtttt acacagggtg tatctcacat taaacaaaac   2220
   accttttttt caattttctt tagttttaat tgaaaatgtt tgcttttaaa actgataggt   2280
   attgttggaa agcaggatga agcctgagcc agtggaaaag cttgttacag aaaaaacatt   2340
   ttgtgttatt gctgtggtgt gcatgatttg caaagattaa gtgcattttc tctgtctata   2400
   ctgattattg tatatagagg atgttataaa tatacatata catttttgcc attatgtaaa   2460
   tcccatgatt tcaactgtaa acatctgtcc attggtgtag ctttacaaac cattcactga   2520
   ttttgtgtaa tttaacaata gatatgaaat aaagtttaaa ttac                     2564
<210> 29
   <211> 2733
   <212> DNA
   <213> homo sapiens
<400> 29
   gctttctaag gcggtcgctc cgggaaatcc gggccctagg attgtccact catcccagta     60
   tcagcgagat acggggagat agagttagcg acaacgtgag ccagagctgg agcacgtttg    120
   gtgagagacc agaaagcaat ggaggccgga gaggggaagg agcgcgttcc gaaacaaagg    180
   caagtcctga tattctttgt tttgctgggc atagctcagg ctagttgcca gcctaggcac    240
   tattcagtgg ccgaggaaac ggagagtggc tcctttgtgg ccaatttgtt aaaagacctg    300
   gggctggaga taggagaact tgctgtgagg ggggccaggg tcgtttccaa aggaaaaaaa    360
   atgcatttgc agttcgatag gcagaccggg gatttgttgt taaatgagaa attggaccgg    420
   gaggagctgt gcggccccac agagccctgt gtcctacctt tccaggtgtt actagaaaat    480
   cccttgcagt tttttcaggc ggagctacgg attagggacg taaatgatca ttccccagtt    540
   ttcctagaca aagaaatact tttgaaaatt ccagaaagta tcactcctgg aactactttc    600
   ttaatagaac gtgcccagga cttggatgta ggaaccaaca gtctccaaaa ttacacaatc    660
   agtcccaatt tccactttca tcttaattta caagacagtc tcgatggcat aatattacca    720
   cagctggtgc tgaacagagc cctggatcgc gaggagcagc ctgagatcag gttaaccctc    780
   acagcgctag atggcgggag tccacccagg tccggcacgg ccctggtacg gattgaagtt    840
   gtggacatca atgacaacgt cccagagttt gcaaagctgc tctatgaggt gcagatcccg    900
   gaggacagcc ccgttggatc ccaggttgcc atcgtctctg ccagggattt agacattgga    960
   actaatggag aaatatctta tgcattttcc caagcatctg aagacattcg caaaacgttt   1020
   cgattaagtg caaaatcggg agaactgctt ttaagacaga aactggattt cgaatccatc   1080
   cagacataca cagtaaatat tcaggcgaca gatggtgggg gcctatctgg aacttgtgtg   1140
   gtatttgtcc aagtgatgga tttgaatgac aatcctccgg aactaactat gtcgacactt   1200
   atcaatcaga tcccagaaaa cttgcaggac accctcattg ctgtattcag cgtttcagat   1260
   cctgactccg gagacaacgg aaggatggtg tgctccatcc aagatgatct tccttttttc    1320
   ttgaaacctt ctgttgagaa cttttacact ctggtgataa gcacggccct ggaccgggag   1380
   accagatccg aatacaacat caccatcacc gtcaccgact tcgggacacc caggctgaaa   1440
   accgagcaca acataaccgt gctggtctcc gacgtcaatg acaacgcccc cgccttcacc   1500
   caaacctcct acaccctgtt cgtccgcgag aacaacagcc ccgccctgca catcggcagc   1560
   gtcagcgcca cagacagaga ctcgggcacc aacgcccagg tcacctactc gctgctgccg   1620
   ccccaggacc cgcacctgcc cctcgcctcc ctggtctcca tcaacgcgga caacggccac   1680
   ctgttcgctc tccagtcgct ggactacgag gccctgcagg cgttcgagtt ccgcgtgggc   1740
   gccgcagacc gcggctcccc ggcgttgagc agcgaggcgc tggtgcgcgt gctggtgctg   1800
   gacgccaacg acaactcgcc cttcgtgctg tacccgctgc agaacggctc cgcgccctgc   1860
   accgagctgg tgccccgggc ggccgagccg ggctacctgg tgaccaaggt ggtggcggtg   1920
   gacggcgact cgggccagaa cgcctggctg tcgtaccagc tgctcaaggc cacggagccc   1980
   gggctgttcg gcgtgtgggc gcacaatggc gaggtgcgca ccgccaggct gctgagggag   2040
   cgcgacgctg ccaagcagag gctggtggtg ctggtcaagg acaatggcga gcctccgcgc   2100
   tcggccaccg ccacgctgca cgtgctcctg gtggacggct tctcccagcc ctacctgctg   2160
   ctcccggagg cggcaccggc ccaggcccag gccgacttgc tcaccgtcta cctggtggtg   2220
   gcgttggcct cggtgtcttc gctcttcctc ttctcggtgc tcctgttcgt ggcggtgcgg   2280
   ctgtgcagga ggagcagggc ggcctcggtg ggtcgctgct cggtgcccga gggccccttt   2340
   ccagggcaga tggtggacgt gagcggcacc gggaccctgt cccagagcta ccagtacgag   2400
   gtgtgtctga ctggaggctc cgggacaaat gagttcaagt tcctgaagcc aattatcccc   2460
   aacttcgttg ctcagggtgc agagagggtt agcgaggcaa atcccagttt caggaagagc   2520
   tttgaattca cttaagtgtt aataaggatc tactgaggct agtctcgttt aatttgtgga   2580
   aagtcctttt ttactgcttt gcccattgga ggtgtctcct tttattagaa agtaaccatc   2640
   ttattccaat tctatgcatg ttactggtat ttataaatgt atgagttttt ttgcggtata   2700
   ataaatgtaa attttctttg tattctaaaa aaa                                2733
<210> 30
   <211> 1007
   <212> DNA
   <213> homo sapiens
<400> 30
   cggaagcgcg acgctggagc tgcggggtta ccatgggaac cgaaccgccg cgcctcgccc     60
   aggacagtta cacttagagg ccttcatcat catgatgtta agctgcctct ttcttctgaa    120
   ggcacttctt gctcttgggt ctctggaatc ctggataact gcaggagaac atgcaaaaga    180
   gggagaatgc cctccccata agaacccatg caaagagctg tgccagggtg atgaattgtg    240
   tccggctgaa cagaagtgct gcaccacagg ctgtggtcgg atctgccgag acattcctaa    300
   ggggaggaaa agagattgcc ctagggttat tcggaaacaa tcctgtttga aaaggtgcat    360
   cactgatgag acatgtccag gtgtaaagaa atgctgcacg cttggctgca acaagagctg    420
   tgtagtccca atctctaaac agaagctggc agagtttggt ggtgaatgtc ccgctgaccc    480
   ccttccgtgt gaggagctgt gtgatgggga tgcatcctgt ccccaggggc ataaatgctg    540
   cagcaccggc tgtggccgca cctgcctcgg agacattgag ggagggcggg gcggtgattg    600
   tccaaaagtt ctggtgggcc tgtgcattgt tggctgtgtg atggatgaga attgtcaagc    660
   tggagaaaaa tgttgcaagt caggctgtgg ccgcttctgt gtcccaccag tcctgccccc    720
   aaaactgacc atgaacccca actggactgt gaggtctgat tccgaattag agatcccggt    780
   gccctagctg tgctgatttg tctggagctt ctttggtaat tctggaagct tttcctggca    840
   gtcaagagag ggtgacatcc tggggcttgt gacatttcca ggggcactca tggccctctc    900
   tgctctgctt ctcctcctgc cgctgaccag agcatgggaa atagccctgg attgggtagt    960
   gggtgtgtgg tgcttctctt tcccgataaa ggctggtgct gacctct                 1007
<210> 31
   <211> 4720
   <212> DNA
   <213> homo sapiens
<400> 31
<210> 32
   <211> 4266
   <212> DNA
   <213> homo sapiens
<400> 32
   agagtttcag ttttggcagc agcgtccagt gccctgccag tagctcctag agaggcaggg     60
   gttaccaact ggccagcagg ctgtgtccct gaagtcagat caacgggaga gaaggaagtg    120
   gctaaaacat tgcacaggag aagtcggcct gagtggtgcg gcgctcggga cccaccagca    180
   atgctgctct tcgtgctcac ctgcctgctg gcggtcttcc cagccatctc cacgaagagt    240
   cccatatttg gtcccgagga ggtgaatagt gtggaaggta actcagtgtc catcacgtgc    300
   tactacccac ccacctctgt caaccggcac acccggaagt actggtgccg gcagggagct    360
   agaggtggct gcataaccct catctcctcg gagggctacg tctccagcaa atatgcaggc    420
   agggctaacc tcaccaactt cccggagaac ggcacatttg tggtgaacat tgcccagctg    480
   agccaggatg actccgggcg ctacaagtgt ggcctgggca tcaatagccg aggcctgtcc    540
   tttgatgtca gcctggaggt cagccagggt cctgggctcc taaatgacac taaagtctac    600
   acagtggacc tgggcagaac ggtgaccatc aactgccctt tcaagactga gaatgctcaa    660
   aagaggaagt ccttgtacaa gcagataggc ctgtaccctg tgctggtcat cgactccagt    720
   ggttatgtaa atcccaacta tacaggaaga atacgccttg atattcaggg tactggccag    780
   ttactgttca gcgttgtcat caaccaactc aggctcagcg atgctgggca gtatctctgc    840
   caggctgggg atgattccaa tagtaataag aagaatgctg acctccaagt gctaaagccc    900
   gagcccgagc tggtttatga agacctgagg ggctcagtga ccttccactg tgccctgggc    960
   cctgaggtgg caaacgtggc caaatttctg tgccgacaga gcagtgggga aaactgtgac   1020
   gtggtcgtca acaccctggg gaagagggcc ccagcctttg agggcaggat cctgctcaac 1080
   ccccaggaca aggatggctc attcagtgtg gtgatcacag gcctgaggaa ggaggatgca 1140
   gggcgctacc tgtgtggagc ccattcggat ggtcagctgc aggaaggctc gcctatccag 1200
   gcctggcaac tcttcgtcaa tgaggagtcc acgattcccc gcagccccac tgtggtgaag 1260
   ggggtggcag gaggctctgt ggccgtgctc tgcccctaca accgtaagga aagcaaaagc 1320
   atcaagtact ggtgtctctg ggaaggggcc cagaatggcc gctgccccct gctggtggac 1380
   agcgaggggt gggttaaggc ccagtacgag ggccgcctct ccctgctgga ggagccaggc 1440
   aacggcacct tcactgtcat cctcaaccag ctcaccagcc gggacgccgg cttctactgg 1500
   tgtctgacca acggcgatac tctctggagg accaccgtgg agatcaagat tatcgaagga 1560
   gaaccaaacc tcaaggtacc agggaatgtc acggctgtgc tgggagagac tctcaaggtc 1620
   ccctgtcact ttccatgcaa attctcctcg tacgagaaat actggtgcaa gtggaataac 1680
   acgggctgcc aggccctgcc cagccaagac gaaggcccca gcaaggcctt cgtgaactgt 1740
   gacgagaaca gccggcttgt ctccctgacc ctgaacctgg tgaccagggc tgatgagggc 1800
   tggtactggt gtggagtgaa gcagggccac ttctatggag agactgcagc cgtctatgtg 1860
   gcagttgaag agaggaaggc agcggggtcc cgcgatgtca gcctagcgaa ggcagacgct 1920
   gctcctgatg agaaggtgct agactctggt tttcgggaga ttgagaacaa agccattcag 1980
   gatcccaggc tttttgcaga ggaaaaggcg gtggcagata caagagatca agccgatggg 2040
   agcagagcat ctgtggattc cggcagctct gaggaacaag gtggaagctc cagagcgctg 2100
   gtctccaccc tggtgcccct gggcctggtg ctggcagtgg gagccgtggc tgtgggggtg 2160
   gccagagccc ggcacaggaa gaacgtcgac cgagtttcaa tcagaagcta caggacagac 2220
   attagcatgt cagacttcga gaactccagg gaatttggag ccaatgacaa catgggagcc 2280
   tcttcgatca ctcaggagac atccctcgga ggaaaagaag agtttgttgc caccactgag 2340
   agcaccacag agaccaaaga acccaagaag gcaaaaaggt catccaagga ggaagccgag 2400
   atggcctaca aagacttcct gctccagtcc agcaccgtgg ccgccgaggc ccaggacggc 2460
   ccccaggaag cctagacggt gtcgccgcct gctccctgca cccatgacaa tcaccttcag 2520
   aatcatgtcg atcctggggc cctcagctcc tggggacccc actccctgct ctaacacctg 2580
   cctaggtttt tcctactgtc ctcagaggcg tgctggtccc ctcctcagtg acatcaaagc 2640
   ctggcctaat tgttcctatt ggggatgagg gtggcatgag gaggtcccac ttgcaacttc 2700
   tttctgttga gagaacctca ggtacggaga agaatagagg tcctcatggg tcccttgaag 2760
   gaagagggac cagggtggga gagctgattg cagaaaggag agacgtgcag cgcccctctg 2820
   cacccttatc atgggatgtc aacagaattt ttccctccac tccatccctc cctcccgtcc 2880
   ttcccctctt cttctttcct tccatcaaaa gatgtatttg aattcatact agaattcagg 2940
   tgctttgcta gatgctgtga caggtatgcc accaacactg ctcacagcct ttctgaggac 3000
   accagtgaaa gaagccacag ctcttcttgg cgtatttata ctcactgagt cttaactttt 3060
   caccaggggt gctcacctct gcccctattg ggagaggtca taaaatgtct cgagtcctaa 3120
   ggccttaggg gtcatgtatg atgagcatac acacaggtaa ttataaaccc acattcttac 3180
   catttcacac ataagaaaat tgaggtttgg aagagtgaag cgtttttctt tttctttttt 3240
   ttttttgaga cggagtctct cactgtcgcc caggctggag tgcagtggcg caatctcggc 3300
   tcactgcaac ctccgcctcc caggttgaca ccattctcct gcctcaccct cccaagtagc 3360
   tgggactaca ggcgcctgcc agcacgcctg gctaattttt tgtattttta gtagagacag 3420
   ggtttcaccg tgttagccag gatggtctcg atctcctgac ctcgtgatcc gcctgcctct 3480
   gcctcccaaa gtgctgggat tacaggcgtg agccaccgcg tccggcctct ttttttcttt 3540
   tctttttttt gagacaaagt ctcactgtgt cacccagact ggaatgcagt gacacaatct 3600
   cggctcactg aaacctctgc cttccaggtt caagctattc tcatgcctca gcctctcaag 3660
   tagctgggac tacagatgtg ggccaccatg tctggctaat tttttttttt tttttttttt 3720
   tttgtagaga cagggtttcg ccatgttgac gagactggtc tcgaactcct ggcctcaagt 3780
   gatctgccgc ctcagcttct caaagtactg ggattatata ggcatgagcc actgagcctg 3840
   gccctgaagc gtttttctca aaggccctca gtgagataaa ttagatttgg catctcctgt 3900
   cctgggccag ggatctctct acaagagccc ctgcccctct gttggaggca cagttttaga 3960
   ataaggagga ggagggagaa gagaaaatgt aaaggaggga gatctttccc aggccgcacc 4020
   atttctgtca ctcacatgga cccaagataa aagaatggcc aaaccctcac aacccctgat 4080
   gtttgaagag ttccaagttg aagggaaaca aagaagtgtt tgatggtgcc agagaggggc 4140
   tgctctccag aaagctaaaa tttaatttct tttttcctct gagttctgta cttcaaccag 4200
   cctacaagct ggcacttgct aacaaatcag aaatatgaca attaatgatt aaagactgtg 4260
   attgcc 4266
<210> 33
   <211> 2618
   <212> DNA
   <213> homo sapiens
<400> 33
   atgaagcacc tgaagcggtg gtggtcggcc ggcggcggcc tcctgcacct caccctcctg      60
   ctgagcttgg cggggctccg cgtagaccta gatctttacc tgctgctgcc gccgcccacc     120
   ctgctgcagg acgagctgct gttcctgggc ggcccggcca gctccgccta cgcgctcagc     180
   cccttctcgg cctcgggagg gtgggggcgc gcgggccact tgcaccccaa gggccgggag     240
   ctggaccctg ccgcgccgcc cgagggccag ctgctccggg aggtgcgcgc gctcggggtc     300
   cccttcgtcc ctcgcaccag cgtggatgca tggctggtgc acagcgtggc tgccgggagc     360
   gcggacgagg cccacgggct gctcggcgcc gccgccgcct cgtccaccgg aggagccggc     420
   gccagcgtgg acggcggcag ccaggctgtg caggggggcg gcggggaccc ccgagcggct     480
   cggagtggcc ccttggacgc cggggaagag gagaaggcac ccgcggaacc gacggctcag     540
   gtgccggacg ctggcggatg tgcgagcgag gagaatgggg tactaagaga aaagcacgaa     600
   gctgtggatc atagttccca gcatgaggaa aatgaagaaa gggtgtcagc ccagaaggag     660
   aactcacttc agcagaatga tgatgatgaa aacaaaatag cagagaaacc tgactgggag     720
   gcagaaaaga ccactgaatc tagaaatgag agacatctga atgggacaga tacttctttc     780
   tctctggaag acttattcca gttgctttca tcacagcctg aaaattcact ggagggcatc     840
   tcattgggag atattcctct tccaggcagt atcagtgatg gcatgaattc ttcagcacat     900
   tatcatgtaa acttcagcca ggctataagt caggatgtga atcttcatga ggccatcttg     960
   ctttgtccca acaatacatt tagaagagat ccaacagcaa ggacttcaca gtcacaagaa    1020
   ccatttctgc agttaaattc tcataccacc aatcctgagc aaacccttcc tggaactaat    1080
   ttgacaggat ttctttcacc ggttgacaat catatgagga atctaacaag ccaagaccta    1140
   ctgtatgacc ttgacataaa tatatttgat gagataaact taatgtcatt ggccacagaa    1200
   gacaactttg atccaatcga tgtttctcag ctttttgatg aaccagattc tgattctggc    1260
   ctttctttag attcaagtca caataatacc tctgtcatca agtctaattc ctctcactct    1320
   gtgtgtgatg aaggtgctat aggttattgc actgaccatg aatctagttc ccatcatgac    1380
   ttagaaggtg ctgtaggtgg ctactaccca gaacccagta agctttgtca cttggatcaa    1440
   agtgattctg atttccatgg agatcttaca tttcaacacg tatttcataa ccacacttac    1500
   cacttacagc caactgcacc agaatctact tctgaacctt ttccgtggcc tgggaagtca    1560
   cagaagataa ggagtagata ccttgaagac acagatagaa acttgagccg tgatgaacag    1620
   cgtgctaaag ctttgcatat ccctttttct gtagatgaaa ttgtcggcat gcctgttgat    1680
   tctttcaata gcatgttaag tagatattat ctgacagacc tacaagtctc acttatccgt    1740
   gacatcagac gaagagggaa aaataaagtt gctgcgcaga actgtcgtaa acgcaaattg    1800
   gacataattt tgaatttaga agatgatgta tgtaacttgc aagcaaagaa ggaaactctt    1860
   aagagagagc aagcacaatg taacaaagct attaacataa tgaaacagaa actgcatgac    1920
   ctttatcatg atatttttag tagattaaga gatgaccaag gtaggccagt caatcccaac    1980
   cactatgctc tccagtgtac ccatgatgga agtatcttga tagtacccaa agaactggtg    2040
   gcctcaggcc acaaaaagga aacccaaaag ggaaagagaa agtgagaaga aactgaagat    2100
   ggactctatt atgtgaagta gtaatgttca gaaactgatt atttggatca gaaaccattg    2160
   aaactgcttc aagaattgta tctttaagta ctgctacttg aataactcag ttaacgctgt    2220
   tttgaagctt acatggacaa atgtttagga cttcaagatc acacttgtgg gcaatctggg    2280
   ggagccacaa cttttcatga agtgcattgt atacaaaatt catagttatg tccaaagaat    2340
   aggttaacat gaaaacccag taagactttc catcttggca gccatccttt ttaagagtaa    2400
   gttggttact tcaaaaagag caaacactgg ggatcaaatt attttaagag gtatttcagt    2460
   tttaaatgca aaatagcctt attttcattt agtttgttag cactatagtg agcttttcaa    2520
   acactatttt aatctttata tttaacttat aaattttgct ttctatggaa ataaattttg    2580
   tatttgtatt aaaaattaac ttttcccttt tatacaga                            2618
<210> 34
   <211> 799
   <212> DNA
   <213> homo sapiens
<400> 34
   gtgcaatggc tagtactatg tgtcaacttg tctaggctat actgctcagc tgtgtggtca      60
   aacagtagtc tagatgttgc tgtgaaggta ttttgtagat gtgatcaaca tttacaatca     120
   gttgatttta agtaaagcag tttaacttcc aatatgtgga tgggcctcat ccaattagtt     180
   gaaggtgtta agagaaaaga ccaaggtttc ctggaaaagg aattctacca caagactaac     240
   ataaaaatgc actgtgagtt tcatgcctgc tggcctgcct tcactgtcct gggggaggct     300
   tggagagacc aggtggactg gagtatactg ttgagagacg ctggtctggt gaagatgtcc     360
   aggaaaccac gagcctccag cccattgtcc aacaaccacc caccaacacc aaagaggttc     420
   ccaagacaac tcggaaggga aaagggaccc atcgaggaag ttccaggaac aaaaggctct     480
   ccataaaaga ccgccgcttc aaaaaaacct gaggaatgga gtgggccaac actatccagc     540
   cactctgacc agccgaacga ggaactcaat caaaatgagc catagcggga ccacaagggc     600
   aaggagacca ccactttctc cagtctcttt tcggacagcc agtaattccc gggcaaggcc     660
   agagacttca agtctatctg aaaagtctcc agaggtctaa ccccagataa atagccaaca     720
   gggtgtagag tacgttttac accccaaagg gtatgcccca tgtgagggaa ataaaatgaa     780
   catgttgtaa aaaaaaaaa                                                  799
<210> 35
   <211> 2050
   <212> DNA
   <213> homo sapiens
<400> 35
   tctagaggat ccgccaagag gtagcaagaa acagtatcca cagtggactc cggggctcct      60
   acagacttgg cacagcttcc tacagtcttg aaacagccct gttgttctgt catggccagt     120
   gggcagtttg tgaacaaact gcaagaggaa gtgatctgcc ccatctgcct ggacattctg     180
   cagaaacctg tcaccatcga ctgtgggcac aatttctgcc tcaaatgcat cactcagatt     240
   ggggaaacat catgtggatt tttcaaatgt cccctctgca aaacttccgt aagaagagac     300
   gcaatcaggt tcaactcgct gttgcggaat ctggtggaga aaatccaagc tctacaagcc     360
   tctgaggtgc agtccaaaag gaaagaggct acatgcccga ggcaccagga gatgttccac     420
   tatttctgcg aggatgatgg gaagttcctc tgttttgtgt gtcgtgaatc caaggaccac     480
   aaatcccata atgtcagctt gatcgaagaa gctgcccaga attatcaggg gcagattcaa     540
   gagcagatcc aagtcttgca gcaaaaggag aaggagacag tacaagtgaa ggcacaaggt     600
   gtacacaggg tcgatgtctt cacggaccag gtagaacatg agaagcaaag gatcctcaca     660
   gaatttgaac tcctgcatca agtcctagag gaggagaaga atttcctgct atcacggatt     720
   tactggctgg gtcatgaggg aacggaagcg gggaaacact atgttgcctc cactgagcca     780
   cagttgaacg atctcaagaa gctcgttgat tccctgaaga ccaagcagaa catgccaccc     840
   aggcagctgc tggaggatat caaagtcgtc ttgtgcagaa gtgaagagtt tcagtttctc     900
   aacccaaccc ctgttcctct ggaactggag aaaaaactca gtgaagcaaa atcaagacac     960
   gactccatca cagggagcct gaaaaaattc aaagaccaac tccaggctga taggaaaaaa    1020
   gatgaaaaca gattcttcaa aagcatgaat aaaaatgaca tgaagagctg gggcttgtta     1080
   cagaaaaata atcataaaat gaacaaaacc tcagagcccg ggtcatcttc tgcaggcggc    1140
   agaactacat cggggccacc aaatcaccac tcttcagccc catcccactc cctgtttcgg    1200
   gcctcgtctg ctgggaaagt cacttttcca gtatgtctcc tggcctctta tgatgagatt    1260
   tctggtcaag gagcgagctc tcaggatacg aagacatttg acgttgcgct gtccgaggag    1320
   ctccatgcgg cactgagtga gtggctgaca gcgatccggg cttggttttg tgaggttcct    1380
   tcaagctaag ccagctcaga gaacacgggg agcggtggtg ctacacggac ttcggagcat    1440
   agagtggcgc tgagtgagtg gctgagaccg accacggttc ttgacttagt ggaattgggt    1500
   cgaaggagtg gagaatggga gggctcgggc tactgagagt ggagatgggg gcgggggtgg    1560
   tggtgaagag agttggagaa ggaatggacg aattcttgag caaaaggagg ggaagagaca    1620
   atctccagcc acccgcccca cgcttgactt cttatcactt tggctgtggt gccgcctagt    1680
   ggaaaaagga agtccctgca gcagtccccg cactctttaa gcagctgttt accgaaggca    1740
   ccagttcagc caggagtgaa atccggagag gagcaacgcc agcctgggtc acagtccatc    1800
   aaaccccatg agcccgacca ctctcgctct tccttacatt cccacgtccc ccttctctcc    1860
   caacccctca tatcagcaag ggaaattaat taatgagatt tgataaatca gtagatagaa    1920
   tgaggtcccc attctgaaat atttagcaga ctggaaccac cacgcaagcc tctgtagggg    1980
   gtggatggag acacttctaa ctttaataaa ctgcgactga acgtggaaaa aaaaaaaaaa    2040
   aaaaaaaaaa                                                           2050
<210> 36
   <211> 1537
   <212> DNA
   <213> homo sapiens
<400> 36
   agttctgtgg agcagcggtg gccggctagg atgggctctc tggggtctga ctctgcccct     60
   tttcttcttc tgctgggagg gtggggtctc taggagctct gcaggcccca gcacccgcag    120
   agcagacact gcgatgacaa cggacgacac agaagtgccc gctatgactc tagcaccggg    180
   ccacgccgct ctggaaactc aaacgctgag cgctgagacc tcttctaggg cctcaacccc    240
   agccggcccc attccagaag cagagaccag gggagccaag agaatttccc ctgcaagaga    300
   gaccaggagt ttcacaaaaa catctcccaa cttcatggtg ctgatcgcca cctccgtgga    360
   gacatcagcc gccagtggca gccccgaggg agctggaatg accacagttc agaccatcac    420
   aggcagtgat cccagggaag ccatctttga caccctttgc accgatgaca tctctgaaga    480
   ggcaaagaca ctcacaatgg acatattgac attggctcac acctccacag aagctaaggg    540
   cctgtcctca gagagcagcg cctcttccga cggcccccat ccagtcatca ccccgtcacg    600
   ggcctcagag agcagcgcct cttccgacgg cctccatcca gtcatcaccc cgtcacgggc    660
   ctcagagagc agcgcctctt ccgacggcct ccatccagtc atcaccccgt cacgggcctc    720
   agagagcagc gcctcttccg acggccccca tccagtcatc accccctcat ggtccccggg    780
   atctgacgtc actctcctcg ctgaagccct ggtgactgtc acaaacatcg aggttattaa    840
   ttgcagcatc acagaaatag aaacaacgac ttccagcatc cctggggcct cagacacaga    900
   tctcatcccc acggaagggg tgaaggcctc gtccacctcc gatccaccag ctctgcctga    960
   ctccactaac acaaaaccac acatcactga ggtcacagcc tctgccgaga ccctgtccac   1020
   agccggcacc acagagtcag ctgcacctga tgccacgatt gggaccccac tccccaccaa   1080
   cagcaccata gaaagagaag tgacagcacc cggggccacg accctcagtg gagctctggc   1140
   cacagggaat cccctggaag aaacctcagc cctctctgtt gagacaccaa gttacgtcaa   1200
   agtctcagga gcagctccgg tctccataga ggctgggtca gcagtgggca aaacaacttc   1260
   ctttgctggg agctctgctt cctcctacag ccccttggaa gccgccctca agaacttcac   1320
   cccttcagag acactgacca cggacatcgc aaccaagggg cccttcccca ccagcagggc   1380
   ccctcttcct tctgtccctc cgactacaac caacagcagc tgaaggacga acagcatctt   1440
   agccaagacc acaacctcag cgaagaccac gatgaagccc ccaacagcca cgcccaccac   1500
   tgctcggacg aggccgacca cagacatgag tgcaggt                            1537
<210> 37
   <211> 777
   <212> DNA
   <213> homo sapiens
<400> 37
   ggccccttgt ctgcagagat ggctcccaat gcttcctgcc tctgtgtgca tgtccgttcc     60
   gaggaatggg atttaatgac ctttgatgcc aacccatatg acagcgtgaa aaaaatcaaa    120
   gaacatgtcc ggtctaagac caaggttcct gtgcaggacc aggttctttt gctgggctcc    180
   aagatcttaa agccacggag aagcctctca tcttatggca ttgacaaaga gaagaccatc    240
   caccttaccc tgaaagtggt gaagcccagt gatgaggagc tgcccttgtt tcttgtggag    300
   tcaggtgatg aggcaaagag gcacctcctc caggtgcgaa ggtccagctc agtggcacaa    360
   gtgaaagcaa tgatcgagac taagacgggt ataatccctg agacccagat tgtgacttgc    420
   aatggaaaga gactggaaga tgggaagatg atggcagatt acggcatcag aaagggcaac    480
   ttactcttcc tggcatctta ttgtattgga gggtgaccac cctggggatg gggtgttggc    540
   aggggtcaaa aagcttattt cttttaatct cttactcaac gaacacatct tctgatgatt    600
   tcccaaaatt aatgagaatg agatgagtag agtaagattt gggtgggatg ggtaggatga    660
   agtatattgc ccaactctat gtttctttga ttctaacaca attaattaag tgacatgatt    720
   tttactaatg tattactgag actagtaaat aaatttttaa ggcaaaatag agcattc       777
<210> 38
   <211> 4231
   <212> DNA
   <213> homo sapiens
<400> 38
   ggaaaagagg gcacccagcc cttccccctc cctcatcctc ccatcccagt aaaccctgcc     60
   aaattggaat cctggactta atttaggaga aaggccctgt aaccaagata ctgactgaac    120
   atggctggcg gactcaggct ggggtctgca gtgcagcatt aatgggccgc tgacatgaat    180
   atggagtagt tttctctagc aaagagtggc ttccagcttc ttaaagtctg acaagaaccg    240
   gataggggga acctacaaga agaccatcta taaagaatac aaggatgact catacacaga    300
   tgaagtggcc cagcctgcct ggttgggctt cctggggcca gtgttgcagg ctgaagtggg    360
   ggatgtcatt cttattcacc tgaagaattt tgccactcgt ccctatacca tccaccctca    420
   tggtgtcttc tacgagaagg actctgaagg ttccctatac ccagatggct cctctgggcc    480
   actgaaagct gatgactctg ttcccccggg gggcagccat atctacaact ggaccattcc    540
   agaaggccat gcacccaccg atgctgaccc agcgtgcctc acctggatct accattctca    600
   tgtagatgct ccacgagaca ttgcaactgg cctaattggg cctctcatca cctgtaaaag    660
   aggagccctg gatgggaact cccctcctca acgccaggat gtagaccatg atttcttcct    720
   cctcttcagt gtggtagatg agaacctcag ctggcatctc aatgagaaca ttgccactta    780
   ctgctcagat cctgcttcag tggacaaaga agatgagaca tttcaggaga gcaataggat    840
   gcatgcaatc aatggctttg tttttgggaa tttacctgag ctgaacatgt gtgcacagaa    900
   acgtgtggcc tggcacttgt ttggcatggg caatgaaatt gatgtccaca cagcattttt    960
   ccatggacag atgctgacta cccgtggaca ccacactgat gtggctaaca tctttccagc   1020
   cacctttgtg actgctgaga tggtgccctg ggaacctggt acctggttaa ttagctgcca   1080
   agtgaacagt cactttcgag atggcatgca ggcactctac aaggtcaagt cttgctccat   1140
   ggcccctcct gtggacctgc tcacaggcaa agttcgacag tacttcattg aggcccatga   1200
   gattcaatgg gactatggcc cgatggggca tgatgggagt actgggaaga atttgagaga   1260
   gccaggcagt atctcagata agtttttcca gaagagctcc agccgaattg gggcacttta   1320
   ctggaaagtg cgatatgaag cctttcaaga tgagacattc caagagaaga tgcatttgga   1380
   ggaagatagg catcttggaa tcctggggcc agtgatccgg gctgaggtgg gtgacaccat   1440
   tcaggtggtc ttctacaacc gtgcctccca gccattcagc atgcagcccc atggggtctt   1500
   ttatgagaaa gactatgaag gcactgtgta caatgatggc tcatcttacc ctggcttggt   1560
   tgccaagccc tttgagaaag taacataccg ctggacagtc ccccctcatg ccggtcccac   1620
   tgctcaggat cctgcttgtc tcacttggat gtacttctct gctgcagatc ccataagaga   1680
   cacaaattct ggcctggtgg gcccgctgct ggtgtgcagg gctggtgcct tgggtgcaga   1740
   tggcaagcag aaaggggtgg ataaagaatt ctttcttctc ttcactgtgt tggatgagaa   1800
   caagagctgg tacagcaatg ccaatcaagc agctgctatg ttggatttcc gactgctttc   1860
   agaggatatt gagggcttcc aagactccaa tcggatgcat gccattaatg ggtttctgtt   1920
   ctctaacctg cccaggctgg acatgtgcaa gggtgacaca gtggcctggc acctgctcgg   1980
   cctgggcaca gagactgatg tgcatggagt catgttccag ggcaacactg tgcagcttca   2040
   gggcatgagg aagggtgcag ctatgctctt tcctcatacc tttgtcatgg ccatcatgca   2100
   gcctgacaac cttgggacat ttgagattta ttgccaggca ggcagccatc gagaagcagg   2160
   gatgagggca atctataatg tctcccagtg tcctggccac caagccaccc ctcgccaacg   2220
   ctaccaagct gcaagaatct actatatcat ggcagaagaa gtagagtggg actattgccc   2280
   tgaccggagc tgggaacggg aatggcacaa ccagtctgag aaggacagtt atggttacat   2340
   tttcctgagc aacaaggatg ggctcctggg ttccagatac aagaaagctg tattcaggga   2400
   atacactgat ggtacattca ggatccctcg gccaaggact ggaccagaag aacacttggg   2460
   aatcttgggt ccacttatca aaggtgaagt tggtgatatc ctgactgtgg tattcaagaa   2520
   taatgccagc cgcccctact ctgtgcatgc tcatggagtg ctagaatcta ctactgtctg   2580
   gccactggct gctgagcctg gtgaggtggt cacttatcag tggaacatcc cagagaggtc   2640
   tggccctggg cccaatgact ctgcttgtgt ttcctggatc tattattctg cagtggatcc   2700
   catcaaggac atgtatagtg gcctggtggg gcccttggct atctgccaaa agggcatcct   2760
   ggagccccat ggaggacgga gtgacatgga tcgggaattt gcattgttgt tcttgatttt   2820
   tgatgaaaat aagtcttggt atttggagga aaatgtggca acccatgggt cccaggatcc   2880
   aggcagtatt aacctacagg atgaaacttt cttggagagc aataaaatgc atgcaatcaa   2940
   tgggaaactc tatgccaacc ttaggggtct taccatgtac caaggagaac gagtggcctg   3000
   gtacatgctg gccatgggcc aagatgtgga tctacacacc atccactttc atgcagagag   3060
   cttcctctat cggaatggcg agaactaccg ggcagatgtg gtggatctgt tcccagggac   3120
   ttttgaggtt gtggagatgg tggccagcaa ccctgggaca tggctgatgc actgccatgt   3180
   gactgaccat gtccatgctg gcatggagac cctcttcact gttttttctc gaacagaaca    3240
   cttaagccct ctcaccgtca tcaccaaaga gactgaaaaa gcagtgcccc ccagagacat   3300
   tgaagaaggc aatgtgaaga tgctgggcat gcagatcccc ataaagaatg ttgagatgct   3360
   ggcctctgtt ttggttgcca ttagtgtcac ccttctgctc gttgttctgg ctcttggtgg   3420
   agtggtttgg taccaacatc gacagagaaa gctacgacgc aataggaggt ccatcctgga   3480
   tgacagcttc aagcttctgt ctttcaaaca gtaacatctg gagcctggag atatcctcag   3540
   gaagcacatc tgtagtgcac tcccagcagg ccatggacta gtcactaacc ccacactcaa   3600
   aggggcatgg gtggtggaga agcagaagga gcaatcaagc ttatctggat atttctttct   3660
   ttatttattt tacatggaaa taatatgatt tcactttttc tttagtttct ttgctctacg   3720
   tgggcacctg gcactaaggg agtaccttat tatcctacat cgcaaatttc aacagctaca   3780
   ttatatttcc ttctgacact tggaaggtat tgaaatttct agaaatgtat ccttctcaca   3840
   aagtagagac caagagaaaa actcattgat tgggtttcta cttctttcaa ggactcagga   3900
   aatttcactt tgaactgagg ccaagtgagc tgttaagata acccacactt aaactaaagg   3960
   ctaagaatat aggcttgatg ggaaattgaa ggtaggctga gtattgggaa tccaaattga   4020
   attttgattc tccttggcag tgaactactt tgaagaagtg gtcaatgggt tgttgctgcc   4080
   atgagcatgt acaacctctg gagctagaag ctcctcagga aagccagttc tccaagttct   4140
   taacctgtgg cactgaaagg aatgttgagt tacctcttca tgttttagac agcaaaccct   4200
   atccattaaa gtacttgtta gaacactgaa a                                  4231
<210> 39
   <211> 6322
   <212> DNA
   <213> homo sapiens
<400> 39
   ctgagagcga catgtccccg gcggctcagg cggagcggcc cgtggcgctg tttttctgag      60
   tccggggtgg cctggcagcc ggccgaggac gagggtcggc gggggctgcc cccgtggtgg     120
   tggccgccat gctgggagcc tgggcggttg agggaaccgc tgtggcgctc ctgcgactgc     180
   tgctgctgct gctgccgccg gcgatccggg gacccgggct cggcgtggcc ggcgtggccg     240
   gcgcggcggg ggccgggctg cccgagagcg tcatttgggc ggtcaacgcg ggtggagagg     300
   cgcatgtgga cgtgcacggg atccacttcc gcaaggaccc tttggaaggc cgggtgggcc     360
   gagcctcaga ctatggcatg aaactgccaa tcctgcgttc caaccctgag gaccagatcc     420
   tgtatcaaac tgagcggtac aatgaggaga cctttggcta cgaagtgccc atcaaagagg     480
   agggggacta cgtgctggtc ttgaaatttg cagaggtcta ctttgcacag tcccagcaaa     540
   aggtatttga tgtacgattg aatggccacg tcgtggtgaa ggacttggat atctttgatc     600
   gtgttgggca tagcacagct cacgatgaaa ttatacctat gagcatcaga aaggggaagc     660
   tgagtgtcca gggggaggtg tccaccttca cagggaaact ctacattgag tttgtcaagg     720
   ggtactatga caatcccaag gtctgtgcac tctacatcat ggctgggaca gtggatgatg     780
   taccaaagct tcagcctcat ccgggattgg agaagaaaga agaggaagaa gaagaagaag     840
   aatatgatga agggtctaat ctcaaaaaac agaccaataa gaaccgggtg cagtcaggcc     900
   cccgcacacc caacccctat gcctcggaca acagcagcct catgtttccc atcctggtgg     960
   ccttcggagt cttcattcca accctcttct gcctctgccg gttgtgagaa caaatgacta    1020
   tcctgaacag ggtggagggg tgtgggaaag aaaccagcca tattggtttt ggtttctgta    1080
   tttttcacaa tgattaatga acaaaaacaa agagaaaaaa aacacacatc aattaaagga    1140
   gacaaaaaga ggcagagcga gtagagagca gccctcattc accacctggt cccagacgtg    1200
   cttcagtcct cgtcctctct ttgtggctgg ctcccagcct tctctttcct cttgaggata    1260
   cttagggtaa actggatcct tcctgctcaa ggatcctcat ttgtatacct agtggaaagg    1320
   actctgaact cagaggagtc actgttcctt tttttaggtt agaaattaac agcagggaaa 1380
   tgccatctta ttacctgaga cgaccagcac tgggagttag gtacggtctg aagttatgtc 1440
   tagataagac ttcagacgtc ctgggattga aagaatgtgt gtgaaggggt agaatttgtg 1500
   cggtaaagac ttaaaaaaaa aagtagggag attaaaaaaa aagaaagaaa atgcttcctt 1560
   atctggaagc ctttctggat taatccagtg atggtcccac ctttagtgtt tgagctttgt 1620
   cattgcttgt ctccctggca tgtgccagtt atagactgtc cagcatccaa gacgtttcgg 1680
   ttatgtcggg tcctcagatc gcctctgact tgttaccaca acaaatcatt ttgatttcag 1740
   tgcctgttgg ggacttgatt tcttctcagg ttttgtttgt ttgtttgttt ccttaatctg 1800
   gctcatttga aatttcttct ccctctcaac catcccacta agttatagcc aagaagggaa 1860
   ggagacacgg ggatttgggg ttctctgctt gaatgtcttc tcctttacca cctcaccttg 1920
   ttggtacctc cctccctgga tctctgagcc agcagccagg aggacctgac ccagcagttc 1980
   tttactggcc cctttgtagg gccttgctgc cagggggcag ggatgctttc cagcctgcag 2040
   caacagaaca cttgacctta aaagtctctt ctggtctttg gattagaaaa ggcttatgtt 2100
   agcatagctt aagagcaacc tcagagactt gagccctact aagtgactga ccactgttta 2160
   gagtgtctgg tatctgatgt tcatttattc ccatgttctt gtgtgtcaca gttcagccag 2220
   ttttggttta tgcctagagc tacttcaagg aactagacta attagctata taggcccagc 2280
   gatgcttctt attgatctta atagtatgcc cttccttccc ctgtcctttc atttctctat 2340
   ccaagtagca gtcaggttct tggtgtgatg ggactgaaag aattccagtc agccagagcc 2400
   ttggcagctc tgaagctaac cttagcatct aagtgtcgat cttgaattcc ctgaaaaaat 2460
   ttctatagga aatgaagctt ccctggtccc ctcctttctg gccattgtca tccatttccc 2520
   agttagggca acaatgaagg aggacccagc caagctagaa ggaattttgt ggatgggaga 2580
   cagcaggatt agcttcagct tgggctggag cagtcaatat aggatctcag gccaggcccg 2640
   cttttctaga atgtgtttaa ttttgagttt gctttattag atatgttttt taagagctct 2700
   gtatatttga actgctcctt atgtgacaaa ataggtagct cttgggctca tgtcctgggt 2760
   tttggctctt taatgattac tccaggccag catttagtcg tttgagaatt gtagcctgtt 2820
   gttttcgctg tgacttgggt ctcagtgcta gggtattgag tcaggcagct ggagggttgt 2880
   ggcccgaggc tgcagtcaga ggtatacttc ccatagtgct tcacacagct cccctgcttc 2940
   taaaggataa ggtactgtag ccttggtcct ggggaccacc tgcctggggc agtggacatc 3000
   ctaactaaac aggcttctgg cagtagcttt ggttcctatc ccatcgaaat tccccaaagc 3060
   cctgggccac tgccattggg ttagtcaaga tgaaggagga ggactggctg cctccatttt 3120
   gccttgtttg ttagtttgcc tgggtctgtc tgaggaagga gggggtcccg ccttccacct 3180
   caacacatcc cttcagtgac tcagagtctc agaaggaaac cctgactcct ggggccattt 3240
   cctaatggta ctgtaagcca agcagctttg cttctgcctc tgtttccaag cccacccttt 3300
   tcccctgagc tcagggttag ggatgggcgc tttcctctct ggttgtgaac gaaaggaagg 3360
   aacatctttc tatggctaac aaaaactaaa ggggaagtga ggaaacagga agaagtatgg 3420
   tgggggctgg ggtagactcc cctggagcca agcctatcca gctaacaaga gctccctggg 3480
   gctggtcaca gctggctcat gatgctgaac ttgaaagttt ttttgttttt gtttttgttt 3540
   tgtggctcct ccaagatata ggtacatgaa gtttaggtta aaggggtggg attctttatt 3600
   tttatttttg tattgtatgt gtcaagaatt actctgttgt tcaccttttg ctttttgcac 3660
   tgtttgttct cttatctgta ttttgagctt agtgctagga ctgagaggct gcaccatagg 3720
   gaatgtatgg gagatggtga ggggtgccag tgaggggtgc gtggaggaga ggcctgggct 3780
   cctctactgg atctacactc tgtcccaggt ttttagatcc cactgagccc agctgactga 3840
   aaacaaggac agtcagggtg aaacttcttt tgccagaagt gtggcctgag ttgaatttct 3900
   gggaggatga cgcagatgtc tgctgcagag ctgggctgag agttctgcag tctagctctg 3960
   acttaggtca ggggcctgtt ggtctctcat tggacgtttt tgggtctcac tcatgcttac 4020
   tgaaacattg tgccaagaaa ctctgtggga tttgtgtccc ttaaaccaga ctcacttttc 4080
   tgaaaaatct ccattgttga ggagaggctg ctcaatcgac accccgagtt ctcatgactg 4140
   ggaagatagt tttcttcagg tgtcaatggc gttagactcc caggaagact agccctgccc 4200
   acagggccac ctgttggttt gagagcgtgt tcgtgttctc ttgccctccc tgcctaagag 4260
   ctactgggat cacgttagcg ggcatttagg ctttgatgag agggcacagt ttgagttagg 4320
   tttacctccc cctttctgtg cctgggaact gtttggtcca gctttagaac tgtggttttg 4380
   acttccttat ctcttgggag aagcttctgt tttaaggaat ttctcttcct tcttctcctg 4440
   cctctagcct ctcctggaaa ggcctggata tggtttctaa aatctcagct gagaacttca 4500
   gaaaacagca gcagtatttt ccttttccta gtgctaaaat ccctttccct agaaattggc 4560
   tcaccttggg aaacccaggg aaagaatcag caggttctct gccctcccta ggggttgggg 4620
   aaggacccac cccggtcagc acagtgcctt ttcctctcct gctctgagcc agggtggggc 4680
   attccctcta gattcaggtt tgggcagggg tcctatagtc cctgccatgg ggctgcttcc 4740
   ctgtcccttc cctccccttt gctggcctac tctggcataa ttcaagtgtc ttcttgcctt 4800
   ggggatcctt agtggcatca aatggcaaca tggaatattg tcctccatgc ccctccagaa 4860
   ggacctagga gagtaggtga gctttccaaa gtgagagacg aatctttctt tctttttttt 4920
   tttaaagggc aggatgggta tgctttgggc tttctccttc tgtggccccg gaggaaggag 4980
   agactgaggc aaggcaaagt gatagtacac tgaagcagaa ccggaaacac ccaggaactg 5040
   ttcagaaatc tcagaagaaa tctgcttctc ttcgatggaa agatataatt aacgatcaaa 5100
   gagctctaag aaaattgcaa agaagcctta atgttcaagc tttagaaaga tcagagcaat   5160
   ttttctcttt cagtccaaac taagactctc tgtatttaaa tctctctggg gcaagagggc   5220
   tagatttcct cattttgtta tgagactaga ttggtaccag tagatcagct gcctagcgag   5280
   ggcaggtttc ttctttgcat ctgtgtggct tgcttccagt ctggcctgtc ctttccagct   5340
   gccttttgtc tagcctgcta tgggggccca gattatcttg ataagagcag gtgatttggg   5400
   gactagctgg gttggtagga aaagagcagg atggatctct tgggacaggt tcccccagga   5460
   gtataaacac aaggagccag gattgtcctg gcagccaagg aaacagtagt gcctgtttga   5520
   gttggcagag agggccttgg cacctcttgc atccaggcag tcttgtgaga tgggggcaca   5580
   tagcactggg gaaagcagaa ctccattctc acctctattt tgagcttcag tgctttattt   5640
   cagtatgagg aaaaacaaca acaaactgaa gtgcgctttc cgtcctttca aaggacaact   5700
   gtcgggaagg gagagccgag ttgcgaggta ggaggggagc actggcaggg agagacattc   5760
   ttgactcctc tcttccctgg tgtgttgtga tccagggaat gaaaagaaat ttgaccctgg   5820
   attggttctc tccttggact taaggaatct taccttttcc ttccacaaag ttctcccagg   5880
   caaggaccag ctgcccattc tgagcccagg gcagcctctt caaccattat tggtctaacc   5940
   tggcttgtca ggaaaccaag cccacccttc cacattgggc ctggctgctc tattctgtac   6000
   caagtactgg agaaaaagca tcaagttctt agcccttgta gcttctaccc tagtttccca   6060
   tcctctctct gtggaggcca aaccaactct ttgccagcag ccacaacatg cattgacagc   6120
   ggcacagtga gatataactg atgggctttg aacctggttg gccggggaag ctgtaggggt   6180
   ggatagagct ggctttcctt ctgggctgtc tccatctgac cctacccctt ccatgtccca   6240
   ccccactccc accaaaaagt acaaaatcag gatgtttttc actgtccatt gctttgtgtt   6300
   ttaataaaca atttgcagtg ac                                              6322
<210> 40
   <211> 3600
   <212> DNA
   <213> homo sapiens
<400> 40
   gaatcaacag aatttgtctt tttgtgactg gtttatttca cttaacttca tcctcaaggt     60
   tcaacttaaa ggtgtatcca tgttgtagca cgtgtcagca ttttctttcg ttctcaggct    120
   aaatagtatt tcattgtgtg tgtacaccat gtttcatgca ttcattcatc ccttgaaaga    180
   ttggtgggtt gtttcctcct ttttgctttt gtgaacagtg ctacgaacat ggttgtacaa    240
   acatctcttg gagccccact agcagttcct ttgggtatat accccaaagt ggaattgctg    300
   gatctggtag ctcccttttt aattttttga ggaatcgcca cacagtttcc ataacagctg     360
   caccatttta cattcccaag accttttttt tttttttttt tttaagaaga aaagatgtgt     420
   ttctgcattt ctggaagtct atgctgcatt tccatttgtt gaaatttaag accagagtca    480
   tcttttctgc tgtaattata atggtcactg gcttgtgcct tttcctcctc tctctgcccc    540
   atctgcacgg ggtctttgaa caagtcccag caccttggtg gacaagcctg tgtccctggc    600
   ccatcatgga agccgctgcc tttcagagtg ggagtctgta ccctgttgcc tcattccttg    660
   ctgcgcccat gagtgagctt gtgcctgacc tctccttcca ggtggactta cacactgggc    720
   tgtcggagtt ctcggtgacg cagcgccggc tggcccatgg ctggaatgag tttgttgctg    780
   acaacagcga acctgtgtgg aagaaatacc tggatcagtt taagaacccc ctgatcctgc    840
   tgctgctggg ctctgccctg gtgagtgtcc tcaccaagga gtatgaggac gccgtcagca    900
   tcgccacggc agtgcttgtc gtggtcactg tcgccttcat ccaggagtac aggtcggaga    960
   aatctctgga agagctgacc aagctggttc ctccagaatg taactgccta agagaaggaa   1020
   aactccagca cctgcttgct cgagaactgg ttcctggtga tgtcgtatct ctctcgatcg   1080
   gagaccggat ccctgcagac atccgactca ctgaggtcac ggacctcttg gtggatgaat   1140
   ccagtttcac cggggaagcc gagccatgta gtaaaacaga cagccccttg acaggcggtg   1200
   gggacctcac caccctcagc aacatcgtct tcatggggac cctggtgcag tatgggaggg   1260
   gccagggggt cgtgattgga acaggggaaa gctctcagtt cggagaagtg tttaagatga   1320
   tgcaggctga agagacacct aaaactcctt tgcagaaaag catggacagg ctaggaaagc   1380
   aactgacact cttctccttt ggcataatcg gtctcatcat gctcattggc tggtcgcaag   1440
   ggaaacaact cctgagtatg ttcacgatcg gggtcagcct ggctgtggcg gctattccag   1500
   agggtctgcc catcgtcgtc atggtgacgc tggtcctggg agtgctgcgg atggccaaga   1560
   agcgggtcat cgtgaagaag ttacccatcg tggagacttt aggttgctgc agcgttctct   1620
   gttctgacaa gacggggact ctgactgcca atgaaatgac agtgacccag cttgtaacgt   1680
   cagatgggct tcgtgccgag gtcagcggag ttgggtatga cggtcaaggg actgtgtgtc   1740
   ttctaccatc caaggaagtc attaaggaat tttccaatgt ctcagtggga aagttagtgg   1800
   aggcgggctg tgttgccaac aatgcggtca tcagaaagaa cgccgtgatg ggcagcccca   1860
   ccgagggtgc attgatggcc ctggcgatga agatggactt aagtgatatt aaaaattcat   1920
   atataagaaa aaaagagatt ccattcagtt cagagcagaa gtggatggcg gtgaaatgca   1980
   gtctgaagac tgaggatcag gaagacattt acttcatgaa aggggccttg gaagaggtga   2040
   tccgctactg caccatgtac aacaacgggg gcatccccct gccgctgacg ccccagcaga   2100
   ggtcattctg cctgcaggaa gagaagagga tggggtcgct cggtttgcgg gtgctggccc   2160
   tggcttctgg gcccgagctg gggcggctga cgtttctagg tcttgtgggc atcattgacc    2220
   ccccgagagt tggcgtgaag gaagcagtcc aggttctctc cgagtctggt gtgtctgtga   2280
   agatgataac gggggatgcc ctggagacgg ccttggccat aggaagaaac atcggcctgt   2340
   gcaacgggaa gctgcaagcc atgtccgggg aggaggtgga cagcgtggag aagggcgagc   2400
   tggccgaccg cgtggggaag gtgtccgtgt tcttcaggac cagcccaaag cacaagctca   2460
   aaatcatcaa ggctctgcag gagtcagggg cgatcgtggc catgactggg gatggggtga   2520
   acgacgcagt ggccctgaag tctgcagaca ttgggatcgc catggggcag acagggacgg   2580
   acgtcagcaa agaggccgcc aacatgatcc tggtggatga tgacttctca gccatcatga   2640
   atgcagtgga ggaaggcaag ggtatttttt acaacatcaa aaactttgtc cgattccagc   2700
   tgagcacgag catctccgcc ctgagtctca tcactctgtc caccgtgttc aacctgccca   2760
   gccccctcaa cgccatgcag atcctatgga tcaacatcat catggatggg ccaccggcgc  2820
   agagcttggg ggtagagccc gttgacaaag acgccttcag gcagccacca cggagtgtgc   2880
   gggacaccat cctcagcaga gccctcatcc tgaagatcct catgtccgcg gccatcatca   2940
   tcagcgggac cctctttatc ttctggaagg agatgcctga agacagagca agcactcccc   3000
   gcaccacgac gatgacgttc acttgttttg tgtttttcga tctcttcaac gccttgacct    3060
   gccgctctca gaccaagctg atatttgaga tcggctttct caggaaccac atgttcctct   3120
   actccgtcct ggggtccatc ctggggcagc tggcggtcat ttacatcccc ccgctgcaga   3180
   gggtcttcca gacggagaac ctgggagcgc ttgatttgct gtttttaact ggattggcct   3240
   catccgtctt cattttgtca gagctcctca aactatgtga aaaatactgt tgcagcccca   3300
   agagagtcca gatgcaccct gaagatgtgt agtggaccgc actccgcggc accttcccta   3360
   atcatctcga tctggttgtg actgtggccc ctgccgtgtc tcctcgtcag ggagactttt   3420
   taggaggccg cagccttcca tcaccggatc agtttttcct cttaggaaag ctgcaggaac   3480
   ctcgtgggct ccagggaccc aggcccacat ccatccagcg ttcccgctgg ctgtgggaca   3540
   gacagggagg ggcctgtaca gaaacaccac actgtttatt aaatcacaat gatttttatt   3600
<210> 41
   <211> 2507
   <212> DNA
   <213> homo sapiens
<400> 41
   cagcacaccc cggcacctcc tctgcggcag ctgcgcctcg caagcgcagt gccgcagcgc      60
   acgccggagt ggctgtagct gcccggcgcg gcgccgccct gcgcgggctg tgggctgcgg     120
   gctgcgcccc cgctgctggc cagctctgca cggctgcggg ctctgcggcg cccggtgctc     180
   tgcaacgctg cggcgggcgg catgggataa cgcggccatg gtgcgccgag atcgcctccg     240
   caggatgagg gagtggtggg tccaggtggg gctgctggcc gtgcccctgc ttgctgcgta     300
   cctgcacatc ccaccccctc agctctcccc tgcccttcac tcatggaagt cttcaggcaa     360
   gtttttcact tacaagggac tgcgtatctt ctaccaagac tctgtgggtg tggttggaag     420
   tccagagata gttgtgcttt tacacggttt tccaacatcc agctacgact ggtacaagat     480
   ttgggaaggt ctgaccttga ggtttcatcg ggtgattgcc cttgatttct taggctttgg     540
   cttcagtgac aaaccgagac cacatcacta ttccatattt gagcaggcca gcatcgtgga     600
   agcgcttttg cggcatctgg ggctccagaa ccgcaggatc aaccttcttt ctcatgacta     660
   tggagatatt gttgctcagg agcttctcta caggtacaag cagaatcgat ctggtcggct     720
   taccataaag agtctctgtc tgtcaaatgg aggtatcttt cctgagactc accgtccact     780
   ccttctccaa aagctactca aagatggagg tgtgctgtca cccatcctca cacgactgat     840
   gaacttcttt gtattctctc gaggtctcac cccagtcttt gggccgtata ctcggccctc     900
   tgagagtgag ctgtgggaca tgtgggcagg gatccgcaac aatgacggga acttagtcat     960
   tgacagtctc ttacagtaca tcaatcagag gaagaagttc agaaggcgct gggtgggagc    1020
   tcttgcctct gtaactatcc ccattcattt tatctatggg ccattggatc ctgtaaatcc    1080
   ctatccagag tttttggagc tgtacaggaa aacgctgccg cggtccacag tgtcgattct    1140
   ggatgaccac attagccact atccacagct agaggatccc atgggcttct tgaatgcata    1200
   tatgggcttc atcaactcct tctgagctgg aaagagtagc ttccctgtat tacctcccct    1260
   actcccttat gtgttgtgta ttccacttag gaagaaatgc ccaaaagagg tcctggccat    1320
   caaacataat tctctcacaa agtccacttt actcaaattg gtgaacagtg tataggaaga    1380
   agccagcagg agctctgact aaggttgaca taatagtcca cctcccatta ctttgatatc    1440
   tgatcaaatg tatagacttg gctttgtttt ttgtgctatt aggaaattct gatgagcatt    1500
   actattcact gatgcagaaa gacgttcttt tgcataaaag actttttttt aacactttgg    1560
   acttctctga aatatttaga agtgctaatt tctggcccac ccccaacagg aattctatag    1620
   taaggaggag gagaaggggg gctccttccc tctcctcgaa tgacgttatg ggcacatgcc    1680
   ttttaaaagt tctttaagca acacagagct gagtcctctt tgtcatacct ttggatttag    1740
   tgtttcatca gctgttttta gttataaaca ttttgttaaa atagatattg gtttaaatga    1800
   tacagtattt taggtatgat ttaagactat gatttaccta tacattatat atattttata    1860
   aagatactaa accagcatac ccttactctg ccagagtagt gaagctaatt aaacacattt    1920
   ggtttctgaa taaattgaac taaatccaaa ctatttccta aaatcacagg acattaagga    1980
   ccaatagcat ctgtgccaga gatgtactgt tattagctgg gaagaccaat tctaacagca    2040
   aataacagtc tgagactcct catacctcag tggttagaag catgtctctc ttgagctaca    2100
   gtagagggga agggattgtt gtgtagtcaa gtcaccatgc tgaatgtaca ctgattcctt    2160
   tatgatgact gcttaactcc ccactgcctg tcccagagag gctttccaat gtagctcagt    2220
   aattcctgtt actttacaga caggaaagtt ccagaaactt taagaacaaa ctctgaaaga    2280
   cctatgagca aatggtgctg aatacttttt ttttaaagcc acatttcatt gtcttagtca    2340
   aagcaggatt attaagtgat tatttaaaat tcgttttttt aaattagcaa cttcaagtat    2400
   aacaactttg aaactggaat aagtgtttat tttctattaa taaaaatgaa ttgtgacaaa    2460
   aaaaaaaaaa aaaaaaaaaa aaaaaaaaaa aaaaaaaaaa aaaaaaa                  2507
<210> 42
   <211> 3560
   <212> DNA
   <213> homo sapiens
<400> 42
<210> 43
   <211> 2340
   <212> DNA
   <213> homo sapiens
<400> 43
   gatagcgccg ggcagaggga cccggctacc ctggacagcg catcgccgcc cgcccgggtc     60
   gccgcgccac agccgctgcg gatcatggaa catctaaagg cctttgatga tgaaatcaat    120
   gcttttttgg acaatatgtt tggaccgcga gattctcgag tcagagggtg gttcacgttg    180
   gactcttacc ttcctacctt ttttcttact gtcatgtatc tgctctcaat atggctgggt    240
   aacaagtata tgaagaacag acctgctctt tctctcaggg gtatcctcac cttgtataat    300
   cttggaatca cacttctctc cgcgtacatg ctggcagagc tcattctctc cacttgggaa    360
   ggaggctaca acttacagtg tcaagatctt accagcgcag gggaagctga catccgggta    420
   gccaaggtgc tttggtggta ctatttctcc aaatcagtag agttcctgga cacaattttc    480
   ttcgttttgc ggaaaaaaac gagtcagatt acttttcttc atgtatatca tcatgcttct    540
   atgtttaaca tctggtggtg tgtcttgaac tggatacctt gtggacaaag tttctttgga    600
   ccaacactga acagttttgt ccacattctt atgtactcct actatggact ttctgtgttt    660
   ccatctatgc acaagtatct ttggtggaag aaatatctca cacaggctca gctggtgcag    720
   ttcgtgctca ccatcacgca caccatgagc gccgtcgtga aaccgtgtgg cttccccttc    780
   ggttgtctca tcttccagtc atcttatatg ctaacgttag tcatcctctt cttaaatttt    840
   tatgttcaga cataccgaaa aaagccaatg aagaaagata tgcaagagcc acctgcaggg    900
   aaagaagtga agaatggttt ttccaaagcc tacttcactg cagcaaatgg agtgatgaac    960
   aagaaagcac aataaaaatg agtaacagaa aaagcacata tactagccta acagattggc   1020
   ttgttttaaa gcaaagactg aattgaaggt tacatgtttt aggataaact aatttctttt   1080
   gagttcataa atcatttgta cccagaatgt attaatatat tgctattagg ttaatctgtt   1140
   aactgaatgc tttgatcagc attgaggtga tgctcacctc cgaggacctc agaactggtg   1200
   cagcttctct ctccctccct cccacagact gaacctttcg ccagaagctg tccttataac   1260
   gccttatacg catacacagc caggaaacgt ggagcattgt ttctcacaga gagtctccaa   1320
   ataaaaaggg ttttgttcag attaaaatgt ttacaacaaa atgttaatta tattctaaat   1380
   acagggtatg ttctaatcta tattaagcaa taatgccagt gcataatcat tccatttgtt   1440
   cctttagcaa tcaaccccag aaaatattaa aatgggatca tacacagaag atagaaaaat   1500
   ctagcaaaac ttctctttct gtaagccaga gtcttgtcta tcagattccc acaaccactc   1560
   ctgattctaa atttagtgat atggtaatga aattggtatt tattttaaat attagttatt   1620
   ctaaggagaa aaaaatgctt ctgcaagatt ttcataattc aggggctgtg gataggattg   1680
   ttcctctgtt tccctaatca ttcatctgtt catgtctccc tcttgtgcca gtcagcctag   1740
   gttatacaga tgccatgctc cacaccacga gcagtgtaca aatctggctg cccgtttact   1800
   ttctgagcaa gcactggagt ccactccgac ctttttcttt gaacatgcat gctgctggaa   1860
   tatgtataaa tcagaactag cagaagtagc agagtgatgg gagcaaaata ggcactgaat   1920
   tcgtcaactc ttttttgtga gcctacttgt gaatattacc tcagatacct gttgtcactc   1980
   ttcacaggtt atttaagttc ttgaagctgg gaggaaaaag atggagtagc ttggaaagat   2040
   tccagcactg agccgtgagc cggtcatgag ccacgataaa aaatgccagt ttggcaaact   2100
   cagcactcct gttccctgct caggtatatg cgatctctac tgagaagcaa gcacaaaagt   2160
   agaccaaagt attaatgagt atttcctttc tccataagtg caggactgtt actcactact   2220
   aaactctacc aagaatggaa accaagaata ttttctgaag atttttttga agattaattt   2280
   ataccctata aaataaaact tgttagcttc gatgaagtca aaaaaaaaaa aaaaaaaaaa   2340
<210> 44
   <211> 7475
   <212> DNA
   <213> homo sapiens
<400> 44
   acttcagacg ccgctgatcc gggaggagct ggggtgagcc gcggcggccg tctctcccac     60
   ccgcagcagc atcctctctg cccttctctg ccaccccggg gagagccggg agctgcctct    120
   ttacagcttc cacgagccag gggtgcaggc agctgccccc aggaagtttg ggcttctgcg    180
   tagtttaggg gtgcctgcga gcgccccaga gggcgagggg ccgagggcga tgttgggcgc    240
   cgcgcggggc tgggggcgcc cagaagacgt gcgagtgtcc gcggtcctgc tgctgtctcc    300
   agtaccctcc gcatccccca agtgatggga acaagggccc gcccaggcag ccgctgtcgc    360
   cgcaccgccc cctcgctcgc tctctgcgcg cggagtcacc cagtcacact cccggcaccc    420
   cgagcccttc ctccggagct gctgcttcta ctttggctgc tatcgccgcc gccgcgggtg    480
   gcccgctgct gactgggctc gccgggagac ggagaagcac tttttggccc tccctcagca    540
   gctctcacac cccaactttg ccgccgccgc cgcgcctgcc ctcgcagcgg cgctcggccg    600
   cacattgtgg gggcgcacgc cgggaggctc cgcaagaccg tggaggcagg aaacggcact    660
   actgcgcttc tgcctcggct ctttgttgtt cgctttggat ggttcttgaa agtgtctgag    720
   cctcctcgga aatcctgggg ccggagaaga caaaccttgg aattcttcct ctgcaaaagt    780
   ctctgagata ctgacaagcg tccggaaagg tcgacgagta attgccctga aaactcttgg    840
   ctaattgacc cacgttgctt atattaagcc tttgtgtgtg gtgtgtggct tcatacattt    900
   ggggacccta tttccactcc ctcctcttgg catgagactg tatacaggat ccacccgagg    960
   acaatgattg cggagcccgc tcacttttac ctgtttggat taatatgtct ctgttcaggc   1020
   tcccgtcttc gtcaggaaga ttttccacct cgcattgttg aacacccttc agacctgatt   1080
   gtctcaaaag gagaacctgc aactttgaac tgcaaagctg aaggccgccc cacacccact   1140
   attgaatggt acaaaggggg agagagagtg gagacagaca aagatgaccc tcgctcacac   1200
   cgaatgttgc tgccgagtgg atctttattt ttcttacgta tagtacatgg acggaaaagt   1260
   agacctgatg aaggagtcta tgtctgtgta gcaaggaatt accttggaga ggctgtgagc   1320
   cacaatgcat cgctggaagt agccatactt cgggatgact tcagacaaaa cccttcggat   1380
   gtcatggttg cagtaggaga gcctgcagta atggaatgcc aacctccacg aggccatcct   1440
   gagcccacca tttcatggaa gaaagatggc tctccactgg atgataaaga tgaaagaata   1500
   actatacgag gaggaaagct catgatcact tacacccgta aaagtgacgc tggcaaatat   1560
   gtttgtgttg gtaccaatat ggttggggaa cgtgagagtg aagtagccga gctgactgtc   1620
   ttagagagac catcatttgt gaagagaccc agtaacttgg cagtaactgt ggatgacagt   1680
   gcagaattta aatgtgaggc ccgaggtgac cctgtaccta cagtacgatg gaggaaagat   1740
   gatggagagc tgcccaaatc cagatatgaa atccgagatg atcatacctt gaaaattagg   1800
   aaggtgacag ctggtgacat gggttcatac acttgtgttg cagaaaatat ggtgggcaaa   1860
   gctgaagcat ctgctactct gactgttcaa gaacctccac attttgttgt gaaaccccgt   1920
   gaccaggttg ttgctttggg acggactgta acttttcagt gtgaagcaac cggaaatcct   1980
   caaccagcta ttttctggag gagagaaggg agtcagaatc tacttttctc atatcaacca   2040
   ccacagtcat ccagccgatt ttcagtctcc cagactggcg acctcacaat tactaatgtc   2100
   cagcgatctg atgttggtta ttacatctgc cagactttaa atgttgctgg aagcatcatc   2160
   acaaaggcat atttggaagt tacagatgtg attgcagatc ggcctccccc agttattcga   2220
   caaggtcctg tgaatcagac tgtagccgtg gatggcactt tcgtcctcag ctgtgtggcc   2280
   acaggcagtc cagtgcccac cattctgtgg agaaaggatg gagtcctcgt ttcaacccaa   2340
   gactctcgaa tcaaacagtt ggagaatgga gtactgcaga tccgatatgc taagctgggt   2400
   gatactggtc ggtacacctg cattgcatca acccccagtg gtgaagcaac atggagtgct   2460
   tacattgaag ttcaagaatt tggagttcca gttcagcctc caagacctac tgacccaaat   2520
   ttaatcccta gtgccccatc aaaacctgaa gtgacagatg tcagcagaaa tacagtcaca   2580
   ttatcgtggc aaccaaattt gaattcagga gcaactccaa catcttatat tatagaagcc   2640
   ttcagccatg catctggtag cagctggcag accgtagcag agaatgtgaa aacagaaaca   2700
   tctgccatta aaggactcaa acctaatgca atttaccttt tccttgtgag ggcagctaat   2760
   gcatatggaa ttagtgatcc aagccaaata tcagatccag tgaaaacaca agatgtccta   2820
   ccaacaagtc agggggtgga ccacaagcag gtccagagag agctgggaaa tgctgttctg   2880
   cacctccaca accccaccgt cctttcttcc tcttccatcg aagtgcactg gacagtagat   2940
   caacagtctc agtatataca aggatataaa attctctatc ggccatctgg agccaaccac   3000
   ggagaatcag actggttagt ttttgaagtg aggacgccag ccaaaaacag tgtggtaatc   3060
   cctgatctca gaaagggagt caactatgaa attaaggctc gccctttttt taatgaattt   3120
   caaggagcag atagtgaaat caagtttgcc aaaaccctgg aagaagcacc cagtgcccca   3180
   ccccaaggtg taactgtatc caagaatgat ggaaacggaa ctgcaattct agttagttgg   3240
   cagccacctc cagaagacac tcaaaatgga atggtccaag agtataaggt ttggtgtctg   3300
   ggcaatgaaa ctcgatacca catcaacaaa acagtggatg gttccacctt ttccgtggtc   3360
   attccctttc ttgttcctgg aatccgatac agtgtggaag tggcagccag cactggggct   3420
   gggtctgggg taaagagtga gcctcagttc atccagctgg atgcccatgg aaaccctgtg   3480
   tcacctgagg accaagtcag cctcgctcag cagatttcag atgtggtgaa gcagccggcc   3540
   ttcatagcag gtattggagc agcctgttgg atcatcctca tggtcttcag catctggctt   3600
   tatcgacacc gcaagaagag aaacggactt actagtacct acgcgggtat cagaaaagtc   3660
   ccgtctttta ccttcacacc aacagtaact taccagagag gaggcgaagc tgtcagcagt   3720
   ggagggaggc ctggacttct caacatcagt gaacctgccg cgcagccatg gctggcagac   3780
   acgtggccta atactggcaa caaccacaat gactgctcca tcagctgctg cacggcaggc   3840
   aatggaaaca gcgacagcaa cctcactacc tacagtcgcc cagctgattg tatagcaaat   3900
   tataacaacc aactggataa caaacaaaca aatctgatgc tccctgagtc aactgtttat   3960
   ggtgatgtgg accttagtaa caaaatcaat gagatgaaaa ccttcaatag cccaaatctg   4020
   aaggatgggc gttttgtcaa tccatcaggg cagcctactc cttacgccac cactcagctc   4080
   atccagtcaa acctcagcaa caacatgaac aatggcagcg gggactctgg cgagaagcac 4140
   tggaaaccac tgggacagca gaaacaagaa gtggcaccag ttcagtacaa catcgtggag 4200
   caaaacaagc tgaacaaaga ttatcgagca aatgacacag ttcctccaac tatcccatac 4260
   aaccaatcat acgaccagaa cacaggagga tcctacaaca gctcagaccg gggcagtagt 4320
   acatctggga gtcaggggca caagaaaggg gcaagaacac ccaaggtacc aaaacagggt 4380
   ggcatgaact gggcagacct gcttcctcct cccccagcac atcctcctcc acacagcaat 4440
   agcgaagagt acaacatttc tgtagatgaa agctatgacc aagaaatgcc atgtcccgtg 4500
   ccaccagcaa ggatgtattt gcaacaagat gaattagaag aggaggaaga tgaacgaggc 4560
   cccactcccc ctgttcgggg agcagcttct tctccagctg ccgtgtccta tagccatcag 4620
   tccactgcca ctctgactcc ctccccacag gaagaactcc agcccatgtt acaggattgt 4680
   ccagaggaga ctggccacat gcagcaccag cccgacagga gacggcagcc tgtgagtcct 4740
   cctccaccac cacggccgat ctcccctcca catacctatg gctacatttc aggacccctg 4800
   gtctcagata tggatacgga tgcgccagaa gaggaagaag acgaagccga catggaggta 4860
   gccaagatgc aaaccagaag gcttttgtta cgtgggcttg agcagacacc tgcctccagt 4920
   gttggggacc tggagagctc tgtcacgggg tccatgatca acggctgggg ctcagcctca 4980
   gaggaggaca acatttccag cggacgctcc agtgttagtt cttcggacgg ctcctttttc 5040
   actgatgctg actttgccca ggcagtcgca gcagcggcag agtatgctgg tctgaaagta 5100
   gcacgacggc aaatgcagga tgctgctggc cgtcgacatt ttcatgcgtc tcagtgccct 5160
   aggcccacaa gtcccgtgtc tacagacagc aacatgagtg ccgccgtaat gcagaaaacc 5220
   agaccagcca agaaactgaa acaccagcca ggacatctgc gcagagaaac ctacacagat 5280
   gatcttccac cacctcctgt gccgccacct gctataaagt cacctactgc ccaatccaag 5340
   acacagctgg aagtacgacc tgtagtggtg ccaaaactcc cttctatgga tgcaagaaca 5400
   gacagatcat cagacagaaa aggaagcagt tacaagggga gagaagtgtt ggatggaaga 5460
   caggttgttg acatgcgaac aaatccaggt gatcccagag aagcacagga acagcaaaat 5520
   gacgggaaag gacgtggaaa caaggcagca aaacgagacc ttccaccagc aaagactcat 5580
   ctcatccaag aggatattct accttattgt agacctactt ttccaacatc aaataatccc 5640
   agagatccca gttcctcaag ctcaatgtca tcaagaggat caggaagcag acaaagagaa 5700
   caagcaaatg taggtcgaag aaatattgca gaaatgcagg tacttggagg atatgaaaga 5760
   ggagaagata ataatgaaga attagaggaa actgaaagct gaagacaacc aagaggctta 5820
   tgagatctaa tgtgaaaatc atcactcaag atgcctcctg tcagatgaca catgacgcca 5880
   gataaaatgt tcagtgcaat cagagtgtac aaattgtcgt ttttattcct cttattggga 5940
   tatcatttta aaaactttat tgggttttta ttgttgttgt ttgatcccta accctacaaa 6000
   gagccttcct attcccctcg ctgttggagc aaaccattat accttacttc cagcaagcaa 6060
   agtgctttga cttcttgctt cagtcatcag ccagcaagag ggaacaaaac tgttcttttg 6120
   cattttgccg ctgagatatg gcattgcact gcttatatgc caagctaatt tatagcaaga 6180
   tattgatcaa atatagaaag ttgatattca acctcacaag ggctctcaaa gtataatctt 6240
   tctatagcca actgctaatg caaattaaaa catatttcat tttaacatga tttcaaaatc 6300
   agtttttcat actacccttt gctggaagaa actaaaaata tagcaaatgc agaaccacaa 6360
   acaattcgaa tggggtagaa acattgtaaa tatttactct ttgcaaaccc tggtggtatt 6420
   ttattttggc ttcatttcaa tcattgaagt atattcttat tggaaatgta cttttggata 6480
   agtagggcta agccagttgg atctctggtt gtctagtcat tgtcataagt aaacctagta 6540
   aaaccttgtt ctatttttca atcatcaaaa agtaattata aatacgtatt acaaacaagt 6600
   ggatgttttt aatgaccaat tgagtaagaa catccctgtc ttaactggcc taaatttctt 6660
   ctggtagtgt cagttcaact ttcagaagtg ccacttaagg aagtttgatt tttgtttttg 6720
   taatgcactg tttttaatct ctctctcttt tttttttttt ttttggtttt aaaagcacaa 6780
   tcactaaact ttatttgtaa accattgtaa ctattaacct tttttgtctt attgaaaaaa 6840
   aaaatgttga gaagcgtttt taacctgttt tgttaatgct ctatgtttgt atttggaata 6900
   tttgaataat gacagatggt gaagtaacat gcatacttta ttgtgggcca tgaaccaaat 6960
   ggttcttact tttcctggac ttaaagaaaa aaagaggttt aagtttgttg tggccaatgt 7020
   cgaaacctac aagatttcct taaaatctct aatagaggca ttacttgctt tcaattgaca 7080
   aatgatgccc tctgactagt agatttctat gatccttttt tgtcatttta tgaatatcat 7140
   tgattttata attggtgcta tttgaagaaa aaaatgtaca tttattcata gatagataag 7200
   tatcaggtct gaccccagtg gaaaacaaag ccaaacaaaa ctgaaccaca aaaaaaaagg 7260
   ctggtgttca ccaaaaccaa acttgttcat ttagataatt tgaaaaagtt ccatagaaaa 7320
   ggcgtgcagt actaagggaa caatccatgt gattaatgtt ttcattatgt tcatgtaaga 7380
   agccccttat ttttagccat aattttgcat actgaaaatc caataatcag aaaagtaatt 7440
   ttgtcacatt atttattaaa aatgttctca aatac 7475
<210> 45
   <211> 1898
   <212> DNA
   <213> homo sapiens
<400> 45
   agcgcagtat ggcgggcggg gcccgggagg tgctcacact gcagttggga cattttgccg     60
   gtttcgtggg cgcgcactgg tggaaccagc aggatgctgc gctgggccga gcgaccgatt    120
   ccaaggagcc cccgggagag ctgtgccccg acgtcctgta tcgtacgggc cggacgctgc    180
   acggccagga gacctacacg ccgcgactca tcctcatgga tctgaagggt agtttgagct    240
   ccctaaaaga ggaaggtgga ctctacaggg acaaacagtt ggatgctgca atagcatggc    300
   aggggaagct caccacacac aaagaggaac tctatcccaa gaacccttat ctccaagact    360
   ttctgagtgc agagggagtg ctgagtagtg atggtgtctg gagggtcaaa tccattccca    420
   atggcaaagg ttcctcacca ctccccaccg ctacaactcc aaaaccactt atccctacag    480
   aggccagcat cagggtctgg tcagacttcc tcagagtcca tctccatccc cggagcatct    540
   gtatgattca gaagtacaac cacgatgggg aagcaggtcg gctggaggct tttggccaag    600
   gggaaagtgt cctaaaggaa cccaagtacc aggaagagct ggaggacagg ctgcatttct    660
   acgtggagga atgtgactac ttgcagggct tccagatcct gtgtgacctg cacgatggct    720
   tctctggggt aggcgcgaag gcggcagagc tgctacaaga tgaatattca gggcggggaa    780
   taataacctg gggcctgcta cctggtccct accatcgtgg ggaggcccag agaaacatct    840
   atcgtctatt aaacacagct tttggtctcg tgcacctgac tgctcacagc tctcttgtct    900
   gccccttgtc cttgggtggg agcctgggcc tgcgacccga gccacctgtc agcttccctt    960
   acctgcatta tgatgccact ctgcccttcc actgcagtgc catcctggct acagccctgg   1020
   acacagtcac tgttccttat cgcctgtgtt cctctccagt ttccatggtt catctggctg   1080
   acatgctgag cttctgtggg aaaaaggtgg tgacagcagg agcaatcatc cctttcccct   1140
   tggctccagg ccagtccctt cctgattccc tggtgcagtt tggaggagcc accccatgga   1200
   ccccactgtc tgcatgtggg gagccttctg gaacacgttg ctttgcccag tcagtggtgc   1260
   tgaggggtat agacagagca tgccacacaa gccagctcac cccagggaca cctccaccct   1320
   ctgcccttca tgcatgtacc actggggaag aaatcttggc tcagtattta caacagcagc   1380
   agcctggagt catgagttct tcccatctgc tgctgactcc ctgcagggtg gctcctcctt   1440
   acccccacct cttctcaagc tgcagtccac cgggtatggt tctggatggt tcccccaagg   1500
   gagcagcagt ggagagcatc ccagtgtttg gggcactgtg ttcctcttcg tccctgcacc   1560
   agaccctgga agccttggcc agagacctca ccaaactcga cttgcggcgc tgggccagct   1620
   tcatggatgc tggagtggag cacgatgacg tagcagagct gctgcaggag ctacaaagcc   1680
   tggcccagtg ctaccagggt ggtgacagcc tcgtggacta aagttcccag tgtgggagaa   1740
   aggagctagt ttgcaataaa aacagctgga tgcaggagcc cagtgtcttc atgcagagga   1800
   gctcaatgtc gcgggactag ctacaccaac atatgcactt tttacattta gaaacactgt   1860
   gattagacca cagaacaata aatatgtgcc atcagacc                           1898
<210> 46
   <211> 782
   <212> DNA
   <213> homo sapiens
<400> 46
   gggctccctg cctcgggctc tcaccctcct ctcctgcagc tccagctttg tgctctgcct     60
   ctgaggagac catggcccag tatctgagta ccctgctgct cctgctggcc accctagctg    120
   tggccctggc ctggagcccc aaggaggagg ataggataat cccgggtggc atctataacg    180
   cagacctcaa tgatgagtgg gtacagcgtg cccttcactt cgccatcagc gagtataaca    240
   aggccaccaa agatgactac tacagacgtc cgctgcgggt actaagagcc aggcaacaga    300
   ccgttggggg ggtgaattac ttcttcgacg tagaggtggg ccgcaccata tgtaccaagt    360
   cccagcccaa cttggacacc tgtgccttcc atgaacagcc agaactgcag aagaaacagt    420
   tgtgctcttt cgagatctac gaagttccct gggagaacag aaggtccctg gtgaaatcca    480
   ggtgtcaaga atcctaggga tctgtgccag gccattcgca ccagccacca cccactccca    540
   ccccctgtag tgctcccacc cctggactgg tggcccccac cctgcgggag gcctccccat    600
   gtgcctgcgc caagagacag acagagaagg ctgcaggagt cctttgttgc tcagcagggc    660
   gctctgccct ccctccttcc ttcttgcttc taatagccct ggtacatggt acacaccccc    720
   ccacctcctg caattaaaca gtagcatcgc ctccctctga aaaaaaaaaa aaaaaaaaaa    780
   aa                                                                   782
<210> 47
   <211> 1107
   <212> DNA
   <213> homo sapiens
<400> 47
   ggcacgaggg gctggcggcc ggcgggagag gcggccggcc tggactggcc cgagagggat     60
   cccggttccc agaacagacc taggaggcgg cctcgagggc ggacggcagg gagggccagc    120
   atgccccgac tgctgcaccc cgccctgccg ctgctcctgg gcgccacgct gaccttccgg    180
   gcgctccggc gcgcgctctg tcgcctgccc ctacccgtgc acgtgcgcgc cgaccccctg    240
   cgcacctggc gctggcacaa cctgctcgtc tccttcgctc actccattgt gtcggggatc    300
   tgggcactgc tgtgtgtatg gcagactcct gacatgttag tggagattga gacggcgtgg    360
   tcactttctg gctatttgct cgtttgcttc tctgcggggt atttcatcca cgatacggtg    420
   gacatcgtgg ctagcggaca gacgcgagcc tcttgggaat accttgtcca tcacgtcatg    480
   gccatgggtg ccttcttctc cggcatcttt tggagcagct ttgtcggtgg gggtgtctta    540
   acactactgg tggaagtcag caacatcttc ctcaccattc gcatgatgat gaaaatcagt    600
   aatgcccagg atcatctcct ctaccgggtt aacaagtatg tgaacctggt catgtacttt    660
   ctcttccgcc tggcccctca ggcctacctc acccatttct tcttgcgtta tgtgaaccag    720
   aggaccctgg gcaccttcct gctgggtatc ctgctcatgc tggacgtgat gatcataatc    780
   tacttttccc gcctcctccg ctctgacttc tgccctgagc atgtccccaa gaagcaacac    840
   aaagacaagt tcttgactga gtgaggggca cagagcctgg gacaacaaaa acggacaagg    900
   ccagaaacag cttcatatgg acactgggac ttagccccaa gcctgggtgt cctctgaggc    960
   cagcctctcc accttctgag cctgcgccca cactattgaa aacactaatg aaagtaaaaa   1020
   aaaaaaaaaa aaaaaaaaaa aaaaaaaaaa aaaaaaaaaa aaaaaaaaaa aaaaaaaaaa   1080
   aaaaaaaaaa aaaaaaaaaa aaaaaaa                                       1107
<210> 48
   <211> 1027
   <212> DNA
   <213> homo sapiens
<400> 48
   aggcccgtgg atctcatcga agatggcggc gcgatctgtg tcgggcatta ccagaagagt     60
   cttcatgtgg acagtctcag ggacaccatg tagagaattt tggtctcgat tcagaaaaga    120
   gaaagagcca gtggttgttg agacagtaga agagaaaaag gaacctatcc tagtgtgtcc    180
   acctttacga agccgagcat acacaccacc tgaagatctc cagagtcgtt tggaatctta    240
   cgttaaagaa gtttttggtt catctcttcc tagtaattgg caagacatct ccctggaaga    300
   tagtcgtcta aagttcaatc ttctggctca tttagctgat gacttgggtc atgtagtccc    360
   taactccaga ctccaccaga tgtgcagggt tagagatgtt cttgatttct ataatgtccc    420
   tattcaagat agatctaaat ttgatgaact cagtgccagt aatctgcccc ccaatttgaa    480
   aatcacttgg agttactaag caattcggaa gagaaacaca ttgaaatcac tgtctttccc    540
   tgagcaaggg ggctgctcat tagatctttt gatactttac catgtgaaat actaccagaa    600
   ctgttctcta aacccacttt ttctgtagag gaatgtatca tctttttttt tctcatatta    660
   caaatggaca aataacggac tttctatttt catatttgct gaaaccattt tttaaatgaa    720
   attaggtcat tatttatgaa aagttttgag agggcactgt caacttgggt ttaagacagg    780
   aggacattgc aagttcacac ctttcataag cataaagtag ttgcaagaaa gtattttcat    840
   cctgttagga ttcatatcta agatagagtt atgcattgca catacacaaa taaactttta    900
   ttagatagat acctataaaa gaaacataaa agtatgttgt gtattactga cagttctaga    960
   ttaatttctt ttagaattaa agtagatttg ttaaagtgaa aaaaaaaaaa aaaaaaaaaa   1020
   aaaaaaa                                                             1027
<210> 49
   <211> 1923
   <212> DNA
   <213> homo sapiens
<400> 49
   ctttcccgga ggctggcaga tgcgcgtggt gcggctgctg cggctccggg cggctctgac     60
   gctgctcggg gaggtccccc gccgcccggc ctcccggggt gtcccgggct cgcgcaggac    120
   gcagaagggc agtggagcca ggtgggagaa ggagaagcac gaagacgggg tgaagtggag    180
   acagctggag cacaagggcc cgtacttcgc acccccatac gagccccttc ccgacggagt    240
   gcgtttcttc tatgaaggaa ggcctgtgag attgagcgtg gcagcggagg aggtcgccac    300
   tttttatggg aggatgttag atcatgaata cacaacaaag gaggttttcc ggaagaactt    360
   cttcaatgac tggcgaaagg aaatggcggt ggaagagagg gaagtcatca agagcctgga    420
   caagtgtgac ttcacggaga tccacagata ctttgtggac aaggccgcag cccggaaagt    480
   cctgagcagg gaggagaagc agaagctaaa agaagaggca gaaaaacttc agcaagagtt    540
   cggctactgt attttagatg gtcaccaaga aaaaataggc aacttcaaga ttgagccgcc    600
   tggcttgttc cgtggccgtg gcgaccatcc caagatgggg atgctgaaga gaaggatcac    660
   gccagaggat gtggttatca actgcagcag ggactcgaag atccccgagc cgccggcggg    720
   gcaccagtgg aaggaggtgc gctccgataa caccgtcacg tggctggcag cttggaccga    780
   gagcgttcag aactccatca agtacatcat gctgaaccct tgctcgaagc tgaaggggga    840
   gacagcttgg cagaagtttg aaacagctcg acgcctgcgg ggatttgtgg acgagatccg    900
   ctcccagtac cgggctgact ggaagtctcg ggaaatgaag acgagacagc gggcggtggc    960
   cctgtatttc atcgataagc tggcactgag agcaggaaat gagaaggagg acggtgaggc   1020
   ggccgacacc gtgggctgct gttccctccg cgtggagcac gtccagctgc acccggaggc   1080
   cgatggctgc caacacgtgg tggaatttga cttcctgggg aaggactgca tccgctacta   1140
   caacagagtg ccggtggaga agccggtgta caagaactta cagctcttta tggagaacaa   1200
   ggacccccgg gacgacctct tcgacaggct gaccacgacc agcctgaaca agcacctcca   1260
   ggagctgatg gacgggctga cggccaaggt gttccggacc tacaacgcct ccatcactct   1320
   gcaggagcag ctgcgggccc tgacgcgcgc cgaggacagc atagcagcta agatcttatc   1380
   ctacaaccga gccaaccgag tcgtggccat tctctgcaac catcagcgag caacccccag   1440
   tacgttcgag aagtcgatgc agaatctcca gacgaagatc caggcaaaga aggagcaggt   1500
   ggctgaggcc agggcagagc tgaggagggc gagggctgag cacaaagccc aaggggatgg   1560
   caagtccagg agtgtcctgg agaagaagag gcggctcctg gagaagctgc aggagcagct   1620
   ggcgcagctg agtgtgcagg ccacggacaa ggaggagaac aagcaggtgg ccctgggcac   1680
   gtccaagctc aactacctgg accccaggat cagcattgcc tggtgcaagc ggttcagggt   1740
   gccagtggag aagatctaca gcaaaacaca gcgggagagg ttcgcctggg ctctcgccat   1800
   ggcaggagaa gactttgaat tctaacgacg agccgtgttg aaacttcttt tgtatgtgtg   1860
   tgtgtttttt tcactattaa agcagtactg gggaattttg tacaataaaa aaaaaaaaaa   1920
   aaa                                                                 1923
<210> 50
   <211> 1794
   <212> DNA
   <213> homo sapiens
<400> 50
   cacatataat aatagcaact cctggtcgac tgattgacca cttgaaacct ttcgtatttt     60
   ccaagtgctg gcaagcgctt cctgcgcagg ccgaggcgac ctggagtttg tgacgctgtg    120
   atggtctaga ggctggagat tcaagatctg ggtgccatca ttttctggtt ctgttgatga    180
   ccctcttcca ggttacatac agcttacatc ttgcatcctc aagcgttttt cttataaggc    240
   taaaaattca caaagcatat atcaatgaat caggaggatc tagatccgga tagtactaca    300
   gatgtgggag atgttacaaa tactgaagaa gaacttatta gagaatgtga agaaatgtgg    360
   aaagatatgg aagaatgtca gaataaatta tcacttattg gaactgaaac actcaccgat    420
   tcaaatgctc agctatcatt gttaattatg caagtaaaat gtttaaccgc tgaactcagt    480
   caatggcaga aaaaaacacc tgaaacaatt cccttgactg aagacgttct cataacatta    540
   ggaaaagaag agttccaaaa gctgagacaa gatcttgaaa tggtactgtc cactaaggag    600
   tcaaagaatg aaaagttaaa ggaagactta gaaagggaac aacggtggtt ggatgaacag    660
   caacagataa tggaatctct taatgtacta cacagtgaat tgaaaaataa ggttgaaaca    720
   ttttctgaat caagaatctt taatgaactg aaaactaaaa tgcttaatat aaaagaatat    780
   aaggagaaac tcttgagtac cttgggcgag tttctagaag accattttcc tctgcctgat     840
   agaagtgtta aaaagaaaaa gaaaaacatt caagaatcat ctgtaaacct gataacactg    900
   catgaaatgt tagagattct tataaataga ttatttgatg ttccacatga tccatatgtc    960
   aaaattagtg attccttttg gccaccttat gttgagctgc tgctgcgtaa tggaattgcc   1020
   ttgagacatc cagaagatcc aacccgaata agattagaag ctttccatca gtaaaaggat   1080
   gttttctttt ttcacacagt aaaaattctt atcattcaag gatattggaa ccacaggact   1140
   atttggataa aaaacattat ttgcaaatta atgcgcatag tacttttatt gcaaaatggc   1200
   atgtgctgcc atctattatt catttttaaa tggtcatttc ttattcagtg agtgctttag   1260
   tgttttaaac tatatggata agaatgcagg tagataatat tctaggcata aaacatttaa   1320
   tgtaccttac ctcatgcaat attctttgga ttctttgttg atttatgata ttgctaatat   1380
   aatattttct taaaatatat aacaatatct tttatgcatt tgagttccag ctggtgcttc   1440
   tttatattta gaaattataa tgggaaggtc atttaattta cagatggttt taaaattgag   1500
   gtaatatctg aggtggcata atttaaaaat atttagcaaa tttgtttcat atatactgtc   1560
   ttatttctag atttgtttaa aattggaata tgaaaaacta atggataaag ctagcataaa   1620
   attgatattt tagtttgtat tattaatata tcatgttacc ttatatatta atctactctt   1680
   gattctgcta attattacca acaaaattgt attcatgaca ttttattaat cctctgtgaa   1740
   ttttctgtaa ataaaattat ttctgaaaat ctctaaaaaa aaaaaaaaaa aaaa         1794
<210> 51
   <211> 3205
   <212> DNA
   <213> homo sapiens
<400> 51

<210> 52
   <211> 2781
   <212> DNA
   <213> homo sapiens
<400> 52
   atggatatgt ttcctctcac ctgggttttc ttagccctct acttttcaag acaccaagtg     60
   agaggccaac cagacccacc gtgcggaggt cgtttgaatt ccaaagatgc tggctatatc    120
   acctctcccg gttaccccca ggactacccc tcccaccaga actgcgagtg gattgtttac    180
   gcccccgaac ccaaccagaa gattgtcctc aacttcaacc ctcactttga aatcgagaag    240
   cacgactgca agtatgactt tatcgagatt cgggatgggg acagtgaatc cgcagacctc    300
   ctgggcaaac actgtgggaa catcgccccg cccaccatca tctcctcggg ctccatgctc    360
   tacatcaagt tcacctccga ctacgcccgg cagggggcag gcttctctct gcgctacgag    420
   atcttcaaga caggctctga agattgctca aaaaacttca caagccccaa cgggaccatc    480
   gaatctcctg ggtttcctga gaagtatcca cacaacttgg actgcacctt taccatcctg    540
   gccaaaccca agatggagat catcctgcag ttcctgatct ttgacctgga gcatgaccct    600
   ttgcaggtgg gagaggggga ctgcaagtac gattggctgg acatctggga tggcattcca    660
   catgttggcc ccctgattgg caagtactgt gggaccaaaa caccctctga acttcgttca    720
   tcgacgggga tcctctccct gacctttcac acggacatgg cggtggccaa ggatggcttc    780
   tctgcgcgtt actacctggt ccaccaagag ccactagaga actttcagtg caatgttcct    840
   ctgggcatgg agtctggccg gattgctaat gaacagatca gtgcctcatc tacctactct    900
   gatgggaggt ggacccctca acaaagccgg ctccatggtg atgacaatgg ctggaccccc    960
   aacttggatt ccaacaagga gtatctccag gtggacctgc gctttttaac catgctcacg   1020
   gccatcgcaa cacagggagc gatttccagg gaaacacaga atggctacta cgtcaaatcc   1080
   tacaagctgg aagtcagcac taatggagag gactggatgg tgtaccggca tggcaaaaac   1140
   cacaaggtat ttcaagccaa caacgatgca actgaggtgg ttctgaacaa gctccacgct   1200
   ccactgctga caaggtttgt tagaatccgc cctcagacct ggcactcagg tatcgccctc   1260
   cggctggagc tcttcggctg ccgggtcaca gatgctccct gctccaacat gctggggatg   1320
   ctctcaggcc tcattgcaga ctcccagatc tccgcctctt ccacccagga atacctctgg   1380
   agccccagtg cagcccgcct ggtcagcagc cgctcgggct ggttccctcg aatccctcag   1440
   gcccagcccg gtgaggagtg gcttcaggta gatctgggaa cacccaagac agtgaaaggt   1500
   gtcatcatcc agggagcccg cggaggagac agtatcactg ctgtggaagc cagagcattt   1560
   gtgcgcaagt tcaaagtctc ctacagccta aacggcaagg actgggaata cattcaggac   1620
   cccaggaccc agcagccaaa gctgttcgaa gggaacatgc actatgacac ccctgacatc   1680
   cgaaggtttg accccattcc ggcacagtat gtgcgggtat acccggagag gtggtcgccg   1740
   gcggggattg ggatgcggct ggaggtgctg ggctgtgact ggacagactc caagcccacg   1800
   gtaaaaacgc tgggacccac tgtgaagagc gaagagacaa ccacccccta ccccaccgaa   1860
   gaggaggcca cagagtgtgg ggagaactgc agctttgagg atgacaaaga tttgcagctc   1920
   ccttcgggat tcaattgcaa cttcgatttc ctcgaggagc cctgtggttg gatgtatgac   1980
   catgccaagt ggctccggac cacctgggcc agcagctcca gcccaaacga ccggacgttt   2040
   ccagatgaca ggaatttctt gcggctgcag agtgacagcc agagagaggg ccagtatgcc   2100
   cggctcatca gcccccctgt ccacctgccc cgaagcccgg tgtgcatgga gttccagtac   2160
   caggccacgg gcggccgcgg ggtggcgctg caggtggtgc gggaagccag ccaggagagc   2220
   aagttgctgt gggtcatccg tgaggaccag ggcggcgagt ggaagcacgg gcggatcatc   2280
   ctgcccagct acgacatgga gtaccagatt gtgttcgagg gagtgatagg gaaaggacgt   2340
   tccggagaga ttgccattga tgacattcgg ataagcactg atgtcccact ggagaactgc   2400
   atggaaccca tctcggcttt tgcagtggac atcccagaaa tacatgagag agaaggatat   2460
   gaagatgaaa ttgatgatga atacgaggtg gactggagca attcttcttc tgcaacctca   2520
   gggtctggcg ccccctcgac cgacaaagaa aagagctggc tgtacaccct ggatcccatc   2580
   ctcatcacca tcatcgccat gagctcactg ggcgtcctcc tgggggccac ctgtgcaggc   2640
   ctcctgctct actgcacctg ttcctactcg ggcctgagct cccgaagctg caccacactg   2700
   gagaactaca acttcgagct ctacgatggc cttaagcaca aggtcaagat gaaccaccaa   2760
   aagtgctgct ccgaggcatg a                                             2781
<210> 53
   <211> 1294
   <212> DNA
   <213> homo sapiens
<400> 53
<210> 54
   <211> 1712
   <212> DNA
   <213> homo sapiens
<400> 54
   ggcacgaggg gcagctgtcg gctggaagga actggtctgc tcacacttgc tggcttgcgc      60
   atcaggactg gctttatctc ctgactcacg gtgcaaaggt gcactctgcg aacgttaagt     120
   ccgtccccag cgcttggaat cctacggccc ccacagccgg atcccctcag ccttccaggt     180
   cctcaactcc cgcggacgct gaacaatggc ctccatgggg ctacaggtaa tgggcatcgc     240
   gctggccgtc ctgggctggc tggccgtcat gctgtgctgc gcgctgccca tgtggcgcgt     300
   gacggccttc atcggcagca acattgtcac ctcgcagacc atctgggagg gcctatggat     360
   gaactgcgtg gtgcagagca ccggccagat gcagtgcaag gtgtacgact cgctgctggc     420
   actgccgcag gacctgcagg cggcccgcgc cctcgtcatc atcagcatca tcgtggctgc     480
   tctgggcgtg ctgctgtccg tggtgggggg caagtgtacc aactgcctgg aggatgaaag     540
   cgccaaggcc aagaccatga tcgtggcggg cgtggtgttc ctgttggccg gccttatggt     600
   gatagtgccg gtgtcctgga cggcccacaa catcatccaa gacttctaca atccgctggt     660
   ggcctccggg cagaagcggg agatgggtgc ctcgctctac gtcggctggg ccgcctccgg     720
   cctgctgctc cttggcgggg ggctgctttg ctgcaactgt ccaccccgca cagacaagcc     780
   ttactccgcc aagtattctg ctgcccgctc tgctgctgcc agcaactacg tgtaaggtgc     840
   cacggctcca ctctgttcct ctctgctttg ttcttccctg gactgagctc agcgcaggct     900
   gtgaccccag gagggccctg ccacgggcca ctggctgctg gggactgggg actgggcaga     960
   gactgagcca ggcaggaagg cagcagcctt cagcctctct ggcccactcg gacaacttcc    1020
   caaggccgcc tcctgctagc aagaacagag tccaccctcc tctggatatt ggggagggac    1080
   ggaagtgaca gggtgtggtg gtggagtggg gagctggctt ctgctggcca ggatggctta    1140
   accctgactt tgggatctgc ctgcatcggt gttggccact gtccccattt acattttccc    1200
   cactctgtct gcctgcatct cctctgttgc gggtaggcct tgatatcacc tctgggactg    1260
   tgccttgctc accgaaaccc gcgcccagga gtatggctga ggccttgccc acccacctgc    1320
   ctgggaagtg cagagtggat ggacgggttt agaggggagg ggcgaaggtg ctgtaaacag    1380
   gtttgggcag tggtggggga gggggccaga gaggcggctc aggttgccca gctctgtggc    1440
   ctcaggactc tctgcctcac ccgcttcagc ccagggcccc tggagactga tcccctctga    1500
   gtcctctgcc ccttccaagg acactaatga gcctgggagg gtggcaggga ggaggggaca    1560
   gcttcaccct tggaagtcct ggggtttttc ctcttccttc tttgtggttt ctgttttgta    1620
   atttaagaag agctattcat cactgtaatt attattattt tctacaataa atgggacctg    1680
   tgcacaggaa aaaaaaaaaa aaaaaaaaaa aa                                  1712
<210> 55
   <211> 2820
   <212> DNA
   <213> homo sapiens
<400> 55
   ggcgggttcg cgccccgaag gctgagagct ggcgctgctc gtgccctgtg tgccagacgg     60
   cggagctccg cggccggacc ccgcggcccc gctttgctgc cgactggagt ttgggggaag    120
   aaactctcct gcgccccaga agatttcttc ctcggcgaag ggacagcgaa agatgagggt    180
   ggcaggaaga gaaggcgctt tctgtctgcc ggggtcgcag cgcgagaggg cagtgccatg    240
   ttcctctcca tcctagtggc gctgtgcctg tggctgcacc tggcgctggg cgtgcgcggc    300
   gcgccctgcg aggcggtgcg catccctatg tgccggcaca tgccctggaa catcacgcgg    360
   atgcccaacc acctgcacca cagcacgcag gagaacgcca tcctggccat cgagcagtac    420
   gaggagctgg tggacgtgaa ctgcagcgcc gtgctgcgct tcttcttctg tgccatgtac    480
   gcgcccattt gcaccctgga gttcctgcac gaccctatca agccgtgcaa gtcggtgtgc    540
   caacgcgcgc gcgacgactg cgagcccctc atgaagatgt acaaccacag ctggcccgaa    600
   agcctggcct gcgacgagct gcctgtctat gaccgtggcg tgtgcatttc gcctgaagcc    660
   atcgtcacgg acctcccgga ggatgttaag tggatagaca tcacaccaga catgatggta    720
   caggaaaggc ctcttgatgt tgactgtaaa cgcctaagcc ccgatcggtg caagtgtaaa    780
   aaggtgaagc caactttggc aacgtatctc agcaaaaact acagctatgt tattcatgcc    840
   aaaataaaag ctgtgcagag gagtggctgc aatgaggtca caacggtggt ggatgtaaaa    900
   gagatcttca agtcctcatc acccatccct cgaactcaag tcccgctcat tacaaattct    960
   tcttgccagt gtccacacat cctgccccat caagatgttc tcatcatgtg ttacgagtgg   1020
   cgttcaagga tgatgcttct tgaaaattgc ttagttgaaa aatggagaga tcagcttagt   1080
   aaaagatcca tacagtggga agagaggctg caggaacagc ggagaacagt tcaggacaag   1140
   aagaaaacag ccgggcgcac cagtcgtagt aatcccccca aaccaaaggg aaagcctcct   1200
   gctcccaaac cagccagtcc caagaagaac attaaaacta ggagtgccca gaagagaaca   1260
   aacccgaaaa gagtgtgagc taactagttt ccaaagcgga gacttccgac ttccttacag   1320
   gatgaggctg ggcattgcct gggacagcct atgtaaggcc atgtgcccct tgccctaaca   1380
   actcactgca gtgctcttca tagacacatc ttgcagcatt tttcttaagg ctatgcttca   1440
   gtttttcttt gtaagccatc acaagccata gtggtaggtt tgccctttgg tacagaaggt   1500
   gagttaaagc tggtggaaaa ggcttattgc attgcattca gagtaacctg tgtgcatact   1560
   ctagaagagt agggaaaata atgcttgtta caattcgacc taatatgtgc attgtaaaat   1620
   aaatgccata tttcaaacaa aacacgtaat ttttttacag tatgttttat taccttttga   1680
   tatctgttgt tgcaatgtta gtgatgtttt aaaatgtgat gaaaatataa tgtttttaag   1740
   aaggaacagt agtggaatga atgttaaaag atctttatgt gtttatggtc tgcagaagga   1800
   tttttgtgat gaaaggggat tttttgaaaa attagagaag tagcatatgg aaaattataa   1860
   tgtgtttttt taccaatgac ttcagtttct gtttttagct agaaacttaa aaacaaaaat   1920
   aataataaag aaaaataaat aaaaaggaga ggcagacaat gtctggattc ctgttttttg   1980
   gttacctgat ttccatgatc atgatgcttc ttgtcaacac cctcttaagc agcaccagaa   2040
   acagtgagtt tgtctgtacc attaggagtt aggtactaat tagttggcta atgctcaagt   2100
   attttatacc cacaagagag gtatgtcact catcttactt cccaggacat ccaccctgag   2160
   aataatttga caagcttaaa aatggccttc atgtgagtgc caaattttgt ttttcttcat   2220
   ttaaatattt tctttgccta aatacatgtg agaggagtta aatataaatg tacagagagg   2280
   aaagttgagt tccacctctg aaatgagaat tacttgacag ttgggatact ttaatcagaa   2340
   aaaaagaact tatttgcagc attttatcaa caaatttcat aattgtggac aattggaggc   2400
   atttatttta aaaaacaatt ttattggcct tttgctaaca cagtaagcat gtattttata   2460
   aggcattcaa taaatgcaca acgcccaaag gaaataaaat cctatctaat cctactctcc   2520
   actacacaga ggtaatcact attagtattt tggcatatta ttctccaggt gtttgcttat   2580
   gcacttataa aatgatttga acaaataaaa ctaggaacct gtatacatgt gtttcataac   2640
   ctgcctcctt tgcttggccc tttattgaga taagttttcc tgtcaagaaa gcagaaacca   2700
   tctcatttct aacagctgtg ttatattcca tagtatgcat tactcaacaa actgttgtgc   2760
   tattggatac ttaggtggtt tcttcactga caatactgaa taaacatctc accggaattc   2820
<210> 56
   <211> 1858
   <212> DNA
   <213> homo sapiens
<400> 56
   gctgcggccc cgccccctgg ccgcgtggct gcgcgtcctg gctgttgccg ataaagttgt     60
   ttgacgccgg cccggcggcg ggtcacgtga gcggaaaatg gcggccccgg caggcggcgg    120
   aggctccgcg gtgtcggtgc tggccccgaa cggccggcgc cacacggtga aggtgacgcc    180
   gagcaccgtg ctgcttcagg ttctggagga cacgtgccgg cggcaggact tcaacccctg    240
   tgaatatgat ctgaagtttc agaggagcgt gctcgacctt tctctccagt ggagatttgc    300
   caacctgccc aacaatgcca agctggagat ggtgcccgct tcccggagcc gtgaggggcc    360
   tgagaacatg gttcgcatcg ctttgcagct ggacgatggc tcgaggttgc aggactcttt    420
   ctgttcaggc cagaccctct gggagcttct cagccatttt ccacagatca gggagtgcct    480
   gcagcacccc ggcggggcca ccccagtctg cgtgtacacg agggatgagg tgacgggtga    540
   agctgccctg cggggcacga cgctgcagtc gctgggcctg accgggggca gcgccaccat    600
   caggtttgtc atgaagtgct acgaccccgt gggcaagacc ccaggaagcc tgggctcgtc    660
   agcgtcggct ggccaggcag ccgccagcgc tccacttccc ttggaatctg gggagctcag     720
   ccgcggcgac ttgagccgtc cggaggacgc ggacacctca gggccctgct gcgagcacac    780
   tcaggagaag cagagcacaa gggcacccgc agctgccccc tttgttcctt tctcgggtgg    840
   gggacagaga ctggggggcc ctcctgggcc cacgaggcct ctgacatcat cttcagctaa   900
   gttgccgaag tccctctcca gccctggagg cccctccaag ccaaagaagt ccaagtcggg    960
   ccaggatccc cagcaggagc aggagcagga gcgggagcgg gatccccagc aggagcagga   1020
   gcgggagcgg cccgtggacc gggagcccgt ggaccgggag ccggtggtgt gccaccccga   1080
   cctggaggag cggctgcagg cctggccagc ggagctgcct gatgagttct ttgagctgac   1140
   ggtggacgac gtgagaagac gcttggccca gctcaagagt gagcggaagc gcctggaaga   1200
   agcccccttg gtgaccaagg ccttcaggga ggcgcagata aaggagaagc tggagcgcta  1260
   cccaaaggtg gctctgaggg tcctgttccc cgaccgctac gtcctacagg gcttcttccg   1320
   ccccagcgag acagtggggg acttgcgaga cttcgtgagg agccacctgg ggaaccccga   1380
   gctgtcattt tacctgttca tcacccctcc aaaaacagtc ctggacgacc acacgcagac   1440
   cctctttcag gcgaacctct tcccggccgc tctggtgcac ttgggagccg aggagccggc   1500
   aggtgtctac ctggagcctg gcctgctgga gcatgccatc tccccatctg cggccgacgt   1560
   gctggtggcc aggtacatgt ccagggccgc cgggtcccct tccccattgc cagcccctga   1620
   ccctgcacct aagtctgagc cagctgctga ggagggggcg ctggtccccc ctgagcccat   1680
   cccagggacg gcccagcccg tgaagaggag cctgggcaag gtgcccaagt ggctgaagct   1740
   gccggccagc aagaggtgag agctgccagc ctgaggtgcc cactccgcca gccacaggac   1800
   cacctcctct gccagcagga ataaagactt gtgcatccct caaaaaaaaa aaaaaaaa      1858
<210> 57
   <211> 493
   <212> DNA
   <213> homo sapiens
<400> 57
   gccacttctc ttcccttcat tcttcgccag gctctctgct gactcaagtt cttcagttca     60
   cgatcttcta gttgcagcga tgagtgcacg agtgagatca agatccagag gaagaggaga    120
   tggtcaggag gctcccgatg tggttgcatt cgtggctccc ggtgaatctc agcaagagga    180
   accaccaact gacaatcagg atattgaacc tggacaagag agagaaggaa cacctccgat    240
   cgaagaacgt aaagtagaag gtgattgcca ggaaatggat ctggaaaaga ctcggagtga    300
   gcgtggagat ggctctgatg taaaagagaa gactccacct aatcctaagc atgctaagac    360
   taaagaagca ggagatgggc agccataagt taaaaagaag acaagctgaa gctacacaca    420
   tggctgatgt cacattgaaa atgtgactga aaatttgaaa attctctcaa taaagtttga    480
   gttttctctg aag                                                       493
<210> 58
   <211> 2957
   <212> DNA
   <213> homo sapiens
<400> 58
   gccagtcacc ttcagtttct ggagctggcc gtcaacatgt cctttcctaa ggcgcccttg     60
   aaacgattca atgacccttc tggttgtgca ccatctccag gtgcttatga tgttaaaact    120
   ttagaagtat tgaaaggacc agtatccttt cagaaatcac aaagatttaa acaacaaaaa    180
   gaatctaaac aaaatcttaa tgttgacaaa gatactacct tgcctgcttc agctagaaaa    240
   gttaagtctt cggaatcaaa gattcgtgtt cttctacagg aacgtggtgc ccaggacagc    300
   cggatccagg atctggaaac tgagttggaa aagatggaag caaggctaaa tgctgcacta    360
   agggaaaaaa catctctctc tgcaaataat gctacactgg aaaaacaact tattgaattg    420
   accaggacta atgaactact aaaatctaag ttttctgaaa atggtaacca gaagaatttg    480
   agaattctaa gcttggagtt gatgaaactt agaaacaaaa gagaaacaaa gatgaggggt    540
   atgatggcta agcaagaagg catggagatg aagctgcagg tcacccaaag gagtctcgaa    600
   gagtctcaag ggaaaatagc ccaactggag ggaaaacttg tttcaataga gaaagaaaag    660
   attgatgaaa aatctgaaac agaaaaactc ttggaataca tcgaagaaat tagttgtgct    720
   tcagatcaag tggaaaaata caagctagat attgcccagt tagaagaaaa tttgaaagag    780
   aagaatgatg aaattttaag ccttaagcag tctcttgagg agaatattgt tatattatct    840
   aaacaagtag aagatctaaa tgtgaaatgt cagctgcttg aaaaagaaaa agaagaccat    900
   gtcaacagga atagagaaca caacgaaaat ctaaatgcag agatgcaaaa cttaaaacag    960
   aagtttattc ttgaacaaca ggaacgtgaa aagcttcaac aaaaagaatt acaaattgat   1020
   tcacttctgc aacaagagaa agaattatct tcgagtcttc atcagaagct ctgttctttt   1080
   caagaggaaa tggttaaaga gaagaatctg tttgaggaag aattaaagca aacactggat   1140
   gagcttgata aattacagca aaaggaggaa caagctgaaa ggctggtcaa gcaattggaa   1200
   gaggaagcaa aatctagagc tgaagaatta aaactcctag aagaaaagct gaaagggaag   1260
   gaggctgaac tggagaaaag tagtgctgct catacccagg ccaccctgct tttgcaggaa   1320
   aagtatgaca gtatggtgca aagccttgaa gatgttactg ctcaatttga aagctataaa   1380
   gcgttaacag ccagtgagat agaagatctt aagctggaga actcatcatt acaggaaaaa   1440
   gcggccaagg ctgggaaaaa tgcagaggat gttcagcatc agattttggc aactgagagc   1500
   tcaaatcaag aatatgtaag gatgcttcta gatctgcaga ccaagtcagc actaaaggaa   1560
   acagaaatta aagaaatcac agtttctttt cttcaaaaaa taactgattt gcagaaccaa   1620
   ctcaagcaac aggaggaaga ctttagaaaa cagctggaag atgaagaagg aagaaaagct   1680
   gaaaaagaaa atacaacagc agaattaact gaagaaatta acaagtggcg tctcctctat   1740
   gaagaactat ataataaaac aaaacctttt cagctacaac tagatgcttt tgaagtagaa   1800
   aaacaggcat tgttgaatga acatggtgca gctcaggaac agctaaataa aataagagat   1860
   tcatatgcta aattattggg tcatcagaat ttgaaacaaa aaatcaagca tgttgtgaag   1920
   ttgaaagatg aaaatagcca actcaaatcg gaagtatcaa aactccgctg tcagcttgct   1980
   aaaaaaaaac aaagtgagac aaaacttcaa gaggaattga ataaagttct aggtatcaaa   2040
   cactttgatc cttcaaaggc ttttcatcat gaaagtaaag aaaattttgc cctgaagacc    2100
   ccattaaaag aaggcaatac aaactgttac cgagctccta tggagtgtca agaatcatgg   2160
   aagtaaacat ctgagaaacc tgttgaagat tatttcattc gtcttgttgt tattgatgtt   2220
   gctgttatta tatttgacat gggtatttta taatgttgta tttaatttta actgccaatc   2280
   cttaaatatg tgaaaggaac attttttacc aaagtgtctt ttgacatttt attttttctt   2340
   gcaaatacct cctccctaat gctcaccttt atcacctcat tctgaaccct ttcgctggct   2400
   ttccagctta gaatgcatct catcaactta aaagtcagta tcatattatt atcctcctgt   2460
   tctgaaacct tagtttcaag agtctaaacc ccagattctt cagcttgatc ctggaggtct   2520
   tttctagtct gagcttcttt agctaggcta aaacaccttg gcttgttatt gcctctactt   2580
   tgattctgat aatgctcact tggtcctacc tattatcctt ctacttgtcc agttcaaata   2640
   agaaataagg acaagcctaa cttcatagaa acctctctat ttttaatcag ttgtttaata   2700
   atttacaggt tcttaggctc catcctgttt gtatgaaatt ataatctgtg gattggcctt   2760
   taagcctgca ttcttaacaa actcttcagt taattcttag atacactaaa aatctgagaa   2820
   actctacatg taactatttc ttcagagttt gtcatatact gcttgtcatc tgcatgtcta   2880
   ctcagcattt gattaacatt tgtgtaatat gaaataaaat tacacagtaa gtcatttaac   2940
   caaaaaaaaa aaaaaaa                                                  2957
<210> 59
   <211> 6399
   <212> DNA
   <213> homo sapiens
<400> 59
   catccttcct gtcttcgcag aggagtcctc gcgtgaaata agcgggtttt gaaaacaaaa 60
   aaaagaagga gtggaagagg gggccaggat ccaggcctcc atccccacag aagtgaagct 120
   acagctggga ggtctcctcc caccccaacc gtcaccctgg gtcccgactg cccacctcct 180
   cctcctcccc ctccccccaa caacaacaac aacaacaact ccaagcacac cggccataag 240
   agtgcgtgtg tccccaacat gaccgaacga agaagggacg agctctctga agagatcaac 300
   aacttaagag agaaggtcat gaagcagtcg gaggagaaca acaacctgca gagccaggtg 360
   cagaagctca cagaggagaa caccaccctt cgagagcaag tggaacccac ccctgaggat 420
   gaggatgatg acatcgagct ccgcggtgct gcagcagctg ctgccccacc ccctccaata 480
   gaggaagagt gcccagaaga cctcccagag aagttcgatg gcaacccaga catgctggct 540
   cctttcatgg cccagtgcca gatcttcatg gaaaagagca ccagggattt ctcagttgat 600
   cgtgtccgtg tctgcttcgt gacaagcatg atgaccggcc gtgctgcccg ttgggcctca 660
   gcaaagctgg agcgctccca ctacctgatg cacaactacc cagctttcat gatggaaatg 720
   aagcatgtct ttgaagaccc tcagaggcga gaggttgcca aacgcaagat cagacgcctg 780
   cgccaaggca tggggtctgt catcgactac tccaatgctt tccagatgat tgcccaggac 840
   ctggattgga acgagcctgc gctgattgac cagtaccacg agggcctcag cgaccacatt 900
   caggaggagc tctcccacct cgaggtcgcc aagtcgctgt ctgctctgat tgggcagtgc 960
   attcacattg agagaaggct ggccagggct gctgcagctc gcaagccacg ctcgccaccc 1020
   cgggcgctgg tgttgcctca cattgcaagc caccaccagg tagatccaac cgagccggtg 1080
   ggaggtgccc gcatgcgcct gacgcaggaa gaaaaagaaa gacgcagaaa gctgaacctg 1140
   tgcctctact gtggaacagg aggtcactac gctgacaatt gtcctgccaa ggcctcaaag 1200
   tcttcgccgg cgggaaactc cccggccccg ctgtagaggg accttcagcg accgggccag 1260
   aaataataag gtccccacaa gatgatgcct catctccaca cttgcaagtg atgctccaga 1320
   ttcatcttcc gggcagacac accctgttcg tccgagccat gatcgattct ggtgcttctg 1380
   gcaacttcat tgatcacgaa tatgttgctc aaaatggaat tcctctaaga atcaaggact 1440
   ggccaatact tgtggaagca attgatgggc gccccatagc atcgggccca gttgtccacg 1500
   aaactcacga cctgatagtt gacctgggag atcaccgaga ggtgctgtca tttgatgtga 1560
   ctcagtctcc attcttccct gtcgtcctag gggttcgctg gctgagcaca catgatccca 1620
   atatcacatg gagcactcga tctatcgtct ttgattctga atactgccgc taccactgcc 1680
   ggatgtattc tccaatacca ccatcgctcc caccaccagc accacaaccg ccactctatt 1740
   atccagtaga tggatacaga gtttaccaac cagtgaggta ttactatgtc cagaatgtgt 1800
   acactccagt agatgagcac gtctacccag atcaccgcct ggttgaccct cacatagaaa 1860
   tgatacctgg agcacacagt attcccagtg gacatgtgta ttcactgtcc gaacctgaaa 1920
   tggcagctct tcgagatttt gtggcaagaa atgtaaaaga tgggctaatt actccaacga 1980
   ttgcacctaa tggagcccaa gttctccagg tgaagagggg gtggaaactg caagtttctt 2040
   atgattgccg agctccaaac aattttacta tccagaatca gtatcctcgc ctatctattc 2100
   caaatttaga agaccaagca cacctggcaa cgtacactga attcgtacct caaatacctg 2160
   gataccaaac ataccccaca tatgccgcgt acccgaccta cccagtagga ttcgcctggt 2220
   acccagtggg acgagacgga caaggaagat cactatatgt acctgtgatg atcacttgga 2280
   atccacactg gtaccgccag cctccggtac cacagtaccc gccgccacag ccgccgcctc 2340
   caccaccacc accgccgccg cctccatctt acagtaccct gtaaatacct gtcatgtcct 2400
   tcaggatctc tgccctcaaa atttattcct gttcagcttc tcaatcagtg actgtgtgct 2460
   aaattttagg ctactgtatc ttcaggccac ctgaggcaca tcctctctga aacggctatg 2520
   gaaggttagg gccactctgg actggcacac atcctaaagc accaaaagac cttcaacatt 2580
   ttctgagagc aacagagtat ttgccaataa atgatctctc atttttccac cttgactgcc 2640
   aatctaacta acaataatta ataagtttac tttccagcca gtcctggaag tctgggtttt 2700
   acctgccaaa acctccatca ccatctaaat tataggctgc caaatttgct gtttaacatt 2760
   tacagagaag ctgatacaaa cgcaggaaat gctgatttct ttatggaggg ggagacgagg 2820
   aggaggagga catgactttt cttgcggttt cggtaccctc tttttaaatc actggaggac 2880
   tgaggcctta ttaaggaagc caaaattatc ggtgcagtgt ggaaaggctt ccgtgatcct 2940
   ctcgctgcac ccttagaaac ttcaccgtct tcaaactcca tttccatggt tctgttaatt 3000
   ctcaaggagc agcaactcga ctggttctcc caggagcagg aaaaaccctt gtgacatgaa 3060
   acatctcagg cctgaaaaga aagtgctctc tcagatggac tcttgcatgt taagactatg 3120
   tcttcacatc atggtgcaaa tcacatgtac ccaatgactc cggctttgac acaacacctt 3180
   accatcatca tgccatgatg gcttccacaa agcattaaac ctggtaacca gagattactg 3240
   gtggctccag cgttgttaga tgttcatgaa atgtgaccac ctctcaatca cctttgaggg 3300
   ctaaagagta gcacatcaaa aggactccaa aatcccatac ccaactctta agagatttgt 3360
   cctggtactt cagaaagaat tttcatgagt gttcttaatt ggctggaaaa gcaccagctg 3420
   acgttttgga agaatctatc catgtgtctg cctccatatg catctgggca tttcatcttc 3480
   agtcccctca ttagactgta gcattaggat gtgtggagag aggagaaatg atttagcacc 3540
   cagattcaca ctcctatgcc tggaaggggg acatctttga agaagaggaa ttagggctgt 3600
   ggacactgtc ttgaggatgt ggacttcctt agtgagctcc acattacttg atggtaacca 3660
   cttcaaaagg atcagaatcc acgtaatgaa aaaggtccct ctagaggatg gagctgatgt 3720
   gaagctgcca atggatgaaa agcctcagaa agcaactcaa aggactcaaa gcaacggaca 3780
   acacaagagt tgtcttcagc ccagtgacac ctctgatgtc ccctggaagc tttgtgctaa   3840
   cctgggactg cctgacttcc tttagcctgg tcccttgcta ctaccttgaa ctgttttatc   3900
   taacctctct ttttctgttt aattctttgc tactgccatt gaccctgctg caggatttgt   3960
   gtcattttcc tgcctggttg ctgagactcc attttgctgc cacacacaga gatgtaagag   4020
   gcaggcttta attgccaaag cacagtttga gcagtagaaa acaacatggt gtatatctca   4080
   aattgcctga catgaagagg agtctaacgg tgaagtttca cttttcatca gcatcatctt   4140
   tcacatgttc attatcatcy gctcttattc tttgcatgtt taaacacttt aaaatttttt   4200
   agtataattt ttagtgtgtt ttgaagtggt gactaggctt tcaaaaactt ccatttgaat   4260
   tacaaagcac tatccagttc ttattgttaa actaagtaaa aatgataagt aacatagtgt   4320
   aaaatattcc tttactgtga acttcttaca atgctgtgaa tgagaggctc ctcagaactg   4380
   gagcatttgt ataataattc atcctgttca tcttcaattt taacatcata tataatttca   4440
   attctatcaa ttgggccttt aaaaatcata taaaaggata taaaatttga aaagagaaac   4500
   ctaattggct atttaatcca aaacaacttt tttttttcct tcaatggaat cagaaagctt   4560
   gtcaatcact catgtgtttt agagtaatta cttttaaaat ggtgcatttg tgcttctgaa   4620
   ctattttgaa gagtcacttc tgtttacctc aagtatcaat tcatcctcca tacatttgaa   4680
   ttcaagttgt tttttgtcaa atttacagtt gtcaattgat cttcaagctg cagggtgcct   4740
   agaaatgggc cgttgtctgt agccctggca tgtgcacacg gacatttgcc accactgcaa   4800
   gcaaaagtct ggagaagttc accaacgaca agaacgatta gggaaaatat gctgctgtgg   4860
   gttaacaact cagaaagtcc ctgatccaca tttggctgtt tactaaagct tgtgattaac   4920
   tttttggcag tgtgtactat gctctattgc tatatatgct atctataaat gtagatgtta   4980
   aggataagta attctaaatt tattattcta tagttttgaa gtttggttaa gtttcctttc   5040
   actcaattga tttattttgt tgttaatcaa atttatgtta attggatcct ttaaattttt   5100
   tttggcattt tccaacaaaa atggctttat tcataagaaa ggaaaaaaat caatggaatt   5160
   tgatatctaa agaagttaga aagggagcaa aataaaaaac ataaaggaga tagatgaatt   5220
   agtaagcaaa tcagtagtcg agtttttcaa actggcaaaa ttaattaatt gacttttagc   5280
   ccaaatttac attgttaatt aaatcaagaa ggaagaagat ctaagagctc ccattgatag   5340
   gcaagcctag agagaactag ctaaatttat catgctagga tattgaaaca cagaaagttt   5400
   acatacattt atgaagggtc aatttagttt ggacagtgag gtatttgtct tagtggaaaa   5460
   aaggagaatt agtctgatca aatcgtgaag taatacagtg aacttgcagg tgcacaaaat   5520
   aagagggcca catctatatg gtgcagtctg gaattctgtt taagtttgta ggtacctctt   5580
   ggacttctga attgatccag ttgtcatcca ccacagacat ctcacatcag atacagacag   5640
   ttccaagatt gacaacagag aacaacctgc tggaaagacc tgggcagaaa tggagagccc   5700
   tgcgggaacc atgctacatt ttcatctaaa gagagaatgc acatctgatg agactgaaag   5760
   ttctttgttg ttttagattg tagaatggta ttgaattggt ctgtggaaaa ttgcattgct   5820
   tttatttctt tgtgtaatca agtttaagta ataggggata tataatcata agcattttag   5880
   ggtgggaggg actattaagt aattttaagt gggtggggtt atttagaatg ttagaataat   5940
   attatgtatt agatatcgct ataagtggac atgcgtactt acttgtaacc ctttacccta   6000
   taattgctat ccttaaagat ttcaaataaa ctcggaggga actgcaggga gaccaactta   6060
   tttagagcga attggacatg gataaaaacc ccagtgggag aaagttcaaa ggtgattaga   6120
   ttaataattt aatagaggat gagtgacctc tgataaatta ctgctagaat gaacttgtca   6180
   atgatggatg gtaaattttc atggaagtta taaaagtgat aaataaaaac ccttgctttt   6240
   acccctgtca gtagccctcc tcctaccact gaaccccatt gcccctaccc ctccttctaa   6300
   ctttattgct gtattctctt cactctatat ttctctctat ttgctaatat tgcattgctg   6360
   ttacaataaa aattcaataa agatttagtg gttaagtgc                          6399
<210> 60
   <211> 811
   <212> DNA
   <213> homo sapiens
<400> 60
   catccctctg gctccagagc tcagagccac ccacagccgc agccatgctg tgcctcctgc      60
   tcaccctggg cgtggccctg gtctgtggtg tcccggccat ggacatcccc cagaccaagc     120
   aggacctgga gctcccaaag ttggcaggga cctggcactc catggccatg gcgaccaaca     180
   acatctccct catggcgaca ctgaaggccc ctctgagggt ccacatcacc tcactgttgc     240
   ccacccccga ggacaacctg gagatcgttc tgcacagatg ggagaacaac agctgtgttg     300
   agaagaaggt ccttggagag aagactggga atccaaagaa gttcaagatc aactatacgg     360
   tggcgaacga ggccacgctg ctcgatactg actacgacaa tttcctgttt ctctgcctac     420
   aggacaccac cacccccatc cagagcatga tgtgccagta cctggccaga gtcctggtgg     480
   aggacgatga gatcatgcag ggattcatca gggctttcag gcccctgccc aggcacctat     540
   ggtacttgct ggacttgaaa cagatggaag agccgtgccg tttctagctc acctccgcct     600
   ccaggaagac cagactccca cccttccaca cctccagagc agtgggactt cctcctgccc     660
   tttcaaagaa taaccacagc tcagaagacg atgacgtggt catctgtgtc gccatcccct     720
   tcctgctgca cacctgcacc attgccatgg ggaggctgct ccctgggggc agagtctctg     780
   gcagaggtta ttaataaacc cttggagcat g                                    811
<210> 61
   <211> 1685
   <212> DNA
   <213> homo sapiens
<400> 61
   gtcaaaggaa gttagtacat taaacaaatg gtggtaggag ggaggatttg gcccaactgg      60
   ctccaaccta tctggtcaac acgtatgttg ggtagaacag aggtggagaa aagcctagat     120
   cagggatgta tacgcttcct tggggcagac gcagcctggc cctgcggagc gatttcaagc     180
   ctcgttcacg aacatggcca aggacactgc cagcctcttc attcccctgt gtggatgctc     240
   caactccaaa agtggaacca cagggcaaat gaatgtcggc acgtgtcggt atggcagcct     300
   cgctcttcga cagctggtgt gggggttacc acctggggct agctggcctc atcttcgtta     360
   gcactgagac tggtctacca gttgcacaga ttcagatttg aaaatctcag gtgcaaatcc     420
   caagtggcga tggcatgtaa ccacatcaaa ctcaacatct gcacctaact cccaccttct     480
   ttctctggga aatcttcctg gacatgccca gatggactca ggagcccatc acctgtaaat     540
   ccccacactg cagccctgcc acttgcttgt ctgcatgtgt gggtccgctg gaccactttg     600
   gtgtatcttt gagccccttg agtgaaaaaa tgctgcctgg ctggctccct gattagacat     660
   ccaacataat gaggactaac accaatacaa acacttaaga gatgacatat cgccctagct     720
   ggatctacta cagggaaggg aagagggtgc tgggtccagg caggctgagt gtctcatgtc     780
   ttaatgcttc tctgcccaat ctatttccgg ctggatgtgg agtctgaagg cctggcaccc     840
   actctggctc tgtgatttac cagctgtgag ccttggggga gctgcttact ctcttggtga     900
   ttcttttctc atttctatga tggggtagag gataatgcct atgcttacaa agtggctgtg     960
   ggaagtaaac cggatgggat aagaatggct tgctgtggac cacaggcacc gcaggataac    1020
   cattcctcag aactcctcgt actgctctag tgcttggagg tccgtgtatt acctcagcta    1080
   ttccaaccgc accaaccacg ggagccacgt gtctacgtct gacagataaa gatgctgagt    1140
   ttagagtctg caaggcttga caaccacaga tcagggacag gagctgaggt ctcctgacct    1200
   ggagcccagg gccacccgga gctgcaagaa acctgcactc acaactgcct cctattttaa    1260
   aatgctgagt cgatcccaca ggtggcaaac cagttctggg cttcaattta caagcagtca    1320
   gaaaagctgg gttgaaatcg ccactgtcct tctatgtggc tgatgaggaa ggatgacggt    1380
   gcccaccgct ccatctctcc agctgacccc aagctggcac tcacgggtgg gcaggctcag    1440
   acaggcccag ctccaccaag tgcacttgaa gccggaatgc aagacatccg atggtatact    1500
   tactcgaacc cgtccttttc acaacagccg cgggatccgc tttctttggc tgactgctca    1560
   gagtcttccc ttttcctgtg accccattag acgccatggg gggcttttta cccttgagct    1620
   gttgaaataa acatataaga acattaaaaa taaacacaaa gtcaaacaaa aaaaaaaaaa    1680
   aaaaa                                                                1685
<210> 62
   <211> 2303
   <212> DNA
   <213> homo sapiens
<400> 62
   cgcttcccgc cgcccgagct tcggaaactt cccggccgcg acgcagggaa ccggcgcgga      60
   gaaccgagca gagcggagcg cccgtggtcc agcgtgtagg gagccgatcg cccatggagg     120
   gtctgggccg ctcgtgcctg tggctgcgtc gggagctgtc gcccccgcgg ccgcggctcc     180
   tgctcctgga ctgccgcagc cgcgagctgt acgagtcggc gcgcatcggt ggggcgctga     240
   gcgtggccct gccggcgctc ctgctgcgcc gcctgcggag gggcagcctg tcggtgcgcg      300
   cgctcctgcc tgggccgccg ctgcagccgc ccccgcctgc ccccgtgctc ctgtacgacc     360
   agggcggggg ccggcgccgg cgcggggagg ccgaggccga ggccgaggag tgggaggccg     420
   agtcggtgct gggcaccctg ctgcagaagc tgcgagagga aggctacctg gcctactacc     480
   tccagggagg cttcagcaga ttccaggccg agtgccctca cctgtgtgag accagccttg     540
   ctggccgtgc cggctccagc atggcgccgg tgcccggtcc agtgcccgtg gtggggttgg     600
   gcagcctgtg cctgggctcc gactgctctg atgcggaatc cgaggctgac cgcgactcca     660
   tgagctgtgg cctggattcg gagggtgcca cacccccacc agtggggctg cgggcatcct    720
   tccctgtcca gatcctgccc aacctctatc tgggcagtgc ccgggattcc gccaatttgg     780
   agagcctggc caaactgggc atccgctaca tcctcaatgt cacccccaac ctcccaaact     840
   tcttcgagaa gaatggtgac tttcactaca agcagatccc catctccgac cactggagcc     900
   agaacctgtc gcggttcttt ccggaggcca ttgagttcat tgatgaggcc ttgtcccaga     960
   actgcggggt gctcgtccac tgcttggcgg gggtcagccg ttctgtcacc gtcactgtgg    1020
   cctacctcat gcagaagctc cacctctctc tcaacgatgc ctatgacctg gtcaagagga    1080
   agaagtctaa catctccccc aacttcaact tcatggggca gttgctggac tttgagcgca    1140
   gcttgcggct ggaggagcgc cactcgcagg agcagggcag tggggggcag gcatctgcgg   1200
   cctccaaccc gccctccttc ttcaccaccc ccaccagtga tggcgccttc gagctggccc    1260
   ccacctaggg ccccgtggcc ggcaggccgg cccctgcccc acccccaccc acgggtgtcc    1320
   ctgcccactc gtgtggcaag ggaggggagg gcaggagggc tcggcctgag cagggtgctg   1380
   gggggagagc gcaatacctc acgcgggctg ccgtcctaat caacgtgcct atggcgggac    1440
   cacgctcgga gcctgcctct tctgcgactg ttactttttc tttgcgggat gggggtgggg    1500
   gttccctctc caggtggttg tccaagccca ggtcccggcc ctgggtgctc agccagctcg    1560
   gctaggccct gcgcctccct gcgcttcccc cttcaggaag ggtgtgtgcc acctcgttgc    1620
   actggatccc agtggctgct tgggggagag gcgtttgcca tcactggtgt tgtcacctcc    1680
   ctgtttctcc accaagggct tgggcctctc ggggctgggg cctcccaggg gatggggacc    1740
   cagaggtgca gtggccgccc acatccatgg cctaggagct actgggcagg ttcccggcca    1800
   cacatctggt gggctgtttt gttttttttt ttcctcttcc cccagatgtc ttgacgggat    1860
   cactggggct ctttgtgagt gagggtggcc aaactaccgc cggaggagat ggggtctcag    1920
   agcgagagct gcggaggggg aggggaagaa gaaggcctca cttttgctgc tgcggggccc    1980
   acacagccgc tgctactttg gggggtgggg aaggggccaa gctgcagaca cacacagtca    2040
   ttcatttctg tccacacccc tgtgggtggc gggtgtgcgt gtgtgtgctt gtgtgtgcgc    2100
   acgtgtcggc gctcacacac acatgctagc ccactgatgc acccagccca gggctggcag    2160
   tctttgcagc gtggggccgt ctcaccctgg agcctggaga ggatctatgc ttgtttgttt    2220
   ttgtaatcca tatcatagtt gctttcttta attgttcctt ctgaataaac agtttattta    2280
   agataaaaaa aaaaaaaaaa aaa                                             2303
<210> 63
   <211> 4578
   <212> DNA
   <213> homo sapiens
<400> 63
<210> 64
   <211> 5542
   <212> DNA
   <213> homo sapiens
<400> 64
   gtaattgaca aagtcacgtg tgctcagggg gccagaaact ggagagagga gagaaaaaaa     60
   tcaaaagaag gaaagcacat tagaccatgc gagctaaatt tgtgatcgca caaaatcaag    120
   atgttagatt gatgcagaag atcactccgt tccaaaggga aagttttcat ctcacgagtt    180
   tggagctgag ggcccgtggg gcaacatggc cgaaggcggg gctagcaaag gtggtggaga    240
   agagcccggg aagctgccgg agccggcaga ggaggaatcc caggttttgc gcggaactgg    300
   ccactgtaag tggttcaatg tgcgcatggg atttggattc atctccatga taaaccgaga    360
   gggaagcccc ttggatattc cagtcgatgt atttgtacac caaagcaaac tattcatgga    420
   aggatttaga agcctaaaag aaggagaacc agtggaattc acatttaaaa aatcttccaa    480
   aggccttgag tcaatacggg taacaggacc tggtgggagc ccctgtttag gaagtgaaag    540
   aagacccaaa gggaagacac tacagaaaag aaaaccaaag ggagatagat gctacaactg    600
   tggtggcctt gatcatcatg ctaaggaatg tagtctacct cctcagccaa agaagtgcca    660
   ttactgtcag agcatcatgc acatggtggc aaactgccca cataaaaatg ttgcacagcc    720
   acccgcgagt tctcagggaa gacaggaagc agaatcccag ccatgcactt caactctccc    780
   tcgagaagtg ggaggcgggc atggctgtac atcaccaccg tttcctcagg aggctagggc    840
   agagatctca gaacggtcag gcaggtcacc tcaagaagct tcctccacga agtcatctat    900
   agcaccagaa gagcaaagca aaaaggggcc ttcagttcaa aaaaggaaaa agacataaca    960
   ggtcttcttc atatgttctt tcctttaccc ggttgcaaag tctacctcat gcaagtatag   1020
   gggaacagta tttcacaagc agtagctgac ctgggatttt aactactatt ggggaactgt   1080
   gaatttttta aacagacaaa tcactctaag caaattacat ttgagcaggg tgtcatgttt   1140
   tatgttaatt cagagaataa gatactatgt ctgtcaatat gtgcatgtgt gagagggaga   1200
   gagcctgagt ctgtgtgtgt acatgaggat ttttatatag gaatgtagac acatatataa   1260
   agaggctttg tctttatata tttgtgtata gatcaaagca cacaccctct ctcatataat   1320
   tggatatttc caagaattga aaacccatgt gaagcattat agatagtttt aaatttaacc   1380
   cactggagtt ttcttgaaat accacttctt ttatattata taaaactaaa aacacgactg   1440
   ttaccttttg tgtgaaccaa aggatacttc agatctcaga gctgccaatt atggggtact   1500
   aaaggttttt aagacatcca gttctcccga atttgggatt gcctcttttt cttgaaatct   1560
   ctggagtagt aatttttttc cccctttttt gaaggcagta ccttaacttc atatgcctct   1620
   gactgccata agcttttttg attctgggat aacataactc cagaaaagac aatgaatgtg   1680
   taatttgggc cgatatttca ctgttttaaa ttctgtgttt aattgtaaaa ttagatgcct   1740
   attaagagaa atgaagggga ggatcatctt agtggcttgt tttcagtagt attttaatat   1800
   cagcttcttg taaccttttc catgttgtga gggttgtaag ggattgtgtg gcaacagcag   1860
   cttcccttgg ctaactcaat cttctaccca ttgcttagag cagggagccc tccttattta   1920
   ctactgaaga ccttagagaa ctccaattgt ttggcatata tttttggtgg tggtttttat   1980
   tcctcctgga gagttatcta atttgtttct aaaacaaaca agcagcaaag aaatgaatta   2040
   aatactgggg ttgagaatta aaattaagtg gatgttcaca gttgcccaat atatatgacc   2100
   tgcaaatgat acgaaaaagt gcagcattta gtggcagtta acaagagtga caagcctggg   2160
   gcagaggtac caaacctctc ccaccagaga gctagaagta ttttatacag taactttgat   2220
   cttatggaag tgaccttcaa tgcttattct gaagtaacct atatggtgga tacaggatga   2280
   acattcagtg ccagggagaa tcttctcagg ttggttctcg ttagagtgat aaactggcta   2340
   ggggccatag tattggtcct gttaggtttc ggtcatggaa aaaaaaatta ttttggggtc   2400
   atcctggctc tagatgttat gggcaaattt ctgaaacatc tgcaagaagg taccagttaa   2460
   ttatagtgct taatattggg aataagatta agcattataa ttataatgta tgggcctgtt   2520
   ggtgtaagct cagataatta aataaaaata gcatgactca aatgagacat attctgctga   2580
   acagtttcta cttcctctcc cgcctgtcct gtcatgggag acgtgtatag ttgctgctgt   2640
   ttcagcaaac caccataaga cgaaaatgcc tcaggttggg ttgccagtcc tttacaactc   2700
   agcttgaatt tcacaacagt gattgtgaga atctgcgtgg tatacactga aatatcggtg   2760
   tgctgtgatg caaagcttac ctttgacgat attgaatgtg atatagctgt agagaagtac   2820
   ttccttgcct tatgtgagga tttcaaactt atttaaatta tgtagacaaa tcaaagtggc   2880
   attgcttaat ttttagcagg cataataagc aagttaacag taaaatgcaa aacatgataa   2940
   gcgttgctca atttttagca ggtataataa gcaggttaac agtaaaaatg caaaacatga   3000
   tagataagtc actttgaaaa ttcaaaccaa agttccttca ccttatggaa ataggaaatt   3060
   atggacttca aaattggaca cttcctgttt acaaaaagaa attcagagct aaaatcatgg   3120
   taaaaaaaaa tagaaacact tgagaactat ggtctttatg ggtgcaattt gaaatccttt   3180
   tcatcatctt accagactaa actaagagca cataccaaac ctatcttatg gttgaaagtt   3240
   ggggtttatt ttttatatga gaatattatc actattacat aacatactca ggacaaagaa   3300
   ctttgctcag ggaacatacc atgtaatatt tttgttgttt ctttacagac tagtctacag   3360
   tcctgcttac tcaaaacaaa ccaaataact tataccttta tataagtatt atgtactgat   3420
   gatagtaact acctctgagt ttgacacaga tcaaaatttt tgaatatcag atatcagtta   3480
   tcctattttt atttcatgtg aaaactcctc taaagcagat tccctcaact ctgtgcatat   3540
   gtgaatatca ctgatgtgaa cacattgttc atttacatag gtaaaatatt actctgttta   3600
   cagcaaaagg ctacctcata gttgatacat agcacacctg tatgtatgct gttccagcct   3660
   tacaggtggc tgataattct ctggtacaga acctttttat ctgtattata aatagcaatt   3720
   cacaactgca tgtttctgac aaacacttgt gaataatgaa gcatctcgtt ttagttagca   3780
   aagtctccaa acatttcctt aaaataatca tgtatttagt ttaaagaatt atgggcactg   3840
   ttcaacttaa gcaaaacaga acacggaagc agtcttagaa gcaccacttt gcccagaggt   3900
   ggaggttgga aggggtagca gggagagggg ttggtgtatg caggtattca tgctaggcaa   3960
   agagtttaaa agacgccaat gtccttcatt tactgtctgt gctgccctga agccaagcgt   4020
   attgcagcat tatagcccca ggcacataac taactagcac tggcttgcca aggaatgaac   4080
   atgcaatgcc attactagct attgagggaa aagggtctgt gtgaagcatc actttgcagg   4140
   gattactaat ggtggggcag caggtctgtg aattaagtta tctcttgacc tcaccctcat   4200
   gtcaacacaa atgtaattcc taaacaagat gcattgccag tctcttagcc ctgtaagctg   4260
   atcttttgct acatggcaga ctataatgaa aacattttta tacttgggtt tctagtcttc   4320
   actagaaggc cttggatgta tttttgcagt tgaaagattt agaaagattt ttacctgctt   4380
   ataacttgga agtttagagt gcaatgtaag aaaaaagatc aagaaatgtc atgttattag   4440
   catcagtcca cctccaatat tgccgatact ttttttattc tggctcagtt ttattttgca   4500
   ccagtgcggc cccaagttac tgctggttgt atttagtttg tgaataggag cccataagtg   4560
   ttaatagact ttgtaacatt cactataaga tgaattatac aggacatggg aaatctcatt   4620
   aagtcttaaa gttaatttaa attaatttat ctgttttctc taagaaatgt ttatcataaa   4680
   atatatatgt gtatttcccc tttggttata aaatttggga aagtatgtac aagtgcagct   4740
   gcactgactt taattttcta gatgtcttaa tgagatttat ttgttttaga gaagaacatc   4800
   ttgttaaaag catcaaactc tgtcttacat agctgtcaac agcctcttta agatgtggtg   4860
   gttgtatgat ctgtgtctta attgttcagt tagagtgaga agttgaccta tgattcattt   4920
   ttaaatttta tatttggaac aaagctgcaa gttatggtaa agtactgtac tgtgagaagt   4980
   attatgatat ttaatgcatc tgtggcttaa cacttgtgag agttaccagc ttgaaaatga   5040
   tggtgttgac tacctcttga atcacatcta tcaaccactg gcacctacca ccaagctggc   5100
   ttcaattagt atgtgttgct ttttggtatt aacaactaac cgtactagag accaaagtga   5160
   accctgattt ttatatgtct ttaataatgg tgttttatct agtgttttta aattatcctg   5220
   tgtagtattt agattacctc attgtccatt ttgactcatg ttgtttacaa gtgaaaataa   5280
   aaacacttga actgtatgtt tttaaaagac aaaaaagggg tagatgtttg gaatgcgttt   5340
   cactcgcatg cagtcatctg gagggactga agcactgttt gcctttctgt acactctggg   5400
   ttttatattc tcatttcatg cctaatgtct tattctgtca attatggata tgttgaggtt   5460
   taaaaaaatt acttgattaa aaataaaaca tataacgttg gcatttaaaa aaaaaaaaaa   5520
   aaaaaaaaaa aaaaaaaaaa aa                                            5542
<210> 65
   <211> 5507
   <212> DNA
   <213> homo sapiens
<400> 65
   agtagctcta aaccatcttc acgatttctc tttcctcctc gtgcccgccg gagagaatag     60
   tttcgctgaa aatttctctt tgtcaatggg atcagtatta aatcagcaat atacaagtaa    120
   agtatcgcat gctgtaatgt aaaatgtggc tgaaaaatgg agttaaatga ataagtacac    180
   gcggggctag caaaggtggt ggagaagagc ccgggaagct gccggagccg gcagaggagg    240
   aatcccaggt tttgcgcgga actggccact gtaagtggtt caatgtgcgc atgggatttg    300
   gattcatctc catgataaac cgagagggaa gccccttgga tattccagtc gatgtatttg    360
   tacaccaaag caaactattc atggaaggat ttagaagcct aaaagaagga gaaccagtgg    420
   aattcacatt taaaaaatct tccaaaggcc ttgagtcaat acgggtaaca ggacctggtg    480
   ggagcccctg tttaggaagt gaaagaagac ccaaagggaa gacactacag aaaagaaaac    540
   caaagggaga tagatgctac aactgtggtg gccttgatca tcatgctaag gaatgtagtc    600
   tacctcctca gccaaagaag tgccattact gtcagagcat catgcacatg gtggcaaact    660
   gcccacataa aaatgttgca cagccacccg cgagttctca gggaagacag gaagcagaat    720
   cccagccatg cacttcaact ctccctcgag aagtgggagg cgggcatggc tgtacatcac    780
   caccgtttcc tcaggaggct agggcagaga tctcagaacg gtcaggcagg tcacctcaag    840
   aagcttcctc cacgaagtca tctatagcac cagaagagca aagcaaaaag gggccttcag    900
   ttcaaaaaag gaaaaagaca taacaggtct tcttcatatg ttctttcctt tacccggttg    960
   caaagtctac ctcatgcaag tataggggaa cagtatttca caagcagtag ctgacctggg   1020
   attttaacta ctattgggga actgtgaatt ttttaaacag acaaatcact ctaagcaaat   1080
   tacatttgag cagggtgtca tgttttatgt taattcagag aataagatac tatgtctgtc   1140
   aatatgtgca tgtgtgagag ggagagagcc tgagtctgtg tgtgtacatg aggattttta   1200
   tataggaatg tagacacata tataaagagg ctttgtcttt atatatttgt gtatagatca   1260
   aagcacacac cctctctcat ataattggat atttccaaga attgaaaacc catgtgaagc   1320
   attatagata gttttaaatt taacccactg gagttttctt gaaataccac ttcttttata   1380
   ttatataaaa ctaaaaacac gactgttacc ttttgtgtga accaaaggat acttcagatc   1440
   tcagagctgc caattatggg gtactaaagg tttttaagac atccagttct cccgaatttg   1500
   ggattgcctc tttttcttga aatctctgga gtagtaattt ttttccccct tttttgaagg   1560
   cagtacctta acttcatatg cctctgactg ccataagctt ttttgattct gggataacat   1620
   aactccagaa aagacaatga atgtgtaatt tgggccgata tttcactgtt ttaaattctg   1680
   tgtttaattg taaaattaga tgcctattaa gagaaatgaa ggggaggatc atcttagtgg   1740
   cttgttttca gtagtatttt aatatcagct tcttgtaacc ttttccatgt tgtgagggtt   1800
   gtaagggatt gtgtggcaac agcagcttcc cttggctaac tcaatcttct acccattgct   1860
   tagagcaggg agccctcctt atttactact gaagacctta gagaactcca attgtttggc   1920
   atatattttt ggtggtggtt tttattcctc ctggagagtt atctaatttg tttctaaaac   1980
   aaacaagcag caaagaaatg aattaaatac tggggttgag aattaaaatt aagtggatgt   2040
   tcacagttgc ccaatatata tgacctgcaa atgatacgaa aaagtgcagc atttagtggc   2100
   agttaacaag agtgacaagc ctggggcaga ggtaccaaac ctctcccacc agagagctag   2160
   aagtatttta tacagtaact ttgatcttat ggaagtgacc ttcaatgctt attctgaagt   2220
   aacctatatg gtggatacag gatgaacatt cagtgccagg gagaatcttc tcaggttggt   2280
   tctcgttaga gtgataaact ggctaggggc catagtattg gtcctgttag gtttcggtca   2340
   tggaaaaaaa aattattttg gggtcatcct ggctctagat gttatgggca aatttctgaa   2400
   acatctgcaa gaaggtacca gttaattata gtgcttaata ttgggaataa gattaagcat   2460
   tataattata atgtatgggc ctgttggtgt aagctcagat aattaaataa aaatagcatg   2520
   actcaaatga gacatattct gctgaacagt ttctacttcc tctcccgcct gtcctgtcat   2580
   gggagacgtg tatagttgct gctgtttcag caaaccacca taagacgaaa atgcctcagg   2640
   ttgggttgcc agtcctttac aactcagctt gaatttcaca acagtgattg tgagaatctg   2700
   cgtggtatac actgaaatat cggtgtgctg tgatgcaaag cttacctttg acgatattga   2760
   atgtgatata gctgtagaga agtacttcct tgccttatgt gaggatttca aacttattta   2820
   aattatgtag acaaatcaaa gtggcattgc ttaattttta gcaggcataa taagcaagtt   2880
   aacagtaaaa tgcaaaacat gataagcgtt gctcaatttt tagcaggtat aataagcagg   2940
   ttaacagtaa aaatgcaaaa catgatagat aagtcacttt gaaaattcaa accaaagttc   3000
   cttcacctta tggaaatagg aaattatgga cttcaaaatt ggacacttcc tgtttacaaa   3060
   aagaaattca gagctaaaat catggtaaaa aaaaatagaa acacttgaga actatggtct   3120
   ttatgggtgc aatttgaaat ccttttcatc atcttaccag actaaactaa gagcacatac 3180
   caaacctatc ttatggttga aagttggggt ttatttttta tatgagaata ttatcactat 3240
   tacataacat actcaggaca aagaactttg ctcagggaac ataccatgta atatttttgt 3300
   tgtttcttta cagactagtc tacagtcctg cttactcaaa acaaaccaaa taacttatac 3360
   ctttatataa gtattatgta ctgatgatag taactacctc tgagtttgac acagatcaaa 3420
   atttttgaat atcagatatc agttatccta tttttatttc atgtgaaaac tcctctaaag 3480
   cagattccct caactctgtg catatgtgaa tatcactgat gtgaacacat tgttcattta 3540
   cataggtaaa atattactct gtttacagca aaaggctacc tcatagttga tacatagcac 3600
   acctgtatgt atgctgttcc agccttacag gtggctgata attctctggt acagaacctt 3660
   tttatctgta ttataaatag caattcacaa ctgcatgttt ctgacaaaca cttgtgaata 3720
   atgaagcatc tcgttttagt tagcaaagtc tccaaacatt tccttaaaat aatcatgtat 3780
   ttagtttaaa gaattatggg cactgttcaa cttaagcaaa acagaacacg gaagcagtct 3840
   tagaagcacc actttgccca gaggtggagg ttggaagggg tagcagggag aggggttggt 3900
   gtatgcaggt attcatgcta ggcaaagagt ttaaaagacg ccaatgtcct tcatttactg 3960
   tctgtgctgc cctgaagcca agcgtattgc agcattatag ccccaggcac ataactaact 4020
   agcactggct tgccaaggaa tgaacatgca atgccattac tagctattga gggaaaaggg 4080
   tctgtgtgaa gcatcacttt gcagggatta ctaatggtgg ggcagcaggt ctgtgaatta 4140
   agttatctct tgacctcacc ctcatgtcaa cacaaatgta attcctaaac aagatgcatt 4200
   gccagtctct tagccctgta agctgatctt ttgctacatg gcagactata atgaaaacat 4260
   ttttatactt gggtttctag tcttcactag aaggccttgg atgtattttt gcagttgaaa 4320
   gatttagaaa gatttttacc tgcttataac ttggaagttt agagtgcaat gtaagaaaaa 4380
   agatcaagaa atgtcatgtt attagcatca gtccacctcc aatattgccg atactttttt 4440
   tattctggct cagttttatt ttgcaccagt gcggccccaa gttactgctg gttgtattta 4500
   gtttgtgaat aggagcccat aagtgttaat agactttgta acattcacta taagatgaat 4560
   tatacaggac atgggaaatc tcattaagtc ttaaagttaa tttaaattaa tttatctgtt 4620
   ttctctaaga aatgtttatc ataaaatata tatgtgtatt tcccctttgg ttataaaatt 4680
   tgggaaagta tgtacaagtg cagctgcact gactttaatt ttctagatgt cttaatgaga 4740
   tttatttgtt ttagagaaga acatcttgtt aaaagcatca aactctgtct tacatagctg 4800
   tcaacagcct ctttaagatg tggtggttgt atgatctgtg tcttaattgt tcagttagag 4860
   tgagaagttg acctatgatt catttttaaa ttttatattt ggaacaaagc tgcaagttat 4920
   ggtaaagtac tgtactgtga gaagtattat gatatttaat gcatctgtgg cttaacactt 4980
   gtgagagtta ccagcttgaa aatgatggtg ttgactacct cttgaatcac atctatcaac 5040
   cactggcacc taccaccaag ctggcttcaa ttagtatgtg ttgctttttg gtattaacaa 5100
   ctaaccgtac tagagaccaa agtgaaccct gatttttata tgtctttaat aatggtgttt 5160
   tatctagtgt ttttaaatta tcctgtgtag tatttagatt acctcattgt ccattttgac 5220
   tcatgttgtt tacaagtgaa aataaaaaca cttgaactgt atgtttttaa aagacaaaaa 5280
   aggggtagat gtttggaatg cgtttcactc gcatgcagtc atctggaggg actgaagcac 5340
   tgtttgcctt tctgtacact ctgggtttta tattctcatt tcatgcctaa tgtcttattc 5400
   tgtcaattat ggatatgttg aggtttaaaa aaattacttg attaaaaata aaacatataa 5460
   cgttggcatt taaaaaaaaa aaaaaaaaaa aaaaaaaaaa aaaaaaa 5507
<210> 66
   <211> 204
   <212> PRT
   <213> homo sapiens
<400> 66
   Met Gly Ala Pro Leu Ala Val Ala Leu Gly Ala Leu His Tyr Leu Ala
   1               5                   10                  15
   Leu Phe Leu Gln Leu Gly Gly Ala Thr Arg Pro Ala Gly His Ala Pro
               20                  25                  30
   Trp Asp Asn His Val Ser Gly His Ala Leu Phe Thr Glu Thr Pro His
           35                  40                  45
   Asp Met Thr Ala Arg Thr Gly Glu Asp Val Glu Met Ala Cys Ser Phe
       50                  55                  60
   Arg Gly Ser Gly Ser Pro Ser Tyr Ser Leu Glu Ile Gln Trp Trp Tyr
   65                  70                  75                  80
   Val Arg Ser His Arg Asp Trp Thr Asp Lys Gln Ala Trp Ala Ser Asn
                   85                  90                  95
   Gln Leu Lys Ala Ser Gln Gln Glu Asp Ala Gly Lys Glu Ala Thr Lys
               100                 105                 110
   Ile Ser Val Val Lys Val Val Gly Ser Asn Ile Ser His Lys Leu Arg
           115                 120                 125
   Leu Ser Arg Val Lys Pro Thr Asp Glu Gly Thr Tyr Glu Cys Arg Val
       130                 135                 140
   Ile Asp Phe Ser Asp Gly Lys Ala Arg His His Lys Val Lys Ala Tyr
   145                 150                 155                 160
   Leu Arg Val Gln Pro Gly Glu Asn Ser Val Leu His Leu Pro Glu Ala
                   165                 170                 175
   Pro Pro Ala Ala Pro Ala Pro Pro Pro Pro Lys Pro Gly Lys Glu Leu
               180                 185                 190
   Arg Lys Arg Ser Val Asp Gln Glu Ala Cys Ser Leu
           195                 200
<210> 67
   <211> 193
   <212> PRT
   <213> homo sapiens
<400> 67
   Met Asp Gly Gly Thr Leu Pro Arg Ser Ala Pro Pro Ala Pro Pro Val
   1               5                   10                  15
   Pro Val Gly Cys Ala Ala Arg Arg Arg Pro Ala Ser Pro Glu Leu Leu
               20                  25                  30
   Arg Cys Ser Arg Arg Arg Arg Pro Ala Thr Ala Glu Thr Gly Gly Gly
           35                  40                  45
   Ala Ala Ala Val Ala Arg Arg Asn Glu Arg Glu Arg Asn Arg Val Lys
       50                  55                  60
   Leu Val Asn Leu Gly Phe Gln Ala Leu Arg Gln His Val Pro His Gly
   65                  70                  75                  80
   Gly Ala Ser Lys Lys Leu Ser Lys Val Glu Thr Leu Arg Ser Ala Val
                   85                  90                  95
   Glu Tyr Ile Arg Ala Leu Gln Arg Leu Leu Ala Glu His Asp Ala Val
               100                 105                 110
   Arg Asn Ala Leu Ala Gly Gly Leu Arg Pro Gln Ala Val Arg Pro Ser
           115                 120                 125
   Ala Pro Arg Gly Pro Pro Gly Thr Thr Pro Val Ala Ala Ser Pro Ser
       130                 135                 140
   Arg Ala Ser Ser Ser Pro Gly Arg Gly Gly Ser Ser Glu Pro Gly Ser
   145                 150                 155                 160
   Pro Arg Ser Ala Tyr Ser Ser Asp Asp Ser Gly Cys Glu Gly Ala Leu
                   165                 170                 175
   Ser Pro Ala Glu Arg Glu Leu Leu Asp Phe Ser Ser Trp Leu Gly Gly
               180                 185                 190
   Tyr
<210> 68
   <211> 354
   <212> PRT
   <213> homo sapiens
<400> 68
   Met Arg Arg Leu Met Ser Ser Arg Asp Trp Pro Arg Thr Arg Thr Gly
   1               5                   10                  15
   Thr Gly Ile Leu Ser Ser Gln Pro Glu Glu Asn Pro Tyr Trp Trp Asn
               20                  25                  30
   Ala Asn Met Val Phe Ile Pro Tyr Cys Ser Ser Asp Val Trp Ser Gly
           35                  40                  45
   Ala Ser Ser Lys Ser Glu Lys Asn Glu Tyr Ala Phe Met Gly Ala Leu
       50                  55                  60
   Ile Ile Gln Glu Val Val Arg Glu Leu Leu Gly Arg Gly Leu Ser Gly
   65                  70                  75                  80
   Ala Lys Val Leu Leu Leu Ala Gly Ser Ser Ala Gly Gly Thr Gly Val
                   85                  90                  95
   Leu Leu Asn Val Asp Arg Val Ala Glu Gln Leu Glu Lys Leu Gly Tyr
               100                 105                 110
   Pro Ala Ile Gln Val Arg Gly Leu Ala Asp Ser Gly Trp Phe Leu Asp
           115                 120                 125
   Asn Lys Gln Tyr Arg His Thr Asp Cys Val Asp Thr Ile Thr Cys Ala
       130                 135                 140
   Pro Thr Glu Ala Ile Arg Arg Gly Ile Arg Tyr Trp Asn Gly Val Val
   145                 150                 155                 160
   Pro Glu Arg Cys Arg Arg Gln Phe Gln Glu Gly Glu Glu Trp Asn Cys
                   165                 170                 175
   Phe Phe Gly Tyr Lys Val Tyr Pro Thr Leu Arg Cys Pro Val Phe Val
               180                 185                 190
   Val Gln Trp Leu Phe Asp Glu Ala Gln Leu Thr Val Asp Asn Val His
           195                 200                 205
   Leu Thr Gly Gln Pro Val Gln Glu Gly Leu Arg Leu Tyr Ile Gln Asn
       210                 215                 220
   Leu Gly Arg Glu Leu Arg His Thr Leu Lys Asp Val Pro Ala Ser Phe
   225                 230                 235                 240
   Ala Pro Ala Cys Leu Ser His Glu Ile Ile Ile Arg Ser His Trp Thr
                   245                 250                 255
   Asp Val Gln Val Lys Gly Thr Ser Leu Pro Arg Ala Leu His Cys Trp
               260                 265                 270
   Asp Arg Ser Leu His Asp Ser His Lys Ala Ser Lys Thr Pro Leu Lys
           275                 280                 285
   Gly Cys Pro Val His Leu Val Asp Ser Cys Pro Trp Pro His Cys Asn
       290                 295                 300
   Pro Ser Cys Pro Thr Val Arg Asp Gln Phe Thr Gly Gln Glu Met Asn
   305                 310                 315                 320
   Val Ala Gln Phe Leu Met His Met Gly Phe Asp Met Gln Thr Val Ala
                   325                 330                 335
   Gln Pro Gln Gly Leu Glu Pro Ser Glu Leu Leu Gly Met Leu Ser Asn
               340                 345                 350
   Gly Ser
<210> 69
   <211> 362
   <212> PRT
   <213> homo sapiens
<400> 69
<210> 70
   <211> 137
   <212> PRT
   <213> homo sapiens
<400> 70
   Met Pro Arg Pro Arg Ala His Arg Cys Val Trp Thr Arg Val Ala Ala
   1               5                   10                  15
   Ser Arg Thr Gly Ala Pro Arg Ser Thr Thr Ala Ser Cys Ser Ala Ser
               20                  25                  30
   Gly Arg Cys Ala Trp Ala Ser Val Trp Arg Cys Glu Cys Cys Gly Arg
           35                  40                  45
   Arg Ala Ala Gly Ala Ala Ala Ser Ala Trp Ala Ser Arg Ala Trp Thr
       50                  55                  60
   Pro Arg Ala Cys Pro Cys Pro Ala Cys Arg Pro Ser Cys Ala Pro Thr
   65                  70                  75                  80
   Trp Arg Ser Arg Ala Arg Arg Gly Arg Pro Cys Cys Leu Arg Ala Ala
                   85                  90                  95
   Arg Ser Leu Gly Thr Trp Ser Ala Ser Gly Trp Thr Ala Ala Ala Ala
               100                 105                 110
   Ser Ser Pro Arg Ser Thr Pro Ala Ala Gly Ser Cys Cys Val Arg Ala
           115                 120                 125
   Cys Pro Ser Ala Pro Arg Ser Gly Pro
       130                 135
<210> 71
   <211> 426
   <212> PRT
   <213> homo sapiens
<400> 71
   Met Pro Leu Leu Trp Leu Arg Gly Phe Leu Leu Ala Ser Cys Trp Ile
   1               5                   10                  15
   Ile Val Arg Ser Ser Pro Thr Pro Gly Ser Glu Gly His Ser Ala Ala
               20                  25                  30
   Pro Asp Cys Pro Ser Cys Ala Leu Ala Ala Leu Pro Lys Asp Val Pro
           35                  40                  45
   Asn Ser Gln Pro Glu Met Val Glu Ala Val Lys Lys His Ile Leu Asn
       50                  55                  60
   Met Leu His Leu Lys Lys Arg Pro Asp Val Thr Gln Pro Val Pro Lys
   65                  70                  75                  80
   Ala Ala Leu Leu Asn Ala Ile Arg Lys Leu His Val Gly Lys Val Gly
                   85                  90                  95
   Glu Asn Gly Tyr Val Glu Ile Glu Asp Asp Ile Gly Arg Arg Ala Glu
               100                 105                 110
   Met Asn Glu Leu Met Glu Gln Thr Ser Glu Ile Ile Thr Phe Ala Glu
           115                 120                 125
   Ser Gly Thr Ala Arg Lys Thr Leu His Phe Glu Ile Ser Lys Glu Gly
       130                 135                 140
   Ser Asp Leu Ser Val Val Glu Arg Ala Glu Val Trp Leu Phe Leu Lys
   145                 150                 155                 160
   Val Pro Lys Ala Asn Arg Thr Arg Thr Lys Val Thr Ile Arg Leu Phe
                   165                 170                 175
   Gln Gln Gln Lys His Pro Gln Gly Ser Leu Asp Thr Gly Glu Glu Ala
               180                 185                 190
   Glu Glu Val Gly Leu Lys Gly Glu Arg Ser Glu Leu Leu Leu Ser Glu
           195                 200                 205
   Lys Val Val Asp Ala Arg Lys Ser Thr Trp His Val Phe Pro Val Ser
       210                 215                 220
   Ser Ser Ile Gln Arg Leu Leu Asp Gln Gly Lys Ser Ser Leu Asp Val
   225                 230                 235                 240
   Arg Ile Ala Cys Glu Gln Cys Gln Glu Ser Gly Ala Ser Leu Val Leu
                   245                 250                 255
   Leu Gly Lys Lys Lys Lys Lys Glu Glu Glu Gly Glu Gly Lys Lys Lys
               260                 265                 270
   Gly Gly Gly Glu Gly Gly Ala Gly Ala Asp Glu Glu Lys Glu Gln Ser
           275                 280                 285
   His Arg Pro Phe Leu Met Leu Gln Ala Arg Gln Ser Glu Asp His Pro
       290                 295                 300
   His Arg Arg Arg Arg Arg Gly Leu Glu Cys Asp Gly Lys Val Asn Ile
   305                 310                 315                 320
   Cys Cys Lys Lys Gln Phe Phe Val Ser Phe Lys Asp Ile Gly Trp Asn
                   325                 330                 335
   Asp Trp Ile Ile Ala Pro Ser Gly Tyr His Ala Asn Tyr Cys Glu Gly
               340                 345                 350
   Glu Cys Pro Ser His Ile Ala Gly Thr Ser Gly Ser Ser Leu Ser Phe
           355                 360                 365
   His Ser Thr Val Ile Asn His Tyr Arg Met Arg Gly His Ser Pro Phe
       370                 375                 380
   Ala Asn Leu Lys Ser Cys Cys Val Pro Thr Lys Leu Arg Pro Met Ser
   385                 390                 395                 400
   Met Leu Tyr Tyr Asp Asp Gly Gln Asn Ile Ile Lys Lys Asp Ile Gln
                   405                 410                 415
   Asn Met Ile Val Glu Glu Cys Gly Cys Ser
               420                 425
<210> 72
   <211> 868
   <212> PRT
   <213> homo sapiens
<400> 72
   Met Ala Ser Phe Pro Glu Thr Asp Phe Gln Ile Cys Leu Leu Cys Lys
   1               5                   10                  15
   Glu Met Cys Gly Ser Pro Ala Pro Leu Ser Ser Asn Ser Ser Ala Ser
               20                  25                  30
   Ser Ser Ser Ser Gln Thr Ser Thr Ser Ser Gly Gly Gly Gly Gly Gly
           35                  40                  45
   Pro Gly Ala Ala Ala Arg Arg Leu His Val Leu Pro Cys Leu His Ala
       50                  55                  60
   Phe Cys Arg Pro Cys Leu Glu Ala His Arg Leu Pro Ala Ala Gly Gly
   65                  70                  75                  80
   Gly Ala Ala Gly Glu Pro Leu Lys Leu Arg Cys Pro Val Cys Asp Gln
                   85                  90                  95
   Lys Val Val Leu Ala Glu Ala Ala Gly Met Asp Ala Leu Pro Ser Ser
               100                 105                 110
   Ala Phe Leu Leu Asn Asn Leu Leu Asp Ala Val Val Ala Thr Ala Asp
           115                 120                 125
   Glu Pro Pro Pro Lys Asn Gly Arg Ala Gly Ala Pro Ala Gly Ala Gly
       130                 135                 140
   Gly His Ser Asn His Arg His His Ala His His Ala His Pro Arg Ala
   145                 150                 155                 160
   Ser Ala Ser Ala Pro Pro Leu Pro Gln Ala Pro Gln Pro Pro Ala Pro
                   165                 170                 175
   Ser Arg Ser Ala Pro Gly Gly Pro Ala Ala Ser Pro Ser Ala Leu Leu
               180                 185                 190
   Leu Arg Arg Pro His Gly Cys Ser Ser Cys Asp Glu Gly Asn Ala Ala
           195                 200                 205
   Ser Ser Arg Cys Leu Asp Cys Gln Glu His Leu Cys Asp Asn Cys Val
       210                 215                 220
   Arg Ala His Gln Arg Val Arg Leu Thr Lys Asp His Tyr Ile Glu Arg
   225                 230                 235                 240
   Gly Pro Pro Gly Pro Gly Ala Ala Ala Ala Ala Gln Gln Leu Gly Leu
                   245                 250                 255
   Gly Pro Pro Phe Pro Gly Pro Pro Phe Ser Ile Leu Ser Val Phe Pro
               260                 265                 270
   Glu Arg Leu Gly Phe Cys Gln His His Asp Asp Glu Val Leu His Leu
           275                 280                 285
   Tyr Cys Asp Thr Cys Ser Val Pro Ile Cys Arg Glu Cys Thr Met Gly
       290                 295                 300
   Arg His Gly Gly His Ser Phe Ile Tyr Leu Gln Glu Ala Leu Gln Asp
   305                 310                 315                 320
   Ser Arg Ala Leu Thr Ile Gln Leu Leu Ala Asp Ala Gln Gln Gly Arg
                   325                 330                 335
   Gln Ala Ile Gln Leu Ser Ile Glu Gln Ala Gln Thr Val Ala Glu Gln
               340                 345                 350
   Val Glu Met Lys Ala Lys Val Val Gln Ser Glu Val Lys Ala Val Thr
           355                 360                 365
   Ala Arg His Lys Lys Ala Leu Glu Glu Arg Glu Cys Glu Leu Leu Trp
       370                 375                 380
   Lys Val Glu Lys Ile Arg Gln Val Lys Ala Lys Ser Leu Tyr Leu Gln
   385                 390                 395                 400
   Val Glu Lys Leu Arg Gln Asn Leu Asn Lys Leu Glu Ser Thr Ile Ser
                   405                 410                 415
   Ala Val Gln Gln Val Leu Glu Glu Gly Arg Ala Leu Asp Ile Leu Leu
               420                 425                 430
   Ala Arg Asp Arg Met Leu Ala Gln Val Gln Glu Leu Lys Thr Val Arg
           435                 440                 445
   Ser Leu Leu Gln Pro Gln Glu Asp Asp Arg Val Met Phe Thr Pro Pro
       450                 455                 460
   Asp Gln Ala Leu Tyr Leu Ala Ile Lys Ser Phe Gly Phe Val Ser Ser
   465                 470                 475                 480
   Gly Ala Phe Ala Pro Leu Thr Lys Ala Thr Gly Asp Gly Leu Lys Arg
                   485                 490                 495
   Ala Leu Gln Gly Lys Val Ala Ser Phe Thr Val Ile Gly Tyr Asp His
               500                 505                 510
   Asp Gly Glu Pro Arg Leu Ser Gly Gly Asp Leu Met Ser Ala Val Val
           515                 520                 525
   Leu Gly Pro Asp Gly Asn Leu Phe Gly Ala Glu Val Ser Asp Gln Gln
       530                 535                 540
   Asn Gly Thr Tyr Val Val Ser Tyr Arg Pro Gln Leu Glu Gly Glu His
   545                 550                 555                 560
   Leu Val Ser Val Thr Leu Cys Asn Gln His Ile Glu Asn Ser Pro Phe
                   565                 570                 575
   Lys Val Val Val Lys Ser Gly Arg Ser Tyr Val Gly Ile Gly Leu Pro
               580                 585                 590
   Gly Leu Ser Phe Gly Ser Glu Gly Asp Ser Asp Gly Lys Leu Cys Arg
           595                 600                 605
   Pro Trp Gly Val Ser Val Asp Lys Glu Gly Tyr Ile Ile Val Ala Asp
       610                 615                 620
   Arg Ser Asn Asn Arg Ile Gln Val Phe Lys Pro Cys Gly Ala Phe His
   625                 630                 635                 640
   His Lys Phe Gly Thr Leu Gly Ser Arg Pro Gly Gln Phe Asp Arg Pro
                   645                 650                 655
   Ala Gly Val Ala Cys Asp Ala Ser Arg Arg Ile Val Val Ala Asp Lys
               660                 665                 670
   Asp Asn His Arg Ile Gln Ile Phe Thr Phe Glu Gly Gln Phe Leu Leu
           675                 680                 685
   Lys Phe Gly Glu Lys Gly Thr Lys Asn Gly Gln Phe Asn Tyr Pro Trp
       690                 695                 700
   Asp Val Ala Val Asn Ser Glu Gly Lys Ile Leu Val Ser Asp Thr Arg
   705                 710                 715                 720
   Asn His Arg Ile Gln Leu Phe Gly Pro Asp Gly Val Phe Leu Asn Lys
                   725                 730                 735
   Tyr Gly Phe Glu Gly Ala Leu Trp Lys His Phe Asp Ser Pro Arg Gly
               740                 745                 750
   Val Ala Phe Asn His Glu Gly His Leu Val Val Thr Asp Phe Asn Asn
           755                 760                 765
   His Arg Leu Leu Val Ile His Pro Asp Cys Gln Ser Ala Arg Phe Leu
       770                 775                 780
   Gly Ser Glu Gly Thr Gly Asn Gly Gln Phe Leu Arg Pro Gln Gly Val
   785                 790                 795                 800
   Ala Val Asp Gln Glu Gly Arg Ile Ile Val Ala Asp Ser Arg Asn His
                   805                 810                 815
   Arg Val Gln Met Phe Glu Ser Asn Gly Ser Phe Leu Cys Lys Phe Gly
               820                 825                 830
   Ala Gln Gly Ser Gly Phe Gly Gln Met Asp Arg Pro Ser Gly Ile Ala
           835                 840                 845
   Ile Thr Pro Asp Gly Met Ile Val Val Val Asp Phe Gly Asn Asn Arg
       850                 855                 860
   Ile Leu Val Phe
   865
<210> 73
   <211> 3477
   <212> PRT
   <213> homo sapiens

<400> 73
   Met Ala Asn Arg Arg Val Gly Arg Gly Cys Trp Glu Val Ser Pro Thr
   1               5                   10                  15
   Glu Arg Arg Pro Pro Ala Gly Leu Arg Gly Pro Ala Ala Glu Glu Glu
               20                  25                  30
   Ala Ser Ser Pro Pro Val Leu Ser Leu Ser His Phe Cys Arg Ser Pro
           35                  40                  45
   Phe Leu Cys Phe Gly Asp Val Leu Leu Gly Ala Ser Arg Thr Leu Ser
       50                  55                  60
   Leu Ala Leu Asp Asn Pro Asn Glu Glu Val Ala Glu Val Lys Ile Ser
   65                  70                  75                  80
   His Phe Pro Ala Ala Asp Leu Gly Phe Ser Val Ser Gln Arg Cys Phe
                   85                  90                  95
   Val Leu Gln Pro Lys Glu Lys Ile Val Ile Ser Val Asn Trp Thr Pro
               100                 105                 110
   Leu Lys Glu Gly Arg Val Arg Glu Ile Met Thr Phe Leu Val Asn Asp
           115                 120                 125
   Val Leu Lys His Gln Ala Ile Leu Leu Gly Asn Ala Glu Glu Gln Lys
       130                 135                 140
   Lys Lys Lys Arg Ser Leu Trp Asp Thr Ile Lys Lys Lys Lys Ile Ser
   145                 150                 155                 160
   Ala Ser Thr Ser His Asn Arg Arg Val Ser Asn Ile Gln Asn Val Asn
                   165                 170                 175
   Lys Thr Phe Ser Val Ser Gln Lys Val Asp Arg Val Arg Ser Pro Leu
               180                 185                 190
   Gln Ala Cys Glu Asn Leu Ala Met Asn Glu Gly Gly Pro Pro Thr Glu
           195                 200                 205
   Asn Asn Ser Leu Ile Leu Glu Glu Asn Lys Ile Pro Ile Ser Pro Ile
       210                 215                 220
   Ser Pro Ala Phe Asn Glu Cys His Gly Ala Thr Cys Leu Pro Leu Ser
   225                 230                 235                 240
   Val Arg Arg Ser Thr Thr Tyr Ser Ser Leu His Ala Ser Glu Asn Arg
                   245                 250                 255
   Glu Leu Leu Asn Val His Ser Ala Asn Val Ser Lys Val Ser Phe Asn
               260                 265                 270
   Glu Lys Ala Val Thr Glu Thr Ser Phe Asn Ser Val Asn Val Asn Gly
               275                 280                 285
   Gln Arg Gly Glu Asn Ser Lys Leu Ser Leu Thr Pro Asn Cys Ser Ser
       290                 295                 300
   Thr Leu Asn Ile Thr Gln Ser Gln Ile His Phe Leu Ser Pro Asp Ser
   305                 310                 315                 320
   Phe Val Asn Asn Ser His Gly Ala Asn Asn Glu Leu Glu Leu Val Thr
                   325                 330                 335
   Cys Leu Ser Ser Asp Met Phe Met Lys Asp Asn Ser Gln Pro Val His
               340                 345                 350
   Leu Glu Ser Thr Ile Ala His Glu Ile Tyr Gln Lys Ile Leu Ser Pro
           355                 360                 365
   Asp Ser Phe Ile Lys Asp Asn Tyr Gly Leu Asn Gln Asp Leu Glu Ser
       370                 375                 380
   Glu Ser Val Asn Pro Ile Leu Ser Pro Asn Gln Phe Leu Lys Asp Asn
   385                 390                 395                 400
   Met Ala Tyr Met Cys Thr Ser Gln Gln Thr Cys Lys Val Pro Leu Ser
                   405                 410                 415
   Asn Glu Asn Ser Gln Val Pro Gln Ser Pro Glu Asp Trp Arg Lys Ser
               420                 425                 430
   Glu Val Ser Pro Arg Ile Pro Glu Cys Gln Gly Ser Lys Ser Pro Lys
           435                 440                 445
   Ala Ile Phe Glu Glu Leu Val Glu Met Lys Ser Asn Tyr Tyr Ser Phe
       450                 455                 460
   Ile Lys Gln Asn Asn Pro Lys Phe Ser Ala Val Gln Asp Ile Ser Ser
   465                 470                 475                 480
   His Ser His Asn Lys Gln Pro Lys Arg Arg Pro Ile Leu Ser Ala Thr
                   485                 490                 495
   Val Thr Lys Arg Lys Ala Thr Cys Thr Arg Glu Asn Gln Thr Glu Ile
               500                 505                 510
   Asn Lys Pro Lys Ala Lys Arg Cys Leu Asn Ser Ala Val Gly Glu His
           515                 520                 525
   Glu Lys Val Ile Asn Asn Gln Lys Glu Lys Glu Asp Phe His Ser Tyr
       530                 535                 540
   Leu Pro Ile Ile Asp Pro Ile Leu Ser Lys Ser Lys Ser Tyr Lys Asn
   545                 550                 555                 560
   Glu Val Thr Pro Ser Ser Thr Thr Ala Ser Val Ala Arg Lys Arg Lys
                   565                 570                 575
   Ser Asp Gly Ser Met Glu Asp Ala Asn Val Arg Val Ala Ile Thr Glu
               580                 585                 590
   His Thr Glu Val Arg Glu Ile Lys Arg Ile His Phe Ser Pro Ser Glu
           595                 600                 605
   Pro Lys Thr Ser Ala Val Lys Lys Thr Lys Asn Val Thr Thr Pro Ile
       610                 615                 620
   Ser Lys Arg Ile Ser Asn Arg Glu Lys Leu Asn Leu Lys Lys Lys Thr
   625                 630                 635                 640
   Asp Leu Ser Ile Phe Arg Thr Pro Ile Ser Lys Thr Asn Lys Arg Thr
                   645                 650                 655
   Lys Pro Ile Ile Ala Val Ala Gln Ser Ser Leu Thr Phe Ile Lys Pro
               660                 665                 670
   Leu Lys Thr Asp Ile Pro Arg His Pro Met Pro Phe Ala Ala Lys Asn
           675                 680                 685
   Met Phe Tyr Asp Glu Arg Trp Lys Glu Lys Gln Glu Gln Gly Phe Thr
       690                 695                 700
   Trp Trp Leu Asn Phe Ile Leu Thr Pro Asp Asp Phe Thr Val Lys Thr
   705                 710                 715                 720
   Asn Ile Ser Glu Val Asn Ala Ala Thr Leu Leu Leu Gly Ile Glu Asn
                   725                 730                 735
   Gln His Lys Ile Ser Val Pro Arg Ala Pro Thr Lys Glu Glu Met Ser
               740                 745                 750
   Leu Arg Ala Tyr Thr Ala Arg Cys Arg Leu Asn Arg Leu Arg Arg Ala
           755                 760                 765
   Ala Cys Arg Leu Phe Thr Ser Glu Lys Met Val Lys Ala Ile Lys Lys
       770                 775                 780
   Leu Glu Ile Glu Ile Glu Ala Arg Arg Leu Ile Val Arg Lys Asp Arg
   785 790 795 800
   His Leu Trp Lys Asp Val Gly Glu Arg Gln Lys Val Leu Asn Trp Leu
   805 810 815
   Leu Ser Tyr Asn Pro Leu Trp Leu Arg Ile Gly Leu Glu Thr Thr Tyr
   820 825 830
   Gly Glu Leu Ile Ser Leu Glu Asp Asn Ser Asp Val Thr Gly Leu Ala
   835 840 845
   Met Phe Ile Leu Asn Arg Leu Leu Trp Asn Pro Asp Ile Ala Ala Glu
   850 855 860
   Tyr Arg His Pro Thr Val Pro His Leu Tyr Arg Asp Gly His Glu Glu
   865 870 875 880
   Ala Leu Ser Lys Phe Thr Leu Lys Lys Leu Leu Leu Leu Val Cys Phe
   885 890 895
   Leu Asp Tyr Ala Lys Ile Ser Arg Leu Ile Asp His Asp Pro Cys Leu
   900 905 910
   Phe Cys Lys Asp Ala Glu Phe Lys Ala Ser Lys Glu Ile Leu Leu Ala
   915 920 925
   Phe Ser Arg Asp Phe Leu Ser Gly Glu Gly Asp Leu Ser Arg His Leu
   930 935 940
   Gly Leu Leu Gly Leu Pro Val Asn His Val Gln Thr Pro Phe Asp Glu
   945 950 955 960
   Phe Asp Phe Ala Val Thr Asn Leu Ala Val Asp Leu Gln Cys Gly Val
   965 970 975
   Arg Leu Val Arg Thr Met Glu Leu Leu Thr Gln Asn Trp Asp Leu Ser
   980 985 990
   Lys Lys Leu Arg Ile Pro Ala Ile Ser Arg Leu Gln Lys Met His Asn
   995 1000 1005
   Val Asp Ile Val Leu Gln Val Leu Lys Ser Arg Gly Ile Glu Leu
   1010 1015 1020
   Ser Asp Glu His Gly Asn Thr Ile Leu Ser Lys Asp Ile Val Asp
   1025 1030 1035
   Arg His Arg Glu Lys Thr Leu Arg Leu Leu Trp Lys Ile Ala Phe
   1040 1045 1050
   Ala Phe Gln Val Asp Ile Ser Leu Asn Leu Asp Gln Leu Lys Glu
   1055 1060 1065
   Glu Ile Ala Phe Leu Lys His Thr Lys Ser Ile Lys Lys Thr Ile
   1070 1075 1080
   Ser Leu Leu Ser Cys His Ser Asp Asp Leu Ile Asn Lys Lys Lys
   1085 1090 1095
   Gly Lys Arg Asp Ser Gly Ser Phe Glu Gln Tyr Ser Glu Asn Ile
   1100 1105 1110
   Lys Leu Leu Met Asp Trp Val Asn Ala Val Cys Ala Phe Tyr Asn
   1115 1120 1125
   Lys Lys Val Glu Asn Phe Thr Val Ser Phe Ser Asp Gly Arg Val
   1130 1135 1140
   Leu Cys Tyr Leu Ile His His Tyr His Pro Cys Tyr Val Pro Phe
   1145 1150 1155
   Asp Ala Ile Cys Gln Arg Thr Thr Gln Thr Val Glu Cys Thr Gln
   1160 1165 1170
   Thr Gly Ser Val Val Leu Asn Ser Ser Ser Glu Ser Asp Asp Ser
   1175 1180 1185
   Ser Leu Asp Met Ser Leu Lys Ala Phe Asp His Glu Asn Thr Ser
   1190 1195 1200
   Glu Leu Tyr Lys Glu Leu Leu Glu Asn Glu Lys Lys Asn Phe His
   1205 1210 1215
   Leu Val Arg Ser Ala Val Arg Asp Leu Gly Gly Ile Pro Ala Met
   1220 1225 1230
   Ile Asn His Ser Asp Met Ser Asn Thr Ile Pro Asp Glu Lys Val
   1235 1240 1245
   Val Ile Thr Tyr Leu Ser Phe Leu Cys Ala Arg Leu Leu Asp Leu
   1250 1255 1260
   Arg Lys Glu Ile Arg Ala Ala Arg Leu Ile Gln Thr Thr Trp Arg
           1265                1270                1275
   Lys Tyr  Lys Leu Lys Thr Asp  Leu Lys Arg His Gln  Glu Arg Glu
       1280                1285                1290
   Lys Ala  Ala Arg Ile Ile Gln  Leu Ala Val Ile Asn  Phe Leu Ala
       1295                1300                1305
   Lys Gln  Arg Leu Arg Lys Arg  Val Asn Ala Ala Leu  Val Ile Gln
       1310                1315                1320
   Lys Tyr  Trp Arg Arg Val Leu  Ala Gln Arg Lys Leu  Leu Met Leu
       1325                1330                1335
   Lys Lys  Glu Lys Leu Glu Lys  Val Gln Asn Lys Ala  Ala Ser Leu
       1340                1345                1350
   Ile Gln  Gly Tyr Trp Arg Arg  Tyr Ser Thr Arg Gln  Arg Phe Leu
       1355                1360                1365
   Lys Leu  Lys Tyr Tyr Ser Ile  Ile Leu Gln Ser Arg  Ile Arg Met
       1370                1375                1380
   Ile Ile  Ala Val Thr Ser Tyr  Lys Arg Tyr Leu Trp  Ala Thr Val
       1385                1390                1395
   Thr Ile  Gln Arg His Trp Arg  Ala Tyr Leu Arg Arg  Lys Gln Asp
       1400                1405                1410
   Gln Gln  Arg Tyr Glu Met Leu  Lys Ser Ser Thr Leu  Ile Ile Gln
       1415                1420                1425
   Ser Met  Phe Arg Lys Trp Lys  Gln Arg Lys Met Gln  Ser Gln Val
       1430                1435                1440
   Lys Ala  Thr Val Ile Leu Gln  Arg Ala Phe Arg Glu  Trp His Leu
       1445                1450                1455
   Arg Lys  Gln Ala Lys Glu Glu  Asn Ser Ala Ile Ile  Ile Gln Ser
       1460                1465                1470
   Trp Tyr  Arg Met His Lys Glu  Leu Arg Lys Tyr Ile  Tyr Ile Arg
       1475                1480                1485
   Ser Cys  Val Val Ile Ile Gln  Lys Arg Phe Arg Cys  Phe Gln Ala
       1490                1495                1500
   Gln Lys  Leu Tyr Lys Arg Arg  Lys Glu Ser Ile Leu  Thr Ile Gln
       1505                1510                1515
   Lys Tyr  Tyr Lys Ala Tyr Leu  Lys Gly Lys Ile Glu  Arg Thr Asn
       1520                1525                1530
   Tyr Leu  Gln Lys Arg Ala Ala  Ala Ile Gln Leu Gln  Ala Ala Phe
       1535                1540                1545
   Arg Arg  Leu Lys Ala His Asn  Leu Cys Arg Gln Ile  Arg Ala Ala
       1550                1555                1560
   Cys Val  Ile Gln Ser Tyr Trp  Arg Met Arg Gln Asp  Arg Val Arg
       1565                1570                1575
   Phe Leu  Asn Leu Lys Lys Thr  Ile Ile Lys Phe Gln  Ala His Val
       1580                1585                1590
   Arg Lys  His Gln Gln Arg Gln  Lys Tyr Lys Lys Met  Lys Lys Ala
       1595                1600                1605
   Ala Val  Ile Ile Gln Thr His  Phe Arg Ala Tyr Ile  Phe Ala Met
       1610                1615                1620
   Lys Val  Leu Ala Ser Tyr Gln  Lys Thr Arg Ser Ala  Val Ile Val
       1625                1630                1635
   Leu Gln  Ser Ala Tyr Arg Gly  Met Gln Ala Arg Lys  Met Tyr Ile
       1640                1645                1650
   His Ile  Leu Thr Ser Val Ile  Lys Ile Gln Ser Tyr  Tyr Arg Ala
       1655                1660                1665
   Tyr Val  Ser Lys Lys Glu Phe  Leu Ser Leu Lys Asn  Ala Thr Ile
       1670                1675                1680
   Lys Leu  Gln Ser Thr Val Lys  Met Lys Gln Thr Arg  Lys Gln Tyr
       1685                1690                1695
   Leu His  Leu Arg Ala Ala Ala  Leu Phe Ile Gln Gln  Cys Tyr Arg
       1700                1705                1710
   Ser Lys  Lys Ile Ala Ala Gln  Lys Arg Glu Glu Tyr  Met Gln Met
       1715                1720                1725
   Arg Glu  Ser Cys Ile Lys Leu  Gln Ala Phe Val Arg  Gly Tyr Leu
       1730                1735                1740
   Val Arg Lys Gln Met Arg Leu Gln Arg Lys Ala Val Ile Ser Leu
   1745 1750 1755
   Gln Ser Tyr Phe Arg Met Arg Lys Ala Arg Gln Tyr Tyr Leu Lys
   1760 1765 1770
   Met Tyr Lys Ala Ile Ile Val Ile Gln Asn Tyr Tyr His Ala Tyr
   1775 1780 1785
   Lys Ala Gln Val Asn Gln Arg Lys Asn Phe Leu Gln Val Lys Lys
   1790 1795 1800
   Ala Ala Thr Cys Leu Gln Ala Ala Tyr Arg Gly Tyr Lys Val Arg
   1805 1810 1815
   Gln Leu Ile Lys Gln Gln Ser Ile Ala Ala Leu Lys Ile Gln Ser
   1820 1825 1830
   Ala Phe Arg Gly Tyr Asn Lys Arg Val Lys Tyr Gln Ser Val Leu
   1835 1840 1845
   Gln Ser Ile Ile Lys Ile Gln Arg Trp Tyr Arg Ala Tyr Lys Thr
   1850 1855 1860
   Leu His Asp Thr Arg Thr His Phe Leu Lys Thr Lys Ala Ala Val
   1865 1870 1875
   Ile Ser Leu Gln Ser Ala Tyr Arg Gly Trp Lys Val Arg Lys Gln
   1880 1885 1890
   Ile Arg Arg Glu His Gln Ala Ala Leu Lys Ile Gln Ser Ala Phe
   1895 1900 1905
   Arg Met Ala Lys Ala Gln Lys Gln Phe Arg Leu Phe Lys Thr Ala
   1910 1915 1920
   Ala Leu Val Ile Gln Gln Asn Phe Arg Ala Trp Thr Ala Gly Arg
   1925 1930 1935
   Lys Gln Cys Met Glu Tyr Ile Glu Leu Arg His Ala Val Leu Val
   1940 1945 1950
   Leu Gln Ser Met Trp Lys Gly Lys Thr Leu Arg Arg Gln Leu Gln
   1955 1960 1965
   Arg Gln His Lys Cys Ala Ile Ile Ile Gln Ser Tyr Tyr Arg Met
   1970 1975 1980
   His Val Gln Gln Lys Lys Trp Lys Ile Met Lys Lys Ala Ala Leu
   1985 1990 1995
   Leu Ile Gln Lys Tyr Tyr Arg Ala Tyr Ser Ile Gly Arg Glu Gln
   2000 2005 2010
   Asn His Leu Tyr Leu Lys Thr Lys Ala Ala Val Val Thr Leu Gln
   2015 2020 2025
   Ser Ala Tyr Arg Gly Met Lys Val Arg Lys Arg Ile Lys Asp Cys
   2030 2035 2040
   Asn Lys Ala Ala Val Thr Ile Gln Ser Lys Tyr Arg Ala Tyr Lys
   2045 2050 2055
   Thr Lys Lys Lys Tyr Ala Thr Tyr Arg Ala Ser Ala Ile Ile Ile
   2060 2065 2070
   Gln Arg Trp Tyr Arg Gly Ile Lys Ile Thr Asn His Gln His Lys
   2075 2080 2085
   Glu Tyr Leu Asn Leu Lys Lys Thr Ala Ile Lys Ile Gln Ser Val
   2090 2095 2100
   Tyr Arg Gly Ile Arg Val Arg Arg His Ile Gln His Met His Arg
   2105 2110 2115
   Ala Ala Thr Phe Ile Lys Ala Met Phe Lys Met His Gln Ser Arg
   2120 2125 2130
   Ile Ser Tyr His Thr Met Arg Lys Ala Ala Ile Val Ile Gln Val
   2135 2140 2145
   Arg Cys Arg Ala Tyr Tyr Gln Gly Lys Met Gln Arg Glu Lys Tyr
   2150 2155 2160
   Leu Thr Ile Leu Lys Ala Val Lys Val Leu Gln Ala Ser Phe Arg
   2165 2170 2175
   Gly Val Arg Val Arg Arg Thr Leu Arg Lys Met Gln Thr Ala Ala
   2180 2185 2190
   Thr Leu Ile Gln Ser Asn Tyr Arg Arg Tyr Arg Gln Gln Thr Tyr
   2195 2200 2205
   Phe Asn Lys Leu Lys Lys Ile Thr Lys Thr Val Gln Gln Arg Tyr
       2210                2215                2220
   Trp Ala  Met Lys Glu Arg Asn  Ile Gln Phe Gln Arg  Tyr Asn Lys
       2225                2230                2235
   Leu Arg  His Ser Val Ile Tyr  Ile Gln Ala Ile Phe  Arg Gly Lys
       2240                2245                2250
   Lys Ala  Arg Arg His Leu Lys  Met Met His Ile Ala  Ala Thr Leu
       2255                2260                2265
   Ile Gln  Arg Arg Phe Arg Thr  Leu Met Met Arg Arg  Arg Phe Leu
       2270                2275                2280
   Ser Leu  Lys Lys Thr Ala Ile  Leu Ile Gln Arg Lys  Tyr Arg Ala
       2285                2290                2295
   His Leu  Cys Thr Lys His His  Leu Gln Phe Leu Gln  Val Gln Asn
       2300                2305                2310
   Ala Val  Ile Lys Ile Gln Ser  Ser Tyr Arg Arg Trp  Met Ile Arg
       2315                2320                2325
   Lys Arg  Met Arg Glu Met His  Arg Ala Ala Thr Phe  Ile Gln Ser
       2330                2335                2340
   Thr Phe  Arg Met His Arg Leu  His Met Arg Tyr Arg  Ala Leu Lys
       2345                2350                2355
   Gln Ala  Ser Val Val Ile Gln  Gln Gln Tyr Gln Ala  Asn Arg Ala
       2360                2365                2370
   Ala Lys  Leu Gln Arg Gln His  Tyr Leu Arg Gln Arg  His Ser Ala
       2375                2380                2385
   Val Ile  Leu Gln Ala Ala Phe  Arg Gly Met Lys Thr  Arg Arg His
       2390                2395                2400
   Leu Lys  Ser Met His Ser Ser  Ala Thr Leu Ile Gln  Ser Arg Phe
       2405                2410                2415
   Arg Ser  Leu Leu Val Arg Arg  Arg Phe Ile Ser Leu  Lys Lys Ala
       2420                2425                2430
   Thr Ile  Phe Val Gln Arg Lys  Tyr Arg Ala Thr Ile  Cys Ala Lys
       2435                2440                2445
   His Lys  Leu Tyr Gln Phe Leu  His Leu Arg Lys Ala  Ala Ile Thr
       2450                2455                2460
   Ile Gln  Ser Ser Tyr Arg Arg  Leu Met Val Lys Lys  Lys Leu Gln
       2465                2470                2475
   Glu Met  Gln Arg Ala Ala Val  Leu Ile Gln Ala Thr  Phe Arg Met
       2480                2485                2490
   His Arg  Thr Tyr Ile Thr Phe  Gln Thr Trp Lys His  Ala Ser Ile
       2495                2500                2505
   Leu Ile  Gln Gln His Tyr Arg  Thr Tyr Arg Ala Ala  Lys Leu Gln
       2510                2515                2520
   Arg Glu  Asn Tyr Ile Arg Gln  Trp His Ser Ala Val  Val Ile Gln
       2525                2530                2535
   Ala Ala  Tyr Lys Gly Met Lys  Ala Arg Gln Leu Leu  Arg Glu Lys
       2540                2545                2550
   His Lys  Ala Ser Ile Val Ile  Gln Ser Thr Tyr Arg  Met Tyr Arg
       2555                2560                2565
   Gln Tyr  Cys Phe Tyr Gln Lys  Leu Gln Trp Ala Thr  Lys Ile Ile
       2570                2575                2580
   Gln Glu  Lys Tyr Arg Ala Asn  Lys Lys Lys Gln Lys  Val Phe Gln
       2585                2590                2595
   His Asn  Glu Leu Lys Lys Glu  Thr Cys Val Gln Ala  Gly Phe Gln
       2600                2605                2610
   Asp Met  Asn Ile Lys Lys Gln  Ile Gln Glu Gln His  Gln Ala Ala
       2615                2620                2625
   Ile Ile  Ile Gln Lys His Cys  Lys Ala Phe Lys Ile  Arg Lys His
       2630                2635                2640
   Tyr Leu  His Leu Arg Ala Thr  Val Val Ser Ile Gln  Arg Arg Tyr
       2645                2650                2655
   Arg Lys  Leu Thr Ala Val Arg  Thr Gln Ala Val Ile  Cys Ile Gln
       2660                2665                2670
   Ser Tyr  Tyr Arg Gly Phe Lys  Val Arg Lys Asp Ile  Gln Asn Met
       2675                2680                2685
   His Arg Ala Ala Thr Leu Ile Gln Ser Phe Tyr Arg Met His Arg
       2690                2695                2700
   Ala Lys Val Asp Tyr Glu Thr Lys Lys Thr Ala Ile Val Val Ile
       2705                2710                2715
   Gln Asn Tyr Tyr Arg Leu Tyr Val Arg Val Lys Thr Glu Arg Lys
       2720                2725                2730
   Asn Phe Leu Ala Val Gln Lys Ser Val Arg Thr Ile Gln Ala Ala
       2735                2740                2745
   Phe Arg Gly Met Lys Val Arg Gln Lys Leu Lys Asn Val Ser Glu
       2750                2755                2760
   Glu Lys Met Ala Ala Ile Val Asn Gln Ser Ala Leu Cys Cys Tyr
       2765                2770                2775
   Arg Ser Lys Thr Gln Tyr Glu Ala Val Gln Ser Glu Gly Val Met
       2780                2785                2790
   Ile Gln Glu Trp Tyr Lys Ala Ser Gly Leu Ala Cys Ser Gln Glu
       2795                2800                2805
   Ala Glu Tyr His Ser Gln Ser Arg Ala Ala Val Thr Ile Gln Lys
       2810                2815                2820
   Ala Phe Cys Arg Met Val Thr Arg Lys Leu Glu Thr Gln Lys Cys
       2825                2830                2835
   Ala Ala Leu Arg Ile Gln Phe Phe Leu Gln Met Ala Val Tyr Arg
       2840                2845                2850
   Arg Arg Phe Val Gln Gln Lys Arg Ala Ala Ile Thr Leu Gln His
       2855                2860                2865
   Tyr Phe Arg Thr Trp Gln Thr Arg Lys Gln Phe Leu Leu Tyr Arg
       2870                2875                2880
   Lys Ala Ala Val Val Leu Gln Asn His Tyr Arg Ala Phe Leu Ser
       2885                2890                2895
   Ala Lys His Gln Arg Gln Val Tyr Leu Gln Ile Arg Ser Ser Val
       2900                2905                2910
   Ile Ile Ile Gln Ala Arg Ser Lys Gly Phe Ile Gln Lys Arg Lys
       2915                2920                2925
   Phe Gln Glu Ile Lys Asn Ser Thr Ile Lys Ile Gln Ala Met Trp
       2930                2935                2940
   Arg Arg Tyr Arg Ala Lys Lys Tyr Leu Cys Lys Val Lys Ala Ala
       2945                2950                2955
   Cys Lys Ile Gln Ala Trp Tyr Arg Cys Trp Arg Ala His Lys Glu
       2960                2965                2970
   Tyr Leu Ala Ile Leu Lys Ala Val Lys Ile Ile Gln Gly Cys Phe
       2975                2980                2985
   Tyr Thr Lys Leu Glu Arg Thr Arg Phe Leu Asn Val Arg Ala Ser
       2990                2995                3000
   Ala Ile Ile Ile Gln Arg Lys Trp Arg Ala Ile Leu Pro Ala Lys
       3005                3010                3015
   Ile Ala His Glu His Phe Leu Met Ile Lys Arg His Arg Ala Ala
       3020                3025                3030
   Cys Leu Ile Gln Ala His Tyr Arg Gly Tyr Lys Gly Arg Gln Val
       3035                3040                3045
   Phe Leu Arg Gln Lys Ser Ala Ala Leu Ile Ile Gln Lys Tyr Ile
       3050                3055                3060
   Arg Ala Arg Glu Ala Gly Lys His Glu Arg Ile Lys Tyr Ile Glu
       3065                3070                3075
   Phe Lys Lys Ser Thr Val Ile Leu Gln Ala Leu Val Arg Gly Trp
       3080                3085                3090
   Leu Val Arg Lys Arg Phe Leu Glu Gln Arg Ala Lys Ile Arg Leu
       3095                3100                3105
   Leu His Phe Thr Ala Ala Ala Tyr Tyr His Leu Asn Ala Val Arg
       3110                3115                3120
   Ile Gln Arg Ala Tyr Lys Leu Tyr Leu Ala Val Lys Asn Ala Asn
       3125                3130                3135
   Lys Gln Val Asn Ser Val Ile Cys Ile Gln Arg Trp Phe Arg Ala
       3140                3145                3150
   Arg Leu Gln Glu Lys Arg Phe Ile Gln Lys Tyr His Ser Ile Lys
   3155 3160 3165
   Lys Ile Glu His Glu Gly Gln Glu Cys Leu Ser Gln Arg Asn Arg
   3170 3175 3180
   Ala Ala Ser Val Ile Gln Lys Ala Val Arg His Phe Leu Leu Arg
   3185 3190 3195
   Lys Lys Gln Glu Lys Phe Thr Ser Gly Ile Ile Lys Ile Gln Ala
   3200 3205 3210
   Leu Trp Arg Gly Tyr Ser Trp Arg Lys Lys Asn Asp Cys Thr Lys
   3215 3220 3225
   Ile Lys Ala Ile Arg Leu Ser Leu Gln Val Val Asn Arg Glu Ile
   3230 3235 3240
   Arg Glu Glu Asn Lys Leu Tyr Lys Arg Thr Ala Leu Ala Leu His
   3245 3250 3255
   Tyr Leu Leu Thr Tyr Lys His Leu Ser Ala Ile Leu Glu Ala Leu
   3260 3265 3270
   Lys His Leu Glu Val Val Thr Arg Leu Ser Pro Leu Cys Cys Glu
   3275 3280 3285
   Asn Met Ala Gln Ser Gly Ala Ile Ser Lys Ile Phe Val Leu Ile
   3290 3295 3300
   Arg Ser Cys Asn Arg Ser Ile Pro Cys Met Glu Val Ile Arg Tyr
   3305 3310 3315
   Ala Val Gln Val Leu Leu Asn Val Ser Lys Tyr Glu Lys Thr Thr
   3320 3325 3330
   Ser Ala Val Tyr Asp Val Glu Asn Cys Ile Asp Ile Leu Leu Glu
   3335 3340 3345
   Leu Leu Gln Ile Tyr Arg Glu Lys Pro Gly Asn Lys Val Ala Asp
   3350 3355 3360
   Lys Gly Gly Ser Ile Phe Thr Lys Thr Cys Cys Leu Leu Ala Ile
   3365 3370 3375
   Leu Leu Lys Thr Thr Asn Arg Ala Ser Asp Val Arg Ser Arg Ser
   3380 3385 3390
   Lys Val Val Asp Arg Ile Tyr Ser Leu Tyr Lys Leu Thr Ala His
   3395 3400 3405
   Lys His Lys Met Asn Thr Glu Arg Ile Leu Tyr Lys Gln Lys Lys
   3410 3415 3420
   Asn Ser Ser Ile Ser Ile Pro Phe Ile Pro Glu Thr Pro Val Arg
   3425 3430 3435
   Thr Arg Ile Val Ser Arg Leu Lys Pro Asp Trp Val Leu Arg Arg
   3440 3445 3450
   Asp Asn Met Glu Glu Ile Thr Asn Pro Leu Gln Ala Ile Gln Met
   3455 3460 3465
   Val Met Asp Thr Leu Gly Ile Pro Tyr
   3470 3475
<210> 74
   <211> 398
   <212> PRT
   <213> homo sapiens
<400> 74
   Met Ala Ala Val Ala Val Leu Arg Asn Asp Ser Leu Gln Ala Phe Leu
   1               5                   10                  15
   Gln Asp Arg Thr Pro Ser Ala Ser Pro Asp Leu Gly Lys His Ser Pro
               20                  25                  30
   Leu Ala Leu Leu Ala Ala Thr Cys Ser Arg Ile Gly Gln Pro Gly Ala
           35                  40                  45
   Ala Ala Pro Pro Asp Phe Leu Gln Val Pro Tyr Asp Pro Ala Leu Gly
       50                  55                  60
   Ser Pro Ser Arg Leu Phe His Pro Trp Thr Ala Asp Met Pro Ala His
   65                  70                  75                  80
   Ser Pro Gly Ala Leu Pro Pro Pro His Pro Ser Leu Gly Leu Thr Pro
                   85                  90                  95
   Gln Lys Thr His Leu Gln Pro Ser Phe Gly Ala Ala His Glu Leu Pro
               100                 105                 110
   Leu Thr Pro Pro Ala Asp Pro Ser Tyr Pro Tyr Glu Phe Ser Pro Val
           115                 120                 125
   Lys Met Leu Pro Ser Ser Met Ala Ala Leu Pro Ala Ser Cys Ala Pro
       130                 135                 140
   Ala Tyr Val Pro Tyr Ala Ala Gln Ala Ala Leu Pro Pro Gly Tyr Ser
   145                 150                 155                 160
   Asn Leu Leu Pro Pro Pro Pro Pro Pro Pro Pro Pro Pro Thr Cys Arg
                   165                 170                 175
   Gln Leu Ser Pro Asn Pro Ala Pro Asp Asp Leu Pro Trp Trp Ser Ile
               180                 185                 190
   Pro Gln Ala Gly Ala Gly Pro Gly Ala Ser Gly Val Pro Gly Ser Gly
           195                 200                 205
   Leu Ser Gly Ala Cys Ala Gly Ala Pro His Ala Pro Arg Phe Pro Ala
       210                 215                 220
   Ser Ala Ala Ala Ala Ala Ala Ala Ala Ala Ala Leu Gln Arg Gly Leu
   225                 230                 235                 240
   Val Leu Gly Pro Ser Asp Phe Ala Gln Tyr Gln Ser Gln Ile Ala Ala
                   245                 250                 255
   Leu Leu Gln Thr Lys Ala Pro Leu Ala Ala Thr Ala Arg Arg Cys Arg
               260                 265                 270
   Arg Cys Arg Cys Pro Asn Cys Gln Ala Ala Gly Gly Ala Pro Glu Ala
           275                 280                 285
   Glu Pro Gly Lys Lys Lys Gln His Val Cys His Val Pro Gly Cys Gly
       290                 295                 300
   Lys Val Tyr Gly Lys Thr Ser His Leu Lys Ala His Leu Arg Trp His
   305                 310                 315                 320
   Thr Gly Glu Arg Pro Phe Val Cys Asn Trp Leu Phe Cys Gly Lys Ser
                   325                 330                 335
   Phe Thr Arg Ser Asp Glu Leu Gln Arg His Leu Arg Thr His Thr Gly
               340                 345                 350
   Glu Lys Arg Phe Ala Cys Pro Glu Cys Gly Lys Arg Phe Met Arg Ser
           355                 360                 365
   Asp His Leu Ala Lys His Val Lys Thr His Gln Asn Lys Lys Leu Lys
       370                 375                 380
   Val Ala Glu Ala Gly Val Lys Arg Glu Asp Ala Arg Asp Leu
   385                 390                 395
<210> 75
   <211> 514
   <212> PRT
   <213> homo sapiens
<400> 75
   Met Gly Lys Asp Phe Arg Tyr Tyr Phe Gln His Pro Trp Ser Arg Met
   1               5                   10                  15
   Ile Val Ala Tyr Leu Val Ile Phe Phe Asn Phe Leu Ile Phe Ala Glu
               20                  25                  30
   Asp Pro Val Ser His Ser Gln Thr Glu Ala Asn Val Ile Val Val Gly
           35                  40                  45
   Asn Cys Phe Ser Phe Val Thr Asn Lys Tyr Pro Arg Gly Val Gly Trp
       50                  55                  60
   Arg Ile Leu Lys Val Leu Leu Trp Leu Leu Ala Ile Leu Thr Gly Leu
   65                  70                  75                  80
   Ile Ala Gly Lys Phe Leu Phe His Gln Arg Leu Phe Gly Gln Leu Leu
                   85                  90                  95
   Arg Leu Lys Met Phe Arg Glu Asp His Gly Ser Trp Met Thr Met Phe
               100                 105                 110
   Phe Ser Thr Ile Leu Phe Leu Phe Ile Phe Ser His Ile Tyr Asn Thr
           115                 120                 125
   Ile Leu Leu Met Asp Gly Asn Met Gly Ala Tyr Ile Ile Thr Asp Tyr
       130                 135                 140
   Met Gly Ile Arg Asn Glu Ser Phe Met Lys Leu Ala Ala Val Gly Thr
   145                 150                 155                 160
   Trp Met Gly Asp Phe Val Thr Ala Trp Met Val Thr Asp Met Met Leu
                   165                 170                 175
   Gln Asp Lys Pro Tyr Pro Asp Trp Gly Lys Ser Ala Arg Ala Phe Trp
                   180                 185                 190
   Lys Lys Gly Asn Val Arg Ile Thr Leu Phe Trp Thr Val Leu Phe Thr
           195                 200                 205
   Leu Thr Ser Val Val Val Leu Val Ile Thr Thr Asp Trp Ile Ser Trp
       210                 215                 220
   Asp Lys Leu Asn Arg Gly Phe Leu Pro Ser Asp Glu Val Ser Arg Ala
   225                 230                 235                 240
   Phe Leu Ala Ser Phe Ile Leu Val Phe Asp Leu Leu Ile Val Met Gln
                   245                 250                 255
   Asp Trp Glu Phe Pro His Phe Met Gly Asp Val Asp Val Asn Leu Pro
               260                 265                 270
   Gly Leu His Thr Pro His Met Gln Phe Lys Ile Pro Phe Phe Gln Lys
           275                 280                 285
   Ile Phe Lys Glu Glu Tyr Arg Ile His Ile Thr Gly Lys Trp Phe Asn
       290                 295                 300
   Tyr Gly Ile Ile Phe Leu Val Leu Ile Leu Asp Leu Asn Met Trp Lys
   305                 310                 315                 320
   Asn Gln Ile Phe Tyr Lys Pro His Glu Tyr Gly Gln Tyr Ile Gly Pro
                   325                 330                 335
   Gly Gln Lys Ile Tyr Thr Val Lys Asp Ser Glu Ser Leu Lys Asp Leu
               340                 345                 350
   Asn Arg Thr Lys Leu Ser Trp Glu Trp Arg Ser Asn His Thr Asn Pro
           355                 360                 365
   Arg Thr Asn Lys Thr Tyr Val Glu Gly Asp Met Phe Leu His Ser Arg
       370                 375                 380
   Phe Ile Gly Ala Ser Leu Asp Val Lys Cys Leu Ala Phe Val Pro Ser
   385                 390                 395                 400
   Leu Ile Ala Phe Val Trp Phe Gly Phe Phe Ile Trp Phe Phe Gly Arg
                   405                 410                 415
   Phe Leu Lys Asn Glu Pro Arg Met Glu Asn Gln Asp Lys Thr Tyr Thr
               420                 425                 430
   Arg Met Lys Arg Lys Ser Pro Ser Glu His Ser Lys Asp Met Gly Ile
           435                 440                 445
   Thr Arg Glu Asn Thr Gln Ala Ser Val Glu Asp Pro Leu Asn Asp Pro
       450                 455                 460
   Ser Leu Val Cys Ile Arg Ser Asp Phe Asn Glu Ile Val Tyr Lys Ser
   465                 470                 475                 480
   Ser His Leu Thr Ser Glu Asn Leu Ser Ser Gln Leu Asn Glu Ser Thr
                   485                 490                 495
   Ser Ala Thr Glu Ala Asp Gln Asp Pro Thr Thr Ser Lys Ser Thr Pro
               500                 505                 510
   Thr Asn
<210> 76
   <211> 316
   <212> PRT
   <213> homo sapiens
<400> 76
<210> 77
   <211> 6307
   <212> PRT
   <213> homo sapiens
<400> 77
   Met Ser Val Phe Leu Gly Pro Gly Met Pro Ser Ala Ser Leu Leu Val
   1               5                   10                  15
   Asn Leu Leu Ser Ala Leu Leu Ile Leu Phe Val Phe Gly Glu Thr Glu
               20                  25                  30
   Ile Arg Phe Thr Gly Gln Thr Glu Phe Val Val Asn Glu Thr Ser Thr
           35                  40                  45
   Thr Val Ile Arg Leu Ile Ile Glu Arg Ile Gly Glu Pro Ala Asn Val
       50                  55                  60
   Thr Ala Ile Val Ser Leu Tyr Gly Glu Asp Ala Gly Asp Phe Phe Asp
   65                  70                  75                  80
   Thr Tyr Ala Ala Ala Phe Ile Pro Ala Gly Glu Thr Asn Arg Thr Val
                   85                  90                  95
   Tyr Ile Ala Val Cys Asp Asp Asp Leu Pro Gly Pro Asp Glu Thr Phe
               100                 105                 110
   Ile Phe His Leu Thr Leu Gln Lys Pro Ser Ala Asn Val Lys Leu Gly
           115                 120                 125
   Trp Pro Arg Thr Val Thr Val Thr Ile Leu Ser Asn Asp Asn Ala Phe
       130                 135                 140
   Gly Ile Ile Ser Phe Asn Met Leu Pro Ser Ile Ala Val Ser Glu Pro
   145                 150                 155                 160
   Lys Gly Arg Asn Glu Ser Met Pro Leu Thr Leu Ile Arg Glu Lys Gly
                   165                 170                 175
   Thr Tyr Gly Met Val Met Val Thr Phe Glu Val Glu Gly Gly Pro Asn
               180                 185                 190
   Pro Pro Asp Glu Asp Leu Ser Pro Val Lys Gly Asn Ile Thr Phe Pro
           195                 200                 205
   Pro Gly Arg Ala Thr Val Ile Tyr Asn Leu Ala Val Leu Asp Asp Glu
       210                 215                 220
   Val Pro Glu Asn Asp Glu Ile Phe Leu Ile Gln Leu Lys Ser Val Glu
   225                 230                 235                 240
   Gly Gly Ala Glu Ile Asn Thr Ser Arg Asn Ser Ile Glu Ile Ile Ile
                   245                 250                 255
   Glu Lys Asn Asp Ser Pro Val Arg Phe Leu Gln Ser Ile Tyr Leu Val
               260                 265                 270
   Pro Glu Glu Asp His Ile Leu Ile Ile Pro Val Val Arg Gly Lys Asp
           275                 280                 285
   Asn Asn Gly Asn Leu Ile Gly Ser Asp Glu Tyr Glu Val Ser Ile Ser
       290                 295                 300
   Tyr Ala Val Thr Thr Gly Asn Ser Thr Ala His Ala Gln Gln Asn Leu
   305                 310                 315                 320
   Asp Phe Ile Asp Leu Gln Pro Asn Thr Thr Val Val Phe Pro Pro Phe
                   325                 330                 335
   Ile His Glu Ser His Leu Lys Phe Gln Ile Val Asp Asp Thr Ile Pro
               340                 345                 350
   Glu Ile Ala Glu Ser Phe His Ile Met Leu Leu Lys Asp Thr Leu Gln
           355                 360                 365
   Gly Asp Ala Val Leu Ile Ser Pro Ser Val Val Gln Val Thr Ile Lys
       370                 375                 380
   Pro Asn Asp Lys Pro Tyr Gly Val Leu Ser Phe Asn Ser Val Leu Phe
   385                 390                 395                 400
   Glu Arg Thr Val Ile Ile Asp Glu Asp Arg Ile Ser Arg Tyr Glu Glu
                   405                 410                 415
   Ile Thr Val Val Arg Asn Gly Gly Thr His Gly Asn Val Ser Ala Asn
               420                 425                 430
   Trp Val Val Thr Pro Asn Ser Thr Asp Pro Ser Pro Val Thr Ala Asp
           435                 440                 445
   Ile Arg Pro Ser Ser Gly Val Leu His Phe Ala Gln Gly Gln Met Leu
       450                 455                 460
   Ala Thr Ile Pro Leu Thr Gly Gly Asp Asp Asp Leu Pro Glu Glu Ala
   465                 470                 475                 480
   Glu Ala Tyr Leu Leu Gln Ile Leu Pro His Thr Ile Arg Gly Gly Ala
                   485                 490                 495
   Glu Val Ser Glu Pro Ala Glu Leu Leu Phe Tyr Ile Gln Asp Ser Asp
               500                 505                 510
   Asp Val Tyr Gly Leu Ile Thr Phe Phe Pro Met Glu Asn Gln Lys Ile
           515                 520                 525
   Glu Ser Ser Pro Gly Gly Arg Tyr Leu Ser Leu Ser Phe Thr Arg Leu
       530                 535                 540
   Gly Gly Thr Lys Gly Asp Val Arg Leu Leu Tyr Ser Val Leu Tyr Ile
   545                 550                 555                 560
   Pro Ala Gly Ala Val Asp Pro Leu Gln Ala Lys Glu Gly Ile Leu Asn
                   565                 570                 575
   Ile Ser Arg Arg Asn Asp Leu Ile Phe Pro Glu Gln Lys Thr Gln Val
               580                 585                 590
   Thr Thr Lys Leu Pro Ile Arg Asn Asp Ala Phe Leu Gln Asn Gly Ala
           595                 600                 605
   His Phe Leu Val Gln Leu Glu Thr Val Glu Leu Leu Asn Ile Ile Pro
       610                 615                 620
   Leu Ile Pro Pro Ile Ser Pro Arg Phe Gly Glu Ile Cys Asn Ile Ser
   625                 630                 635                 640
   Leu Leu Val Thr Pro Ala Ile Ala Asn Gly Glu Ile Gly Phe Leu Ser
                   645                 650                 655
   Asn Leu Pro Ile Ile Leu His Glu Pro Glu Asp Phe Ala Ala Glu Val
               660                 665                 670
   Val Tyr Ile Pro Leu His Arg Asp Gly Thr Asp Gly Gln Ala Thr Val
           675                 680                 685
   Tyr Trp Ser Leu Lys Pro Ser Gly Phe Asn Ser Lys Ala Val Thr Pro
       690                 695                 700
   Asp Asp Ile Gly Pro Phe Asn Gly Ser Val Leu Phe Leu Ser Gly Gln
   705                 710                 715                 720
   Ser Asp Thr Thr Ile Asn Ile Thr Ile Lys Gly Asp Asp Ile Pro Glu
                   725                 730                 735
   Met Asn Glu Thr Val Thr Leu Ser Leu Asp Trp Val Asn Val Glu Asn
               740                 745                 750
   Gln Val Leu Lys Ser Gly Tyr Thr Ser Arg Asp Leu Ile Ile Leu Glu
           755                 760                 765
   Asn Asp Asp Pro Gly Gly Val Phe Glu Phe Ser Pro Ala Ser Arg Gly
   770 775 780
   Pro Tyr Val Ile Lys Glu Gly Glu Ser Val Glu Leu His Ile Ile Arg
   785 790 795 800
   Ser Arg Gly Ser Leu Val Lys Gln Phe Leu His Tyr Arg Val Glu Pro
   805 810 815
   Arg Asp Ser Asn Glu Phe Tyr Gly Asn Thr Gly Val Leu Glu Phe Lys
   820 825 830
   Pro Gly Glu Arg Glu Ile Val Ile Thr Leu Leu Ala Arg Leu Asp Gly
   835 840 845
   Ile Pro Glu Leu Asp Glu His Tyr Trp Val Val Leu Ser Ser His Gly
   850 855 860
   Glu Arg Glu Ser Lys Leu Gly Ser Ala Thr Ile Val Asn Ile Thr Ile
   865 870 875 880
   Leu Lys Asn Asp Asp Pro His Gly Ile Ile Glu Phe Val Ser Asp Gly
   885 890 895
   Leu Ile Val Met Ile Asn Glu Ser Lys Gly Asp Ala Ile Tyr Ser Ala
   900 905 910
   Val Tyr Asp Val Val Arg Asn Arg Gly Asn Phe Gly Asp Val Ser Val
   915 920 925
   Ser Trp Val Val Ser Pro Asp Phe Thr Gln Asp Val Phe Pro Val Gln
   930 935 940
   Gly Thr Val Val Phe Gly Asp Gln Glu Phe Ser Lys Asn Ile Thr Ile
   945 950 955 960
   Tyr Ser Leu Pro Asp Glu Ile Pro Glu Glu Met Glu Glu Phe Thr Val
   965 970 975
   Ile Leu Leu Asn Gly Thr Gly Gly Ala Lys Val Gly Asn Arg Thr Thr
   980 985 990
   Ala Thr Leu Arg Ile Arg Arg Asn Asp Asp Pro Ile Tyr Phe Ala Glu
   995 1000 1005
   Pro Arg Val Val Arg Val Gln Glu Gly Glu Thr Ala Asn Phe Thr
   1010 1015 1020
   Val Leu Arg Asn Gly Ser Val Asp Val Thr Cys Met Val Gln Tyr
   1025 1030 1035
   Ala Thr Lys Asp Gly Lys Ala Thr Ala Arg Glu Arg Asp Phe Ile
   1040 1045 1050
   Pro Val Glu Lys Gly Glu Thr Leu Ile Phe Glu Val Gly Ser Arg
   1055 1060 1065
   Gln Gln Ser Ile Ser Ile Phe Val Asn Glu Asp Gly Ile Pro Glu
   1070 1075 1080
   Thr Asp Glu Pro Phe Tyr Ile Ile Leu Leu Asn Ser Pro Gly Asp
   1085 1090 1095
   Pro Val Val Tyr Gln Tyr Gly Val Ala Thr Val Ile Ile Glu Ala
   1100 1105 1110
   Asn Asp Asp Pro Asn Gly Ile Phe Ser Leu Glu Pro Ile Asp Lys
   1115 1120 1125
   Ala Val Glu Glu Gly Lys Thr Asn Ala Phe Trp Ile Leu Arg His
   1130 1135 1140
   Arg Gly Tyr Phe Gly Ser Val Ser Val Ser Trp Gln Leu Phe Gln
   1145 1150 1155
   Asn Asp Ser Ala Leu Gln Pro Gly Gln Glu Phe Tyr Glu Thr Ser
   1160 1165 1170
   Gly Thr Val Asn Phe Met Asp Gly Glu Glu Ala Lys Pro Ile Ile
   1175 1180 1185
   Leu His Ala Phe Pro Asp Lys Ile Pro Glu Phe Asn Glu Phe Tyr
   1190 1195 1200
   Phe Leu Lys Leu Val Asn Ile Ser Gly Gly Ser Pro Gly Pro Gly
   1205 1210 1215
   Gly Gln Leu Ala Glu Thr Asn Leu Gln Val Thr Val Met Ala Pro
   1220 1225 1230
   Phe Asn Asp Asp Pro Phe Gly Val Phe Ile Leu Asp Pro Glu Cys
   1235 1240 1245
   Leu Glu Arg Glu Val Ala Glu Asp Val Leu Ser Glu Asp Asp Met
       1250                1255                1260
   Ser Tyr  Ile Thr Asn Phe Thr  Ile Leu Arg Gln Gln  Gly Val Phe
       1265                1270                1275
   Gly Asp  Val Gln Leu Gly Trp  Glu Ile Leu Ser Ser  Glu Phe Pro
       1280                1285                1290
   Ala Gly  Leu Pro Pro Met Ile  Asp Phe Leu Leu Val  Gly Ile Phe
       1295                1300                1305
   Pro Thr  Thr Val His Leu Gln  Gln His Met Arg Gly  His His Ser
       1310                1315                1320
   Gly Thr  Asp Ala Leu Tyr Phe  Thr Gly Leu Glu Gly  Ala Phe Gly
       1325                1330                1335
   Thr Gly  Asn Ser Lys Tyr His  Pro Phe Arg Asn Asn  Thr Ile Ala
       1340                1345                1350
   Asn Phe  Thr Phe Ser Ala Trp  Val Met Pro Asn Ala  Asn Thr Asn
       1355                1360                1365
   Gly Phe  Ile Ile Ala Lys Asp  Asp Gly Asn Gly Ser  Ile Tyr Tyr
       1370                1375                1380
   Gly Val  Lys Ile Gln Thr Asn  Glu Ser His Val Thr  Leu Ser Leu
       1385                1390                1395
   His Tyr  Lys Thr Leu Gly Ser  Asn Ala Thr Tyr Ile  Ala Lys Thr
       1400                1405                1410
   Thr Val  Met Lys Tyr Leu Glu  Glu Ser Val Trp Leu  His Leu Leu
       1415                1420                1425
   Ile Ile  Leu Glu Asp Gly Ile  Ile Glu Phe Tyr Leu  Asp Gly Asn
       1430                1435                1440
   Ala Met  Pro Arg Gly Ile Lys  Ser Leu Lys Gly Glu  Ala Ile Thr
       1445                1450                1455
   Asp Gly  Pro Gly Ile Leu Arg  Ile Gly Ala Gly Ile  Asn Gly Asn
       1460                1465                1470
   Asp Arg  Phe Thr Gly Leu Met  Gln Asp Val Arg Ser  Tyr Glu Arg
       1475                1480                1485
   Lys Leu  Thr Leu Glu Glu Ile  Tyr Glu Leu His Ala  Met Pro Ala
       1490                1495                1500
   Lys Ser  Asp Leu His Pro Ile  Ser Gly Tyr Leu Glu  Phe Arg Gln
       1505                1510                1515
   Gly Glu  Thr Asn Lys Ser Phe  Ile Ile Ser Ala Arg  Asp Asp Asn
       1520                1525                1530
   Asp Glu  Glu Gly Glu Glu Leu  Phe Ile Leu Lys Leu  Val Ser Val
       1535                1540                1545
   Tyr Gly  Gly Ala Arg Ile Ser  Glu Glu Asn Thr Thr  Ala Arg Leu
       1550                1555                1560
   Thr Ile  Gln Lys Ser Asp Asn  Ala Asn Gly Leu Phe  Gly Phe Thr
       1565                1570                1575
   Gly Ala  Cys Ile Pro Glu Ile  Ala Glu Glu Gly Ser  Thr Ile Ser
       1580                1585                1590
   Cys Val  Val Glu Arg Thr Arg  Gly Ala Leu Asp Tyr  Val His Val
       1595                1600                1605
   Phe Tyr  Thr Ile Ser Gln Ile  Glu Thr Asp Gly Ile  Asn Tyr Leu
       1610                1615                1620
   Val Asp  Asp Phe Ala Asn Ala  Ser Gly Thr Ile Thr  Phe Leu Pro
       1625                1630                1635
   Trp Gln  Arg Ser Glu Val Leu  Asn Ile Tyr Val Leu  Asp Asp Asp
       1640                1645                1650
   Ile Pro  Glu Leu Asn Glu Tyr  Phe Arg Val Thr Leu  Val Ser Ala
       1655                1660                1665
   Ile Pro  Gly Asp Gly Lys Leu  Gly Ser Thr Pro Thr  Ser Gly Ala
       1670                1675                1680
   Ser Ile  Asp Pro Glu Lys Glu  Thr Thr Asp Ile Thr  Ile Lys Ala
       1685                1690                1695
   Ser Asp  His Pro Tyr Gly Leu  Leu Gln Phe Ser Thr  Gly Leu Pro
       1700                1705                1710
   Pro Gln  Pro Lys Asp Ala Met  Thr Leu Pro Ala Ser  Ser Val Pro
       1715                1720                1725
   His Ile Thr Val Glu Glu Glu Asp Gly Glu Ile Arg Leu Leu Val
   1730 1735 1740
   Ile Arg Ala Gln Gly Leu Leu Gly Arg Val Thr Ala Glu Phe Arg
   1745 1750 1755
   Thr Val Ser Leu Thr Ala Phe Ser Pro Glu Asp Tyr Gln Asn Val
   1760 1765 1770
   Ala Gly Thr Leu Glu Phe Gln Pro Gly Glu Arg Tyr Lys Tyr Ile
   1775 1780 1785
   Phe Ile Asn Ile Thr Asp Asn Ser Ile Pro Glu Leu Glu Lys Ser
   1790 1795 1800
   Phe Lys Val Glu Leu Leu Asn Leu Glu Gly Gly Val Ala Glu Leu
   1805 1810 1815
   Phe Arg Val Asp Gly Ser Gly Ser Gly Asp Gly Asp Met Glu Phe
   1820 1825 1830
   Phe Leu Pro Thr Ile His Lys Arg Ala Ser Leu Gly Val Ala Ser
   1835 1840 1845
   Gln Ile Leu Val Thr Ile Ala Ala Ser Asp His Ala His Gly Val
   1850 1855 1860
   Phe Glu Phe Ser Pro Glu Ser Leu Phe Val Ser Gly Thr Glu Pro
   1865 1870 1875
   Glu Asp Gly Tyr Ser Thr Val Thr Leu Asn Val Ile Arg His His
   1880 1885 1890
   Gly Thr Leu Ser Pro Val Thr Leu His Trp Asn Ile Asp Ser Asp
   1895 1900 1905
   Pro Asp Gly Asp Leu Ala Phe Thr Ser Gly Asn Ile Thr Phe Glu
   1910 1915 1920
   Ile Gly Gln Thr Ser Ala Asn Ile Thr Val Glu Ile Leu Pro Asp
   1925 1930 1935
   Glu Asp Pro Glu Leu Asp Lys Ala Phe Ser Val Ser Val Leu Ser
   1940 1945 1950
   Val Ser Ser Gly Ser Leu Gly Ala His Ile Asn Ala Thr Leu Thr
   1955 1960 1965
   Val Leu Ala Ser Asp Asp Pro Tyr Gly Ile Phe Ile Phe Ser Glu
   1970 1975 1980
   Lys Asn Arg Pro Val Lys Val Glu Glu Ala Thr Gln Asn Ile Thr
   1985 1990 1995
   Leu Ser Ile Ile Arg Leu Lys Gly Leu Met Gly Lys Val Leu Val
   2000 2005 2010
   Ser Tyr Ala Thr Leu Asp Asp Met Glu Lys Pro Pro Tyr Phe Pro
   2015 2020 2025
   Pro Asn Leu Ala Arg Ala Thr Gln Gly Arg Asp Tyr Ile Pro Ala
   2030 2035 2040
   Ser Gly Phe Ala Leu Phe Gly Ala Asn Gln Ser Glu Ala Thr Ile
   2045 2050 2055
   Ala Ile Ser Ile Leu Asp Asp Asp Glu Pro Glu Arg Ser Glu Ser
   2060 2065 2070
   Val Phe Ile Glu Leu Leu Asn Ser Thr Leu Val Ala Lys Val Gln
   2075 2080 2085
   Ser Arg Ser Ile Pro Asn Ser Pro Arg Leu Gly Pro Lys Val Glu
   2090 2095 2100
   Thr Ile Ala Gln Leu Ile Ile Ile Ala Asn Asp Asp Ala Phe Gly
   2105 2110 2115
   Thr Leu Gln Leu Ser Ala Pro Ile Val Arg Val Ala Glu Asn His
   2120 2125 2130
   Val Gly Pro Ile Ile Asn Val Thr Arg Thr Gly Gly Ala Phe Ala
   2135 2140 2145
   Asp Val Ser Val Lys Phe Lys Ala Val Pro Ile Thr Ala Ile Ala
   2150 2155 2160
   Gly Glu Asp Tyr Ser Ile Ala Ser Ser Asp Val Val Leu Leu Glu
   2165 2170 2175
   Gly Glu Thr Ser Lys Ala Val Pro Ile Tyr Val Ile Asn Asp Ile
   2180 2185 2190
   Tyr Pro Glu Leu Glu Glu Ser Phe Leu Val Gln Leu Met Asn Glu
   2195 2200 2205
   Thr Thr Gly Gly Ala Arg Leu Gly Ala Leu Thr Glu Ala Val Ile
   2210 2215 2220
   Ile Ile Glu Ala Ser Asp Asp Pro Tyr Gly Leu Phe Gly Phe Gln
   2225 2230 2235
   Ile Thr Lys Leu Ile Val Glu Glu Pro Glu Phe Asn Ser Val Lys
   2240 2245 2250
   Val Asn Leu Pro Ile Ile Arg Asn Ser Gly Thr Leu Gly Asn Val
   2255 2260 2265
   Thr Val Gln Trp Val Ala Thr Ile Asn Gly Gln Leu Ala Thr Gly
   2270 2275 2280
   Asp Leu Arg Val Val Ser Gly Asn Val Thr Phe Ala Pro Gly Glu
   2285 2290 2295
   Thr Ile Gln Thr Leu Leu Leu Glu Val Leu Ala Asp Asp Val Pro
   2300 2305 2310
   Glu Ile Glu Glu Val Ile Gln Val Gln Leu Thr Asp Ala Ser Gly
   2315 2320 2325
   Gly Gly Thr Ile Gly Leu Asp Arg Ile Ala Asn Ile Ile Ile Pro
   2330 2335 2340
   Ala Asn Asp Asp Pro Tyr Gly Thr Val Ala Phe Ala Gln Met Val
   2345 2350 2355
   Tyr Arg Val Gln Glu Pro Leu Glu Arg Ser Ser Cys Ala Asn Ile
   2360 2365 2370
   Thr Val Arg Arg Ser Gly Gly His Phe Gly Arg Leu Leu Leu Phe
   2375 2380 2385
   Tyr Ser Thr Ser Asp Ile Asp Val Val Ala Leu Ala Met Glu Glu
   2390 2395 2400
   Gly Gln Asp Leu Leu Ser Tyr Tyr Glu Ser Pro Ile Gln Gly Val
   2405 2410 2415
   Pro Asp Pro Leu Trp Arg Thr Trp Met Asn Val Ser Ala Val Gly
   2420 2425 2430
   Glu Pro Leu Tyr Thr Cys Ala Thr Leu Cys Leu Lys Glu Gln Ala
   2435 2440 2445
   Cys Ser Ala Phe Ser Phe Phe Ser Ala Ser Glu Gly Pro Gln Cys
   2450 2455 2460
   Phe Trp Met Thr Ser Trp Ile Ser Pro Ala Val Asn Asn Ser Asp
   2465 2470 2475
   Phe Trp Thr Tyr Arg Lys Asn Met Thr Arg Val Ala Ser Leu Phe
   2480 2485 2490
   Ser Gly Gln Ala Val Ala Gly Ser Asp Tyr Glu Pro Val Thr Arg
   2495 2500 2505
   Gln Trp Ala Ile Met Gln Glu Gly Asp Glu Phe Ala Asn Leu Thr
   2510 2515 2520
   Val Ser Ile Leu Pro Asp Asp Phe Pro Glu Met Asp Glu Ser Phe
   2525 2530 2535
   Leu Ile Ser Leu Leu Glu Val His Leu Met Asn Ile Ser Ala Ser
   2540 2545 2550
   Leu Lys Asn Gln Pro Thr Ile Gly Gln Pro Asn Ile Ser Thr Val
   2555 2560 2565
   Val Ile Ala Leu Asn Gly Asp Ala Phe Gly Val Phe Val Ile Tyr
   2570 2575 2580
   Asn Ile Ser Pro Asn Thr Ser Glu Asp Gly Leu Phe Val Glu Val
   2585 2590 2595
   Gln Glu Gln Pro Gln Thr Leu Val Glu Leu Met Ile His Arg Thr
   2600 2605 2610
   Gly Gly Ser Leu Gly Gln Val Ala Val Glu Trp Arg Val Val Gly
   2615 2620 2625
   Gly Thr Ala Thr Glu Gly Leu Asp Phe Ile Gly Ala Gly Glu Ile
   2630 2635 2640
   Leu Thr Phe Ala Glu Gly Glu Thr Lys Lys Thr Val Ile Leu Thr
   2645 2650 2655
   Ile Leu Asp Asp Ser Glu Pro Glu Asp Asp Glu Ser Ile Ile Val
   2660 2665 2670
   Ser Leu Val Tyr Thr Glu Gly Gly Ser Arg Ile Leu Pro Ser Ser
   2675 2680 2685
   Asp Thr Val Arg Val Asn Ile Leu Ala Asn Asp Asn Val Ala Gly
   2690 2695 2700
   Ile Val Ser Phe Gln Thr Ala Ser Arg Ser Val Ile Gly His Glu
   2705 2710 2715
   Gly Glu Ile Leu Gln Phe His Val Ile Arg Thr Phe Pro Gly Arg
   2720 2725 2730
   Gly Asn Val Thr Val Asn Trp Lys Ile Ile Gly Gln Asn Leu Glu
   2735 2740 2745
   Leu Asn Phe Ala Asn Phe Ser Gly Gln Leu Phe Phe Pro Glu Gly
   2750 2755 2760
   Ser Leu Asn Thr Thr Leu Phe Val His Leu Leu Asp Asp Asn Ile
   2765 2770 2775
   Pro Glu Glu Lys Glu Val Tyr Gln Val Ile Leu Tyr Asp Val Arg
   2780 2785 2790
   Thr Gln Gly Val Pro Pro Ala Gly Ile Ala Leu Leu Asp Ala Gln
   2795 2800 2805
   Gly Tyr Ala Ala Val Leu Thr Val Glu Ala Ser Asp Glu Pro His
   2810 2815 2820
   Gly Val Leu Asn Phe Ala Leu Ser Ser Arg Phe Val Leu Leu Gln
   2825 2830 2835
   Glu Ala Asn Ile Thr Ile Gln Leu Phe Ile Asn Arg Glu Phe Gly
   2840 2845 2850
   Ser Leu Gly Ala Ile Asn Val Thr Tyr Thr Thr Val Pro Gly Met
   2855 2860 2865
   Leu Ser Leu Lys Asn Gln Thr Val Gly Asn Leu Ala Glu Pro Glu
   2870 2875 2880
   Val Asp Phe Val Pro Ile Ile Gly Phe Leu Ile Leu Glu Glu Gly
   2885 2890 2895
   Glu Thr Ala Ala Ala Ile Asn Ile Thr Ile Leu Glu Asp Asp Val
   2900 2905 2910
   Pro Glu Leu Glu Glu Tyr Phe Leu Val Asn Leu Thr Tyr Val Gly
   2915 2920 2925
   Leu Thr Met Ala Ala Ser Thr Ser Phe Pro Pro Arg Leu Asp Ser
   2930 2935 2940
   Glu Gly Leu Thr Ala Gln Val Ile Ile Asp Ala Asn Asp Gly Ala
   2945 2950 2955
   Arg Gly Val Ile Glu Trp Gln Gln Ser Arg Phe Glu Val Asn Glu
   2960 2965 2970
   Thr His Gly Ser Leu Thr Leu Val Ala Gln Arg Ser Arg Glu Pro
   2975 2980 2985
   Leu Gly His Val Ser Leu Phe Val Tyr Ala Gln Asn Leu Glu Ala
   2990 2995 3000
   Gln Val Gly Leu Asp Tyr Ile Phe Thr Pro Met Ile Leu His Phe
   3005 3010 3015
   Ala Asp Gly Glu Arg Tyr Lys Asn Val Asn Ile Met Ile Leu Asp
   3020 3025 3030
   Asp Asp Ile Pro Glu Gly Asp Glu Lys Phe Gln Leu Ile Leu Thr
   3035 3040 3045
   Asn Pro Ser Pro Gly Leu Glu Leu Gly Lys Asn Thr Ile Ala Leu
   3050 3055 3060
   Ile Ile Val Leu Ala Asn Asp Asp Gly Pro Gly Val Leu Ser Phe
   3065 3070 3075
   Asn Asn Ser Glu His Phe Phe Leu Arg Glu Pro Thr Ala Leu Tyr
   3080 3085 3090
   Val Gln Glu Ser Val Ala Val Leu Tyr Ile Val Arg Glu Pro Ala
   3095 3100 3105
   Gln Gly Leu Phe Gly Thr Val Thr Val Gln Phe Ile Val Thr Glu
   3110 3115 3120
   Val Asn Ser Ser Asn Glu Ser Lys Asp Leu Thr Pro Ser Lys Gly
   3125 3130 3135
   Tyr Ile Val Leu Glu Glu Gly Val Arg Phe Lys Ala Leu Gln Ile
       3140                3145                3150
   Ser Ala  Ile Leu Asp Thr Glu  Pro Glu Met Asp Glu  Tyr Phe Val
       3155                3160                3165
   Cys Thr  Leu Phe Asn Pro Thr  Gly Gly Ala Arg Leu  Gly Val His
       3170                3175                3180
   Val Gln  Thr Leu Ile Thr Val  Leu Gln Asn Gln Ala  Pro Leu Gly
       3185                3190                3195
   Leu Phe  Ser Ile Ser Ala Val  Glu Asn Arg Ala Thr  Ser Ile Asp
       3200                3205                3210
   Ile Glu  Glu Ala Asn Arg Thr  Val Tyr Leu Asn Val  Ser Arg Thr
       3215                3220                3225
   Asn Asp  Ile Asp Leu Ala Val  Ser Val Gln Trp Glu  Thr Val Ser
       3230                3235                3240
   Glu Thr  Ala Phe Gly Met Arg  Gly Met Asp Val Val  Phe Ser Val
       3245                3250                3255
   Phe Gln  Ser Phe Leu Asp Glu  Ser Ala Ser Gly Trp  Cys Phe Phe
       3260                3265                3270
   Thr Leu  Glu Asn Leu Ile Tyr  Gly Ile Met Leu Arg  Lys Ser Ser
       3275                3280                3285
   Val Thr  Val Tyr Arg Trp Gln  Gly Ile Phe Ile Pro  Val Glu Asp
       3290                3295                3300
   Leu Asn  Ile Glu Asn Pro Lys  Thr Cys Glu Ala Phe  Asn Ile Gly
       3305                3310                3315
   Phe Ser  Pro Tyr Phe Val Ile  Thr His Glu Glu Arg  Asn Glu Glu
       3320                3325                3330
   Lys Pro  Ser Leu Asn Ser Val  Phe Thr Phe Thr Ser  Gly Phe Lys
       3335                3340                3345
   Leu Phe  Leu Val Gln Thr Ile  Ile Ile Leu Glu Ser  Ser Gln Val
       3350                3355                3360
   Arg Tyr  Phe Thr Ser Asp Ser  Gln Asp Tyr Leu Ile  Ile Ala Ser
       3365                3370                3375
   Gln Arg  Asp Asp Ser Glu Leu  Thr Gln Val Phe Arg  Trp Asn Gly
       3380                3385                3390
   Gly Ser  Phe Val Leu His Gln  Lys Leu Pro Val Arg  Gly Val Leu
       3395                3400                3405
   Thr Val  Ala Leu Phe Asn Lys  Gly Gly Ser Val Phe  Leu Ala Ile
       3410                3415                3420
   Ser Gln  Ala Asn Ala Arg Leu  Asn Ser Leu Leu Phe  Arg Trp Ser
       3425                3430                3435
   Gly Ser  Gly Phe Ile Asn Phe  Gln Glu Val Pro Val  Ser Gly Thr
       3440                3445                3450
   Thr Glu  Val Glu Ala Leu Ser  Ser Ala Asn Asp Ile  Tyr Leu Ile
       3455                3460                3465
   Phe Ala  Glu Asn Val Phe Leu  Gly Asp Gln Asn Ser  Ile Asp Ile
       3470                3475                3480
   Phe Ile  Trp Glu Met Gly Gln  Ser Ser Phe Arg Tyr  Phe Gln Ser
       3485                3490                3495
   Val Asp  Phe Ala Ala Val Asn  Arg Ile His Ser Phe  Thr Pro Ala
       3500                3505                3510
   Ser Gly  Ile Ala His Ile Leu  Leu Ile Gly Pro Arg  Tyr Val Tyr
       3515                3520                3525
   Ser Phe  Thr Ala Gly Asn Ser  Glu Arg Asn Gln Phe  Ser Phe Val
       3530                3535                3540
   Leu Glu  Val Pro Ser Ala Tyr  Asp Val Val Ser Val  Thr Val Lys
       3545                3550                3555
   Ser Leu  Asn Ser Ser Lys Asn  Leu Ile Ala Leu Val  Gly Ala His
       3560                3565                3570
   Ser His  Ile Tyr Glu Leu Ala  Tyr Ile Ser Ser His  Ser Asp Phe
       3575                3580                3585
   Ile Pro  Ser Ser Gly Glu Leu  Ile Phe Glu Pro Gly  Glu Arg Glu
       3590                3595                3600
   Ala Thr  Ile Ala Val Asn Ile  Leu Asp Asp Thr Val  Pro Glu Lys
       3605                3610                3615
   Glu Glu Ser Phe Lys Val Gln Leu Lys Asn Pro Lys Gly Gly Ala
       3620                3625                3630
   Glu Ile Gly Ile Asn Asp Ser Val Thr Ile Thr Ile Leu Ser Asn
       3635                3640                3645
   Asp Asp Ala Tyr Gly Ile Val Ala Phe Ala Gln Asn Ser Leu Tyr
       3650                3655                3660
   Lys Gln Val Glu Glu Met Glu Gln Asp Ser Leu Val Thr Leu Asn
       3665                3670                3675
   Val Glu Arg Leu Lys Gly Thr Tyr Gly Arg Ile Thr Ile Ala Trp
       3680                3685                3690
   Glu Ala Asp Gly Ser Ile Ser Asp Ile Phe Pro Thr Ser Gly Val
       3695                3700                3705
   Ile Leu Phe Thr Glu Gly Gln Val Leu Ser Thr Ile Thr Leu Thr
       3710                3715                3720
   Ile Leu Ala Asp Asn Ile Pro Glu Leu Ser Glu Val Val Ile Val
       3725                3730                3735
   Thr Leu Thr Arg Ile Thr Thr Glu Gly Val Glu Asp Ser Tyr Lys
       3740                3745                3750
   Gly Ala Thr Ile Asp Gln Asp Arg Ser Lys Ser Val Ile Thr Thr
       3755                3760                3765
   Leu Pro Asn Asp Ser Pro Phe Gly Leu Val Gly Trp Arg Ala Ala
       3770                3775                3780
   Ser Val Phe Ile Arg Val Ala Glu Pro Lys Glu Asn Thr Thr Thr
       3785                3790                3795
   Leu Gln Leu Gln Ile Ala Arg Asp Lys Gly Leu Leu Gly Asp Ile
       3800                3805                3810
   Ala Ile His Leu Arg Ala Gln Pro Asn Phe Leu Leu His Val Asp
       3815                3820                3825
   Asn Gln Ala Thr Glu Asn Glu Asp Tyr Val Leu Gln Glu Thr Ile
       3830                3835                3840
   Ile Ile Met Lys Glu Asn Ile Lys Glu Ala His Ala Glu Val Ser
       3845                3850                3855
   Ile Leu Pro Asp Asp Leu Pro Glu Leu Glu Glu Gly Phe Ile Val
       3860                3865                3870
   Thr Ile Thr Glu Val Asn Leu Val Asn Ser Asp Phe Ser Thr Gly
       3875                3880                3885
   Gln Pro Ser Val Arg Arg Pro Gly Met Glu Ile Ala Glu Ile Met
       3890                3895                3900
   Ile Glu Glu Asn Asp Asp Pro Arg Gly Ile Phe Met Phe His Val
       3905                3910                3915
   Thr Arg Gly Ala Gly Glu Val Ile Thr Ala Tyr Glu Val Pro Pro
       3920                3925                3930
   Pro Leu Asn Val Leu Gln Val Pro Val Val Arg Leu Ala Gly Ser
       3935                3940                3945
   Phe Gly Ala Val Asn Val Tyr Trp Lys Ala Ser Pro Asp Ser Ala
       3950                3955                3960
   Gly Leu Glu Asp Phe Lys Pro Ser His Gly Ile Leu Glu Phe Ala
       3965                3970                3975
   Asp Lys Gln Val Thr Ala Met Ile Glu Ile Thr Ile Ile Asp Asp
       3980                3985                3990
   Ala Glu Phe Glu Leu Thr Glu Thr Phe Asn Ile Ser Leu Ile Ser
       3995                4000                4005
   Val Ala Gly Gly Gly Arg Leu Gly Asp Asp Val Val Val Thr Val
       4010                4015                4020
   Val Ile Pro Gln Asn Asp Ser Pro Phe Gly Val Phe Gly Phe Glu
       4025                4030                4035
   Glu Lys Thr Val Met Ile Asp Glu Ser Leu Ser Ser Asp Asp Pro
       4040                4045                4050
   Asp Ser Tyr Val Thr Leu Thr Val Val Arg Ser Pro Gly Gly Lys
       4055                4060                4065
   Gly Thr Val Arg Leu Glu Trp Thr Ile Asp Glu Lys Ala Lys His
       4070                4075                4080
   Asn Leu Ser Pro Leu Asn Gly Thr Leu His Phe Asp Glu Thr Glu
   4085 4090 4095
   Ser Gln Lys Thr Ile Val Leu His Thr Leu Gln Asp Thr Val Leu
   4100 4105 4110
   Glu Glu Asp Arg Arg Phe Thr Ile Gln Leu Ile Ser Ile Asp Glu
   4115 4120 4125
   Val Glu Ile Ser Pro Val Lys Gly Ser Ala Ser Ile Ile Ile Arg
   4130 4135 4140
   Gly Asp Lys Arg Ala Ser Gly Glu Val Gly Ile Ala Pro Ser Ser
   4145 4150 4155
   Arg His Ile Leu Ile Gly Glu Pro Ser Ala Lys Tyr Asn Gly Thr
   4160 4165 4170
   Ala Ile Ile Ser Leu Val Arg Gly Pro Gly Ile Leu Gly Glu Val
   4175 4180 4185
   Thr Val Phe Trp Arg Ile Phe Pro Pro Ser Val Gly Glu Phe Ala
   4190 4195 4200
   Glu Thr Ser Gly Lys Leu Thr Met Arg Asp Glu Gln Ser Ala Val
   4205 4210 4215
   Ile Val Val Ile Gln Ala Leu Asn Asp Asp Ile Pro Glu Glu Lys
   4220 4225 4230
   Ser Phe Tyr Glu Phe Gln Leu Thr Ala Val Ser Gly Gly Gly Val
   4235 4240 4245
   Leu Ser Glu Ser Ser Ser Thr Ala Asn Ile Thr Val Val Ala Ser
   4250 4255 4260
   Asp Ser Pro Tyr Gly Arg Phe Ala Phe Ser His Glu Gln Leu Arg
   4265 4270 4275
   Val Ser Glu Ala Gln Arg Val Asn Ile Thr Ile Ile Arg Ser Ser
   4280 4285 4290
   Gly Asp Phe Gly His Val Arg Leu Trp Tyr Lys Thr Met Ser Gly
   4295 4300 4305
   Thr Ala Glu Ala Gly Leu Asp Phe Val Pro Ala Ala Gly Glu Leu
   4310 4315 4320
   Leu Phe Glu Ala Gly Glu Met Arg Lys Ser Leu His Val Glu Ile
   4325 4330 4335
   Leu Asp Asp Asp Tyr Pro Glu Gly Pro Glu Glu Phe Ser Leu Thr
   4340 4345 4350
   Ile Thr Lys Val Glu Leu Gln Gly Arg Gly Tyr Asp Phe Thr Ile
   4355 4360 4365
   Gln Glu Asn Gly Leu Gln Ile Asp Gln Pro Pro Glu Ile Gly Asn
   4370 4375 4380
   Ile Ser Ile Val Arg Ile Ile Ile Met Lys Asn Asp Asn Ala Glu
   4385 4390 4395
   Gly Ile Ile Glu Phe Asp Pro Lys Tyr Thr Ala Phe Glu Val Glu
   4400 4405 4410
   Glu Asp Val Gly Leu Ile Met Ile Pro Val Val Arg Leu His Gly
   4415 4420 4425
   Thr Tyr Gly Tyr Val Thr Ala Asp Phe Ile Ser Gln Ser Ser Ser
   4430 4435 4440
   Ala Ser Pro Gly Gly Val Asp Tyr Ile Leu His Gly Ser Thr Val
   4445 4450 4455
   Thr Phe Gln His Gly Gln Asn Leu Ser Phe Ile Asn Ile Ser Ile
   4460 4465 4470
   Ile Asp Asp Asn Glu Ser Glu Phe Glu Glu Pro Ile Glu Ile Leu
   4475 4480 4485
   Leu Thr Gly Ala Thr Gly Gly Ala Val Leu Gly Arg His Leu Val
   4490 4495 4500
   Ser Arg Ile Ile Ile Ala Lys Ser Asp Ser Pro Phe Gly Val Ile
   4505 4510 4515
   Arg Phe Leu Asn Gln Ser Lys Ile Ser Ile Ala Asn Pro Asn Ser
   4520 4525 4530
   Thr Met Ile Leu Ser Leu Val Leu Glu Arg Thr Gly Gly Leu Leu
   4535 4540 4545
   Gly Glu Ile Gln Val Asn Trp Glu Thr Val Gly Pro Asn Ser Gln
   4550 4555 4560
   Glu Ala Leu Leu Pro Gln Asn Arg Asp Ile Ala Asp Pro Val Ser
   4565 4570 4575
   Gly Leu Phe Tyr Phe Gly Glu Gly Glu Gly Gly Val Arg Thr Ile
   4580 4585 4590
   Ile Leu Thr Ile Tyr Pro His Glu Glu Ile Glu Val Glu Glu Thr
   4595 4600 4605
   Phe Ile Ile Lys Leu His Leu Val Lys Gly Glu Ala Lys Leu Asp
   4610 4615 4620
   Ser Arg Ala Lys Asp Val Thr Leu Thr Ile Gln Glu Phe Gly Asp
   4625 4630 4635
   Pro Asn Gly Val Val Gln Phe Ala Pro Glu Thr Leu Ser Lys Lys
   4640 4645 4650
   Thr Tyr Ser Glu Pro Leu Ala Leu Glu Gly Pro Leu Leu Ile Thr
   4655 4660 4665
   Phe Phe Val Arg Arg Val Lys Gly Thr Phe Gly Glu Ile Met Val
   4670 4675 4680
   Tyr Trp Glu Leu Ser Ser Glu Phe Asp Ile Thr Glu Asp Phe Leu
   4685 4690 4695
   Ser Thr Ser Gly Phe Phe Thr Ile Ala Asp Gly Glu Ser Glu Ala
   4700 4705 4710
   Ser Phe Asp Val His Leu Leu Pro Asp Glu Val Pro Glu Ile Glu
   4715 4720 4725
   Glu Asp Tyr Val Ile Gln Leu Val Ser Val Glu Gly Gly Ala Glu
   4730 4735 4740
   Leu Asp Leu Glu Lys Ser Ile Thr Trp Phe Ser Val Tyr Ala Asn
   4745 4750 4755
   Asp Asp Pro His Gly Val Phe Ala Leu Tyr Ser Asp Arg Gln Ser
   4760 4765 4770
   Ile Leu Ile Gly Gln Asn Leu Ile Arg Ser Ile Gln Ile Asn Ile
   4775 4780 4785
   Thr Arg Leu Ala Gly Thr Phe Gly Asp Val Ala Val Gly Leu Arg
   4790 4795 4800
   Ile Ser Ser Asp His Lys Glu Gln Pro Ile Val Thr Glu Asn Ala
   4805 4810 4815
   Glu Arg Gln Leu Val Val Lys Asp Gly Ala Thr Tyr Lys Val Asp
   4820 4825 4830
   Val Val Pro Ile Lys Asn Gln Val Phe Leu Ser Leu Gly Ser Asn
   4835 4840 4845
   Phe Thr Leu Gln Leu Val Thr Val Met Leu Val Gly Gly Arg Phe
   4850 4855 4860
   Tyr Gly Met Pro Thr Ile Leu Gln Glu Ala Lys Ser Ala Val Leu
   4865 4870 4875
   Pro Val Ser Glu Lys Ala Ala Asn Ser Gln Val Gly Phe Glu Ser
   4880 4885 4890
   Thr Ala Phe Gln Leu Met Asn Ile Thr Ala Gly Thr Ser His Val
   4895 4900 4905
   Met Ile Ser Arg Arg Gly Thr Tyr Gly Ala Leu Ser Val Ala Trp
   4910 4915 4920
   Thr Thr Gly Tyr Ala Pro Gly Leu Glu Ile Pro Glu Phe Ile Val
   4925 4930 4935
   Val Gly Asn Met Thr Pro Thr Leu Gly Ser Leu Ser Phe Ser His
   4940 4945 4950
   Gly Glu Gln Arg Lys Gly Val Phe Leu Trp Thr Phe Pro Ser Pro
   4955 4960 4965
   Gly Trp Pro Glu Ala Phe Val Leu His Leu Ser Gly Val Gln Ser
   4970 4975 4980
   Ser Ala Pro Gly Gly Ala Gln Leu Arg Ser Gly Phe Ile Val Ala
   4985 4990 4995
   Glu Ile Glu Pro Met Gly Val Phe Gln Phe Ser Thr Ser Ser Arg
   5000 5005 5010
   Asn Ile Ile Val Ser Glu Asp Thr Gln Met Ile Arg Leu His Val
   5015 5020 5025
   Gln Arg Leu Phe Gly Phe His Ser Asp Leu Ile Lys Val Ser Tyr
       5030                5035                5040
   Gln Thr  Thr Ala Gly Ser Ala  Lys Pro Leu Glu Asp  Phe Glu Pro
       5045                5050                5055
   Val Gln  Asn Gly Glu Leu Phe  Phe Gln Lys Phe Gln  Thr Glu Val
       5060                5065                5070
   Asp Phe  Glu Ile Thr Ile Ile  Asn Asp Gln Leu Ser  Glu Ile Glu
       5075                5080                5085
   Glu Phe  Phe Tyr Ile Asn Leu  Thr Ser Val Glu Ile  Arg Gly Leu
       5090                5095                5100
   Gln Lys  Phe Asp Val Asn Trp  Ser Pro Arg Leu Asn  Leu Asp Phe
       5105                5110                5115
   Ser Val  Ala Val Ile Thr Ile  Leu Asp Asn Asp Asp  Leu Ala Gly
       5120                5125                5130
   Met Asp  Ile Ser Phe Pro Glu  Thr Thr Val Ala Val  Ala Val Asp
       5135                5140                5145
   Thr Thr  Leu Ile Pro Val Glu  Thr Glu Ser Thr Thr  Tyr Leu Ser
       5150                5155                5160
   Thr Ser  Lys Thr Thr Thr Ile  Leu Gln Pro Thr Asn  Val Val Ala
       5165                5170                5175
   Ile Val  Thr Glu Ala Thr Gly  Val Ser Ala Ile Pro  Glu Lys Leu
       5180                5185                5190
   Val Thr  Leu His Gly Thr Pro  Ala Val Ser Glu Lys  Pro Asp Val
       5195                5200                5205
   Ala Thr  Val Thr Ala Asn Val  Ser Ile His Gly Thr  Phe Ser Leu
       5210                5215                5220
   Gly Pro  Ser Ile Val Tyr Ile  Glu Glu Glu Met Lys  Asn Gly Thr
       5225                5230                5235
   Phe Asn  Thr Ala Glu Val Leu  Ile Arg Arg Thr Gly  Gly Phe Thr
       5240                5245                5250
   Gly Asn  Val Ser Ile Thr Val  Lys Thr Phe Gly Glu  Arg Cys Ala
       5255                5260                5265
   Gln Met  Glu Pro Asn Ala Leu  Pro Phe Arg Gly Ile  Tyr Gly Ile
       5270                5275                5280
   Ser Asn  Leu Thr Trp Ala Val  Glu Glu Glu Asp Phe  Glu Glu Gln
       5285                5290                5295
   Thr Leu  Thr Leu Ile Phe Leu  Asp Gly Glu Arg Glu  Arg Lys Val
       5300                5305                5310
   Ser Val  Gln Ile Leu Asp Asp  Asp Glu Pro Glu Gly  Gln Glu Phe
       5315                5320                5325
   Phe Tyr  Val Phe Leu Thr Asn  Pro Gln Gly Gly Ala  Gln Ile Val
       5330                5335                5340
   Glu Gly  Lys Asp Asp Thr Gly  Phe Ala Ala Phe Ala  Met Val Ile
       5345                5350                5355
   Ile Thr  Gly Ser Asp Leu His  Asn Gly Ile Ile Gly  Phe Ser Glu
       5360                5365                5370
   Glu Ser  Gln Ser Gly Leu Glu  Leu Arg Glu Gly Ala  Val Met Arg
       5375                5380                5385
   Arg Leu  His Leu Ile Val Thr  Arg Gln Pro Asn Arg  Ala Phe Glu
       5390                5395                5400
   Asp Val  Lys Val Phe Trp Arg  Val Thr Leu Asn Lys  Thr Val Val
       5405                5410                5415
   Val Leu  Gln Lys Asp Gly Val  Asn Leu Met Glu Glu  Leu Gln Ser
       5420                5425                5430
   Val Ser  Gly Thr Thr Thr Cys  Thr Met Gly Gln Thr  Lys Cys Phe
       5435                5440                5445
   Ile Ser  Ile Glu Leu Lys Pro  Glu Lys Val Pro Gln  Val Glu Val
       5450                5455                5460
   Tyr Phe  Phe Val Glu Leu Tyr  Glu Ala Thr Ala Gly  Ala Ala Ile
       5465                5470                5475
   Asn Asn  Ser Ala Arg Phe Ala  Gln Ile Lys Ile Leu  Glu Ser Asp
       5480                5485                5490
   Glu Ser  Gln Ser Leu Val Tyr  Phe Ser Val Gly Ser  Arg Leu Ala
       5495                5500                5505
   Val Ala His Lys Lys Ala Thr Leu Ile Ser Leu Gln Val Ala Arg
   5510 5515 5520
   Asp Ser Gly Thr Gly Leu Met Met Ser Val Asn Phe Ser Thr Gln
   5525 5530 5535
   Glu Leu Arg Ser Ala Glu Thr Ile Gly Arg Thr Ile Ile Ser Pro
   5540 5545 5550
   Ala Ile Ser Gly Lys Asp Phe Val Ile Thr Glu Gly Thr Leu Val
   5555 5560 5565
   Phe Glu Pro Gly Gln Arg Ser Thr Val Leu Asp Val Ile Leu Thr
   5570 5575 5580
   Pro Glu Thr Gly Ser Leu Asn Ser Phe Pro Lys Arg Phe Gln Ile
   5585 5590 5595
   Val Leu Phe Asp Pro Lys Gly Gly Ala Arg Ile Asp Lys Val Tyr
   5600 5605 5610
   Gly Thr Ala Asn Ile Thr Leu Val Ser Asp Ala Asp Ser Gln Ala
   5615 5620 5625
   Ile Trp Gly Leu Ala Asp Gln Leu His Gln Pro Val Asn Asp Asp
   5630 5635 5640
   Ile Leu Asn Arg Val Leu His Thr Ile Ser Met Lys Val Ala Thr
   5645 5650 5655
   Glu Asn Thr Asp Glu Gln Leu Ser Ala Met Met His Leu Ile Glu
   5660 5665 5670
   Lys Ile Thr Thr Glu Gly Lys Ile Gln Ala Phe Ser Val Ala Ser
   5675 5680 5685
   Arg Thr Leu Phe Tyr Glu Ile Leu Cys Ser Leu Ile Asn Pro Lys
   5690 5695 5700
   Arg Lys Asp Thr Arg Gly Phe Ser His Phe Ala Glu Val Thr Glu
   5705 5710 5715
   Asn Phe Ala Phe Ser Leu Leu Thr Asn Val Thr Cys Gly Ser Pro
   5720 5725 5730
   Gly Glu Lys Ser Lys Thr Ile Leu Asp Ser Cys Pro Tyr Leu Ser
   5735 5740 5745
   Ile Leu Ala Leu His Trp Tyr Pro Gln Gln Ile Asn Gly His Lys
   5750 5755 5760
   Phe Glu Gly Lys Glu Gly Asp Tyr Ile Arg Ile Pro Glu Arg Leu
   5765 5770 5775
   Leu Asp Val Gln Asp Ala Glu Ile Met Ala Gly Lys Ser Thr Cys
   5780 5785 5790
   Lys Leu Val Gln Phe Thr Glu Tyr Ser Ser Gln Gln Trp Phe Ile
   5795 5800 5805
   Ser Gly Asn Asn Leu Pro Thr Leu Lys Asn Lys Val Leu Ser Leu
   5810 5815 5820
   Ser Val Lys Gly Gln Ser Ser Gln Leu Leu Thr Asn Asp Asn Glu
   5825 5830 5835
   Val Leu Tyr Arg Ile Tyr Ala Ala Glu Pro Arg Ile Ile Pro Gln
   5840 5845 5850
   Thr Ser Leu Cys Leu Leu Trp Asn Gln Ala Ala Ala Ser Trp Leu
   5855 5860 5865
   Ser Asp Ser Gln Phe Cys Lys Val Ile Glu Glu Thr Ala Asp Tyr
   5870 5875 5880
   Val Glu Cys Ala Cys Ser His Met Ser Val Tyr Ala Val Tyr Ala
   5885 5890 5895
   Arg Thr Asp Asn Leu Ser Ser Tyr Asn Glu Ala Phe Phe Thr Ser
   5900 5905 5910
   Gly Phe Ile Cys Ile Ser Gly Leu Cys Leu Ala Val Leu Ser His
   5915 5920 5925
   Ile Phe Cys Ala Arg Tyr Ser Met Phe Ala Ala Lys Leu Leu Thr
   5930 5935 5940
   His Met Met Ala Ala Ser Leu Gly Thr Gln Ile Leu Phe Leu Ala
   5945 5950 5955
   Ser Ala Tyr Ala Ser Pro Gln Leu Ala Glu Glu Ser Cys Ser Ala
   5960 5965 5970
   Met Ala Ala Val Thr His Tyr Leu Tyr Leu Cys Gln Phe Ser Trp
   5975                5980                5985
   Met Leu  Ile Gln Ser Val Asn  Phe Trp Tyr Val Leu  Val Met Asn
       5990                5995                6000
   Asp Glu  His Thr Glu Arg Arg  Tyr Leu Leu Phe Phe  Leu Leu Ser
       6005                6010                6015
   Trp Gly  Leu Pro Ala Phe Val  Val Ile Leu Leu Ile  Val Ile Leu
       6020                6025                6030
   Lys Gly  Ile Tyr His Gln Ser  Met Ser Gln Ile Tyr  Gly Leu Ile
       6035                6040                6045
   His Gly  Asp Leu Cys Phe Ile  Pro Asn Val Tyr Ala  Ala Leu Phe
       6050                6055                6060
   Thr Ala  Ala Leu Val Pro Leu  Thr Cys Leu Val Val  Val Phe Val
       6065                6070                6075
   Val Phe  Ile His Ala Tyr Gln  Val Lys Pro Gln Trp  Lys Ala Tyr
       6080                6085                6090
   Asp Asp  Val Phe Arg Gly Arg  Thr Asn Ala Ala Glu  Ile Pro Leu
       6095                6100                6105
   Ile Leu  Tyr Leu Phe Ala Leu  Ile Ser Val Thr Trp  Leu Trp Gly
       6110                6115                6120
   Gly Leu  His Met Ala Tyr Arg  His Phe Trp Met Leu  Val Leu Phe
       6125                6130                6135
   Val Ile  Phe Asn Ser Leu Gln  Gly Leu Tyr Val Phe  Met Val Tyr
       6140                6145                6150
   Phe Ile  Leu His Asn Gln Met  Cys Cys Pro Met Lys  Ala Ser Tyr
       6155                6160                6165
   Thr Val  Glu Met Asn Gly His  Pro Gly Pro Ser Thr  Ala Phe Phe
       6170                6175                6180
   Thr Pro  Gly Ser Gly Met Pro  Pro Ala Gly Gly Glu  Ile Ser Lys
       6185                6190                6195
   Ser Thr  Gln Asn Leu Ile Gly  Ala Met Glu Glu Val  Pro Pro Asp
       6200                6205                6210
   Trp Glu  Arg Ala Ser Phe Gln  Gln Gly Ser Gln Ala  Ser Pro Asp
       6215                6220                6225
   Leu Lys  Pro Ser Pro Gln Asn  Gly Ala Thr Phe Pro  Ser Ser Gly
       6230                6235                6240
   Gly Tyr  Gly Gln Gly Ser Leu  Ile Ala Asp Glu Glu  Ser Gln Glu
       6245                6250                6255
   Phe Asp  Asp Leu Ile Phe Ala  Leu Lys Thr Gly Ala  Gly Leu Ser
       6260                6265                6270
   Val Ser  Asp Asn Glu Ser Gly  Gln Gly Ser Gln Glu  Gly Gly Thr
       6275                6280                6285
   Leu Thr  Asp Ser Gln Ile Val  Glu Leu Arg Arg Ile  Pro Ile Ala
       6290                6295                6300
   Asp Thr  His Leu
       6305

<210> 78
   <211> 465
   <212> PRT
   <213> homo sapiens
<400> 78
   Met His Leu Leu Val Cys Val Phe Gly Met Gly Ser Trp Val Thr Ile
   1               5                   10                  15
   Asn Gly Leu Trp Val Glu Leu Pro Leu Leu Val Met Glu Leu Pro Glu
               20                  25                  30
   Gly Trp Tyr Leu Pro Ser Tyr Leu Thr Val Val Ile Gln Leu Ala Asn
           35                  40                  45
   Ile Gly Pro Leu Leu Val Thr Leu Leu His His Phe Arg Pro Ser Cys
       50                  55                  60
   Leu Ser Glu Val Pro Ile Ile Phe Thr Leu Leu Gly Val Gly Thr Val
   65                  70                  75                  80
   Thr Cys Ile Ile Phe Ala Phe Leu Trp Asn Met Thr Ser Trp Val Leu
                   85                  90                  95
   Asp Gly His His Ser Ile Ala Phe Leu Val Leu Thr Phe Phe Leu Ala
               100                 105                 110
   Leu Val Asp Cys Thr Ser Ser Val Thr Phe Leu Pro Phe Met Ser Arg
           115                 120                 125
   Leu Pro Thr Tyr Tyr Leu Thr Thr Phe Phe Val Gly Glu Gly Leu Ser
       130                 135                 140
   Gly Leu Leu Pro Ala Leu Val Ala Leu Ala Gln Gly Ser Gly Leu Thr
   145                 150                 155                 160
   Thr Cys Val Asn Val Thr Glu Ile Ser Asp Ser Val Pro Ser Pro Val
                   165                 170                 175
   Pro Thr Arg Glu Thr Asp Ile Ala Gln Gly Val Pro Arg Ala Leu Val
               180                 185                 190
   Ser Ala Leu Pro Gly Met Glu Ala Pro Leu Ser His Leu Glu Ser Arg
           195                 200                 205
   Tyr Leu Pro Ala His Phe Ser Pro Leu Val Phe Phe Leu Leu Leu Ser
       210                 215                 220
   Ile Met Met Ala Cys Cys Leu Val Ala Phe Phe Val Leu Gln Arg Gln
   225                 230                 235                 240
   Pro Arg Cys Trp Glu Ala Ser Val Glu Asp Leu Leu Asn Asp Gln Val
                   245                 250                 255
   Thr Leu His Ser Ile Arg Leu Arg Glu Glu Asn Asp Leu Gly Pro Ala
               260                 265                 270
   Gly Met Val Asp Ser Ser Gln Gly Gln Gly Tyr Leu Glu Glu Lys Ala
           275                 280                 285
   Ala Pro Cys Cys Pro Ala His Leu Ala Phe Val Tyr Thr Leu Val Ala
       290                 295                 300
   Phe Val Asn Ala Leu Thr Asn Gly Met Leu Pro Ser Val Gln Thr Tyr
   305                 310                 315                 320
   Ser Cys Leu Ser Tyr Gly Pro Val Ala Tyr His Leu Ala Ala Thr Leu
                   325                 330                 335
   Ser Ile Val Ala Asn Pro Leu Ala Ser Leu Val Ser Met Phe Leu Pro
               340                 345                 350
   Asn Arg Ser Leu Leu Phe Leu Gly Val Leu Ser Val Leu Gly Thr Cys
           355                 360                 365
   Phe Gly Gly Tyr Asn Met Ala Met Ala Val Met Ser Pro Cys Pro Leu
       370                 375                 380
   Leu Gln Gly His Trp Gly Gly Glu Val Leu Ile Val Ala Ser Trp Val
   385                 390                 395                 400
   Leu Phe Ser Gly Cys Leu Ser Tyr Val Lys Val Met Leu Gly Val Val
                   405                 410                 415
   Leu Arg Asp Leu Ser Arg Ser Ala Leu Leu Trp Cys Gly Ala Ala Val
               420                 425                 430
   Gln Leu Gly Ser Leu Leu Gly Ala Leu Leu Met Phe Pro Leu Val Asn
           435                 440                 445
   Val Leu Arg Leu Phe Ser Ser Ala Asp Phe Cys Asn Leu His Cys Pro
       450                 455                 460
   Ala
   465
<210> 79
   <211> 458
   <212> PRT
   <213> homo sapiens
<400> 79
   Met Ala Gly Ser Pro Ser Arg Ala Ala Gly Arg Arg Leu Gln Leu Pro
   1               5                   10                  15
   Leu Leu Cys Leu Phe Leu Gln Gly Ala Thr Ala Val Leu Phe Ala Val
               20                  25                  30
   Phe Val Arg Tyr Asn His Lys Thr Asp Ala Ala Leu Trp His Arg Ser
           35                  40                  45
   Asn His Ser Asn Ala Asp Asn Glu Phe Tyr Phe Arg Tyr Pro Ser Phe
       50                  55                  60
   Gln Asp Val His Ala Met Val Phe Val Gly Phe Gly Phe Leu Met Val
   65                  70                  75                  80
   Phe Leu Gln Arg Tyr Gly Phe Ser Ser Val Gly Phe Thr Phe Leu Leu
                   85                  90                  95
   Ala Ala Phe Ala Leu Gln Trp Ser Thr Leu Val Gln Gly Phe Leu His
               100                 105                 110
   Ser Phe His Gly Gly His Ile His Val Gly Val Glu Ser Met Ile Asn
           115                 120                 125
   Ala Asp Phe Cys Ala Gly Ala Val Leu Ile Ser Phe Gly Ala Val Leu
       130                 135                 140
   Gly Lys Thr Gly Pro Thr Gln Leu Leu Leu Met Ala Leu Leu Glu Val
   145                 150                 155                 160
   Val Leu Phe Gly Ile Asn Glu Phe Val Leu Leu His Leu Leu Gly Val
                   165                 170                 175
   Arg Asp Ala Gly Gly Ser Met Thr Ile His Thr Phe Gly Ala Tyr Phe
               180                 185                 190
   Gly Leu Val Leu Ser Arg Val Leu Tyr Arg Pro Gln Leu Glu Lys Ser
           195                 200                 205
   Lys His Arg Gln Gly Ser Val Tyr His Ser Asp Leu Phe Ala Met Ile
       210                 215                 220
   Gly Thr Ile Phe Leu Trp Ile Phe Trp Pro Ser Phe Asn Ala Ala Leu
   225                 230                 235                 240
   Thr Ala Leu Gly Ala Gly Gln His Arg Thr Ala Leu Asn Thr Tyr Tyr
                   245                 250                 255
   Ser Leu Ala Ala Ser Thr Leu Gly Thr Phe Ala Leu Ser Ala Leu Val
               260                 265                 270
   Gly Glu Asp Gly Arg Leu Asp Met Val His Ile Gln Asn Ala Ala Leu
           275                 280                 285
   Ala Gly Gly Val Val Val Gly Thr Ser Ser Glu Met Met Leu Thr Pro
       290                 295                 300
   Phe Gly Ala Leu Ala Ala Gly Phe Leu Ala Gly Thr Val Ser Thr Leu
   305                 310                 315                 320
   Gly Tyr Lys Phe Phe Thr Pro Ile Leu Glu Ser Lys Phe Lys Val Gln
                   325                 330                 335
   Asp Thr Cys Gly Val His Asn Leu His Gly Met Pro Gly Val Leu Gly
               340                 345                 350
   Ala Leu Leu Gly Val Leu Val Ala Gly Leu Ala Thr His Glu Ala Tyr
           355                 360                 365
   Gly Asp Gly Leu Glu Ser Val Phe Pro Leu Ile Ala Glu Gly Gln Arg
       370                 375                 380
   Ser Ala Thr Ser Gln Ala Met His Gln Leu Phe Gly Leu Phe Val Thr
   385                 390                 395                 400
   Leu Met Phe Ala Ser Val Gly Gly Gly Leu Gly Gly Leu Leu Leu Lys
                   405                 410                 415
   Leu Pro Phe Leu Asp Ser Pro Pro Asp Ser Gln His Tyr Glu Asp Gln
               420                 425                 430
   Val His Trp Gln Val Pro Gly Glu His Glu Asp Lys Ala Gln Arg Pro
           435                 440                 445
   Leu Arg Val Glu Glu Ala Asp Thr Gln Ala
       450                 455
<210> 80
   <211> 871
   <212> PRT
   <213> homo sapiens
<400> 80
   Met Ile Lys Lys Phe Asp Lys Lys Asp Glu Glu Ser Gly Ser Gly Ser
   1               5                   10                  15
   Asn Pro Phe Gln His Leu Glu Lys Ser Ala Val Leu Gln Glu Ala Arg
               20                  25                  30
   Ile Phe Asn Glu Thr Pro Ile Asn Pro Arg Arg Cys Leu His Ile Leu
           35                  40                  45
   Thr Lys Ile Leu Tyr Leu Leu Asn Gln Gly Glu His Phe Gly Thr Thr
       50                  55                  60
   Glu Ala Thr Glu Ala Phe Phe Ala Met Thr Arg Leu Phe Gln Ser Asn
   65                  70                  75                  80
   Asp Gln Thr Leu Arg Arg Met Cys Tyr Leu Thr Ile Lys Glu Met Ala
                       85                  90                  95
   Thr Ile Ser Glu Asp Val Ile Ile Val Thr Ser Ser Leu Thr Lys Asp
                   100                 105                 110
   Met Thr Gly Lys Glu Asp Val Tyr Arg Gly Pro Ala Ile Arg Ala Leu
               115                 120                 125
   Cys Arg Ile Thr Asp Gly Thr Met Leu Gln Ala Ile Glu Arg Tyr Met
       130                 135                 140
   Lys Gln Ala Ile Val Asp Lys Val Ser Ser Val Ser Ser Ser Ala Leu
   145                 150                 155                 160
   Val Ser Ser Leu His Met Met Lys Ile Ser Tyr Asp Val Val Lys Arg
                   165                 170                 175
   Trp Ile Asn Glu Ala Gln Glu Ala Ala Ser Ser Asp Asn Ile Met Val
                   180                 185                 190
   Gln Tyr His Ala Leu Gly Val Leu Tyr His Leu Arg Lys Asn Asp Arg
               195                 200                 205
   Leu Ala Val Ser Lys Met Leu Asn Lys Phe Thr Lys Ser Gly Leu Lys
       210                 215                 220
   Ser Gln Phe Ala Tyr Cys Met Leu Ile Arg Ile Ala Ser Arg Leu Leu
   225                 230                 235                 240
   Lys Glu Thr Glu Asp Gly His Glu Ser Pro Leu Phe Asp Phe Ile Glu
                   245                 250                 255
   Ser Cys Leu Arg Asn Lys His Glu Met Val Ile Tyr Glu Ala Ala Ser
               260                 265                 270
   Ala Ile Ile His Leu Pro Asn Cys Thr Ala Arg Glu Leu Ala Pro Ala
           275                 280                 285
   Val Ser Val Leu Gln Leu Phe Cys Ser Ser Pro Lys Pro Ala Leu Arg
       290                 295                 300
   Tyr Ala Ala Val Arg Thr Leu Asn Lys Val Ala Met Lys His Pro Ser
   305                 310                 315                 320
   Ala Val Thr Ala Cys Asn Leu Asp Leu Glu Asn Leu Ile Thr Asp Ser
                   325                 330                 335
   Asn Arg Ser Ile Ala Thr Leu Ala Ile Thr Thr Leu Leu Lys Thr Gly
               340                 345                 350
   Ser Glu Ser Ser Val Asp Arg Leu Met Lys Gln Ile Ser Ser Phe Val
           355                 360                 365
   Ser Glu Ile Ser Asp Glu Phe Lys Val Val Val Val Gln Ala Ile Ser
       370                 375                 380
   Ala Leu Cys Gln Lys Tyr Pro Arg Lys His Ser Val Met Met Thr Phe
   385                 390                 395                 400
   Leu Ser Asn Met Leu Arg Asp Asp Gly Gly Phe Glu Tyr Lys Arg Ala
                   405                 410                 415
   Ile Val Asp Cys Ile Ile Ser Ile Val Glu Glu Asn Pro Glu Ser Lys
               420                 425                 430
   Glu Ala Gly Leu Ala His Leu Cys Glu Phe Ile Glu Asp Cys Glu His
           435                 440                 445
   Thr Val Leu Ala Thr Lys Ile Leu His Leu Leu Gly Lys Glu Gly Pro
       450                 455                 460
   Arg Thr Pro Val Pro Ser Lys Tyr Ile Arg Phe Ile Phe Asn Arg Val
   465                 470                 475                 480
   Val Leu Glu Asn Glu Ala Val Arg Ala Ala Ala Val Ser Ala Leu Ala
                   485                 490                 495
   Lys Phe Gly Ala Gln Asn Glu Ser Leu Leu Pro Ser Ile Leu Val Leu
               500                 505                 510
   Leu Gln Arg Cys Met Met Asp Thr Asp Asp Glu Val Arg Asp Arg Ala
           515                 520                 525
   Thr Phe Tyr Leu Asn Val Leu Gln Gln Arg Gln Met Ala Leu Asn Ala
       530                 535                 540
   Thr Tyr Ile Phe Asn Gly Leu Thr Val Ser Val Pro Gly Met Glu Lys
   545                 550                 555                 560
   Ala Leu His Gln Tyr Thr Leu Glu Pro Ser Glu Lys Pro Phe Asp Met
                   565                 570                 575
   Lys Ser Ile Pro Leu Ala Met Ala Pro Val Phe Glu Gln Lys Ala Glu
               580                 585                 590
   Ile Thr Leu Val Ala Thr Lys Pro Glu Lys Leu Ala Pro Ser Arg Gln
           595                 600                 605
   Asp Ile Phe Gln Glu Gln Leu Ala Ala Ile Pro Glu Phe Leu Asn Ile
       610                 615                 620
   Gly Pro Leu Phe Lys Ser Ser Glu Pro Val Gln Leu Thr Glu Ala Glu
   625                 630                 635                 640
   Thr Glu Tyr Phe Val Arg Cys Ile Lys His Met Phe Thr Asn His Ile
                   645                 650                 655
   Val Phe Gln Phe Asp Cys Thr Asn Thr Leu Asn Asp Gln Leu Leu Glu
               660                 665                 670
   Lys Val Thr Val Gln Met Glu Pro Ser Asp Ser Tyr Glu Val Leu Ser
           675                 680                 685
   Cys Ile Pro Ala Pro Ser Leu Pro Tyr Asn Gln Pro Gly Ile Cys Tyr
       690                 695                 700
   Thr Leu Val Arg Leu Pro Asp Asp Asp Pro Thr Ala Val Ala Gly Ser
   705                 710                 715                 720
   Phe Ser Cys Thr Met Lys Phe Thr Val Arg Asp Cys Asp Pro Asn Thr
                   725                 730                 735
   Gly Val Pro Asp Glu Asp Gly Tyr Asp Asp Glu Tyr Val Leu Glu Asp
               740                 745                 750
   Leu Glu Val Thr Val Ser Asp His Ile Gln Lys Val Leu Lys Pro Asn
           755                 760                 765
   Phe Ala Ala Ala Trp Glu Glu Val Gly Asp Thr Phe Glu Lys Glu Glu
       770                 775                 780
   Thr Phe Ala Leu Ser Ser Thr Lys Thr Leu Glu Glu Ala Val Asn Asn
   785                 790                 795                 800
   Ile Ile Thr Phe Leu Gly Met Gln Pro Cys Glu Arg Ser Asp Lys Val
                   805                 810                 815
   Pro Glu Asn Lys Asn Ser His Ser Leu Tyr Leu Ala Gly Ile Phe Arg
               820                 825                 830
   Gly Gly Tyr Asp Leu Leu Val Arg Ser Arg Leu Ala Leu Ala Asp Gly
           835                 840                 845
   Val Thr Met Gln Val Thr Val Arg Ser Lys Glu Arg Thr Pro Val Asp
       850                 855                 860
   Val Ile Leu Ala Ser Val Gly
   865                 870
<210> 81
   <211> 250
   <212> PRT
   <213> homo sapiens
<400> 81
   Met Ala Glu Gly Gly Ala Ser Lys Gly Gly Gly Glu Glu Pro Gly Lys
   1               5                   10                  15
   Leu Pro Glu Pro Ala Glu Glu Glu Ser Gln Val Leu Arg Gly Thr Gly
               20                  25                  30
   His Cys Lys Trp Phe Asn Val Arg Met Gly Phe Gly Phe Ile Ser Met
           35                  40                  45
   Ile Asn Arg Glu Gly Ser Pro Leu Asp Ile Pro Val Asp Val Phe Val
       50                  55                  60
   His Gln Ser Lys Leu Phe Met Glu Gly Phe Arg Ser Leu Lys Glu Gly
   65                  70                  75                  80
   Glu Pro Val Glu Phe Thr Phe Lys Lys Ser Ser Lys Gly Leu Glu Ser
                   85                  90                  95
   Ile Arg Val Thr Gly Pro Gly Gly Ser Pro Cys Leu Gly Ser Glu Arg
               100                 105                 110
   Arg Pro Lys Gly Lys Thr Leu Gln Lys Arg Lys Pro Lys Gly Asp Arg
           115                 120                 125
   Cys Tyr Asn Cys Gly Gly Leu Asp His His Ala Lys Glu Cys Ser Leu
       130                 135                 140
   Pro Pro Gln Pro Lys Lys Cys His Tyr Cys Gln Ser Ile Met His Met
   145                 150                 155                 160
   Val Ala Asn Cys Pro His Lys Asn Val Ala Gln Pro Pro Ala Ser Ser
                   165                 170                 175
   Gln Gly Arg Gln Glu Ala Glu Ser Gln Pro Cys Thr Ser Thr Leu Pro
               180                 185                 190
   Arg Glu Val Gly Gly Gly His Gly Cys Thr Ser Pro Pro Phe Pro Gln
           195                 200                 205
   Glu Ala Arg Ala Glu Ile Ser Glu Arg Ser Gly Arg Ser Pro Gln Glu
       210                 215                 220
   Ala Ser Ser Thr Lys Ser Ser Ile Ala Pro Glu Glu Gln Ser Lys Lys
   225                 230                 235                 240
   Gly Pro Ser Val Gln Lys Arg Lys Lys Thr
                   245                 250
<210> 82
   <211> 210
   <212> PRT
   <213> homo sapiens
<400> 82
   Met Gly Phe Gly Phe Ile Ser Met Ile Asn Arg Glu Gly Ser Pro Leu
   1 5 10 15
   Asp Ile Pro Val Asp Val Phe Val His Gln Ser Lys Leu Phe Met Glu
   20 25 30
   Gly Phe Arg Ser Leu Lys Glu Gly Glu Pro Val Glu Phe Thr Phe Lys
   35 40 45
   Lys Ser Ser Lys Gly Leu Glu Ser Ile Arg Val Thr Gly Pro Gly Gly
   50 55 60
   Ser Pro Cys Leu Gly Ser Glu Arg Arg Pro Lys Gly Lys Thr Leu Gln
   65 70 75 80
   Lys Arg Lys Pro Lys Gly Asp Arg Cys Tyr Asn Cys Gly Gly Leu Asp
   85 90 95
   His His Ala Lys Glu Cys Ser Leu Pro Pro Gln Pro Lys Lys Cys His
   100 105 110
   Tyr Cys Gln Ser Ile Met His Met Val Ala Asn Cys Pro His Lys Asn
   115 120 125
   Val Ala Gln Pro Pro Ala Ser Ser Gln Gly Arg Gln Glu Ala Glu Ser
   130 135 140
   Gln Pro Cys Thr Ser Thr Leu Pro Arg Glu Val Gly Gly Gly His Gly
   145 150 155 160
   Cys Thr Ser Pro Pro Phe Pro Gln Glu Ala Arg Ala Glu Ile Ser Glu
   165 170 175
   Arg Ser Gly Arg Ser Pro Gln Glu Ala Ser Ser Thr Lys Ser Ser Ile
   180 185 190
   Ala Pro Glu Glu Gln Ser Lys Lys Gly Pro Ser Val Gln Lys Arg Lys
   195 200 205
   Lys Thr
   210
<210> 83
   <211> 391
   <212> PRT
   <213> homo sapiens
<400> 83
   Met Arg Gln Leu Cys Arg Gly Arg Val Leu Gly Ile Ser Val Ala Ile
   1 5 10 15
   Ala His Gly Val Phe Ser Gly Ser Leu Asn Ile Leu Leu Lys Phe Leu
   20 25 30
   Ile Ser Arg Tyr Gln Phe Ser Phe Leu Thr Leu Val Gln Cys Leu Thr
   35 40 45
   Ser Ser Thr Ala Ala Leu Ser Leu Glu Leu Leu Arg Arg Leu Gly Leu
   50 55 60
   Ile Ala Val Pro Pro Phe Gly Leu Ser Leu Ala Arg Ser Phe Ala Gly
   65 70 75 80
   Val Ala Val Leu Ser Thr Leu Gln Ser Ser Leu Thr Leu Trp Ser Leu
   85 90 95
   Arg Gly Leu Ser Leu Pro Met Tyr Val Val Phe Lys Arg Cys Leu Pro
   100 105 110
   Leu Val Thr Met Leu Ile Gly Val Leu Val Leu Lys Asn Gly Ala Pro
           115                 120                 125
   Ser Pro Gly Val Leu Ala Ala Val Leu Ile Thr Thr Cys Gly Ala Ala
       130                 135                 140
   Leu Ala Gly Ala Gly Asp Leu Thr Gly Asp Pro Ile Gly Tyr Val Thr
   145                 150                 155                 160
   Gly Val Leu Ala Val Leu Val His Ala Ala Tyr Leu Val Leu Ile Gln
                   165                 170                 175
   Lys Ala Ser Ala Asp Thr Glu His Gly Pro Leu Thr Ala Gln Tyr Val
               180                 185                 190
   Ile Ala Val Ser Ala Thr Pro Leu Leu Val Ile Cys Ser Phe Ala Ser
           195                 200                 205
   Thr Asp Ser Ile His Ala Trp Thr Phe Pro Gly Trp Lys Asp Pro Ala
       210                 215                 220
   Met Val Cys Ile Phe Val Ala Cys Ile Leu Ile Gly Cys Ala Met Asn
   225                 230                 235                 240
   Phe Thr Thr Leu His Cys Thr Tyr Ile Asn Ser Ala Val Thr Thr Ser
                   245                 250                 255
   Leu Phe Ile Ala Gly Val Val Val Asn Thr Leu Gly Ser Ile Ile Tyr
               260                 265                 270
   Cys Val Ala Lys Phe Met Glu Thr Arg Lys Gln Ser Asn Tyr Glu Asp
           275                 280                 285
   Leu Glu Ala Gln Pro Arg Gly Glu Glu Ala Gln Leu Ser Gly Asp Gln
       290                 295                 300
   Leu Pro Phe Val Met Glu Glu Leu Pro Gly Glu Gly Gly Asn Gly Arg
   305                 310                 315                 320
   Ser Glu Gly Gly Glu Ala Ala Gly Gly Pro Ala Gln Glu Ser Arg Gln
                   325                 330                 335
   Glu Val Arg Gly Ser Pro Arg Gly Val Pro Leu Val Ala Gly Ser Ser
               340                 345                 350
   Glu Glu Gly Ser Arg Arg Ser Leu Lys Asp Ala Tyr Leu Glu Val Trp
           355                 360                 365
   Arg Leu Val Arg Gly Thr Arg Tyr Met Lys Lys Asp Tyr Leu Ile Glu
       370                 375                 380
   Asn Glu Glu Leu Pro Ser Pro
   385                 390
<210> 84
   <211> 907
   <212> PRT
   <213> homo sapiens
<400> 84
   Met Asp Thr Ser Arg Leu Gly Val Leu Leu Ser Leu Pro Val Leu Leu
   1               5                   10                  15
   Gln Leu Ala Thr Gly Gly Ser Ser Pro Arg Ser Gly Val Leu Leu Arg
               20                  25                  30
   Gly Cys Pro Thr His Cys His Cys Glu Pro Asp Gly Arg Met Leu Leu
           35                  40                  45
   Arg Val Asp Cys Ser Asp Leu Gly Leu Ser Glu Leu Pro Ser Asn Leu
       50                  55                  60
   Ser Val Phe Thr Ser Tyr Leu Asp Leu Ser Met Asn Asn Ile Ser Gln
   65                  70                  75                  80
   Leu Leu Pro Asn Pro Leu Pro Ser Leu Arg Phe Leu Glu Glu Leu Arg
                   85                  90                  95
   Leu Ala Gly Asn Ala Leu Thr Tyr Ile Pro Lys Gly Ala Phe Thr Gly
               100                 105                 110
   Leu Tyr Ser Leu Lys Val Leu Met Leu Gln Asn Asn Gln Leu Arg His
           115                 120                 125
   Val Pro Thr Glu Ala Leu Gln Asn Leu Arg Ser Leu Gln Ser Leu Arg
       130                 135                 140
   Leu Asp Ala Asn His Ile Ser Tyr Val Pro Pro Ser Cys Phe Ser Gly
   145                 150                 155                 160
   Leu His Ser Leu Arg His Leu Trp Leu Asp Asp Asn Ala Leu Thr Glu
                   165                 170                 175
   Ile Pro Val Gln Ala Phe Arg Ser Leu Ser Ala Leu Gln Ala Met Thr
               180                 185                 190
   Leu Ala Leu Asn Lys Ile His His Ile Pro Asp Tyr Ala Phe Gly Asn
           195                 200                 205
   Leu Ser Ser Leu Val Val Leu His Leu His Asn Asn Arg Ile His Ser
       210                 215                 220
   Leu Gly Lys Lys Cys Phe Asp Gly Leu His Ser Leu Glu Thr Leu Asp
   225                 230                 235                 240
   Leu Asn Tyr Asn Asn Leu Asp Glu Phe Pro Thr Ala Ile Arg Thr Leu
                   245                 250                 255
   Ser Asn Leu Lys Glu Leu Gly Phe His Ser Asn Asn Ile Arg Ser Ile
               260                 265                 270
   Pro Glu Lys Ala Phe Val Gly Asn Pro Ser Leu Ile Thr Ile His Phe
           275                 280                 285
   Tyr Asp Asn Pro Ile Gln Phe Val Gly Arg Ser Ala Phe Gln His Leu
       290                 295                 300
   Pro Glu Leu Arg Thr Leu Thr Leu Asn Gly Ala Ser Gln Ile Thr Glu
   305                 310                 315                 320
   Phe Pro Asp Leu Thr Gly Thr Ala Asn Leu Glu Ser Leu Thr Leu Thr
                   325                 330                 335
   Gly Ala Gln Ile Ser Ser Leu Pro Gln Thr Val Cys Asn Gln Leu Pro
               340                 345                 350
   Asn Leu Gln Val Leu Asp Leu Ser Tyr Asn Leu Leu Glu Asp Leu Pro
           355                 360                 365
   Ser Phe Ser Val Cys Gln Lys Leu Gln Lys Ile Asp Leu Arg His Asn
       370                 375                 380
   Glu Ile Tyr Glu Ile Lys Val Asp Thr Phe Gln Gln Leu Leu Ser Leu
   385                 390                 395                 400
   Arg Ser Leu Asn Leu Ala Trp Asn Lys Ile Ala Ile Ile His Pro Asn
                   405                 410                 415
   Ala Phe Ser Thr Leu Pro Ser Leu Ile Lys Leu Asp Leu Ser Ser Asn
               420                 425                 430
   Leu Leu Ser Ser Phe Pro Ile Thr Gly Leu His Gly Leu Thr His Leu
           435                 440                 445
   Lys Leu Thr Gly Asn His Ala Leu Gln Ser Leu Ile Ser Ser Glu Asn
       450                 455                 460
   Phe Pro Glu Leu Lys Val Ile Glu Met Pro Tyr Ala Tyr Gln Cys Cys
   465                 470                 475                 480
   Ala Phe Gly Val Cys Glu Asn Ala Tyr Lys Ile Ser Asn Gln Trp Asn
                   485                 490                 495
   Lys Gly Asp Asn Ser Ser Met Asp Asp Leu His Lys Lys Asp Ala Gly
               500                 505                 510
   Met Phe Gln Ala Gln Asp Glu Arg Asp Leu Glu Asp Phe Leu Leu Asp
           515                 520                 525
   Phe Glu Glu Asp Leu Lys Ala Leu His Ser Val Gln Cys Ser Pro Ser
       530                 535                 540
   Pro Gly Pro Phe Lys Pro Cys Glu His Leu Leu Asp Gly Trp Leu Ile
   545                 550                 555                 560
   Arg Ile Gly Val Trp Thr Ile Ala Val Leu Ala Leu Thr Cys Asn Ala
                   565                 570                 575
   Leu Val Thr Ser Thr Val Phe Arg Ser Pro Leu Tyr Ile Ser Pro Ile
               580                 585                 590
   Lys Leu Leu Ile Gly Val Ile Ala Ala Val Asn Met Leu Thr Gly Val
           595                 600                 605
   Ser Ser Ala Val Leu Ala Gly Val Asp Ala Phe Thr Phe Gly Ser Phe
       610                 615                 620
   Ala Arg His Gly Ala Trp Trp Glu Asn Gly Val Gly Cys His Val Ile
   625                 630                 635                 640
   Gly Phe Leu Ser Ile Phe Ala Ser Glu Ser Ser Val Phe Leu Leu Thr
                   645                 650                 655
   Leu Ala Ala Leu Glu Arg Gly Phe Ser Val Lys Tyr Ser Ala Lys Phe
               660                 665                 670
   Glu Thr Lys Ala Pro Phe Ser Ser Leu Lys Val Ile Ile Leu Leu Cys
           675                 680                 685
   Ala Leu Leu Ala Leu Thr Met Ala Ala Val Pro Leu Leu Gly Gly Ser
       690                 695                 700
   Lys Tyr Gly Ala Ser Pro Leu Cys Leu Pro Leu Pro Phe Gly Glu Pro
   705                 710                 715                 720
   Ser Thr Met Gly Tyr Met Val Ala Leu Ile Leu Leu Asn Ser Leu Cys
                   725                 730                 735
   Phe Leu Met Met Thr Ile Ala Tyr Thr Lys Leu Tyr Cys Asn Leu Asp
               740                 745                 750
   Lys Gly Asp Leu Glu Asn Ile Trp Asp Cys Ser Met Val Lys His Ile
           755                 760                 765
   Ala Leu Leu Leu Phe Thr Asn Cys Ile Leu Asn Cys Pro Val Ala Phe
       770                 775                 780
   Leu Ser Phe Ser Ser Leu Ile Asn Leu Thr Phe Ile Ser Pro Glu Val
   785                 790                 795                 800
   Ile Lys Phe Ile Leu Leu Val Val Val Pro Leu Pro Ala Cys Leu Asn
                   805                 810                 815
   Pro Leu Leu Tyr Ile Leu Phe Asn Pro His Phe Lys Glu Asp Leu Val
               820                 825                 830
   Ser Leu Arg Lys Gln Thr Tyr Val Trp Thr Arg Ser Lys His Pro Ser
           835                 840                 845
   Leu Met Ser Ile Asn Ser Asp Asp Val Glu Lys Gln Ser Cys Asp Ser
       850                 855                 860
   Thr Gln Ala Leu Val Thr Phe Thr Ser Ser Ser Ile Thr Tyr Asp Leu
   865                 870                 875                 880
   Pro Pro Ser Ser Val Pro Ser Pro Ala Tyr Pro Val Thr Glu Ser Cys
                   885                 890                 895
   His Leu Ser Ser Val Ala Phe Val Pro Cys Leu
               900                 905
<210> 85
   <211> 982
   <212> PRT
   <213> homo sapiens
<400> 85
   Met Pro Thr Ser Thr Ala Gly Glu Gly Ser Thr Pro Leu Thr Asn Met
   1               5                   10                  15
   Pro Val Ser Thr Thr Pro Val Ala Ser Ser Glu Ala Ser Thr Leu Ser
               20                  25                  30
   Thr Thr Pro Val Asp Ser Asn Thr Phe Val Thr Ser Ser Ser Gln Ala
           35                  40                  45
   Ser Ser Ser Pro Ala Thr Leu Gln Val Thr Thr Met Arg Met Ser Thr
       50                  55                  60
   Pro Ser Glu Gly Ser Ser Ser Leu Thr Thr Met Leu Leu Ser Ser Thr
   65                  70                  75                  80
   Tyr Val Thr Ser Ser Glu Ala Ser Thr Pro Ser Thr Pro Ser Val Asp
                   85                  90                  95
   Arg Ser Thr Pro Val Thr Thr Ser Thr Gln Ser Asn Ser Thr Pro Thr
               100                 105                 110
   Pro Pro Glu Val Ile Thr Leu Pro Met Ser Thr Pro Ser Glu Val Ser
           115                 120                 125
   Thr Pro Leu Thr Ile Met Pro Val Ser Thr Thr Ser Val Thr Ile Ser
       130                 135                 140
   Glu Ala Gly Thr Ala Ser Thr Leu Pro Val Asp Thr Ser Thr Pro Val
   145                 150                 155                 160
   Ile Thr Ser Thr Gln Val Ser Ser Ser Pro Val Thr Pro Glu Gly Thr
                   165                 170                 175
   Thr Met Pro Ile Trp Thr Pro Ser Glu Gly Ser Thr Pro Leu Thr Thr
               180                 185                 190
   Met Pro Val Ser Thr Thr Arg Val Thr Ser Ser Glu Gly Ser Thr Leu
           195                 200                 205
   Ser Thr Pro Ser Val Val Thr Ser Thr Pro Val Thr Thr Ser Thr Glu
       210                 215                 220
   Ala Ile Ser Ser Ser Ala Thr Leu Asp Ser Thr Thr Met Ser Val Ser
   225                 230                 235                 240
   Met Pro Met Glu Ile Ser Thr Leu Gly Thr Thr Ile Leu Val Ser Thr
                   245                 250                 255
   Thr Pro Val Thr Arg Phe Pro Glu Ser Ser Thr Pro Ser Ile Pro Ser
               260                 265                 270
   Val Tyr Thr Ser Met Ser Met Thr Thr Ala Ser Glu Gly Ser Ser Ser
           275                 280                 285
   Pro Thr Thr Leu Glu Gly Thr Thr Thr Met Pro Met Ser Thr Thr Ser
       290                 295                 300
   Glu Arg Ser Thr Leu Leu Thr Thr Val Leu Ile Ser Pro Ile Ser Val
   305                 310                 315                 320
   Met Ser Pro Ser Glu Ala Ser Thr Leu Ser Thr Pro Pro Gly Asp Thr
                   325                 330                 335
   Ser Thr Pro Leu Leu Thr Ser Thr Lys Ala Gly Ser Phe Ser Ile Pro
               340                 345                 350
   Ala Glu Val Thr Thr Ile Arg Ile Ser Ile Thr Ser Glu Arg Ser Thr
           355                 360                 365
   Pro Leu Thr Thr Leu Leu Val Ser Thr Thr Leu Pro Thr Ser Phe Pro
       370                 375                 380
   Gly Ala Ser Ile Ala Ser Thr Pro Pro Leu Asp Thr Ser Thr Thr Phe
   385                 390                 395                 400
   Thr Pro Ser Thr Asp Thr Ala Ser Thr Pro Thr Ile Pro Val Ala Thr
                   405                 410                 415
   Thr Ile Ser Val Ser Val Ile Thr Glu Gly Ser Thr Pro Gly Thr Thr
               420                 425                 430
   Ile Phe Ile Pro Ser Thr Pro Val Thr Ser Ser Thr Ala Asp Val Phe
           435                 440                 445
   Pro Ala Thr Thr Gly Ala Val Ser Thr Pro Val Ile Thr Ser Thr Glu
       450                 455                 460
   Leu Asn Thr Pro Ser Thr Ser Ser Ser Ser Thr Thr Thr Ser Phe Ser
   465                 470                 475                 480
   Thr Thr Lys Glu Phe Thr Thr Pro Ala Met Thr Thr Ala Ala Pro Leu
                   485                 490                 495
   Thr Tyr Val Thr Met Ser Thr Ala Pro Ser Thr Pro Arg Thr Thr Ser
               500                 505                 510
   Arg Gly Cys Thr Thr Ser Ala Ser Thr Leu Ser Ala Thr Ser Thr Pro
           515                 520                 525
   His Thr Ser Thr Ser Val Thr Thr Arg Pro Val Thr Pro Ser Ser Glu
       530                 535                 540
   Ser Ser Arg Pro Ser Thr Ile Thr Ser His Thr Ile Pro Pro Thr Phe
   545                 550                 555                 560
   Pro Pro Ala His Ser Ser Thr Pro Pro Thr Thr Ser Ala Ser Ser Thr
                   565                 570                 575
   Thr Val Asn Pro Glu Ala Val Thr Thr Met Thr Thr Arg Thr Lys Pro
               580                 585                 590
   Ser Thr Arg Thr Thr Ser Phe Pro Thr Val Thr Thr Thr Ala Val Pro
           595                 600                 605
   Thr Asn Thr Thr Ile Lys Ser Asn Pro Thr Ser Thr Pro Thr Val Pro
       610                 615                 620
   Arg Thr Thr Thr Cys Phe Gly Asp Gly Cys Gln Asn Thr Ala Ser Arg
   625                 630                 635                 640
   Cys Lys Asn Gly Gly Thr Trp Asp Gly Leu Lys Cys Gln Cys Pro Asn
                   645                 650                 655
   Leu Tyr Tyr Gly Glu Leu Cys Glu Glu Val Val Ser Ser Ile Asp Ile
               660                 665                 670
   Gly Pro Pro Glu Thr Ile Ser Ala Gln Met Glu Leu Thr Val Thr Val
           675                 680                 685
   Thr Ser Val Lys Phe Thr Glu Glu Leu Lys Asn His Ser Ser Gln Glu
       690                 695                 700
   Phe Gln Glu Phe Lys Gln Thr Phe Thr Glu Gln Met Asn Ile Val Tyr
   705                 710                 715                 720
   Ser Gly Ile Pro Glu Tyr Val Gly Val Asn Ile Thr Lys Leu Arg Leu
                   725                 730                 735
   Gly Ser Val Val Val Glu His Asp Val Leu Leu Arg Thr Lys Tyr Thr
                   740                 745                 750
   Pro Glu Tyr Lys Thr Val Leu Asp Asn Ala Thr Glu Val Val Lys Glu
               755                 760                 765
   Lys Ile Thr Lys Val Thr Thr Gln Gln Ile Met Ile Asn Asp Ile Cys
       770                 775                 780
   Ser Asp Met Met Cys Phe Asn Thr Thr Gly Thr Gln Val Gln Asn Ile
   785                 790                 795                 800
   Thr Val Thr Gln Tyr Asp Pro Glu Glu Asp Cys Arg Lys Met Ala Lys
                   805                 810                 815
   Glu Tyr Gly Asp Tyr Phe Val Val Glu Tyr Arg Asp Gln Lys Pro Tyr
               820                 825                 830
   Cys Ile Ser Pro Cys Glu Pro Gly Phe Ser Val Ser Lys Asn Cys Asn
           835                 840                 845
   Leu Gly Lys Cys Gln Met Ser Leu Ser Gly Pro Gln Cys Leu Cys Val
       850                 855                 860
   Thr Thr Glu Thr His Trp Tyr Ser Gly Glu Thr Cys Asn Gln Gly Thr
   865                 870                 875                 880
   Gln Lys Ser Leu Val Tyr Gly Leu Val Gly Ala Gly Val Val Leu Met
                   885                 890                 895
   Leu Ile Ile Leu Val Ala Leu Leu Met Leu Val Phe Arg Ser Lys Arg
               900                 905                 910
   Glu Val Lys Arg Gln Lys Tyr Arg Leu Ser Gln Leu Tyr Lys Trp Gln
           915                 920                 925
   Glu Glu Asp Ser Gly Pro Ala Pro Gly Thr Phe Gln Asn Ile Gly Phe
       930                 935                 940
   Asp Ile Cys Gln Asp Asp Asp Ser Ile His Leu Glu Ser Ile Tyr Ser
   945                 950                 955                 960
   Asn Phe Gln Pro Ser Leu Arg His Ile Asp Pro Glu Thr Lys Arg Ser
                   965                 970                 975
   Glu Phe Arg Gly Leu Arg
               980
<210> 86
   <211> 987
   <212> PRT
   <213> homo sapiens
<400> 86
   Met Ala Leu Arg Arg Leu Gly Ala Ala Leu Leu Leu Leu Pro Leu Leu
   1               5                   10                  15
   Ala Ala Val Glu Glu Thr Leu Met Asp Ser Thr Thr Ala Thr Ala Glu
               20                  25                  30
   Leu Gly Trp Met Val His Pro Pro Ser Gly Trp Glu Glu Val Ser Gly
           35                  40                  45
   Tyr Asp Glu Asn Met Asn Thr Ile Arg Thr Tyr Gln Val Cys Asn Val
       50                  55                  60
   Phe Glu Ser Ser Gln Asn Asn Trp Leu Arg Thr Lys Phe Ile Arg Arg
   65                  70                  75                  80
   Arg Gly Ala His Arg Ile His Val Glu Met Lys Phe Ser Val Arg Asp
                   85                  90                  95
   Cys Ser Ser Ile Pro Ser Val Pro Gly Ser Cys Lys Glu Thr Phe Asn
               100                 105                 110
   Leu Tyr Tyr Tyr Glu Ala Asp Phe Asp Ser Ala Thr Lys Thr Phe Pro
           115                 120                 125
   Asn Trp Met Glu Asn Pro Trp Val Lys Val Asp Thr Ile Ala Ala Asp
       130                 135                 140
   Glu Ser Phe Ser Gln Val Asp Leu Gly Gly Arg Val Met Lys Ile Asn
   145                 150                 155                 160
   Thr Glu Val Arg Ser Phe Gly Pro Val Ser Arg Ser Gly Phe Tyr Leu
                   165                 170                 175
   Ala Phe Gln Asp Tyr Gly Gly Cys Met Ser Leu Ile Ala Val Arg Val
               180                 185                 190
   Phe Tyr Arg Lys Cys Pro Arg Ile Ile Gln Asn Gly Ala Ile Phe Gln
           195                 200                 205
   Glu Thr Leu Ser Gly Ala Glu Ser Thr Ser Leu Val Ala Ala Arg Gly
       210                 215                 220
   Ser Cys Ile Ala Asn Ala Glu Glu Val Asp Val Pro Ile Lys Leu Tyr
   225                 230                 235                 240
   Cys Asn Gly Asp Gly Glu Trp Leu Val Pro Ile Gly Arg Cys Met Cys
                   245                 250                 255
   Lys Ala Gly Phe Glu Ala Val Glu Asn Gly Thr Val Cys Arg Gly Cys
               260                 265                 270
   Pro Ser Gly Thr Phe Lys Ala Asn Gln Gly Asp Glu Ala Cys Thr His
           275                 280                 285
   Cys Pro Ile Asn Ser Arg Thr Thr Ser Glu Gly Ala Thr Asn Cys Val
       290                 295                 300
   Cys Arg Asn Gly Tyr Tyr Arg Ala Asp Leu Asp Pro Leu Asp Met Pro
   305                 310                 315                 320
   Cys Thr Thr Ile Pro Ser Ala Pro Gln Ala Val Ile Ser Ser Val Asn
                   325                 330                 335
   Glu Thr Ser Leu Met Leu Glu Trp Thr Pro Pro Arg Asp Ser Gly Gly
               340                 345                 350
   Arg Glu Asp Leu Val Tyr Asn Ile Ile Cys Lys Ser Cys Gly Ser Gly
           355                 360                 365
   Arg Gly Ala Cys Thr Arg Cys Gly Asp Asn Val Gln Tyr Ala Pro Arg
       370                 375                 380
   Gln Leu Gly Leu Thr Glu Pro Arg Ile Tyr Ile Ser Asp Leu Leu Ala
   385                 390                 395                 400
   His Thr Gln Tyr Thr Phe Glu Ile Gln Ala Val Asn Gly Val Thr Asp
                   405                 410                 415
   Gln Ser Pro Phe Ser Pro Gln Phe Ala Ser Val Asn Ile Thr Thr Asn
               420                 425                 430
   Gln Ala Ala Pro Ser Ala Val Ser Ile Met His Gln Val Ser Arg Thr
           435                 440                 445
   Val Asp Ser Ile Thr Leu Ser Trp Ser Gln Pro Asp Gln Pro Asn Gly
       450                 455                 460
   Val Ile Leu Asp Tyr Glu Leu Gln Tyr Tyr Glu Lys Glu Leu Ser Glu
   465                 470                 475                 480
   Tyr Asn Ala Thr Ala Ile Lys Ser Pro Thr Asn Thr Val Thr Val Gln
                   485                 490                 495
   Gly Leu Lys Ala Gly Ala Ile Tyr Val Phe Gln Val Arg Ala Arg Thr
               500                 505                 510
   Val Ala Gly Tyr Gly Arg Tyr Ser Gly Lys Met Tyr Phe Gln Thr Met
           515                 520                 525
   Thr Glu Ala Glu Tyr Gln Thr Ser Ile Gln Glu Lys Leu Pro Leu Ile
       530                 535                 540
   Ile Gly Ser Ser Ala Ala Gly Leu Val Phe Leu Ile Ala Val Val Val
   545                 550                 555                 560
   Ile Ala Ile Val Cys Asn Arg Arg Arg Gly Phe Glu Arg Ala Asp Ser
                   565                 570                 575
   Glu Tyr Thr Asp Lys Leu Gln His Tyr Thr Ser Gly His Met Thr Pro
               580                 585                 590
   Gly Met Lys Ile Tyr Ile Asp Pro Phe Thr Tyr Glu Asp Pro Asn Glu
           595                 600                 605
   Ala Val Arg Glu Phe Ala Lys Glu Ile Asp Ile Ser Cys Val Lys Ile
       610                 615                 620
   Glu Gln Val Ile Gly Ala Gly Glu Phe Gly Glu Val Cys Ser Gly His
   625                 630                 635                 640
   Leu Lys Leu Pro Gly Lys Arg Glu Ile Phe Val Ala Ile Lys Thr Leu
                   645                 650                 655
   Lys Ser Gly Tyr Thr Glu Lys Gln Arg Arg Asp Phe Leu Ser Glu Ala
               660                 665                 670
   Ser Ile Met Gly Gln Phe Asp His Pro Asn Val Ile His Leu Glu Gly
           675                 680                 685
   Val Val Thr Lys Ser Thr Pro Val Met Ile Ile Thr Glu Phe Met Glu
       690                 695                 700
   Asn Gly Ser Leu Asp Ser Phe Leu Arg Gln Asn Asp Gly Gln Phe Thr
   705                 710                 715                 720
   Val Ile Gln Leu Val Gly Met Leu Arg Gly Ile Ala Ala Gly Met Lys
                   725                 730                 735
   Tyr Leu Ala Asp Met Asn Tyr Val His Arg Asp Leu Ala Ala Arg Asn
               740                 745                 750
   Ile Leu Val Asn Ser Asn Leu Val Cys Lys Val Ser Asp Phe Gly Leu
           755                 760                 765
   Ser Arg Phe Leu Glu Asp Asp Thr Ser Asp Pro Thr Tyr Thr Ser Ala
       770                 775                 780
   Leu Gly Gly Lys Ile Pro Ile Arg Trp Thr Ala Pro Glu Ala Ile Gln
   785                 790                 795                 800
   Tyr Arg Lys Phe Thr Ser Ala Ser Asp Val Trp Ser Tyr Gly Ile Val
                   805                 810                 815
   Met Trp Glu Val Met Ser Tyr Gly Glu Arg Pro Tyr Trp Asp Met Thr
               820                 825                 830
   Asn Gln Asp Val Ile Asn Ala Ile Glu Gln Asp Tyr Arg Leu Pro Pro
           835                 840                 845
   Pro Met Asp Cys Pro Ser Ala Leu His Gln Leu Met Leu Asp Cys Trp
       850                 855                 860
   Gln Lys Asp Arg Asn His Arg Pro Lys Phe Gly Gln Ile Val Asn Thr
   865                 870                 875                 880
   Leu Asp Lys Met Ile Arg Asn Pro Asn Ser Leu Lys Ala Met Ala Pro
                   885                 890                 895
   Leu Ser Ser Gly Ile Asn Leu Pro Leu Leu Asp Arg Thr Ile Pro Asp
               900                 905                 910
   Tyr Thr Ser Phe Asn Thr Val Asp Glu Trp Leu Glu Ala Ile Lys Met
           915                 920                 925
   Gly Gln Tyr Lys Glu Ser Phe Ala Asn Ala Gly Phe Thr Ser Phe Asp
       930                 935                 940
   Val Val Ser Gln Met Met Met Glu Asp Ile Leu Arg Val Gly Val Thr
   945                 950                 955                 960
   Leu Ala Gly His Gln Lys Lys Ile Leu Asn Ser Ile Gln Val Met Arg
                   965                 970                 975
   Ala Gln Met Asn Gln Ile Gln Ser Val Glu Val
               980                 985
<210> 87
   <211> 445
   <212> PRT
   <213> homo sapiens
<400> 87
<210> 88
   <211> 459
   <212> PRT
   <213> homo sapiens
<400> 88
   Met Asp Glu Lys Ser Asn Lys Leu Leu Leu Ala Leu Val Met Leu Phe
   1               5                   10                  15
   Leu Phe Ala Val Ile Val Leu Gln Tyr Val Cys Pro Gly Thr Glu Cys
               20                  25                  30
   Gln Leu Leu Arg Leu Gln Ala Phe Ser Ser Pro Val Pro Asp Pro Tyr
           35                  40                  45
   Arg Ser Glu Asp Glu Ser Ser Ala Arg Phe Val Pro Arg Tyr Asn Phe
       50                  55                  60
   Thr Arg Gly Asp Leu Leu Arg Lys Val Asp Phe Asp Ile Lys Gly Asp
   65                  70                  75                  80
   Asp Leu Ile Val Phe Leu His Ile Gln Lys Thr Gly Gly Thr Thr Phe
                   85                  90                  95
   Gly Arg His Leu Val Arg Asn Ile Gln Leu Glu Gln Pro Cys Glu Cys
               100                 105                 110
   Arg Val Gly Gln Lys Lys Cys Thr Cys His Arg Pro Gly Lys Arg Glu
           115                 120                 125
   Thr Trp Leu Phe Ser Arg Phe Ser Thr Gly Trp Ser Cys Gly Leu His
       130                 135                 140
   Ala Asp Trp Thr Glu Leu Thr Ser Cys Val Pro Ser Val Val Asp Gly
   145                 150                 155                 160
   Lys Arg Asp Ala Arg Leu Arg Pro Ser Arg Asn Phe His Tyr Ile Thr
                   165                 170                 175
   Ile Leu Arg Asp Pro Val Ser Arg Tyr Leu Ser Glu Trp Arg His Val
               180                 185                 190
   Gln Arg Gly Ala Thr Trp Lys Ala Ser Leu His Val Cys Asp Gly Arg
           195                 200                 205
   Pro Pro Thr Ser Glu Glu Leu Pro Ser Cys Tyr Thr Gly Asp Asp Trp
       210                 215                 220
   Ser Gly Cys Pro Leu Lys Glu Phe Met Asp Cys Pro Tyr Asn Leu Ala
   225                 230                 235                 240
   Asn Asn Arg Gln Val Arg Met Leu Ser Asp Leu Thr Leu Val Gly Cys
                   245                 250                 255
   Tyr Asn Leu Ser Val Met Pro Glu Lys Gln Arg Asn Lys Val Leu Leu
               260                 265                 270
   Glu Ser Ala Lys Ser Asn Leu Lys His Met Ala Phe Phe Gly Leu Thr
           275                 280                 285
   Glu Phe Gln Arg Lys Thr Gln Tyr Leu Phe Glu Lys Thr Phe Asn Met
       290                 295                 300
   Asn Phe Ile Ser Pro Phe Thr Gln Tyr Asn Thr Thr Arg Ala Ser Ser
   305                 310                 315                 320
   Val Glu Ile Asn Glu Glu Ile Gln Lys Arg Ile Glu Gly Leu Asn Phe
                   325                 330                 335
   Leu Asp Met Glu Leu Tyr Ser Tyr Ala Lys Asp Leu Phe Leu Gln Arg
               340                 345                 350
   Tyr Gln Phe Met Arg Gln Lys Glu His Gln Glu Ala Arg Arg Lys Arg
           355                 360                 365
   Gln Glu Gln Arg Lys Phe Leu Lys Gly Arg Leu Leu Gln Thr His Phe
       370                 375                 380
   Gln Ser Gln Gly Gln Gly Gln Ser Gln Asn Pro Asn Gln Asn Gln Ser
   385                 390                 395                 400
   Gln Asn Pro Asn Pro Asn Ala Asn Gln Asn Leu Thr Gln Asn Leu Met
                   405                 410                 415
   Gln Asn Leu Thr Gln Ser Leu Ser Gln Lys Glu Asn Arg Glu Ser Pro
               420                 425                 430
   Lys Gln Asn Ser Gly Lys Glu Gln Asn Asp Asn Thr Ser Asn Gly Thr
           435                 440                 445
   Asn Asp Tyr Ile Gly Ser Val Glu Lys Trp Arg
       450                 455
<210> 89
   <211> 798
   <212> PRT
   <213> homo sapiens
<400> 89
   Met Glu Ala Gly Glu Gly Lys Glu Arg Val Pro Lys Gln Arg Gln Val
   1               5                   10                  15
   Leu Ile Phe Phe Val Leu Leu Gly Ile Ala Gln Ala Ser Cys Gln Pro
               20                  25                  30
   Arg His Tyr Ser Val Ala Glu Glu Thr Glu Ser Gly Ser Phe Val Ala
           35                  40                  45
   Asn Leu Leu Lys Asp Leu Gly Leu Glu Ile Gly Glu Leu Ala Val Arg
       50                  55                  60
   Gly Ala Arg Val Val Ser Lys Gly Lys Lys Met His Leu Gln Phe Asp
   65                  70                  75                  80
   Arg Gln Thr Gly Asp Leu Leu Leu Asn Glu Lys Leu Asp Arg Glu Glu
                   85                  90                  95
   Leu Cys Gly Pro Thr Glu Pro Cys Val Leu Pro Phe Gln Val Leu Leu
               100                 105                 110
   Glu Asn Pro Leu Gln Phe Phe Gln Ala Glu Leu Arg Ile Arg Asp Val
           115                 120                 125
   Asn Asp His Ser Pro Val Phe Leu Asp Lys Glu Ile Leu Leu Lys Ile
       130                 135                 140
   Pro Glu Ser Ile Thr Pro Gly Thr Thr Phe Leu Ile Glu Arg Ala Gln
   145                 150                 155                 160
   Asp Leu Asp Val Gly Thr Asn Ser Leu Gln Asn Tyr Thr Ile Ser Pro
                   165                 170                 175
   Asn Phe His Phe His Leu Asn Leu Gln Asp Ser Leu Asp Gly Ile Ile
               180                 185                 190
   Leu Pro Gln Leu Val Leu Asn Arg Ala Leu Asp Arg Glu Glu Gln Pro
           195                 200                 205
   Glu Ile Arg Leu Thr Leu Thr Ala Leu Asp Gly Gly Ser Pro Pro Arg
   210 215 220
   Ser Gly Thr Ala Leu Val Arg Ile Glu Val Val Asp Ile Asn Asp Asn
   225 230 235 240
   Val Pro Glu Phe Ala Lys Leu Leu Tyr Glu Val Gln Ile Pro Glu Asp
   245 250 255
   Ser Pro Val Gly Ser Gln Val Ala Ile Val Ser Ala Arg Asp Leu Asp
   260 265 270
   Ile Gly Thr Asn Gly Glu Ile Ser Tyr Ala Phe Ser Gln Ala Ser Glu
   275 280 285
   Asp Ile Arg Lys Thr Phe Arg Leu Ser Ala Lys Ser Gly Glu Leu Leu
   290 295 300
   Leu Arg Gln Lys Leu Asp Phe Glu Ser Ile Gln Thr Tyr Thr Val Asn
   305 310 315 320
   Ile Gln Ala Thr Asp Gly Gly Gly Leu Ser Gly Thr Cys Val Val Phe
   325 330 335
   Val Gln Val Met Asp Leu Asn Asp Asn Pro Pro Glu Leu Thr Met Ser
   340 345 350
   Thr Leu Ile Asn Gln Ile Pro Glu Asn Leu Gln Asp Thr Leu Ile Ala
   355 360 365
   Val Phe Ser Val Ser Asp Pro Asp Ser Gly Asp Asn Gly Arg Met Val
   370 375 380
   Cys Ser Ile Gln Asp Asp Leu Pro Phe Phe Leu Lys Pro Ser Val Glu
   385 390 395 400
   Asn Phe Tyr Thr Leu Val Ile Ser Thr Ala Leu Asp Arg Glu Thr Arg
   405 410 415
   Ser Glu Tyr Asn Ile Thr Ile Thr Val Thr Asp Phe Gly Thr Pro Arg
   420 425 430
   Leu Lys Thr Glu His Asn Ile Thr Val Leu Val Ser Asp Val Asn Asp
   435 440 445
   Asn Ala Pro Ala Phe Thr Gln Thr Ser Tyr Thr Leu Phe Val Arg Glu
   450 455 460
   Asn Asn Ser Pro Ala Leu His Ile Gly Ser Val Ser Ala Thr Asp Arg
   465 470 475 480
   Asp Ser Gly Thr Asn Ala Gln Val Thr Tyr Ser Leu Leu Pro Pro Gln
   485 490 495
   Asp Pro His Leu Pro Leu Ala Ser Leu Val Ser Ile Asn Ala Asp Asn
   500 505 510
   Gly His Leu Phe Ala Leu Gln Ser Leu Asp Tyr Glu Ala Leu Gln Ala
   515 520 525
   Phe Glu Phe Arg Val Gly Ala Ala Asp Arg Gly Ser Pro Ala Leu Ser
   530 535 540
   Ser Glu Ala Leu Val Arg Val Leu Val Leu Asp Ala Asn Asp Asn Ser
   545 550 555 560
   Pro Phe Val Leu Tyr Pro Leu Gln Asn Gly Ser Ala Pro Cys Thr Glu
   565 570 575
   Leu Val Pro Arg Ala Ala Glu Pro Gly Tyr Leu Val Thr Lys Val Val
   580 585 590
   Ala Val Asp Gly Asp Ser Gly Gln Asn Ala Trp Leu Ser Tyr Gln Leu
   595 600 605
   Leu Lys Ala Thr Glu Pro Gly Leu Phe Gly Val Trp Ala His Asn Gly
   610 615 620
   Glu Val Arg Thr Ala Arg Leu Leu Arg Glu Arg Asp Ala Ala Lys Gln
   625 630 635 640
   Arg Leu Val Val Leu Val Lys Asp Asn Gly Glu Pro Pro Arg Ser Ala
   645 650 655
   Thr Ala Thr Leu His Val Leu Leu Val Asp Gly Phe Ser Gln Pro Tyr
   660 665 670
   Leu Leu Leu Pro Glu Ala Ala Pro Ala Gln Ala Gln Ala Asp Leu Leu
   675 680 685
   Thr Val Tyr Leu Val Val Ala Leu Ala Ser Val Ser Ser Leu Phe Leu
   690 695 700
   Phe Ser Val Leu Leu Phe Val Ala Val Arg Leu Cys Arg Arg Ser Arg
   705                 710                 715                 720
   Ala Ala Ser Val Gly Arg Cys Ser Val Pro Glu Gly Pro Phe Pro Gly
                   725                 730                 735
   Gln Met Val Asp Val Ser Gly Thr Gly Thr Leu Ser Gln Ser Tyr Gln
               740                 745                 750
   Tyr Glu Val Cys Leu Thr Gly Gly Ser Gly Thr Asn Glu Phe Lys Phe
           755                 760                 765
   Leu Lys Pro Ile Ile Pro Asn Phe Val Ala Gln Gly Ala Glu Arg Val
       770                 775                 780
   Ser Glu Ala Asn Pro Ser Phe Arg Lys Ser Phe Glu Phe Thr
   785                 790                 795
<210> 90
   <211> 231
   <212> PRT
   <213> homo sapiens
<400> 90
   Met Met Leu Ser Cys Leu Phe Leu Leu Lys Ala Leu Leu Ala Leu Gly
   1               5                   10                  15
   Ser Leu Glu Ser Trp Ile Thr Ala Gly Glu His Ala Lys Glu Gly Glu
               20                  25                  30
   Cys Pro Pro His Lys Asn Pro Cys Lys Glu Leu Cys Gln Gly Asp Glu
           35                  40                  45
   Leu Cys Pro Ala Glu Gln Lys Cys Cys Thr Thr Gly Cys Gly Arg Ile
       50                  55                  60
   Cys Arg Asp Ile Pro Lys Gly Arg Lys Arg Asp Cys Pro Arg Val Ile
   65                  70                  75                  80
   Arg Lys Gln Ser Cys Leu Lys Arg Cys Ile Thr Asp Glu Thr Cys Pro
                   85                  90                  95
   Gly Val Lys Lys Cys Cys Thr Leu Gly Cys Asn Lys Ser Cys Val Val
               100                 105                 110
   Pro Ile Ser Lys Gln Lys Leu Ala Glu Phe Gly Gly Glu Cys Pro Ala
           115                 120                 125
   Asp Pro Leu Pro Cys Glu Glu Leu Cys Asp Gly Asp Ala Ser Cys Pro
       130                 135                 140
   Gln Gly His Lys Cys Cys Ser Thr Gly Cys Gly Arg Thr Cys Leu Gly
   145                 150                 155                 160
   Asp Ile Glu Gly Gly Arg Gly Gly Asp Cys Pro Lys Val Leu Val Gly
                   165                 170                 175
   Leu Cys Ile Val Gly Cys Val Met Asp Glu Asn Cys Gln Ala Gly Glu
               180                 185                 190
   Lys Cys Cys Lys Ser Gly Cys Gly Arg Phe Cys Val Pro Pro Val Leu
           195                 200                 205
   Pro Pro Lys Leu Thr Met Asn Pro Asn Trp Thr Val Arg Ser Asp Ser
       210                 215                 220
   Glu Leu Glu Ile Pro Val Pro
   225                 230
<210> 91
   <211> 677
   <212> PRT
   <213> homo sapiens
<400> 91
   Met Leu Ser Ser Thr Asp Phe Thr Phe Ala Ser Trp Glu Leu Val Val
   1               5                   10                  15
   Arg Val Asp His Pro Asn Glu Glu Gln Gln Lys Asp Val Thr Leu Arg
               20                  25                  30
   Val Ser Gly Asp Leu His Val Gly Gly Val Met Leu Lys Leu Val Glu
           35                  40                  45
   Gln Ile Asn Ile Ser Gln Asp Trp Ser Asp Phe Ala Leu Trp Trp Glu
       50                  55                  60
   Gln Lys His Cys Trp Leu Leu Lys Thr His Trp Thr Leu Asp Lys Tyr
   65                  70                  75                  80
   Gly Val Gln Ala Asp Ala Lys Leu Leu Phe Thr Pro Gln His Lys Met
                   85                  90                  95
   Leu Arg Leu Arg Leu Pro Asn Leu Lys Met Val Arg Leu Arg Val Ser
               100                 105                 110
   Phe Ser Ala Val Val Phe Lys Ala Val Ser Asp Ile Cys Lys Ile Leu
           115                 120                 125
   Asn Ile Arg Arg Ser Glu Glu Leu Ser Leu Leu Lys Pro Ser Gly Asp
       130                 135                 140
   Tyr Phe Lys Lys Lys Lys Lys Lys Asp Lys Asn Asn Lys Glu Pro Ile
   145                 150                 155                 160
   Ile Glu Asp Ile Leu Asn Leu Glu Ser Ser Pro Thr Ala Ser Gly Ser
                   165                 170                 175
   Ser Val Ser Pro Gly Leu Tyr Ser Lys Thr Met Thr Pro Ile Tyr Asp
               180                 185                 190
   Pro Ile Asn Gly Thr Pro Ala Ser Ser Thr Met Thr Trp Phe Ser Asp
           195                 200                 205
   Ser Pro Leu Thr Glu Gln Asn Cys Ser Ile Leu Ala Phe Ser Gln Pro
       210                 215                 220
   Pro Gln Ser Pro Glu Ala Leu Ala Asp Met Tyr Gln Pro Arg Ser Leu
   225                 230                 235                 240
   Val Asp Lys Ala Lys Leu Asn Ala Gly Trp Leu Asp Ser Ser Arg Ser
                   245                 250                 255
   Leu Met Glu Gln Gly Ile Gln Glu Asp Glu Gln Leu Leu Leu Arg Phe
               260                 265                 270
   Lys Tyr Tyr Ser Phe Phe Asp Leu Asn Pro Lys Tyr Asp Ala Val Arg
           275                 280                 285
   Ile Asn Gln Leu Tyr Glu Gln Ala Arg Trp Ala Ile Leu Leu Glu Glu
       290                 295                 300
   Ile Asp Cys Thr Glu Glu Glu Met Leu Ile Phe Ala Ala Leu Gln Tyr
   305                 310                 315                 320
   His Ile Ser Lys Leu Ser Leu Ser Ala Glu Thr Gln Asp Phe Ala Gly
                   325                 330                 335
   Glu Ser Glu Val Asp Glu Ile Glu Ala Ala Leu Ser Asn Leu Glu Val
               340                 345                 350
   Thr Leu Glu Gly Gly Lys Ala Asp Ser Leu Leu Glu Asp Ile Thr Asp
           355                 360                 365
   Ile Pro Lys Leu Ala Asp Asn Leu Lys Leu Phe Arg Pro Lys Lys Leu
       370                 375                 380
   Leu Pro Lys Ala Phe Lys Gln Tyr Trp Phe Ile Phe Lys Asp Thr Ser
   385                 390                 395                 400
   Ile Ala Tyr Phe Lys Asn Lys Glu Leu Glu Gln Gly Glu Pro Leu Glu
                   405                 410                 415
   Lys Leu Asn Leu Arg Gly Cys Glu Val Val Pro Asp Val Asn Val Ala
               420                 425                 430
   Gly Arg Lys Phe Gly Ile Lys Leu Leu Ile Pro Val Ala Asp Gly Met
           435                 440                 445
   Asn Glu Met Tyr Leu Arg Cys Asp His Glu Asn Gln Tyr Ala Gln Trp
       450                 455                 460
   Met Ala Ala Cys Met Leu Ala Ser Lys Gly Lys Thr Met Ala Asp Ser
   465                 470                 475                 480
   Ser Tyr Gln Pro Glu Val Leu Asn Ile Leu Ser Phe Leu Arg Met Lys
                   485                 490                 495
   Asn Arg Asn Ser Ala Ser Gln Val Ala Ser Ser Leu Glu Asn Met Asp
               500                 505                 510
   Met Asn Pro Glu Cys Phe Val Ser Pro Arg Cys Ala Lys Lys His Lys
           515                 520                 525
   Ser Lys Gln Leu Ala Ala Arg Ile Leu Glu Ala His Gln Asn Val Ala
       530                 535                 540
   Gln Met Pro Leu Val Glu Ala Lys Leu Arg Phe Ile Gln Ala Trp Gln
   545                 550                 555                 560
   Ser Leu Pro Glu Phe Gly Leu Thr Tyr Tyr Leu Val Arg Phe Lys Gly
                   565                 570                 575
   Ser Lys Lys Asp Asp Ile Leu Gly Val Ser Tyr Asn Arg Leu Ile Lys
               580                 585                 590
   Ile Asp Ala Ala Thr Gly Ile Pro Val Thr Thr Trp Arg Phe Thr Asn
           595                 600                 605
   Ile Lys Gln Trp Asn Val Asn Trp Glu Thr Arg Gln Val Val Ile Glu
       610                 615                 620
   Phe Asp Gln Asn Val Phe Thr Ala Phe Thr Cys Leu Ser Ala Asp Cys
   625                 630                 635                 640
   Lys Ile Val His Glu Tyr Ile Gly Gly Tyr Ile Phe Leu Ser Thr Arg
                   645                 650                 655
   Ser Lys Asp Gln Asn Glu Thr Leu Asp Glu Asp Leu Phe His Lys Leu
               660                 665                 670
   Thr Gly Gly Gln Asp
           675
<210> 92
   <211> 764
   <212> PRT
   <213> homo sapiens
<400> 92
   Met Leu Leu Phe Val Leu Thr Cys Leu Leu Ala Val Phe Pro Ala Ile
   1               5                   10                  15
   Ser Thr Lys Ser Pro Ile Phe Gly Pro Glu Glu Val Asn Ser Val Glu
               20                  25                  30
   Gly Asn Ser Val Ser Ile Thr Cys Tyr Tyr Pro Pro Thr Ser Val Asn
           35                  40                  45
   Arg His Thr Arg Lys Tyr Trp Cys Arg Gln Gly Ala Arg Gly Gly Cys
       50                  55                  60
   Ile Thr Leu Ile Ser Ser Glu Gly Tyr Val Ser Ser Lys Tyr Ala Gly
   65                  70                  75                  80
   Arg Ala Asn Leu Thr Asn Phe Pro Glu Asn Gly Thr Phe Val Val Asn
                   85                  90                  95
   Ile Ala Gln Leu Ser Gln Asp Asp Ser Gly Arg Tyr Lys Cys Gly Leu
               100                 105                 110
   Gly Ile Asn Ser Arg Gly Leu Ser Phe Asp Val Ser Leu Glu Val Ser
           115                 120                 125
   Gln Gly Pro Gly Leu Leu Asn Asp Thr Lys Val Tyr Thr Val Asp Leu
       130                 135                 140
   Gly Arg Thr Val Thr Ile Asn Cys Pro Phe Lys Thr Glu Asn Ala Gln
   145                 150                 155                 160
   Lys Arg Lys Ser Leu Tyr Lys Gln Ile Gly Leu Tyr Pro Val Leu Val
                   165                 170                 175
   Ile Asp Ser Ser Gly Tyr Val Asn Pro Asn Tyr Thr Gly Arg Ile Arg
               180                 185                 190
   Leu Asp Ile Gln Gly Thr Gly Gln Leu Leu Phe Ser Val Val Ile Asn
           195                 200                 205
   Gln Leu Arg Leu Ser Asp Ala Gly Gln Tyr Leu Cys Gln Ala Gly Asp
       210                 215                 220
   Asp Ser Asn Ser Asn Lys Lys Asn Ala Asp Leu Gln Val Leu Lys Pro
   225                 230                 235                 240
   Glu Pro Glu Leu Val Tyr Glu Asp Leu Arg Gly Ser Val Thr Phe His
                   245                 250                 255
   Cys Ala Leu Gly Pro Glu Val Ala Asn Val Ala Lys Phe Leu Cys Arg
               260                 265                 270
   Gln Ser Ser Gly Glu Asn Cys Asp Val Val Val Asn Thr Leu Gly Lys
           275                 280                 285
   Arg Ala Pro Ala Phe Glu Gly Arg Ile Leu Leu Asn Pro Gln Asp Lys
       290                 295                 300
   Asp Gly Ser Phe Ser Val Val Ile Thr Gly Leu Arg Lys Glu Asp Ala
   305                 310                 315                 320
   Gly Arg Tyr Leu Cys Gly Ala His Ser Asp Gly Gln Leu Gln Glu Gly
                   325                 330                 335
   Ser Pro Ile Gln Ala Trp Gln Leu Phe Val Asn Glu Glu Ser Thr Ile
               340                 345                 350
   Pro Arg Ser Pro Thr Val Val Lys Gly Val Ala Gly Gly Ser Val Ala
           355                 360                 365
   Val Leu Cys Pro Tyr Asn Arg Lys Glu Ser Lys Ser Ile Lys Tyr Trp
           370                 375                 380
   Cys Leu Trp Glu Gly Ala Gln Asn Gly Arg Cys Pro Leu Leu Val Asp
   385                 390                 395                 400
   Ser Glu Gly Trp Val Lys Ala Gln Tyr Glu Gly Arg Leu Ser Leu Leu
                   405                 410                 415
   Glu Glu Pro Gly Asn Gly Thr Phe Thr Val Ile Leu Asn Gln Leu Thr
               420                 425                 430
   Ser Arg Asp Ala Gly Phe Tyr Trp Cys Leu Thr Asn Gly Asp Thr Leu
           435                 440                 445
   Trp Arg Thr Thr Val Glu Ile Lys Ile Ile Glu Gly Glu Pro Asn Leu
       450                 455                 460
   Lys Val Pro Gly Asn Val Thr Ala Val Leu Gly Glu Thr Leu Lys Val
   465                 470                 475                 480
   Pro Cys His Phe Pro Cys Lys Phe Ser Ser Tyr Glu Lys Tyr Trp Cys
                   485                 490                 495
   Lys Trp Asn Asn Thr Gly Cys Gln Ala Leu Pro Ser Gln Asp Glu Gly
               500                 505                 510
   Pro Ser Lys Ala Phe Val Asn Cys Asp Glu Asn Ser Arg Leu Val Ser
           515                 520                 525
   Leu Thr Leu Asn Leu Val Thr Arg Ala Asp Glu Gly Trp Tyr Trp Cys
       530                 535                 540
   Gly Val Lys Gln Gly His Phe Tyr Gly Glu Thr Ala Ala Val Tyr Val
   545                 550                 555                 560
   Ala Val Glu Glu Arg Lys Ala Ala Gly Ser Arg Asp Val Ser Leu Ala
                   565                 570                 575
   Lys Ala Asp Ala Ala Pro Asp Glu Lys Val Leu Asp Ser Gly Phe Arg
               580                 585                 590
   Glu Ile Glu Asn Lys Ala Ile Gln Asp Pro Arg Leu Phe Ala Glu Glu
           595                 600                 605
   Lys Ala Val Ala Asp Thr Arg Asp Gln Ala Asp Gly Ser Arg Ala Ser
       610                 615                 620
   Val Asp Ser Gly Ser Ser Glu Glu Gln Gly Gly Ser Ser Arg Ala Leu
   625                 630                 635                 640
   Val Ser Thr Leu Val Pro Leu Gly Leu Val Leu Ala Val Gly Ala Val
                   645                 650                 655
   Ala Val Gly Val Ala Arg Ala Arg His Arg Lys Asn Val Asp Arg Val
               660                 665                 670
   Ser Ile Arg Ser Tyr Arg Thr Asp Ile Ser Met Ser Asp Phe Glu Asn
           675                 680                 685
   Ser Arg Glu Phe Gly Ala Asn Asp Asn Met Gly Ala Ser Ser Ile Thr
       690                 695                 700
   Gln Glu Thr Ser Leu Gly Gly Lys Glu Glu Phe Val Ala Thr Thr Glu
   705                 710                 715                 720
   Ser Thr Thr Glu Thr Lys Glu Pro Lys Lys Ala Lys Arg Ser Ser Lys
                   725                 730                 735
   Glu Glu Ala Glu Met Ala Tyr Lys Asp Phe Leu Leu Gln Ser Ser Thr
               740                 745                 750
   Val Ala Ala Glu Ala Gln Asp Gly Pro Gln Glu Ala
           755                 760

<210> 93
   <211> 694
   <212> PRT
   <213> homo sapiens
<400> 93
   Met Lys His Leu Lys Arg Trp Trp Ser Ala Gly Gly Gly Leu Leu His
   1               5                   10                  15
   Leu Thr Leu Leu Leu Ser Leu Ala Gly Leu Arg Val Asp Leu Asp Leu
               20                  25                  30
   Tyr Leu Leu Leu Pro Pro Pro Thr Leu Leu Gln Asp Glu Leu Leu Phe
           35                  40                  45
   Leu Gly Gly Pro Ala Ser Ser Ala Tyr Ala Leu Ser Pro Phe Ser Ala
       50                  55                  60
   Ser Gly Gly Trp Gly Arg Ala Gly His Leu His Pro Lys Gly Arg Glu
   65                  70                  75                  80
   Leu Asp Pro Ala Ala Pro Pro Glu Gly Gln Leu Leu Arg Glu Val Arg
                   85                  90                  95
   Ala Leu Gly Val Pro Phe Val Pro Arg Thr Ser Val Asp Ala Trp Leu
               100                 105                 110
   Val His Ser Val Ala Ala Gly Ser Ala Asp Glu Ala His Gly Leu Leu
           115                 120                 125
   Gly Ala Ala Ala Ala Ser Ser Thr Gly Gly Ala Gly Ala Ser Val Asp
       130                 135                 140
   Gly Gly Ser Gln Ala Val Gln Gly Gly Gly Gly Asp Pro Arg Ala Ala
   145                 150                 155                 160
   Arg Ser Gly Pro Leu Asp Ala Gly Glu Glu Glu Lys Ala Pro Ala Glu
                   165                 170                 175
   Pro Thr Ala Gln Val Pro Asp Ala Gly Gly Cys Ala Ser Glu Glu Asn
               180                 185                 190
   Gly Val Leu Arg Glu Lys His Glu Ala Val Asp His Ser Ser Gln His
           195                 200                 205
   Glu Glu Asn Glu Glu Arg Val Ser Ala Gln Lys Glu Asn Ser Leu Gln
       210                 215                 220
   Gln Asn Asp Asp Asp Glu Asn Lys Ile Ala Glu Lys Pro Asp Trp Glu
   225                 230                 235                 240
   Ala Glu Lys Thr Thr Glu Ser Arg Asn Glu Arg His Leu Asn Gly Thr
                   245                 250                 255
   Asp Thr Ser Phe Ser Leu Glu Asp Leu Phe Gln Leu Leu Ser Ser Gln
               260                 265                 270
   Pro Glu Asn Ser Leu Glu Gly Ile Ser Leu Gly Asp Ile Pro Leu Pro
           275                 280                 285
   Gly Ser Ile Ser Asp Gly Met Asn Ser Ser Ala His Tyr His Val Asn
       290                 295                 300
   Phe Ser Gln Ala Ile Ser Gln Asp Val Asn Leu His Glu Ala Ile Leu
   305                 310                 315                 320
   Leu Cys Pro Asn Asn Thr Phe Arg Arg Asp Pro Thr Ala Arg Thr Ser
                   325                 330                 335
   Gln Ser Gln Glu Pro Phe Leu Gln Leu Asn Ser His Thr Thr Asn Pro
               340                 345                 350
   Glu Gln Thr Leu Pro Gly Thr Asn Leu Thr Gly Phe Leu Ser Pro Val
           355                 360                 365
   Asp Asn His Met Arg Asn Leu Thr Ser Gln Asp Leu Leu Tyr Asp Leu
       370                 375                 380
   Asp Ile Asn Ile Phe Asp Glu Ile Asn Leu Met Ser Leu Ala Thr Glu
   385                 390                 395                 400
   Asp Asn Phe Asp Pro Ile Asp Val Ser Gln Leu Phe Asp Glu Pro Asp
                   405                 410                 415
   Ser Asp Ser Gly Leu Ser Leu Asp Ser Ser His Asn Asn Thr Ser Val
               420                 425                 430
   Ile Lys Ser Asn Ser Ser His Ser Val Cys Asp Glu Gly Ala Ile Gly
           435                 440                 445
   Tyr Cys Thr Asp His Glu Ser Ser Ser His His Asp Leu Glu Gly Ala
       450                 455                 460
   Val Gly Gly Tyr Tyr Pro Glu Pro Ser Lys Leu Cys His Leu Asp Gln
   465                 470                 475                 480
   Ser Asp Ser Asp Phe His Gly Asp Leu Thr Phe Gln His Val Phe His
                   485                 490                 495
   Asn His Thr Tyr His Leu Gln Pro Thr Ala Pro Glu Ser Thr Ser Glu
               500                 505                 510
   Pro Phe Pro Trp Pro Gly Lys Ser Gln Lys Ile Arg Ser Arg Tyr Leu
           515                 520                 525
   Glu Asp Thr Asp Arg Asn Leu Ser Arg Asp Glu Gln Arg Ala Lys Ala
       530                 535                 540
   Leu His Ile Pro Phe Ser Val Asp Glu Ile Val Gly Met Pro Val Asp
   545                 550                 555                 560
   Ser Phe Asn Ser Met Leu Ser Arg Tyr Tyr Leu Thr Asp Leu Gln Val
                   565                 570                 575
   Ser Leu Ile Arg Asp Ile Arg Arg Arg Gly Lys Asn Lys Val Ala Ala
               580                 585                 590
   Gln Asn Cys Arg Lys Arg Lys Leu Asp Ile Ile Leu Asn Leu Glu Asp
           595                 600                 605
   Asp Val Cys Asn Leu Gln Ala Lys Lys Glu Thr Leu Lys Arg Glu Gln
       610                 615                 620
   Ala Gln Cys Asn Lys Ala Ile Asn Ile Met Lys Gln Lys Leu His Asp
   625                 630                 635                 640
   Leu Tyr His Asp Ile Phe Ser Arg Leu Arg Asp Asp Gln Gly Arg Pro
                   645                 650                 655
   Val Asn Pro Asn His Tyr Ala Leu Gln Cys Thr His Asp Gly Ser Ile
               660                 665                 670
   Leu Ile Val Pro Lys Glu Leu Val Ala Ser Gly His Lys Lys Glu Thr
           675                 680                 685
   Gln Lys Gly Lys Arg Lys
       690
<210> 94
   <211> 110
   <212> PRT
   <213> homo sapiens
<400> 94
   Met Trp Met Gly Leu Ile Gln Leu Val Glu Gly Val Lys Arg Lys Asp
   1               5                   10                  15
   Gln Gly Phe Leu Glu Lys Glu Phe Tyr His Lys Thr Asn Ile Lys Met
               20                  25                  30
   His Cys Glu Phe His Ala Cys Trp Pro Ala Phe Thr Val Leu Gly Glu
           35                  40                  45
   Ala Trp Arg Asp Gln Val Asp Trp Ser Ile Leu Leu Arg Asp Ala Gly
       50                  55                  60
   Leu Val Lys Met Ser Arg Lys Pro Arg Ala Ser Ser Pro Leu Ser Asn
   65                  70                  75                  80
   Asn His Pro Pro Thr Pro Lys Arg Phe Pro Arg Gln Leu Gly Arg Glu
                   85                  90                  95
   Lys Gly Pro Ile Glu Glu Val Pro Gly Thr Lys Gly Ser Pro
               100                 105                 110
<210> 95
   <211> 425
   <212> PRT
   <213> homo sapiens
<400> 95
   Met Ala Ser Gly Gln Phe Val Asn Lys Leu Gln Glu Glu Val Ile Cys
   1               5                   10                  15
   Pro Ile Cys Leu Asp Ile Leu Gln Lys Pro Val Thr Ile Asp Cys Gly
               20                  25                  30
   His Asn Phe Cys Leu Lys Cys Ile Thr Gln Ile Gly Glu Thr Ser Cys
           35                  40                  45
   Gly Phe Phe Lys Cys Pro Leu Cys Lys Thr Ser Val Arg Arg Asp Ala
       50                  55                  60
   Ile Arg Phe Asn Ser Leu Leu Arg Asn Leu Val Glu Lys Ile Gln Ala
   65                  70                  75                  80
   Leu Gln Ala Ser Glu Val Gln Ser Lys Arg Lys Glu Ala Thr Cys Pro
                   85                  90                  95
   Arg His Gln Glu Met Phe His Tyr Phe Cys Glu Asp Asp Gly Lys Phe
               100                 105                 110
   Leu Cys Phe Val Cys Arg Glu Ser Lys Asp His Lys Ser His Asn Val
           115                 120                 125
   Ser Leu Ile Glu Glu Ala Ala Gln Asn Tyr Gln Gly Gln Ile Gln Glu
       130                 135                 140
   Gln Ile Gln Val Leu Gln Gln Lys Glu Lys Glu Thr Val Gln Val Lys
   145                 150                 155                 160
   Ala Gln Gly Val His Arg Val Asp Val Phe Thr Asp Gln Val Glu His
                   165                 170                 175
   Glu Lys Gln Arg Ile Leu Thr Glu Phe Glu Leu Leu His Gln Val Leu
               180                 185                 190
   Glu Glu Glu Lys Asn Phe Leu Leu Ser Arg Ile Tyr Trp Leu Gly His
           195                 200                 205
   Glu Gly Thr Glu Ala Gly Lys His Tyr Val Ala Ser Thr Glu Pro Gln
       210                 215                 220
   Leu Asn Asp Leu Lys Lys Leu Val Asp Ser Leu Lys Thr Lys Gln Asn
   225                 230                 235                 240
   Met Pro Pro Arg Gln Leu Leu Glu Asp Ile Lys Val Val Leu Cys Arg
                   245                 250                 255
   Ser Glu Glu Phe Gln Phe Leu Asn Pro Thr Pro Val Pro Leu Glu Leu
               260                 265                 270
   Glu Lys Lys Leu Ser Glu Ala Lys Ser Arg His Asp Ser Ile Thr Gly
           275                 280                 285
   Ser Leu Lys Lys Phe Lys Asp Gln Leu Gln Ala Asp Arg Lys Lys Asp
       290                 295                 300
   Glu Asn Arg Phe Phe Lys Ser Met Asn Lys Asn Asp Met Lys Ser Trp
   305                 310                 315                 320
   Gly Leu Leu Gln Lys Asn Asn His Lys Met Asn Lys Thr Ser Glu Pro
                   325                 330                 335
   Gly Ser Ser Ser Ala Gly Gly Arg Thr Thr Ser Gly Pro Pro Asn His
               340                 345                 350
   His Ser Ser Ala Pro Ser His Ser Leu Phe Arg Ala Ser Ser Ala Gly
           355                 360                 365
   Lys Val Thr Phe Pro Val Cys Leu Leu Ala Ser Tyr Asp Glu Ile Ser
       370                 375                 380
   Gly Gln Gly Ala Ser Ser Gln Asp Thr Lys Thr Phe Asp Val Ala Leu
   385                 390                 395                 400
   Ser Glu Glu Leu His Ala Ala Leu Ser Glu Trp Leu Thr Ala Ile Arg
                   405                 410                 415
   Ala Trp Phe Cys Glu Val Pro Ser Ser
               420                 425
<210> 96
   <211> 429
   <212> PRT
   <213> homo sapiens
<400> 96
   Met Thr Thr Asp Asp Thr Glu Val Pro Ala Met Thr Leu Ala Pro Gly
   1               5                   10                  15
   His Ala Ala Leu Glu Thr Gln Thr Leu Ser Ala Glu Thr Ser Ser Arg
               20                  25                  30
   Ala Ser Thr Pro Ala Gly Pro Ile Pro Glu Ala Glu Thr Arg Gly Ala
           35                  40                  45
   Lys Arg Ile Ser Pro Ala Arg Glu Thr Arg Ser Phe Thr Lys Thr Ser
       50                  55                  60
   Pro Asn Phe Met Val Leu Ile Ala Thr Ser Val Glu Thr Ser Ala Ala
   65                  70                  75                  80
   Ser Gly Ser Pro Glu Gly Ala Gly Met Thr Thr Val Gln Thr Ile Thr
                   85                  90                  95
   Gly Ser Asp Pro Arg Glu Ala Ile Phe Asp Thr Leu Cys Thr Asp Asp
               100                 105                 110
   Ile Ser Glu Glu Ala Lys Thr Leu Thr Met Asp Ile Leu Thr Leu Ala
           115                 120                 125
   His Thr Ser Thr Glu Ala Lys Gly Leu Ser Ser Glu Ser Ser Ala Ser
       130                 135                 140
   Ser Asp Gly Pro His Pro Val Ile Thr Pro Ser Arg Ala Ser Glu Ser
   145                 150                 155                 160
   Ser Ala Ser Ser Asp Gly Leu His Pro Val Ile Thr Pro Ser Arg Ala
                   165                 170                 175
   Ser Glu Ser Ser Ala Ser Ser Asp Gly Leu His Pro Val Ile Thr Pro
               180                 185                 190
   Ser Arg Ala Ser Glu Ser Ser Ala Ser Ser Asp Gly Pro His Pro Val
           195                 200                 205
   Ile Thr Pro Ser Trp Ser Pro Gly Ser Asp Val Thr Leu Leu Ala Glu
       210                 215                 220
   Ala Leu Val Thr Val Thr Asn Ile Glu Val Ile Asn Cys Ser Ile Thr
   225                 230                 235                 240
   Glu Ile Glu Thr Thr Thr Ser Ser Ile Pro Gly Ala Ser Asp Thr Asp
                   245                 250                 255
   Leu Ile Pro Thr Glu Gly Val Lys Ala Ser Ser Thr Ser Asp Pro Pro
               260                 265                 270
   Ala Leu Pro Asp Ser Thr Asn Thr Lys Pro His Ile Thr Glu Val Thr
           275                 280                 285
   Ala Ser Ala Glu Thr Leu Ser Thr Ala Gly Thr Thr Glu Ser Ala Ala
       290                 295                 300
   Pro Asp Ala Thr Ile Gly Thr Pro Leu Pro Thr Asn Ser Thr Ile Glu
   305                 310                 315                 320
   Arg Glu Val Thr Ala Pro Gly Ala Thr Thr Leu Ser Gly Ala Leu Ala
                   325                 330                 335
   Thr Gly Asn Pro Leu Glu Glu Thr Ser Ala Leu Ser Val Glu Thr Pro
               340                 345                 350
   Ser Tyr Val Lys Val Ser Gly Ala Ala Pro Val Ser Ile Glu Ala Gly
           355                 360                 365
   Ser Ala Val Gly Lys Thr Thr Ser Phe Ala Gly Ser Ser Ala Ser Ser
       370                 375                 380
   Tyr Ser Pro Leu Glu Ala Ala Leu Lys Asn Phe Thr Pro Ser Glu Thr
   385                 390                 395                 400
   Leu Thr Thr Asp Ile Ala Thr Lys Gly Pro Phe Pro Thr Ser Arg Ala
                   405                 410                 415
   Pro Leu Pro Ser Val Pro Pro Thr Thr Thr Asn Ser Ser
               420                 425
<210> 97
   <211> 165
   <212> PRT
   <213> homo sapiens
<400> 97
   Met Ala Pro Asn Ala Ser Cys Leu Cys Val His Val Arg Ser Glu Glu
   1               5                   10                  15
   Trp Asp Leu Met Thr Phe Asp Ala Asn Pro Tyr Asp Ser Val Lys Lys
               20                  25                  30
   Ile Lys Glu His Val Arg Ser Lys Thr Lys Val Pro Val Gln Asp Gln
           35                  40                  45
   Val Leu Leu Leu Gly Ser Lys Ile Leu Lys Pro Arg Arg Ser Leu Ser
       50                  55                  60
   Ser Tyr Gly Ile Asp Lys Glu Lys Thr Ile His Leu Thr Leu Lys Val
   65                  70                  75                  80
   Val Lys Pro Ser Asp Glu Glu Leu Pro Leu Phe Leu Val Glu Ser Gly
                   85                  90                  95
   Asp Glu Ala Lys Arg His Leu Leu Gln Val Arg Arg Ser Ser Ser Val
               100                 105                 110
   Ala Gln Val Lys Ala Met Ile Glu Thr Lys Thr Gly Ile Ile Pro Glu
           115                 120                 125
   Thr Gln Ile Val Thr Cys Asn Gly Lys Arg Leu Glu Asp Gly Lys Met
       130                 135                 140
   Met Ala Asp Tyr Gly Ile Arg Lys Gly Asn Leu Leu Phe Leu Ala Ser
   145                 150                 155                 160
   Tyr Cys Ile Gly Gly
                   165
<210> 98
   <211> 891
   <212> PRT
   <213> homo sapiens
<400> 98
   Met His Ala Ile Asn Gly Phe Val Phe Gly Asn Leu Pro Glu Leu Asn
   1               5                   10                  15
   Met Cys Ala Gln Lys Arg Val Ala Trp His Leu Phe Gly Met Gly Asn
               20                  25                  30
   Glu Ile Asp Val His Thr Ala Phe Phe His Gly Gln Met Leu Thr Thr
               35                  40                  45
   Arg Gly His His Thr Asp Val Ala Asn Ile Phe Pro Ala Thr Phe Val
       50                  55                  60
   Thr Ala Glu Met Val Pro Trp Glu Pro Gly Thr Trp Leu Ile Ser Cys
   65                  70                  75                  80
   Gln Val Asn Ser His Phe Arg Asp Gly Met Gln Ala Leu Tyr Lys Val
                   85                  90                  95
   Lys Ser Cys Ser Met Ala Pro Pro Val Asp Leu Leu Thr Gly Lys Val
               100                 105                 110
   Arg Gln Tyr Phe Ile Glu Ala His Glu Ile Gln Trp Asp Tyr Gly Pro
           115                 120                 125
   Met Gly His Asp Gly Ser Thr Gly Lys Asn Leu Arg Glu Pro Gly Ser
       130                 135                 140
   Ile Ser Asp Lys Phe Phe Gln Lys Ser Ser Ser Arg Ile Gly Gly Thr
   145                 150                 155                 160
   Tyr Trp Lys Val Arg Tyr Glu Ala Phe Gln Asp Glu Thr Phe Gln Glu
                   165                 170                 175
   Lys Met His Leu Glu Glu Asp Arg His Leu Gly Ile Leu Gly Pro Val
               180                 185                 190
   Ile Arg Ala Glu Val Gly Asp Thr Ile Gln Val Val Phe Tyr Asn Arg
           195                 200                 205
   Ala Ser Gln Pro Phe Ser Met Gln Pro His Gly Val Phe Tyr Glu Lys
       210                 215                 220
   Asp Tyr Glu Gly Thr Val Tyr Asn Asp Gly Ser Ser Tyr Pro Gly Leu
   225                 230                 235                 240
   Val Ala Lys Pro Phe Glu Lys Val Thr Tyr Arg Trp Thr Val Pro Pro
                   245                 250                 255
   His Ala Gly Pro Thr Ala Gln Asp Pro Ala Cys Leu Thr Trp Met Tyr
               260                 265                 270
   Phe Ser Ala Ala Asp Pro Ile Arg Asp Thr Asn Ser Gly Leu Val Gly
           275                 280                 285
   Pro Leu Leu Val Cys Arg Ala Gly Ala Leu Gly Ala Asp Gly Lys Gln
       290                 295                 300
   Lys Gly Val Asp Lys Glu Phe Phe Leu Leu Phe Thr Val Leu Asp Glu
   305                 310                 315                 320
   Asn Lys Ser Trp Tyr Ser Asn Ala Asn Gln Ala Ala Ala Met Leu Asp
                   325                 330                 335
   Phe Arg Leu Leu Ser Glu Asp Ile Glu Gly Phe Gln Asp Ser Asn Arg
               340                 345                 350
   Met His Ala Ile Asn Gly Phe Leu Phe Ser Asn Leu Pro Arg Leu Asp
           355                 360                 365
   Met Cys Lys Gly Asp Thr Val Ala Trp His Leu Leu Gly Leu Gly Thr
       370                 375                 380
   Glu Thr Asp Val His Gly Val Met Phe Gln Gly Asn Thr Val Gln Leu
   385                 390                 395                 400
   Gln Gly Met Arg Lys Gly Ala Ala Met Leu Phe Pro His Thr Phe Val
                   405                 410                 415
   Met Ala Ile Met Gln Pro Asp Asn Leu Gly Thr Phe Glu Ile Tyr Cys
               420                 425                 430
   Gln Ala Gly Ser His Arg Glu Ala Gly Met Arg Ala Ile Tyr Asn Val
           435                 440                 445
   Ser Gln Cys Pro Gly His Gln Ala Thr Pro Arg Gln Arg Tyr Gln Ala
       450                 455                 460
   Ala Arg Ile Tyr Tyr Ile Met Ala Glu Glu Val Glu Trp Asp Tyr Cys
   465                 470                 475                 480
   Pro Asp Arg Ser Trp Glu Arg Glu Trp His Asn Gln Ser Glu Lys Asp
                   485                 490                 495
   Ser Tyr Gly Tyr Ile Phe Leu Ser Asn Lys Asp Gly Leu Leu Gly Ser
               500                 505                 510
   Arg Tyr Lys Lys Ala Val Phe Arg Glu Tyr Thr Asp Gly Thr Phe Arg
           515                 520                 525
   Ile Pro Arg Pro Arg Thr Gly Pro Glu Glu His Leu Gly Ile Leu Gly
       530                 535                 540
   Pro Leu Ile Lys Gly Glu Val Gly Asp Ile Leu Thr Val Val Phe Lys
   545                 550                 555                 560
   Asn Asn Ala Ser Arg Pro Tyr Ser Val His Ala His Gly Val Leu Glu
                   565                 570                 575
   Ser Thr Thr Val Trp Pro Leu Ala Ala Glu Pro Gly Glu Val Val Thr
               580                 585                 590
   Tyr Gln Trp Asn Ile Pro Glu Arg Ser Gly Pro Gly Pro Asn Asp Ser
           595                 600                 605
   Ala Cys Val Ser Trp Ile Tyr Tyr Ser Ala Val Asp Pro Ile Lys Asp
       610                 615                 620
   Met Tyr Ser Gly Leu Val Gly Pro Leu Ala Ile Cys Gln Lys Gly Ile
   625                 630                 635                 640
   Leu Glu Pro His Gly Gly Arg Ser Asp Met Asp Arg Glu Phe Ala Leu
                   645                 650                 655
   Leu Phe Leu Ile Phe Asp Glu Asn Lys Ser Trp Tyr Leu Glu Glu Asn
               660                 665                 670
   Val Ala Thr His Gly Ser Gln Asp Pro Gly Ser Ile Asn Leu Gln Asp
           675                 680                 685
   Glu Thr Phe Leu Glu Ser Asn Lys Met His Ala Ile Asn Gly Lys Leu
       690                 695                 700
   Tyr Ala Asn Leu Arg Gly Leu Thr Met Tyr Gln Gly Glu Arg Val Ala
   705                 710                 715                 720
   Trp Tyr Met Leu Ala Met Gly Gln Asp Val Asp Leu His Thr Ile His
                   725                 730                 735
   Phe His Ala Glu Ser Phe Leu Tyr Arg Asn Gly Glu Asn Tyr Arg Ala
               740                 745                 750
   Asp Val Val Asp Leu Phe Pro Gly Thr Phe Glu Val Val Glu Met Val
           755                 760                 765
   Ala Ser Asn Pro Gly Thr Trp Leu Met His Cys His Val Thr Asp His
       770                 775                 780
   Val His Ala Gly Met Glu Thr Leu Phe Thr Val Phe Ser Arg Thr Glu
   785                 790                 795                 800
   His Leu Ser Pro Leu Thr Val Ile Thr Lys Glu Thr Glu Lys Ala Val
                   805                 810                 815
   Pro Pro Arg Asp Ile Glu Glu Gly Asn Val Lys Met Leu Gly Met Gln
               820                 825                 830
   Ile Pro Ile Lys Asn Val Glu Met Leu Ala Ser Val Leu Val Ala Ile
           835                 840                 845
   Ser Val Thr Leu Leu Leu Val Val Leu Ala Leu Gly Gly Val Val Trp
       850                 855                 860
   Tyr Gln His Arg Gln Arg Lys Leu Arg Arg Asn Arg Arg Ser Ile Leu
   865                 870                 875                 880
   Asp Asp Ser Phe Lys Leu Leu Ser Phe Lys Gln
                   885                 890
<210> 99
   <211> 292
   <212> PRT
   <213> homo sapiens
<400> 99
   Met Leu Gly Ala Trp Ala Val Glu Gly Thr Ala Val Ala Leu Leu Arg
   1               5                   10                  15
   Leu Leu Leu Leu Leu Leu Pro Pro Ala Ile Arg Gly Pro Gly Leu Gly
               20                  25                  30
   Val Ala Gly Val Ala Gly Ala Ala Gly Ala Gly Leu Pro Glu Ser Val
           35                  40                  45
   Ile Trp Ala Val Asn Ala Gly Gly Glu Ala His Val Asp Val His Gly
       50                  55                  60
   Ile His Phe Arg Lys Asp Pro Leu Glu Gly Arg Val Gly Arg Ala Ser
   65                  70                  75                  80
   Asp Tyr Gly Met Lys Leu Pro Ile Leu Arg Ser Asn Pro Glu Asp Gln
                   85                  90                  95
   Ile Leu Tyr Gln Thr Glu Arg Tyr Asn Glu Glu Thr Phe Gly Tyr Glu
               100                 105                 110
   Val Pro Ile Lys Glu Glu Gly Asp Tyr Val Leu Val Leu Lys Phe Ala
           115                 120                 125
   Glu Val Tyr Phe Ala Gln Ser Gln Gln Lys Val Phe Asp Val Arg Leu
       130                 135                 140
   Asn Gly His Val Val Val Lys Asp Leu Asp Ile Phe Asp Arg Val Gly
   145                 150                 155                 160
   His Ser Thr Ala His Asp Glu Ile Ile Pro Met Ser Ile Arg Lys Gly
                   165                 170                 175
   Lys Leu Ser Val Gln Gly Glu Val Ser Thr Phe Thr Gly Lys Leu Tyr
               180                 185                 190
   Ile Glu Phe Val Lys Gly Tyr Tyr Asp Asn Pro Lys Val Cys Ala Leu
           195                 200                 205
   Tyr Ile Met Ala Gly Thr Val Asp Asp Val Pro Lys Leu Gln Pro His
       210                 215                 220
   Pro Gly Leu Glu Lys Lys Glu Glu Glu Glu Glu Glu Glu Glu Tyr Asp
   225                 230                 235                 240
   Glu Gly Ser Asn Leu Lys Lys Gln Thr Asn Lys Asn Arg Val Gln Ser
                   245                 250                 255
   Gly Pro Arg Thr Pro Asn Pro Tyr Ala Ser Asp Asn Ser Ser Leu Met
               260                 265                 270
   Phe Pro Ile Leu Val Ala Phe Gly Val Phe Ile Pro Thr Leu Phe Cys
           275                 280                 285
   Leu Cys Arg Leu
       290
<210> 100
   <211> 963
   <212> PRT
   <213> homo sapiens
<400> 100
   Met Leu His Phe His Leu Leu Lys Phe Lys Thr Arg Val Ile Phe Ser
   1               5                   10                  15
   Ala Val Ile Ile Met Val Thr Gly Leu Cys Leu Phe Leu Leu Ser Leu
               20                  25                  30
   Pro His Leu His Gly Val Phe Glu Gln Val Pro Ala Pro Trp Trp Thr
           35                  40                  45
   Ser Leu Cys Pro Trp Pro Ile Met Glu Ala Ala Ala Phe Gln Ser Gly
       50                  55                  60
   Ser Leu Tyr Pro Val Ala Ser Phe Leu Ala Ala Pro Met Ser Glu Leu
   65                  70                  75                  80
   Val Pro Asp Leu Ser Phe Gln Val Asp Leu His Thr Gly Leu Ser Glu
                   85                  90                  95
   Phe Ser Val Thr Gln Arg Arg Leu Ala His Gly Trp Asn Glu Phe Val
               100                 105                 110
   Ala Asp Asn Ser Glu Pro Val Trp Lys Lys Tyr Leu Asp Gln Phe Lys
           115                 120                 125
   Asn Pro Leu Ile Leu Leu Leu Leu Gly Ser Ala Leu Val Ser Val Leu
       130                 135                 140
   Thr Lys Glu Tyr Glu Asp Ala Val Ser Ile Ala Thr Ala Val Leu Val
   145                 150                 155                 160
   Val Val Thr Val Ala Phe Ile Gln Glu Tyr Arg Ser Glu Lys Ser Leu
                   165                 170                 175
   Glu Glu Leu Thr Lys Leu Val Pro Pro Glu Cys Asn Cys Leu Arg Glu
               180                 185                 190
   Gly Lys Leu Gln His Leu Leu Ala Arg Glu Leu Val Pro Gly Asp Val
           195                 200                 205
   Val Ser Leu Ser Ile Gly Asp Arg Ile Pro Ala Asp Ile Arg Leu Thr
       210                 215                 220
   Glu Val Thr Asp Leu Leu Val Asp Glu Ser Ser Phe Thr Gly Glu Ala
   225                 230                 235                 240
   Glu Pro Cys Ser Lys Thr Asp Ser Pro Leu Thr Gly Gly Gly Asp Leu
                   245                 250                 255
   Thr Thr Leu Ser Asn Ile Val Phe Met Gly Thr Leu Val Gln Tyr Gly
               260                 265                 270
   Arg Gly Gln Gly Val Val Ile Gly Thr Gly Glu Ser Ser Gln Phe Gly
           275                 280                 285
   Glu Val Phe Lys Met Met Gln Ala Glu Glu Thr Pro Lys Thr Pro Leu
       290                 295                 300
   Gln Lys Ser Met Asp Arg Leu Gly Lys Gln Leu Thr Leu Phe Ser Phe
   305                 310                 315                 320
   Gly Ile Ile Gly Leu Ile Met Leu Ile Gly Trp Ser Gln Gly Lys Gln
                   325                 330                 335
   Leu Leu Ser Met Phe Thr Ile Gly Val Ser Leu Ala Val Ala Ala Ile
               340                 345                 350
   Pro Glu Gly Leu Pro Ile Val Val Met Val Thr Leu Val Leu Gly Val
           355                 360                 365
   Leu Arg Met Ala Lys Lys Arg Val Ile Val Lys Lys Leu Pro Ile Val
       370                 375                 380
   Glu Thr Leu Gly Cys Cys Ser Val Leu Cys Ser Asp Lys Thr Gly Thr
   385                 390                 395                 400
   Leu Thr Ala Asn Glu Met Thr Val Thr Gln Leu Val Thr Ser Asp Gly
                   405                 410                 415
   Leu Arg Ala Glu Val Ser Gly Val Gly Tyr Asp Gly Gln Gly Thr Val
               420                 425                 430
   Cys Leu Leu Pro Ser Lys Glu Val Ile Lys Glu Phe Ser Asn Val Ser
           435                 440                 445
   Val Gly Lys Leu Val Glu Ala Gly Cys Val Ala Asn Asn Ala Val Ile
       450                 455                 460
   Arg Lys Asn Ala Val Met Gly Gln Pro Thr Glu Gly Ala Leu Met Ala
   465                 470                 475                 480
   Leu Ala Met Lys Met Asp Leu Ser Asp Ile Lys Asn Ser Tyr Ile Arg
                   485                 490                 495
   Lys Lys Glu Ile Pro Phe Ser Ser Glu Gln Lys Trp Met Ala Val Lys
               500                 505                 510
   Cys Ser Leu Lys Thr Glu Asp Gln Glu Asp Ile Tyr Phe Met Lys Gly
           515                 520                 525
   Ala Leu Glu Glu Val Ile Arg Tyr Cys Thr Met Tyr Asn Asn Gly Gly
       530                 535                 540
   Ile Pro Leu Pro Leu Thr Pro Gln Gln Arg Ser Phe Cys Leu Gln Glu
   545                 550                 555                 560
   Glu Lys Arg Met Gly Ser Leu Gly Leu Arg Val Leu Ala Leu Ala Ser
                   565                 570                 575
   Gly Pro Glu Leu Gly Arg Leu Thr Phe Leu Gly Leu Val Gly Ile Ile
               580                 585                 590
   Asp Pro Pro Arg Val Gly Val Lys Glu Ala Val Gln Val Leu Ser Glu
           595                 600                 605
   Ser Gly Val Ser Val Lys Met Ile Thr Gly Asp Ala Leu Glu Thr Ala
       610                 615                 620
   Leu Ala Ile Gly Arg Asn Ile Gly Leu Cys Asn Gly Lys Leu Gln Ala
   625                 630                 635                 640
   Met Ser Gly Glu Glu Val Asp Ser Val Glu Lys Gly Glu Leu Ala Asp
                   645                 650                 655
   Arg Val Gly Lys Val Ser Val Phe Phe Arg Thr Ser Pro Lys His Lys
               660                 665                 670
   Leu Lys Ile Ile Lys Ala Leu Gln Glu Ser Gly Ala Ile Val Ala Met
           675                 680                 685
   Thr Gly Asp Gly Val Asn Asp Ala Val Ala Leu Lys Ser Ala Asp Ile
       690                 695                 700
   Gly Ile Ala Met Gly Gln Thr Gly Thr Asp Val Ser Lys Glu Ala Ala
   705                 710                 715                 720
   Asn Met Ile Leu Val Asp Asp Asp Phe Ser Ala Ile Met Asn Ala Val
                   725                 730                 735
   Glu Glu Gly Lys Gly Ile Phe Tyr Asn Ile Lys Asn Phe Val Arg Phe
               740                 745                 750
   Gln Leu Ser Thr Ser Ile Ser Ala Leu Ser Leu Ile Thr Leu Ser Thr
           755                 760                 765
   Val Phe Asn Leu Pro Ser Pro Leu Asn Ala Met Gln Ile Leu Trp Ile
       770                 775                 780
   Asn Ile Ile Met Asp Gly Pro Pro Ala Gln Ser Leu Gly Val Glu Pro
   785                 790                 795                 800
   Val Asp Lys Asp Ala Phe Arg Gln Pro Pro Arg Ser Val Arg Asp Thr
                   805                 810                 815
   Ile Leu Ser Arg Ala Leu Ile Leu Lys Ile Leu Met Ser Ala Ala Ile
               820                 825                 830
   Ile Ile Ser Gly Thr Leu Phe Ile Phe Trp Lys Glu Met Pro Glu Asp
           835                 840                 845
   Arg Ala Ser Thr Pro Arg Thr Thr Thr Met Thr Phe Thr Cys Phe Val
       850                 855                 860
   Phe Phe Asp Leu Phe Asn Ala Leu Thr Cys Arg Ser Gln Thr Lys Leu
   865                 870                 875                 880
   Ile Phe Glu Ile Gly Phe Leu Arg Asn His Met Phe Leu Tyr Ser Val
                   885                 890                 895
   Leu Gly Ser Ile Leu Gly Gln Leu Ala Val Ile Tyr Ile Pro Pro Leu
               900                 905                 910
   Gln Arg Val Phe Gln Thr Glu Asn Leu Gly Ala Leu Asp Leu Leu Phe
           915                 920                 925
   Leu Thr Gly Leu Ala Ser Ser Val Phe Ile Leu Ser Glu Leu Leu Lys
       930                 935                 940
   Leu Cys Glu Lys Tyr Cys Cys Ser Pro Lys Arg Val Gln Met His Pro
   945                 950                 955                 960
   Glu Asp Val
<210> 101
   <211> 335
   <212> PRT
   <213> homo sapiens
<400> 101
<210> 102
   <211> 992
   <212> PRT
   <213> homo sapiens
<400> 102
   Met Gly Ala Ala Gly Arg Gln Asp Phe Leu Phe Lys Ala Met Leu Thr
   1               5                   10                  15
   Ile Ser Trp Leu Thr Leu Thr Cys Phe Pro Gly Ala Thr Ser Thr Val
               20                  25                  30
   Ala Ala Gly Cys Pro Asp Gln Ser Pro Glu Leu Gln Pro Trp Asn Pro
           35                  40                  45
   Gly His Asp Gln Asp His His Val His Ile Gly Gln Gly Lys Thr Leu
       50                  55                  60
   Leu Leu Thr Ser Ser Ala Thr Val Tyr Ser Ile His Ile Ser Glu Gly
   65                  70                  75                  80
   Gly Lys Leu Val Ile Lys Asp His Asp Glu Pro Ile Val Leu Arg Thr
                   85                  90                  95
   Arg His Ile Leu Ile Asp Asn Gly Gly Glu Leu His Ala Gly Ser Ala
               100                 105                 110
   Leu Cys Pro Phe Gln Gly Asn Phe Thr Ile Ile Leu Tyr Gly Arg Ala
           115                 120                 125
   Asp Glu Gly Ile Gln Pro Asp Pro Tyr Tyr Gly Leu Lys Tyr Ile Gly
       130                 135                 140
   Val Gly Lys Gly Gly Ala Leu Glu Leu His Gly Gln Lys Lys Leu Ser
   145                 150                 155                 160
   Trp Thr Phe Leu Asn Lys Thr Leu His Pro Gly Gly Met Ala Glu Gly
                   165                 170                 175
   Gly Tyr Phe Phe Glu Arg Ser Trp Gly His Arg Gly Val Ile Val His
               180                 185                 190
   Val Ile Asp Pro Lys Ser Gly Thr Val Ile His Ser Asp Arg Phe Asp
           195                 200                 205
   Thr Tyr Arg Ser Lys Lys Glu Ser Glu Arg Leu Val Gln Tyr Leu Asn
       210                 215                 220
   Ala Val Pro Asp Gly Arg Ile Leu Ser Val Ala Val Asn Asp Glu Gly
   225                 230                 235                 240
   Ser Arg Asn Leu Asp Asp Met Ala Arg Lys Ala Met Thr Lys Leu Gly
                   245                 250                 255
   Ser Lys His Phe Leu His Leu Gly Phe Arg His Pro Trp Ser Phe Leu
               260                 265                 270
   Thr Val Lys Gly Asn Pro Ser Ser Ser Val Glu Asp His Ile Glu Tyr
           275                 280                 285
   His Gly His Arg Gly Ser Ala Ala Ala Arg Val Phe Lys Leu Phe Gln
       290                 295                 300
   Thr Glu His Gly Glu Tyr Phe Asn Val Ser Leu Ser Ser Glu Trp Val
   305                 310                 315                 320
   Gln Asp Val Glu Trp Thr Glu Trp Phe Asp His Asp Lys Val Ser Gln
                   325                 330                 335
   Thr Lys Gly Gly Glu Lys Ile Ser Asp Leu Trp Lys Ala His Pro Gly
               340                 345                 350
   Lys Ile Cys Asn Arg Pro Ile Asp Ile Gln Ala Thr Thr Met Asp Gly
           355                 360                 365
   Val Asn Leu Ser Thr Glu Val Val Tyr Lys Lys Gly Gln Asp Tyr Arg
       370                 375                 380
   Phe Ala Cys Tyr Asp Arg Gly Arg Ala Cys Arg Ser Tyr Arg Val Arg
   385 390 395 400
   Phe Leu Cys Gly Lys Pro Val Arg Pro Lys Leu Thr Val Thr Ile Asp
   405 410 415
   Thr Asn Val Asn Ser Thr Ile Leu Asn Leu Glu Asp Asn Val Gln Ser
   420 425 430
   Trp Lys Pro Gly Asp Thr Leu Val Ile Ala Ser Thr Asp Tyr Ser Met
   435 440 445
   Tyr Gln Ala Glu Glu Phe Gln Val Leu Pro Cys Arg Ser Cys Ala Pro
   450 455 460
   Asn Gln Val Lys Val Ala Gly Lys Pro Met Tyr Leu His Ile Gly Glu
   465 470 475 480
   Glu Ile Asp Gly Val Asp Met Arg Ala Glu Val Gly Leu Leu Ser Arg
   485 490 495
   Asn Ile Ile Val Met Gly Glu Met Glu Asp Lys Cys Tyr Pro Tyr Arg
   500 505 510
   Asn His Ile Cys Asn Phe Phe Asp Phe Asp Thr Phe Gly Gly His Ile
   515 520 525
   Lys Phe Ala Leu Gly Phe Lys Ala Ala His Leu Glu Gly Thr Glu Leu
   530 535 540
   Lys His Met Gly Gln Gln Leu Val Gly Gln Tyr Pro Ile His Phe His
   545 550 555 560
   Leu Ala Gly Asp Val Asp Glu Arg Gly Gly Tyr Asp Pro Pro Thr Tyr
   565 570 575
   Ile Arg Asp Leu Ser Ile His His Thr Phe Ser Arg Cys Val Thr Val
   580 585 590
   His Gly Ser Asn Gly Leu Leu Ile Lys Asp Val Val Gly Tyr Asn Ser
   595 600 605
   Leu Gly His Cys Phe Phe Thr Glu Asp Gly Pro Glu Glu Arg Asn Thr
   610 615 620
   Phe Asp His Cys Leu Gly Leu Leu Val Lys Ser Gly Thr Leu Leu Pro
   625 630 635 640
   Ser Asp Arg Asp Ser Lys Met Cys Lys Met Ile Thr Glu Asp Ser Tyr
   645 650 655
   Pro Gly Tyr Ile Pro Lys Pro Arg Gln Asp Cys Asn Ala Val Ser Thr
   660 665 670
   Phe Trp Met Ala Asn Pro Asn Asn Asn Leu Ile Asn Cys Ala Ala Ala
   675 680 685
   Gly Ser Glu Glu Thr Gly Phe Trp Phe Ile Phe His His Val Pro Thr
   690 695 700
   Gly Pro Ser Val Gly Met Tyr Ser Pro Gly Tyr Ser Glu His Ile Pro
   705 710 715 720
   Leu Gly Lys Phe Tyr Asn Asn Arg Ala His Ser Asn Tyr Arg Ala Gly
   725 730 735
   Met Ile Ile Asp Asn Gly Val Lys Thr Thr Glu Ala Ser Ala Lys Asp
   740 745 750
   Lys Arg Pro Phe Leu Ser Ile Ile Ser Ala Arg Tyr Ser Pro His Gln
   755 760 765
   Asp Ala Asp Pro Leu Lys Pro Arg Glu Pro Ala Ile Ile Arg His Phe
   770 775 780
   Ile Ala Tyr Lys Asn Gln Asp His Gly Ala Trp Leu Arg Gly Gly Asp
   785 790 795 800
   Val Trp Leu Asp Ser Cys Arg Phe Ala Asp Asn Gly Ile Gly Leu Thr
   805 810 815
   Leu Ala Ser Gly Gly Thr Phe Pro Tyr Asp Asp Gly Ser Lys Gln Glu
   820 825 830
   Ile Lys Asn Ser Leu Phe Val Gly Glu Ser Gly Asn Val Gly Thr Glu
   835 840 845
   Met Met Asp Asn Arg Ile Trp Gly Pro Gly Gly Leu Asp His Ser Gly
   850 855 860
   Arg Thr Leu Pro Ile Gly Gln Asn Phe Pro Ile Arg Gly Ile Gln Leu
   865 870 875 880
   Tyr Asp Gly Pro Ile Asn Ile Gln Asn Cys Thr Phe Arg Lys Phe Val
                       885                 890                 895
   Ala Leu Glu Gly Arg His Thr Ser Ala Leu Ala Phe Arg Leu Asn Asn
                   900                 905                 910
   Ala Trp Gln Ser Cys Pro His Asn Asn Val Thr Gly Ile Ala Phe Glu
               915                 920                 925
   Asp Val Pro Ile Thr Ser Arg Val Phe Phe Gly Glu Pro Gly Pro Trp
       930                 935                 940
   Phe Asn Gln Leu Asp Met Asp Gly Asp Lys Thr Ser Val Phe His Asp
   945                 950                 955                 960
   Val Asp Gly Ser Val Ser Glu Tyr Pro Gly Ser Tyr Leu Thr Lys Asn
                   965                 970                 975
   Asp Asn Trp His Ser Leu Ala Ser Lys Ala Ala Ser Gly Pro Ser Gly
               980                 985                 990
<210> 103
   <211> 296
   <212> PRT
   <213> homo sapiens
<400> 103
   Met Glu His Leu Lys Ala Phe Asp Asp Glu Ile Asn Ala Phe Leu Asp
   1               5                   10                  15
   Asn Met Phe Gly Pro Arg Asp Ser Arg Val Arg Gly Trp Phe Thr Leu
               20                  25                  30
   Asp Ser Tyr Leu Pro Thr Phe Phe Leu Thr Val Met Tyr Leu Leu Ser
           35                  40                  45
   Ile Trp Leu Gly Asn Lys Tyr Met Lys Asn Arg Pro Ala Leu Ser Leu
       50                  55                  60
   Arg Gly Ile Leu Thr Leu Tyr Asn Leu Gly Ile Thr Leu Leu Ser Ala
   65                  70                  75                  80
   Tyr Met Leu Ala Glu Leu Ile Leu Ser Thr Trp Glu Gly Gly Tyr Asn
                   85                  90                  95
   Leu Gln Cys Gln Asp Leu Thr Ser Ala Gly Glu Ala Asp Ile Arg Val
               100                 105                 110
   Ala Lys Val Leu Trp Trp Tyr Tyr Phe Ser Lys Ser Val Glu Phe Leu
           115                 120                 125
   Asp Thr Ile Phe Phe Val Leu Arg Lys Lys Thr Ser Gln Ile Thr Phe
       130                 135                 140
   Leu His Val Tyr His His Ala Ser Met Phe Asn Ile Trp Trp Cys Val
   145                 150                 155                 160
   Leu Asn Trp Ile Pro Cys Gly Gln Ser Phe Phe Gly Pro Thr Leu Asn
                   165                 170                 175
   Ser Phe Val His Ile Leu Met Tyr Ser Tyr Tyr Gly Leu Ser Val Phe
               180                 185                 190
   Pro Ser Met His Lys Tyr Leu Trp Trp Lys Lys Tyr Leu Thr Gln Ala
           195                 200                 205
   Gln Leu Val Gln Phe Val Leu Thr Ile Thr His Thr Met Ser Ala Val
       210                 215                 220
   Val Lys Pro Cys Gly Phe Pro Phe Gly Cys Leu Ile Phe Gln Ser Ser
   225                 230                 235                 240
   Tyr Met Leu Thr Leu Val Ile Leu Phe Leu Asn Phe Tyr Val Gln Thr
                   245                 250                 255
   Tyr Arg Lys Lys Pro Met Lys Lys Asp Met Gln Glu Pro Pro Ala Gly
               260                 265                 270
   Lys Glu Val Lys Asn Gly Phe Ser Lys Ala Tyr Phe Thr Ala Ala Asn
           275                 280                 285
   Gly Val Met Asn Lys Lys Ala Gln
       290                 295
<210> 104
   <211> 1612
   <212> PRT
   <213> homo sapiens
<400> 104
   Met Ile Ala Glu Pro Ala His Phe Tyr Leu Phe Gly Leu Ile Cys Leu
   1               5                   10                  15
   Cys Ser Gly Ser Arg Leu Arg Gln Glu Asp Phe Pro Pro Arg Ile Val
               20                  25                  30
   Glu His Pro Ser Asp Leu Ile Val Ser Lys Gly Glu Pro Ala Thr Leu
           35                  40                  45
   Asn Cys Lys Ala Glu Gly Arg Pro Thr Pro Thr Ile Glu Trp Tyr Lys
       50                  55                  60
   Gly Gly Glu Arg Val Glu Thr Asp Lys Asp Asp Pro Arg Ser His Arg
   65                  70                  75                  80
   Met Leu Leu Pro Ser Gly Ser Leu Phe Phe Leu Arg Ile Val His Gly
                   85                  90                  95
   Arg Lys Ser Arg Pro Asp Glu Gly Val Tyr Val Cys Val Ala Arg Asn
               100                 105                 110
   Tyr Leu Gly Glu Ala Val Ser His Asn Ala Ser Leu Glu Val Ala Ile
           115                 120                 125
   Leu Arg Asp Asp Phe Arg Gln Asn Pro Ser Asp Val Met Val Ala Val
       130                 135                 140
   Gly Glu Pro Ala Val Met Glu Cys Gln Pro Pro Arg Gly His Pro Glu
   145                 150                 155                 160
   Pro Thr Ile Ser Trp Lys Lys Asp Gly Ser Pro Leu Asp Asp Lys Asp
                   165                 170                 175
   Glu Arg Ile Thr Ile Arg Gly Gly Lys Leu Met Ile Thr Tyr Thr Arg
               180                 185                 190
   Lys Ser Asp Ala Gly Lys Tyr Val Cys Val Gly Thr Asn Met Val Gly
           195                 200                 205
   Glu Arg Glu Ser Glu Val Ala Glu Leu Thr Val Leu Glu Arg Pro Ser
       210                 215                 220
   Phe Val Lys Arg Pro Ser Asn Leu Ala Val Thr Val Asp Asp Ser Ala
   225                 230                 235                 240
   Glu Phe Lys Cys Glu Ala Arg Gly Asp Pro Val Pro Thr Val Arg Trp
                   245                 250                 255
   Arg Lys Asp Asp Gly Glu Leu Pro Lys Ser Arg Tyr Glu Ile Arg Asp
               260                 265                 270
   Asp His Thr Leu Lys Ile Arg Lys Val Thr Ala Gly Asp Met Gly Ser
           275                 280                 285
   Tyr Thr Cys Val Ala Glu Asn Met Val Gly Lys Ala Glu Ala Ser Ala
       290                 295                 300
   Thr Leu Thr Val Gln Glu Pro Pro His Phe Val Val Lys Pro Arg Asp
   305                 310                 315                 320
   Gln Val Val Ala Leu Gly Arg Thr Val Thr Phe Gln Cys Glu Ala Thr
                   325                 330                 335
   Gly Asn Pro Gln Pro Ala Ile Phe Trp Arg Arg Glu Gly Ser Gln Asn
               340                 345                 350
   Leu Leu Phe Ser Tyr Gln Pro Pro Gln Ser Ser Ser Arg Phe Ser Val
           355                 360                 365
   Ser Gln Thr Gly Asp Leu Thr Ile Thr Asn Val Gln Arg Ser Asp Val
       370                 375                 380
   Gly Tyr Tyr Ile Cys Gln Thr Leu Asn Val Ala Gly Ser Ile Ile Thr
   385                 390                 395                 400
   Lys Ala Tyr Leu Glu Val Thr Asp Val Ile Ala Asp Arg Pro Pro Pro
                   405                 410                 415
   Val Ile Arg Gln Gly Pro Val Asn Gln Thr Val Ala Val Asp Gly Thr
               420                 425                 430
   Phe Val Leu Ser Cys Val Ala Thr Gly Ser Pro Val Pro Thr Ile Leu
           435                 440                 445
   Trp Arg Lys Asp Gly Val Leu Val Ser Thr Gln Asp Ser Arg Ile Lys
       450                 455                 460
   Gln Leu Glu Asn Gly Val Leu Gln Ile Arg Tyr Ala Lys Leu Gly Asp
   465                 470                 475                 480
   Thr Gly Arg Tyr Thr Cys Ile Ala Ser Thr Pro Ser Gly Glu Ala Thr
                   485                 490                 495
   Trp Ser Ala Tyr Ile Glu Val Gln Glu Phe Gly Val Pro Val Gln Pro
               500                 505                 510
   Pro Arg Pro Thr Asp Pro Asn Leu Ile Pro Ser Ala Pro Ser Lys Pro
           515                 520                 525
   Glu Val Thr Asp Val Ser Arg Asn Thr Val Thr Leu Ser Trp Gln Pro
       530                 535                 540
   Asn Leu Asn Ser Gly Ala Thr Pro Thr Ser Tyr Ile Ile Glu Ala Phe
   545                 550                 555                 560
   Ser His Ala Ser Gly Ser Ser Trp Gln Thr Val Ala Glu Asn Val Lys
                   565                 570                 575
   Thr Glu Thr Ser Ala Ile Lys Gly Leu Lys Pro Asn Ala Ile Tyr Leu
               580                 585                 590
   Phe Leu Val Arg Ala Ala Asn Ala Tyr Gly Ile Ser Asp Pro Ser Gln
           595                 600                 605
   Ile Ser Asp Pro Val Lys Thr Gln Asp Val Leu Pro Thr Ser Gln Gly
       610                 615                 620
   Val Asp His Lys Gln Val Gln Arg Glu Leu Gly Asn Ala Val Leu His
   625                 630                 635                 640
   Leu His Asn Pro Thr Val Leu Ser Ser Ser Ser Ile Glu Val His Trp
                   645                 650                 655
   Thr Val Asp Gln Gln Ser Gln Tyr Ile Gln Gly Tyr Lys Ile Leu Tyr
               660                 665                 670
   Arg Pro Ser Gly Ala Asn His Gly Glu Ser Asp Trp Leu Val Phe Glu
           675                 680                 685
   Val Arg Thr Pro Ala Lys Asn Ser Val Val Ile Pro Asp Leu Arg Lys
       690                 695                 700
   Gly Val Asn Tyr Glu Ile Lys Ala Arg Pro Phe Phe Asn Glu Phe Gln
   705                 710                 715                 720
   Gly Ala Asp Ser Glu Ile Lys Phe Ala Lys Thr Leu Glu Glu Ala Pro
                   725                 730                 735
   Ser Ala Pro Pro Gln Gly Val Thr Val Ser Lys Asn Asp Gly Asn Gly
               740                 745                 750
   Thr Ala Ile Leu Val Ser Trp Gln Pro Pro Pro Glu Asp Thr Gln Asn
           755                 760                 765
   Gly Met Val Gln Glu Tyr Lys Val Trp Cys Leu Gly Asn Glu Thr Arg
       770                 775                 780
   Tyr His Ile Asn Lys Thr Val Asp Gly Ser Thr Phe Ser Val Val Ile
   785                 790                 795                 800
   Pro Phe Leu Val Pro Gly Ile Arg Tyr Ser Val Glu Val Ala Ala Ser
                   805                 810                 815
   Thr Gly Ala Gly Ser Gly Val Lys Ser Glu Pro Gln Phe Ile Gln Leu
               820                 825                 830
   Asp Ala His Gly Asn Pro Val Ser Pro Glu Asp Gln Val Ser Leu Ala
           835                 840                 845
   Gln Gln Ile Ser Asp Val Val Lys Gln Pro Ala Phe Ile Ala Gly Ile
       850                 855                 860
   Gly Ala Ala Cys Trp Ile Ile Leu Met Val Phe Ser Ile Trp Leu Tyr
   865                 870                 875                 880
   Arg His Arg Lys Lys Arg Asn Gly Leu Thr Ser Thr Tyr Ala Gly Ile
                   885                 890                 895
   Arg Lys Val Pro Ser Phe Thr Phe Thr Pro Thr Val Thr Tyr Gln Arg
               900                 905                 910
   Gly Gly Glu Ala Val Ser Ser Gly Gly Arg Pro Gly Leu Leu Asn Ile
           915                 920                 925
   Ser Glu Pro Ala Ala Gln Pro Trp Leu Ala Asp Thr Trp Pro Asn Thr
       930                 935                 940
   Gly Asn Asn His Asn Asp Cys Ser Ile Ser Cys Cys Thr Ala Gly Asn
   945                 950                 955                 960
   Gly Asn Ser Asp Ser Asn Leu Thr Thr Tyr Ser Arg Pro Ala Asp Cys
                   965                 970                 975
   Ile Ala Asn Tyr Asn Asn Gln Leu Asp Asn Lys Gln Thr Asn Leu Met
               980                 985                 990
   Leu Pro Glu Ser Thr Val Tyr Gly  Asp Val Asp Leu Ser  Asn Lys Ile
           995                 1000                1005
   Asn Glu  Met Lys Thr Phe Asn  Ser Pro Asn Leu Lys  Asp Gly Arg
       1010                1015                1020
   Phe Val  Asn Pro Ser Gly Gln  Pro Thr Pro Tyr Ala  Thr Thr Gln
       1025                 1030                 1035
   Leu Ile  Gln Ser Asn Leu Ser  Asn Asn Met Asn Asn  Gly Ser Gly
       1040                 1045                 1050
   Asp Ser  Gly Glu Lys His Trp  Lys Pro Leu Gly Gln  Gln Lys Gln
       1055                 1060                 1065
   Glu Val  Ala Pro Val Gln Tyr  Asn Ile Val Glu Gln  Asn Lys Leu
       1070                 1075                 1080
   Asn Lys  Asp Tyr Arg Ala Asn  Asp Thr Val Pro Pro  Thr Ile Pro
       1085                 1090                 1095
   Tyr Asn  Gln Ser Tyr Asp Gln  Asn Thr Gly Gly Ser  Tyr Asn Ser
       1100                 1105                 1110
   Ser Asp  Arg Gly Ser Ser Thr  Ser Gly Ser Gln Gly  His Lys Lys
       1115                 1120                 1125
   Gly Ala  Arg Thr Pro Lys Val  Pro Lys Gln Gly Gly  Met Asn Trp
       1130                 1135                 1140
   Ala Asp  Leu Leu Pro Pro Pro  Pro Ala His Pro Pro  Pro His Ser
       1145                 1150                 1155
   Asn Ser  Glu Glu Tyr Asn Ile  Ser Val Asp Glu Ser  Tyr Asp Gln
       1160                 1165                 1170
   Glu Met  Pro Cys Pro Val Pro  Pro Ala Arg Met Tyr  Leu Gln Gln
       1175                 1180                 1185
   Asp Glu  Leu Glu Glu Glu Glu  Asp Glu Arg Gly Pro  Thr Pro Pro
       1190                 1195                 1200
   Val Arg  Gly Ala Ala Ser Ser  Pro Ala Ala Val Ser  Tyr Ser His
       1205                 1210                 1215
   Gln Ser  Thr Ala Thr Leu Thr  Pro Ser Pro Gln Glu  Glu Leu Gln
       1220                 1225                 1230
   Pro Met  Leu Gln Asp Cys Pro  Glu Glu Thr Gly His  Met Gln His
       1235                 1240                 1245
   Gln Pro  Asp Arg Arg Arg Gln  Pro Val Ser Pro Pro  Pro Pro Pro
       1250                 1255                 1260
   Arg Pro  Ile Ser Pro Pro His  Thr Tyr Gly Tyr Ile  Ser Gly Pro
       1265                 1270                 1275
   Leu Val  Ser Asp Met Asp Thr  Asp Ala Pro Glu Glu  Glu Glu Asp
       1280                 1285                 1290
   Glu Ala  Asp Met Glu Val Ala  Lys Met Gln Thr Arg  Arg Leu Leu
       1295                 1300                 1305
   Leu Arg  Gly Leu Glu Gln Thr  Pro Ala Ser Ser Val  Gly Asp Leu
       1310                 1315                 1320
   Glu Ser  Ser Val Thr Gly Ser  Met Ile Asn Gly Trp  Gly Ser Ala
       1325                 1330                 1335
   Ser Glu  Glu Asp Asn Ile Ser  Ser Gly Arg Ser Ser  Val Ser Ser
       1340                 1345                 1350
   Ser Asp  Gly Ser Phe Phe Thr  Asp Ala Asp Phe Ala  Gln Ala Val
       1355                 1360                 1365
   Ala Ala  Ala Ala Glu Tyr Ala  Gly Leu Lys Val Ala  Arg Arg Gln
       1370                 1375                 1380
   Met Gln  Asp Ala Ala Gly Arg  Arg His Phe His Ala  Ser Gln Cys
       1385                 1390                 1395
   Pro Arg  Pro Thr Ser Pro Val  Ser Thr Asp Ser Asn  Met Ser Ala
       1400                 1405                 1410
   Ala Val  Met Gln Lys Thr Arg  Pro Ala Lys Lys Leu  Lys His Gln
       1415                 1420                 1425
   Pro Gly  His Leu Arg Arg Glu  Thr Tyr Thr Asp Asp  Leu Pro Pro
       1430                 1435                 1440
   Pro Pro  Val Pro Pro Pro Ala  Ile Lys Ser Pro Thr  Ala Gln Ser
       1445                 1450                 1455
   Lys Thr  Gln Leu Glu Val Arg  Pro Val Val Val Pro  Lys Leu Pro
       1460                 1465                 1470
   Ser Met  Asp Ala Arg Thr Asp  Arg Ser Ser Asp Arg  Lys Gly Ser
       1475                 1480                 1485
   Ser Tyr  Lys Gly Arg Glu Val  Leu Asp Gly Arg Gln  Val Val Asp
       1490                    1495                    1500
   Met Arg  Thr Asn Pro Gly Asp  Pro Arg Glu Ala Gln  Glu Gln Gln
       1505                    1510                    1515
   Asn Asp  Gly Lys Gly Arg Gly  Asn Lys Ala Ala Lys  Arg Asp Leu
       1520                    1525                    1530
   Pro Pro  Ala Lys Thr His Leu  Ile Gln Glu Asp Ile  Leu Pro Tyr
       1535                    1540                    1545
   Cys Arg  Pro Thr Phe Pro Thr  Ser Asn Asn Pro Arg  Asp Pro Ser
       1550                    1555                    1560
   Ser Ser  Ser Ser Met Ser Ser  Arg Gly Ser Gly Ser  Arg Gln Arg
       1565                    1570                    1575
   Glu Gln  Ala Asn Val Gly Arg  Arg Asn Ile Ala Glu  Met Gln Val
       1580                    1585                    1590
   Leu Gly  Gly Tyr Glu Arg Gly  Glu Asp Asn Asn Glu  Glu Leu Glu
       1595                    1600                    1605
   Glu Thr  Glu Ser
       1610
<210> 105
   <211> 570
   <212> PRT
   <213> homo sapiens
<400> 105
   Met Ala Gly Gly Ala Arg Glu Val Leu Thr Leu Gln Leu Gly His Phe
   1               5                   10                  15
   Ala Gly Phe Val Gly Ala His Trp Trp Asn Gln Gln Asp Ala Ala Leu
               20                  25                  30
   Gly Arg Ala Thr Asp Ser Lys Glu Pro Pro Gly Glu Leu Cys Pro Asp
           35                  40                  45
   Val Leu Tyr Arg Thr Gly Arg Thr Leu His Gly Gln Glu Thr Tyr Thr
       50                  55                  60
   Pro Arg Leu Ile Leu Met Asp Leu Lys Gly Ser Leu Ser Ser Leu Lys
   65                  70                  75                  80
   Glu Glu Gly Gly Leu Tyr Arg Asp Lys Gln Leu Asp Ala Ala Ile Ala
                   85                  90                  95
   Trp Gln Gly Lys Leu Thr Thr His Lys Glu Glu Leu Tyr Pro Lys Asn
               100                 105                 110
   Pro Tyr Leu Gln Asp Phe Leu Ser Ala Glu Gly Val Leu Ser Ser Asp
           115                 120                 125
   Gly Val Trp Arg Val Lys Ser Ile Pro Asn Gly Lys Gly Ser Ser Pro
       130                 135                 140
   Leu Pro Thr Ala Thr Thr Pro Lys Pro Leu Ile Pro Thr Glu Ala Ser
   145                 150                 155                 160
   Ile Arg Val Trp Ser Asp Phe Leu Arg Val His Leu His Pro Arg Ser
                   165                 170                 175
   Ile Cys Met Ile Gln Lys Tyr Asn His Asp Gly Glu Ala Gly Arg Leu
               180                 185                 190
   Glu Ala Phe Gly Gln Gly Glu Ser Val Leu Lys Glu Pro Lys Tyr Gln
           195                 200                 205
   Glu Glu Leu Glu Asp Arg Leu His Phe Tyr Val Glu Glu Cys Asp Tyr
       210                 215                 220
   Leu Gln Gly Phe Gln Ile Leu Cys Asp Leu His Asp Gly Phe Ser Gly
   225                 230                 235                 240
   Val Gly Ala Lys Ala Ala Glu Leu Leu Gln Asp Glu Tyr Ser Gly Arg
                   245                 250                 255
   Gly Ile Ile Thr Trp Gly Leu Leu Pro Gly Pro Tyr His Arg Gly Glu
               260                 265                 270
   Ala Gln Arg Asn Ile Tyr Arg Leu Leu Asn Thr Ala Phe Gly Leu Val
           275                 280                 285
   His Leu Thr Ala His Ser Ser Leu Val Cys Pro Leu Ser Leu Gly Gly
       290                 295                 300
   Ser Leu Gly Leu Arg Pro Glu Pro Pro Val Ser Phe Pro Tyr Leu His
   305                 310                 315                 320
   Tyr Asp Ala Thr Leu Pro Phe His Cys Ser Ala Ile Leu Ala Thr Ala
                           325                 330                 335
   Leu Asp Thr Val Thr Val Pro Tyr Arg Leu Cys Ser Ser Pro Val Ser
               340                 345                 350
   Met Val His Leu Ala Asp Met Leu Ser Phe Cys Gly Lys Lys Val Val
           355                 360                 365
   Thr Ala Gly Ala Ile Ile Pro Phe Pro Leu Ala Pro Gly Gln Ser Leu
       370                 375                 380
   Pro Asp Ser Leu Val Gln Phe Gly Gly Ala Thr Pro Trp Thr Pro Leu
   385                 390                 395                 400
   Ser Ala Cys Gly Glu Pro Ser Gly Thr Arg Cys Phe Ala Gln Ser Val
                   405                 410                 415
   Val Leu Arg Gly Ile Asp Arg Ala Cys His Thr Ser Gln Leu Thr Pro
               420                 425                 430
   Gly Thr Pro Pro Pro Ser Ala Leu His Ala Cys Thr Thr Gly Glu Glu
           435                 440                 445
   Ile Leu Ala Gln Tyr Leu Gln Gln Gln Gln Pro Gly Val Met Ser Ser
       450                 455                 460
   Ser His Leu Leu Leu Thr Pro Cys Arg Val Ala Pro Pro Tyr Pro His
   465                 470                 475                 480
   Leu Phe Ser Ser Cys Ser Pro Pro Gly Met Val Leu Asp Gly Ser Pro
                   485                 490                 495
   Lys Gly Ala Ala Val Glu Ser Ile Pro Val Phe Gly Ala Leu Cys Ser
               500                 505                 510
   Ser Ser Ser Leu His Gln Thr Leu Glu Ala Leu Ala Arg Asp Leu Thr
           515                 520                 525
   Lys Leu Asp Leu Arg Arg Trp Ala Ser Phe Met Asp Ala Gly Val Glu
       530                 535                 540
   His Asp Asp Val Ala Glu Leu Leu Gln Glu Leu Gln Ser Leu Ala Gln
   545                 550                 555                 560
   Cys Tyr Gln Gly Gly Asp Ser Leu Val Asp
                   565                 570
<210> 106
   <211> 141
   <212> PRT
   <213> homo sapiens
<400> 106
   Met Ala Gln Tyr Leu Ser Thr Leu Leu Leu Leu Leu Ala Thr Leu Ala
   1               5                   10                  15
   Val Ala Leu Ala Trp Ser Pro Lys Glu Glu Asp Arg Ile Ile Pro Gly
               20                  25                  30
   Gly Ile Tyr Asn Ala Asp Leu Asn Asp Glu Trp Val Gln Arg Ala Leu
           35                  40                  45
   His Phe Ala Ile Ser Glu Tyr Asn Lys Ala Thr Lys Asp Asp Tyr Tyr
       50                  55                  60
   Arg Arg Pro Leu Arg Val Leu Arg Ala Arg Gln Gln Thr Val Gly Gly
   65                  70                  75                  80
   Val Asn Tyr Phe Phe Asp Val Glu Val Gly Arg Thr Ile Cys Thr Lys
                   85                  90                  95
   Ser Gln Pro Asn Leu Asp Thr Cys Ala Phe His Glu Gln Pro Glu Leu
               100                 105                 110
   Gln Lys Lys Gln Leu Cys Ser Phe Glu Ile Tyr Glu Val Pro Trp Glu
           115                 120                 125
   Asn Arg Arg Ser Leu Val Lys Ser Arg Cys Gln Glu Ser
       130                 135                 140
<210> 107
   <211> 247
   <212> PRT
   <213> homo sapiens
<400> 107
   Met Pro Arg Leu Leu His Pro Ala Leu Pro Leu Leu Leu Gly Ala Thr
   1               5                   10                  15
   Leu Thr Phe Arg Ala Leu Arg Arg Ala Leu Cys Arg Leu Pro Leu Pro
               20                  25                  30
   Val His Val Arg Ala Asp Pro Leu Arg Thr Trp Arg Trp His Asn Leu
           35                  40                  45
   Leu Val Ser Phe Ala His Ser Ile Val Ser Gly Ile Trp Ala Leu Leu
       50                  55                  60
   Cys Val Trp Gln Thr Pro Asp Met Leu Val Glu Ile Glu Thr Ala Trp
   65                  70                  75                  80
   Ser Leu Ser Gly Tyr Leu Leu Val Cys Phe Ser Ala Gly Tyr Phe Ile
                   85                  90                  95
   His Asp Thr Val Asp Ile Val Ala Ser Gly Gln Thr Arg Ala Ser Trp
               100                 105                 110
   Glu Tyr Leu Val His His Val Met Ala Met Gly Ala Phe Phe Ser Gly
           115                 120                 125
   Ile Phe Trp Ser Ser Phe Val Gly Gly Gly Val Leu Thr Leu Leu Val
       130                 135                 140
   Glu Val Ser Asn Ile Phe Leu Thr Ile Arg Met Met Met Lys Ile Ser
   145                 150                 155                 160
   Asn Ala Gln Asp His Leu Leu Tyr Arg Val Asn Lys Tyr Val Asn Leu
                   165                 170                 175
   Val Met Tyr Phe Leu Phe Arg Leu Ala Pro Gln Ala Tyr Leu Thr His
               180                 185                 190
   Phe Phe Leu Arg Tyr Val Asn Gln Arg Thr Leu Gly Thr Phe Leu Leu
           195                 200                 205
   Gly Ile Leu Leu Met Leu Asp Val Met Ile Ile Ile Tyr Phe Ser Arg
       210                 215                 220
   Leu Leu Arg Ser Asp Phe Cys Pro Glu His Val Pro Lys Lys Gln His
   225                 230                 235                 240
   Lys Asp Lys Phe Leu Thr Glu
                   245
<210> 108
   <211> 158
   <212> PRT
   <213> homo sapiens
<400> 108
   Met Ala Ala Arg Ser Val Ser Gly Ile Thr Arg Arg Val Phe Met Trp
   1               5                   10                  15
   Thr Val Ser Gly Thr Pro Cys Arg Glu Phe Trp Ser Arg Phe Arg Lys
               20                  25                  30
   Glu Lys Glu Pro Val Val Val Glu Thr Val Glu Glu Lys Lys Glu Pro
           35                  40                  45
   Ile Leu Val Cys Pro Pro Leu Arg Ser Arg Ala Tyr Thr Pro Pro Glu
       50                  55                  60
   Asp Leu Gln Ser Arg Leu Glu Ser Tyr Val Lys Glu Val Phe Gly Ser
   65                  70                  75                  80
   Ser Leu Pro Ser Asn Trp Gln Asp Ile Ser Leu Glu Asp Ser Arg Leu
                   85                  90                  95
   Lys Phe Asn Leu Leu Ala His Leu Ala Asp Asp Leu Gly His Val Val
               100                 105                 110
   Pro Asn Ser Arg Leu His Gln Met Cys Arg Val Arg Asp Val Leu Asp
           115                 120                 125
   Phe Tyr Asn Val Pro Ile Gln Asp Arg Ser Lys Phe Asp Glu Leu Ser
       130                 135                 140
   Ala Ser Asn Leu Pro Pro Asn Leu Lys Ile Thr Trp Ser Tyr
   145                 150                 155
<210> 109
   <211> 601
   <212> PRT
   <213> homo sapiens
<400> 109
   Met Arg Val Val Arg Leu Leu Arg Leu Arg Ala Ala Leu Thr Leu Leu
   1               5                   10                  15
   Gly Glu Val Pro Arg Arg Pro Ala Ser Arg Gly Val Pro Gly Ser Arg
               20                  25                  30
   Arg Thr Gln Lys Gly Ser Gly Ala Arg Trp Glu Lys Glu Lys His Glu
           35                  40                  45
   Asp Gly Val Lys Trp Arg Gln Leu Glu His Lys Gly Pro Tyr Phe Ala
       50                  55                  60
   Pro Pro Tyr Glu Pro Leu Pro Asp Gly Val Arg Phe Phe Tyr Glu Gly
   65                  70                  75                  80
   Arg Pro Val Arg Leu Ser Val Ala Ala Glu Glu Val Ala Thr Phe Tyr
                   85                  90                  95
   Gly Arg Met Leu Asp His Glu Tyr Thr Thr Lys Glu Val Phe Arg Lys
               100                 105                 110
   Asn Phe Phe Asn Asp Trp Arg Lys Glu Met Ala Val Glu Glu Arg Glu
           115                 120                 125
   Val Ile Lys Ser Leu Asp Lys Cys Asp Phe Thr Glu Ile His Arg Tyr
       130                 135                 140
   Phe Val Asp Lys Ala Ala Ala Arg Lys Val Leu Ser Arg Glu Glu Lys
   145                 150                 155                 160
   Gln Lys Leu Lys Glu Glu Ala Glu Lys Leu Gln Gln Glu Phe Gly Tyr
                   165                 170                 175
   Cys Ile Leu Asp Gly His Gln Glu Lys Ile Gly Asn Phe Lys Ile Glu
               180                 185                 190
   Pro Pro Gly Leu Phe Arg Gly Arg Gly Asp His Pro Lys Met Gly Met
           195                 200                 205
   Leu Lys Arg Arg Ile Thr Pro Glu Asp Val Val Ile Asn Cys Ser Arg
       210                 215                 220
   Asp Ser Lys Ile Pro Glu Pro Pro Ala Gly His Gln Trp Lys Glu Val
   225                 230                 235                 240
   Arg Ser Asp Asn Thr Val Thr Trp Leu Ala Ala Trp Thr Glu Ser Val
                   245                 250                 255
   Gln Asn Ser Ile Lys Tyr Ile Met Leu Asn Pro Cys Ser Lys Leu Lys
               260                 265                 270
   Gly Glu Thr Ala Trp Gln Lys Phe Glu Thr Ala Arg Arg Leu Arg Gly
           275                 280                 285
   Phe Val Asp Glu Ile Arg Ser Gln Tyr Arg Ala Asp Trp Lys Ser Arg
       290                 295                 300
   Glu Met Lys Thr Arg Gln Arg Ala Val Ala Leu Tyr Phe Ile Asp Lys
   305                 310                 315                 320
   Leu Ala Leu Arg Ala Gly Asn Glu Lys Glu Asp Gly Glu Ala Ala Asp
                   325                 330                 335
   Thr Val Gly Cys Cys Ser Leu Arg Val Glu His Val Gln Leu His Pro
               340                 345                 350
   Glu Ala Asp Gly Cys Gln His Val Val Glu Phe Asp Phe Leu Gly Lys
           355                 360                 365
   Asp Cys Ile Arg Tyr Tyr Asn Arg Val Pro Val Glu Lys Pro Val Tyr
       370                 375                 380
   Lys Asn Leu Gln Leu Phe Met Glu Asn Lys Asp Pro Arg Asp Asp Leu
   385                 390                 395                 400
   Phe Asp Arg Leu Thr Thr Thr Ser Leu Asn Lys His Leu Gln Glu Leu
                   405                 410                 415
   Met Asp Gly Leu Thr Ala Lys Val Phe Arg Thr Tyr Asn Ala Ser Ile
               420                 425                 430
   Thr Leu Gln Glu Gln Leu Arg Ala Leu Thr Arg Ala Glu Asp Ser Ile
           435                 440                 445
   Ala Ala Lys Ile Leu Ser Tyr Asn Arg Ala Asn Arg Val Val Ala Ile
       450                 455                 460
   Leu Cys Asn His Gln Arg Ala Thr Pro Ser Thr Phe Glu Lys Ser Met
   465                 470                 475                 480
   Gln Asn Leu Gln Thr Lys Ile Gln Ala Lys Lys Glu Gln Val Ala Glu
                   485                 490                 495
   Ala Arg Ala Glu Leu Arg Arg Ala Arg Ala Glu His Lys Ala Gln Gly
               500                 505                 510
   Asp Gly Lys Ser Arg Ser Val Leu Glu Lys Lys Arg Arg Leu Leu Glu
           515                 520                 525
   Lys Leu Gln Glu Gln Leu Ala Gln Leu Ser Val Gln Ala Thr Asp Lys
       530                 535                 540
   Glu Glu Asn Lys Gln Val Ala Leu Gly Thr Ser Lys Leu Asn Tyr Leu
   545                 550                 555                 560
   Asp Pro Arg Ile Ser Ile Ala Trp Cys Lys Arg Phe Arg Val Pro Val
                   565                 570                 575
   Glu Lys Ile Tyr Ser Lys Thr Gln Arg Glu Arg Phe Ala Trp Ala Leu
               580                 585                 590
   Ala Met Ala Gly Glu Asp Phe Glu Phe
           595                 600

<210> 110
   <211> 269
   <212> PRT
   <213> homo sapiens
<400> 110
   Met Asn Gln Glu Asp Leu Asp Pro Asp Ser Thr Thr Asp Val Gly Asp
   1               5                   10                  15
   Val Thr Asn Thr Glu Glu Glu Leu Ile Arg Glu Cys Glu Glu Met Trp
               20                  25                  30
   Lys Asp Met Glu Glu Cys Gln Asn Lys Leu Ser Leu Ile Gly Thr Glu
           35                  40                  45
   Thr Leu Thr Asp Ser Asn Ala Gln Leu Ser Leu Leu Ile Met Gln Val
       50                  55                  60
   Lys Cys Leu Thr Ala Glu Leu Ser Gln Trp Gln Lys Lys Thr Pro Glu
   65                  70                  75                  80
   Thr Ile Pro Leu Thr Glu Asp Val Leu Ile Thr Leu Gly Lys Glu Glu
                   85                  90                  95
   Phe Gln Lys Leu Arg Gln Asp Leu Glu Met Val Leu Ser Thr Lys Glu
               100                 105                 110
   Ser Lys Asn Glu Lys Leu Lys Glu Asp Leu Glu Arg Glu Gln Arg Trp
           115                 120                 125
   Leu Asp Glu Gln Gln Gln Ile Met Glu Ser Leu Asn Val Leu His Ser
       130                 135                 140
   Glu Leu Lys Asn Lys Val Glu Thr Phe Ser Glu Ser Arg Ile Phe Asn
   145                 150                 155                 160
   Glu Leu Lys Thr Lys Met Leu Asn Ile Lys Glu Tyr Lys Glu Lys Leu
                   165                 170                 175
   Leu Ser Thr Leu Gly Glu Phe Leu Glu Asp His Phe Pro Leu Pro Asp
               180                 185                 190
   Arg Ser Val Lys Lys Lys Lys Lys Asn Ile Gln Glu Ser Ser Val Asn
           195                 200                 205
   Leu Ile Thr Leu His Glu Met Leu Glu Ile Leu Ile Asn Arg Leu Phe
       210                 215                 220
   Asp Val Pro His Asp Pro Tyr Val Lys Ile Ser Asp Ser Phe Trp Pro
   225                 230                 235                 240
   Pro Tyr Val Glu Leu Leu Leu Arg Asn Gly Ile Ala Leu Arg His Pro
                   245                 250                 255
   Glu Asp Pro Thr Arg Ile Arg Leu Glu Ala Phe His Gln
               260                 265
<210> 111
   <211> 829
   <212> PRT
   <213> homo sapiens
<400> 111
<210> 112
   <211> 926
   <212> PRT
   <213> homo sapiens
<400> 112
   Met Asp Met Phe Pro Leu Thr Trp Val Phe Leu Ala Leu Tyr Phe Ser
   1               5                   10                  15
   Arg His Gln Val Arg Gly Gln Pro Asp Pro Pro Cys Gly Gly Arg Leu
               20                  25                  30
   Asn Ser Lys Asp Ala Gly Tyr Ile Thr Ser Pro Gly Tyr Pro Gln Asp
           35                  40                  45
   Tyr Pro Ser His Gln Asn Cys Glu Trp Ile Val Tyr Ala Pro Glu Pro
       50                  55                  60
   Asn Gln Lys Ile Val Leu Asn Phe Asn Pro His Phe Glu Ile Glu Lys
   65                  70                  75                  80
   His Asp Cys Lys Tyr Asp Phe Ile Glu Ile Arg Asp Gly Asp Ser Glu
                   85                  90                  95
   Ser Ala Asp Leu Leu Gly Lys His Cys Gly Asn Ile Ala Pro Pro Thr
               100                 105                 110
   Ile Ile Ser Ser Gly Ser Met Leu Tyr Ile Lys Phe Thr Ser Asp Tyr
           115                 120                 125
   Ala Arg Gln Gly Ala Gly Phe Ser Leu Arg Tyr Glu Ile Phe Lys Thr
       130                 135                 140
   Gly Ser Glu Asp Cys Ser Lys Asn Phe Thr Ser Pro Asn Gly Thr Ile
   145                 150                 155                 160
   Glu Ser Pro Gly Phe Pro Glu Lys Tyr Pro His Asn Leu Asp Cys Thr
                   165                 170                 175
   Phe Thr Ile Leu Ala Lys Pro Lys Met Glu Ile Ile Leu Gln Phe Leu
               180                 185                 190
   Ile Phe Asp Leu Glu His Asp Pro Leu Gln Val Gly Glu Gly Asp Cys
           195                 200                 205
   Lys Tyr Asp Trp Leu Asp Ile Trp Asp Gly Ile Pro His Val Gly Pro
       210                 215                 220
   Leu Ile Gly Lys Tyr Cys Gly Thr Lys Thr Pro Ser Glu Leu Arg Ser
   225 230 235 240
   Ser Thr Gly Ile Leu Ser Leu Thr Phe His Thr Asp Met Ala Val Ala
   245 250 255
   Lys Asp Gly Phe Ser Ala Arg Tyr Tyr Leu Val His Gln Glu Pro Leu
   260 265 270
   Glu Asn Phe Gln Cys Asn Val Pro Leu Gly Met Glu Ser Gly Arg Ile
   275 280 285
   Ala Asn Glu Gln Ile Ser Ala Ser Ser Thr Tyr Ser Asp Gly Arg Trp
   290 295 300
   Thr Pro Gln Gln Ser Arg Leu His Gly Asp Asp Asn Gly Trp Thr Pro
   305 310 315 320
   Asn Leu Asp Ser Asn Lys Glu Tyr Leu Gln Val Asp Leu Arg Phe Leu
   325 330 335
   Thr Met Leu Thr Ala Ile Ala Thr Gln Gly Ala Ile Ser Arg Glu Thr
   340 345 350
   Gln Asn Gly Tyr Tyr Val Lys Ser Tyr Lys Leu Glu Val Ser Thr Asn
   355 360 365
   Gly Glu Asp Trp Met Val Tyr Arg His Gly Lys Asn His Lys Val Phe
   370 375 380
   Gln Ala Asn Asn Asp Ala Thr Glu Val Val Leu Asn Lys Leu His Ala
   385 390 395 400
   Pro Leu Leu Thr Arg Phe Val Arg Ile Arg Pro Gln Thr Trp His Ser
   405 410 415
   Gly Ile Ala Leu Arg Leu Glu Leu Phe Gly Cys Arg Val Thr Asp Ala
   420 425 430
   Pro Cys Ser Asn Met Leu Gly Met Leu Ser Gly Leu Ile Ala Asp Ser
   435 440 445
   Gln Ile Ser Ala Ser Ser Thr Gln Glu Tyr Leu Trp Ser Pro Ser Ala
   450 455 460
   Ala Arg Leu Val Ser Ser Arg Ser Gly Trp Phe Pro Arg Ile Pro Gln
   465 470 475 480
   Ala Gln Pro Gly Glu Glu Trp Leu Gln Val Asp Leu Gly Thr Pro Lys
   485 490 495
   Thr Val Lys Gly Val Ile Ile Gln Gly Ala Arg Gly Gly Asp Ser Ile
   500 505 510
   Thr Ala Val Glu Ala Arg Ala Phe Val Arg Lys Phe Lys Val Ser Tyr
   515 520 525
   Ser Leu Asn Gly Lys Asp Trp Glu Tyr Ile Gln Asp Pro Arg Thr Gln
   530 535 540
   Gln Pro Lys Leu Phe Glu Gly Asn Met His Tyr Asp Thr Pro Asp Ile
   545 550 555 560
   Arg Arg Phe Asp Pro Ile Pro Ala Gln Tyr Val Arg Val Tyr Pro Glu
   565 570 575
   Arg Trp Ser Pro Ala Gly Ile Gly Met Arg Leu Glu Val Leu Gly Cys
   580 585 590
   Asp Trp Thr Asp Ser Lys Pro Thr Val Lys Thr Leu Gly Pro Thr Val
   595 600 605
   Lys Ser Glu Glu Thr Thr Thr Pro Tyr Pro Thr Glu Glu Glu Ala Thr
   610 615 620
   Glu Cys Gly Glu Asn Cys Ser Phe Glu Asp Asp Lys Asp Leu Gln Leu
   625 630 635 640
   Pro Ser Gly Phe Asn Cys Asn Phe Asp Phe Leu Glu Glu Pro Cys Gly
   645 650 655
   Trp Met Tyr Asp His Ala Lys Trp Leu Arg Thr Thr Trp Ala Ser Ser
   660 665 670
   Ser Ser Pro Asn Asp Arg Thr Phe Pro Asp Asp Arg Asn Phe Leu Arg
   675 680 685
   Leu Gln Ser Asp Ser Gln Arg Glu Gly Gln Tyr Ala Arg Leu Ile Ser
   690 695 700
   Pro Pro Val His Leu Pro Arg Ser Pro Val Cys Met Glu Phe Gln Tyr
   705 710 715 720
   Gln Ala Thr Gly Gly Arg Gly Val Ala Leu Gln Val Val Arg Glu Ala
                   725                 730                 735
   Ser Gln Glu Ser Lys Leu Leu Trp Val Ile Arg Glu Asp Gln Gly Gly
               740                 745                 750
   Glu Trp Lys His Gly Arg Ile Ile Leu Pro Ser Tyr Asp Met Glu Tyr
           755                 760                 765
   Gln Ile Val Phe Glu Gly Val Ile Gly Lys Gly Arg Ser Gly Glu Ile
       770                 775                 780
   Ala Ile Asp Asp Ile Arg Ile Ser Thr Asp Val Pro Leu Glu Asn Cys
   785                 790                 795                 800
   Met Glu Pro Ile Ser Ala Phe Ala Val Asp Ile Pro Glu Ile His Glu
                   805                 810                 815
   Arg Glu Gly Tyr Glu Asp Glu Ile Asp Asp Glu Tyr Glu Val Asp Trp
               820                 825                 830
   Ser Asn Ser Ser Ser Ala Thr Ser Gly Ser Gly Ala Pro Ser Thr Asp
           835                 840                 845
   Lys Glu Lys Ser Trp Leu Tyr Thr Leu Asp Pro Ile Leu Ile Thr Ile
       850                 855                 860
   Ile Ala Met Ser Ser Leu Gly Val Leu Leu Gly Ala Thr Cys Ala Gly
   865                 870                 875                 880
   Leu Leu Leu Tyr Cys Thr Cys Ser Tyr Ser Gly Leu Ser Ser Arg Ser
                   885                 890                 895
   Cys Thr Thr Leu Glu Asn Tyr Asn Phe Glu Leu Tyr Asp Gly Leu Lys
               900                 905                 910
   His Lys Val Lys Met Asn His Gln Lys Cys Cys Ser Glu Ala
           915                 920                 925
<210> 113
   <211> 220
   <212> PRT
   <213> homo sapiens
<400> 113
   Met Ser Met Gly Leu Glu Ile Thr Gly Thr Ala Leu Ala Val Leu Gly
   1               5                   10                  15
   Trp Leu Gly Thr Ile Val Cys Cys Ala Leu Pro Met Trp Arg Val Ser
               20                  25                  30
   Ala Phe Ile Gly Ser Asn Ile Ile Thr Ser Gln Asn Ile Trp Glu Gly
           35                  40                  45
   Leu Trp Met Asn Cys Val Val Gln Ser Thr Gly Gln Met Gln Cys Lys
       50                  55                  60
   Val Tyr Asp Ser Leu Leu Ala Leu Pro Gln Asp Leu Gln Ala Ala Arg
   65                  70                  75                  80
   Ala Leu Ile Val Val Ala Ile Leu Leu Ala Ala Phe Gly Leu Leu Val
                   85                  90                  95
   Ala Leu Val Gly Ala Gln Cys Thr Asn Cys Val Gln Asp Asp Thr Ala
               100                 105                 110
   Lys Ala Lys Ile Thr Ile Val Ala Gly Val Leu Phe Leu Leu Ala Ala
           115                 120                 125
   Leu Leu Thr Leu Val Pro Val Ser Trp Ser Ala Asn Thr Ile Ile Arg
       130                 135                 140
   Asp Phe Tyr Asn Pro Val Val Pro Glu Ala Gln Lys Arg Glu Met Gly
   145                 150                 155                 160
   Ala Gly Leu Tyr Val Gly Trp Ala Ala Ala Ala Leu Gln Leu Leu Gly
                   165                 170                 175
   Gly Ala Leu Leu Cys Cys Ser Cys Pro Pro Arg Glu Lys Lys Tyr Thr
               180                 185                 190
   Ala Thr Lys Val Val Tyr Ser Ala Pro Arg Ser Thr Gly Pro Gly Ala
           195                 200                 205
   Ser Leu Gly Thr Gly Tyr Asp Arg Lys Asp Tyr Val
       210                 215                 220
<210> 114
   <211> 209
   <212> PRT
   <213> homo sapiens
<400> 114
   Met Ala Ser Met Gly Leu Gln Val Met Gly Ile Ala Leu Ala Val Leu
   1               5                   10                  15
   Gly Trp Leu Ala Val Met Leu Cys Cys Ala Leu Pro Met Trp Arg Val
               20                  25                  30
   Thr Ala Phe Ile Gly Ser Asn Ile Val Thr Ser Gln Thr Ile Trp Glu
           35                  40                  45
   Gly Leu Trp Met Asn Cys Val Val Gln Ser Thr Gly Gln Met Gln Cys
       50                  55                  60
   Lys Val Tyr Asp Ser Leu Leu Ala Leu Pro Gln Asp Leu Gln Ala Ala
   65                  70                  75                  80
   Arg Ala Leu Val Ile Ile Ser Ile Ile Val Ala Ala Leu Gly Val Leu
                   85                  90                  95
   Leu Ser Val Val Gly Gly Lys Cys Thr Asn Cys Leu Glu Asp Glu Ser
               100                 105                 110
   Ala Lys Ala Lys Thr Met Ile Val Ala Gly Val Val Phe Leu Leu Ala
           115                 120                 125
   Gly Leu Met Val Ile Val Pro Val Ser Trp Thr Ala His Asn Ile Ile
       130                 135                 140
   Gln Asp Phe Tyr Asn Pro Leu Val Ala Ser Gly Gln Lys Arg Glu Met
   145                 150                 155                 160
   Gly Ala Ser Leu Tyr Val Gly Trp Ala Ala Ser Gly Leu Leu Leu Leu
                   165                 170                 175
   Gly Gly Gly Leu Leu Cys Cys Asn Cys Pro Pro Arg Thr Asp Lys Pro
               180                 185                 190
   Tyr Ser Ala Lys Tyr Ser Ala Ala Arg Ser Ala Ala Ala Ser Asn Tyr
           195                 200                 205
   Val
<210> 115
   <211> 346
   <212> PRT
   <213> homo sapiens
<400> 115
   Met Phe Leu Ser Ile Leu Val Ala Leu Cys Leu Trp Leu His Leu Ala
   1               5                   10                  15
   Leu Gly Val Arg Gly Ala Pro Cys Glu Ala Val Arg Ile Pro Met Cys
               20                  25                  30
   Arg His Met Pro Trp Asn Ile Thr Arg Met Pro Asn His Leu His His
           35                  40                  45
   Ser Thr Gln Glu Asn Ala Ile Leu Ala Ile Glu Gln Tyr Glu Glu Leu
       50                  55                  60
   Val Asp Val Asn Cys Ser Ala Val Leu Arg Phe Phe Phe Cys Ala Met
   65                  70                  75                  80
   Tyr Ala Pro Ile Cys Thr Leu Glu Phe Leu His Asp Pro Ile Lys Pro
                   85                  90                  95
   Cys Lys Ser Val Cys Gln Arg Ala Arg Asp Asp Cys Glu Pro Leu Met
               100                 105                 110
   Lys Met Tyr Asn His Ser Trp Pro Glu Ser Leu Ala Cys Asp Glu Leu
           115                 120                 125
   Pro Val Tyr Asp Arg Gly Val Cys Ile Ser Pro Glu Ala Ile Val Thr
       130                 135                 140
   Asp Leu Pro Glu Asp Val Lys Trp Ile Asp Ile Thr Pro Asp Met Met
   145                 150                 155                 160
   Val Gln Glu Arg Pro Leu Asp Val Asp Cys Lys Arg Leu Ser Pro Asp
                   165                 170                 175
   Arg Cys Lys Cys Lys Lys Val Lys Pro Thr Leu Ala Thr Tyr Leu Ser
               180                 185                 190
   Lys Asn Tyr Ser Tyr Val Ile His Ala Lys Ile Lys Ala Val Gln Arg
           195                 200                 205
   Ser Gly Cys Asn Glu Val Thr Thr Val Val Asp Val Lys Glu Ile Phe
       210                 215                 220
   Lys Ser Ser Ser Pro Ile Pro Arg Thr Gln Val Pro Leu Ile Thr Asn
   225                 230                 235                 240
   Ser Ser Cys Gln Cys Pro His Ile Leu Pro His Gln Asp Val Leu Ile
                   245                 250                 255
   Met Cys Tyr Glu Trp Arg Ser Arg Met Met Leu Leu Glu Asn Cys Leu
               260                 265                 270
   Val Glu Lys Trp Arg Asp Gln Leu Ser Lys Arg Ser Ile Gln Trp Glu
           275                 280                 285
   Glu Arg Leu Gln Glu Gln Arg Arg Thr Val Gln Asp Lys Lys Lys Thr
       290                 295                 300
   Ala Gly Arg Thr Ser Arg Ser Asn Pro Pro Lys Pro Lys Gly Lys Pro
   305                 310                 315                 320
   Pro Ala Pro Lys Pro Ala Ser Pro Lys Lys Asn Ile Lys Thr Arg Ser
                   325                 330                 335
   Ala Gln Lys Arg Thr Asn Pro Lys Arg Val
               340                 345
<210> 116
   <211> 553
   <212> PRT
   <213> homo sapiens
<400> 116
   Met Ala Ala Pro Ala Gly Gly Gly Gly Ser Ala Val Ser Val Leu Ala
   1               5                   10                  15
   Pro Asn Gly Arg Arg His Thr Val Lys Val Thr Pro Ser Thr Val Leu
               20                  25                  30
   Leu Gln Val Leu Glu Asp Thr Cys Arg Arg Gln Asp Phe Asn Pro Cys
           35                  40                  45
   Glu Tyr Asp Leu Lys Phe Gln Arg Ser Val Leu Asp Leu Ser Leu Gln
       50                  55                  60
   Trp Arg Phe Ala Asn Leu Pro Asn Asn Ala Lys Leu Glu Met Val Pro
   65                  70                  75                  80
   Ala Ser Arg Ser Arg Glu Gly Pro Glu Asn Met Val Arg Ile Ala Leu
                   85                  90                  95
   Gln Leu Asp Asp Gly Ser Arg Leu Gln Asp Ser Phe Cys Ser Gly Gln
               100                 105                 110
   Thr Leu Trp Glu Leu Leu Ser His Phe Pro Gln Ile Arg Glu Cys Leu
           115                 120                 125
   Gln His Pro Gly Gly Ala Thr Pro Val Cys Val Tyr Thr Arg Asp Glu
       130                 135                 140
   Val Thr Gly Glu Ala Ala Leu Arg Gly Thr Thr Leu Gln Ser Leu Gly
   145                 150                 155                 160
   Leu Thr Gly Gly Ser Ala Thr Ile Arg Phe Val Met Lys Cys Tyr Asp
                   165                 170                 175
   Pro Val Gly Lys Thr Pro Gly Ser Leu Gly Ser Ser Ala Ser Ala Gly
               180                 185                 190
   Gln Ala Ala Ala Ser Ala Pro Leu Pro Leu Glu Ser Gly Glu Leu Ser
           195                 200                 205
   Arg Gly Asp Leu Ser Arg Pro Glu Asp Ala Asp Thr Ser Gly Pro Cys
       210                 215                 220
   Cys Glu His Thr Gln Glu Lys Gln Ser Thr Arg Ala Pro Ala Ala Ala
   225                 230                 235                 240
   Pro Phe Val Pro Phe Ser Gly Gly Gly Gln Arg Leu Gly Gly Pro Pro
                   245                 250                 255
   Gly Pro Thr Arg Pro Leu Thr Ser Ser Ser Ala Lys Leu Pro Lys Ser
               260                 265                 270
   Leu Ser Ser Pro Gly Gly Pro Ser Lys Pro Lys Lys Ser Lys Ser Gly
           275                 280                 285
   Gln Asp Pro Gln Gln Glu Gln Glu Gln Glu Arg Glu Arg Asp Pro Gln
       290                 295                 300
   Gln Glu Gln Glu Arg Glu Arg Pro Val Asp Arg Glu Pro Val Asp Arg
   305                 310                 315                 320
   Glu Pro Val Val Cys His Pro Asp Leu Glu Glu Arg Leu Gln Ala Trp
                   325                 330                 335
   Pro Ala Glu Leu Pro Asp Glu Phe Phe Glu Leu Thr Val Asp Asp Val
               340                 345                 350
   Arg Arg Arg Leu Ala Gln Leu Lys Ser Glu Arg Lys Arg Leu Glu Glu
   355 360 365
   Ala Pro Leu Val Thr Lys Ala Phe Arg Glu Ala Gln Ile Lys Glu Lys
   370 375 380
   Leu Glu Arg Tyr Pro Lys Val Ala Leu Arg Val Leu Phe Pro Asp Arg
   385 390 395 400
   Tyr Val Leu Gln Gly Phe Phe Arg Pro Ser Glu Thr Val Gly Asp Leu
   405 410 415
   Arg Asp Phe Val Arg Ser His Leu Gly Asn Pro Glu Leu Ser Phe Tyr
   420 425 430
   Leu Phe Ile Thr Pro Pro Lys Thr Val Leu Asp Asp His Thr Gln Thr
   435 440 445
   Leu Phe Gln Ala Asn Leu Phe Pro Ala Ala Leu Val His Leu Gly Ala
   450 455 460
   Glu Glu Pro Ala Gly Val Tyr Leu Glu Pro Gly Leu Leu Glu His Ala
   465 470 475 480
   Ile Ser Pro Ser Ala Ala Asp Val Leu Val Ala Arg Tyr Met Ser Arg
   485 490 495
   Ala Ala Gly Ser Pro Ser Pro Leu Pro Ala Pro Asp Pro Ala Pro Lys
   500 505 510
   Ser Glu Pro Ala Ala Glu Glu Gly Ala Leu Val Pro Pro Glu Pro Ile
   515 520 525
   Pro Gly Thr Ala Gln Pro Val Lys Arg Ser Leu Gly Lys Val Pro Lys
   530 535 540
   Trp Leu Lys Leu Pro Ala Ser Lys Arg
   545 550
<210> 117
   <211> 102
   <212> PRT
   <213> homo sapiens
<400> 117
   Met Ser Ala Arg Val Arg Ser Arg Ser Arg Gly Arg Gly Asp Gly Gln
   1 5 10 15
   Glu Ala Pro Asp Val Val Ala Phe Val Ala Pro Gly Glu Ser Gln Gln
   20 25 30
   Glu Glu Pro Pro Thr Asp Asn Gln Asp Ile Glu Pro Gly Gln Glu Arg
   35 40 45
   Glu Gly Thr Pro Pro Ile Glu Glu Arg Lys Val Glu Gly Asp Cys Gln
   50 55 60
   Glu Met Asp Leu Glu Lys Thr Arg Ser Glu Arg Gly Asp Gly Ser Asp
   65 70 75 80
   Val Lys Glu Lys Thr Pro Pro Asn Pro Lys His Ala Lys Thr Lys Glu
   85 90 95
   Ala Gly Asp Gly Gln Pro
   100
<210> 118
   <211> 724
   <212> PRT
   <213> homo sapiens
<400> 118
   Met Ser Phe Pro Lys Ala Pro Leu Lys Arg Phe Asn Asp Pro Ser Gly
   1               5                   10                  15
   Cys Ala Pro Ser Pro Gly Ala Tyr Asp Val Lys Thr Leu Glu Val Leu
               20                  25                  30
   Lys Gly Pro Val Ser Phe Gln Lys Ser Gln Arg Phe Lys Gln Gln Lys
           35                  40                  45
   Glu Ser Lys Gln Asn Leu Asn Val Asp Lys Asp Thr Thr Leu Pro Ala
       50                  55                  60
   Ser Ala Arg Lys Val Lys Ser Ser Glu Ser Lys Glu Ser Gln Lys Asn
   65                  70                  75                  80
   Asp Lys Asp Leu Lys Ile Leu Glu Lys Glu Ile Arg Val Leu Leu Gln
                   85                  90                  95
   Glu Arg Gly Ala Gln Asp Ser Arg Ile Gln Asp Leu Glu Thr Glu Leu
                   100                 105                 110
   Glu Lys Met Glu Ala Arg Leu Asn Ala Ala Leu Arg Glu Lys Thr Ser
               115                 120                 125
   Leu Ser Ala Asn Asn Ala Thr Leu Glu Lys Gln Leu Ile Glu Leu Thr
       130                 135                 140
   Arg Thr Asn Glu Leu Leu Lys Ser Lys Phe Ser Glu Asn Gly Asn Gln
   145                 150                 155                 160
   Lys Asn Leu Arg Ile Leu Ser Leu Glu Leu Met Lys Leu Arg Asn Lys
                   165                 170                 175
   Arg Glu Thr Lys Met Arg Gly Met Met Ala Lys Gln Glu Gly Met Glu
               180                 185                 190
   Met Lys Leu Gln Val Thr Gln Arg Ser Leu Glu Glu Ser Gln Gly Lys
           195                 200                 205
   Ile Ala Gln Leu Glu Gly Lys Leu Val Ser Ile Glu Lys Glu Lys Ile
       210                 215                 220
   Asp Glu Lys Ser Glu Thr Glu Lys Leu Leu Glu Tyr Ile Glu Glu Ile
   225                 230                 235                 240
   Ser Cys Ala Ser Asp Gln Val Glu Lys Tyr Lys Leu Asp Ile Ala Gln
                   245                 250                 255
   Leu Glu Glu Asn Leu Lys Glu Lys Asn Asp Glu Ile Leu Ser Leu Lys
               260                 265                 270
   Gln Ser Leu Glu Glu Asn Ile Val Ile Leu Ser Lys Gln Val Glu Asp
           275                 280                 285
   Leu Asn Val Lys Cys Gln Leu Leu Glu Lys Glu Lys Glu Asp His Val
       290                 295                 300
   Asn Arg Asn Arg Glu His Asn Glu Asn Leu Asn Ala Glu Met Gln Asn
   305                 310                 315                 320
   Leu Lys Gln Lys Phe Ile Leu Glu Gln Gln Glu Arg Glu Lys Leu Gln
                   325                 330                 335
   Gln Lys Glu Leu Gln Ile Asp Ser Leu Leu Gln Gln Glu Lys Glu Leu
               340                 345                 350
   Ser Ser Ser Leu His Gln Lys Leu Cys Ser Phe Gln Glu Glu Met Val
           355                 360                 365
   Lys Glu Lys Asn Leu Phe Glu Glu Glu Leu Lys Gln Thr Leu Asp Glu
       370                 375                 380
   Leu Asp Lys Leu Gln Gln Lys Glu Glu Gln Ala Glu Arg Leu Val Lys
   385                 390                 395                 400
   Gln Leu Glu Glu Glu Ala Lys Ser Arg Ala Glu Glu Leu Lys Leu Leu
                   405                 410                 415
   Glu Glu Lys Leu Lys Gly Lys Glu Ala Glu Leu Glu Lys Ser Ser Ala
               420                 425                 430
   Ala His Thr Gln Ala Thr Leu Leu Leu Gln Glu Lys Tyr Asp Ser Met
           435                 440                 445
   Val Gln Ser Leu Glu Asp Val Thr Ala Gln Phe Glu Ser Tyr Lys Ala
       450                 455                 460
   Leu Thr Ala Ser Glu Ile Glu Asp Leu Lys Leu Glu Asn Ser Ser Leu
   465                 470                 475                 480
   Gln Glu Lys Ala Ala Lys Ala Gly Lys Asn Ala Glu Asp Val Gln His
                   485                 490                 495
   Gln Ile Leu Ala Thr Glu Ser Ser Asn Gln Glu Tyr Val Arg Met Leu
               500                 505                 510
   Leu Asp Leu Gln Thr Lys Ser Ala Leu Lys Glu Thr Glu Ile Lys Glu
           515                 520                 525
   Ile Thr Val Ser Phe Leu Gln Lys Ile Thr Asp Leu Gln Asn Gln Leu
       530                 535                 540
   Lys Gln Gln Glu Glu Asp Phe Arg Lys Gln Leu Glu Asp Glu Glu Gly
   545                 550                 555                 560
   Arg Lys Ala Glu Lys Glu Asn Thr Thr Ala Glu Leu Thr Glu Glu Ile
                   565                 570                 575
   Asn Lys Trp Arg Leu Leu Tyr Glu Glu Leu Tyr Asn Lys Thr Lys Pro
               580                 585                 590
   Phe Gln Leu Gln Leu Asp Ala Phe Glu Val Glu Lys Gln Ala Leu Leu
           595                 600                 605
   Asn Glu His Gly Ala Ala Gln Glu Gln Leu Asn Lys Ile Arg Asp Ser
       610                 615                 620
   Tyr Ala Lys Leu Leu Gly His Gln Asn Leu Lys Gln Lys Ile Lys His
   625                 630                 635                 640
   Val Val Lys Leu Lys Asp Glu Asn Ser Gln Leu Lys Ser Glu Val Ser
                   645                 650                 655
   Lys Leu Arg Cys Gln Leu Ala Lys Lys Lys Gln Ser Glu Thr Lys Leu
               660                 665                 670
   Gln Glu Glu Leu Asn Lys Val Leu Gly Ile Lys His Phe Asp Pro Ser
           675                 680                 685
   Lys Ala Phe His His Glu Ser Lys Glu Asn Phe Ala Leu Lys Thr Pro
       690                 695                 700
   Leu Lys Glu Gly Asn Thr Asn Cys Tyr Arg Ala Pro Met Glu Cys Gln
   705                 710                 715                 720
   Glu Ser Trp Lys
<210> 119
   <211> 325
   <212> PRT
   <213> homo sapiens
<400> 119
   Met Thr Glu Arg Arg Arg Asp Glu Leu Ser Glu Glu Ile Asn Asn Leu
   1               5                   10                  15
   Arg Glu Lys Val Met Lys Gln Ser Glu Glu Asn Asn Asn Leu Gln Ser
               20                  25                  30
   Gln Val Gln Lys Leu Thr Glu Glu Asn Thr Thr Leu Arg Glu Gln Val
           35                  40                  45
   Glu Pro Thr Pro Glu Asp Glu Asp Asp Asp Ile Glu Leu Arg Gly Ala
       50                  55                  60
   Ala Ala Ala Ala Ala Pro Pro Pro Pro Ile Glu Glu Glu Cys Pro Glu
   65                  70                  75                  80
   Asp Leu Pro Glu Lys Phe Asp Gly Asn Pro Asp Met Leu Ala Pro Phe
                   85                  90                  95
   Met Ala Gln Cys Gln Ile Phe Met Glu Lys Ser Thr Arg Asp Phe Ser
               100                 105                 110
   Val Asp Arg Val Arg Val Cys Phe Val Thr Ser Met Met Thr Gly Arg
           115                 120                 125
   Ala Ala Arg Trp Ala Ser Ala Lys Leu Glu Arg Ser His Tyr Leu Met
       130                 135                 140
   His Asn Tyr Pro Ala Phe Met Met Glu Met Lys His Val Phe Glu Asp
   145                 150                 155                 160
   Pro Gln Arg Arg Glu Val Ala Lys Arg Lys Ile Arg Arg Leu Arg Gln
                   165                 170                 175
   Gly Met Gly Ser Val Ile Asp Tyr Ser Asn Ala Phe Gln Met Ile Ala
               180                 185                 190
   Gln Asp Leu Asp Trp Asn Glu Pro Ala Leu Ile Asp Gln Tyr His Glu
           195                 200                 205
   Gly Leu Ser Asp His Ile Gln Glu Glu Leu Ser His Leu Glu Val Ala
       210                 215                 220
   Lys Ser Leu Ser Ala Leu Ile Gly Gln Cys Ile His Ile Glu Arg Arg
   225                 230                 235                 240
   Leu Ala Arg Ala Ala Ala Ala Arg Lys Pro Arg Ser Pro Pro Arg Ala
                   245                 250                 255
   Leu Val Leu Pro His Ile Ala Ser His His Gln Val Asp Pro Thr Glu
               260                 265                 270
   Pro Val Gly Gly Ala Arg Met Arg Leu Thr Gln Glu Glu Lys Glu Arg
           275                 280                 285
   Arg Arg Lys Leu Asn Leu Cys Leu Tyr Cys Gly Thr Gly Gly His Tyr
       290                 295                 300
   Ala Asp Asn Cys Pro Ala Lys Ala Ser Lys Ser Ser Pro Ala Gly Asn
   305                 310                 315                 320
   Ser Pro Ala Pro Leu
                   325
<210> 120
   <211> 162
   <212> PRT
   <213> homo sapiens
<400> 120
   Met Asp Ile Pro Gln Thr Lys Gln Asp Leu Glu Leu Pro Lys Leu Ala
   1               5                   10                  15
   Gly Thr Trp His Ser Met Ala Met Ala Thr Asn Asn Ile Ser Leu Met
               20                  25                  30
   Ala Thr Leu Lys Ala Pro Leu Arg Val His Ile Thr Ser Leu Leu Pro
           35                  40                  45
   Thr Pro Glu Asp Asn Leu Glu Ile Val Leu His Arg Trp Glu Asn Asn
       50                  55                  60
   Ser Cys Val Glu Lys Lys Val Leu Gly Glu Lys Thr Gly Asn Pro Lys
   65                  70                  75                  80
   Lys Phe Lys Ile Asn Tyr Thr Val Ala Asn Glu Ala Thr Leu Leu Asp
                   85                  90                  95
   Thr Asp Tyr Asp Asn Phe Leu Phe Leu Cys Leu Gln Asp Thr Thr Thr
               100                 105                 110
   Pro Ile Gln Ser Met Met Cys Gln Tyr Leu Ala Arg Val Leu Val Glu
           115                 120                 125
   Asp Asp Glu Ile Met Gln Gly Phe Ile Arg Ala Phe Arg Pro Leu Pro
       130                 135                 140
   Arg His Leu Trp Tyr Leu Leu Asp Leu Lys Gln Met Glu Glu Pro Cys
   145                 150                 155                 160
   Arg Phe
<210> 121
   <211> 55
   <212> PRT
   <213> homo sapiens
<400> 121
   Met Ala Lys Asp Thr Ala Ser Leu Phe Ile Pro Leu Cys Gly Cys Ser
   1               5                   10                  15
   Asn Ser Lys Ser Gly Thr Thr Gly Gln Met Asn Val Gly Thr Cys Arg
               20                  25                  30
   Tyr Gly Ser Leu Ala Leu Arg Gln Leu Val Trp Gly Leu Pro Pro Gly
           35                  40                  45
   Ala Ser Trp Pro His Leu Arg
       50                  55
<210> 122
   <211> 384
   <212> PRT
   <213> homo sapiens
<400> 122
   Met Glu Gly Leu Gly Arg Ser Cys Leu Trp Leu Arg Arg Glu Leu Ser
   1               5                   10                  15
   Pro Pro Arg Pro Arg Leu Leu Leu Leu Asp Cys Arg Ser Arg Glu Leu
               20                  25                  30
   Tyr Glu Ser Ala Arg Ile Gly Gly Ala Leu Ser Val Ala Leu Pro Ala
           35                  40                  45
   Leu Leu Leu Arg Arg Leu Arg Arg Gly Ser Leu Ser Val Arg Ala Leu
       50                  55                  60
   Leu Pro Gly Pro Pro Leu Gln Pro Pro Pro Pro Ala Pro Val Leu Leu
   65                  70                  75                  80
   Tyr Asp Gln Gly Gly Gly Arg Arg Arg Arg Gly Glu Ala Glu Ala Glu
                   85                  90                  95
   Ala Glu Glu Trp Glu Ala Glu Ser Val Leu Gly Thr Leu Leu Gln Lys
               100                 105                 110
   Leu Arg Glu Glu Gly Tyr Leu Ala Tyr Tyr Leu Gln Gly Gly Phe Ser
           115                 120                 125
   Arg Phe Gln Ala Glu Cys Pro His Leu Cys Glu Thr Ser Leu Ala Gly
       130                 135                 140
   Arg Ala Gly Ser Ser Met Ala Pro Val Pro Gly Pro Val Pro Val Val
   145                 150                 155                 160
   Gly Leu Gly Ser Leu Cys Leu Gly Ser Asp Cys Ser Asp Ala Glu Ser
                   165                 170                 175
   Glu Ala Asp Arg Asp Ser Met Ser Cys Gly Leu Asp Ser Glu Gly Ala
               180                 185                 190
   Thr Pro Pro Pro Val Gly Leu Arg Ala Ser Phe Pro Val Gln Ile Leu
           195                 200                 205
   Pro Asn Leu Tyr Leu Gly Ser Ala Arg Asp Ser Ala Asn Leu Glu Ser
       210                 215                 220
   Leu Ala Lys Leu Gly Ile Arg Tyr Ile Leu Asn Val Thr Pro Asn Leu
   225                 230                 235                 240
   Pro Asn Phe Phe Glu Lys Asn Gly Asp Phe His Tyr Lys Gln Ile Pro
                   245                 250                 255
   Ile Ser Asp His Trp Ser Gln Asn Leu Ser Arg Phe Phe Pro Glu Ala
               260                 265                 270
   Ile Glu Phe Ile Asp Glu Ala Leu Ser Gln Asn Cys Gly Val Leu Val
           275                 280                 285
   His Cys Leu Ala Gly Val Ser Arg Ser Val Thr Val Thr Val Ala Tyr
       290                 295                 300
   Leu Met Gln Lys Leu His Leu Ser Leu Asn Asp Ala Tyr Asp Leu Val
   305                 310                 315                 320
   Lys Arg Lys Lys Ser Asn Ile Ser Pro Asn Phe Asn Phe Met Gly Gln
                   325                 330                 335
   Leu Leu Asp Phe Glu Arg Ser Leu Arg Leu Glu Glu Arg His Ser Gln
               340                 345                 350
   Glu Gln Gly Ser Gly Gly Gln Ala Ser Ala Ala Ser Asn Pro Pro Ser
           355                 360                 365
   Phe Phe Thr Thr Pro Thr Ser Asp Gly Ala Phe Glu Leu Ala Pro Thr
       370                 375                 380
<210> 123
   <211> 1016
   <212> PRT
   <213> homo sapiens
<400> 123
   Met Ser Gln Gly Pro Pro Thr Gly Glu Ser Ser Glu Pro Glu Ala Lys
   1               5                   10                  15
   Val Leu His Thr Lys Arg Leu Tyr Arg Ala Val Val Glu Ala Val His
               20                  25                  30
   Arg Leu Asp Leu Ile Leu Cys Asn Lys Thr Ala Tyr Gln Glu Val Phe
           35                  40                  45
   Lys Pro Glu Asn Ile Ser Leu Arg Asn Lys Leu Arg Glu Leu Cys Val
       50                  55                  60
   Lys Leu Met Phe Leu His Pro Val Asp Tyr Gly Arg Lys Ala Glu Glu
   65                  70                  75                  80
   Leu Leu Trp Arg Lys Val Tyr Tyr Glu Val Ile Gln Leu Ile Lys Thr
                   85                  90                  95
   Asn Lys Lys His Ile His Ser Arg Ser Thr Leu Glu Cys Ala Tyr Arg
               100                 105                 110
   Thr His Leu Val Ala Gly Ile Gly Phe Tyr Gln His Leu Leu Leu Tyr
           115                 120                 125
   Ile Gln Ser His Tyr Gln Leu Glu Leu Gln Cys Cys Ile Asp Trp Thr
       130                 135                 140
   His Val Thr Asp Pro Leu Ile Gly Cys Lys Lys Pro Val Ser Ala Ser
   145                 150                 155                 160
   Gly Lys Glu Met Asp Trp Ala Gln Met Ala Cys His Arg Cys Leu Val
                   165                 170                 175
   Tyr Leu Gly Asp Leu Ser Arg Tyr Gln Asn Glu Leu Ala Gly Val Asp
               180                 185                 190
   Thr Glu Leu Leu Ala Glu Arg Phe Tyr Tyr Gln Ala Leu Ser Val Ala
           195                 200                 205
   Pro Gln Ile Gly Met Pro Phe Asn Gln Leu Gly Thr Leu Ala Gly Ser
       210                 215                 220
   Lys Tyr Tyr Asn Val Glu Ala Met Tyr Cys Tyr Leu Arg Cys Ile Gln
   225                 230                 235                 240
   Ser Glu Val Ser Phe Glu Gly Ala Tyr Gly Asn Leu Lys Arg Leu Tyr
                   245                 250                 255
   Asp Lys Ala Ala Lys Met Tyr His Gln Leu Lys Lys Cys Glu Thr Arg
               260                 265                 270
   Lys Leu Ser Pro Gly Lys Lys Arg Cys Lys Asp Ile Lys Arg Leu Leu
           275                 280                 285
   Val Asn Phe Met Tyr Leu Gln Ser Leu Leu Gln Pro Lys Ser Ser Ser
       290                 295                 300
   Val Asp Ser Glu Leu Thr Ser Leu Cys Gln Ser Val Leu Glu Asp Phe
   305                 310                 315                 320
   Asn Leu Cys Leu Phe Tyr Leu Pro Ser Ser Pro Asn Leu Ser Leu Ala
                   325                 330                 335
   Ser Glu Asp Glu Glu Glu Tyr Glu Ser Gly Tyr Ala Phe Leu Pro Asp
               340                 345                 350
   Leu Leu Ile Phe Gln Met Val Ile Ile Cys Leu Met Cys Val His Ser
           355                 360                 365
   Leu Glu Arg Ala Gly Ser Lys Gln Tyr Ser Ala Ala Ile Ala Phe Thr
       370                 375                 380
   Leu Ala Leu Phe Ser His Leu Val Asn His Val Asn Ile Arg Leu Gln
   385                 390                 395                 400
   Ala Glu Leu Glu Glu Gly Glu Asn Pro Val Pro Ala Phe Gln Ser Asp
                   405                 410                 415
   Gly Thr Asp Glu Pro Glu Ser Lys Glu Pro Val Glu Lys Glu Glu Glu
               420                 425                 430
   Pro Asp Pro Glu Pro Pro Pro Val Thr Pro Gln Val Gly Glu Gly Arg
           435                 440                 445
   Lys Ser Arg Lys Phe Ser Arg Leu Ser Cys Leu Arg Arg Arg Arg His
       450                 455                 460
   Pro Pro Lys Val Gly Asp Asp Ser Asp Leu Ser Glu Gly Phe Glu Ser
   465                 470                 475                 480
   Asp Ser Ser His Asp Ser Ala Arg Ala Ser Glu Gly Ser Asp Ser Gly
                   485                 490                 495
   Ser Asp Lys Ser Leu Glu Gly Gly Gly Thr Ala Phe Asp Ala Glu Thr
               500                 505                 510
   Asp Ser Glu Met Asn Ser Gln Glu Ser Arg Ser Asp Leu Glu Asp Met
           515                 520                 525
   Glu Glu Glu Glu Gly Thr Arg Ser Pro Thr Leu Glu Pro Pro Arg Gly
       530                 535                 540
   Arg Ser Glu Ala Pro Asp Ser Leu Asn Gly Pro Leu Gly Pro Ser Glu
   545                 550                 555                 560
   Ala Ser Ile Ala Ser Asn Leu Gln Ala Met Ser Thr Gln Met Phe Gln
                   565                 570                 575
   Thr Lys Arg Cys Phe Arg Leu Ala Pro Thr Phe Ser Asn Leu Leu Leu
               580                 585                 590
   Gln Pro Thr Thr Asn Pro His Thr Ser Ala Ser His Arg Pro Cys Val
           595                 600                 605
   Asn Gly Asp Val Asp Lys Pro Ser Glu Pro Ala Ser Glu Glu Gly Ser
       610                 615                 620
   Glu Ser Glu Gly Ser Glu Ser Ser Gly Arg Ser Cys Arg Asn Glu Arg
   625                 630                 635                 640
   Ser Ile Gln Glu Lys Leu Gln Val Leu Met Ala Glu Gly Leu Leu Pro
                   645                 650                 655
   Ala Val Lys Val Phe Leu Asp Trp Leu Arg Thr Asn Pro Asp Leu Ile
               660                 665                 670
   Ile Val Cys Ala Gln Ser Ser Gln Ser Leu Trp Asn Arg Leu Ser Val
           675                 680                 685
   Leu Leu Asn Leu Leu Pro Ala Ala Gly Glu Leu Gln Glu Ser Gly Leu
       690                 695                 700
   Ala Leu Cys Pro Glu Val Gln Asp Leu Leu Glu Gly Cys Glu Leu Pro
   705                 710                 715                 720
   Asp Leu Pro Ser Ser Leu Leu Leu Pro Glu Asp Met Ala Leu Arg Asn
                   725                 730                 735
   Leu Pro Pro Leu Arg Ala Ala His Arg Arg Phe Asn Phe Asp Thr Asp
               740                 745                 750
   Arg Pro Leu Leu Ser Thr Leu Glu Glu Ser Val Val Arg Ile Cys Cys
           755                 760                 765
   Ile Arg Ser Phe Gly His Phe Ile Ala Arg Leu Gln Gly Ser Ile Leu
       770                 775                 780
   Gln Phe Asn Pro Glu Val Gly Ile Phe Val Ser Ile Ala Gln Ser Glu
   785                 790                 795                 800
   Gln Glu Ser Leu Leu Gln Gln Ala Gln Ala Gln Phe Arg Met Ala Gln
                   805                 810                 815
   Glu Glu Ala Arg Arg Asn Arg Leu Met Arg Asp Met Ala Gln Leu Arg
               820                 825                 830
   Leu Gln Leu Glu Val Ser Gln Leu Glu Gly Ser Leu Gln Gln Pro Lys
           835                 840                 845
   Ala Gln Ser Ala Met Ser Pro Tyr Leu Val Pro Asp Thr Gln Ala Leu
       850                 855                 860
   Cys His His Leu Pro Val Ile Arg Gln Leu Ala Thr Ser Gly Arg Phe
   865                 870                 875                 880
   Ile Val Ile Ile Pro Arg Thr Val Ile Asp Gly Leu Asp Leu Leu Lys
                   885                 890                 895
   Lys Glu His Pro Gly Ala Arg Asp Gly Ile Arg Tyr Leu Glu Ala Glu
               900                 905                 910
   Phe Lys Lys Gly Asn Arg Tyr Ile Arg Cys Gln Lys Glu Val Gly Lys
           915                 920                 925
   Ser Phe Glu Arg His Lys Leu Lys Arg Gln Asp Ala Asp Ala Trp Thr
       930                 935                 940
   Leu Tyr Lys Ile Leu Asp Ser Cys Lys Gln Leu Thr Leu Ala Gln Gly
   945                 950                 955                 960
   Ala Gly Glu Glu Asp Pro Ser Gly Met Val Thr Ile Ile Thr Gly Leu
                   965                 970                 975
   Pro Leu Asp Asn Pro Ser Val Leu Ser Gly Pro Met Gln Ala Ala Leu
               980                 985                 990
   Gln Ala Ala Ala His Ala Ser Val Asp Ile Lys Asp Val Leu Asp Phe
           995                 1000                1005
   Tyr Lys Gln Trp Lys Glu Ile Gly
       1010                1015
<210> 124
   <211> 21
   <212> DNA
   <213> homo sapiens
<400> 124
   ggattctctg ccctgtcaca c 21
<210> 125
   <211> 20
   <212> DNA
   <213> homo sapiens
<400> 125
   cttggcacag gacccaagag 20
<210> 126
   <211> 23
   <212> DNA
   <213> homo sapiens
<400> 126
   cggaggggag aggattttct aag 23
<210> 127
   <211> 24
   <212> DNA
   <213> homo sapiens
<400> 127
   ggtccaggtc atctttatta cgcc 24
<210> 128
   <211> 22
   <212> DNA
   <213> homo sapiens
<400> 128
   gggattagga atatgggctc tg 22
<210> 129
   <211> 23
   <212> DNA
   <213> homo sapiens
<400> 129
   aatgaggaaa ctgaggcata aag 23
<210> 130
   <211> 22
   <212> DNA
   <213> homo sapiens
<400> 130
   catcacatca tttcaccccc ac 22
<210> 131
   <211> 23
   <212> DNA
   <213> homo sapiens
<400> 131
   cccctttttt gtccagctta ctc 23
<210> 132
   <211> 22
   <212> DNA
   <213> homo sapiens
<400> 132
   atgtgcctgc cactacctca tc 22
<210> 133
   <211> 20
   <212> DNA
   <213> homo sapiens
<400> 133
   gccactgaac caaaatcggg 20
<210> 134
   <211> 20
   <212> DNA
   <213> homo sapiens
<400> 134
   actcgcacag gcacagggat 20
<210> 135
   <211> 20
   <212> DNA
   <213> homo sapiens
<400> 135
   gccccgctcc aaacatcact 20
<210> 136
   <211> 23
   <212> DNA
   <213> homo sapiens
<400> 136
   ttctgcctga agaagcgtca tac 23
<210> 137
   <211> 24
   <212> DNA
   <213> homo sapiens
<400> 137
   gccatcctct ctgtcaagta ccag 24
<210> 138
   <211> 28
   <212> DNA
   <213> homo sapiens
<400> 138
   gcgcattttg agagaagttg ggtactgg 28
<210> 139
   <211> 24
   <212> DNA
   <213> homo sapiens
<400> 139
   gaattcgtgg tggcatgccc ttct 24
<210> 140
   <211> 24
   <212> DNA
   <213> homo sapiens
<400> 140
   aacccctctt tctgtccatg ccag 24
<210> 141
   <211> 24
   <212> DNA
   <213> homo sapiens
<400> 141
   tcttcaatac ccaggaggta cagg 24
<210> 142
   <211> 22
   <212> DNA
   <213> homo sapiens
<400> 142
   gcagtcttgg atgatgggtt cc 22
<210> 143
   <211> 24
   <212> DNA
   <213> homo sapiens
<400> 143
   aaggagttag cagcagccta gttg 24
<210> 144
   <211> 20
   <212> DNA
   <213> homo sapiens
<400> 144
   tttctatggc attccagcgg 20
<210> 145
   <211> 23
   <212> DNA
   <213> homo sapiens
<400> 145
   agaagctatc aggcgttgct gaa 23
<210> 146
   <211> 22
   <212> DNA
   <213> homo sapiens
<400> 146
   cttcacctgc tcattgcctg tc 22
<210> 147
   <211> 21
   <212> DNA
   <213> homo sapiens
<400> 147
   tgccgtggta atgtgaatcc c 21
<210> 148
   <211> 22
   <212> DNA
   <213> homo sapiens
<400> 148
   aacgacgaaa agagaaggac cc 22
<210> 149
   <211> 23
   <212> DNA
   <213> homo sapiens
<400> 149
   ggaaagtgtt agacgcagaa ggc 23
<210> 150
   <211> 24
   <212> DNA
   <213> homo sapiens
<400> 150
   aaagttgcag acaaaggcgg aagc 24
<210> 151
   <211> 25
   <212> DNA
   <213> homo sapiens
<400> 151
   tggacctact tcgtacatca gaggc 25
<210> 152
   <211> 21
   <212> DNA
   <213> homo sapiens
<400> 152
   gacggtggga acggtttaga g 21
<210> 153
   <211> 20
   <212> DNA
   <213> homo sapiens
<400> 153
   aggcttccaa cttccgctgc 20
<210> 154
   <211> 21
   <212> DNA
   <213> homo sapiens
<400> 154
   cacctgcatc catagcacag c 21
<210> 155
   <211> 22
   <212> DNA
   <213> homo sapiens
<400> 155
   tcggaagggt gtgaaagagg ac 22
<210> 156
   <211> 25
   <212> DNA
   <213> homo sapiens
<400> 156
   cccttctttg gtttgcatca ggtct 25
<210> 157
   <211> 26
   <212> DNA
   <213> homo sapiens
<400> 157
   cgttgggtct tgatcagctt ctgttg 26
<210> 158
   <211> 20
   <212> DNA
   <213> homo sapiens
<400> 158
   tgtctgttgc atgcggttca 20
<210> 159
   <211> 22
   <212> DNA
   <213> homo sapiens
<400> 159
   ccacggtgta gaagagcgat ac 22
<210> 160
   <211> 21
   <212> DNA
   <213> homo sapiens
<400> 160
   ggtcactgat agccgatgag g 21
<210> 161
   <211> 20
   <212> DNA
   <213> homo sapiens
<400> 161
   ctcctgagct ccacgatctg 20
<210> 162
   <211> 25
   <212> DNA
   <213> homo sapiens
<400> 162
   gttggtctcc atgttcctgc ctaac 25
<210> 163
   <211> 23
   <212> DNA
   <213> homo sapiens
<400> 163
   cagcatcacc ttgacgtagc tga 23
<210> 164
   <211> 22
   <212> DNA
   <213> homo sapiens
<400> 164
   cgagcatgag gataaagccc ag 22
<210> 165
   <211> 23
   <212> DNA
   <213> homo sapiens
<400> 165
   gtagcagcca gtcagcatct tcg 23
<210> 166
   <211> 20
   <212> DNA
   <213> homo sapiens
<400> 166
   tggcaatgaa gcacccctct 20
<210> 167
   <211> 23
   <212> DNA
   <213> homo sapiens
<400> 167
   ggtccacact gctctcactt cct 23
<210> 168
   <211> 28
   <212> DNA
   <213> homo sapiens
<400> 168
   tcacatctat caaccactgg cacctacc 28
<210> 169
   <211> 25
   <212> DNA
   <213> homo sapiens
<400> 169
   gggttcactt tggtctctag tacgg 25
<210> 170
   <211> 20
   <212> DNA
   <213> homo sapiens
<400> 170
   caagcaaatg caatggctgg 20
<210> 171
   <211> 24
   <212> DNA
   <213> homo sapiens
<400> 171
   ggatgtgcag tgaaacttga aagg 24
<210> 172
   <211> 25
   <212> DNA
   <213> homo sapiens
<400> 172
   gctgtgttct ctctggataa cccac 25
<210> 173
   <211> 25
   <212> DNA
   <213> homo sapiens
<400> 173
   gccatttggt ttggatgtat tgaag 25
<210> 174
   <211> 24
   <212> DNA
   <213> homo sapiens
<400> 174
   ctgggacctt ccaaaacatt ggct 24
<210> 175
   <211> 25
   <212> DNA
   <213> homo sapiens
<400> 175
   cattacctga ggcctctgaa ttcgg 25
<210> 176
   <211> 24
   <212> DNA
   <213> homo sapiens
<400> 176
   tccaggtaca tatcacgcgc acag 24
<210> 177
   <211> 25
   <212> DNA
   <213> homo sapiens
<400> 177
   ccagatgcag gatcaaccct tctca 25
<210> 178
   <211> 24
   <212> DNA
   <213> homo sapiens
<400> 178
   gtgctgtcgt gggtgctgtg tctt 24
<210> 179
   <211> 22
   <212> DNA
   <213> homo sapiens
<400> 179
   cacgtgatag atgctggtcg gg 22
<210> 180
   <211> 23
   <212> DNA
   <213> homo sapiens
<400> 180
   cttgataatg tgggcaaacc ctt 23
<210> 181
   <211> 20
   <212> DNA
   <213> homo sapiens
<400> 181
   gcccggaatc atgatgcttg 20
<210> 182
   <211> 21
   <212> DNA
   <213> homo sapiens
<400> 182
   ggccctagga ttgtccactc a 21
<210> 183
   <211> 22
   <212> DNA
   <213> homo sapiens
<400> 183
   tcaggacttg cctttgtttc gg 22
<210> 184
   <211> 20
   <212> DNA
   <213> homo sapiens
<400> 184
   gtgggcctgt gcattgttgg 20
<210> 185
   <211> 20
   <212> DNA
   <213> homo sapiens
<400> 185
   tagggcaccg ggatctctaa 20
<210> 186
   <211> 24
   <212> DNA
   <213> homo sapiens
<400> 186
   cctgtttgct gctgagaaca tctc 24
<210> 187
   <211> 23
   <212> DNA
   <213> homo sapiens
<400> 187
   aacgctcccc tgaaaactgt aac 23
<210> 188
   <211> 22
   <212> DNA
   <213> homo sapiens
<400> 188
   acagagacca aagaacccaa ga 22
<210> 189
   <211> 23
   <212> DNA
   <213> homo sapiens
<400> 189
   ggatcgacat gattctgaag gtg 23
<210> 190
   <211> 24
   <212> DNA
   <213> homo sapiens
<400> 190
   tctccagtgt acccatgatg gaag 24
<210> 191
   <211> 24
   <212> DNA
   <213> homo sapiens

<400> 191
   cccacaagtg tgatcttgaa gtcc 24
<210> 192
   <211> 23
   <212> DNA
   <213> homo sapiens
<400> 192
   cactgtgagt ttcatgcctg ctg 23
<210> 193
   <211> 22
   <212> DNA
   <213> homo sapiens
<400> 193
   tcgtggtttc ctggacatct tc 22
<210> 194
   <211> 22
   <212> DNA
   <213> homo sapiens
<400> 194
   gggcttggtt ttgtgaggtt cc 22
<210> 195
   <211> 23
   <212> DNA
   <213> homo sapiens
<400> 195
   cagggacttc ctttttccat cag 23
<210> 196
   <211> 27
   <212> DNA
   <213> homo sapiens
<400> 196
   ccaagttacg tcaaagtctc aggagca 27
<210> 197
   <211> 25
   <212> DNA
   <213> homo sapiens
<400> 197
   tctgaagggg tgaagttctt gaggg 25
<210> 198
   <211> 24
   <212> DNA
   <213> homo sapiens
<400> 198
   aagagaagac catccacctt accc 24
<210> 199
   <211> 22
   <212> DNA
   <213> homo sapiens
<400> 199
   tgctttcact tgtgccactg ag 22
<210> 200
   <211> 20
   <212> DNA
   <213> homo sapiens
<400> 200
   cggccaagga ctggaccaga 20
<210> 201
   <211> 25
   <212> DNA
   <213> homo sapiens
<400> 201
   actccatgag catgcacaga gtagg 25
<210> 202
   <211> 20
   <212> DNA
   <213> homo sapiens
<400> 202
   actgccaatc ctgcgttcca 20
<210> 203
   <211> 23
   <212> DNA
   <213> homo sapiens
<400> 203
   cgacgtggcc attcaatcgt aca 23
<210> 204
   <211> 21
   <212> DNA
   <213> homo sapiens
<400> 204
   cgcaccacga cgatgacgtt c 21
<210> 205
   <211> 22
   <212> DNA
   <213> homo sapiens
<400> 205
   gatggacccc aggacggagt ag 22
<210> 206
   <211> 23
   <212> DNA
   <213> homo sapiens
<400> 206
   gaccaatagc atctgtgcca gag 23
<210> 207
   <211> 25
   <212> DNA
   <213> homo sapiens
<400> 207
   tgcttctaac cactgaggta tgagg 25
<210> 208
   <211> 23
   <212> DNA
   <213> homo sapiens
<400> 208
   tcctaaacca ttcaccaaga gcc 23
<210> 209
   <211> 23
   <212> DNA
   <213> homo sapiens
<400> 209
   gagcgttgct ttccttaaag ace 23
<210> 210
   <211> 22
   <212> DNA
   <213> homo sapiens
<400> 210
   agcctccctg tctactccat tc 22
<210> 211
   <211> 20
   <212> DNA
   <213> homo sapiens
<400> 211
   ggtaaagtcc tcacccctgc 20
<210> 212
   <211> 18
   <212> DNA
   <213> homo sapiens
<400> 212
   gtggagggag gcctggac 18
<210> 213
   <211> 19
   <212> DNA
   <213> homo sapiens
<400> 213
   ttaggccacg tgtctgcca 19
<210> 214
   <211> 21
   <212> DNA
   <213> homo sapiens
<400> 214
   atgccacaca agccagctca c 21
<210> 215
   <211> 22
   <212> DNA
   <213> homo sapiens
<400> 215
   cagcagcaga tgggaagaac tc 22
<210> 216
   <211> 23
   <212> DNA
   <213> homo sapiens
<400> 216
   gctatgtctt tgcaccagcc acc 23
<210> 217
   <211> 23
   <212> DNA
   <213> homo sapiens
<400> 217
   gcccacctct acgtcgaaga agt 23
<210> 218
   <211> 20
   <212> DNA
   <213> homo sapiens
<400> 218
   tccattgtgt cggggatctg 20
<210> 219
   <211> 20
   <212> DNA
   <213> homo sapiens
<400> 219
   taccccgcag agaagcaaac 20
<210> 220
   <211> 20
   <212> DNA
   <213> homo sapiens
<400> 220
   agccgagcat acacaccacc 20
<210> 221
   <211> 20
   <212> DNA
   <213> homo sapiens
<400> 221
   tccagggaga tgtcttgcca 20
<210> 222
   <211> 21
   <212> DNA
   <213> homo sapiens
<400> 222
   atcctacaac cgagccaacc g 21
<210> 223
   <211> 20
   <212> DNA
   <213> homo sapiens
<400> 223
   cctgctcctt ctttgcctgg 20
<210> 224
   <211> 21
   <212> DNA
   <213> homo sapiens
<400> 224
   ccgctgaact cagtcaatgg c 21
<210> 225
   <211> 20
   <212> DNA
   <213> homo sapiens
<400> 225
   tgctgttcat ccaaccaccg 20
<210> 226
   <211> 20
   <212> DNA
   <213> homo sapiens
<400> 226
   tgggcagttt gacttcagca 20
<210> 227
   <211> 21
   <212> DNA
   <213> homo sapiens
<400> 227
   cactgtaggt cagtcacagc a 21
<210> 228
   <211> 21
   <212> DNA
   <213> homo sapiens
<400> 228
   gtgttcgagg gagtgatagg g 21
<210> 229
   <211> 20
   <212> DNA
   <213> homo sapiens
<400> 229
   cagaccctga ggttgcagaa 20
<210> 230
   <211> 23
   <212> DNA
   <213> homo sapiens
<400> 230
   tgcaccaact gcgtgcagga cga 23
<210> 231
   <211> 25
   <212> DNA
   <213> homo sapiens
<400> 231
   ggcaccacgg ggttgtagaa gtccc 25
<210> 232
   <211> 20
   <212> DNA
   <213> homo sapiens
<400> 232
   tgttggccgg ccttatggtg 20
<210> 233
   <211> 21
   <212> DNA
   <213> homo sapiens
<400> 233
   ggcggagtaa ggcttgtctg t 21
<210> 234
   <211> 24
   <212> DNA
   <213> homo sapiens
<400> 234
   ggagacttcc gacttcctta cagg 24
<210> 235
   <211> 23
   <212> DNA
   <213> homo sapiens
<400> 235
   cctaccacta tggcttgtga tgg 23
<210> 236
   <211> 24
   <212> DNA
   <213> homo sapiens
<400> 236
   ggacttgcga gacttcgtga ggag 24
<210> 237
   <211> 22
   <212> DNA
   <213> homo sapiens
<400> 237
   ctgaaagagg gtctgcgtgt gg 22
<210> 238
   <211> 24
   <212> DNA
   <213> homo sapiens
<400> 238
   cttctcttcc cttcattctt cgcc 24
<210> 239
   <211> 24
   <212> DNA
   <213> homo sapiens
<400> 239
   cctcctgacc atctcctctt cctc 24
<210> 240
   <211> 24
   <212> DNA
   <213> homo sapiens
<400> 240
   ggttcttagg ctccatcctg tttg 24
<210> 241
   <211> 26
   <212> DNA
   <213> homo sapiens
<400> 241
   gctgagtaga catgcagatg acaagc 26
<210> 242
   <211> 22
   <212> DNA
   <213> homo sapiens
<400> 242
   agaccaagca cacctggcaa cg 22
<210> 243
   <211> 22
   <212> DNA
   <213> homo sapiens
<400> 243
   atcttccttg tccgtctcgt cc 22
<210> 244
   <211> 25
   <212> DNA
   <213> homo sapiens
<400> 244
   ccccgaggac aacctggaga tcgtt 25
<210> 245
   <211> 25
   <212> DNA
   <213> homo sapiens
<400> 245
   cctcgttcgc caccgtatag ttgat 25
<210> 246
   <211> 24
   <212> DNA
   <213> homo sapiens
<400> 246
   caaccacaga tcagggacag gagc 24
<210> 247
   <211> 20
   <212> DNA
   <213> homo sapiens
<400> 247
   ggacagtggc gatttcaacc 20
<210> 248
   <211> 21
   <212> DNA
   <213> homo sapiens
<400> 248
   gctctttgtg agtgagggtg g 21
<210> 249
   <211> 21
   <212> DNA
   <213> homo sapiens
<400> 249
   acaggggtgt ggacagaaat g 21
<210> 250
   <211> 22
   <212> DNA
   <213> homo sapiens
<400> 250
   cagtgggcag cagaaaggag ag 22
<210> 251
   <211> 19
   <212> DNA
   <213> homo sapiens
<400> 251
   gggaggagct gaggcaatc 19
<210> 252
   <211> 25
   <212> DNA
   <213> homo sapiens
<400> 252
   agaaggagat cactgccctg gcacc 25
<210> 253
   <211> 24
   <212> DNA
   <213> homo sapiens
<400> 253
   cctgcttgct gatccacatc tgct 24
<210> 254
   <211> 613
   <212> DNA
   <213> homo sapiens
<400> 254
   gccatggaaa aaccctcgtt tatttgatta aacaaaaata aaataagctg cataggaaca     60
   attttaaagt ccaaagagac accaactttg ttttaaggct gtagtagctg atacagcatc    120
   tccttgctac ctcctccagc cttctctgtg gaccacagtg atacattcag aagcctgtta    180
   gctaacacag gagtttttga acacttttcc attggttctt cacctgctca ttgcctgtca    240
   tgcctgcggc ctgcaattag taacatttta agatttaaaa tgtgaaagcc aaaagaggag    300
   gggggaaaaa aacccaaaat caaccaaaca aaaactcact tttgcctaaa ggtttggggg    360
   gaaatcttca tttccccacc catctactgc attgatggga ttcacattac cacggcagtg    420
   cccatttaaa agtggcaaat ggcagtcagc accccacccc ggacaggctt gcagtgtggt    480
   ttctgcggcc aggggagtta ctacggtagt gcatattcct tatatcatgt ctgccttgga    540
   caaatacaaa ttaagaggtt taacttagtc attctaacat aaggcaactt gcccacatta    600
   attcccccaa ttg                                                       613
<210> 255
   <211> 23
   <212> DNA
   <213> homo sapiens
<400> 255
   atccgccagg tgaaagccaa gtc 23
<210> 256
   <211> 25
   <212> DNA
   <213> homo sapiens
<400> 256
   gggattcaca ttaccacggc agtgc 25
<210> 257
   <211> 2237
   <212> DNA
   <213> homo sapiens
<400> 257
   atccgccagg tgaaagccaa gtctctgtac ctgcaggtgg agaagctgcg gcaaaacctc     60
   aacaagcttg agagcaccat cagtgccgtg cagcaggtcc tggaggaggg tagagcgcta    120
   gacatcctac tggcccgaga ccggatgctg gcccaggtgc aggagctgaa gaccgtgcgg    180
   agcctcctgc agccccagga agacgaccga gtcatgttca caccccccga tcaggcactg    240
   taccttgcca tcaagtcttt tggctttgtt agcagcgggg cctttgcccc actcaccaag    300
   gccacaggcg atggcctcaa gcgtgccctc cagggtaagg tggcctcctt cacagtcatt    360
   ggttatgacc acgatggtga gccccgcctc tcaggaggcg acctgatgtc ggctgtggtc    420
   ctgggccctg atggcaacct gtttggtgca gaggtgagtg atcagcagaa tgggacatac    480
   gtggtgagtt accgacccca gctggagggt gagcacctgg tatctgtgac actgtgcaac    540
   cagcacattg agaacagccc tttcaaggtg gtggtcaagt caggccgcag ctacgtgggc    600
   attgggctcc cgggcctgag cttcggcagt gagggtgaca gcgatggcaa gctctgccgc    660
   ccttggggtg tgagtgtaga caaggagggc tacatcattg tcgccgaccg cagcaacaac    720
   cgcatccagg tgttcaagcc ctgcggcgcc ttccaccaca aattcggcac cctgggctcc    780
   cggcctgggc agttcgaccg accagccggc gtggcctgtg acgcctcacg caggatcgtg    840
   gtggctgaca aggacaatca tcgcatccag atcttcacgt tcgagggcca gttcctcctc    900
   aagtttggtg agaaaggaac caagaatggg cagttcaact acccttggga tgtggcggtg    960
   aattctgagg gcaagatcct ggtctcagac acgaggaacc accggatcca gctgtttggg   1020
   cctgatggtg tcttcctaaa caagtatggc ttcgaggggg ctctctggaa gcactttgac   1080
   tccccacggg gtgtggcctt caaccatgag ggccacttgg tggtcactga cttcaacaac   1140
   caccggctcc tggttattca ccccgactgc cagtcggcac gctttctggg ctcggagggc   1200
   acaggcaatg ggcagttcct gcgcccacaa ggggtagctg tggaccagga agggcgcatc   1260
   attgtggcgg attccaggaa ccatcgggta cagatgtttg aatccaacgg cagcttcctg   1320
   tgcaagtttg gtgctcaagg cagcggcttt gggcagatgg accgcccttc cggcatcgcc   1380
   atcacccccg acggaatgat cgttgtggtg gactttggca acaatcgaat cctcgtcttc   1440
   taattgcatt tcctaggttt ctgtgtttgg ggtgtgtgtg cgtgtctctc tctctctctc   1500
   tctctttctc tttctctctc tttttgaatt tcaaagaaga aacagtctca gggaaatttc   1560
   ttttttcttt ttttttttta aagagaacaa gaaaagtaca acattgctta agtcctacct   1620
   catctttatt tttttacaga tgaatgtact tatcttttct gcagggattg agcctgtgaa   1680
   gtgataattt ctatctacct cataaatctt tacatttcct tctgcaacag gccctcttcc   1740
   cctcctcagt ggagtttgca tttccctctt cccctgcgtg gggcatgata tgcacaagcc   1800
   tggcatctgt atggctggga gggcactgga tgtgtgtggt ggggtgtatt ctgtagattg   1860
   agccaaggaa acacaaaaaa aaactactaa gtaaaaaaac aaaaaactat aaaacatgga   1920
   aaaaatagga tttgaaatgc ataattatag aatacctgtg ttcttgagaa tactgtttat   1980
   atgggtttta gattatgttg tgttgttttg atctttttgg aaaatcttct ctttttaaat   2040
   gctgcaacag agaaatttcc tctgttctct gtttatacct cttaattgta ttgtccaagg   2100
   cagacatgat ataaggaata tgcactaccg tagtaactcc cctggccgca gaaaccacac   2160
   tgcaagcctg tccggggtgg ggtgctgact gccatttgcc acttttaaat gggcactgcc   2220
   gtggtaatgt gaatccc                                                  2237
<210> 258
   <211> 27
   <212> DNA
   <213> homo sapiens
<400> 258
   atggcttcgt tccccgagac cgatttc 27
<210> 259
   <211> 34
   <212> DNA
   <213> homo sapiens
<400> 259
   gaagacgagg attcgattgt tgccaaagtc cacc 34
<210> 260
   <211> 2604
   <212> DNA
   <213> homo sapiens
<400> 260
   atggcttcgt tccccgagac cgatttccag atctgcttgc tgtgcaagga gatgtgcggc      60
   tcgccggcgc cgctctcctc caactcgtcc gcgtcgtcgt cctcctcgca gacgtccacg     120
   tcgtcggggg gcggcggcgg gggccctggg gcggcggcgc gccgcctaca cgtcctgccc     180
   tgcctgcacg ccttctgccg cccctgcctc gaggcgcacc ggctgccggc ggcgggcggc     240
   ggcgcggcgg gagagccgct caagctgcgc tgccccgtgt gcgaccagaa agtagtgcta     300
   gccgaggcgg cgggtatgga cgcgctgcct tcgtccgcct tcctgcttaa caacctgctc     360
   gacgcggtgg tggccactgc cgacgagccg ccgcccaaga acgggcgcgc cggcgctccg     420
   gcgggagcgg gcggccacag caaccaccgg caccacgctc accacgcgca cccgcgcgcg     480
   tccgcctccg cgccgccact cccgcaggcg ccgcagccgc ccgcgccttc ccgctcggca     540
   cccggcggcc ctgccgcttc cccgtcggcg ctgctgctcc gccgtcctca cggctgcagc     600
   tcgtgcgatg agggcaacgc agcttcttcg cgctgcctcg actgccagga gcacctgtgc     660
   gacaactgcg tccgagcgca ccagcgcgtg cgcctcacca aggaccacta catcgagcgc     720
   ggcccgccgg gtcccggtgc cgcagcagcg gcgcagcagc tcgggctcgg gccgcccttt     780
   cccggcccgc ccttctccat cctctcagtg tttcccgagc gcctcggctt ctgccagcac     840
   cacgacgacg aggtgctgca cctgtactgt gacacttgct ctgtacccat ctgtcgtgag     900
   tgcacaatgg gccggcatgg gggccacagc ttcatctacc tccaggaggc actgcaggac     960
   tcacgggcac tcaccatcca gctgctggca gatgcccagc agggacgaca ggcaatccag    1020
   ctgagcatcg agcaggccca gacggtggcg gaacaggtgg agatgaaggc gaaggttgtg    1080
   cagtcggagg tcaaagccgt gactgcgagg cataagaaag ccctggagga acgcgagtgt    1140
   gagctgctgt ggaaggtaga aaagatccgc caggtgaaag ccaagtctct gtacctgcag    1200
   gtggagaagc tgcggcaaaa cctcaacaag cttgagagca ccatcagtgc cgtgcagcag    1260
   gtcctggagg agggtagagc gctagacatc ctactggccc gagaccggat gctggcccag    1320
   gtgcaggagc tgaagaccgt gcggagcctc ctgcagcccc aggaagacga ccgagtcatg    1380
   ttcacacccc ccgatcaggc actgtacctt gccatcaagt cttttggctt tgttagcagc    1440
   ggggcctttg ccccactcac caaggccaca ggcgatggcc tcaagcgtgc cctccagggt    1500
   aaggtggcct ccttcacagt cattggttat gaccacgatg gtgagccccg cctctcagga    1560
   ggcgacctga tgtcggctgt ggtcctgggc cctgatggca acctgtttgg tgcagaggtg    1620
   agtgatcagc agaatgggac atacgtggtg agttaccgac cccagctgga gggtgagcac    1680
   ctggtatctg tgacactgtg caaccagcac attgagaaca gccctttcaa ggtggtggtc    1740
   aagtcaggcc gcagctacgt gggcattggg ctcccgggcc tgagcttcgg cagtgagggt    1800
   gacagcgatg gcaagctctg ccgcccttgg ggtgtgagtg tagacaagga gggctacatc    1860
   attgtcgccg accgcagcaa caaccgcatc caggtgttca agccctgcgg cgccttccac    1920
   cacaaattcg gcaccctggg ctcccggcct gggcagttcg accgaccagc cggcgtggcc    1980
   tgtgacgcct cacgcaggat cgtggtggct gacaaggaca atcatcgcat ccagatcttc    2040
   acgttcgagg gccagttcct cctcaagttt ggtgagaaag gaaccaagaa tgggcagttc    2100
   aactaccctt gggatgtggc ggtgaattct gagggcaaga tcctggtctc agacacgagg    2160
   aaccaccgga tccagctgtt tgggcctgat ggtgtcttcc taaacaagta tggcttcgag    2220
   ggggctctct ggaagcactt tgactcccca cggggtgtgg ccttcaacca tgagggccac    2280
   ttggtggtca ctgacttcaa caaccaccgg ctcctggtta ttcaccccga ctgccagtcg    2340
   gcacgctttc tgggctcgga gggcacaggc aatgggcagt tcctgcgccc acaaggggta    2400
   gctgtggacc aggaagggcg catcattgtg gcggattcca ggaaccatcg ggtacagatg    2460
   tttgaatcca acggcagctt cctgtgcaag tttggtgctc aaggcagcgg ctttgggcag    2520
   atggaccgcc cttccggcat cgccatcacc cccgacggaa tgatcgttgt ggtggacttt    2580
   ggcaacaatc gaatcctcgt cttc                                           2604
<210> 261
   <211> 649
   <212> DNA
   <213> homo sapiens
<400> 261
   tttttttttt tttttttttt tttttttttt ttttttaaag gccaacgtta tatgttttat      60
   ttttaatcaa gtaatttttt taaacctcaa catatccata attgacagaa taagacatta     120
   ggcatgaaat gagaatataa aacccagagt gtacagaaag gcaaacagtg cttcagtccc     180
   tccagatgac tgcatgcgag tgaaacgcat tccaaacatc tacccctttt ttgtctttta     240
   aaaacataca gttcaagtgt ttttattttc acttgtaaac aacatgagtc aaaatggaca     300
   atgaggtaat ctaaatacta cacaggataa tttaaaaaca ctagataaaa caccattatt     360
   aaagacatat aaaaatcagg gttcactttg gtctctagta cggttagttg ttaataccaa     420
   aaagcaacac atactaattg aagccagctt ggtggtaggt gccagtggtt gatagatgtg     480
   attcaagagg tagtcaacac catcattttc aagctggtaa ctctcacaag tgttaagcca     540
   cagatgcatt aaatatcata atacttctca cagtacagta ctttaccata acttgcagct     600
   ttgttccaaa tataaaattt aaaaatgaat cataggtcaa cttctcact                 649
<210> 262
   <211> 25
   <212> DNA
   <213> homo sapiens
<400> 262
   gggttcactt tggtctctag tacgg 25
<210> 263
   <211> 3087
   <212> DNA
   <213> homo sapiens
<400> 263
   atgcgcggac tgacccagcg gccggcgcgg cggcgccggg cggacttaat cgcgggcgca      60
   gcgcgaggct cgggacccag agcaccacct accggcggca cggtcggcgc agcaggcccc     120
   agaagggcgg ggaacgctgt caagcccagg ggcacttcgg cgaggagccc cacccgccct     180
   ccagctgacc ctcagctgtg gcccacatcc ggggcccaga gcgccgcgga aacgccgaag     240
   cccggccggc agatagcgcg gaaagcgaag aaggaagttc ccgtccctcc taaagccgaa     300
   gccaaagcga agtctttaaa ggccaagaag gcagtgttga aaggtgtccg cagccacaaa     360
   aaaaagaaga tccgcacgtc acccacctta cggcggccca agacaccgcg actccggaga     420
   cagcccaaat atcctcggaa gagcgctcct aggagaaaca agcttgacca ctatgctatc     480
   atcaagtttc tgctgaccac tgagtctgcc atgaagaaga tagaagacaa taacacactt     540
   gtgttcattg tggatgttaa agccaacaag caccagatta aacaggctgt gaagaagctc     600
   tatgacaaag atgtggtcaa ggtcaacacc ctgattcggc ctgatggaga gaagaaggcg     660
   ccgcagccgc ccgcgccttc ccgctcggca cccggcggcc ctgccgcttc cccgtcggcg     720
   ctgctgctcc gccgtcctca cggctgcagc tcgtgcgatg agggcaacgc agcttcttcg     780
   cgctgcctcg actgccagga gcacctgtgc gacaactgcg tccgagcgca ccagcgcgtg     840
   cgcctcacca aggaccacta catcgagcgc ggcccgccgg gtcccggtgc cgcagcagcg     900
   gcgcagcagc tcgggctcgg gccgcccttt cccggcccgc ccttctccat cctctcagtg     960
   tttcccgagc gcctcggctt ctgccagcac cacgacgacg agttggggct tttcactagt    1020
   tctgtgcctc cagagtccga aaggcctgca ggctccgtgg cccagccggc atccgggcgg    1080
   ggaatccaag gcgaggaatc cgaggtcgcc gtccccggaa cagctggccg cgggcccgct    1140
   gcgtgccgcg ggtcccggga gaggcgggcg caggctagag cagcaaagga aacttttctg    1200
   gtacattctt acatccaggc cactaatatc agactaggta acacagtctt aacaactttt    1260
   ctggataatg aagctaagat tcagggcaaa ctctcatgcc aggaggtgct gcacctgtac    1320
   tgtgacactt gctctgtacc catctgtcgt gagtgcacaa tgggccggca tgggggccac    1380
   agcttcatct acctccagga ggcactgcag gactcacggg cactcaccat ccagctgctg    1440
   gcagatgccc agcagggacg acaggcaatc cagacaaagc agaagaagct gcttctgcag    1500
   ctgagcatcg agcaggccca gacggtggcg gaacaggtgg agatgaaggc gaaggttgtg    1560
   cagtcggagg tcaaagccgt gacggcgagg cataagaaag ccctggagga acgcgagtgt    1620
   gagctgctgt ggaaggtaga aaagatccgc caggtgaaag ccaagtctct gtacctgcag    1680
   gtggagaagc tgcggcaaaa cctcaacaag cttgagagca ccatcagtgc cgtgcagcag    1740
   gtcctggagg agggtagagc gctagacatc ctactggccc gagaccggat gctggcccag    1800
   gtgcaggagc tgaagaccgt gcggagcctc ctgcagcccc aggaagacga ccgagtcatg    1860
   ttcacacccc ccgatcaggc actgtacctt gccatcaagt cttttggctt tgttagcagc    1920
   ggggcctttg ccccactcac caaggccaca ggcgatggcc tcaagcgtgc cctccagggt    1980
   aaggtggcct ccttcacagt cattggttat gaccacgatg gtgagccccg cctctcagga    2040
   ggcgacctga tgtcggctgt ggtcctgggc cctgatggca acctgtttgg tgcagaggtg    2100
   agtgatcagc agaatgggac atacgtggtg agttaccgac cccagctgga gggtgagcac    2160
   ctggtatctg tgacactgtg caaccagcac attgagaaca gccctttcaa ggtggtggtc    2220
   aagtcaggcc gcagctacgt gggcattggg ctcccgggcc tgagcttcgg cagtgagggt    2280
   gacagcgatg gcaagctctg ccgcccttgg ggtgtgagtg tagacaagga gggctacatc    2340
   attgtcgccg accgcagcaa caaccgcatc caggtgttca agccctgcgg cgccttccac    2400
   cacaaattcg gcaccctggg ctcccggcct gggcagttcg accgaccagc cggcgtggcc    2460
   tgtgacgcct cacgcaggat cgtggtggct gacaaggaca atcatcgcat ccagatcttc    2520
   acgttcgagg gccagttcct cctcaagttt ggtgagaaag gaaccaagaa tgggcagttc    2580
   aactaccctt gggatgtggc ggtgaattct gagggcaaga tcctggtctc agacacgagg    2640
   aaccaccgga tccagctgtt tgggcctgat ggtgtcttcc taaacaagta tggcttcgag    2700
   ggggctctct ggaagcactt tgactcccca cggggtgtgg ccttcaacca tgagggccac    2760
   ttggtggtca ctgacttcaa caaccaccgg ctcctggtta ttcaccccga ctgccagtcg    2820
   gcacgctttc tgggctcgga gggcacaggc aatgggcagt tcctgcgccc acaaggggta    2880
   gctgtggacc aggaagggcg catcattgtg gcggattcca ggaaccatcg ggtacagatg    2940
   tttgaatcca acggcagctt cctgtgcaag tttggtgctc aaggcagcgg ctttgggcag    3000
   atggaccgcc cttccggcat cgccatcacc cccgacggaa tgatcgttgt ggtggacttt    3060
   ggcaacaatc gaatcctcgt cttctaa                                        3087
<210> 264
   <211> 209
   <212> PRT
   <213> Homo sapiens
<400> 264
   Met Gly Ser Val Ser Asn Gln Gln Phe Ala Gly Gly Cys Ala Lys Ala
   1               5                   10                  15
   Ala Glu Glu Ala Pro Glu Glu Ala Pro Glu Asp Ala Ala Arg Ala Ala
               20                  25                  30
   Asp Glu Pro Gln Leu Leu His Gly Ala Gly Ile Cys Lys Trp Phe Asn
           35                  40                  45
   Val Arg Met Gly Phe Gly Phe Leu Ser Met Thr Ala Arg Ala Gly Val
       50                  55                  60
   Ala Leu Asp Pro Pro Val Asp Val Phe Val His Gln Ser Lys Leu His
   65                  70                  75                  80
   Met Glu Gly Phe Arg Ser Leu Lys Glu Gly Glu Ala Val Glu Phe Thr
                   85                  90                  95
   Phe Lys Lys Ser Ala Lys Gly Leu Glu Ser Ile Arg Val Thr Gly Pro
               100                 105                 110
   Gly Gly Val Phe Cys Ile Gly Ser Glu Arg Arg Pro Lys Gly Lys Ser
           115                 120                 125
   Met Gln Lys Arg Arg Ser Lys Gly Asp Arg Cys Tyr Asn Cys Gly Gly
       130                 135                 140
   Leu Asp His His Ala Lys Glu Cys Lys Leu Pro Pro Gln Pro Lys Lys
   145                 150                 155                 160
   Cys His Phe Cys Gln Ser Ile Ser His Met Val Ala Ser Cys Pro Leu
                   165                 170                 175
   Lys Ala Gln Gln Gly Pro Ser Ala Gln Gly Lys Pro Thr Tyr Phe Arg
               180                 185                 190
   Glu Glu Glu Glu Glu Ile His Ser Pro Thr Leu Leu Pro Glu Ala Gln
           195                 200                 205
   Asn
<210> 265
   <211> 227
   <212> PRT
   <213> Caenorhabditis elegans
<400> 265
   Met Ser Thr Val Val Ser Glu Gly Arg Asn Asp Gly Asn Asn Arg Tyr
   1               5                   10                  15
   Ser Pro Gln Asp Glu Val Glu Asp Arg Leu Pro Asp Val Val Asp Asn
               20                  25                  30
   Arg Leu Thr Glu Asn Met Arg Val Pro Ser Phe Glu Arg Leu Pro Ser
           35                  40                  45
   Pro Thr Pro Arg Tyr Phe Gly Ser Cys Lys Trp Phe Asn Val Ser Lys
       50                  55                  60
   Gly Tyr Gly Phe Val Ile Asp Asp Ile Thr Gly Glu Asp Leu Phe Val
   65                  70                  75                  80
   His Gln Ser Asn Leu Asn Met Gln Gly Phe Arg Ser Leu Asp Glu Gly
                   85                  90                  95
   Glu Arg Val Ser Tyr Tyr Ile Gln Glu Arg Ser Asn Gly Lys Gly Arg
               100                 105                 110
   Glu Ala Tyr Ala Val Ser Gly Glu Val Glu Gly Gln Gly Leu Lys Gly
           115                 120                 125
   Ser Arg Ile His Pro Leu Gly Arg Lys Lys Ala Val Ser Leu Arg Cys
       130                 135                 140
   Phe Arg Cys Gly Lys Phe Ala Thr His Lys Ala Lys Ser Cys Pro Asn
   145                 150                 155                 160
   Val Lys Thr Asp Ala Lys Val Cys Tyr Thr Cys Gly Ser Glu Glu His
                   165                 170                 175
   Val Ser Ser Ile Cys Pro Glu Arg Arg Arg Lys His Arg Pro Glu Gln
               180                 185                 190
   Val Ala Ala Glu Glu Ala Glu Ala Ala Arg Met Ala Ala Glu Lys Ser
           195                 200                 205
   Ser Pro Thr Thr Ser Asp Asp Asp Ile Arg Glu Lys Asn Ser Asn Ser
       210                 215                 220
   Ser Asp Glu
   225
<210> 266
   <211> 195
   <212> PRT
   <213> Xenopus laevis
<400> 266
   Met Gly Ser Val Ser Asn Gln Glu Ile Thr Glu Gly Leu Pro Lys Ser
   1               5                   10                  15
   Leu Asp Gly Thr Ala Asp Ile His Lys Ser Asp Lys Ser Val Ile Phe
               20                  25                  30
   Gln Gly Ser Gly Val Cys Lys Trp Phe Asn Val Arg Met Gly Phe Gly
           35                  40                  45
   Phe Leu Thr Met Thr Lys Lys Glu Gly Thr Asp Leu Glu Thr Pro Leu
       50                  55                  60
   Asp Val Phe Val His Gln Ser Lys Leu His Met Glu Gly Phe Arg Ser
   65                  70                  75                  80
   Leu Lys Glu Gly Glu Ser Val Glu Phe Thr Phe Lys Lys Ser Ser Lys
                   85                  90                  95
   Gly Leu Glu Ser Thr Gln Val Thr Gly Pro Gly Gly Ala Pro Cys Ile
               100                 105                 110
   Gly Ser Glu Arg Arg Pro Lys Val Lys Gly Gln Gln Lys Arg Arg Gln
           115                 120                 125
   Arg Gly Asp Arg Cys Tyr Asn Cys Gly Gly Leu Asp His His Ala Lys
       130                 135                 140
   Glu Cys Lys Leu Pro Pro Gln Pro Lys Lys Cys His Phe Cys Gln Asn
   145                 150                 155                 160
   Pro Asn His Met Val Ala Gln Cys Pro Glu Lys Ala Met Gln Ala Ala
                   165                 170                 175
   Asn Leu Glu Asp Gln Pro Ile Thr Glu Glu Gln Glu Leu Ile Pro Glu
               180                 185                 190
   Ile Met Glu
           195
<210> 267
   <211> 195
   <212> PRT
   <213> Drosophila melanogaster
<400> 267
   Met Glu Asn Val Gln Leu Glu Asn Gly Leu Glu Arg Arg Thr Thr Ser
   1               5                   10                  15
   Gln Ser Ser Thr Ser Ser Ala Asn Pro Ala Asn Leu Ala Ser Pro Thr
               20                  25                  30
   Glu Glu Cys Gly Cys Val Arg Leu Gly Lys Cys Lys Trp Phe Asn Val
           35                  40                  45
   Ala Lys Gly Trp Gly Phe Leu Thr Pro Asn Asp Gly Gly Gln Glu Val
       50                  55                  60
   Phe Val His Gln Ser Val Ile Gln Met Ser Gly Phe Arg Ser Leu Gly
   65                  70                  75                  80
   Glu Gln Glu Glu Val Glu Phe Glu Cys Gln Arg Thr Ser Arg Gly Leu
                   85                  90                  95
   Glu Ala Thr Arg Val Ser Ser Arg His Gly Gly Ser Cys Gln Gly Ser
               100                 105                 110
   Thr Tyr Arg Pro Arg Ile Asn Arg Arg Thr Arg Arg Met Arg Cys Tyr
           115                 120                 125
   Asn Cys Gly Glu Phe Ala Asn His Ile Ala Ser Glu Cys Ala Leu Gly
       130                 135                 140
   Pro Gln Pro Lys Arg Cys His Arg Cys Arg Gly Glu Asp His Leu His
   145                 150                 155                 160
   Ala Asp Cys Pro His Lys Asn Val Thr Gln Ser His Ser Asn Ser Lys
                   165                 170                 175
   Ser Ile Ser Asn Asn Ser Ser Ser Ser Ala Ala Gln Glu Lys Ser Glu
               180                 185                 190
   Glu Ala Thr
           195
<210> 268
   <211> 209
   <212> PRT
   <213> Mus musculus
<400> 268
   Met Gly Ser Val Ser Asn Gln Gln Phe Ala Gly Gly Cys Ala Lys Ala
   1               5                   10                  15
   Ala Glu Lys Ala Pro Glu Glu Ala Pro Pro Asp Ala Ala Arg Ala Ala
               20                  25                  30
   Asp Glu Pro Gln Leu Leu His Gly Ala Gly Ile Cys Lys Trp Phe Asn
           35                  40                  45
   Val Arg Met Gly Phe Gly Phe Leu Ser Met Thr Ala Arg Ala Gly Val
       50                  55                  60
   Ala Leu Asp Pro Pro Val Asp Val Phe Val His Gln Ser Lys Leu His
   65                  70                  75                  80
   Met Glu Gly Phe Arg Ser Leu Lys Glu Gly Glu Ala Val Glu Phe Thr
                   85                  90                  95
   Phe Lys Lys Ser Ala Lys Gly Leu Glu Ser Ile Arg Val Thr Gly Pro
               100                 105                 110
   Gly Gly Val Phe Cys Ile Gly Ser Glu Arg Arg Pro Lys Gly Lys Asn
           115                 120                 125
   Met Gln Lys Arg Arg Ser Lys Gly Asp Arg Cys Tyr Asn Cys Gly Gly
       130                 135                 140
   Leu Asp His His Ala Lys Glu Cys Lys Leu Pro Pro Gln Pro Lys Lys
   145                 150                 155                 160
   Cys His Phe Cys Gln Ser Ile Asn His Met Val Ala Ser Cys Pro Leu
                   165                 170                 175
   Lys Ala Gln Gln Gly Pro Ser Ser Gln Gly Lys Pro Ala Tyr Phe Arg
               180                 185                 190
   Glu Glu Glu Glu Glu Ile His Ser Pro Ala Leu Leu Pro Glu Ala Gln
           195                 200                 205
   Asn
<210> 269
   <211> 30
   <212> DNA
   <213> homo sapiens
<400> 269
   cgaattcatg ggggccccgc tcgccgtagc 30
<210> 270
   <211> 30
   <212> DNA
   <213> homo sapiens
<400> 270
   cctcgaggag gctgcaggcc tcctggtcca 30
<210> 271
   <211> 21
   <212> DNA
   <213> homo sapiens
<400> 271
   ttcgaacgcc agcacatgga c 21
<210> 272
   <211> 21
   <212> DNA
   <213> homo sapiens
<400> 272
   gctagttatt gctcagcggt g 21
<210> 273
   <211> 2233
   <212> DNA
   <213> homo sapiens
<221> CDS
   <222> (281)..(1768)
<400> 273
   cgattggagc gggccgcagc cagcgcaccc agaccctgcg ctgccctcgg acggccgggc     60
   gcggagcccc agctgcggag gccgacggca cccggccccg agcgcctcga cgccgagccg    120
   cgcgcgcctt ctccgccagg cccggcgggc gggagcgggg gcgagggagc aggagcggcc    180
   agtgcccccg acacccccgg cccggcaccc ccggcccggc atcccccgcc gccgccgccg    240
   ccgcctcaag gccgcccgct ccccgcaggt ggacgcggcc atgggccgag gggtgcgcgt     300
   gctgctgctg ctgagcctgc tgcactgcgc cgggggcagc gagggcagga agacctggcg    360
   gcgccggggt cagcagccgc ctcctccccc gcggaccgag gcggcgccgg cggccggaca    420
   gcccgtggag agcttcccgc tggacttcac ggccgtggag ggtaacatgg acagcttcat    480
   ggcgcaagtc aagagcctgg cgcagtccct gtacccctgc tccgcgcagc agctcaacga    540
   ggacctgcgc ctgcacctcc tactcaacac ctcggtgacc tgcaacgacg gcagccccgc    600
   cggctactac ctgaaggagt ccaggggcag ccggcggtgg ctcctcttcc tggaaggcgg    660
   ctggtactgc ttcaaccgcg agaactgcga ctccagatac gacaccatgc ggcgcctcat    720
   gagctcccgg gactggccgc gcactcgcac aggcacaggg atcctgtcct cacagccgga    780
   ggagaacccc tactggtgga acgcaaacat ggtcttcatc ccctactgct ccagtgatgt    840
   ttggagcggg gcttcatcca agtctgagaa gaacgagtac gccttcatgg gcgccctcat    900
   catccaggag gtggtgcggg agcttctggg cagagggctg agcggggcca aggtgctgct   960
   gctggccggg agcagcgcgg ggggcaccgg ggtgctcctg aatgtggacc gtgtggctga   1020
   gcagctggag aagctgggct acccagccat ccaggtgcga ggcctggctg actccggctg   1080
   gttcctggac aacaagcagt atcgccacac agactgcgtc gacacgatca cgtgcgcgcc   1140
   cacggaggcc atccgccgtg gcatcaggta ctggaacggg gtggtcccgg agcgctgccg   1200
   acgccagttc caggagggcg aggagtggaa ctgcttcttt ggctacaagg tctacccgac   1260
   cctgcgctgc cctgtgttcg tggtgcagtg gctgtttgac gaggcacagc tgacggtgga   1320
   caacgtgcac ctgacggggc agccggtgca ggagggcctg cggctgtaca tccagaacct   1380
   cggccgcgag ctgcgccaca cactcaagga cgtgccggcc agctttgccc ccgcctgcct   1440
   ctcccatgag atcatcatcc ggagccactg gacggatgtc caggtgaagg ggacgtcgct   1500
   gccccgagca ctgcactgct gggacaggag cctccatgac agccacaagg ccagcaagac   1560
   ccccctcaag ggctgccccg tccacctggt ggacagctgc ccctggcccc actgcaaccc   1620
   ctcatgcccc accgtccgag accagttcac ggggcaagag atgaacgtgg cccagttcct   1680
   catgcacatg ggcttcgaca tgcagacggt ggcccagccg cagggactgg agcccagtga   1740
   gctgctgggg atgctgagca acggaagcta ggcagactgt ctggaggagg agccggcact   1800
   gaggggccca gacacccgct gccccagtgc cacctcaccc cccaccagca ggccctcccg   1860
   tctcttcggg acagggcccc agccgtcccc cctgtctggg tctgcccact gccctcctgc   1920
   cccggctttc cctgcccctc tcccacagcc cagccagaga caagggacct gctgtcatcc   1980
   ccatctgtgg cctgggggtc cttcctgaca acgagggggt agccagaaga gaagcactgg   2040
   attcctcagt ccaccagctc agacagcacc caccggcccc acccatcaag cccttttata  2100
   ttattttata aagtgacttt tttattactt taatttttta aaaaaaggaa aataagaata   2160
   tatgatgaat gatattgttt tgtaactttt taaaaatgat tttaaagaga caaaaaagaa   2220
   aaaaaaaaaa aaa                                                      2233
<210> 274
   <211> 496
   <212> PRT
   <213> homo sapiens
<400> 274
   Met Gly Arg Gly Val Arg Val Leu Leu Leu Leu Ser Leu Leu His Cys
   1               5                   10                  15
   Ala Gly Gly Ser Glu Gly Arg Lys Thr Trp Arg Arg Arg Gly Gln Gln
               20                  25                  30
   Pro Pro Pro Pro Pro Arg Thr Glu Ala Ala Pro Ala Ala Gly Gln Pro
           35                  40                  45
   Val Glu Ser Phe Pro Leu Asp Phe Thr Ala Val Glu Gly Asn Met Asp
       50                  55                  60
   Ser Phe Met Ala Gln Val Lys Ser Leu Ala Gln Ser Leu Tyr Pro Cys
   65                  70                  75                  80
   Ser Ala Gln Gln Leu Asn Glu Asp Leu Arg Leu His Leu Leu Leu Asn
                   85                  90                  95
   Thr Ser Val Thr Cys Asn Asp Gly Ser Pro Ala Gly Tyr Tyr Leu Lys
               100                 105                 110
   Glu Ser Arg Gly Ser Arg Arg Trp Leu Leu Phe Leu Glu Gly Gly Trp
           115                 120                 125
   Tyr Cys Phe Asn Arg Glu Asn Cys Asp Ser Arg Tyr Asp Thr Met Arg
       130                 135                 140
   Arg Leu Met Ser Ser Arg Asp Trp Pro Arg Thr Arg Thr Gly Thr Gly
   145                 150                 155                 160
   Ile Leu Ser Ser Gln Pro Glu Glu Asn Pro Tyr Trp Trp Asn Ala Asn
                   165                 170                 175
   Met Val Phe Ile Pro Tyr Cys Ser Ser Asp Val Trp Ser Gly Ala Ser
               180                 185                 190
   Ser Lys Ser Glu Lys Asn Glu Tyr Ala Phe Met Gly Ala Leu Ile Ile
           195                 200                 205
   Gln Glu Val Val Arg Glu Leu Leu Gly Arg Gly Leu Ser Gly Ala Lys
       210                 215                 220
   Val Leu Leu Leu Ala Gly Ser Ser Ala Gly Gly Thr Gly Val Leu Leu
   225                 230                 235                 240
   Asn Val Asp Arg Val Ala Glu Gln Leu Glu Lys Leu Gly Tyr Pro Ala
                   245                 250                 255
   Ile Gln Val Arg Gly Leu Ala Asp Ser Gly Trp Phe Leu Asp Asn Lys
               260                 265                 270
   Gln Tyr Arg His Thr Asp Cys Val Asp Thr Ile Thr Cys Ala Pro Thr
           275                 280                 285
   Glu Ala Ile Arg Arg Gly Ile Arg Tyr Trp Asn Gly Val Val Pro Glu
       290                 295                 300
   Arg Cys Arg Arg Gln Phe Gln Glu Gly Glu Glu Trp Asn Cys Phe Phe
   305                 310                 315                 320
   Gly Tyr Lys Val Tyr Pro Thr Leu Arg Cys Pro Val Phe Val Val Gln
                   325                 330                 335
   Trp Leu Phe Asp Glu Ala Gln Leu Thr Val Asp Asn Val His Leu Thr
               340                 345                 350
   Gly Gln Pro Val Gln Glu Gly Leu Arg Leu Tyr Ile Gln Asn Leu Gly
           355                 360                 365
   Arg Glu Leu Arg His Thr Leu Lys Asp Val Pro Ala Ser Phe Ala Pro
       370                 375                 380
   Ala Cys Leu Ser His Glu Ile Ile Ile Arg Ser His Trp Thr Asp Val
   385                 390                 395                 400
   Gln Val Lys Gly Thr Ser Leu Pro Arg Ala Leu His Cys Trp Asp Arg
                   405                 410                 415
   Ser Leu His Asp Ser His Lys Ala Ser Lys Thr Pro Leu Lys Gly Cys
               420                 425                 430
   Pro Val His Leu Val Asp Ser Cys Pro Trp Pro His Cys Asn Pro Ser
           435                 440                 445
   Cys Pro Thr Val Arg Asp Gln Phe Thr Gly Gln Glu Met Asn Val Ala
       450                 455                 460
   Gln Phe Leu Met His Met Gly Phe Asp Met Gln Thr Val Ala Gln Pro
   465                 470                 475                 480
   Gln Gly Leu Glu Pro Ser Glu Leu Leu Gly Met Leu Ser Asn Gly Ser
                   485                 490                 495
<210> 275
   <211> 31
   <212> DNA
   <213> homo sapiens
<400> 275
   caccgaattc atgggccgag gggtgcgcgt g 31
<210> 276
   <211> 30
   <212> DNA
   <213> homo sapiens
<400> 276
   ctcgaggctt ccgttgctca gcatccccag 30
<210> 277
   <211> 32
   <212> DNA
   <213> homo sapiens
<400> 277
   gaattcatgc ggcgcctcat gagctcccgg ga 32
<210> 278
   <211> 30
   <212> DNA
   <213> homo sapiens
<400> 278
   caccatggga tttggattca tctccatgat 30
<210> 279
   <211> 31
   <212> DNA
   <213> homo sapiens
<400> 279
   tgtctttttc cttttttgaa ctgaaggccc c 31
<210> 280
   <211> 14
   <212> PRT
   <213> homo sapiens
<400> 280
   Pro Glu Ala Glu Thr Arg Gly Ala Lys Arg Ile Ser Pro Ala
   1               5                   10
<210> 281
   <211> 34
   <212> DNA
   <213> homo sapiens
<400> 281
   ggatccatgg gctgtctctg gggtctggct ctgc 34
<210> 282
   <211> 34
   <212> DNA
   <213> homo sapiens
<400> 282
   ctcgaggcct ctcctgacac gcagtaagga gacc 34
<210> 283
   <211> 402
   <212> PRT
   <213> homo sapiens
<400> 283
   Gln Leu Ser Cys Gln Gly Leu Lys Val Phe Ala Gly Gly Lys Leu Pro
   1               5                   10                  15
   Gly Pro Ala Val Glu Gly Pro Ser Ala Thr Gly Pro Glu Ile Ile Arg
               20                  25                  30
   Ser Pro Gln Asp Asp Ala Ser Ser Pro His Leu Gln Val Met Leu Gln
           35                  40                  45
   Ile His Leu Pro Gly Arg His Thr Leu Phe Val Arg Ala Met Ile Asp
       50                  55                  60
   Ser Gly Ala Ser Gly Asn Phe Ile Asp His Glu Tyr Val Ala Gln Asn
   65                  70                  75                  80
   Gly Ile Pro Leu Arg Ile Lys Asp Trp Pro Ile Leu Val Glu Ala Ile
                   85                  90                  95
   Asp Gly Arg Pro Ile Ala Ser Gly Pro Val Val His Glu Thr His Asp
               100                 105                 110
   Leu Ile Val Asp Leu Gly Asp His Arg Glu Val Leu Ser Phe Asp Val
           115                 120                 125
   Thr Gln Ser Pro Phe Phe Pro Val Val Leu Gly Val Arg Trp Leu Ser
       130                 135                 140
   Thr His Asp Pro Asn Ile Thr Trp Ser Thr Arg Ser Ile Val Phe Asp
   145                 150                 155                 160
   Ser Glu Tyr Cys Arg Tyr His Cys Arg Met Tyr Ser Pro Ile Pro Pro
                   165                 170                 175
   Ser Leu Pro Pro Pro Ala Pro Gln Pro Pro Leu Tyr Tyr Pro Val Asp
               180                 185                 190
   Gly Tyr Arg Val Tyr Gln Pro Val Arg Tyr Tyr Tyr Val Gln Asn Val
           195                 200                 205
   Tyr Thr Pro Val Asp Glu His Val Tyr Pro Asp His Arg Leu Val Asp
       210                 215                 220
   Pro His Ile Glu Met Ile Pro Gly Ala His Ser Ile Pro Ser Gly His
   225                 230                 235                 240
   Val Tyr Ser Leu Ser Glu Pro Glu Met Ala Ala Leu Arg Asp Phe Val
                   245                 250                 255
   Ala Arg Asn Val Lys Asp Gly Leu Ile Thr Pro Thr Ile Ala Pro Asn
               260                 265                 270
   Gly Ala Gln Val Leu Gln Val Lys Arg Gly Trp Lys Leu Gln Val Ser
           275                 280                 285
   Tyr Asp Cys Arg Ala Pro Asn Asn Phe Thr Ile Gln Asn Gln Tyr Pro
       290                 295                 300
   Arg Leu Ser Ile Pro Asn Leu Glu Asp Gln Ala His Leu Ala Thr Tyr
   305                 310                 315                 320
   Thr Glu Phe Val Pro Gln Ile Pro Gly Tyr Gln Thr Tyr Pro Thr Tyr
                   325                 330                 335
   Ala Ala Tyr Pro Thr Tyr Pro Val Gly Phe Ala Trp Tyr Pro Val Gly
               340                 345                 350
   Arg Asp Gly Gln Gly Arg Ser Leu Tyr Val Pro Val Met Ile Thr Trp
           355                 360                 365
   Asn Pro His Trp Tyr Arg Gln Pro Pro Val Pro Gln Tyr Pro Pro Pro
       370                 375                 380
   Gln Pro Pro Pro Pro Pro Pro Pro Pro Pro Pro Pro Pro Ser Tyr Ser
   385                 390                 395                 400
   Thr Leu
<210> 284
   <211> 30
   <212> DNA
   <213> homo sapiens
<400> 284
   ggatccatga ccgaacgaag aagggacgag 30
<210> 285
   <211> 30
   <212> DNA
   <213> homo sapiens
<400> 285
   tctagacagg gtactgtaag atggaggcgg 30
<210> 286
   <211> 28
   <212> DNA
   <213> homo sapiens
<400> 286
   ggatccatct tccgggcaga cacaccct 28
<210> 287
   <211> 29
   <212> DNA
   <213> homo sapiens
<400> 287
   ctcgagtgcc atttcaggtt cggacagtg 29
<210> 288
   <211> 26
   <212> DNA
   <213> homo sapiens
<400> 288
   gaattcatgg agggtctggg ccgctc 26
<210> 289
   <211> 25
   <212> DNA
   <213> homo sapiens
<400> 289
   ctcgagggtg ggggccagct cgaag 25
<210> 290
   <211> 16
   <212> PRT
   <213> homo sapiens
<400> 290
   Lys Asp Asp Tyr Tyr Arg Arg Pro Leu Arg Val Leu Arg Ala Arg Gln
   1               5                   10                  15
<210> 291
   <211> 30
   <212> DNA
   <213> homo sapiens
<400> 291
   cgggatccat gttcctctcc atcctagtgg 30
<210> 292
   <211> 29
   <212> DNA
   <213> homo sapiens
<400> 292
   cgctcgagac actcttttcg ggtttgttc 29